# EUROPEAN PATENT APPLICATION

(11) **EP 3 816 282 A1**
(43) Date of publication of application: **05.05.2021**
(21) Application number: 19825013.6
(22) Date of filing: 27.06.2019
(51) Int. Cl.: C12N 15/04, C07K 4/06, C07K 1/00, C12N 9/14, C12N 9/02, C12N 9/00

(54) **ENZYMES OF LUCIFERIN BIOSYNTHESIS AND USE THEREOF**

(30) Priority: 28.06.2018 RU 2018123601
(71) Applicant: Light Bio, Inc, Ketchum, ID 83340 (US)
(72) Inventor: YAMPOL'SKIY, Il'ya Viktorovich, Moscow, 127083 (RU)
(74) Representative: Berggren Oy
(86) International application number: PCT/RU2019/050097
(87) International publication number: WO 2020/005120

(57) **Abstract**

Present invention is aimed at identification of new fungal luciferin biosynthesis enzymes, nucleic acids able to encode these enzymes, and proteins able to catalyze certain stages of the fungal luciferin biosynthesis. The invention also provides for application of nucleic acids for producing said enzymes in a cell or organism. Methods for *in vitro* or *in vivo* preparation of chemical compounds identical to fungal luciferins and preluciferins are also provided. Vectors comprising nucleic acid described in the present invention are also provided. In addition, the present invention provides expression cassettes comprising the nucleic acid of the present invention and regulatory elements necessary for nucleic acid expression in a selected host cell. Besides, cells, stable cell lines, transgenic organisms (e.g. plants, animals, fungi, or microorganisms) including nucleic acids, vectors, or expression cassettes of the present invention are also provided. Present invention also provides combinations of nucleic acids to obtain autonomously luminous cells, cell lines, or transgenic organisms. In preferred embodiments, cells or transgenic organisms are capable to produce fungal luciferin from precursors. In some embodiments, cells or transgenic organisms are capable to produce fungal preluciferin from precursors. In some embodiments, cells or transgenic organisms are capable of bioluminescence in the presence of a fungal luciferin precursor. In some embodiments, cells or transgenic organisms are capable of autonomous bioluminescence. Combinations of proteins for producing luciferin or its precursors from more simple chemical compounds are also provided. A kit containing nucleic acids, vectors, or expression cassettes of the present invention for producing luminous cells, cell lines, or transgenic organisms is also provided.

LIST OF SEQUENCES

<110>  Light Bio, Inc

<120>  ENZYMES OF LUCIFERIN BIOSYNTHESIS AND USE THEREOF

<130> 413825

<150> RU 2018123601
<151> 2018-06-28

<160>  141

<170>  PatentIn version 3.5

<210>  1
<211>  1266
<212>  DNA
<213>  Neonothopanus nambi

<400>  1
atggcatcgt ttgagaattc tctaagcgtt ttgattgtcg gggccggact tggtgggctt      60

gctgctgcca tcgcgctgcg tcgccaaggg catgtcgtga aaatatacga ctcctctagc     120

ttcaaagccg aacttggtgc gggactcgct gtgccgccta acaccttgcg cagtctacag     180

caacttggtt gcaataccga gaacctcaat ggtgtggata atctttgctt cactgcgatg     240

gggtatgacg ggagtgtagg gatgatgaac aacatgactg actatcgaga ggcatacggt     300

acttcttgga tcatggtcca ccgcgttgac ttgcataacg agctgatgcg cgtagcactt     360

gatccaggtg ggctcggacc tcctgcgaca ctccatctta atcatcgtgt cacattctgc     420

gatgtcgacg cttgcaccgt gacattcacc aacgggacca ctcaatcagc tgatctcatc     480

gttggtgcag acggtatacg ctctaccatt cggcggtttg tcttagaaga agacgtgact     540

gtgcctgcgt caggaatcgt cgggtttcga tggcttgtac aagctgacgc gctggaccca     600

tatcctgaac tcgactggat tgttaaaaag cctcctctag gcgcgcgact gatctccact     660

cctcagaatc cacagtctgg tgttggcttg gctgacaggc gcactatcat catctacgca     720

tgtcgtggcg gcaccatggt caatgtcctt gcagtgcatg atgacgaacg tgaccagaac     780

accgcagatt ggagtgtacc ggcttccaaa gacgatctat ttcgtgtttt ccacgattac     840

catccacgct ttcggcggct tttagagctt gcgcaggata ttaatctctg gcaaatgcgt     900

gttgtacctg ttttgaaaaa atgggttaac aagcgggttt gcttgttagg agatgctgcg     960

cacgcttctt taccgacgtt gggtcaaggt tttggtatgg gtctggaaga tgccgtagca    1020

cttggtacac tccttccaaa gggtaccact gcatctcaga tcgagactcg acttgcggtg 1080

tacgaacagc tacgtaagga tcgtgcggaa tttgttgcgg ctgaatcata tgaagagcaa 1140

tatgttcctg aaatgcgggg actttatctg aggtcaaagg aactgcgtga tagagtcatg 1200

ggttatgata tcaaagtgga gagcgagaag gttctcgaga cgctcctaag aagttctaat 1260

tctgcc 1266

<210> 2
<211> 422
<212> PRT
<213> Neonothopanus nambi

<400> 2

Met Ala Ser Phe Glu Asn Ser Leu Ser Val Leu Ile Val Gly Ala Gly
1               5                   10                  15

Leu Gly Gly Leu Ala Ala Ala Ile Ala Leu Arg Arg Gln Gly His Val
            20                  25                  30

Val Lys Ile Tyr Asp Ser Ser Ser Phe Lys Ala Glu Leu Gly Ala Gly
        35                  40                  45

Leu Ala Val Pro Pro Asn Thr Leu Arg Ser Leu Gln Gln Leu Gly Cys
    50                  55                  60

Asn Thr Glu Asn Leu Asn Gly Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly Thr Ser Trp Ile Met Val His Arg Val Asp Leu His
            100                 105                 110

Asn Glu Leu Met Arg Val Ala Leu Asp Pro Gly Gly Leu Gly Pro Pro
        115                 120                 125

Ala Thr Leu His Leu Asn His Arg Val Thr Phe Cys Asp Val Asp Ala
    130                 135                 140

Cys Thr Val Thr Phe Thr Asn Gly Thr Thr Gln Ser Ala Asp Leu Ile
145                 150                 155                 160

Val Gly Ala Asp Gly Ile Arg Ser Thr Ile Arg Arg Phe Val Leu Glu
                165                 170                 175

Glu Asp Val Thr Val Pro Ala Ser Gly Ile Val Gly Phe Arg Trp Leu
            180                 185                 190

Val Gln Ala Asp Ala Leu Asp Pro Tyr Pro Glu Leu Asp Trp Ile Val
        195                 200                 205

Lys Lys Pro Pro Leu Gly Ala Arg Leu Ile Ser Thr Pro Gln Asn Pro
    210                 215                 220

Gln Ser Gly Val Gly Leu Ala Asp Arg Arg Thr Ile Ile Ile Tyr Ala
225                 230                 235                 240

Cys Arg Gly Gly Thr Met Val Asn Val Leu Ala Val His Asp Asp Glu
                245                 250                 255

Arg Asp Gln Asn Thr Ala Asp Trp Ser Val Pro Ala Ser Lys Asp Asp
            260                 265                 270

Leu Phe Arg Val Phe His Asp Tyr His Pro Arg Phe Arg Arg Leu Leu
        275                 280                 285

Glu Leu Ala Gln Asp Ile Asn Leu Trp Gln Met Arg Val Val Pro Val
    290                 295                 300

Leu Lys Lys Trp Val Asn Lys Arg Val Cys Leu Leu Gly Asp Ala Ala
305                 310                 315                 320

His Ala Ser Leu Pro Thr Leu Gly Gln Gly Phe Gly Met Gly Leu Glu
                325                 330                 335

Asp Ala Val Ala Leu Gly Thr Leu Leu Pro Lys Gly Thr Thr Ala Ser
            340                 345                 350

Gln Ile Glu Thr Arg Leu Ala Val Tyr Glu Gln Leu Arg Lys Asp Arg

355                 360                 365

Ala Glu Phe Val Ala Ala Glu Ser Tyr Glu Glu Gln Tyr Val Pro Glu
    370                 375                 380

Met Arg Gly Leu Tyr Leu Arg Ser Lys Glu Leu Arg Asp Arg Val Met
385                 390                 395                 400

Gly Tyr Asp Ile Lys Val Glu Ser Glu Lys Val Leu Glu Thr Leu Leu
                405                 410                 415

Arg Ser Ser Asn Ser Ala
            420

<210>  3
<211>  1620
<212>  DNA
<213>  Omphalotus olearius

<400>  3
atgacgccct ccgagagtcc tttgaatatc tcgattgttg gtgctgggct cgggggcctt        60

gctgcagcta ttgcgctgcg tcgtcaaggt catatcatca gaatcttcga ctcgtcaagt       120

tttaaaacgg aactgggtgc tggacttgct gttccaccca atacattacg cagtcttcag       180

gaacttggct gtgatattca gaacttcaat gccgtggaca atctttgttt caccgcgatg       240

ggctacgacg ggagtgtagg gatgatgaac aatatgactg actatcgtga ggcgtatggt       300

gttccctggg tcatggtcca ccgcgttgac ctacataatg aactgcgacg tgtggcactc       360

gatccagatg gccttggacc tcctgcagca ttgcacctca atcatcgtgt gacatcctgc       420

gatgtcgatt cctgcaccgt cacattcgct aacggaaccg ctcatacagc ggatctcatc       480

gttggcgcgg atggtatacg ctcttccatc cgacccttcg tgttgggaga agacgtaatc       540

gtacctgcaa caggaatcgc aggatttcga tggctcatac aagccgaccg gctagatgcg       600

tatcctgaac tcgactggat tgtcaagaac cctcctctcg gcgcgcgatt gatttctgct       660

ccggctcgga aggaacgttc tgtaatcagc gaagcccggc ctgatcgacg tacgattata       720

ttatatgcgt gtcgtggtgg tactattgtc aatgtccttg cggtgcacga cgacgaacgt       780

gatcaggaca ccgtagaatg gagcgtgcca gctaccaaag acgacctatt tcgcgttttc       840

aacgattatc acccaagatt tcggcgactt ctggacctgg cggaggatgt taatctctgg       900

cagatgcgtg ttgtgcctgt tttgagacga tgggttaata aacgggtttg cttgctggga 960

gatgcagcac atgcttcttt accgacattg ggtcaaggtt ttggtatggg tctcgaggat 1020

gcggtggcac ttggtacgct tcttccgagc gggactactg tgtcacagat tgaaatccga 1080

ctttgggtgt atgaaaaact gcgcaaggag cgtgctgaat ttgtttcggc tgaatcgtat 1140

gaagaacact gctctgtgga ttgctataaa tctcataaag cccagtcgac atccaataca 1200

gtgacagaag cagacgacat cttggaagaa ccaaagcctc tgaagccttt atcgtctctg 1260

aaatggccgt acgttcccga agaaccttcg tatcctgatc ccctccaaag aaacgacccc 1320

aaacccctac aactcagaca ctacgaagca atagctacat ctcctgcagt acgggaggtc 1380

ctatcgagtc atccgaatct ccctgcttta ttgacgtcta tcgacaaact gagaggtttt 1440

gatcgcgaac gagctttaga aaaggcgttg gaggttactg cgcctgcgct tgttgatgat 1500

tcaagggctg tagcgctgga ggacgatgta ctcgcaatga gagcattggt agaagcgatt 1560

gaaggtgctg ttaggggcaa taaagaagac gcattaggtc tggattggac tggtagtact 1620

<210> 4
<211> 540
<212> PRT
<213> Omphalotus olearius

<400> 4

Met Thr Pro Ser Glu Ser Pro Leu Asn Ile Ser Ile Val Gly Ala Gly
1               5                   10                  15

Leu Gly Gly Leu Ala Ala Ala Ile Ala Leu Arg Arg Gln Gly His Ile
            20                  25                  30

Ile Arg Ile Phe Asp Ser Ser Ser Phe Lys Thr Glu Leu Gly Ala Gly
        35                  40                  45

Leu Ala Val Pro Pro Asn Thr Leu Arg Ser Leu Gln Glu Leu Gly Cys
    50                  55                  60

Asp Ile Gln Asn Phe Asn Ala Val Asp Asn Leu Cys Phe Thr Ala Met
65                  70                  75                  80

Gly Tyr Asp Gly Ser Val Gly Met Met Asn Asn Met Thr Asp Tyr Arg

                85                  90                  95

Glu Ala Tyr Gly Val Pro Trp Val Met Val His Arg Val Asp Leu His
            100                 105                 110

Asn Glu Leu Arg Arg Val Ala Leu Asp Pro Asp Gly Leu Gly Pro Pro
        115                 120                 125

Ala Ala Leu His Leu Asn His Arg Val Thr Ser Cys Asp Val Asp Ser
    130                 135                 140

Cys Thr Val Thr Phe Ala Asn Gly Thr Ala His Thr Ala Asp Leu Ile
145                 150                 155                 160

Val Gly Ala Asp Gly Ile Arg Ser Ser Ile Arg Pro Phe Val Leu Gly
                165                 170                 175

Glu Asp Val Ile Val Pro Ala Thr Gly Ile Ala Gly Phe Arg Trp Leu
            180                 185                 190

Ile Gln Ala Asp Arg Leu Asp Ala Tyr Pro Glu Leu Asp Trp Ile Val
        195                 200                 205

Lys Asn Pro Pro Leu Gly Ala Arg Leu Ile Ser Ala Pro Ala Arg Lys
    210                 215                 220

Glu Arg Ser Val Ile Ser Glu Ala Arg Pro Asp Arg Arg Thr Ile Ile
225                 230                 235                 240

Leu Tyr Ala Cys Arg Gly Gly Thr Ile Val Asn Val Leu Ala Val His
                245                 250                 255

Asp Asp Glu Arg Asp Gln Asp Thr Val Glu Trp Ser Val Pro Ala Thr
            260                 265                 270

Lys Asp Asp Leu Phe Arg Val Phe Asn Asp Tyr His Pro Arg Phe Arg
        275                 280                 285

Arg Leu Leu Asp Leu Ala Glu Asp Val Asn Leu Trp Gln Met Arg Val
    290                 295                 300

Val Pro Val Leu Arg Arg Trp Val Asn Lys Arg Val Cys Leu Leu Gly
305                 310                 315                 320

Asp Ala Ala His Ala Ser Leu Pro Thr Leu Gly Gln Gly Phe Gly Met
                325                 330                 335

Gly Leu Glu Asp Ala Val Ala Leu Gly Thr Leu Leu Pro Ser Gly Thr
            340                 345                 350

Thr Val Ser Gln Ile Glu Ile Arg Leu Trp Val Tyr Glu Lys Leu Arg
        355                 360                 365

Lys Glu Arg Ala Glu Phe Val Ser Ala Glu Ser Tyr Glu Glu His Cys
    370                 375                 380

Ser Val Asp Cys Tyr Lys Ser His Lys Ala Gln Ser Thr Ser Asn Thr
385                 390                 395                 400

Val Thr Glu Ala Asp Asp Ile Leu Glu Glu Pro Lys Pro Leu Lys Pro
                405                 410                 415

Leu Ser Ser Leu Lys Trp Pro Tyr Val Pro Glu Glu Pro Ser Tyr Pro
            420                 425                 430

Asp Pro Leu Gln Arg Asn Asp Pro Lys Pro Leu Gln Leu Arg His Tyr
        435                 440                 445

Glu Ala Ile Ala Thr Ser Pro Ala Val Arg Glu Val Leu Ser Ser His
    450                 455                 460

Pro Asn Leu Pro Ala Leu Leu Thr Ser Ile Asp Lys Leu Arg Gly Phe
465                 470                 475                 480

Asp Arg Glu Arg Ala Leu Glu Lys Ala Leu Glu Val Thr Ala Pro Ala
                485                 490                 495

Leu Val Asp Asp Ser Arg Ala Val Ala Leu Glu Asp Asp Val Leu Ala
            500                 505                 510

Met Arg Ala Leu Val Glu Ala Ile Glu Gly Ala Val Arg Gly Asn Lys

        515                 520                 525

Glu Asp Ala Leu Gly Leu Asp Trp Thr Gly Ser Thr
    530                 535                 540

<210>  5
<211>  1329
<212>  DNA
<213>  Guyanagaster necrorhiza

<400>  5
atgcaacaaa tcgacgaagt gtgcccattg aaagtgatcg ttgtaggtgc tggacttggg      60

ggcctttctg ctgccattgc ccttcgtagg caaggccatt gtgtccatat actggaatcg     120

tcaagtttca agagcgaact tggcgcaggt ctcgcagtac cgcccaatac tgtacgctca     180

cttcgaggcc taggctgtaa catcgacaat ctcaagcctg tggataatct ttgtttcact     240

gccatggcgc atgacggaag tcctggtatg atgaataaca tgacggacta tggccaggcg     300

tatggagatc cttgggtaat ggcgcatcgt gttgaccttc acaatgagct catgcgagtg     360

gcccttgaac ccgaaaaaac gggacctcct gcccagcttc gtctggacag ccaggtggca     420

tcttgcaatg tagatgcctg taccgtttct cttgtcgacg gaacaattta ttccgctgat     480

cttatcgttg gtgcagacgg aattaggtct accatacgct cctatgtttt ggacgcagaa     540

atagacatac ctcctaccgg tatcgctgga taccgttggc tcacacctgc agaagctttg     600

gagccatatc ccgaactcga ctggatcatc aagaacccac ccctaggagc acgtttaatc     660

acagctcctg tacgccgaaa cgacagcatt gagcagtcgg gtcctgctcc tatttctgag     720

aaggctgaca agcgtacgat catcatctac gcgtgccgga atggtactat gcttaacgtt     780

ctcggtgtac acgatgaccc tcgcaaccag aacgaagttg gatggaacgt gccagttacc     840

caagaaagtt tgctggattt ttttaaagac tatcatcccc gattcaagcg tctgcttgag     900

ctggctgaca atgttcatct gtggcaaatg cgcgtcgtcc cgcggcttga gacttggatc     960

aataaacgcg tgtgtttgtt gggcgattct gcgcatgcat cattaccaac actcggtcaa    1020

ggcttcggga tgggacttga ggatgccgta gcccttgcaa ccctccttcc gatgggaacc    1080

aaagtgtctg acatcgagaa ccgcctcgtc gcctacgaaa gcttgcgtaa ggggcgcgct    1140

gagtatgtgg ccatggaatc gttcgaacaa cagaatatcc cggaaaagcg aggcttgtat    1200

ctcaggtctt ctatgatgcg tgacgaaatc atgggttatg atgtcaaagc cgaggctgag    1260

aaggttttta aagaattaat gacctcgact gataaggtta cataccgtcc ccatgtggac 1320

tgtctatgg 1329

<210> 6
<211> 443
<212> PRT
<213> Guyanagaster necrorhiza

<400> 6

Met Gln Gln Ile Asp Glu Val Cys Pro Leu Lys Val Ile Val Val Gly
1               5                   10                  15

Ala Gly Leu Gly Gly Leu Ser Ala Ala Ile Ala Leu Arg Arg Gln Gly
            20                  25                  30

His Cys Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Thr
65                  70                  75                  80

Ala Met Ala His Asp Gly Ser Pro Gly Met Met Asn Asn Met Thr Asp
                85                  90                  95

Tyr Gly Gln Ala Tyr Gly Asp Pro Trp Val Met Ala His Arg Val Asp
            100                 105                 110

Leu His Asn Glu Leu Met Arg Val Ala Leu Glu Pro Glu Lys Thr Gly
        115                 120                 125

Pro Pro Ala Gln Leu Arg Leu Asp Ser Gln Val Ala Ser Cys Asn Val
    130                 135                 140

Asp Ala Cys Thr Val Ser Leu Val Asp Gly Thr Ile Tyr Ser Ala Asp
145                 150                 155                 160

Leu Ile Val Gly Ala Asp Gly Ile Arg Ser Thr Ile Arg Ser Tyr Val

165                 170                 175

Leu Asp Ala Glu Ile Asp Ile Pro Pro Thr Gly Ile Ala Gly Tyr Arg
            180                 185                 190

Trp Leu Thr Pro Ala Glu Ala Leu Glu Pro Tyr Pro Glu Leu Asp Trp
        195                 200                 205

Ile Ile Lys Asn Pro Pro Leu Gly Ala Arg Leu Ile Thr Ala Pro Val
    210                 215                 220

Arg Arg Asn Asp Ser Ile Glu Gln Ser Gly Pro Ala Pro Ile Ser Glu
225                 230                 235                 240

Lys Ala Asp Lys Arg Thr Ile Ile Ile Tyr Ala Cys Arg Asn Gly Thr
                245                 250                 255

Met Leu Asn Val Leu Gly Val His Asp Asp Pro Arg Asn Gln Asn Glu
            260                 265                 270

Val Gly Trp Asn Val Pro Val Thr Gln Glu Ser Leu Leu Asp Phe Phe
        275                 280                 285

Lys Asp Tyr His Pro Arg Phe Lys Arg Leu Leu Glu Leu Ala Asp Asn
    290                 295                 300

Val His Leu Trp Gln Met Arg Val Val Pro Arg Leu Glu Thr Trp Ile
305                 310                 315                 320

Asn Lys Arg Val Cys Leu Leu Gly Asp Ser Ala His Ala Ser Leu Pro
                325                 330                 335

Thr Leu Gly Gln Gly Phe Gly Met Gly Leu Glu Asp Ala Val Ala Leu
            340                 345                 350

Ala Thr Leu Leu Pro Met Gly Thr Lys Val Ser Asp Ile Glu Asn Arg
        355                 360                 365

Leu Val Ala Tyr Glu Ser Leu Arg Lys Gly Arg Ala Glu Tyr Val Ala
    370                 375                 380

Met Glu Ser Phe Glu Gln Gln Asn Ile Pro Glu Lys Arg Gly Leu Tyr
385                 390                 395                 400

Leu Arg Ser Ser Met Met Arg Asp Glu Ile Met Gly Tyr Asp Val Lys
                405                 410                 415

Ala Glu Ala Glu Lys Val Phe Lys Glu Leu Met Thr Ser Thr Asp Lys
            420                 425                 430

Val Thr Tyr Arg Pro His Val Asp Cys Leu Trp
        435                 440

<210>  7
<211>  1284
<212>  DNA
<213>  Panellus stipticus

<400>  7
atgtcacaag gcactgaaga ctcgccctca ctcttcgcaa tagttgtcgg tgctggtctg        60

gtcggctttg ccgctgccgt cgcgctgcgc cgtcaaggtc accgtgtcac gatctacgaa       120

tcttcaagct ttaagacaga acttggggcg ggcctcgcga tcccttcaaa cacattgcga       180

tgcttggttg gccttggatg tactgtcgcc aacatggatc ccgtcaataa tctttgtttt       240

acatcgatgg catacgatgg taccgcgggg atgaaaagcg acagctccga ctacgaggcg       300

cagtatggca ctccctggat catggcccac cgcgtcgatc tgcacaagga gcttcgtcgc       360

ctggcagtgg atcccgaggg caccggtccc cccgcagaac tgcaccttag ccaccgggtt       420

gtctcctgcg acgtcgaatc cggctccgtc acgctcctgg acggctccgt tcagtcagca       480

gacttgataa ttggggctga tggaattcgt tcaaccgttc gcaaatttgt cgtaggcgaa       540

gaaatagcaa tccccccatc tcgtacggcg ggcttccgct ggctcacaca agcaagcgct       600

cttgacccct accccgaact ggattggatc gtgaaaacgc cgccgttggg tgctcgggta       660

atctccgctc cgattcaacc tccagaagtc acccacatcg accaccgtac gatcgtcatc       720

tatgcctgtc gcggaggtcg tctcgtaaat gttctaggaa tacatgagga tctgcgcgac       780

caggattctg tcgactggaa cgtccccata acgcgagaag cgctgctcca ctttttcgga       840

gactaccacc cacggttcaa gcggctcttg gagctcgcgg aggacgtaca cgtctggcag       900

atgcgcgtgg tccccccact gccgacatgg gtgaacggcc gcgtatgcat catgggcgac       960

gcagcgcatg catcgcttcc cacactggga caagggtttg gcatgggcct cgaagacgcg      1020

gtcgcgctcg ggacgctgct ttctagttcg acgccgtcaa gcgacattcc aagccgtctc      1080

gtcgcgtacg agaagcttcg caaagcgcgc gctgagtatg tttccaaaga gtcgtacgaa      1140

cagcagcatg tccgagaaaa gagagggctg tatctccggt cccgagaaat gcgggatgtg      1200

atcatgggat acgacgtgaa gaaggaggca gaacggatct tgagcgagat cagcattgca      1260

caagaacagt gtgctgttca tgat                                            1284

<210>  8
<211>  428
<212>  PRT
<213>  Panellus stipticus

<400>  8

Met Ser Gln Gly Thr Glu Asp Ser Pro Ser Leu Phe Ala Ile Val Val
1               5                   10                  15

Gly Ala Gly Leu Val Gly Phe Ala Ala Ala Val Ala Leu Arg Arg Gln
            20                  25                  30

Gly His Arg Val Thr Ile Tyr Glu Ser Ser Ser Phe Lys Thr Glu Leu
        35                  40                  45

Gly Ala Gly Leu Ala Ile Pro Ser Asn Thr Leu Arg Cys Leu Val Gly
    50                  55                  60

Leu Gly Cys Thr Val Ala Asn Met Asp Pro Val Asn Asn Leu Cys Phe
65                  70                  75                  80

Thr Ser Met Ala Tyr Asp Gly Thr Ala Gly Met Lys Ser Asp Ser Ser
                85                  90                  95

Asp Tyr Glu Ala Gln Tyr Gly Thr Pro Trp Ile Met Ala His Arg Val
            100                 105                 110

Asp Leu His Lys Glu Leu Arg Arg Leu Ala Val Asp Pro Glu Gly Thr
        115                 120                 125

Gly Pro Pro Ala Glu Leu His Leu Ser His Arg Val Val Ser Cys Asp

130                 135                 140

Val Glu Ser Gly Ser Val Thr Leu Leu Asp Gly Ser Val Gln Ser Ala
145                 150                 155                 160

Asp Leu Ile Ile Gly Ala Asp Gly Ile Arg Ser Thr Val Arg Lys Phe
                165                 170                 175

Val Val Gly Glu Glu Ile Ala Ile Pro Pro Ser Arg Thr Ala Gly Phe
            180                 185                 190

Arg Trp Leu Thr Gln Ala Ser Ala Leu Asp Pro Tyr Pro Glu Leu Asp
        195                 200                 205

Trp Ile Val Lys Thr Pro Pro Leu Gly Ala Arg Val Ile Ser Ala Pro
    210                 215                 220

Ile Gln Pro Pro Glu Val Thr His Ile Asp His Arg Thr Ile Val Ile
225                 230                 235                 240

Tyr Ala Cys Arg Gly Gly Arg Leu Val Asn Val Leu Gly Ile His Glu
                245                 250                 255

Asp Leu Arg Asp Gln Asp Ser Val Asp Trp Asn Val Pro Ile Thr Arg
            260                 265                 270

Glu Ala Leu Leu His Phe Phe Gly Asp Tyr His Pro Arg Phe Lys Arg
        275                 280                 285

Leu Leu Glu Leu Ala Glu Asp Val His Val Trp Gln Met Arg Val Val
    290                 295                 300

Pro Pro Leu Pro Thr Trp Val Asn Gly Arg Val Cys Ile Met Gly Asp
305                 310                 315                 320

Ala Ala His Ala Ser Leu Pro Thr Leu Gly Gln Gly Phe Gly Met Gly
                325                 330                 335

Leu Glu Asp Ala Val Ala Leu Gly Thr Leu Leu Ser Ser Ser Thr Pro
            340                 345                 350

Ser Ser Asp Ile Pro Ser Arg Leu Val Ala Tyr Glu Lys Leu Arg Lys
        355                 360                 365

Ala Arg Ala Glu Tyr Val Ser Lys Glu Ser Tyr Glu Gln Gln His Val
    370                 375                 380

Arg Glu Lys Arg Gly Leu Tyr Leu Arg Ser Arg Glu Met Arg Asp Val
385                 390                 395                 400

Ile Met Gly Tyr Asp Val Lys Lys Glu Ala Glu Arg Ile Leu Ser Glu
                405                 410                 415

Ile Ser Ile Ala Gln Glu Gln Cys Ala Val His Asp
            420                 425

<210>  9
<211>  1284
<212>  DNA
<213>  Panellus stipticus

<400>  9
atgtcacaag gcactgaaga ctcgccctca ctcttcgcaa tagttgtcgg tgctggtctg        60

gtcggctttg ccgctgccgt cgcgctgcgc cgtcaaggtc accgtgtcac gatctacgaa       120

tcttcaagct ttaagacaga acttggggcg ggcctcgcga tcccttcaaa cacattgcga       180

tgcttggttg gccttggatg tactgtcgcc aacatggatc ccgtcaataa tctttgtttt       240

acatcgatgg catacgatgg taccgcgggg atgaaaagcg acagctccga ctacgaggcg       300

cagtatggca ctccctggat catggcccac cgcgtcgatc tgcacaagga gcttcgtcgc       360

ctggcagtgg atcccgaggg caccggtccc cccgcagaac tgcaccttag ccaccgggtt       420

gtctcctgcg acgtcgaatc cggctccgtc acgctcctgg acggctccgt tcagtcagca       480

gacttgataa ttggggctga tggaattcgt tcaaccgttc gcaaatttgt cgtaggcgaa       540

gaaatagcaa tccccccatc tcgtacggcg ggcttccgct ggctcacaca agcaagcgct       600

cttgacccct accccgaact ggattggatc gtgaaaacgc cgccgttggg tgctcgggta       660

atctccgctc cgattcaacc tccagaagtc acccacatcg accaccgtac gatcgtcatc       720

tatgcctgtc gcggaggtcg tctcgtaaat gttctaggaa tacatgagga tctgcgcgac       780

caggattctg tcgactggaa cgtccccata acgcgagaag cgctgctcca ctttttcgga       840

gactaccacc cacggttcaa gcggctcttg gagctcgcgg aggacgtaca cgtctggcag 900

atgcgcgtgg tccccccact gccgacatgg gtgaacggcc gcgtatgcat catgggcgac 960

gcagcgcatg catcgcttcc cacactggga caagggtttg gcatgggcct cgaagacgcg 1020

gtcgcgctcg ggacgctgct tcctagttcg acgccgtcaa gcgacattcc aagccgtctc 1080

gtcgcgtacg agaagcttcg caaagcgcgc gctgagtatg tttccaaaga gtcgtacgaa 1140

cagcagcatg tccgagaaaa gagagggctg tatctccggt cccgagaaat gcgggatgtg 1200

atcatgggat acgacgtgaa gaaggaggca gaacggatct tgagcgagat cagcattgca 1260

caagaacagt gtgctgttca tgat 1284

<210> 10
<211> 428
<212> PRT
<213> Panellus stipticus

<400> 10

Met Ser Gln Gly Thr Glu Asp Ser Pro Ser Leu Phe Ala Ile Val Val
1               5                   10                  15

Gly Ala Gly Leu Val Gly Phe Ala Ala Ala Val Ala Leu Arg Arg Gln
            20                  25                  30

Gly His Arg Val Thr Ile Tyr Glu Ser Ser Ser Phe Lys Thr Glu Leu
        35                  40                  45

Gly Ala Gly Leu Ala Ile Pro Ser Asn Thr Leu Arg Cys Leu Val Gly
    50                  55                  60

Leu Gly Cys Thr Val Ala Asn Met Asp Pro Val Asn Asn Leu Cys Phe
65                  70                  75                  80

Thr Ser Met Ala Tyr Asp Gly Thr Ala Gly Met Lys Ser Asp Ser Ser
                85                  90                  95

Asp Tyr Glu Ala Gln Tyr Gly Thr Pro Trp Ile Met Ala His Arg Val
            100                 105                 110

Asp Leu His Lys Glu Leu Arg Arg Leu Ala Val Asp Pro Glu Gly Thr

        115                 120                 125

Gly Pro Pro Ala Glu Leu His Leu Ser His Arg Val Val Ser Cys Asp
    130                 135                 140

Val Glu Ser Gly Ser Val Thr Leu Leu Asp Gly Ser Val Gln Ser Ala
145                 150                 155                 160

Asp Leu Ile Ile Gly Ala Asp Gly Ile Arg Ser Thr Val Arg Lys Phe
                165                 170                 175

Val Val Gly Glu Glu Ile Ala Ile Pro Pro Ser Arg Thr Ala Gly Phe
            180                 185                 190

Arg Trp Leu Thr Gln Ala Ser Ala Leu Asp Pro Tyr Pro Glu Leu Asp
        195                 200                 205

Trp Ile Val Lys Thr Pro Pro Leu Gly Ala Arg Val Ile Ser Ala Pro
    210                 215                 220

Ile Gln Pro Pro Glu Val Thr His Ile Asp His Arg Thr Ile Val Ile
225                 230                 235                 240

Tyr Ala Cys Arg Gly Gly Arg Leu Val Asn Val Leu Gly Ile His Glu
                245                 250                 255

Asp Leu Arg Asp Gln Asp Ser Val Asp Trp Asn Val Pro Ile Thr Arg
            260                 265                 270

Glu Ala Leu Leu His Phe Phe Gly Asp Tyr His Pro Arg Phe Lys Arg
        275                 280                 285

Leu Leu Glu Leu Ala Glu Asp Val His Val Trp Gln Met Arg Val Val
    290                 295                 300

Pro Pro Leu Pro Thr Trp Val Asn Gly Arg Val Cys Ile Met Gly Asp
305                 310                 315                 320

Ala Ala His Ala Ser Leu Pro Thr Leu Gly Gln Gly Phe Gly Met Gly
                325                 330                 335

Leu Glu Asp Ala Val Ala Leu Gly Thr Leu Leu Pro Ser Ser Thr Pro
            340                 345                 350

Ser Ser Asp Ile Pro Ser Arg Leu Val Ala Tyr Glu Lys Leu Arg Lys
        355                 360                 365

Ala Arg Ala Glu Tyr Val Ser Lys Glu Ser Tyr Glu Gln Gln His Val
    370                 375                 380

Arg Glu Lys Arg Gly Leu Tyr Leu Arg Ser Arg Glu Met Arg Asp Val
385                 390                 395                 400

Ile Met Gly Tyr Asp Val Lys Lys Glu Ala Glu Arg Ile Leu Ser Glu
                405                 410                 415

Ile Ser Ile Ala Gln Glu Gln Cys Ala Val His Asp
            420                 425

<210>  11
<211>  1284
<212>  DNA
<213>  Panellus stipticus

<400>  11
atgtcacaag gcactgaaga ctcgccctca ctcttcgcaa tagttgtcgg tgctggtctg        60

gtcggctttg ccgctgccgt cgcgctgcgc cgtcaaggtc accgtgtcac tatctacgaa       120

tcttcaagct ttaagacaga acttggggcg ggcctcgcga tcccttcaaa cacattgcga       180

tgcttggttg gccttggatg tactgtcgcc aatctggatc ccgtcaataa tctttgtttt       240

acatcgatgg cttacgatgg taccgcgggg atgaaaagcg acagctccga ctacgaggcg       300

cagtatggca ctccctggat catggcccac cgcgtcgatc tgcacaagga gcttcgtcgc       360

ctggcagtgg atcccgaggg caccggtccc cccgcagaac tgcaccttag ccaccgggtt       420

gtctcctgcg acgtcgaatc cggctccgtc acgctcctgg acggctccat tcagtcagca       480

gacttgataa ttggggctga tggaattcgt tcaaccgttc gcaaatttgt cgtaggcgaa       540

gaaatagcaa tccccccatc tcgtacggcg ggcttccgtt ggctcacaca agcaagcgct       600

cttgacccct accccgaact ggattggatc gtgaaaacgc cgccgttggg tgctcgggta       660

atctccgctc cgattcaacc tccagaagtc acccacatcg accaccgtac gatcgtcatc       720

tatgcctgtc gcggaggtcg tctcgtaaat gttctaggaa tacatgagga tctgcgcgac 780

caggattctg tcgactggaa cgtccccata acgcgagaag cgctgctcca ctttttcgga 840

gactaccacc cacggttcaa gcggctcttg gagctcgcgg aggacgtaca cgtctggcag 900

atgcgcgtgg tccccccact gccgacatgg gtgaacggcc gcgtatgcat catgggcgac 960

gcagcgcatg catcgcttcc cacactggga caagggtttg gcatgggcct cgaagacgcg 1020

gtcgcgctcg ggacgctgct tcctagttcg acgccgtcag gcgacattcc aagccgtctc 1080

gtcgcgtacg agaagcttcg caaagcgcgc gctgagtatg tttccaaaga gtcgtacgaa 1140

cagcagcatg tccgagaaaa gagagggctg tatctccggt cccgagaaat gcgggatgtg 1200

atcatgggat acgacgtgaa gaaggaggca gaacggatct ttagcgagat cagcattgca 1260

caagaacagc gtgctgttca tgat 1284

<210> 12
<211> 428
<212> PRT
<213> Panellus stipticus

<400> 12

Met Ser Gln Gly Thr Glu Asp Ser Pro Ser Leu Phe Ala Ile Val Val
1               5                   10                  15

Gly Ala Gly Leu Val Gly Phe Ala Ala Ala Val Ala Leu Arg Arg Gln
            20                  25                  30

Gly His Arg Val Thr Ile Tyr Glu Ser Ser Ser Phe Lys Thr Glu Leu
        35                  40                  45

Gly Ala Gly Leu Ala Ile Pro Ser Asn Thr Leu Arg Cys Leu Val Gly
    50                  55                  60

Leu Gly Cys Thr Val Ala Asn Leu Asp Pro Val Asn Asn Leu Cys Phe
65                  70                  75                  80

Thr Ser Met Ala Tyr Asp Gly Thr Ala Gly Met Lys Ser Asp Ser Ser
                85                  90                  95

Asp Tyr Glu Ala Gln Tyr Gly Thr Pro Trp Ile Met Ala His Arg Val

            100                 105                 110

Asp Leu His Lys Glu Leu Arg Arg Leu Ala Val Asp Pro Glu Gly Thr
        115                 120                 125

Gly Pro Pro Ala Glu Leu His Leu Ser His Arg Val Val Ser Cys Asp
    130                 135                 140

Val Glu Ser Gly Ser Val Thr Leu Leu Asp Gly Ser Ile Gln Ser Ala
145                 150                 155                 160

Asp Leu Ile Ile Gly Ala Asp Gly Ile Arg Ser Thr Val Arg Lys Phe
                165                 170                 175

Val Val Gly Glu Glu Ile Ala Ile Pro Pro Ser Arg Thr Ala Gly Phe
            180                 185                 190

Arg Trp Leu Thr Gln Ala Ser Ala Leu Asp Pro Tyr Pro Glu Leu Asp
        195                 200                 205

Trp Ile Val Lys Thr Pro Pro Leu Gly Ala Arg Val Ile Ser Ala Pro
    210                 215                 220

Ile Gln Pro Pro Glu Val Thr His Ile Asp His Arg Thr Ile Val Ile
225                 230                 235                 240

Tyr Ala Cys Arg Gly Gly Arg Leu Val Asn Val Leu Gly Ile His Glu
                245                 250                 255

Asp Leu Arg Asp Gln Asp Ser Val Asp Trp Asn Val Pro Ile Thr Arg
            260                 265                 270

Glu Ala Leu Leu His Phe Phe Gly Asp Tyr His Pro Arg Phe Lys Arg
        275                 280                 285

Leu Leu Glu Leu Ala Glu Asp Val His Val Trp Gln Met Arg Val Val
    290                 295                 300

Pro Pro Leu Pro Thr Trp Val Asn Gly Arg Val Cys Ile Met Gly Asp
305                 310                 315                 320

Ala Ala His Ala Ser Leu Pro Thr Leu Gly Gln Gly Phe Gly Met Gly
                325                 330                 335

Leu Glu Asp Ala Val Ala Leu Gly Thr Leu Leu Pro Ser Ser Thr Pro
            340                 345                 350

Ser Gly Asp Ile Pro Ser Arg Leu Val Ala Tyr Glu Lys Leu Arg Lys
        355                 360                 365

Ala Arg Ala Glu Tyr Val Ser Lys Glu Ser Tyr Glu Gln Gln His Val
    370                 375                 380

Arg Glu Lys Arg Gly Leu Tyr Leu Arg Ser Arg Glu Met Arg Asp Val
385                 390                 395                 400

Ile Met Gly Tyr Asp Val Lys Lys Glu Ala Glu Arg Ile Phe Ser Glu
                405                 410                 415

Ile Ser Ile Ala Gln Glu Gln Arg Ala Val His Asp
            420                 425

<210>  13
<211>  1248
<212>  DNA
<213>  Neonothopanus gardneri

<400>  13
atggcactat ctgagagtcc tttgaacgtc ttgatagtag gagcggggct cgggggcctt      60

gctgctgcca tagcactacg tcgtcaaggg catatcgtga aaatattcga ttcctccagt     120

ttcaaaaccg aacttggtgc aggacttgct gtcccgccta ataccctgcg tagtctgcag     180

gaactcgggt gcagtgtcga gaacctcaat gctgtggata atctttgctt cactgcgatg     240

gggtatgacg ggagtgtagg aatgatgaac aatatgaccg actatcgaga ggcgtacggt     300

catccttggg tcatggttca ccgtgtcgac ttgcataatg agctgaagcg cgtggcgctt     360

gatccagacg gcctcggacc tcctgcaact ttgcatctca accatcgtgt cacattctgc     420

gacatcgact cttgcactgt cacgttcgct aatggacttt ctaaatcggc agatcttatc     480

gtaggcgcag acggtatacg ctctaccatt cgcaagttca ttcttggaga agacgtcgtt     540

atacccgcgt caggaatagc agggtttcga tggcttgtgc aagctgacgc gctggatccg     600

tatcctgaac tcgactggat cgtgaagaac cctcctctag gagcccgact gatttccgct 660

cctaggaatc aacagtctac tgataggcgc actatcatca tctatgcgtg tcgtagcggc 720

accatggtca acgtactcgc agtacatgat gatgatcgtg accagaacgc cgtagattgg 780

agtgcaccag cttccaaaga tgatttattc cacatcttcc acgactacca cccacgattc 840

cagcggcttc tggagctggc gcaagatatc aatctctggc aaatgcgtgt tgttcctgtt 900

ctgaaacaat gggttaacaa acgtgtttgc ttgttaggag atgcggcaca cgcttcttta 960

cctacattag ggcagggatt tggtatgggt ctagaagatg ccgtagcact gggtacgctt 1020

cttccaaagg gggccacagt atctcagatc gagagccgac tcgcggtgta tgaaattctg 1080

cgcaaggagc gtgctgaatt tgtttcggct gagtcatatg aagagcagta cgttccagaa 1140

aaacgcgggc tttacttaag atcgaaggaa ttgcgcgaca gtgtcatggg ttacgatatt 1200

aaaatggaga gcgagaaggt tctcgtggca ttacttagcg gttcttca 1248

<210> 14
<211> 416
<212> PRT
<213> Neonothopanus gardneri

<400> 14

Met Ala Leu Ser Glu Ser Pro Leu Asn Val Leu Ile Val Gly Ala Gly
1               5                   10                  15

Leu Gly Gly Leu Ala Ala Ala Ile Ala Leu Arg Arg Gln Gly His Ile
            20                  25                  30

Val Lys Ile Phe Asp Ser Ser Ser Phe Lys Thr Glu Leu Gly Ala Gly
        35                  40                  45

Leu Ala Val Pro Pro Asn Thr Leu Arg Ser Leu Gln Glu Leu Gly Cys
    50                  55                  60

Ser Val Glu Asn Leu Asn Ala Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly His Pro Trp Val Met Val His Arg Val Asp Leu His
            100                 105                 110

Asn Glu Leu Lys Arg Val Ala Leu Asp Pro Asp Gly Leu Gly Pro Pro
        115                 120                 125

Ala Thr Leu His Leu Asn His Arg Val Thr Phe Cys Asp Ile Asp Ser
    130                 135                 140

Cys Thr Val Thr Phe Ala Asn Gly Thr Ser Lys Ser Ala Asp Leu Ile
145                 150                 155                 160

Val Gly Ala Asp Gly Ile Arg Ser Thr Ile Arg Lys Phe Ile Leu Gly
                165                 170                 175

Glu Asp Val Val Ile Pro Ala Ser Gly Ile Ala Gly Phe Arg Trp Leu
            180                 185                 190

Val Gln Ala Asp Ala Leu Asp Pro Tyr Pro Glu Leu Asp Trp Ile Val
        195                 200                 205

Lys Asn Pro Pro Leu Gly Ala Arg Leu Ile Ser Ala Pro Arg Asn Gln
    210                 215                 220

Gln Ser Thr Asp Arg Arg Thr Ile Ile Ile Tyr Ala Cys Arg Ser Gly
225                 230                 235                 240

Thr Met Val Asn Val Leu Ala Val His Asp Asp Asp Arg Asp Gln Asn
                245                 250                 255

Ala Val Asp Trp Ser Ala Pro Ala Ser Lys Asp Asp Leu Phe His Ile
            260                 265                 270

Phe His Asp Tyr His Pro Arg Phe Gln Arg Leu Leu Glu Leu Ala Gln
        275                 280                 285

Asp Ile Asn Leu Trp Gln Met Arg Val Val Pro Val Leu Lys Gln Trp
    290                 295                 300

Val Asn Lys Arg Val Cys Leu Leu Gly Asp Ala Ala His Ala Ser Leu

305                 310                 315                 320

Pro Thr Leu Gly Gln Gly Phe Gly Met Gly Leu Glu Asp Ala Val Ala
                325                 330                 335

Leu Gly Thr Leu Leu Pro Lys Gly Ala Thr Val Ser Gln Ile Glu Ser
            340                 345                 350

Arg Leu Ala Val Tyr Glu Ile Leu Arg Lys Glu Arg Ala Glu Phe Val
        355                 360                 365

Ser Ala Glu Ser Tyr Glu Glu Gln Tyr Val Pro Glu Lys Arg Gly Leu
    370                 375                 380

Tyr Leu Arg Ser Lys Glu Leu Arg Asp Ser Val Met Gly Tyr Asp Ile
385                 390                 395                 400

Lys Met Glu Ser Glu Lys Val Leu Val Ala Leu Leu Ser Gly Ser Ser
                405                 410                 415

<210>  15
<211>  1257
<212>  DNA
<213>  Mycena citricolor

<400>  15
atgaacaccc ccaataacgc tctcgatgtt attgttgtcg gtgctggcct ggttggtttc        60

gcggccgctg ctgctctacg ccgacaaggt catcgcgtga ctatctacga gacttccagc       120

ttcaagaacg agctaggagc tggccttgct attccaccca acactgtccg tggcctaatt       180

ggcttgggat gtgtgattga gaacttggac ccggtggaga atctatgcgt ttccgtcgct       240

tttgacggaa gtgctggtat gcgcagtgac cagaccaact acgaagcaag ctacggcctt       300

ccctggatca tggtgcatcg cgtcgatttg cacaatgagc ttcgtcgggt tgctctcagt       360

gccgagggga acattggtcc cccagccgag ctacgcctgg accaccgagt cagctcgtgt       420

gatgtcgaga aatgcactgt gacgctgagc aatggcgata cccaccacgc ggatctgatc       480

attggagcag acgggatcca ttctacaatc cgatccttcg tcgtgggcga ggaaatcgtc       540

attccgccct ccaagacagc cggtttccgc tggctcacag agagtactgc gttggagccc       600

tatccggaat tggactggat tgtgaagatc ccaccacttg gcgcccggct gatctctgcg       660

ccaatgaacc ctgcgccacc gcaggtcgac caccggacga tcatcatcta cgcctgtcgt 720

ggcagtacac tgataaatgt actcggagtc catgaggatc tccgcgatca agatacagtt 780

ccctggaatg cacccgtaac ccaatcggag ctgctccagt tctttggcga ttaccatccg 840

cgattcaaac gattgttgga gcttgcaaat gatgttcatg tgtggcagat gcgagtagtg 900

ccccgcttgg agacctgggt caatcgtcgg gtttgcatta tgggcgatgc tgcgcatgca 960

tcactcccca cgctgggtca aggtttcgga atggggctcg aggatgcagt cgctcttgga 1020

acactccttc cgcttgggac aactcccgaa gagatcccgg accgtctcac cctctggcag 1080

gatctcgtca aacctcgggc tgagtttgtc gcgactgaat cctacgaaca gcagcatatt 1140

cctgcgaaac ggggactcta tcttcgctcg caggagatgc gcgactgggt catgggatac 1200

gatgtccagg ctgaggcaca gaaggtcttg gcgggagctg tgaatagatc caaggga 1257

<210> 16
<211> 419
<212> PRT
<213> Mycena citricolor

<400> 16

Met Asn Thr Pro Asn Asn Ala Leu Asp Val Ile Val Val Gly Ala Gly
1               5                   10                  15

Leu Val Gly Phe Ala Ala Ala Ala Ala Leu Arg Arg Gln Gly His Arg
            20                  25                  30

Val Thr Ile Tyr Glu Thr Ser Ser Phe Lys Asn Glu Leu Gly Ala Gly
        35                  40                  45

Leu Ala Ile Pro Pro Asn Thr Val Arg Gly Leu Ile Gly Leu Gly Cys
    50                  55                  60

Val Ile Glu Asn Leu Asp Pro Val Glu Asn Leu Cys Val Ser Val Ala
65                  70                  75                  80

Phe Asp Gly Ser Ala Gly Met Arg Ser Asp Gln Thr Asn Tyr Glu Ala
                85                  90                  95

Ser Tyr Gly Leu Pro Trp Ile Met Val His Arg Val Asp Leu His Asn

100                 105                 110

Glu Leu Arg Arg Val Ala Leu Ser Ala Glu Gly Asn Ile Gly Pro Pro
        115                 120                 125

Ala Glu Leu Arg Leu Asp His Arg Val Ser Ser Cys Asp Val Glu Lys
    130                 135                 140

Cys Thr Val Thr Leu Ser Asn Gly Asp Thr His His Ala Asp Leu Ile
145                 150                 155                 160

Ile Gly Ala Asp Gly Ile His Ser Thr Ile Arg Ser Phe Val Val Gly
                165                 170                 175

Glu Glu Ile Val Ile Pro Pro Ser Lys Thr Ala Gly Phe Arg Trp Leu
            180                 185                 190

Thr Glu Ser Thr Ala Leu Glu Pro Tyr Pro Glu Leu Asp Trp Ile Val
        195                 200                 205

Lys Ile Pro Pro Leu Gly Ala Arg Leu Ile Ser Ala Pro Met Asn Pro
    210                 215                 220

Ala Pro Pro Gln Val Asp His Arg Thr Ile Ile Ile Tyr Ala Cys Arg
225                 230                 235                 240

Gly Ser Thr Leu Ile Asn Val Leu Gly Val His Glu Asp Leu Arg Asp
                245                 250                 255

Gln Asp Thr Val Pro Trp Asn Ala Pro Val Thr Gln Ser Glu Leu Leu
            260                 265                 270

Gln Phe Phe Gly Asp Tyr His Pro Arg Phe Lys Arg Leu Leu Glu Leu
        275                 280                 285

Ala Asn Asp Val His Val Trp Gln Met Arg Val Val Pro Arg Leu Glu
    290                 295                 300

Thr Trp Val Asn Arg Arg Val Cys Ile Met Gly Asp Ala Ala His Ala
305                 310                 315                 320

Ser Leu Pro Thr Leu Gly Gln Gly Phe Gly Met Gly Leu Glu Asp Ala
                325                 330                 335

Val Ala Leu Gly Thr Leu Leu Pro Leu Gly Thr Thr Pro Glu Glu Ile
            340                 345                 350

Pro Asp Arg Leu Thr Leu Trp Gln Asp Leu Val Lys Pro Arg Ala Glu
        355                 360                 365

Phe Val Ala Thr Glu Ser Tyr Glu Gln Gln His Ile Pro Ala Lys Arg
    370                 375                 380

Gly Leu Tyr Leu Arg Ser Gln Glu Met Arg Asp Trp Val Met Gly Tyr
385                 390                 395                 400

Asp Val Gln Ala Glu Ala Gln Lys Val Leu Ala Gly Ala Val Asn Arg
                405                 410                 415

Ser Lys Gly

<210>  17
<211>  1260
<212>  DNA
<213>  Mycena citricolor

<400>  17
atgaacaccc ccaataacgc tctcgatgtt attgttgtcg gtgctggcct ggttggtttc      60

gcggccgctg ctgctctacg ccgacaaggt catcgcgtga ctatctatga gacttccagc     120

ttcaagaacg agttaggagc tggcctggct attccaccca acactgtccg tggtctaatt     180

ggcttgggat gtgtgattga gaacttggac ccggtggaga atctatgctt cactgccgtc     240

gcgtttgacg gaagtgctgg tatgcgcagc gaccaaacca actacgaagc aagttacggc     300

cttccctgga tcatggtgca tcgtgtcgat ttgcacaacg agcttcgtcg ggttgctctc     360

agcgccgagg ggaacactgg tcccccagcg gagctacgcc tggaccaccg agtcagctcg     420

tgtgatgtcg agaaatgcac tgtgacgctg agcaatgggg atacccacca cgcagatctg     480

atcattggag cagacgggat ccattctaca atccgatcct tcgttgtggg cgaggaaatc     540

gtcattccgc cctccaagac agccggtttc cgctggctca cagagagtac tgcgttggag     600

ccctatccgg aattggactg gattgtgaag atcccaccac ttggcgcccg gctgatctct 660

gcgccaatga accctgcgcc accgcaggtc gaccaccgga cgatcatcat ctacgcctgt 720

cgtggcagta cactgataaa tgtactcgga gtccatgagg atctccgcga tcaagataca 780

gtcccctgga atgcacccgt aacccaatcg gagctgctcc agttctttgg cgattaccat 840

ccgcggttca aacgattgtt ggagcttgca aatgatgttc atgtgtggca gatgcgagta 900

gtgccccgct tggagacctg ggtcaatcgt cgggtttgca ttatgggcga tgctgcgcat 960

gcatcactcc ccacgctggg tcaaggtttc ggaatggggc tcgaggatgc agtcgctctt 1020

ggaacactcc ttccgcttgg gacaactccc gaagagatcc cggaccgtct caccctctgg 1080

caggatctcg tcaaacctcg ggctgagttt gtcgcgactg aatcctacga acagcagcat 1140

attcctgcga aacggggact ctatcttcgc tcgcaggaga tgcgcgactg ggtcatggga 1200

tacgatgtcc aggctgaggc acagaaggtc ttggcgggag ctgtgaatag atccaaggga 1260

<210> 18
<211> 420
<212> PRT
<213> Mycena citricolor

<400> 18

Met Asn Thr Pro Asn Asn Ala Leu Asp Val Ile Val Val Gly Ala Gly
1               5                   10                  15

Leu Val Gly Phe Ala Ala Ala Ala Ala Leu Arg Arg Gln Gly His Arg
            20                  25                  30

Val Thr Ile Tyr Glu Thr Ser Ser Phe Lys Asn Glu Leu Gly Ala Gly
        35                  40                  45

Leu Ala Ile Pro Pro Asn Thr Val Arg Gly Leu Ile Gly Leu Gly Cys
    50                  55                  60

Val Ile Glu Asn Leu Asp Pro Val Glu Asn Leu Cys Phe Thr Ala Val
65                  70                  75                  80

Ala Phe Asp Gly Ser Ala Gly Met Arg Ser Asp Gln Thr Asn Tyr Glu
                85                  90                  95

Ala Ser Tyr Gly Leu Pro Trp Ile Met Val His Arg Val Asp Leu His
            100                 105                 110

Asn Glu Leu Arg Arg Val Ala Leu Ser Ala Glu Gly Asn Thr Gly Pro
        115                 120                 125

Pro Ala Glu Leu Arg Leu Asp His Arg Val Ser Ser Cys Asp Val Glu
    130                 135                 140

Lys Cys Thr Val Thr Leu Ser Asn Gly Asp Thr His His Ala Asp Leu
145                 150                 155                 160

Ile Ile Gly Ala Asp Gly Ile His Ser Thr Ile Arg Ser Phe Val Val
                165                 170                 175

Gly Glu Glu Ile Val Ile Pro Pro Ser Lys Thr Ala Gly Phe Arg Trp
            180                 185                 190

Leu Thr Glu Ser Thr Ala Leu Glu Pro Tyr Pro Glu Leu Asp Trp Ile
        195                 200                 205

Val Lys Ile Pro Pro Leu Gly Ala Arg Leu Ile Ser Ala Pro Met Asn
    210                 215                 220

Pro Ala Pro Pro Gln Val Asp His Arg Thr Ile Ile Ile Tyr Ala Cys
225                 230                 235                 240

Arg Gly Ser Thr Leu Ile Asn Val Leu Gly Val His Glu Asp Leu Arg
                245                 250                 255

Asp Gln Asp Thr Val Pro Trp Asn Ala Pro Val Thr Gln Ser Glu Leu
            260                 265                 270

Leu Gln Phe Phe Gly Asp Tyr His Pro Arg Phe Lys Arg Leu Leu Glu
        275                 280                 285

Leu Ala Asn Asp Val His Val Trp Gln Met Arg Val Val Pro Arg Leu
    290                 295                 300

Glu Thr Trp Val Asn Arg Arg Val Cys Ile Met Gly Asp Ala Ala His

305                 310                 315                 320

Ala Ser Leu Pro Thr Leu Gly Gln Gly Phe Gly Met Gly Leu Glu Asp
                325                 330                 335

Ala Val Ala Leu Gly Thr Leu Leu Pro Leu Gly Thr Thr Pro Glu Glu
            340                 345                 350

Ile Pro Asp Arg Leu Thr Leu Trp Gln Asp Leu Val Lys Pro Arg Ala
        355                 360                 365

Glu Phe Val Ala Thr Glu Ser Tyr Glu Gln Gln His Ile Pro Ala Lys
    370                 375                 380

Arg Gly Leu Tyr Leu Arg Ser Gln Glu Met Arg Asp Trp Val Met Gly
385                 390                 395                 400

Tyr Asp Val Gln Ala Glu Ala Gln Lys Val Leu Ala Gly Ala Val Asn
                405                 410                 415

Arg Ser Lys Gly
            420

<210>  19
<211>  1287
<212>  DNA
<213>  Armillaria mellea

<400>  19
atgcaacaaa tcgacgaagc gcgcccattg aaagtgatag tagtgggtgc tggactttgt      60

gggctttccg ccgccattgc acttcgtagg caagggcatc atgttcatat acttgaatct     120

tcaagtttta agagcgagct tggcgcaggt ctcgccgtcc cacccaatac tgtacgctct     180

cttcgaggcc taggttgtaa catcgacaat ctcaagcccg tggataattt gtgtttctct     240

gccatggcgc atgacggaag cccaggcatg atgaataaca tgacagacta tcacaaggcg     300

tacggtgatc cttgggtaat ggcacatcgt gtcgacctcc ataacgagct cttgcgagtg     360

gctttcgacc ccgaaggaac agggcctcct gctcaacttc gtttgggcgt ccaggtagtg     420

acttgcgata tggaagcttg tacaatttcc cttgtcgatg gaacagtctg ttccgccgat     480

cttatcgtag gagctgacgg tattaagtcg accatacgct cctgtgttct aggcaaagaa     540

atagacatac ctcctaccgg tatcgccgga taccgctggc tcataccggc agaagctttg 600

gagccctatc ccgagctcga ctggattatc aagaacccac ccctaggagc acgtttaatc 660

acggatcccg tacgccgaac tgaacaaacg gatgacggta agaaggctga caagcgcacg 720

atcataatct atgcgtgccg cagtggcacg atgatcaacg ttcttggtgt gcacgatgac 780

ctgcgcaacc agaatgaagt cggatggaac gtaccagtca cacggaaaaa cttgctggag 840

tttttcgggg actaccaccc acggtttaag cgtttactcc agctagccga tagtattcat 900

ttgtggcaaa tgcgtgttgt cccacggctt gacacatgga ttaatagatg cgtgtgtttg 960

ctgggcgatt ctgcacatgc gtcattacca actctcgggc aaggcttcgg aatgggtctt 1020

gaggatgccg tagctctcgc agccctcctt ccgatgggaa ccaatgcgtc tgacgttgag 1080

aaccgcctta tcgcctacga aagcttgcgt aaggagcgtg cagagtatgt agccacggaa 1140

tcattagaac agcaggatat tgcaggaaag cgaggcttgt atctcaggtc tcctatgatg 1200

cgcgataaaa taatgggtta tgatattaaa gcggaagctg agaaggtttt aatcgaatta 1260

aaaaattcga cagctcagca ggtcact 1287

<210> 20
<211> 429
<212> PRT
<213> Armillaria mellea

<400> 20

Met Gln Gln Ile Asp Glu Ala Arg Pro Leu Lys Val Ile Val Val Gly
1               5                   10                  15

Ala Gly Leu Cys Gly Leu Ser Ala Ala Ile Ala Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Ser
65                  70                  75                  80

Ala Met Ala His Asp Gly Ser Pro Gly Met Met Asn Asn Met Thr Asp
                85                  90                  95

Tyr His Lys Ala Tyr Gly Asp Pro Trp Val Met Ala His Arg Val Asp
            100                 105                 110

Leu His Asn Glu Leu Leu Arg Val Ala Phe Asp Pro Glu Gly Thr Gly
        115                 120                 125

Pro Pro Ala Gln Leu Arg Leu Gly Val Gln Val Val Thr Cys Asp Met
    130                 135                 140

Glu Ala Cys Thr Ile Ser Leu Val Asp Gly Thr Val Cys Ser Ala Asp
145                 150                 155                 160

Leu Ile Val Gly Ala Asp Gly Ile Lys Ser Thr Ile Arg Ser Cys Val
                165                 170                 175

Leu Gly Lys Glu Ile Asp Ile Pro Pro Thr Gly Ile Ala Gly Tyr Arg
            180                 185                 190

Trp Leu Ile Pro Ala Glu Ala Leu Glu Pro Tyr Pro Glu Leu Asp Trp
        195                 200                 205

Ile Ile Lys Asn Pro Pro Leu Gly Ala Arg Leu Ile Thr Asp Pro Val
    210                 215                 220

Arg Arg Thr Glu Gln Thr Asp Asp Gly Lys Lys Ala Asp Lys Arg Thr
225                 230                 235                 240

Ile Ile Ile Tyr Ala Cys Arg Ser Gly Thr Met Ile Asn Val Leu Gly
                245                 250                 255

Val His Asp Asp Leu Arg Asn Gln Asn Glu Val Gly Trp Asn Val Pro
            260                 265                 270

Val Thr Arg Glu Asn Leu Leu Glu Phe Phe Gly Asp Tyr His Pro Arg
        275                 280                 285

Phe Lys Arg Leu Leu Gln Leu Ala Asp Ser Ile His Leu Trp Gln Met

290                 295                 300

Arg Val Val Pro Arg Leu Asp Thr Trp Ile Asn Arg Cys Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Val Ala Leu Ala Ala Leu Leu Pro Met
            340                 345                 350

Gly Thr Asn Ala Ser Asp Val Glu Asn Arg Leu Ile Ala Tyr Glu Ser
        355                 360                 365

Leu Arg Lys Glu Arg Ala Glu Tyr Val Ala Thr Glu Ser Leu Glu Gln
    370                 375                 380

Gln Asp Ile Ala Gly Lys Arg Gly Leu Tyr Leu Arg Ser Pro Met Met
385                 390                 395                 400

Arg Asp Lys Ile Met Gly Tyr Asp Ile Lys Ala Glu Ala Glu Lys Val
                405                 410                 415

Leu Ile Glu Leu Lys Asn Ser Thr Ala Gln Gln Val Thr
            420                 425

<210>  21
<211>  1278
<212>  DNA
<213>  Armillaria fuscipes

<400>  21
atgcaacaaa tcgacgaagc gcgtccattg aaggtgctag tcgtgggtgc tggactctgt      60

gggctttccg ccgccatcgc acttcgtaga caagggcatc acgtccatat acttgaatct     120

tcaagtttca agagcgaact tggggcaggt ctcgccgtgc cacctaatac tgtgcgctct     180

cttcgaggtc tcggctgtga catagacaat ctcaagcccg tggataatct ttgtttcact     240

gctatgtcgc atgacggaag cccaggcatg atgaataaca tgacggacta tcgcaaggcg     300

tatggtgatc cttgggtgat ggcacatcgt gtcgaccttc ataacgagct tatgcgagtg     360

gctctcgacc ctgatgggac aggtcctcct gcccaacttc gtttgggcgt ccaggttgtg     420

tcttgcgatg ttgaagcttg tactgtttcc cttgtcgatg gagaggtctg ttccgccgat 480

cttatcgttg gagctgatgg tatcaggtca accatacgct cctatgttct aggcaaagaa 540

atagatatac ctcctaccgg cattgccgga taccgctggc ttacaccatc agaggctttg 600

gagccttttc ccgaacttga ttggattatc aagaacccac ctctaggagc acgtctaatc 660

accgctccca tacgccggaa cgaacaaatg aatgacggtg agatggctga caagcgtacg 720

atcatcatct acgcgtgccg caacggcaca atgattaacg ttctcggtgt gcacgatgac 780

ccgcgcaacc agaatgaagt cggatggaac gtgccagtaa cccaagaaaa attgctcgaa 840

tttttcggag actaccaccc acggttcaaa agtttacttc agctatctga tagtattcat 900

ttgtggcaaa tgcgtgttgt tccacggctt gacacatgga tcaatcaacg tgtgtgtttg 960

ctgggcgatt ctgcacatgc gtcattacca acgctcgggc aaggcttcgg gatgggtctt 1020

gaggacgcca tagctcttgc aaccctcctt ccgatgggcg ccaaagtgtc ggacattgag 1080

aatcgcctta tcgcctacga aagcctgcgt aaggagcgtg cagagtttgt agccacggaa 1140

tcattagaac agcaagatat tcccgaaaag cgaggcttgt atctcagatc ccctatgatg 1200

cgcgataaaa taatgggtta cgatatcaaa gccgaagctg aaaaggtttt aatggagtta 1260

ttgagctcga aagctcaa 1278

<210> 22
<211> 426
<212> PRT
<213> Armillaria fuscipes

<400> 22

Met Gln Gln Ile Asp Glu Ala Arg Pro Leu Lys Val Leu Val Val Gly
1               5                   10                  15

Ala Gly Leu Cys Gly Leu Ser Ala Ala Ile Ala Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asp Ile Asp Asn Leu Lys Pro Val Asp Asn Leu Cys Phe Thr
65                  70                  75                  80

Ala Met Ser His Asp Gly Ser Pro Gly Met Met Asn Asn Met Thr Asp
                85                  90                  95

Tyr Arg Lys Ala Tyr Gly Asp Pro Trp Val Met Ala His Arg Val Asp
            100                 105                 110

Leu His Asn Glu Leu Met Arg Val Ala Leu Asp Pro Asp Gly Thr Gly
        115                 120                 125

Pro Pro Ala Gln Leu Arg Leu Gly Val Gln Val Val Ser Cys Asp Val
    130                 135                 140

Glu Ala Cys Thr Val Ser Leu Val Asp Gly Glu Val Cys Ser Ala Asp
145                 150                 155                 160

Leu Ile Val Gly Ala Asp Gly Ile Arg Ser Thr Ile Arg Ser Tyr Val
                165                 170                 175

Leu Gly Lys Glu Ile Asp Ile Pro Pro Thr Gly Ile Ala Gly Tyr Arg
            180                 185                 190

Trp Leu Thr Pro Ser Glu Ala Leu Glu Pro Phe Pro Glu Leu Asp Trp
        195                 200                 205

Ile Ile Lys Asn Pro Pro Leu Gly Ala Arg Leu Ile Thr Ala Pro Ile
    210                 215                 220

Arg Arg Asn Glu Gln Met Asn Asp Gly Glu Met Ala Asp Lys Arg Thr
225                 230                 235                 240

Ile Ile Ile Tyr Ala Cys Arg Asn Gly Thr Met Ile Asn Val Leu Gly
                245                 250                 255

Val His Asp Asp Pro Arg Asn Gln Asn Glu Val Gly Trp Asn Val Pro
            260                 265                 270

Val Thr Gln Glu Lys Leu Leu Glu Phe Phe Gly Asp Tyr His Pro Arg

275                 280                 285

Phe Lys Ser Leu Leu Gln Leu Ser Asp Ser Ile His Leu Trp Gln Met
    290                 295                 300

Arg Val Val Pro Arg Leu Asp Thr Trp Ile Asn Gln Arg Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Ile Ala Leu Ala Thr Leu Leu Pro Met
            340                 345                 350

Gly Ala Lys Val Ser Asp Ile Glu Asn Arg Leu Ile Ala Tyr Glu Ser
        355                 360                 365

Leu Arg Lys Glu Arg Ala Glu Phe Val Ala Thr Glu Ser Leu Glu Gln
    370                 375                 380

Gln Asp Ile Pro Glu Lys Arg Gly Leu Tyr Leu Arg Ser Pro Met Met
385                 390                 395                 400

Arg Asp Lys Ile Met Gly Tyr Asp Ile Lys Ala Glu Ala Glu Lys Val
                405                 410                 415

Leu Met Glu Leu Leu Ser Ser Lys Ala Gln
            420                 425

<210>  23
<211>  1266
<212>  DNA
<213>  Armillaria gallica

<400>  23
atgcaacaaa tcgacgaagc gcgcgcattg aaagtgatag tcgtgggtgc tggactttgc      60

gggctttccg ccgccattgt acttcgtagg caagggcatc acgtccatat acttgaatct     120

tcaagtttca agagcgaact tggtgcaggt cttgccgtgc cgcccaacac tgtacgctct     180

cttcgaggtc taggttgtaa catcgacaat ctcaagcccg tggataatct ttgtttcact     240

gccatggctc atgacggaag cccaggcatg atgaataaca tgacagacta tcagaaggcg     300

tacggcgatc cttgggtaat ggcgcatcgt gtcgacctcc ataacgagct catgcgagtg 360

gctctcgacc ctgaaggaac aggccctgct gcccagcttc gtttgggcgt ccaggtggtg 420

tcttgtgatg tggaagcttg taccatttct cttgtcgatg gatcaatctg taccgccgat 480

cttatcgtcg gagctgatgg tattaggtca accatacggt cctatgtttt gggcaaagaa 540

atagacatac ctcctaccgg tattgctgga taccgctggc tcacaccggc agaagctttg 600

gacccatatc ccgaactcga ctggattatc aagaacccac ccctgggagc acgtttaatc 660

acagctcccg ttcgccgaaa cgataaggcg gatgacggtg agaaagctga caagcgcacg 720

atcataatct acgcgtgccg cagtggcact atgatcaacg ttctcggtgt gcacgatgac 780

ccgcgcaacc agaatgaagt cggatggaac gtaccagtta cccaggaaaa tttgttggag 840

tttttcgaag actaccaccc acggtttaag cgtttacttc agctgaccga taacattcat 900

ttgtggcaaa tgcgtgttgt cccgcggctt gacacatgga ttaataaacg cgtgtgtttg 960

ctgggcgatt ctgcacatgc gtcattacca acactcgggc aaggcttcgg gatgggtctt 1020

gaggacgccg tagctctcgc aaccctcctt ccgatgggaa ccaaattgtc tgacattgaa 1080

aaccgtcttg tcgcctacga aagcttgcgt aaggagcgtg ctgagtatgt agccacggaa 1140

tcattagaac agcaggatat tccgggaaag cgaggcttgt atctcaggtc tcctgtgatg 1200

cgcgataaaa taatgggtta tgatatcaaa gccgaagctg agaaggtttt aatggaattg 1260

ataacc 1266

<210> 24
<211> 422
<212> PRT
<213> Armillaria gallica

<400> 24

Met Gln Gln Ile Asp Glu Ala Arg Ala Leu Lys Val Ile Val Val Gly
1               5                   10                  15

Ala Gly Leu Cys Gly Leu Ser Ala Ala Ile Val Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Thr
65                  70                  75                  80

Ala Met Ala His Asp Gly Ser Pro Gly Met Met Asn Asn Met Thr Asp
                85                  90                  95

Tyr Gln Lys Ala Tyr Gly Asp Pro Trp Val Met Ala His Arg Val Asp
            100                 105                 110

Leu His Asn Glu Leu Met Arg Val Ala Leu Asp Pro Glu Gly Thr Gly
        115                 120                 125

Pro Ala Ala Gln Leu Arg Leu Gly Val Gln Val Val Ser Cys Asp Val
    130                 135                 140

Glu Ala Cys Thr Ile Ser Leu Val Asp Gly Ser Ile Cys Thr Ala Asp
145                 150                 155                 160

Leu Ile Val Gly Ala Asp Gly Ile Arg Ser Thr Ile Arg Ser Tyr Val
                165                 170                 175

Leu Gly Lys Glu Ile Asp Ile Pro Pro Thr Gly Ile Ala Gly Tyr Arg
            180                 185                 190

Trp Leu Thr Pro Ala Glu Ala Leu Asp Pro Tyr Pro Glu Leu Asp Trp
        195                 200                 205

Ile Ile Lys Asn Pro Pro Leu Gly Ala Arg Leu Ile Thr Ala Pro Val
    210                 215                 220

Arg Arg Asn Asp Lys Ala Asp Asp Gly Glu Lys Ala Asp Lys Arg Thr
225                 230                 235                 240

Ile Ile Ile Tyr Ala Cys Arg Ser Gly Thr Met Ile Asn Val Leu Gly
                245                 250                 255

Val His Asp Asp Pro Arg Asn Gln Asn Glu Val Gly Trp Asn Val Pro

260                 265                 270

Val Thr Gln Glu Asn Leu Leu Glu Phe Phe Glu Asp Tyr His Pro Arg
        275                 280                 285

Phe Lys Arg Leu Leu Gln Leu Thr Asp Asn Ile His Leu Trp Gln Met
    290                 295                 300

Arg Val Val Pro Arg Leu Asp Thr Trp Ile Asn Lys Arg Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Val Ala Leu Ala Thr Leu Leu Pro Met
            340                 345                 350

Gly Thr Lys Leu Ser Asp Ile Glu Asn Arg Leu Val Ala Tyr Glu Ser
        355                 360                 365

Leu Arg Lys Glu Arg Ala Glu Tyr Val Ala Thr Glu Ser Leu Glu Gln
    370                 375                 380

Gln Asp Ile Pro Gly Lys Arg Gly Leu Tyr Leu Arg Ser Pro Val Met
385                 390                 395                 400

Arg Asp Lys Ile Met Gly Tyr Asp Ile Lys Ala Glu Ala Glu Lys Val
                405                 410                 415

Leu Met Glu Leu Ile Thr
            420

<210>  25
<211>  1278
<212>  DNA
<213>  Armillaria ostoyae

<400>  25
atgcaacaaa tcgacgaagc gcacccattg acagtgatag tcgtgggtgc tggactttgc        60

gggctttccg ccgccattgc acttcgtagg caagggcatc acgtccatat acttgaatct       120

tcaagtttca aaagcgaact tggcgcaggt ctcgccgtgc cgcccaacac tgtacgctct       180

cttcgaggtc taggttgtaa catcgacaat ctcaagcccg tggataatct ttgtttcact 240

gccatggcgc atgacggaag cccaggcatg atgaataaca tgacagacta tcacaaggcg 300

tacggcgagc cttgggtaat ggcgcatcgt gtcgacctcc ataacgagct catgcgagtg 360

gctctcgacc ctgaaggaac aggtcctcct gctcagcttc gtttgggtgt ccaggtggtg 420

tcttgcgatg tggaagcttg taccatttct cttgtcgatg gatcaatctg ttccgccgat 480

cttatcgtcg gagctgatgg tattaggtca accatacgct cctatgtttt gggcaaagaa 540

atagacatac ctcctaccgg tattgctgga taccgttggc tcacaccggc agaagctttg 600

gagccatatc ccgaactcga ctggattatc aagaacccac ccctgggagc acgtttaatc 660

acggctcccg tacgccgaaa cgataagacg gatgacggtg agaagactga caagcgcacg 720

atcataatct acgcgtgccg caatggcact atgatcaacg ttctcggtgt gcacgatgac 780

ccgcgcaacc agaatgaagt cggatggaac gtaccagtta cccaggaaaa tttgttggag 840

tttttcgaag actaccaccc acggtttaag cgtttacttc agttggccga tagtattcat 900

ttgtggcaaa tgcgtgttgt cccacggctt gacacatgga ttaataaacg cgtgtgtttg 960

ctgggcgatt ctgcacatgc gtcattacca acactcgggc aaggcttcgg gatgggtctt 1020

gaggacgccg tagctctcgc agccctcctt ccgatgggaa ccaaagtgtc tgacgttgag 1080

agtcgtctta tcgcctacga aagcttgcgt aaggagcgtg ctgagtatgt agccacggaa 1140

tcattagaac agcagaatat tccgggaaag cgaggcttgt atctcaggtc tcctatgatg 1200

cgcgataaaa taatgggtta tgatatcaaa gccgaagctg agaaggtttt aatggaatta 1260

ataacctcga cagctcag 1278

<210> 26
<211> 426
<212> PRT
<213> Armillaria ostoyae

<400> 26

Met Gln Gln Ile Asp Glu Ala His Pro Leu Thr Val Ile Val Val Gly
1               5                   10                  15

Ala Gly Leu Cys Gly Leu Ser Ala Ala Ile Ala Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Thr
65                  70                  75                  80

Ala Met Ala His Asp Gly Ser Pro Gly Met Met Asn Asn Met Thr Asp
                85                  90                  95

Tyr His Lys Ala Tyr Gly Glu Pro Trp Val Met Ala His Arg Val Asp
            100                 105                 110

Leu His Asn Glu Leu Met Arg Val Ala Leu Asp Pro Glu Gly Thr Gly
        115                 120                 125

Pro Pro Ala Gln Leu Arg Leu Gly Val Gln Val Val Ser Cys Asp Val
    130                 135                 140

Glu Ala Cys Thr Ile Ser Leu Val Asp Gly Ser Ile Cys Ser Ala Asp
145                 150                 155                 160

Leu Ile Val Gly Ala Asp Gly Ile Arg Ser Thr Ile Arg Ser Tyr Val
                165                 170                 175

Leu Gly Lys Glu Ile Asp Ile Pro Pro Thr Gly Ile Ala Gly Tyr Arg
            180                 185                 190

Trp Leu Thr Pro Ala Glu Ala Leu Glu Pro Tyr Pro Glu Leu Asp Trp
        195                 200                 205

Ile Ile Lys Asn Pro Pro Leu Gly Ala Arg Leu Ile Thr Ala Pro Val
    210                 215                 220

Arg Arg Asn Asp Lys Thr Asp Asp Gly Glu Lys Thr Asp Lys Arg Thr
225                 230                 235                 240

Ile Ile Ile Tyr Ala Cys Arg Asn Gly Thr Met Ile Asn Val Leu Gly

245                 250                 255

Val His Asp Asp Pro Arg Asn Gln Asn Glu Val Gly Trp Asn Val Pro
            260                 265                 270

Val Thr Gln Glu Asn Leu Leu Glu Phe Phe Glu Asp Tyr His Pro Arg
        275                 280                 285

Phe Lys Arg Leu Leu Gln Leu Ala Asp Ser Ile His Leu Trp Gln Met
    290                 295                 300

Arg Val Val Pro Arg Leu Asp Thr Trp Ile Asn Lys Arg Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Val Ala Leu Ala Ala Leu Leu Pro Met
            340                 345                 350

Gly Thr Lys Val Ser Asp Val Glu Ser Arg Leu Ile Ala Tyr Glu Ser
        355                 360                 365

Leu Arg Lys Glu Arg Ala Glu Tyr Val Ala Thr Glu Ser Leu Glu Gln
    370                 375                 380

Gln Asn Ile Pro Gly Lys Arg Gly Leu Tyr Leu Arg Ser Pro Met Met
385                 390                 395                 400

Arg Asp Lys Ile Met Gly Tyr Asp Ile Lys Ala Glu Ala Glu Lys Val
                405                 410                 415

Leu Met Glu Leu Ile Thr Ser Thr Ala Gln
            420                 425

<210>  27
<211>  1266
<212>  DNA
<213>  Mycena chlorophos

<400>  27
atgccctcca ccgccgaatc tccgcagccg ctcaagatcg tcatcgtcgg tgctgggctc        60

gttggtctcg ctgctgccat tgcgcttcgt cgcgagggtc atcatgtaga gatatacgaa 120

tcgtcgacgt tcaagaccga actcggcgcc ggtctcgcga taccgtgcaa taccctccgt 180

agcctcattg agctggggtg cattgtggct aacttgaacc cggtggagaa cctctgtttc 240

acgtctatgg cgcacgacgg aagcgagtca ggcatgcgaa gcgaccacac cgactacgag 300

gcgcgttatg ggaccccttg ggtcatggcg catcgcgtcg acatacacgc agagctgctc 360

cgaatggcca ccacctccga tattcccggc ccaccggcga cactgcatct cggccaacgc 420

gtccttgcct gcaatgtgtc cgactgctcc attgcactgg ccaccggcaa aacgatctca 480

gcggatctcg tcgttggtgc cgatgggatt cgctcgacca ttcgagctgc tgttcttggc 540

gaagatatcc acattccggc atcgggcact gccggcttcc gatggctcgt agattccgcc 600

gcgctggatc cctatcccga gctggactgg attgtgaaag cccgtccgct tggcgctcgc 660

gttatttctg ccccgatggg cctcgcactc gaagatcatc gtaccattgt gatctatgcg 720

tgtcgcggcg gtaacttgat caacgttctt gcggtccacg aagacaagcg cgaccaggag 780

gctgtccctt ggaatgtccc tttgacgcgc gaagccctct tggacttctt tagcgactac 840

cacccgcgtt tccgccgtct cttcgagctc gcgccggtcg acggaattca cgtctggcag 900

atgcgggtcg taccaccttt ggccaactgg atccgtgacc gcgtttgcat tctcggcgac 960

gcggcgcatg cgtctcttcc tactatgggc caggctttgg gccaaggtct cgaagacgcc 1020

gttgcgctag cgactttgct cccgctagga acgcgtagaa cggatatccc cgctcgtcta 1080

gtggcgtatg aggggatgcg caagcctcgg accgagtgga ttgcacgcga atcgtttgag 1140

cagcaggccg tcgcggaaaa gcgcggcatt tacttgcgct ctatcgaaat gcgcgatgcg 1200

gttatggggt ataatgttcg cgaggaggct aagcgcgtct tgtccgagct cactaaatct 1260

gattgt 1266

<210> 28
<211> 422
<212> PRT
<213> Mycena chlorophos

<400> 28

Met Pro Ser Thr Ala Glu Ser Pro Gln Pro Leu Lys Ile Val Ile Val
1               5                   10                  15

Gly Ala Gly Leu Val Gly Leu Ala Ala Ala Ile Ala Leu Arg Arg Glu
            20                  25                  30

Gly His His Val Glu Ile Tyr Glu Ser Ser Thr Phe Lys Thr Glu Leu
        35                  40                  45

Gly Ala Gly Leu Ala Ile Pro Cys Asn Thr Leu Arg Ser Leu Ile Glu
    50                  55                  60

Leu Gly Cys Ile Val Ala Asn Leu Asn Pro Val Glu Asn Leu Cys Phe
65                  70                  75                  80

Thr Ser Met Ala His Asp Gly Ser Glu Ser Gly Met Arg Ser Asp His
                85                  90                  95

Thr Asp Tyr Glu Ala Arg Tyr Gly Thr Pro Trp Val Met Ala His Arg
            100                 105                 110

Val Asp Ile His Ala Glu Leu Leu Arg Met Ala Thr Thr Ser Asp Ile
        115                 120                 125

Pro Gly Pro Pro Ala Thr Leu His Leu Gly Gln Arg Val Leu Ala Cys
    130                 135                 140

Asn Val Ser Asp Cys Ser Ile Ala Leu Ala Thr Gly Lys Thr Ile Ser
145                 150                 155                 160

Ala Asp Leu Val Val Gly Ala Asp Gly Ile Arg Ser Thr Ile Arg Ala
                165                 170                 175

Ala Val Leu Gly Glu Asp Ile His Ile Pro Ala Ser Gly Thr Ala Gly
            180                 185                 190

Phe Arg Trp Leu Val Asp Ser Ala Ala Leu Asp Pro Tyr Pro Glu Leu
        195                 200                 205

Asp Trp Ile Val Lys Ala Arg Pro Leu Gly Ala Arg Val Ile Ser Ala
    210                 215                 220

Pro Met Gly Leu Ala Leu Glu Asp His Arg Thr Ile Val Ile Tyr Ala

225                 230                 235                 240

Cys Arg Gly Gly Asn Leu Ile Asn Val Leu Ala Val His Glu Asp Lys
                245                 250                 255

Arg Asp Gln Glu Ala Val Pro Trp Asn Val Pro Leu Thr Arg Glu Ala
            260                 265                 270

Leu Leu Asp Phe Phe Ser Asp Tyr His Pro Arg Phe Arg Arg Leu Phe
        275                 280                 285

Glu Leu Ala Pro Val Asp Gly Ile His Val Trp Gln Met Arg Val Val
    290                 295                 300

Pro Pro Leu Ala Asn Trp Ile Arg Asp Arg Val Cys Ile Leu Gly Asp
305                 310                 315                 320

Ala Ala His Ala Ser Leu Pro Thr Met Gly Gln Gly Phe Gly Gln Gly
                325                 330                 335

Leu Glu Asp Ala Val Ala Leu Ala Thr Leu Leu Pro Leu Gly Thr Arg
            340                 345                 350

Arg Thr Asp Ile Pro Ala Arg Leu Val Ala Tyr Glu Gly Met Arg Lys
        355                 360                 365

Pro Arg Thr Glu Trp Ile Ala Arg Glu Ser Phe Glu Gln Gln Ala Val
    370                 375                 380

Ala Glu Lys Arg Gly Ile Tyr Leu Arg Ser Ile Glu Met Arg Asp Ala
385                 390                 395                 400

Val Met Gly Tyr Asn Val Arg Glu Glu Ala Lys Arg Val Leu Ser Glu
                405                 410                 415

Leu Thr Lys Ser Asp Cys
            420

<210>  29
<211>  72
<212>  PRT

<213>  Artificial sequence

<220>
<223>  Consensus amino acid sequence 1 for
       hispidin hydroxylase

<220>
<221>  MISC_FEATURE
<222>  (1)..(72)
<223>  X - any amino acid

<400>  29

Val Gly Ala Gly Leu Xaa Gly Xaa Xaa Ala Ala Xaa Xaa Leu Arg Arg
1               5                   10                  15

Xaa Gly His Xaa Xaa Xaa Xaa Xaa Xaa Xaa Ser Xaa Phe Lys Xaa Glu
            20                  25                  30

Xaa Gly Ala Gly Xaa Ala Xaa Pro Xaa Asn Thr Xaa Xaa Xaa Leu Xaa
        35                  40                  45

Xaa Leu Gly Cys Xaa Xaa Xaa Asn Xaa Xaa Xaa Val Xaa Asn Leu Cys
    50                  55                  60

Xaa Xaa Xaa Xaa Xaa Xaa Asp Gly
65                  70

<210>  30
<211>  33
<212>  PRT
<213>  Artificial sequence

<220>
<223>  Consensus amino acid sequence 2 for
       hispidin hydroxylase

<220>
<221>  MISC_FEATURE
<222>  (1)..(33)
<223>  X - any amino acid

<400>  30

Gly Met Xaa Xaa Xaa Xaa Xaa Xaa Tyr Xaa Xaa Xaa Tyr Gly Xaa Xaa
1               5                   10                  15

Trp Xaa Met Xaa His Arg Val Asp Xaa His Xaa Glu Leu Xaa Arg Xaa
            20                  25                  30

Ala

<210>  31
<211>  96
<212>  PRT
<213>  Artificial sequence

<220>
<223>  Consensus amino acid sequence 3 for
       hispidin hydroxylase

<220>
<221>  MISC_FEATURE
<222>  (1)..(96)
<223>  X - any amino acid

<400>  31

Gly Pro Xaa Ala Xaa Leu Xaa Leu Xaa Xaa Xaa Val Xaa Xaa Cys Xaa
1               5                   10                  15

Xaa Xaa Xaa Xaa Xaa Xaa Xaa Xaa Xaa Xaa Gly Xaa Xaa Xaa Xaa Ala
            20                  25                  30

Asp Leu Xaa Xaa Gly Ala Asp Gly Ile Xaa Ser Xaa Xaa Arg Xaa Xaa
        35                  40                  45

Xaa Xaa Xaa Xaa Xaa Xaa Xaa Xaa Pro Xaa Xaa Xaa Xaa Xaa Gly Xaa
    50                  55                  60

Arg Trp Xaa Xaa Xaa Xaa Xaa Xaa Leu Xaa Xaa Xaa Pro Glu Leu Asp
65                  70                  75                  80

Trp Xaa Xaa Lys Xaa Xaa Pro Leu Gly Ala Arg Xaa Xaa Xaa Xaa Pro
                85                  90                  95

<210>  32
<211>  57
<212>  PRT
<213>  Artificial sequence

<220>
<223>  Amino acid consensus sequence 4 for
       hispidin hydroxylase

<220>
<221>  MISC_FEATURE
<222>  (1)..(57)
<223>  X - any amino acid

<400>  32

Arg Thr Ile Xaa Xaa Tyr Ala Cys Arg Xaa Xaa Xaa Xaa Xaa Asn Val
1               5                   10                  15

Leu Xaa Xaa His Xaa Asp Xaa Arg Xaa Gln Xaa Xaa Xaa Xaa Trp Xaa
            20                  25                  30

Xaa Pro Xaa Xaa Xaa Xaa Xaa Xaa Xaa Xaa Xaa Xaa Xaa Asp Tyr His
        35                  40                  45

Pro Arg Phe Xaa Xaa Leu Xaa Xaa Leu
    50                  55

<210>  33
<211>  83
<212>  PRT
<213>  Artificial sequence

<220>
<223>  Consensus amino acid sequence 5 for
       hispidin hydroxylase

<220>
<221>  MISC_FEATURE
<222>  (1)..(82)
<223>  X - any amino acid

<400>  33

Trp Gln Xaa Arg Val Xaa Pro Xaa Leu Xaa Xaa Trp Xaa Xaa Xaa Xaa
1               5                   10                  15

Xaa Cys Xaa Xaa Gly Asp Xaa Ala His Ala Ser Leu Pro Thr Xaa Gly
            20                  25                  30

Gln Gly Phe Gly Xaa Gly Leu Glu Asp Ala Xaa Ala Leu Xaa Xaa Leu
        35                  40                  45

Leu Xaa Xaa Xaa Xaa Xaa Xaa Xaa Xaa Xaa Xaa Xaa Arg Leu Xaa Xaa
    50                  55                  60

Xaa Xaa Xaa Xaa Xaa Lys Xaa Arg Xaa Glu Xaa Xaa Xaa Xaa Glu Ser
65                  70                  75                  80

Xaa Glu Gln

<210>  34
<211>  4832
<212>  DNA
<213>  Neonothopanus nambi

<400>  34
aaaaccatcc ttatattctc gctttgatgc tggctgtatg gaaacttgga ggcaccttcg        60

ctcctattga tgtccattct cctgccgaat tggtagctgg catgctgaac atagtctctc       120

cttcttgctt ggttattccg agctcagatg taactaatca aactcttgcg tgcgatctta       180

atatccccgt cgttgcattt cacccacatc aatccactat tcctgagctg aacaagaagt       240

acctcaccga ttctcaaatt tctccggatc ttcctttttc agatccaaac cggcctgctc        300

tgtacctctt cacttcgtcc gccacttctc gaagtaatct caaatgcgtg cctctcactc       360

acacctttat cttacgcaac agcctctcga agcgtgcatg gtgcaagcgt atgcgtccag        420

agacagactt tgacggcata cgcgttcttg gatgggcccc gtggtctcac gtcctagcac       480

acatgcaaga catcggacca ctcaccttac ttaatgccgg atgctacgtt tttgcgacta       540

ctccatccac gtaccctacg gaattgaagg acgacaggga cctgatatct tgcgcggcaa       600

atgctatcat gtacaagggc gtcaagtcat ttgcttgtct tccctttgta ctcggagggc       660

tgaaggcatt atgcgagtct gagccatccg tgaaggcgca tctacaggtc gaggagagag       720

ctcaactcct gaagtctctg caacacatgg aaattcttga gtgtggaggt gccatgctcg       780

aagcaagtgt tgcgtcttgg gctattgaga actgcattcc catttcgatc ggtattggta       840

tgacggagac tggtggagcg ctctttgcag gccccgttca ggccatcaaa accgggtttt       900

cttcagagga taaattcatt gaagatgcta cttacttgct cgttaaggat gatcatgaga       960

gtcatgctga ggaggatatt aacgagggtg aactagttgt gaaaagtaaa atgctcccac 1020

gaggctacct tggctatagt gatccttcct tctcagtcga cgatgctggc tgggttacat 1080

ttagaacagg agacagatac agcgttacac ctgacggaaa gttttcctgg ctgggccgga 1140

acactgattt cattcagatg accagtggtg agacgctgga tccccgacca attgagagct 1200

cgctctgcga aagttctctt atttctagag catgcgttat cggagataaa tttctcaacg 1260

ggcctgctgc tgctgtttgt gcgatcattg agcttgagcc cacagcggtg gaaaaaggac 1320

aagctcactc gcgtgagata gcaagagttt tcgcacctat taatcgagac ctaccgcctc 1380

ctcttaggat tgcatggtcg cacgttttgg ttctccagcc ctcggagaag ataccgatga 1440

cgaagaaggg taccatcttc cgcaagaaaa ttgagcaggt gtttggctct gcgttgggtg 1500

gcagctctgg agataactct caagccactg cggatgctgg cgttgttcga cgagacgagt 1560

tatcgaacac tgtcaagcac ataattagcc gtgttttagg agtttccgat gacgaattac 1620

tttggacgct atcatttgcg gagttaggaa tgacgtcagc actagccact cgcatcgcca 1680

acgagttgaa cgaagtttta gttggagtta atctccctat caacgcttgc tatatacatg 1740

tcgaccttcc ttctctaagc aatgccgtct atgcgaaact tgcacacctc aagttaccag 1800

atcgtactcc cgaacccagg caagcccctg tcgaaaactc tggtgggaag gagatcgttg 1860

tcgttggcca ggcctttcgt cttcctggct caataaacga tgtcgcctct cttcgagacg 1920

cattcctggc gagacaagca tcatccatta tcactgaaat accatccgat cgctgggacc 1980

acgccagctt ctatcccaag gatatacgtt tcaacaaggc tggccttgtg gatatagcca 2040

attatgatca tagctttttc ggactgacgg caaccgaagc gctctatctg tcgccaacta 2100

tgcgtctagc attagaagtt tcgtttgaag cgctagagaa tgctaatatc ccggtgtcac 2160

aactcaaggg ttcgcaaaca gcggtttatg ttgctactac agatgacgga tttgagaccc 2220

ttttgaatgc cgaggccggc tatgatgctt atacaagatt ctatggcact ggtcgagcag 2280

caagtacagc gagcgggcgc ataagctgtc ttcttgatgt ccatggaccc tctattactg 2340

ttgatacggc atgcagtgga ggggctgttt gtattgacca agcaatcgac tatctacaat 2400

catcgagtgc agcagacacc gctatcatat gtgctagtaa cacgcactgc tggccaggct 2460

cgttcaggtt tctttccgca caaggatggt tatccccagg aggacgatgc gcgacattta 2520

caactgatgc tgatggctac gtgccctctg agggcgcggt cgccttcata ttgaaaaccc 2580

gagaagcagc tatgcgtgac aaggacacta tcctcgcgac aatcaaagcg acacagatat 2640

cgcacaatgg ccgatctcaa ggtcttgtgg caccgaatgt caactcgcaa gctgaccttc 2700

atcgctcgtt gcttcaaaaa gctggcctta gcccggctga tatccgtttc attgaagctc 2760

atgggacagg aacgtcactg ggagacctct cagaaattca agctataaat gatgcttata 2820

cctcctctca gccgcgcacg accggcccac tcatagtcag cgcttccaaa acggtcattg 2880

gtcataccga accagctggc cccttggtcg gtatgctgtc ggtcttgaac tctttcaaag 2940

aaggcgccgt ccctggtctc gcccatctta ccgcagacaa tttgaatccc tcgctggact 3000

gttcttctgt gccacttctc attccctatc aacctgttca cctggctgca cccaagcctc 3060

accgagctgc tgtaaggtca tacggctttt caggtaccct gggcggcatc gttctagagg 3120

ctcctgacga agaaagatta gaagaagagc tgccaaatga caagcccatg ttgttcgtcg 3180

tcagcgcaaa gacacataca gcactaatcg aatacctggg gcggtatctc gagttcctct 3240

tgcaggcgaa cccccaagat ttttgtgaca tttgttatac aagctgcgtt gggcgggagc 3300

actatagata tcgctatgct tgtgtagcaa atgatatgga ggacctcata ggccaactcc 3360

agaaacgttt gggcagcaag gtgccgccaa agccgtcata caaacgcggt gctttggcct 3420

ttgccttttc tggtcagggt acacaattcc gagggatggc gacagagctt gcaaaagcgt 3480

actccggctt ccgaaagatc gtgtcggatc tcgcaaagag agctagcgag ttgtcaggtc 3540

atgccattga ccgttttctt cttgcatatg acataggcgc tgaaaatgta gctcctgata 3600

gtgaggcaga ccagatttgc atctttgtgt atcagtgttc tgtccttcgc tggctgcaga 3660

ctatggggat tagacccagt gcagtgatag gccatagcct cggggagatc tcagcttctg 3720

tggcggcagg agcactttct cttgactccg ctttggatct tgtcatctca cgagctcgcc 3780

ttttgcgctc ttcggcaagt gctcctgcag gaatggcagc tatgtctgcc tcgcaagacg 3840

aggttgtgga gttgattggg aaactagacc tcgacaaggc taattcgctc agcgtttcgg 3900

tcataaatgg tccccaaaat actgtcgtgt ccggctcttc agcggctatt gaaagcatag 3960

tggctttagc gaaaggagaa aagatcaaag cgtctgccct gaatatcaat caagcttttc 4020

atagtccata cgtcgacagt gccgtccctg gtctccgtgc ttggtcagaa aagcatatct 4080

cctcagctcg gccattgcaa attccgctgt attcaacgtt gttgggagca caaatctctg 4140

agggagagat gttgaatcca gatcactggg tcgaccatgc acggaagcct gtacagttcg 4200

cacaagcagc cacaaccatg aaagaatcct tcaccggagt catcatagat atcggccctc 4260

aagtagtggc ttggtcactt ctgctctcga acgggctcac gtccgtgact gcgctcgctg 4320

cgaaaagagg gagaagtcaa caggtggctt tcttaagcgc cttggcggat ttgtatcaag 4380

attacggtgt tgttcctgat tttgtcgggc tttatgctca gcaggaagat gcttcgaggt 4440

tgaagaagac ggatatcttg acgtatccgt tccagcgggg cgaagagact ctttctagtg 4500

gttctagcac tccgacattg gaaaacacgg atttggattc cggtaaggaa ttacttatgg 4560

gaccgactcg ggggttgtta cgcgcggacg acttgcgtga cagtatcgtt tcttctgtga 4620

aggatgttct ggaactcaag tcaaatgaag acctcgattt gtctgaaagt ctgaatgcgc 4680

ttggtatgga ctcgatcatg ttcgctcagt tacggaagcg tattggggaa ggactcggat 4740

tgaatgttcc gatggttttt ctgtcggacg cgttttctat tggtgagatg gttagtaatc 4800

ttgtggaaca ggcggaggcg tctgaggaca at 4832

<210> 35
<211> 1678
<212> PRT
<213> Neonothopanus nambi

<400> 35

Met Asn Ser Ser Lys Asn Pro Pro Ser Thr Leu Leu Asp Val Phe Leu
1               5                   10                  15

Asp Thr Ala Arg Asn Leu Asp Thr Ala Leu Arg Asn Val Leu Glu Cys
            20                  25                  30

Gly Glu His Arg Trp Ser Tyr Arg Glu Leu Asp Thr Val Ser Ser Ala
        35                  40                  45

Leu Ala Gln His Leu Arg Tyr Thr Val Gly Leu Ser Pro Thr Val Ala
    50                  55                  60

Val Ile Ser Glu Asn His Pro Tyr Ile Leu Ala Leu Met Leu Ala Val
65                  70                  75                  80

Trp Lys Leu Gly Gly Thr Phe Ala Pro Ile Asp Val His Ser Pro Ala
                85                  90                  95

Glu Leu Val Ala Gly Met Leu Asn Ile Val Ser Pro Ser Cys Leu Val
        100                 105                 110

Ile Pro Ser Ser Asp Val Thr Asn Gln Thr Leu Ala Cys Asp Leu Asn
    115                 120                 125

Ile Pro Val Val Ala Phe His Pro His Gln Ser Thr Ile Pro Glu Leu
    130                 135                 140

Asn Lys Lys Tyr Leu Thr Asp Ser Gln Ile Ser Pro Asp Leu Pro Phe
145                 150                 155                 160

Ser Asp Pro Asn Arg Pro Ala Leu Tyr Leu Phe Thr Ser Ser Ala Thr
                165                 170                 175

Ser Arg Ser Asn Leu Lys Cys Val Pro Leu Thr His Thr Phe Ile Leu
        180                 185                 190

Arg Asn Ser Leu Ser Lys Arg Ala Trp Cys Lys Arg Met Arg Pro Glu
    195                 200                 205

Thr Asp Phe Asp Gly Ile Arg Val Leu Gly Trp Ala Pro Trp Ser His
    210                 215                 220

Val Leu Ala His Met Gln Asp Ile Gly Pro Leu Thr Leu Leu Asn Ala
225                 230                 235                 240

Gly Cys Tyr Val Phe Ala Thr Thr Pro Ser Thr Tyr Pro Thr Glu Leu
                245                 250                 255

Lys Asp Asp Arg Asp Leu Ile Ser Cys Ala Ala Asn Ala Ile Met Tyr
        260                 265                 270

Lys Gly Val Lys Ser Phe Ala Cys Leu Pro Phe Val Leu Gly Gly Leu
    275                 280                 285

Lys Ala Leu Cys Glu Ser Glu Pro Ser Val Lys Ala His Leu Gln Val
    290                 295                 300

Glu Glu Arg Ala Gln Leu Leu Lys Ser Leu Gln His Met Glu Ile Leu
305                 310                 315                 320

Glu Cys Gly Gly Ala Met Leu Glu Ala Ser Val Ala Ser Trp Ala Ile
                325                 330                 335

Glu Asn Cys Ile Pro Ile Ser Ile Gly Ile Gly Met Thr Glu Thr Gly
            340                 345                 350

Gly Ala Leu Phe Ala Gly Pro Val Gln Ala Ile Lys Thr Gly Phe Ser
        355                 360                 365

Ser Glu Asp Lys Phe Ile Glu Asp Ala Thr Tyr Leu Leu Val Lys Asp
    370                 375                 380

Asp His Glu Ser His Ala Glu Glu Asp Ile Asn Glu Gly Glu Leu Val
385                 390                 395                 400

Val Lys Ser Lys Met Leu Pro Arg Gly Tyr Leu Gly Tyr Ser Asp Pro
                405                 410                 415

Ser Phe Ser Val Asp Asp Ala Gly Trp Val Thr Phe Arg Thr Gly Asp
            420                 425                 430

Arg Tyr Ser Val Thr Pro Asp Gly Lys Phe Ser Trp Leu Gly Arg Asn
        435                 440                 445

Thr Asp Phe Ile Gln Met Thr Ser Gly Glu Thr Leu Asp Pro Arg Pro
    450                 455                 460

Ile Glu Ser Ser Leu Cys Glu Ser Ser Leu Ile Ser Arg Ala Cys Val
465                 470                 475                 480

Ile Gly Asp Lys Phe Leu Asn Gly Pro Ala Ala Ala Val Cys Ala Ile
                485                 490                 495

Ile Glu Leu Glu Pro Thr Ala Val Glu Lys Gly Gln Ala His Ser Arg
            500                 505                 510

Glu Ile Ala Arg Val Phe Ala Pro Ile Asn Arg Asp Leu Pro Pro Pro
        515                 520                 525

Leu Arg Ile Ala Trp Ser His Val Leu Val Leu Gln Pro Ser Glu Lys
    530                 535                 540

Ile Pro Met Thr Lys Lys Gly Thr Ile Phe Arg Lys Lys Ile Glu Gln
545                 550                 555                 560

Val Phe Gly Ser Ala Leu Gly Gly Ser Ser Gly Asp Asn Ser Gln Ala
                565                 570                 575

Thr Ala Asp Ala Gly Val Val Arg Arg Asp Glu Leu Ser Asn Thr Val
            580                 585                 590

Lys His Ile Ile Ser Arg Val Leu Gly Val Ser Asp Asp Glu Leu Leu
        595                 600                 605

Trp Thr Leu Ser Phe Ala Glu Leu Gly Met Thr Ser Ala Leu Ala Thr
    610                 615                 620

Arg Ile Ala Asn Glu Leu Asn Glu Val Leu Val Gly Val Asn Leu Pro
625                 630                 635                 640

Ile Asn Ala Cys Tyr Ile His Val Asp Leu Pro Ser Leu Ser Asn Ala
                645                 650                 655

Val Tyr Ala Lys Leu Ala His Leu Lys Leu Pro Asp Arg Thr Pro Glu
            660                 665                 670

Pro Arg Gln Ala Pro Val Glu Asn Ser Gly Gly Lys Glu Ile Val Val
        675                 680                 685

Val Gly Gln Ala Phe Arg Leu Pro Gly Ser Ile Asn Asp Val Ala Ser
    690                 695                 700

Leu Arg Asp Ala Phe Leu Ala Arg Gln Ala Ser Ser Ile Ile Thr Glu
705                 710                 715                 720

Ile Pro Ser Asp Arg Trp Asp His Ala Ser Phe Tyr Pro Lys Asp Ile
                725                 730                 735

Arg Phe Asn Lys Ala Gly Leu Val Asp Ile Ala Asn Tyr Asp His Ser
            740                 745                 750

Phe Phe Gly Leu Thr Ala Thr Glu Ala Leu Tyr Leu Ser Pro Thr Met
        755                 760                 765

Arg Leu Ala Leu Glu Val Ser Phe Glu Ala Leu Glu Asn Ala Asn Ile
    770                 775                 780

Pro Val Ser Gln Leu Lys Gly Ser Gln Thr Ala Val Tyr Val Ala Thr
785                 790                 795                 800

Thr Asp Asp Gly Phe Glu Thr Leu Leu Asn Ala Glu Ala Gly Tyr Asp
                805                 810                 815

Ala Tyr Thr Arg Phe Tyr Gly Thr Gly Arg Ala Ala Ser Thr Ala Ser
            820                 825                 830

Gly Arg Ile Ser Cys Leu Leu Asp Val His Gly Pro Ser Ile Thr Val
        835                 840                 845

Asp Thr Ala Cys Ser Gly Gly Ala Val Cys Ile Asp Gln Ala Ile Asp
    850                 855                 860

Tyr Leu Gln Ser Ser Ser Ala Ala Asp Thr Ala Ile Ile Cys Ala Ser
865                 870                 875                 880

Asn Thr His Cys Trp Pro Gly Ser Phe Arg Phe Leu Ser Ala Gln Gly
                885                 890                 895

Met Val Ser Pro Gly Gly Arg Cys Ala Thr Phe Thr Thr Asp Ala Asp
            900                 905                 910

Gly Tyr Val Pro Ser Glu Gly Ala Val Ala Phe Ile Leu Lys Thr Arg
        915                 920                 925

Glu Ala Ala Met Arg Asp Lys Asp Thr Ile Leu Ala Thr Ile Lys Ala
    930                 935                 940

Thr Gln Ile Ser His Asn Gly Arg Ser Gln Gly Leu Val Ala Pro Asn
945                 950                 955                 960

Val Asn Ser Gln Ala Asp Leu His Arg Ser Leu Leu Gln Lys Ala Gly
                965                 970                 975

Leu Ser Pro Ala Asp Ile Arg Phe Ile Glu Ala His Gly Thr Gly Thr
            980                 985                 990

Ser Leu Gly Asp Leu Ser Glu Ile  Gln Ala Ile Asn Asp  Ala Tyr Thr
        995                 1000                1005

Ser Ser  Gln Pro Arg Thr Thr  Gly Pro Leu Ile Val  Ser Ala Ser
    1010                 1015                 1020

Lys Thr  Val Ile Gly His Thr  Glu Pro Ala Gly Pro  Leu Val Gly
    1025                 1030                 1035

Met Leu  Ser Val Leu Asn Ser  Phe Lys Glu Gly Ala  Val Pro Gly
    1040                 1045                 1050

Leu Ala  His Leu Thr Ala Asp  Asn Leu Asn Pro Ser  Leu Asp Cys
    1055                 1060                 1065

Ser Ser  Val Pro Leu Leu Ile  Pro Tyr Gln Pro Val  His Leu Ala
    1070                 1075                 1080

Ala Pro  Lys Pro His Arg Ala  Ala Val Arg Ser Tyr  Gly Phe Ser
    1085                 1090                 1095

Gly Thr  Leu Gly Gly Ile Val  Leu Glu Ala Pro Asp  Glu Glu Arg
    1100                 1105                 1110

Leu Glu  Glu Glu Leu Pro Asn  Asp Lys Pro Met Leu  Phe Val Val
    1115                 1120                 1125

Ser Ala  Lys Thr His Thr Ala  Leu Ile Glu Tyr Leu  Gly Arg Tyr
    1130                 1135                 1140

Leu Glu  Phe Leu Leu Gln Ala  Asn Pro Gln Asp Phe  Cys Asp Ile
    1145                 1150                 1155

Cys Tyr  Thr Ser Cys Val Gly  Arg Glu His Tyr Arg  Tyr Arg Tyr
    1160                 1165                 1170

Ala Cys Val Ala Asn Asp Met Glu Asp Leu Ile Gly Gln Leu Gln
    1175                1180                1185

Lys Arg Leu Gly Ser Lys Val Pro Pro Lys Pro Ser Tyr Lys Arg
    1190                1195                1200

Gly Ala Leu Ala Phe Ala Phe Ser Gly Gln Gly Thr Gln Phe Arg
    1205                1210                1215

Gly Met Ala Thr Glu Leu Ala Lys Ala Tyr Ser Gly Phe Arg Lys
    1220                1225                1230

Ile Val Ser Asp Leu Ala Lys Arg Ala Ser Glu Leu Ser Gly His
    1235                1240                1245

Ala Ile Asp Arg Phe Leu Leu Ala Tyr Asp Ile Gly Ala Glu Asn
    1250                1255                1260

Val Ala Pro Asp Ser Glu Ala Asp Gln Ile Cys Ile Phe Val Tyr
    1265                1270                1275

Gln Cys Ser Val Leu Arg Trp Leu Gln Thr Met Gly Ile Arg Pro
    1280                1285                1290

Ser Ala Val Ile Gly His Ser Leu Gly Glu Ile Ser Ala Ser Val
    1295                1300                1305

Ala Ala Gly Ala Leu Ser Leu Asp Ser Ala Leu Asp Leu Val Ile
    1310                1315                1320

Ser Arg Ala Arg Leu Leu Arg Ser Ser Ala Ser Ala Pro Ala Gly
    1325                1330                1335

Met Ala Ala Met Ser Ala Ser Gln Asp Glu Val Val Glu Leu Ile
    1340                1345                1350

Gly Lys Leu Asp Leu Asp Lys Ala Asn Ser Leu Ser Val Ser Val
    1355                1360                1365

Ile Asn Gly Pro Gln Asn Thr Val Val Ser Gly Ser Ser Ala Ala
    1370                1375                1380

Ile Glu Ser Ile Val Ala Leu Ala Lys Gly Arg Lys Ile Lys Ala
    1385                1390                1395

Ser Ala Leu Asn Ile Asn Gln Ala Phe His Ser Pro Tyr Val Asp
    1400                1405                1410

Ser Ala Val Pro Gly Leu Arg Ala Trp Ser Glu Lys His Ile Ser
    1415                1420                1425

Ser Ala Arg Pro Leu Gln Ile Pro Leu Tyr Ser Thr Leu Leu Gly
    1430                1435                1440

Ala Gln Ile Ser Glu Gly Glu Met Leu Asn Pro Asp His Trp Val
    1445                1450                1455

Asp His Ala Arg Lys Pro Val Gln Phe Ala Gln Ala Ala Thr Thr
    1460                1465                1470

Met Lys Glu Ser Phe Thr Gly Val Ile Ile Asp Ile Gly Pro Gln
    1475                1480                1485

Val Val Ala Trp Ser Leu Leu Leu Ser Asn Gly Leu Thr Ser Val
    1490                1495                1500

Thr Ala Leu Ala Ala Lys Arg Gly Arg Ser Gln Gln Val Ala Phe
    1505                1510                1515

Leu Ser Ala Leu Ala Asp Leu Tyr Gln Asp Tyr Gly Val Val Pro
    1520                1525                1530

Asp Phe Val Gly Leu Tyr Ala Gln Gln Glu Asp Ala Ser Arg Leu
    1535                1540                1545

Lys Lys Thr Asp Ile Leu Thr Tyr Pro Phe Gln Arg Gly Glu Glu
    1550                1555                1560

Thr Leu Ser Ser Gly Ser Ser Thr Pro Thr Leu Glu Asn Thr Asp
    1565                1570                1575

Leu Asp Ser Gly Lys Glu Leu Leu Met Gly Pro Thr Arg Gly Leu
    1580                1585                1590

Leu Arg Ala Asp Asp Leu Arg Asp Ser Ile Val Ser Ser Val Lys
    1595                1600                1605

Asp Val Leu Glu Leu Lys Ser Asn Glu Asp Leu Asp Leu Ser Glu
    1610                1615                1620

Ser Leu Asn Ala Leu Gly Met Asp Ser Ile Met Phe Ala Gln Leu
    1625                1630                1635

Arg Lys Arg Ile Gly Glu Gly Leu Gly Leu Asn Val Pro Met Val
    1640                1645                1650

Phe Leu Ser Asp Ala Phe Ser Ile Gly Glu Met Val Ser Asn Leu
    1655                1660                1665

Val Glu Gln Ala Glu Ala Ser Glu Asp Asn
    1670                1675

<210> 36
<211> 4512
<212> DNA
<213> Panellus stipticus

<400> 36
   atgcctcctg ctccttcctc catccttgat gtgttcaccg agactgcata caatcctcgc 60
   acctccaacc gtcctgtagt agaatgtggc gagcacctct ggacatactc acaacttgaa 120
   gcagtttcca atgccatgtc gcaagacctg gagaactcga tcggtcttta cgcgaaggtc 180
   gcttttgtcg gtgaaaacca tccatttgtt ttcgctctca tgcttgcggt ctggaaaatt 240
   gctggcacat tcatccccat tgatgcgcac attcctcccg ccctcctaga tggcatggtc 300
   gacattgtga agccgatgcg catctatgta tcatcggccg atacctctaa ttcctcctgg 360
   gcctcagaac tcgcggtcga gtccaaactc gcgtggtggc gacgcatgca gccaagcatc 420
   aacctagaca acacccgagt cctcggctgg gccccgtggt cgcatgtatt atcccacatg 480
   caggacattg gcaccgctac cattctcacc gcaggctgtt acgtctttgc ctctatccct 540
   tccacatatc agcttcagca ggcgccaaca gatcttacga cccaagtcat caacggtatc 600
   ctcaataaaa acatctcggc gcttgctgcc cttccatttg ttttcggcgg tatcaaagca 660
   gcgtgcgagt cgggcgatct ggatgtggag gcacttctgg gtgccctgcg ccgcatgacg 720
   atgctcgaat gtggcggggc tgcgctcgac cctgcaattg caaattggac ggatataaat 780
   ggtgtatcgc tcatggttgg gattggaatg acggaaacgg gtggtgcaat cttcgcgggg 840
   agggcgaaag actcgctctc cgggtttctt gctgagggcc tgatctcaga tgccagaatt 900
   gagcttgaca agggtaaatc tgatggctcg gatgagggag agcttgttgt cacaagcaag 960
   ctacttccac atggctacat tggcttcgat gacgggtcat tttccgtgga tgcgcaaggg 1020
   tgggtgacgt tcaagacaag ggattgctat cgtgtcaagg actcgaagtt tatttggcta 1080
   ggccgggcca ctgattacat tcaggcgagc acatctctcc ttcctcagcg ctttaaacat 1140
   gaactgcgct atcagatgac aagcggcgaa tcccttgacc cgcgtcccct cgaaaatttc 1200
   ttacgctccg ccgagttcat ctccaatgtg tgcgtcatag gtgacaactt cctccggggt 1260
   gcctccactt ctgtgtgggc cgttatcgag ctcacggaca gcgcgcaccg tctggatgcc 1320
   gcttcagcga gaaagcaggt ggcgcgcgtg ctggcgccgc tcaatggtgg cctcccaccc 1380
   gctctccgga tctcgatgtc ctcggtgttg atattgagcg gatcacagaa aattccgcgc 1440
   acgaagaagg gcgaaatctt ccgtaagcag attgaggatc tgtttggtgc cgcgatgagc 1500
   atgccgcctg aggctgagcc ggctctggag gatgaactaa caagtattgt tggaaatgtc 1560
   ctcaacatat cggacagcga catgttgacc tcgatgactt ttgccgaact tggaatgacc 1620
   tctgttctcg cagtaaaaat ctcagcaagg ctcaacgagc accttgctgg gcgggccgtc 1680
   ctcccggcca acatctgtta catctacatc gatactccat cactgattgc cggcatccgg 1740
   aacttccttt cgccggcatc ttctgatcaa gagccatcga actttgccac cacggcagac 1800
   aagaaggacg aaatcgtcat catagccaaa gccttccggc ttcccggcgg gatcaacgac 1860
   gactctgctc tctgggctgc tcttatgggc aagaacgact ctgttattgc agacatcccg 1920
   ccagaccgtt gggatcatgc tagcttctat cccgcccaca tctgcttgcg caaggccggg 1980
   cttatagata tgtccagcta tgactatggg ttctttggcc tttcggcgac tgaggcgtac 2040
   tatgtgtcgc ccaccatgcg cgcagcgctc gaggtggcgt tcgaagcgct ggaggatgcg 2100
   aacatcccgg tgtcaagaat caaggggaca aatacgagtg tttttgttgc aacgaaagat 2160
   gatggatttg agacattgct gaatgcggcg catggatttg atgatgctga cggatacgtc 2220
   ccttcagaag gcgccgtcgc ttttatactg aagacacgga cggcggcgga gagggatggg 2280
   gatcgaatta tggccatcat caaagccacg gaagtctcgc ataatggaag atctcaggga 2340
   ctcgccgcgc cgaacgtcaa ggcgcaagcc gctcttcata gagcagtctt gcgcaaagct 2400
   aagctcgacc ctctggacat ccatttcatt gaagcccacg gaactggaac accgctgggc 2460
   gatctttgtg aaatacaagg cataaatgaa gcctttgtct ccgcacgtcc acgtgcggag 2520
   gatccactca ttgtcagcgc ctcaaagtct tcccttgggc acaccgaacc ttcggctgga 2580
   ttggttggga tgctatctgt gttgatggcc ttgaagcacg gcatcgtgcc tgggttgctc 2640
   catctccgag cggacaatgc taaccaccaa ctggacctga cacaagttcc acttcgtata 2700
   tcaccggaac ctgtagtcat cgccgcatcc aagccgcatc acgccatggt gctatcctat 2760
   ggattttcag gtacattggc agacattgtt ttggagagtc cggaagaacc atcgtcccca 2820
   aacccgggag ccgcgggccc aatgatattt gtcctcagcg cgaagacttc cgcggctctg 2880
   tcggcatata tcaaggctta cattgcattt ctacagaatg cagacgcgca cgagttttac 2940
   aacatctgct acaccgcctg cgttggaaga gaacattaca aacacagatt tgcttgcgtt 3000
   gcaaatgacc ttgctgatct gattcgccaa ttacaagact gtgcgagtgc gctggctcca 3060
   accaaaagta gtactggtgc cttggcgttc gcgttccctg gccaaggcgt gcaatttcca 3120
   ggcatggccg ctgcacttgc taaaaggcat tccatatttc gcgactatgt catggaattc 3180
   ggtgatagag cacaagatct ttgtggcttg cccatcgcaa agatgttgct ggacgtggat 3240
   gcagcagagg aagaagatat ccacagtgac gtagaccaga tttgcgtctt tgtctatcag 3300
   tattcgatgt gtcgatggct tagggagctc gggctcgagg caagtgcggt tatcgggcac 3360
   agtttgggtg aaataacggc cgcacttatc ggggacgcat ttacatttga agtggctctc 3420
   gatctcgttg tcactcgagg acggctactc cgcccttctc aagggaatcc aggcgggatg 3480
   gctgcgctgg catgccccga ggagaatgtc ccggcgattt tggaccaatg tcgtgtggac 3540
   agtaccatta gcgtctccgt tatcaatggt ccgaggagcc tttgtgtatc cggagcttcc 3600
   aacgacatcg acgagtttgt caagatggca aaacggcaaa acatcaaagc gactcgactg 3660
   agggtggacc aaggatttca tagccctcga gttgatagtg ccgcggttgg actgcgtgca 3720
   tggtcaggtt ctttttccaa atcatttcag ccgttgcgca tcacattata ctctacttct 3780
   ctgggtgctg caatctcgaa aggagagatt ttgaatcaga cgcattgggc cgatcacgtc 3840
   cgccgtccgg tcatattctc aaaagcagca gcagccatcc tcgaggacaa gtccattggc 3900
   gcgatcctgg atatcggacc acagacggtg gcatggtctc tccttctggc gaacggctgc 3960
   aacgtcgcgt cagctgttgc cctgtccggc cgaagagtac aagatcagga aacagccttt 4020
   ttatctgcac tggcgaatct gtatcaaaat cacggggtga cgccgaattt tcgcgtattt 4080
   tatgctcacc aggcagtcca ggcgcgctat agaaccgtgg acatcccgaa gtatcccttc 4140
   caacgccgac atcgatatcc atcctacatt ccatcgcgca atgccacggg tgccaacaga 4200
   ctgaaagaac cattccgtag cgacctggat gaaccggctc aagacacgga gcacaccgcg 4260
   gaactgagag tggacatgac tccggagaag ctccgggacg ccctgatgca ctgtgtgcgg 4320
   gacacattgg aaggcgaaga ttttgatgaa tcggaatccc tcgtttcgcg tggaattgac 4380
   tccattactt ttgcgggttt acggaagcgt gttcaagaac gacacggact taatctttcc 4440
   atcattttct ggtctgatgg gttttctgtg aaagacatgg tcgacagcct catcgaacag 4500
   cattttgtcc ac 4512

<210> 37
<211> 1504
<212> PRT
<213> Panellus stipticus

<400> 37

Met Pro Pro Ala Pro Ser Ser Ile Leu Asp Val Phe Thr Glu Thr Ala
1               5                   10                  15

Tyr Asn Pro Arg Thr Ser Asn Arg Pro Val Val Glu Cys Gly Glu His
            20                  25                  30

Leu Trp Thr Tyr Ser Gln Leu Glu Ala Val Ser Asn Ala Met Ser Gln
        35                  40                  45

Asp Leu Glu Asn Ser Ile Gly Leu Tyr Ala Lys Val Ala Phe Val Gly
    50                  55                  60

Glu Asn His Pro Phe Val Phe Ala Leu Met Leu Ala Val Trp Lys Ile
65                  70                  75                  80

Ala Gly Thr Phe Ile Pro Ile Asp Ala His Ile Pro Pro Ala Leu Leu
                85                  90                  95

Asp Gly Met Val Asp Ile Val Lys Pro Met Arg Ile Tyr Val Ser Ser
            100                 105                 110

Ala Asp Thr Ser Asn Ser Ser Trp Ala Ser Glu Leu Ala Val Glu Ser
        115                 120                 125

Lys Leu Ala Trp Trp Arg Arg Met Gln Pro Ser Ile Asn Leu Asp Asn
    130                 135                 140

Thr Arg Val Leu Gly Trp Ala Pro Trp Ser His Val Leu Ser His Met
145                 150                 155                 160

Gln Asp Ile Gly Thr Ala Thr Ile Leu Thr Ala Gly Cys Tyr Val Phe
                165                 170                 175

Ala Ser Ile Pro Ser Thr Tyr Gln Leu Gln Gln Ala Pro Thr Asp Leu
            180                 185                 190

Thr Thr Gln Val Ile Asn Gly Ile Leu Asn Lys Asn Ile Ser Ala Leu
        195                 200                 205

Ala Ala Leu Pro Phe Val Phe Gly Gly Ile Lys Ala Ala Cys Glu Ser
    210                 215                 220

Gly Asp Leu Asp Val Glu Ala Leu Leu Gly Ala Leu Arg Arg Met Thr
225                 230                 235                 240

Met Leu Glu Cys Gly Gly Ala Ala Leu Asp Pro Ala Ile Ala Asn Trp
                245                 250                 255

Thr Asp Ile Asn Gly Val Ser Leu Met Val Gly Ile Gly Met Thr Glu
            260                 265                 270

Thr Gly Gly Ala Ile Phe Ala Gly Arg Ala Lys Asp Ser Leu Ser Gly
        275                 280                 285

Phe Leu Ala Glu Gly Leu Ile Ser Asp Ala Arg Ile Glu Leu Asp Lys
    290                 295                 300

Gly Lys Ser Asp Gly Ser Asp Glu Gly Glu Leu Val Val Thr Ser Lys
305                 310                 315                 320

Leu Leu Pro His Gly Tyr Ile Gly Phe Asp Asp Gly Ser Phe Ser Val
                325                 330                 335

Asp Ala Gln Gly Trp Val Thr Phe Lys Thr Arg Asp Cys Tyr Arg Val
            340                 345                 350

Lys Asp Ser Lys Phe Ile Trp Leu Gly Arg Ala Thr Asp Tyr Ile Gln
        355                 360                 365

Ala Ser Thr Ser Leu Leu Pro Gln Arg Phe Lys His Glu Leu Arg Tyr
    370                 375                 380

Gln Met Thr Ser Gly Glu Ser Leu Asp Pro Arg Pro Leu Glu Asn Phe
385                 390                 395                 400

Leu Arg Ser Ala Glu Phe Ile Ser Asn Val Cys Val Ile Gly Asp Asn
                405                 410                 415

Phe Leu Arg Gly Ala Ser Thr Ser Val Trp Ala Val Ile Glu Leu Thr
            420                 425                 430

Asp Ser Ala His Arg Leu Asp Ala Ala Ser Ala Arg Lys Gln Val Ala
        435                 440                 445

Arg Val Leu Ala Pro Leu Asn Gly Gly Leu Pro Pro Ala Leu Arg Ile
    450                 455                 460

Ser Met Ser Ser Val Leu Ile Leu Ser Gly Ser Gln Lys Ile Pro Arg
465                 470                 475                 480

Thr Lys Lys Gly Glu Ile Phe Arg Lys Gln Ile Glu Asp Leu Phe Gly
                485                 490                 495

Ala Ala Met Ser Met Pro Pro Glu Ala Glu Pro Ala Leu Glu Asp Glu
            500                 505                 510

Leu Thr Ser Ile Val Gly Asn Val Leu Asn Ile Ser Asp Ser Asp Met
        515                 520                 525
Leu Thr Ser Met Thr Phe Ala Glu Leu Gly Met Thr Ser Val Leu Ala
    530                 535                 540
Val Lys Ile Ser Ala Arg Leu Asn Glu His Leu Ala Gly Arg Ala Val
545                 550                 555                 560
Leu Pro Ala Asn Ile Cys Tyr Ile Tyr Ile Asp Thr Pro Ser Leu Ile
                565                 570                 575
Ala Gly Ile Arg Asn Phe Leu Ser Pro Ala Ser Ser Asp Gln Glu Pro
            580                 585                 590
Ser Asn Phe Ala Thr Thr Ala Asp Lys Lys Asp Glu Ile Val Ile Ile
        595                 600                 605
Gly Lys Ala Phe Arg Leu Pro Gly Gly Ile Asn Asp Asp Ser Ala Leu
    610                 615                 620
Trp Ala Ala Leu Met Gly Lys Asn Asp Ser Val Ile Ala Asp Ile Pro
625                 630                 635                 640
Pro Asp Arg Trp Asp His Ala Ser Phe Tyr Pro Ala His Ile Cys Leu
                645                 650                 655
Arg Lys Ala Gly Leu Ile Asp Met Ser Ser Tyr Asp Tyr Gly Phe Phe
            660                 665                 670
Gly Leu Ser Ala Thr Glu Ala Tyr Tyr Val Ser Pro Thr Met Arg Ala
        675                 680                 685
Ala Leu Glu Val Ala Phe Glu Ala Leu Glu Asp Ala Asn Ile Pro Val
    690                 695                 700
Ser Arg Ile Lys Gly Thr Asn Thr Ser Val Phe Val Ala Thr Lys Asp
705                 710                 715                 720
Asp Gly Phe Glu Thr Leu Leu Asn Ala Ala His Gly Phe Asp Asp Ala
                725                 730                 735

Asp Gly Tyr Val Pro Ser Glu Gly Ala Val Ala Phe Ile Leu Lys Thr
            740                 745                 750

Arg Thr Ala Ala Glu Arg Asp Gly Asp Arg Ile Met Ala Ile Ile Lys
        755                 760                 765

Ala Thr Glu Val Ser His Asn Gly Arg Ser Gln Gly Leu Ala Ala Pro
    770                 775                 780

Asn Val Lys Ala Gln Ala Ala Leu His Arg Ala Val Leu Arg Lys Ala
785                 790                 795                 800

Lys Leu Asp Pro Leu Asp Ile His Phe Ile Glu Ala His Gly Thr Gly
                805                 810                 815

Thr Pro Leu Gly Asp Leu Cys Glu Ile Gln Gly Ile Asn Glu Ala Phe
            820                 825                 830

Val Ser Ala Arg Pro Arg Ala Glu Asp Pro Leu Ile Val Ser Ala Ser
        835                 840                 845

Lys Ser Ser Leu Gly His Thr Glu Pro Ser Ala Gly Leu Val Gly Met
    850                 855                 860

Leu Ser Val Leu Met Ala Leu Lys His Gly Ile Val Pro Gly Leu Leu
865                 870                 875                 880

His Leu Arg Ala Asp Asn Ala Asn His Gln Leu Asp Leu Thr Gln Val
                885                 890                 895

Pro Leu Arg Ile Ser Pro Glu Pro Val Val Ile Ala Ala Ser Lys Pro
            900                 905                 910

His His Ala Met Val Leu Ser Tyr Gly Phe Ser Gly Thr Leu Ala Asp
        915                 920                 925

Ile Val Leu Glu Ser Pro Glu Glu Pro Ser Ser Pro Asn Pro Gly Ala
    930                 935                 940

Ala Gly Pro Met Ile Phe Val Leu Ser Ala Lys Thr Ser Ala Ala Leu
945                 950                 955                 960

Ser Ala Tyr Ile Lys Ala Tyr Ile Ala Phe Leu Gln Asn Ala Asp Ala
                965                 970                 975

His Glu Phe Tyr Asn Ile Cys Tyr Thr Ala Cys Val Gly Arg Glu His
            980                 985                 990

Tyr Lys His Arg Phe Ala Cys Val  Ala Asn Asp Leu Ala  Asp Leu Ile
        995                 1000                 1005

Arg Gln  Leu Gln Asp Cys Ala  Ser Ala Leu Ala Pro  Thr Lys Ser
    1010                 1015                 1020

Ser Thr  Gly Ala Leu Ala Phe  Ala Phe Pro Gly Gln  Gly Val Gln
    1025                 1030                 1035

Phe Pro  Gly Met Ala Ala Ala  Leu Ala Lys Arg His  Ser Ile Phe
    1040                 1045                 1050

Arg Asp  Tyr Val Met Glu Phe  Gly Asp Arg Ala Gln  Asp Leu Cys
    1055                 1060                 1065

Gly Leu  Pro Ile Ala Lys Met  Leu Leu Asp Val Asp  Ala Ala Glu
    1070                 1075                 1080

Glu Glu  Asp Ile His Ser Asp  Val Asp Gln Ile Cys  Val Phe Val
    1085                 1090                 1095

Tyr Gln  Tyr Ser Met Cys Arg  Trp Leu Arg Glu Leu  Gly Leu Glu
    1100                 1105                 1110

Ala Ser  Ala Val Ile Gly His  Ser Leu Gly Glu Ile  Thr Ala Ala
    1115                 1120                 1125

Leu Ile  Gly Asp Ala Phe Thr  Phe Glu Val Ala Leu  Asp Leu Val
    1130                 1135                 1140

Val Thr  Arg Gly Arg Leu Leu  Arg Pro Ser Gln Gly  Asn Pro Gly
    1145                 1150                 1155

Gly Met Ala Ala Leu Ala Cys Pro Glu Glu Asn Val Pro Ala Ile
    1160                1165                1170

Leu Asp Gln Cys Arg Val Asp Ser Thr Ile Ser Val Ser Val Ile
    1175                1180                1185

Asn Gly Pro Arg Ser Leu Cys Val Ser Gly Ala Ser Asn Asp Ile
    1190                1195                1200

Asp Glu Phe Val Lys Met Ala Lys Arg Gln Asn Ile Lys Ala Thr
    1205                1210                1215

Arg Leu Arg Val Asp Gln Gly Phe His Ser Pro Arg Val Asp Ser
    1220                1225                1230

Ala Ala Val Gly Leu Arg Ala Trp Ser Gly Ser Phe Ser Lys Ser
    1235                1240                1245

Phe Gln Pro Leu Arg Ile Thr Leu Tyr Ser Thr Ser Leu Gly Ala
    1250                1255                1260

Ala Ile Ser Lys Gly Glu Ile Leu Asn Gln Thr His Trp Ala Asp
    1265                1270                1275

His Val Arg Arg Pro Val Ile Phe Ser Lys Ala Ala Ala Ala Ile
    1280                1285                1290

Leu Glu Asp Lys Ser Ile Gly Ala Ile Leu Asp Ile Gly Pro Gln
    1295                1300                1305

Thr Val Ala Trp Ser Leu Leu Leu Ala Asn Gly Cys Asn Val Ala
    1310                1315                1320

Ser Ala Val Ala Leu Ser Gly Arg Arg Val Gln Asp Gln Glu Thr
    1325                1330                1335

Ala Phe Leu Ser Ala Leu Ala Asn Leu Tyr Gln Asn His Gly Val
    1340                1345                1350

Thr Pro Asn Phe Arg Val Phe Tyr Ala His Gln Ala Val Gln Ala
    1355                1360                1365

Arg Tyr Arg Thr Val Asp Ile Pro Lys Tyr Pro Phe Gln Arg Arg
    1370                1375                1380

His Arg Tyr Pro Ser Tyr Ile Pro Ser Arg Asn Ala Thr Gly Ala
    1385                1390                1395

Asn Arg Leu Lys Glu Pro Phe Arg Ser Asp Leu Asp Glu Pro Ala
    1400                1405                1410

Gln Asp Thr Glu His Thr Ala Glu Leu Arg Val Asp Met Thr Pro
    1415                1420                1425

Glu Lys Leu Arg Asp Ala Leu Met His Cys Val Arg Asp Thr Leu
    1430                1435                1440

Glu Gly Glu Asp Phe Asp Glu Ser Glu Ser Leu Val Ser Arg Gly
    1445                1450                1455

Ile Asp Ser Ile Thr Phe Ala Gly Leu Arg Lys Arg Val Gln Glu
    1460                1465                1470

Arg His Gly Leu Asn Leu Ser Ile Ile Phe Trp Ser Asp Gly Phe
    1475                1480                1485

Ser Val Lys Asp Met Val Asp Ser Leu Ile Glu Gln His Phe Val
    1490                1495                1500

His

<210> 38
<211> 4803
<212> DNA
<213> Panellus stipticus

<400> 38
atggcactgg tctctcccac ttgttcgtct cagatgcctc ctgctccttc ctccatcctt      60

gatgtgttca ccgagactgc atacaatcct cgcacctcca accgtcctgt agtagaatgt     120

ggcgagcacg tctggaccta ctcacaactt gaagcagttt ccaatgccat gtcgcaagac 180

ctggagaact cgatcggtct ttacgcgaag gtcgcttttg tcggtgaaaa ccatccattt 240

gttttcgctc tcatgcttgc agtctggaaa attgctggca cattcatccc cattgatgcg 300

cacattcctc ccgccctcct agatggcatg gtcgacattg tgaagccgat gtgcatctat 360

gtatcatcgg ccgatacctc taattcctcc tgggcctcag aactcgcggt cgaggttcgg 420

gtattccgtc cggaagaatc tacgattccg gctttgaacg aacactatgg aagatccagc 480

atcactcccg ctcagcatcg gccaatactc aacgtggtcc aggcagttcc cctgacacac 540

aaatttatcc tcagcaactg tcagtccaaa ctcgcgtggt ggcgacgcat gcagccaagc 600

atcaacctag acaacacccg agtcctcggc tgggccccgt ggtcgcatgt attatcccac 660

atgcaggaca ttggcaccgc taccattctc accgcaggct gttacgtctt tgcctctatc 720

ccttccacat atcagcttca gcaggcgcca acagatctta cgacccaagt catcaacggt 780

atcctcaata aaaacatctc ggcgcttgct gcccttccat ttgttttcgg cggtatcaaa 840

gcagcgtgcg agtcgggcga tctggatgtg gaggcacttc tgggtgccct gcgccgcatg 900

acgatgctcg aatgtggcgg ggctgcgctc gaccctgcaa ttgcaaattg gacggatata 960

aacggtgtat cgctcatggt tgggattgga atgacagaaa cgggcggtgc aatcttcgcg 1020

gggagggcga aagactcgct ctccgggttt cttgctgagg gtctgatctc agacgccaga 1080

attgagcttg acaagggtga atctgatggc tcggatggta cgacgttctc catcttctcg 1140

accactgccg gaattaaacc tgattgtcag agggagagct tgttgtcaca agcaagctac 1200

ttccacatgg ctacattggc ttcgatgacg ggtcattttc cgtggatgcg caagggtgac 1260

aacttcctcc ggggtgcctc cacttctgtg tgggccatta tcgagctcac ggacagcgcg 1320

caccgcctgg atgccgcttc agcgagaaag caggtggcgc gcgtactggc gccgctcaat 1380

ggtggcctcc cacccgctct ccggatctca atgtcctcgg tgttgatatt gagcggatca 1440

cagaaaattc cgcgcacgaa gaagggcgag atcttccgta agcagattga ggatctgttt 1500

ggtgccgcga tgagcatgcc gcctgaggct gagccggctc tggaggatga actaacaagt 1560

attgttggaa atgtcctcaa catatcggac agcgacatgt tgacctcgat gacttttgcc 1620

gaacttggaa tgacctctgt tctggcagta aaaatctcag caaggctcaa cgagcacctt 1680

gctgggcggg ccgtcctccc ggccaacatc tgttacatct acatcgatac tccatcactg 1740

attgccggca tccggaactt cctttcgccg acatcttctg atcaagagcc atcgaacttt 1800

gccaccacgg cagacaagaa ggacgagatc gtcatcatag gcaaagcctt tcggcttccc 1860

ggcgggatca acgacgactc tgctctctgg gctgctctta tgggcaagaa cgactctgtt 1920

attgcagaca tcccgccaga ccgttgggat catgctagct tctatcccgc ccacatctgc 1980

ttgcgcaagg ctgggcttat agatatgtcc agctatgact atgggttctt tggcctttcg 2040

gcgactgagg cgtactatgt gtcgcccacc atgcgcgcag cgctcgaggt ggcgttcgaa 2100

gcgctggagg atgcgaacat cccggtgtca agaattaagg ggacaaatac gagtgttttt 2160

gttgcaacga aagatgatgg atttgagaca ttgctgaatg cggcgcatgg atttgatgcg 2220

tatacccggt tctacgggac tgggcgggct ccaagtactg ccagtgggcg cataagctac 2280

cttcttgaca tccatggccc ctcactcaca gtagatacgg cctgtagcgg aggaattgtc 2340

tgcattgatc aagatatacc agcgaattta tgtatcatag cgatcgcata cctccagtct 2400

ggcgccggtg aatcagccat cgaacgacaa tacaggtttc tcacggcgca gaacatggca 2460

tcgcccacta gccgctgttc caccttcact gcagatgctg acggatatgt cccttcagaa 2520

ggcgccgtcg cttttatact gaagacacgg actgcggcgg agagggatgg ggatcgaatt 2580

atggccatca tcaaagccac agaagtctcg cataatggaa gatctcaggg actcgccgcg 2640

ccgaacgtca aagcgcaagc cgctcttcat agagcagtct tgcgcaaagc taagctcgac 2700

cctctggaca tccatttcat tgaagcccac ggaactgttt taggaacacc gctgggtgat 2760

ctttgtgaaa tacaaggcat aaatgaagcc tttgtctccg cacgtccacg tgcggaggat 2820

ccactcattg tcagcgcctc caagtcttcc cttgggcaca ccgaaccttc ggctggattg 2880

gttgggatgc tatctgtgtt gatggccttg aagcacggca tcgtgcctgg gttgctccat 2940

ctccgagcgg acaatgctaa ccaccaactg gacctgacac gagtcccact gcgtatatca 3000

ccggaacctg tagtcatcgc cgcatccaag ccgcatcacg ccatggtgct gctcacagtc 3060

cgtacattgg cagacattgt tttggagagt ccggaagaac caccgtccca gaacccggga 3120

gccgcgggcc caatgatatt tgtcctcagc gcgaagactt ccgcggctct gtcggcatat 3180

atcaaggctt acattgcatt tctacagaat gcagacgcgc acgagtttta caacatctgc 3240

tacaccgcct gtgttggaag agaacattac aaacacagat ttgcttgcgt tgcaaatgac 3300

cttgctgatc tgattcgcca attacaagac tgtgcgagtg cgctggctcc aaccaaaagt 3360

agtactggtg ccttggcgtt cgcgttccct ggccaaggcg tgcaatttcc aggcatggcc 3420

gctgcacttg ctaaaaggca ttccatattt cgcgactatg tcatggaatt tggtcataga 3480

gcacaagatc tttgtggctt gcccatcgca aagatgttgc tggacgtgga tgcagcagag 3540

gaagaagata tccacagtga cgtagaccag atttgcgtct ttgtctatca gtattcgatg 3600

tgtcgatggc ttagggagct cgggctcgag gcaagtgcgg ttatcgggca cagtttgggc 3660

gaaataacag ccgcacttat cggggacgca tttacatttg aagtggctct cgatctcgtt 3720

gtcactcgag gacggctact ccgcccttct caagggaatc caggcgggat ggctgcgctg 3780

gcatgccccg aggagaatgt cccagcgatt ttggaccaat gtcgtgtgga cagtaccatt 3840

agcgtctccg ttatcaatgg tccgaggagc ctttgtgtat ccggagcttc caacgacatc 3900

gacgagtttg tcaagatggc aaaacggcaa aacatcaaag cgactcgact gagggtggac 3960

caaggatttc atagccctcg agttgatagt gccgcggttg gactgcgtga atggtcaggt 4020

tctttttcga aatcatttca gccgttgcgc atcacattat actctacgtc tctgggtgct 4080

gcaatctcga aaggagagat tttgactcag acacattggg ccgatcacgt ccgccgtccg 4140

gtcatattct caaaagcagc agcagccatc ctcgaggaca agtccattgg cgcgatcctg 4200

gatatcggac cacagacggt ggcatggtct ctccttctgg cgaacggctg caacgtcgcg 4260

tcagctgttg ccctgtccgg ccgaagagta caagatcagg aaacggcctt tttatctgca 4320

ctggcgaatc tgtatcaaaa tcacggggtg acgccgaatt ttcgcgtatt ttacgctcac 4380

caggcagtcc aggcgcgcta tagaaccgtg gacattccga agtatccctt ccaacgccga 4440

catcgatatc catcctacat tccatcgcgc aatgccacgg gtgccaacag actgaaagaa 4500

ccattccgta gcgacctgga tgaaccggct caagacacgg agcacaccgc ggaaatgaga 4560

gtggacatga ctccggagaa gctccgggac gccctgatgc actgtgtgcg ggacacactg 4620

gaaggcgaag attttgatga atcggaatcc ctcgtttcgc gtggaattga ctccattact 4680

tttgcgggtc tacggaagcg tgttcaagaa cgacacggac ttaatctttc catcattttc 4740

tggtctgatg ggttttctgt gaaagacatg gtcgacagcc tcatcgaaca gcattttgtc 4800

cac 4803

<210> 39
<211> 1601
<212> PRT

<213>  Panellus stipticus

<400>  39

Met Ala Leu Val Ser Pro Thr Cys Ser Ser Gln Met Pro Pro Ala Pro
1               5                   10                  15

Ser Ser Ile Leu Asp Val Phe Thr Glu Thr Ala Tyr Asn Pro Arg Thr
            20                  25                  30

Ser Asn Arg Pro Val Val Glu Cys Gly Glu His Val Trp Thr Tyr Ser
        35                  40                  45

Gln Leu Glu Ala Val Ser Asn Ala Met Ser Gln Asp Leu Glu Asn Ser
    50                  55                  60

Ile Gly Leu Tyr Ala Lys Val Ala Phe Val Gly Glu Asn His Pro Phe
65                  70                  75                  80

Val Phe Ala Leu Met Leu Ala Val Trp Lys Ile Ala Gly Thr Phe Ile
                85                  90                  95

Pro Ile Asp Ala His Ile Pro Pro Ala Leu Leu Asp Gly Met Val Asp
            100                 105                 110

Ile Val Lys Pro Met Cys Ile Tyr Val Ser Ser Ala Asp Thr Ser Asn
        115                 120                 125

Ser Ser Trp Ala Ser Glu Leu Ala Val Glu Val Arg Val Phe Arg Pro
    130                 135                 140

Glu Glu Ser Thr Ile Pro Ala Leu Asn Glu His Tyr Gly Arg Ser Ser
145                 150                 155                 160

Ile Thr Pro Ala Gln His Arg Pro Ile Leu Asn Val Val Gln Ala Val
                165                 170                 175

Pro Leu Thr His Lys Phe Ile Leu Ser Asn Cys Gln Ser Lys Leu Ala
            180                 185                 190

Trp Trp Arg Arg Met Gln Pro Ser Ile Asn Leu Asp Asn Thr Arg Val
        195                 200                 205

Leu Gly Trp Ala Pro Trp Ser His Val Leu Ser His Met Gln Asp Ile
    210                 215                 220

Gly Thr Ala Thr Ile Leu Thr Ala Gly Cys Tyr Val Phe Ala Ser Ile
225                 230                 235                 240

Pro Ser Thr Tyr Gln Leu Gln Gln Ala Pro Thr Asp Leu Thr Thr Gln
                245                 250                 255

Val Ile Asn Gly Ile Leu Asn Lys Asn Ile Ser Ala Leu Ala Ala Leu
            260                 265                 270

Pro Phe Val Phe Gly Gly Ile Lys Ala Ala Cys Glu Ser Gly Asp Leu
        275                 280                 285

Asp Val Glu Ala Leu Leu Gly Ala Leu Arg Arg Met Thr Met Leu Glu
    290                 295                 300

Cys Gly Gly Ala Ala Leu Asp Pro Ala Ile Ala Asn Trp Thr Asp Ile
305                 310                 315                 320

Asn Gly Val Ser Leu Met Val Gly Ile Gly Met Thr Glu Thr Gly Gly
                325                 330                 335

Ala Ile Phe Ala Gly Arg Ala Lys Asp Ser Leu Ser Gly Phe Leu Ala
            340                 345                 350

Glu Gly Leu Ile Ser Asp Ala Arg Ile Glu Leu Asp Lys Gly Glu Ser
        355                 360                 365

Asp Gly Ser Asp Gly Thr Thr Phe Ser Ile Phe Ser Thr Thr Ala Gly
    370                 375                 380

Ile Lys Pro Asp Cys Gln Arg Glu Ser Leu Leu Ser Gln Ala Ser Tyr
385                 390                 395                 400

Phe His Met Ala Thr Leu Ala Ser Met Thr Gly His Phe Pro Trp Met
                405                 410                 415

Arg Lys Gly Asp Asn Phe Leu Arg Gly Ala Ser Thr Ser Val Trp Ala
            420                 425                 430

Ile Ile Glu Leu Thr Asp Ser Ala His Arg Leu Asp Ala Ala Ser Ala
        435                 440                 445

Arg Lys Gln Val Ala Arg Val Leu Ala Pro Leu Asn Gly Gly Leu Pro
    450                 455                 460

Pro Ala Leu Arg Ile Ser Met Ser Ser Val Leu Ile Leu Ser Gly Ser
465                 470                 475                 480

Gln Lys Ile Pro Arg Thr Lys Lys Gly Glu Ile Phe Arg Lys Gln Ile
                485                 490                 495

Glu Asp Leu Phe Gly Ala Ala Met Ser Met Pro Pro Glu Ala Glu Pro
            500                 505                 510

Ala Leu Glu Asp Glu Leu Thr Ser Ile Val Gly Asn Val Leu Asn Ile
        515                 520                 525

Ser Asp Ser Asp Met Leu Thr Ser Met Thr Phe Ala Glu Leu Gly Met
    530                 535                 540

Thr Ser Val Leu Ala Val Lys Ile Ser Ala Arg Leu Asn Glu His Leu
545                 550                 555                 560

Ala Gly Arg Ala Val Leu Pro Ala Asn Ile Cys Tyr Ile Tyr Ile Asp
                565                 570                 575

Thr Pro Ser Leu Ile Ala Gly Ile Arg Asn Phe Leu Ser Pro Thr Ser
            580                 585                 590

Ser Asp Gln Glu Pro Ser Asn Phe Ala Thr Thr Ala Asp Lys Lys Asp
        595                 600                 605

Glu Ile Val Ile Ile Gly Lys Ala Phe Arg Leu Pro Gly Gly Ile Asn
    610                 615                 620

Asp Asp Ser Ala Leu Trp Ala Ala Leu Met Gly Lys Asn Asp Ser Val
625                 630                 635                 640

Ile Ala Asp Ile Pro Pro Asp Arg Trp Asp His Ala Ser Phe Tyr Pro
                645                 650                 655

Ala His Ile Cys Leu Arg Lys Ala Gly Leu Ile Asp Met Ser Ser Tyr
            660                 665                 670

Asp Tyr Gly Phe Phe Gly Leu Ser Ala Thr Glu Ala Tyr Tyr Val Ser
        675                 680                 685

Pro Thr Met Arg Ala Ala Leu Glu Val Ala Phe Glu Ala Leu Glu Asp
    690                 695                 700

Ala Asn Ile Pro Val Ser Arg Ile Lys Gly Thr Asn Thr Ser Val Phe
705                 710                 715                 720

Val Ala Thr Lys Asp Asp Gly Phe Glu Thr Leu Leu Asn Ala Ala His
                725                 730                 735

Gly Phe Asp Ala Tyr Thr Arg Phe Tyr Gly Thr Gly Arg Ala Pro Ser
            740                 745                 750

Thr Ala Ser Gly Arg Ile Ser Tyr Leu Leu Asp Ile His Gly Pro Ser
        755                 760                 765

Leu Thr Val Asp Thr Ala Cys Ser Gly Gly Ile Val Cys Ile Asp Gln
    770                 775                 780

Asp Ile Pro Ala Asn Leu Cys Ile Ile Ala Ile Ala Tyr Leu Gln Ser
785                 790                 795                 800

Gly Ala Gly Glu Ser Ala Ile Glu Arg Gln Tyr Arg Phe Leu Thr Ala
                805                 810                 815

Gln Asn Met Ala Ser Pro Thr Ser Arg Cys Ser Thr Phe Thr Ala Asp
            820                 825                 830

Ala Asp Gly Tyr Val Pro Ser Glu Gly Ala Val Ala Phe Ile Leu Lys
        835                 840                 845

Thr Arg Thr Ala Ala Glu Arg Asp Gly Asp Arg Ile Met Ala Ile Ile
    850                 855                 860

Lys Ala Thr Glu Val Ser His Asn Gly Arg Ser Gln Gly Leu Ala Ala
865                 870                 875                 880

Pro Asn Val Lys Ala Gln Ala Ala Leu His Arg Ala Val Leu Arg Lys
                885                 890                 895

Ala Lys Leu Asp Pro Leu Asp Ile His Phe Ile Glu Ala His Gly Thr
            900                 905                 910

Val Leu Gly Thr Pro Leu Gly Asp Leu Cys Glu Ile Gln Gly Ile Asn
        915                 920                 925

Glu Ala Phe Val Ser Ala Arg Pro Arg Ala Glu Asp Pro Leu Ile Val
    930                 935                 940

Ser Ala Ser Lys Ser Ser Leu Gly His Thr Glu Pro Ser Ala Gly Leu
945                 950                 955                 960

Val Gly Met Leu Ser Val Leu Met Ala Leu Lys His Gly Ile Val Pro
                965                 970                 975

Gly Leu Leu His Leu Arg Ala Asp Asn Ala Asn His Gln Leu Asp Leu
            980                 985                 990

Thr Arg Val Pro Leu Arg Ile Ser Pro Glu Pro Val Val Ile Ala Ala
        995                 1000                1005

Ser Lys Pro His His Ala Met Val Leu Leu Thr Val Arg Thr Leu
    1010                1015                1020

Ala Asp Ile Val Leu Glu Ser Pro Glu Glu Pro Pro Ser Gln Asn
    1025                1030                1035

Pro Gly Ala Ala Gly Pro Met Ile Phe Val Leu Ser Ala Lys Thr
    1040                1045                1050

Ser Ala Ala Leu Ser Ala Tyr Ile Lys Ala Tyr Ile Ala Phe Leu
    1055                1060                1065

Gln Asn Ala Asp Ala His Glu Phe Tyr Asn Ile Cys Tyr Thr Ala
    1070                1075                1080

Cys Val Gly Arg Glu His Tyr Lys His Arg Phe Ala Cys Val Ala
    1085                1090                1095

Asn Asp Leu Ala Asp Leu Ile Arg Gln Leu Gln Asp Cys Ala Ser
    1100                1105                1110

Ala Leu Ala Pro Thr Lys Ser Ser Thr Gly Ala Leu Ala Phe Ala
    1115                1120                1125

Phe Pro Gly Gln Gly Val Gln Phe Pro Gly Met Ala Ala Ala Leu
    1130                1135                1140

Ala Lys Arg His Ser Ile Phe Arg Asp Tyr Val Met Glu Phe Gly
    1145                1150                1155

His Arg Ala Gln Asp Leu Cys Gly Leu Pro Ile Ala Lys Met Leu
    1160                1165                1170

Leu Asp Val Asp Ala Ala Glu Glu Glu Asp Ile His Ser Asp Val
    1175                1180                1185

Asp Gln Ile Cys Val Phe Val Tyr Gln Tyr Ser Met Cys Arg Trp
    1190                1195                1200

Leu Arg Glu Leu Gly Leu Glu Ala Ser Ala Val Ile Gly His Ser
    1205                1210                1215

Leu Gly Glu Ile Thr Ala Ala Leu Ile Gly Asp Ala Phe Thr Phe
    1220                1225                1230

Glu Val Ala Leu Asp Leu Val Val Thr Arg Gly Arg Leu Leu Arg
    1235                1240                1245

Pro Ser Gln Gly Asn Pro Gly Gly Met Ala Ala Leu Ala Cys Pro
    1250                1255                1260

Glu Glu Asn Val Pro Ala Ile Leu Asp Gln Cys Arg Val Asp Ser
    1265                1270                1275

Thr Ile Ser Val Ser Val Ile Asn Gly Pro Arg Ser Leu Cys Val
    1280                1285                1290

Ser Gly Ala Ser Asn Asp Ile Asp Glu Phe Val Lys Met Ala Lys
    1295                1300                1305

Arg Gln Asn Ile Lys Ala Thr Arg Leu Arg Val Asp Gln Gly Phe
    1310                1315                1320

His Ser Pro Arg Val Asp Ser Ala Ala Val Gly Leu Arg Glu Trp
    1325                1330                1335

Ser Gly Ser Phe Ser Lys Ser Phe Gln Pro Leu Arg Ile Thr Leu
    1340                1345                1350

Tyr Ser Thr Ser Leu Gly Ala Ala Ile Ser Lys Gly Glu Ile Leu
    1355                1360                1365

Thr Gln Thr His Trp Ala Asp His Val Arg Arg Pro Val Ile Phe
    1370                1375                1380

Ser Lys Ala Ala Ala Ala Ile Leu Glu Asp Lys Ser Ile Gly Ala
    1385                1390                1395

Ile Leu Asp Ile Gly Pro Gln Thr Val Ala Trp Ser Leu Leu Leu
    1400                1405                1410

Ala Asn Gly Cys Asn Val Ala Ser Ala Val Ala Leu Ser Gly Arg
    1415                1420                1425

Arg Val Gln Asp Gln Glu Thr Ala Phe Leu Ser Ala Leu Ala Asn
    1430                1435                1440

Leu Tyr Gln Asn His Gly Val Thr Pro Asn Phe Arg Val Phe Tyr
    1445                1450                1455

Ala His Gln Ala Val Gln Ala Arg Tyr Arg Thr Val Asp Ile Pro
    1460                1465                1470

Lys Tyr Pro Phe Gln Arg Arg His Arg Tyr Pro Ser Tyr Ile Pro
    1475                1480                1485

Ser Arg Asn Ala Thr Gly Ala Asn Arg Leu Lys Glu Pro Phe Arg
    1490                1495                1500

Ser Asp Leu Asp Glu Pro Ala Gln Asp Thr Glu His Thr Ala Glu
    1505                1510                1515

Met Arg Val Asp Met Thr Pro Glu Lys Leu Arg Asp Ala Leu Met
    1520                1525                1530

His Cys Val Arg Asp Thr Leu Glu Gly Glu Asp Phe Asp Glu Ser
    1535                1540                1545

Glu Ser Leu Val Ser Arg Gly Ile Asp Ser Ile Thr Phe Ala Gly
    1550                1555                1560

Leu Arg Lys Arg Val Gln Glu Arg His Gly Leu Asn Leu Ser Ile
    1565                1570                1575

Ile Phe Trp Ser Asp Gly Phe Ser Val Lys Asp Met Val Asp Ser
    1580                1585                1590

Leu Ile Glu Gln His Phe Val His
    1595                1600

<210> 40
<211> 5073
<212> DNA
<213> Neonothopanus gardneri

<400> 40
atgaattcca aagtgaatct tccctccact ttgcttgatg ttttcctcga gatcgctggc 60

gagccatcta ccgcttcgcg tgatgttttg gaatgtggcg agcacagatg gacttaccag 120

cagcttgacg ttgtttcatc tgctttagcc cagcatctca agtacactgt cggtctatct 180

cctacagtcg cagtgatcag cgaaaatcat ccttatgttt ttgccttgat cctggctgtg 240

tggaaagttg gggcattttt cgcgcccctc gacgcacatg ctcctgctga gttggtagct 300

ggcatgttaa gcataatctc tccttcgtgc ttagtacttc agagcacaga tgtagctaat 360

caaactcttg cgtgtgatct cgatattcct gttgaggtat tccactcgcg tcaatccact 420

attcctgaac taaacaagaa atatctcacc gattctgggt taccggcggg ttttccgctc 480

tcagattcaa acaaaccggc tctgtatctc ttcacctcgt ctgccacttc tcggagcaat 540

cttaaatgcg tgcctctcac tcacgctttc atcctgagca atagcctctc gaaacgcgca 600

tggtgccaac gtatgcggcc agagacagac ttcgatggca tacgcgttct tggatgggct 660

ccatggtctc atgtcttggc gcacatgcag gacatcgggc ccgtcacttt actcaatgct 720

ggatgctacg tctttgctac tatcccttcc tcgtacccca cggatgtgca gagtgacagc 780

aatttgatat ctcatgtcgc aaatgctatc atacacaagg gtgtaaaatc gtttgcttgt 840

ctcccttttg tactcggagg gctgaaagca ttatgcgagt ccaagccatc cgtcaaagca 900

gatctacagg tcgaagagca agctcagctt ttgatctctc taaggcgcat ggaaattctt 960

gaatgcggag gtgcgatgct cgaagcgaat gttgcgtctt gggctatcga gaatcatatt 1020

ccggtctcta ttggtatcgg tatgacagaa actggtggcg cgcttttcgc tggacctgtt 1080

caggatatcc ggaccggttt ttctgcggac aataaattca tcaaggacgc tacctacttc 1140

ctagttgcaa atggagatga atctgggaac gatgtaactg aaggggagct agttgtgaaa 1200

agtaaaatgc tcccgcgagg ctatcttggc tacaatgatt cttccttttc cgttgacgat 1260

gacggattgg ttacgttcaa gacaggcgac agatacagtg ttacacgcga cggaagattc 1320

tcttggctag gcaggaatac tgatttcatt cagatgacta gtggagagac attagatccc 1380

cgacctgtcg agagttcgct ctgccaaagt cctctcatct ctcaagcatg cgttattgga 1440

gacaagtttc tcaacgggcc tgccactgct gtttgcgcga tcgttgagct tgagccgaca 1500

atggtagaaa cgggagaggc taactcgcgg gacatagtcc aagtctttgc acccatcaac 1560

cgagacctgc ctcctccttt aaggatagca tggtcgcaca ttttaattct caagcattcc 1620

gagaagatac cgatgacgaa gaagggaacg attttccgta agaagattga gcagatgttt 1680

ggcgctgcat tgggcattgc ttccatccct acaaacagtg tcaatggatc cgttggctcc 1740

ggagaagagc ctcaagctac tgcagatgag actgttctac aagatgaact atcaaatacc 1800

gtgaagaaaa taatcagccg tgttctagga gtaactgatg aggaattgct ttggacactg 1860

tcattcgcag agctgggaat gacctcaaca ctggctattc gtatcgtaaa cgagctgaac 1920

gaaactctcg ttgggggcga tctccctatc aatgcttgct acatttatgt cgacctttcc 1980

tctctgagca aggctgtgta tgcgaaactg gctcatctcg agctgtcaga tcatgttcct 2040

gagctcaaac aagctccctt caagtcttct ggcgggaaag agattgtcat tgtcggccaa 2100

gcgtttcgtc tacccggctc aatcaatgac gttgcctctc ttcgggatgc attcctagca 2160

agacagacat cgtccatcat cgctggaata ccttccgatc gctgggacca tgccagcttc 2220

tatcccaagg acatatgttt cgacaaggct ggtcttgtgg atatagctca ttatgatcat 2280

agcttcttcg gaatcacagc aacggaagcg ctccatctgt ctccaaccat gcgtcttgca 2340

ctggaagttt cgtttgaggc gcttgagaat gctaatatcc caacgtcgcg attgaaaggc 2400

tcgcagacgg cagtttatgt tgcaacaaca gatgacggat ttgagaccct gttgaatgct 2460

gaggctggct atgaagctta tacacggttt tatggtactg gtcgggcagc aagtacggcg 2520

agtgggcgca taagctatct tcttgatata catggaccct ccgtcactat tgacacggca 2580

tgcagtggtg gagttgtttg tatcgatcaa gcaatcaact atttacaatc gtcgagttca 2640

gcggacaccg ctatcgtgtt cctttctgct caagggatgg tctccccgag gggacgatgt 2700

gcgacattta cggctgatgc tgacggctat gtcccttctg agggtgcagt tgctttcgta 2760

ttgaaaactc gcgaagccgc tatacgcgac aaagacaaca ttctcgctac aatcaaagcg 2820

acacagattt cgcacaacgg ccgctcacaa ggacttgtag caccgaacgt aaactcgcaa 2880

gttgaccttc atcgttcgtt gctcgagaaa gctcgtctta gtcctgctga tgtccaattc 2940

attgaagctc atgggacagg aacgtcattg ggagatctct cagagattca agctataaac 3000

gctgcttaca cttcctctca gccacgtacg accggcccac tcatagtcag cgcttccaag 3060

actgtcgttg gccataccga accagctggt cctttggttg gtatgttgtc ggtcttgctg 3120

tctttccagg aaggcgcagt ccctggtctc gctcatctta ccacacgtaa cctcaatcct 3180

acgttggact attcttcagt gccgcttctc attccctctg aacctgttcg tctacaaaca 3240

ccaaagcctt atagagctgc cgtaatgtcc tacggctttt cgggtaccct ggccggcctc 3300

gttctagaga gccctgacga acgtagctca gaagaagagc cgcctgatga caagccgatg 3360

ctgttcgtcg ttagcgcaaa gacacacacg gcactaattg aatacctgca acggtatctc 3420

gagttcctct tacatgcgaa tccccgcgat ttctgtgata tctgctacac aagctgtgtc 3480

gggagggaac actatagata tcggtttgct tgtgttgcaa atgacatgga agacctcatt 3540

ggccaacttc aaagacgttt gtctagcaag ttaccatcga agccgctata caaacgcggt 3600

gctctggcct ttgcgttttc tggacagggg acacagtttc aaggaatggc gacagatctt 3660

gcgaagaggt actctggctt ccgaaaaatc gtttccggac tcgcaaagag tgctggcgag 3720

ctctcgggtt acgctattga ccgttatctt ctcgcgtatg acgttggtag cagtattgct 3780

actcctaata gtgaggtgga ccagatctgc attttcgtat accaatgctc tgtccttcgc 3840

tggttgggga gtattggaat taaaccaaac gtggtaatcg gccatagcct tggagagatt 3900

tcggcttctg tggcggcagg ggcactttct cttgacattg ctctggatct tgtcatctca 3960

cgagctgggt tgttgcgccc ctcgacagat gttcctgcgg gaatggctgc tgtggccgct 4020

tcacaacagg aggtcattga gttgattgat gcgctggacc ccgacaaggc aaattcgctc 4080

agtgtttcgg ttataaatgg acctcaaaat atcgttgtgt caggcgcttc agcagctatt 4140

gagagaatgg tcgcttctgc gaaggagaag aagatcaaag cttctgttct gaatattagc 4200

caagcttttc acagttcgta tgttgacagt gccattacgg gtcttcgagc ttggtcagaa 4260

aaacatattt cctcagcgat accactgcag attccgctgt attcgacatt gctgggagct 4320

cggatatcaa agggccaaaa actgaaccca gaccactggg tcgaccacgc acggaagccc 4380

gtacagttcg cacaagcagc tacggcgatg aaagaaacct tcaccggagt catcatggat 4440

atcggacccc aagcagtagc atggtcactt ctgcttgcaa acggactcac atccgtaacc 4500

gcgcttgctg cgaagagagg gaggagtcag caggtggctt tcttgagcgc cttggcggag 4560

ttgtatcagg attatggcat tgttcctgat tttgtggcgc tttatgctca ggaggaggaa 4620

atagacaagt tgaggaagac ggatattttg acatatccgt ttcagcgtgt taggaggtat 4680

ccgagtttta taccttcaag gcgtgtgaga ggtggagaaa ttccttcgag ccatcccagc 4740

gagtctgcga cgttggagaa cacgaatgag ggtacggctt tgcgtgctga gtcgagggtg 4800

ttgtgcaggg aggatctgca tgatagcatc gttacctctg tgaaggatgt tctagagctc 4860

aaaccaaatg aagatctaga tttgtctgaa agcctgaacg cgcttggtgt ggactctata 4920

atgtttgctc agttaaggaa acgtattggg gagggactcg ggttgagtat cccgacagtg 4980

ttcctttcgg atgccttttc tattaatgag atggttaata atcttatgga acaggcggag 5040

acgcctggtg aagagggcgt aatgcaggag aat 5073

<210> 41

<211>  1691
<212>  PRT
<213>  Neonothopanus gardneri

<400>  41

Met Asn Ser Lys Val Asn Leu Pro Ser Thr Leu Leu Asp Val Phe Leu
1               5                   10                  15

Glu Ile Ala Gly Glu Pro Ser Thr Ala Ser Arg Asp Val Leu Glu Cys
            20                  25                  30

Gly Glu His Arg Trp Thr Tyr Gln Gln Leu Asp Val Val Ser Ser Ala
        35                  40                  45

Leu Ala Gln His Leu Lys Tyr Thr Val Gly Leu Ser Pro Thr Val Ala
    50                  55                  60

Val Ile Ser Glu Asn His Pro Tyr Val Phe Ala Leu Ile Leu Ala Val
65                  70                  75                  80

Trp Lys Val Gly Gly Ile Phe Ala Pro Leu Asp Ala His Ala Pro Ala
                85                  90                  95

Glu Leu Val Ala Gly Met Leu Ser Ile Ile Ser Pro Ser Cys Leu Val
            100                 105                 110

Leu Gln Ser Thr Asp Val Ala Asn Gln Thr Leu Ala Cys Asp Leu Asp
        115                 120                 125

Ile Pro Val Glu Val Phe His Ser Arg Gln Ser Thr Ile Pro Glu Leu
    130                 135                 140

Asn Lys Lys Tyr Leu Thr Asp Ser Gly Leu Pro Ala Gly Phe Pro Leu
145                 150                 155                 160

Ser Asp Ser Asn Lys Pro Ala Leu Tyr Leu Phe Thr Ser Ser Ala Thr
                165                 170                 175

Ser Arg Ser Asn Leu Lys Cys Val Pro Leu Thr His Ala Phe Ile Leu
            180                 185                 190

Ser Asn Ser Leu Ser Lys Arg Ala Trp Cys Gln Arg Met Arg Pro Glu
        195                 200                 205

Thr Asp Phe Asp Gly Ile Arg Val Leu Gly Trp Ala Pro Trp Ser His
    210                 215                 220

Val Leu Ala His Met Gln Asp Ile Gly Pro Val Thr Leu Leu Asn Ala
225                 230                 235                 240

Gly Cys Tyr Val Phe Ala Thr Ile Pro Ser Ser Tyr Pro Thr Asp Val
                245                 250                 255

Gln Ser Asp Ser Asn Leu Ile Ser His Val Ala Asn Ala Ile Ile His
            260                 265                 270

Lys Gly Val Lys Ser Phe Ala Cys Leu Pro Phe Val Leu Gly Gly Leu
        275                 280                 285

Lys Ala Leu Cys Glu Ser Lys Pro Ser Val Lys Ala Asp Leu Gln Val
    290                 295                 300

Glu Glu Gln Ala Gln Leu Leu Ile Ser Leu Arg Arg Met Glu Ile Leu
305                 310                 315                 320

Glu Cys Gly Gly Ala Met Leu Glu Ala Asn Val Ala Ser Trp Ala Ile
                325                 330                 335

Glu Asn His Ile Pro Val Ser Ile Gly Ile Gly Met Thr Glu Thr Gly
            340                 345                 350

Gly Ala Leu Phe Ala Gly Pro Val Gln Asp Ile Arg Thr Gly Phe Ser
        355                 360                 365

Ala Asp Asn Lys Phe Ile Lys Asp Ala Thr Tyr Phe Leu Val Ala Asn
    370                 375                 380

Gly Asp Glu Ser Gly Asn Asp Val Thr Glu Gly Glu Leu Val Val Lys
385                 390                 395                 400

Ser Lys Met Leu Pro Arg Gly Tyr Leu Gly Tyr Asn Asp Ser Ser Phe
                405                 410                 415

Ser Val Asp Asp Asp Gly Leu Val Thr Phe Lys Thr Gly Asp Arg Tyr
            420                 425                 430

Ser Val Thr Arg Asp Gly Arg Phe Ser Trp Leu Gly Arg Asn Thr Asp
        435                 440                 445

Phe Ile Gln Met Thr Ser Gly Glu Thr Leu Asp Pro Arg Pro Val Glu
    450                 455                 460

Ser Ser Leu Cys Gln Ser Pro Leu Ile Ser Gln Ala Cys Val Ile Gly
465                 470                 475                 480

Asp Lys Phe Leu Asn Gly Pro Ala Thr Ala Val Cys Ala Ile Val Glu
                485                 490                 495

Leu Glu Pro Thr Met Val Glu Thr Gly Glu Ala Asn Ser Arg Asp Ile
            500                 505                 510

Val Gln Val Phe Ala Pro Ile Asn Arg Asp Leu Pro Pro Pro Leu Arg
        515                 520                 525

Ile Ala Trp Ser His Ile Leu Ile Leu Lys His Ser Glu Lys Ile Pro
    530                 535                 540

Met Thr Lys Lys Gly Thr Ile Phe Arg Lys Lys Ile Glu Gln Met Phe
545                 550                 555                 560

Gly Ala Ala Leu Gly Ile Ala Ser Ile Pro Thr Asn Ser Val Asn Gly
                565                 570                 575

Ser Val Gly Ser Gly Glu Glu Pro Gln Ala Thr Ala Asp Glu Thr Val
            580                 585                 590

Leu Gln Asp Glu Leu Ser Asn Thr Val Lys Lys Ile Ile Ser Arg Val
        595                 600                 605

Leu Gly Val Thr Asp Glu Glu Leu Leu Trp Thr Leu Ser Phe Ala Glu
    610                 615                 620

Leu Gly Met Thr Ser Thr Leu Ala Ile Arg Ile Val Asn Glu Leu Asn
625                 630                 635                 640

Glu Thr Leu Val Gly Gly Asp Leu Pro Ile Asn Ala Cys Tyr Ile Tyr
                645                 650                 655

Val Asp Leu Ser Ser Leu Ser Lys Ala Val Tyr Ala Lys Leu Ala His
            660                 665                 670

Leu Glu Leu Ser Asp His Val Pro Glu Leu Lys Gln Ala Pro Phe Lys
        675                 680                 685

Ser Ser Gly Gly Lys Glu Ile Val Ile Val Gly Gln Ala Phe Arg Leu
    690                 695                 700

Pro Gly Ser Ile Asn Asp Val Ala Ser Leu Arg Asp Ala Phe Leu Ala
705                 710                 715                 720

Arg Gln Thr Ser Ser Ile Ile Ala Gly Ile Pro Ser Asp Arg Trp Asp
                725                 730                 735

His Ala Ser Phe Tyr Pro Lys Asp Ile Cys Phe Asp Lys Ala Gly Leu
            740                 745                 750

Val Asp Ile Ala His Tyr Asp His Ser Phe Phe Gly Ile Thr Ala Thr
        755                 760                 765

Glu Ala Leu His Leu Ser Pro Thr Met Arg Leu Ala Leu Glu Val Ser
    770                 775                 780

Phe Glu Ala Leu Glu Asn Ala Asn Ile Pro Thr Ser Arg Leu Lys Gly
785                 790                 795                 800

Ser Gln Thr Ala Val Tyr Val Ala Thr Thr Asp Asp Gly Phe Glu Thr
                805                 810                 815

Leu Leu Asn Ala Glu Ala Gly Tyr Glu Ala Tyr Thr Arg Phe Tyr Gly
            820                 825                 830

Thr Gly Arg Ala Ala Ser Thr Ala Ser Gly Arg Ile Ser Tyr Leu Leu
        835                 840                 845

Asp Ile His Gly Pro Ser Val Thr Ile Asp Thr Ala Cys Ser Gly Gly
    850                 855                 860

Val Val Cys Ile Asp Gln Ala Ile Asn Tyr Leu Gln Ser Ser Ser Ser
865                 870                 875                 880

Ala Asp Thr Ala Ile Val Phe Leu Ser Ala Gln Gly Met Val Ser Pro
                885                 890                 895

Arg Gly Arg Cys Ala Thr Phe Thr Ala Asp Ala Asp Gly Tyr Val Pro
            900                 905                 910

Ser Glu Gly Ala Val Ala Phe Val Leu Lys Thr Arg Glu Ala Ala Ile
        915                 920                 925

Arg Asp Lys Asp Asn Ile Leu Ala Thr Ile Lys Ala Thr Gln Ile Ser
    930                 935                 940

His Asn Gly Arg Ser Gln Gly Leu Val Ala Pro Asn Val Asn Ser Gln
945                 950                 955                 960

Val Asp Leu His Arg Ser Leu Leu Glu Lys Ala Arg Leu Ser Pro Ala
                965                 970                 975

Asp Val Gln Phe Ile Glu Ala His Gly Thr Gly Thr Ser Leu Gly Asp
            980                 985                 990

Leu Ser Glu Ile Gln Ala Ile Asn  Ala Ala Tyr Thr Ser  Ser Gln Pro
        995                 1000                1005

Arg Thr  Thr Gly Pro Leu Ile  Val Ser Ala Ser Lys  Thr Val Val
    1010                1015                1020

Gly His  Thr Glu Pro Ala Gly  Pro Leu Val Gly Met  Leu Ser Val
    1025                1030                1035

Leu Leu  Ser Phe Gln Glu Gly  Ala Val Pro Gly Leu  Ala His Leu
    1040                1045                1050

Thr Thr Arg Asn Leu Asn Pro Thr Leu Asp Tyr Ser Ser Val Pro
    1055                1060                1065

Leu Leu Ile Pro Ser Glu Pro Val Arg Leu Gln Thr Pro Lys Pro
    1070                1075                1080

Tyr Arg Ala Ala Val Met Ser Tyr Gly Phe Ser Gly Thr Leu Ala
    1085                1090                1095

Gly Leu Val Leu Glu Ser Pro Asp Glu Arg Ser Ser Glu Glu Glu
    1100                1105                1110

Pro Pro Asp Asp Lys Pro Met Leu Phe Val Val Ser Ala Lys Thr
    1115                1120                1125

His Thr Ala Leu Ile Glu Tyr Leu Gln Arg Tyr Leu Glu Phe Leu
    1130                1135                1140

Leu His Ala Asn Pro Arg Asp Phe Cys Asp Ile Cys Tyr Thr Ser
    1145                1150                1155

Cys Val Gly Arg Glu His Tyr Arg Tyr Arg Phe Ala Cys Val Ala
    1160                1165                1170

Asn Asp Met Glu Asp Leu Ile Gly Gln Leu Gln Arg Arg Leu Ser
    1175                1180                1185

Ser Lys Leu Pro Ser Lys Pro Leu Tyr Lys Arg Gly Ala Leu Ala
    1190                1195                1200

Phe Ala Phe Ser Gly Gln Gly Thr Gln Phe Gln Gly Met Ala Thr
    1205                1210                1215

Asp Leu Ala Lys Arg Tyr Ser Gly Phe Arg Lys Ile Val Ser Gly
    1220                1225                1230

Leu Ala Lys Ser Ala Gly Glu Leu Ser Gly Tyr Ala Ile Asp Arg
    1235                1240                1245

Tyr Leu Leu Ala Tyr Asp Val Gly Ser Ser Ile Ala Thr Pro Asn
    1250                1255                1260

Ser Glu Val Asp Gln Ile Cys Ile Phe Val Tyr Gln Cys Ser Val
    1265                1270                1275

Leu Arg Trp Leu Gly Ser Ile Gly Ile Lys Pro Asn Val Val Ile
    1280                1285                1290

Gly His Ser Leu Gly Glu Ile Ser Ala Ser Val Ala Ala Gly Ala
    1295                1300                1305

Leu Ser Leu Asp Ile Ala Leu Asp Leu Val Ile Ser Arg Ala Gly
    1310                1315                1320

Leu Leu Arg Pro Ser Thr Asp Val Pro Ala Gly Met Ala Ala Val
    1325                1330                1335

Ala Ala Ser Gln Gln Glu Val Ile Glu Leu Ile Asp Ala Leu Asp
    1340                1345                1350

Pro Asp Lys Ala Asn Ser Leu Ser Val Ser Val Ile Asn Gly Pro
    1355                1360                1365

Gln Asn Ile Val Val Ser Gly Ala Ser Ala Ala Ile Glu Arg Met
    1370                1375                1380

Val Ala Ser Ala Lys Glu Lys Lys Ile Lys Ala Ser Val Leu Asn
    1385                1390                1395

Ile Ser Gln Ala Phe His Ser Ser Tyr Val Asp Ser Ala Ile Thr
    1400                1405                1410

Gly Leu Arg Ala Trp Ser Glu Lys His Ile Ser Ser Ala Ile Pro
    1415                1420                1425

Leu Gln Ile Pro Leu Tyr Ser Thr Leu Leu Gly Ala Arg Ile Ser
    1430                1435                1440

Lys Gly Gln Lys Leu Asn Pro Asp His Trp Val Asp His Ala Arg
    1445                1450                1455

Lys Pro Val Gln Phe Ala Gln Ala Ala Thr Ala Met Lys Glu Thr
    1460                1465                1470

Phe Thr Gly Val Ile Met Asp Ile Gly Pro Gln Ala Val Ala Trp
    1475                1480                1485

Ser Leu Leu Leu Ala Asn Gly Leu Thr Ser Val Thr Ala Leu Ala
    1490                1495                1500

Ala Lys Arg Gly Arg Ser Gln Gln Val Ala Phe Leu Ser Ala Leu
    1505                1510                1515

Ala Glu Leu Tyr Gln Asp Tyr Gly Ile Val Pro Asp Phe Val Ala
    1520                1525                1530

Leu Tyr Ala Gln Glu Glu Glu Ile Asp Lys Leu Arg Lys Thr Asp
    1535                1540                1545

Ile Leu Thr Tyr Pro Phe Gln Arg Val Arg Arg Tyr Pro Ser Phe
    1550                1555                1560

Ile Pro Ser Arg Arg Val Arg Gly Gly Glu Ile Pro Ser Ser His
    1565                1570                1575

Pro Ser Glu Ser Ala Thr Leu Glu Asn Thr Asn Glu Gly Thr Ala
    1580                1585                1590

Leu Arg Ala Glu Ser Arg Val Leu Cys Arg Glu Asp Leu His Asp
    1595                1600                1605

Ser Ile Val Thr Ser Val Lys Asp Val Leu Glu Leu Lys Pro Asn
    1610                1615                1620

Glu Asp Leu Asp Leu Ser Glu Ser Leu Asn Ala Leu Gly Val Asp
    1625                1630                1635

Ser Ile Met Phe Ala Gln Leu Arg Lys Arg Ile Gly Glu Gly Leu
    1640                1645                1650

Gly Leu Ser Ile Pro Thr Val Phe Leu Ser Asp Ala Phe Ser Ile
    1655                1660                1665

Asn Glu Met Val Asn Asn Leu Met Glu Gln Ala Glu Thr Pro Gly
    1670                1675                1680

Glu Glu Gly Val Met Gln Glu Asn
    1685                1690

<210> 42
<211> 5298
<212> DNA
<213> Guyanagaster necrorhiza

<400> 42
atggcggccg ataggcactt ttctcttctc gatgtctttc tcgacgttgc ccataatgct 60

gagacgtcac aacgcaatat tttggaatgc ggcctggaca cctggacata ctcagatttg 120

gacatcatct cgtcggccct ggcccaggat ctcagtacta ccttgggttg ctctcccagg 180

gttgcagtcg tcagcgagaa ccatccctat gtatttgctc tcatgctggc cgtatggaag 240

cttggaggaa tattcatccc catcgacgtc catgttcccg ccgagctttt aacgggcatg 300

ctacgcatcg ttgctccaga ctgcgtggtg attcctgaga ctgatctttc taatcagcgc 360

gtcatctctg cactttacct ccacgttatt cccttcaatg tcaatgcgtc gacaatgtct 420

acacttcgac agaaatatgt cctatctact cagaaaccct tgctgtctga gttccctctt 480

cctcatgttg atcgtgcatg cctctatctc tttacgtcct ccgcgtcttc caccgccaac 540

ttgaaatgcg tacctttgac tcatgcactt atcctcagca actgtcgttc caagcttgca 600

tggtggcggc gtgttcgtcc agaagaaaac atggatggga tgcgtgtttt ggggtgggcg 660

ccttggtcac atattctcgc gtacatgcag gatattggga cagcaacgct cctgaatgca 720

ggctgctatg tctttgcatc tgttccgtcc acatatccta cgcagcacgt agccaatggc 780

ctgcaagacc ccacttcaaa tataatcaag tcgcttctca accgccgtat cacggcgttc 840

gcatgtgtgc cgttcattct tagtgaactg aaagctatgt gcgacccagc ttccggtccg 900

gacgccaagg atcaaatgtg cttgggagct ggggagaaag tgcgtcttat aagcacactg 960

cagaatctca tcatgttcga gtgtggaggc gctgcgctcg aggcagatat cacggattgg 1020

accgtcgaaa atggtatatc agtcatggtc ggtattggaa tgacggagac cgccggcacg 1080

gttttggcag cgcgtgcaca agacgctcgt tcgaatggct attctgccga agaggctctc 1140

atcgctgatg gcatactatc attggtcgga cctgacgagg aggaaatggc ctttgacgga 1200

gaacttgtcg tgaaaagcaa gctcatccca catggataca tcaagtacca cgattcggca 1260

ttttcagtgg actcagatga ctgggtgacg ttcaaaactg gggacaaata tcagcgtaca 1320

ccagatggac atttcaagtg gcttggaaga aagaccgatt ttattcagat gacaagcagc 1380

gaaaccttag atcccagacc tatcgaaaaa gccctctgca tgaaccccat tatcgcaaat 1440

gcgtgtgtca ttggtgacag gtttctaagg gaacctgcga cgggtgtatg tgccattgtc 1500

gagatcaggt cagatgtgga tatggcttcc gccgaggttg acagggagat tgcgaatgtc 1560

ctcgctccaa tcaatcgtga tcttcctccg gctcttcgaa tagcatggtc tcgcgtactc 1620

ataatccgac ctcctcagaa aataccactc acaaagaaag gtgaggtgtt tcgcaagaag 1680

attgaagata tatttggcac cttcttgggt gtcggtgtta ctaccaaggt ggaagtggac 1740

catgaaagta aagaagatga tacggaacac atcgtgagac aggttgtgag caatcttctt 1800

ggagtccatg atcctgagct attgtctact ttatctttcg ctgagctcgg gatgacctca 1860

tttatggccg ttagcatcgt taacacttta aacaagcata ttggcggcct cgcacttcca 1920

cttaactcat gctacattca tattgatctt gattctcttg tagatgccat ttcacttgaa 1980

cgtggtcatc gaaagaaccc tacgcccttt tcttctaacc ctttcctcgc ctttgaatcc 2040

agccagcaaa aggatatgga gattgtgatt gttggtaaag cattccgttt gcccggctca 2100

ctcgacaata gcacctctct ctgggaagct ttgttgtcaa agagtgattc agtcatcggt 2160

gacatcccac ccgatcgctg ggatcatgca agtttttacc cccacgatat atgctttacg 2220

aaagcaggcc ttgtcgatgt ttcccattat gactatagat ttttcggtct cactgctaca 2280

gaggcgttgt acgtatctcc gacaatgcgc ctcgccttgg aagtgtcatt tgaggctctg 2340

gagaatgcaa atattccctt atccaagttg aaggggacac gaactgctgt ttatgttgcc 2400

actaaagacg atgggttcga gacactctta aatgctgaac aaggctacga tgcctacacg 2460

cgattctacg gcacgggacg tgctccgagt accgcaagtg gccgtataag ctatcttctt 2520

gatattcatg gaccttctgt taccgttgac acagcatgca gcggaggcat tgtatgtata 2580

gaccaagcca tcacttttct gcaatccgga ggggcagata ctgctattgt ttgttcgagc 2640

aatacgcact gttggccggg atcatttatg ttcctgacag cgcaaggcat ggtctctcca 2700

aatggaagat gtgccacatt cactacaaat gcagatggat atgtaccttc agaaggcgca 2760

gtggctttcg ttctcaagac acacagcgca gcaacacgcg acaaagacaa tatactggcc 2820

gtgataaagt caacagacgt gtctcataac ggccgttctc aagggctagt tgcaccaaac 2880

gtaaaggcac agacaaacct acaccagtcg ctgttacgaa aagctgggct gcatcctgat 2940

ctaattaact ttatcgaagc tcatggaaca ggtacatctt taggagacct ttcagaaatc 3000

cagggtatca ataatgccta cacctcatct cgacctcgtc cagccggtcc tctgatcatt 3060

agcgcgtcaa aaacggtttt gggacatagt gaaccaactg caggaatggc cggcatcctc 3120

acaaccttgc tcgcctttga gaaagagacg gttcctggtc tcaaccactt aacggaacat 3180

aatttaaacc cttcgcttga ttgctctgca gtgcctctcc tgattcctca cgagcctgtt 3240

catatcagtg gtgcaaagcc ccatcgagct gcggttctat catacggatt cgcggggaca 3300

ctggctggta ccatcttaga gagtccacct tgcgacctta caaggccctt gtcaaacgac 3360

atacaagaac atcctatgat tttcgtcgtc agtgggaaaa cggtgccctc tctggaagca 3420

tatctagagc ggtatttggt atttttacgg gtcgcaaaac caagcgaatt ccatgacatc 3480

tgctacacca cttgcatcgg gagggagctg tataaatacc ggttctcctg cgttgcccga 3540

aacatggccg acctcatttc tcaaattgaa catcgactga cgacttgttc cacttcgaaa 3600

gagaagcccc gtggctcgtt aggattcgtg ttctcgggtc aaggtaccca gttccccggc 3660

atggcagcag cacttgccga acgatattca gggttccgag cgctcgtctc caagtttggg 3720

cagatcgccc aagagcagtc gggctacccg atcgataggc tgctcctcga agttaccgat 3780

acattaccag aagcaaacag cgaggtcgac caaatttgca tttttgtcta ccaatattct 3840

gttctgcaat ggctgcaacg tctaggcatt caaccgaaaa cagtccttgg tcacagtcta 3900

ggagaaatta ccgcctcagt cgcagttggc gccttctctt ttaggtctgc attggacctt 3960

gtggtcaccc gcgctcgtct tcttcgtcct caaccaaaat tctctgcggg aatggctgca 4020

gtagcagcgt ccaaggaaga agttgaagga cttatagata tgctcaaact tgcggagtcg 4080

ctgagcgttg cggttcataa cagtccacgg agtatcgttg tatcaggcgc atcagctgcg 4140

gtcgatgcca tggttgtcgc tgctaaaaag cagggcttga aggcctcccg cttaaaggtt 4200

gaccaagcct ttcacagccc ttacgttgat tctgctgtat cggggttgct cgactggtcc 4260

aataagcatc gttcgacctt cctcccattg aacatccctt tatactcaac tttgactggc 4320

gcgcgtattc cgaaaggagg gaagttctgc tgggatcact gggttaatca tgctcgaaag 4380

cccgtccagt ttgcggcggc agctgcagca atggacgaag atcaatccat cggtgttctt 4440

gttgatgttg gaccccaacc tgttgcgtgg accctccttc aagcgaataa cctcctcaat 4500

acctcttcga ttgctctatc agcaaaaatt ggaaaggatc aggagatggc gttgctctct 4560

gctttgagct acctcgtcca agagcacaac ctttctctca gcttttatga gctttactct 4620

cagcgtcacg gtactctgaa gaagacagac gttcctacct acccgttccg tcgcgtccac 4680

cgctacccga ctttcatacc gtcacggaat agaagtcctg ctgacatgag ggtagctata 4740

ccacctaccg acctctctgt ccgaaagaat gtggatgcaa caccgcagtc tcgtcgtgct 4800

ggcctgatag cctgtcttaa agttatcctc gagttaacac caggagagga atttgacctt 4860

tctgagactc tcaatgctcg tggggtcgat tcagttatgt tcgcacagtt gcggaagcgt 4920

gttggggaag aattcgacct agatatacca atgatctatt tatcagacgt gttcacgatg 4980

gaacagatga ttgactacct cgtcgaacag tccagcccca catcaaagtt ggtagagatt 5040

tcggttaatc aatcattaga cggagaaggc ctccggacag ggcttgtatc atgccttagg 5100

gacgtactgg aaatctcctc cgacgaagaa cttgactttt ccgaaacttt gaacgctcgt 5160

ggtacggact caattatgtt cgctcagcta cgaaaacgtg tcggggaagg atttggtctt 5220

gaaattccga tgatatatct gtctgatgtg tttaccatgg aagacatgat caatttcctt 5280

gtctcggagc gctcgtgc 5298

<210> 43
<211> 1766
<212> PRT
<213> Guyanagaster necrorhiza

<400> 43

Met Ala Ala Asp Arg His Phe Ser Leu Leu Asp Val Phe Leu Asp Val
1               5                   10                  15

Ala His Asn Ala Glu Thr Ser Gln Arg Asn Ile Leu Glu Cys Gly Leu
            20                  25                  30

Asp Thr Trp Thr Tyr Ser Asp Leu Asp Ile Ile Ser Ser Ala Leu Ala
        35                  40                  45

Gln Asp Leu Ser Thr Thr Leu Gly Cys Ser Pro Arg Val Ala Val Val
    50                  55                  60

Ser Glu Asn His Pro Tyr Val Phe Ala Leu Met Leu Ala Val Trp Lys
65                  70                  75                  80

Leu Gly Gly Ile Phe Ile Pro Ile Asp Val His Val Pro Ala Glu Leu
                85                  90                  95

Leu Thr Gly Met Leu Arg Ile Val Ala Pro Asp Cys Val Val Ile Pro
            100                 105                 110

Glu Thr Asp Leu Ser Asn Gln Arg Val Ile Ser Ala Leu Tyr Leu His
        115                 120                 125

Val Ile Pro Phe Asn Val Asn Ala Ser Thr Met Ser Thr Leu Arg Gln
    130                 135                 140

Lys Tyr Val Leu Ser Thr Gln Lys Pro Leu Leu Ser Glu Phe Pro Leu
145                 150                 155                 160

Pro His Val Asp Arg Ala Cys Leu Tyr Leu Phe Thr Ser Ser Ala Ser
                165                 170                 175

Ser Thr Ala Asn Leu Lys Cys Val Pro Leu Thr His Ala Leu Ile Leu
            180                 185                 190

Ser Asn Cys Arg Ser Lys Leu Ala Trp Trp Arg Arg Val Arg Pro Glu
        195                 200                 205

Glu Asn Met Asp Gly Met Arg Val Leu Gly Trp Ala Pro Trp Ser His
    210                 215                 220

Ile Leu Ala Tyr Met Gln Asp Ile Gly Thr Ala Thr Leu Leu Asn Ala
225                 230                 235                 240

Gly Cys Tyr Val Phe Ala Ser Val Pro Ser Thr Tyr Pro Thr Gln His
                245                 250                 255

Val Ala Asn Gly Leu Gln Asp Pro Thr Ser Asn Ile Ile Lys Ser Leu
            260                 265                 270

Leu Asn Arg Arg Ile Thr Ala Phe Ala Cys Val Pro Phe Ile Leu Ser
        275                 280                 285

Glu Leu Lys Ala Met Cys Asp Pro Ala Ser Gly Pro Asp Ala Lys Asp
    290                 295                 300

Gln Met Cys Leu Gly Ala Gly Glu Lys Val Arg Leu Ile Ser Thr Leu
305                 310                 315                 320

Gln Asn Leu Ile Met Phe Glu Cys Gly Gly Ala Ala Leu Glu Ala Asp
                325                 330                 335

Ile Thr Asp Trp Thr Val Glu Asn Gly Ile Ser Val Met Val Gly Ile
            340                 345                 350

Gly Met Thr Glu Thr Ala Gly Thr Val Leu Ala Ala Arg Ala Gln Asp
        355                 360                 365

Ala Arg Ser Asn Gly Tyr Ser Ala Glu Glu Ala Leu Ile Ala Asp Gly
    370                 375                 380

Ile Leu Ser Leu Val Gly Pro Asp Glu Glu Glu Met Ala Phe Asp Gly
385                 390                 395                 400

Glu Leu Val Val Lys Ser Lys Leu Ile Pro His Gly Tyr Ile Lys Tyr
                405                 410                 415

His Asp Ser Ala Phe Ser Val Asp Ser Asp Asp Trp Val Thr Phe Lys
            420                 425                 430

Thr Gly Asp Lys Tyr Gln Arg Thr Pro Asp Gly His Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Lys Ala Leu Cys Met Asn Pro Ile Ile Ala Asn
465                 470                 475                 480

Ala Cys Val Ile Gly Asp Arg Phe Leu Arg Glu Pro Ala Thr Gly Val
                485                 490                 495

Cys Ala Ile Val Glu Ile Arg Ser Asp Val Asp Met Ala Ser Ala Glu
            500                 505                 510

Val Asp Arg Glu Ile Ala Asn Val Leu Ala Pro Ile Asn Arg Asp Leu
        515                 520                 525

Pro Pro Ala Leu Arg Ile Ala Trp Ser Arg Val Leu Ile Ile Arg Pro
    530                 535                 540

Pro Gln Lys Ile Pro Leu Thr Lys Lys Gly Glu Val Phe Arg Lys Lys
545                 550                 555                 560

Ile Glu Asp Ile Phe Gly Thr Phe Leu Gly Val Gly Val Thr Thr Lys
                565                 570                 575

Val Glu Val Asp His Glu Ser Lys Glu Asp Asp Thr Glu His Ile Val
            580                 585                 590

Arg Gln Val Val Ser Asn Leu Leu Gly Val His Asp Pro Glu Leu Leu
        595                 600                 605

Ser Thr Leu Ser Phe Ala Glu Leu Gly Met Thr Ser Phe Met Ala Val
    610                 615                 620

Ser Ile Val Asn Thr Leu Asn Lys His Ile Gly Gly Leu Ala Leu Pro
625                 630                 635                 640

Leu Asn Ser Cys Tyr Ile His Ile Asp Leu Asp Ser Leu Val Asp Ala
                645                 650                 655

Ile Ser Leu Glu Arg Gly His Arg Lys Asn Pro Thr Pro Phe Ser Ser
            660                 665                 670

Asn Pro Phe Leu Ala Phe Glu Ser Ser Gln Gln Lys Asp Met Glu Ile
        675                 680                 685

Val Ile Val Gly Lys Ala Phe Arg Leu Pro Gly Ser Leu Asp Asn Ser
    690                 695                 700

Thr Ser Leu Trp Glu Ala Leu Leu Ser Lys Ser Asp Ser Val Ile Gly
705                 710                 715                 720

Asp Ile Pro Pro Asp Arg Trp Asp His Ala Ser Phe Tyr Pro His Asp
                725                 730                 735

Ile Cys Phe Thr Lys Ala Gly Leu Val Asp Val Ser His Tyr Asp Tyr
            740                 745                 750

Arg Phe Phe Gly Leu Thr Ala Thr Glu Ala Leu Tyr Val Ser Pro Thr
        755                 760                 765

Met Arg Leu Ala Leu Glu Val Ser Phe Glu Ala Leu Glu Asn Ala Asn
    770                 775                 780

Ile Pro Leu Ser Lys Leu Lys Gly Thr Arg Thr Ala Val Tyr Val Ala
785                 790                 795                 800

Thr Lys Asp Asp Gly Phe Glu Thr Leu Leu Asn Ala Glu Gln Gly Tyr
                805                 810                 815

Asp Ala Tyr Thr Arg Phe Tyr Gly Thr Gly Arg Ala Pro Ser Thr Ala
            820                 825                 830

Ser Gly Arg Ile Ser Tyr Leu Leu Asp Ile His Gly Pro Ser Val Thr
        835                 840                 845

Val Asp Thr Ala Cys Ser Gly Gly Ile Val Cys Ile Asp Gln Ala Ile
    850                 855                 860

Thr Phe Leu Gln Ser Gly Gly Ala Asp Thr Ala Ile Val Cys Ser Ser
865                 870                 875                 880

Asn Thr His Cys Trp Pro Gly Ser Phe Met Phe Leu Thr Ala Gln Gly
                885                 890                 895

Met Val Ser Pro Asn Gly Arg Cys Ala Thr Phe Thr Thr Asn Ala Asp
            900                 905                 910

Gly Tyr Val Pro Ser Glu Gly Ala Val Ala Phe Val Leu Lys Thr His
        915                 920                 925

Ser Ala Ala Thr Arg Asp Lys Asp Asn Ile Leu Ala Val Ile Lys Ser
    930                 935                 940

Thr Asp Val Ser His Asn Gly Arg Ser Gln Gly Leu Val Ala Pro Asn
945                 950                 955                 960

Val Lys Ala Gln Thr Asn Leu His Gln Ser Leu Leu Arg Lys Ala Gly
                965                 970                 975

Leu His Pro Asp Leu Ile Asn Phe Ile Glu Ala His Gly Thr Gly Thr
            980                 985                 990

Ser Leu Gly Asp Leu Ser Glu Ile  Gln Gly Ile Asn Asn  Ala Tyr Thr
        995                 1000                1005

Ser Ser  Arg Pro Arg Pro Ala  Gly Pro Leu Ile Ile  Ser Ala Ser
    1010                1015                1020

Lys Thr  Val Leu Gly His Ser  Glu Pro Thr Ala Gly  Met Ala Gly
    1025                1030                1035

Ile Leu  Thr Thr Leu Leu Ala  Phe Glu Lys Glu Thr  Val Pro Gly
    1040                1045                1050

Leu Asn  His Leu Thr Glu His  Asn Leu Asn Pro Ser  Leu Asp Cys
    1055                1060                1065

Ser Ala  Val Pro Leu Leu Ile  Pro His Glu Pro Val  His Ile Ser
    1070                1075                1080

Gly Ala  Lys Pro His Arg Ala  Ala Val Leu Ser Tyr  Gly Phe Ala
    1085                1090                1095

Gly Thr  Leu Ala Gly Thr Ile  Leu Glu Ser Pro Pro  Cys Asp Leu
    1100                1105                1110

Thr Arg  Pro Leu Ser Asn Asp  Ile Gln Glu His Pro  Met Ile Phe
    1115                1120                1125

Val Val Ser Gly Lys Thr Val Pro Ser Leu Glu Ala Tyr Leu Glu
    1130                1135                1140

Arg Tyr Leu Val Phe Leu Arg Val Ala Lys Pro Ser Glu Phe His
    1145                1150                1155

Asp Ile Cys Tyr Thr Thr Cys Ile Gly Arg Glu Leu Tyr Lys Tyr
    1160                1165                1170

Arg Phe Ser Cys Val Ala Arg Asn Met Ala Asp Leu Ile Ser Gln
    1175                1180                1185

Ile Glu His Arg Leu Thr Thr Cys Ser Thr Ser Lys Glu Lys Pro
    1190                1195                1200

Arg Gly Ser Leu Gly Phe Val Phe Ser Gly Gln Gly Thr Gln Phe
    1205                1210                1215

Pro Gly Met Ala Ala Ala Leu Ala Glu Arg Tyr Ser Gly Phe Arg
    1220                1225                1230

Ala Leu Val Ser Lys Phe Gly Gln Ile Ala Gln Glu Gln Ser Gly
    1235                1240                1245

Tyr Pro Ile Asp Arg Leu Leu Leu Glu Val Thr Asp Thr Leu Pro
    1250                1255                1260

Glu Ala Asn Ser Glu Val Asp Gln Ile Cys Ile Phe Val Tyr Gln
    1265                1270                1275

Tyr Ser Val Leu Gln Trp Leu Gln Arg Leu Gly Ile Gln Pro Lys
    1280                1285                1290

Thr Val Leu Gly His Ser Leu Gly Glu Ile Thr Ala Ser Val Ala
    1295                1300                1305

Val Gly Ala Phe Ser Phe Arg Ser Ala Leu Asp Leu Val Val Thr
    1310                1315                1320

Arg Ala Arg Leu Leu Arg Pro Gln Pro Lys Phe Ser Ala Gly Met
    1325                1330                1335

Ala Ala Val Ala Ala Ser Lys Glu Glu Val Glu Gly Leu Ile Asp
    1340                1345                1350

Met Leu Lys Leu Ala Glu Ser Leu Ser Val Ala Val His Asn Ser
    1355                1360                1365

Pro Arg Ser Ile Val Val Ser Gly Ala Ser Ala Ala Val Asp Ala
    1370                1375                1380

Met Val Val Ala Ala Lys Lys Gln Gly Leu Lys Ala Ser Arg Leu
    1385                1390                1395

Lys Val Asp Gln Ala Phe His Ser Pro Tyr Val Asp Ser Ala Val
    1400                1405                1410

Ser Gly Leu Leu Asp Trp Ser Asn Lys His Arg Ser Thr Phe Leu
    1415                1420                1425

Pro Leu Asn Ile Pro Leu Tyr Ser Thr Leu Thr Gly Ala Arg Ile
    1430                1435                1440

Pro Lys Gly Gly Lys Phe Cys Trp Asp His Trp Val Asn His Ala
    1445                1450                1455

Arg Lys Pro Val Gln Phe Ala Ala Ala Ala Ala Ala Met Asp Glu
    1460                1465                1470

Asp Gln Ser Ile Gly Val Leu Val Asp Val Gly Pro Gln Pro Val
    1475                1480                1485

Ala Trp Thr Leu Leu Gln Ala Asn Asn Leu Leu Asn Thr Ser Ser
    1490                1495                1500

Ile Ala Leu Ser Ala Lys Ile Gly Lys Asp Gln Glu Met Ala Leu
    1505                1510                1515

Leu Ser Ala Leu Ser Tyr Leu Val Gln Glu His Asn Leu Ser Leu
    1520                1525                1530

Ser Phe Tyr Glu Leu Tyr Ser Gln Arg His Gly Thr Leu Lys Lys
    1535                1540                1545

Thr Asp Val Pro Thr Tyr Pro Phe Arg Arg Val His Arg Tyr Pro
    1550                1555                1560

Thr Phe Ile Pro Ser Arg Asn Arg Ser Pro Ala Asp Met Arg Val
    1565                1570                1575

Ala Ile Pro Pro Thr Asp Leu Ser Val Arg Lys Asn Val Asp Ala
    1580                1585                1590

Thr Pro Gln Ser Arg Arg Ala Gly Leu Ile Ala Cys Leu Lys Val
    1595                1600                1605

Ile Leu Glu Leu Thr Pro Gly Glu Glu Phe Asp Leu Ser Glu Thr
    1610                1615                1620

Leu Asn Ala Arg Gly Val Asp Ser Val Met Phe Ala Gln Leu Arg
    1625                1630                1635

Lys Arg Val Gly Glu Glu Phe Asp Leu Asp Ile Pro Met Ile Tyr
    1640                1645                1650

Leu Ser Asp Val Phe Thr Met Glu Gln Met Ile Asp Tyr Leu Val
    1655                1660                1665

Glu Gln Ser Ser Pro Thr Ser Lys Leu Val Glu Ile Ser Val Asn
    1670                1675                1680

Gln Ser Leu Asp Gly Glu Gly Leu Arg Thr Gly Leu Val Ser Cys
    1685                1690                1695

Leu Arg Asp Val Leu Glu Ile Ser Ser Asp Glu Glu Leu Asp Phe
    1700                1705                1710

Ser Glu Thr Leu Asn Ala Arg Gly Thr Asp Ser Ile Met Phe Ala
    1715                1720                1725

Gln Leu Arg Lys Arg Val Gly Glu Gly Phe Gly Leu Glu Ile Pro
    1730                1735                1740

Met Ile Tyr Leu Ser Asp Val Phe Thr Met Glu Asp Met Ile Asn
    1745                1750                1755

Phe Leu Val Ser Glu Arg Ser Cys
    1760                1765

<210> 44
<211> 5034
<212> DNA
<213> Mycena citricolor

<400> 44
atgtccgcct cttcctccta ttctgaactg gagtctccga cgtcgctgct tgacgtattc 60

gtgcacgccg cgcgagaccc ccacaccgcg tcgcgtcgcg tgctggagtg cggctcggac 120

acatggacat acgcggcctt ggatgcggta tccgatggca tagcgaggga gctcgcgccc 180

ttcggtctgg cgcccaaggt cgctgtggtc agcgagaacc atccatttgt gttcgcgctg 240

ctgttcgcag tctggaagct cgggggaacg ttcatcccca tcgacgcgca tgtccccttt 300

gccatgctca cggggatggt gaacatcgtg aagccgacgt gtctctacct ccccgcctcc 360

gccacatcca acatatctct cgtgaaggcc ttcgacatac ggaccgtcgt atttgggcac 420

aaggagaatt ccatgcaagc tttgttcgac aagtactcat tgcacgcggc gccgttgcac 480

gcggcaccat tgcattacgc gcctcccagt gccgaccatg cctgtctgta tctcttcacg 540

tcgtcagcgt cctcgaccaa aaacctcaag gcggtcccgc tgacgcatac actcgtcctg 600

cgggggtgtc aatccaaact cgcttggtgg cgccgagtcc aacccggaaa gaatctcgac 660

gctatccgga tcctcggctg ggcaccctgg tctcacgttc tcgcctatat gcaggatatc 720

gggacagcga cactgctcaa cgcgggctgc tacgtctttg cgaccatccc ttcgtcctac 780

ccgcagctgc ctaccactac ccccgtcgat ctgacgactt cgctcatcga cgcgctcgtc 840

acgcggcgcg tcgcggcatt cgcctgtgtc cccttcgtcc tggcgaacct caaagccgcg 900

tgccagagca accaccccgc gcgctcccag ctgctcgagg cgctgcagaa gacgatcatg 960

ctcgagtgcg gtggggcggt gctggacgac gcgacggtgg actgggccga gcgcaacggc 1020

atccgcatct tcacgggaat cggatgaccc gagactggcg gggccgtttt cgtcggcctc 1080

gcgagcgaat ctagacgcgg gtttctaccg gaaggcctac tcggtgatgc atccttctcg 1140

atctcgagtg acacggacgc tcttgacgaa ggggaacttg tcgtgaaaag taaactcatc 1200

gcgcccggat atgtcggcta cgacgacgga gcgcactccg tggactgcga cggctgggtc 1260

acattcagga caggagatcg atatcgccag acccagccag acgggcgctt tacgtggcta 1320

ggaaggatca cagacttcat tcagatggtc agcggcgagt acctcgatcc gcggcccctc 1380

gaggagagtc tgcgtgcgtc cccgctgatc gccaacgcct gcatcgtggg cgacgcgttc 1440

ctcagcagcg cctcgacgag catcctcgcc atcatcgagc tcgcgacccc ggatctggcg 1500

cacacggcct ccatccgggc gcagctcgcg cgcgtcctcg cgcccctcaa ccgcgatctg 1560

cctccaccgc ttcggatcgc agcgtcttcg atcctggttc tggacgggat gcggaagatc 1620

ccgaagacga agaagggcga catcttccgc aaaaagctgg aagacacgtt cggtgcggag 1680

tttgagcaga tgctgcggac cgagaaagtc gggctgggtg acttggcgga tgtggatgcc 1740

ggtatcactc gtatcatcgg caacttgctc ggcatctccg acgacgagct tctgtcgacc 1800

atgtcgtttg ctgagctcgg gatgacctcg cttctcgccg tcaagatcgc gagcgaacta 1860

aacaagtttc tggacggccg ggctgtgctg cccacgaaca tctgttacat acactttgac 1920

gtgccctcgc tcacgagtag cgtccgagaa aggctatcct ccgcgccctc ctcgatcaca 1980

tccgccgcca gcgagcccgc ggcctcgtcc cccggcaccc cccgcgccga cgaaatcgtg 2040

atcgtgggca aggccttccg gctgccggac ggcgtgaacg acgacgccgc gctgtgggac 2100

gtcctgacgg gcgagtccgc gtcgatcatc aaggacatcc ccgccgaccg ctgggaccac 2160

gcgagcttct acccgaagga catccacttt ggcagagctg gtctggtgga tgtcgcgcgg 2220

ttcgactacg gctttttcgg catgacggcg agcgaggcgt attcgctgtc gccgacgatg 2280

cgcttggcgc tcgaagtcgc gtatgaggcg ttagaggacg cgaacattcc gttccgggcc 2340

gtcaagggct cgcgcatggg tgtgttcgtc gctgtgaaag acgatggatt cgagaccctg 2400

ttgcatgcgg ggcaggttta tgacgcttac acgcggttct atggaactgg aagggcgccg 2460

agcaccgcca gcggccgcat caactatctg ctcgatctcc acgggccgtc gatcaccgtc 2520

gacacggcct gcagcggcgg catcgtgtgc atcgaccagg ctgtcaccta tcttcaatcg 2580

ggcgctgcag agacggcgat cgtgtgttcg agcaacacac actgctggcc cggatccttc 2640

atgttcttga ccgcgcaagg catggcctcc cccaacggcc ggtgcgcatc cttcacatca 2700

gacgccgacg gatacgcgcc gtcggagggc gcggtggctt tcgtgctgaa gacgcggtct 2760

gccgctgtgc gcgacggcga tcggatactg gctacgatca gggccacgga gatcggacac 2820

aatggacggt ctcagggact cgctgcgccg aatgtcaggt cccaggcggc tgctcatcgg 2880

gcggtgctgc ggagagcgag gctggacccg tccgagattg acttcatcga agcgcatggg 2940

actggcacca cattgggcga tctgtgcgaa gtgcagggca tcaacgacag ctttgtctcg 3000

cccaagaaac gcgccaaccc tcttgtggtc agcgcgtcca agagcaccat cggccacacc 3060

gagccgtcgg cgggtctcgt cgggatcctg tccgcgctga tgtcgttcga gaagcgcatc 3120

gtgccgagat tggcatatct gactgagagc aatgtgaacc cggcgctcga cgcgagtgtc 3180

gttccgctgc actttcccac aaagcacatc gagctgcgcg ctgatgtgcc gtacaaggcc 3240

gtagtgatgt cgtacggatt cgcaggtacc ctagccgaca tcgtcctcga gagcgaggta 3300

ccccaaccca cgcccgcggt cgctcaggac acggccggcc aacagccaat gctcttcgtg 3360

ctcagcgcca aaaccccacg cgcactcgca gcctacatcg agctgtacct aggcttcctg 3420

cggcacgcgg acccgggcct cttcgcgcgc atctgctaca cggcgtgtgt cgcgcgcgag 3480

cactacaagc accgcgtcgc gtgcgttgcg acggacctcg tcgacctcat cgcgcagctc 3540

gagacgcgtc tggtgcagac ggcgtatgcg ggcggcggcg gggcccgggc cgcgcgcacc 3600

gggccgctgg tgtttgcgtt ttcggggcag ggcacgcagt tcccggcgat ggcggcgccg 3660

ctggcgcggc ggtacgcgcg cttcggggag atcgtggggg gctgtgcgcg catggcgcgc 3720

gagctgagcg ggttccccgt ggatggcatt ctcctcggag acgatgtgac gcccgtgaag 3780

gacaacagcg ctgcggcgga ggtgcacagc gaggtggacc agatctgcat atttgtgtac 3840

cagtatgcga tgtgtcggtg gttgggcgag ctgggtgtgg agcccaaggc tgccataggc 3900

cacagcttgg gagagatcac agcagcggtc atcgcaggag cactcccttt cgaagccgcc 3960

ctcgatctgg tcgtcacgcg cgcccggctg ctcaagccgt gcgccgagca accgagcggc 4020

atggcggcgc tcgcctgcac cccagacgtc gcatcgaagc tcacactcgg cgcgtcggtg 4080

tcggtgtcgg tctacaacgg cccgcagagc atctgcctgt ccggcgcgtc ggccgagctc 4140

gacgatgcgg tgcgggccgc gaaggcgcgg aacatcaagg cgacgcggct gcaggtcgac 4200

cagggcttcc actcgccgtg cgtcgacgcc gcggtgccgg ggctgcaggc gtggtgcgcg 4260

gcgcaccgtg cgtcagctgc gccgctgaag atgccgctgt actcgaccgt gcgcggggac 4320

gtcgttccga agggagcggc gctggatccg gagcactggg tggcgcacgc gcggaacccg 4380

gtgctgttcg cgcagacggc gcaggctctg aaagagtccc ttccgcatgg gatcactttg 4440

gacgtcggcc cgcaggcggt ggcgtggtcg ctgctgctgc tgaacgggct cagcgcgacg 4500

cgcacggtcg ctgccggggc gaagaagggc gcagaccagg agcgcgcgct gctgggggcg 4560

ctgggggcgc tgttcgagca gcacaaggtc acgccggact ttgggcggct gtacgcgccg 4620

ctcgagaaga cgcggatccc gacgtacccc tttgagcgcg cgcggtgcta cccgacgttc 4680

ataccctcgc gcttcgcgca cggggctgcg gctgctgcga agacagatgg cgaggtcctg 4740

tcggccgaag aagaaaatgt ggcacccgtg ggattgctga caaaggagga tctgcgtgcg 4800

gctctcgtcg cgtgtctccg agcgacgctc gagctgcgcc ccgacgaaga gctggacgaa 4860

gcagagccgc tgaccgtgca cggcgtggac tcgatcgggt ttgcgaagct gcgcaagcac 4920

gtcgaggacc gctgggggct ggacatcccc gtcgtgtact ggtccgacgc gttcaccgtc 4980

ggcgagatgc tcggcaactt ggtcggccag tatgacgtag tgtctactgc tgcg 5034

<210> 45
<211> 1678
<212> PRT
<213> Mycena citricolor

<400> 45

Met Ser Ala Ser Ser Ser Tyr Ser Glu Leu Glu Ser Pro Thr Ser Leu
1               5                   10                  15

Leu Asp Val Phe Val His Ala Ala Arg Asp Pro His Thr Ala Ser Arg
            20                  25                  30

Arg Val Leu Glu Cys Gly Ser Asp Thr Trp Thr Tyr Ala Ala Leu Asp
        35                  40                  45

Ala Val Ser Asp Gly Ile Ala Arg Glu Leu Ala Pro Phe Gly Leu Ala
    50                  55                  60

Pro Lys Val Ala Val Val Ser Glu Asn His Pro Phe Val Phe Ala Leu
65                  70                  75                  80

Leu Phe Ala Val Trp Lys Leu Gly Gly Thr Phe Ile Pro Ile Asp Ala
                85                  90                  95

His Val Pro Phe Ala Met Leu Thr Gly Met Val Asn Ile Val Lys Pro
            100                 105                 110

Thr Cys Leu Tyr Leu Pro Ala Ser Ala Thr Ser Asn Ile Ser Leu Val
        115                 120                 125

Lys Ala Phe Asp Ile Arg Thr Val Val Phe Gly His Lys Glu Asn Ser
    130                 135                 140

Met Gln Ala Leu Phe Asp Lys Tyr Ser Leu His Ala Ala Pro Leu His
145                 150                 155                 160

Ala Ala Pro Leu His Tyr Ala Pro Pro Ser Ala Asp His Ala Cys Leu
                165                 170                 175

Tyr Leu Phe Thr Ser Ser Ala Ser Ser Thr Lys Asn Leu Lys Ala Val
            180                 185                 190

Pro Leu Thr His Thr Leu Val Leu Arg Gly Cys Gln Ser Lys Leu Ala
        195                 200                 205

Trp Trp Arg Arg Val Gln Pro Gly Lys Asn Leu Asp Ala Ile Arg Ile
    210                 215                 220

Leu Gly Trp Ala Pro Trp Ser His Val Leu Ala Tyr Met Gln Asp Ile
225                 230                 235                 240

Gly Thr Ala Thr Leu Leu Asn Ala Gly Cys Tyr Val Phe Ala Thr Ile
                245                 250                 255

Pro Ser Ser Tyr Pro Gln Leu Pro Thr Thr Thr Pro Val Asp Leu Thr
            260                 265                 270

Thr Ser Leu Ile Asp Ala Leu Val Thr Arg Arg Val Ala Ala Phe Ala
        275                 280                 285

Cys Val Pro Phe Val Leu Ala Asn Leu Lys Ala Ala Cys Gln Ser Asn
    290                 295                 300

His Pro Ala Arg Ser Gln Leu Leu Glu Ala Leu Gln Lys Thr Ile Met
305                 310                 315                 320

Leu Glu Cys Gly Gly Ala Val Leu Asp Asp Ala Thr Val Asp Trp Ala
                325                 330                 335

Glu Arg Asn Gly Ile Arg Ile Phe Thr Gly Ile Gly Met Thr Glu Thr
            340                 345                 350

Gly Gly Ala Val Phe Val Gly Leu Ala Ser Glu Ser Arg Arg Gly Phe
        355                 360                 365

Leu Pro Glu Gly Leu Leu Gly Asp Ala Ser Phe Ser Ile Ser Ser Asp
    370                 375                 380

Thr Asp Ala Leu Asp Glu Gly Glu Leu Val Val Lys Ser Lys Leu Ile
385                 390                 395                 400

Ala Pro Gly Tyr Val Gly Tyr Asp Asp Gly Ala His Ser Val Asp Cys
                405                 410                 415

Asp Gly Trp Val Thr Phe Arg Thr Gly Asp Arg Tyr Arg Gln Thr Gln
            420                 425                 430

Pro Asp Gly Arg Phe Thr Trp Leu Gly Arg Ile Thr Asp Phe Ile Gln
        435                 440                 445

Met Val Ser Gly Glu Tyr Leu Asp Pro Arg Pro Leu Glu Glu Ser Leu
    450                 455                 460

Arg Ala Ser Pro Leu Ile Ala Asn Ala Cys Ile Val Gly Asp Ala Phe
465                 470                 475                 480

Leu Ser Ser Ala Ser Thr Ser Ile Leu Ala Ile Ile Glu Leu Ala Thr
                485                 490                 495

Pro Asp Leu Ala His Thr Ala Ser Ile Arg Ala Gln Leu Ala Arg Val
            500                 505                 510

Leu Ala Pro Leu Asn Arg Asp Leu Pro Pro Pro Leu Arg Ile Ala Ala
        515                 520                 525

Ser Ser Ile Leu Val Leu Asp Gly Met Arg Lys Ile Pro Lys Thr Lys
    530                 535                 540

Lys Gly Asp Ile Phe Arg Lys Lys Leu Glu Asp Thr Phe Gly Ala Glu
545                 550                 555                 560

Phe Glu Gln Met Leu Arg Thr Glu Lys Val Gly Leu Gly Asp Leu Ala
                565                 570                 575

Asp Val Asp Ala Gly Ile Thr Arg Ile Ile Gly Asn Leu Leu Gly Ile
            580                 585                 590

Ser Asp Asp Glu Leu Leu Ser Thr Met Ser Phe Ala Glu Leu Gly Met
        595                 600                 605

Thr Ser Leu Leu Ala Val Lys Ile Ala Ser Glu Leu Asn Lys Phe Leu
    610                 615                 620

Asp Gly Arg Ala Val Leu Pro Thr Asn Ile Cys Tyr Ile His Phe Asp
625                 630                 635                 640

Val Pro Ser Leu Thr Ser Ser Val Arg Glu Arg Leu Ser Ser Ala Pro
                645                 650                 655

Ser Ser Ile Thr Ser Ala Ala Ser Glu Pro Ala Ala Ser Ser Pro Gly
            660                 665                 670

Thr Pro Arg Ala Asp Glu Ile Val Ile Val Gly Lys Ala Phe Arg Leu
        675                 680                 685

Pro Asp Gly Val Asn Asp Asp Ala Ala Leu Trp Asp Val Leu Thr Gly
    690                 695                 700

Glu Ser Ala Ser Ile Ile Lys Asp Ile Pro Ala Asp Arg Trp Asp His
705                 710                 715                 720

Ala Ser Phe Tyr Pro Lys Asp Ile His Phe Gly Arg Ala Gly Leu Val
                725                 730                 735

Asp Val Ala Arg Phe Asp Tyr Gly Phe Phe Gly Met Thr Ala Ser Glu
            740                 745                 750

Ala Tyr Ser Leu Ser Pro Thr Met Arg Leu Ala Leu Glu Val Ala Tyr
        755                 760                 765

Glu Ala Leu Glu Asp Ala Asn Ile Pro Phe Arg Ala Val Lys Gly Ser
    770                 775                 780

Arg Met Gly Val Phe Val Ala Val Lys Asp Asp Gly Phe Glu Thr Leu
785                 790                 795                 800

Leu His Ala Gly Gln Gly Tyr Asp Ala Tyr Thr Arg Phe Tyr Gly Thr
                805                 810                 815

Gly Arg Ala Pro Ser Thr Ala Ser Gly Arg Ile Asn Tyr Leu Leu Asp
            820                 825                 830

Leu His Gly Pro Ser Ile Thr Val Asp Thr Ala Cys Ser Gly Gly Ile
        835                 840                 845

Val Cys Ile Asp Gln Ala Val Thr Tyr Leu Gln Ser Gly Ala Ala Glu
    850                 855                 860

Thr Ala Ile Val Cys Ser Ser Asn Thr His Cys Trp Pro Gly Ser Phe
865                 870                 875                 880

Met Phe Leu Thr Ala Gln Gly Met Ala Ser Pro Asn Gly Arg Cys Ala
                885                 890                 895

Ser Phe Thr Ser Asp Ala Asp Gly Tyr Ala Pro Ser Glu Gly Ala Val
            900                 905                 910

Gly Phe Val Leu Lys Thr Arg Ser Ala Ala Val Arg Asp Gly Asp Arg
        915                 920                 925

Ile Leu Ala Thr Ile Arg Ala Thr Glu Ile Gly His Asn Gly Arg Ser
    930                 935                 940

Gln Gly Leu Ala Ala Pro Asn Val Arg Ser Gln Ala Ala Ala His Arg
945                 950                 955                 960

Ala Val Leu Arg Arg Ala Arg Leu Asp Pro Ser Glu Ile Asp Phe Ile
                965                 970                 975

Glu Ala His Gly Thr Gly Thr Thr Leu Gly Asp Leu Cys Glu Val Gln
            980                 985                 990

Gly Ile Asn Asp Ser Phe Val Ser  Pro Lys Lys Arg Ala  Asn Pro Leu
        995                 1000                1005

Val Val  Ser Ala Ser Lys Ser  Thr Ile Gly His Thr  Glu Pro Ser
    1010                1015                1020

Ala Gly  Leu Val Gly Ile Leu  Ser Ala Leu Met Ser  Phe Glu Lys
    1025                1030                1035

Arg Ile  Val Pro Arg Leu Ala  Tyr Leu Thr Glu Ser  Asn Val Asn
    1040                1045                1050

Pro Ala  Leu Asp Ala Ser Val  Val Pro Leu His Phe  Pro Thr Lys
    1055                1060                1065

His Ile  Glu Leu Arg Ala Asp  Val Pro Tyr Lys Ala  Val Val Met
    1070                1075                1080

Ser Tyr  Gly Phe Ala Gly Thr  Leu Ala Asp Ile Val  Leu Glu Ser
    1085                1090                1095

Glu Val  Pro Gln Pro Thr Pro  Ala Val Ala Gln Asp  Thr Ala Gly
    1100                1105                1110

Gln Gln  Pro Met Leu Phe Val  Leu Ser Ala Lys Thr  Pro Arg Ala
    1115                1120                1125

Leu Ala  Ala Tyr Ile Glu Leu  Tyr Leu Gly Phe Leu  Arg His Ala
    1130                1135                1140

Asp Pro  Gly Leu Phe Ala Arg  Ile Cys Tyr Thr Ala  Cys Val Ala
    1145                1150                1155

Arg Glu  His Tyr Lys His Arg  Val Ala Cys Val Ala  Thr Asp Leu
    1160                1165                1170

Val Asp Leu Ile Ala Gln Leu Glu Thr Arg Leu Val Gln Thr Ala
    1175                1180                1185

Tyr Ala Gly Gly Gly Gly Ala Arg Ala Ala Arg Thr Gly Pro Leu
    1190                1195                1200

Val Phe Ala Phe Ser Gly Gln Gly Thr Gln Phe Pro Ala Met Ala
    1205                1210                1215

Ala Pro Leu Ala Arg Arg Tyr Ala Arg Phe Gly Glu Ile Val Gly
    1220                1225                1230

Gly Cys Ala Arg Met Ala Arg Glu Leu Ser Gly Phe Pro Val Asp
    1235                1240                1245

Gly Ile Leu Leu Gly Asp Asp Val Thr Pro Val Lys Asp Asn Ser
    1250                1255                1260

Ala Ala Ala Glu Val His Ser Glu Val Asp Gln Ile Cys Ile Phe
    1265                1270                1275

Val Tyr Gln Tyr Ala Met Cys Arg Trp Leu Gly Glu Leu Gly Val
    1280                1285                1290

Glu Pro Lys Ala Ala Ile Gly His Ser Leu Gly Glu Ile Thr Ala
    1295                1300                1305

Ala Val Ile Ala Gly Ala Leu Pro Phe Glu Ala Ala Leu Asp Leu
    1310                1315                1320

Val Val Thr Arg Ala Arg Leu Leu Lys Pro Cys Ala Glu Gln Pro
    1325                1330                1335

Ser Gly Met Ala Ala Leu Ala Cys Thr Pro Asp Val Ala Ser Lys
    1340                1345                1350

Leu Thr Leu Gly Ala Ser Val Ser Val Ser Val Tyr Asn Gly Pro
    1355                1360                1365

Gln Ser Ile Cys Leu Ser Gly Ala Ser Ala Glu Leu Asp Asp Ala
    1370                1375                1380

Val Arg Ala Ala Lys Ala Arg Asn Ile Lys Ala Thr Arg Leu Gln
    1385                1390                1395

Val Asp Gln Gly Phe His Ser Pro Cys Val Asp Ala Ala Val Pro
    1400                1405                1410

Gly Leu Gln Ala Trp Cys Ala Ala His Arg Ala Ser Ala Ala Pro
    1415                1420                1425

Leu Lys Met Pro Leu Tyr Ser Thr Val Arg Gly Asp Val Val Pro
    1430                1435                1440

Lys Gly Ala Ala Leu Asp Pro Glu His Trp Val Ala His Ala Arg
    1445                1450                1455

Asn Pro Val Leu Phe Ala Gln Thr Ala Gln Ala Leu Lys Glu Ser
    1460                1465                1470

Leu Pro His Gly Ile Thr Leu Asp Val Gly Pro Gln Ala Val Ala
    1475                1480                1485

Trp Ser Leu Leu Leu Leu Asn Gly Leu Ser Ala Thr Arg Thr Val
    1490                1495                1500

Ala Ala Gly Ala Lys Lys Gly Ala Asp Gln Glu Arg Ala Leu Leu
    1505                1510                1515

Gly Ala Leu Gly Ala Leu Phe Glu Gln His Lys Val Thr Pro Asp
    1520                1525                1530

Phe Gly Arg Leu Tyr Ala Pro Leu Glu Lys Thr Arg Ile Pro Thr
    1535                1540                1545

Tyr Pro Phe Glu Arg Ala Arg Cys Tyr Pro Thr Phe Ile Pro Ser
    1550                1555                1560

Arg Phe Ala His Gly Ala Ala Ala Ala Ala Lys Thr Asp Gly Glu
    1565                1570                1575

Val Leu Ser Ala Glu Glu Glu Asn Val Ala Pro Val Gly Leu Leu
    1580                1585                1590

Thr Lys Glu Asp Leu Arg Ala Ala Leu Val Ala Cys Leu Arg Ala
    1595                1600                1605

Thr Leu Glu Leu Arg Pro Asp Glu Glu Leu Asp Glu Ala Glu Pro
    1610                1615                1620

Leu Thr Val His Gly Val Asp Ser Ile Gly Phe Ala Lys Leu Arg
    1625                1630                1635

Lys His Val Glu Asp Arg Trp Gly Leu Asp Ile Pro Val Val Tyr
    1640                1645                1650

Trp Ser Asp Ala Phe Thr Val Gly Glu Met Leu Gly Asn Leu Val
    1655                1660                1665

Gly Gln Tyr Asp Val Val Ser Thr Ala Ala
    1670                1675

<210> 46
<211> 4956
<212> DNA
<213> Mycena chlorophos

<400> 46
atgaatccgc cctcgtctat cctcgaagtc ttccagcgta ccgccctcga cccctcgggc 60

gccgaccggc gcgttctgga atgcgggccc gacttttgga cctacgcggg tctcgacgcg 120

gtttccacag gccttgcggc ggacttggct gctctcggag attctcccat cgtggctgtc 180

gtcgctgaga accatccctt cgtcttcgca ctcatgtttg ccgtctggaa attgcacggg 240

acgtttgttc ccatcgacgc gcatattccg tggaacctgc tcgacggcat gttggacatc 300

gtcaagccga cttgcatgtt tctcgtcgag tcggatacca acaatatctc gaacaccaag 360

gcccagggag tcgacttcgc tgtgcgcctc ttcggaggag aaggattcac catcccggcg 420

ctctcggcca aatatgcggg gaacgtctcg aatggcgccc ccgagtcact cccttctcca 480

gacgcgactg ctctgtatct gttcacgtcg tctgcctcgt cgcggcacaa tttgaaggcc 540

gttccgctca cgcatcgatt cattgccgct ggctgcgaag cgaaactcgc cttctggcac 600

cgtcttcatc ctcacaaccc caccgatgcg attcgcgtgc tgggatgggc tccgttgtcg 660

cacgtcctcg cgcatatgca ggatatcggc accgcggccc tcctcaacgc cggctgctat 720

gtcttttcga cgatcccctt gtcttacacc tcagcagaaa ctcagcccgc gcaagatatc 780

acctcggctc tcattcactc cgtgctccat tacgaggtca aagcatttgc gggcctgcct 840

tttgttattg cggcattcaa ggctgcttgt gaaggcggga acgaccgtct cctagcgcaa 900

ctacgctcca tgaccatgct cgagtgtggc ggggcgcagt tggacaagga catcgtggat 960

tgggcagtca agcaagcgat cccgctcgtg gttggggtcg ggatgacgga aacgggtggc 1020

gcgatactgg cgggccccgt cggggatgcg tcggatgggt ttcaccccca agggctgctg 1080

ctagatgcac agttctccct tatcggcaat gacgatgaat cggaaggcga gctggtcatc 1140

aagagtccca atcttccgcg cggatatctg aagtacgagg acggctcgtt cgacatcgac 1200

gcgcagggtg tcgtcacgtt caagactgga gacatctacc gcaaaagtgt cgagggtaaa 1260

ctcctttggg ttgggcgtag tacggatttc atccagatgg ctaccggcga gacgctcgat 1320

ccccgtcgtg ttgaacgggc gctacgcttc gcatcgggga tcaacgatgc ctgcatcatc 1380

gggaatgcgt tcctgaatgg ctcctctacc gcaatttacg ccatcatcga gctcgctccg 1440

cgcaccgtca acatcaataa tgattcaaat gtctcccatc tgcaggtggt tgcccgcgcc 1500

ctgtctccta tcaaccgcga ccttcctccc gcattacgca ttgtgttgtc ttctgtattg 1560

attctggctg aaggcatgaa gattccgagg acaaagaaag gcgaaatctt ccggaagaag 1620

attgatgaag ttttcggagc tgctctccgg gctttaggtc actcggcaac tccaacagag 1680

gttgttcttg agcaggaacc agcggcagcc agcaaaccca tttttgacaa gaacaagctg 1740

cagactgcta ttgcgcatag cttgggtctg gatattctgg agattgacct gctggacaag 1800

ctgacctttg ctgagcttgg catgacatct attcttgcaa ttcgggttgc agaagatctc 1860

aacaaattgc tgcaggacaa agttaccctg ccagtcaata tctgctacct ctatccagat 1920

gcccagctgt tgtttgcagc agttcaggaa cagctgctca agcagcagca cccttcaacc 1980

ccaactgctc cctccgtgcc ggctttgctg tcagcaactt cctctgttcc aattctcttg 2040

caggaactag atgatgttgt cattgttggc aaatcattcc ggttgcctgg cggaatctac 2100

gatgatcgag cactctgggc agctctcacc aatcaagcta cccgaaaccc catctcatat 2160

atttcgggcc agcgctggga ccatacaagc ttttacccag ctgatattgc attcttgcag 2220

gcagggttgc ttgactccga ccactttacg gattttgatg cagctttctt cgggatgacc 2280

gagaaagagg catactatct gtccccgacc atgcggcttg ctcttgaagt agcctttgag 2340

gctctagaag atgcaaatat tcctgttggt caggtgaaag gcactagcat gggagtatat 2400

gcagctgtca aggatgatgg attcgaaacc cttttgaatg ctgctcatgg gtatgatgcc 2460

tacacacgat tctacggaac tggacgggca ccaagtacca ctagcgggcg gatcagtcaa 2520

ggagaatcag cgattgtctg ctccagcaat acacactgtt ggcctggctc cttcatgttc 2580

ttgactgccc aaggaatggt gtctcctcat ggacgttgtg cctccttcag tgctcaagca 2640

gatggatatg ttccttcaga gggtgctgtt gcatttatcc tgaagacccg caaagcagca 2700

gttcgggatg gaaaccaaat ttttgccaca attcgggctg cggtggtatc acacaatggt 2760

cgatcacaag gtcttgcagc accaaacatt caagcccaat ccgagttgca tcaacaagca 2820

ttgcagaagg caaatatcca acccactgat attcattttg tggaaactca tggaacaggg 2880

acttcgcttg ggatgtctgt tgagattcat gggataaatg ctgcttttgc agcaggtcac 2940

cgtccctctg gacctctcat cattagtgca agcaaaggca ctattggaca tacagagcct 3000

tctgcaggtc ttgtgggcat catggcggca ctgctctcct tcaagcatgg ccttgttcct 3060

gggctgatcc atacatctca tgggcaactc aacccggcac ttgatcaatc caaagttccg 3120

cttatcttca gcccacaaac aatttccctg ggcggagaaa agccttacag atctgtggtc 3180

atgtcatatg gctttgcagg cacactagcg gatattgtgc ttgaaggccc tgctgaggag 3240

gctttttccg ggccaggcaa aaacagcagt gctcctccgc ctatgatctt tgccctcagt 3300

gccaaatctg catcagccct ccaagaatac aagcagaagt acatcacctt cctgcagaat 3360

gttggctctg gaggccaact gttcagcaag atctgtttga cttcttgcat tgcccgagag 3420

cactacaagc atagattctc ctgtgctgct cagaacacac tggatcttct tctgcagcta 3480

gagcactctg ttgctgccag ccacaaacct ccaacaactc gtaccggaac agtcaccttt 3540

gctttctctg gacagggagc ccaattcccc agcatggatg cagctctggc tcaaggctac 3600

tctgccttca aatccatctt gctggaactt ggaaacaagg ctgccaaact ctctggattc 3660

cccatcactg attgcctgtt ggcaacaaca gcatcagctg atgaagaagc cgtccatagt 3720

gaggtggacc aaatttgcat ttttgttcat caatatgcaa tggctctttt cctcgagatg 3780

ctaggaattg tccccggtgc tgccataggc cacagcttgg gagagatcac agcagcggtc 3840

gttgctggtg gactttcgtt tgaacttggc ctagagttgg tcatcctccg tgcacatctg 3900

ctccgtccag agcagaacaa gcccgctggc atggctgcct tggcatgctc agaagcggac 3960

ttcctcaagt ttccgtccac cgatgcaact atttctgttt tcaactctcc tcggagcatt 4020

gcagtctctg gagcagcaag ctccattgag acagttctta ctgctgccaa agagcagaat 4080

atcaaggcca cgaagctcag ggttgatcaa ggattccata gcagctatgt ggagcatgcg 4140

cttcccgggc tcaagcactg gtcagcaatg aattcaggct ccttccaggc actcaggatt 4200

ccactctatt caactgcact tggccatgtt gttcctgctg gagagaccct tcagccagat 4260

cactggatga accatacccg caatgctgtt cattttacgc aaactgcgca ggctctgaaa 4320

gagtcccttc cgcatgggat cactttggat cttggtcctc aggctgtagc tcaaactctc 4380

ctgctggcca atgaccatcc tgttggccgc accattggat tgtgtggcaa acgcacagga 4440

gatcaaagac atgcattcct gctcgctctt gctgagcttt accagcagca tggtcttgtg 4500

cccaactttc atgcacttta tggcgtagct gcccaggatc tcaaggacca tctcaccagc 4560

ctgccaacat atccattcca acgtgtccgc tgctatccca gctacattcc atcccgtcac 4620

tccaacactc ccgggaccac cgtggtgatt gatgcaaagc cgcgggatga agtgaaacct 4680

gtggcagagg tctcaaagtc ggacacggat tcttccacat cattttcttc gaccattctc 4740

ttccacattc gctccatcct tgagcttcgc ccccatgagg ttctggatac gtctgaatcc 4800

ctcttgacgt acggggtcga ctcgattggg tttgcagcac tgcagaaggc cctggagcag 4860

cagcatgggc taaacttgtc gattgtgttc tggagcgacg tgttttccat tgccgacatt 4920

gtgaagaatc ttgaggagca gaagagcttg aagatg 4956

<210> 47
<211> 1652
<212> PRT
<213> Mycena chlorophos

<400> 47

Met Asn Pro Pro Ser Ser Ile Leu Glu Val Phe Gln Arg Thr Ala Leu
1               5                   10                  15

Asp Pro Ser Gly Ala Asp Arg Arg Val Leu Glu Cys Gly Pro Asp Phe
            20                  25                  30

Trp Thr Tyr Ala Gly Leu Asp Ala Val Ser Thr Gly Leu Ala Ala Asp
        35                  40                  45

Leu Ala Ala Leu Gly Asp Ser Pro Ile Val Ala Val Val Ala Glu Asn
    50                  55                  60

His Pro Phe Val Phe Ala Leu Met Phe Ala Val Trp Lys Leu His Gly
65                  70                  75                  80

Thr Phe Val Pro Ile Asp Ala His Ile Pro Trp Asn Leu Leu Asp Gly
                85                  90                  95

Met Leu Asp Ile Val Lys Pro Thr Cys Met Phe Leu Val Glu Ser Asp
            100                 105                 110

Thr Asn Asn Ile Ser Asn Thr Lys Ala Gln Gly Val Asp Phe Ala Val
        115                 120                 125

Arg Leu Phe Gly Gly Glu Gly Phe Thr Ile Pro Ala Leu Ser Ala Lys
    130                 135                 140

Tyr Ala Gly Asn Val Ser Asn Gly Ala Pro Glu Ser Leu Pro Ser Pro
145                 150                 155                 160

Asp Ala Thr Ala Leu Tyr Leu Phe Thr Ser Ser Ala Ser Ser Arg His
                165                 170                 175

Asn Leu Lys Ala Val Pro Leu Thr His Arg Phe Ile Ala Ala Gly Cys
            180                 185                 190

Glu Ala Lys Leu Ala Phe Trp His Arg Leu His Pro His Asn Pro Thr
        195                 200                 205

Asp Ala Ile Arg Val Leu Gly Trp Ala Pro Leu Ser His Val Leu Ala
    210                 215                 220

His Met Gln Asp Ile Gly Thr Ala Ala Leu Leu Asn Ala Gly Cys Tyr
225                 230                 235                 240

Val Phe Ser Thr Ile Pro Leu Ser Tyr Thr Ser Ala Glu Thr Gln Pro
                245                 250                 255

Ala Gln Asp Ile Thr Ser Ala Leu Ile His Ser Val Leu His Tyr Glu
            260                 265                 270

Val Lys Ala Phe Ala Gly Leu Pro Phe Val Ile Ala Ala Phe Lys Ala
        275                 280                 285

Ala Cys Glu Gly Gly Asn Asp Arg Leu Leu Ala Gln Leu Arg Ser Met
    290                 295                 300

Thr Met Leu Glu Cys Gly Gly Ala Gln Leu Asp Lys Asp Ile Val Asp
305                 310                 315                 320

Trp Ala Val Lys Gln Ala Ile Pro Leu Val Val Gly Val Gly Met Thr
                325                 330                 335

Glu Thr Gly Gly Ala Ile Leu Ala Gly Pro Val Gly Asp Ala Ser Asp
            340                 345                 350

Gly Phe His Pro Gln Gly Leu Leu Leu Asp Ala Gln Phe Ser Leu Ile
        355                 360                 365

Gly Asn Asp Asp Glu Ser Glu Gly Glu Leu Val Ile Lys Ser Pro Asn
    370                 375                 380

Leu Pro Arg Gly Tyr Leu Lys Tyr Glu Asp Gly Ser Phe Asp Ile Asp
385                 390                 395                 400

Ala Gln Gly Val Val Thr Phe Lys Thr Gly Asp Ile Tyr Arg Lys Ser
                405                 410                 415

Val Glu Gly Lys Leu Leu Trp Val Gly Arg Ser Thr Asp Phe Ile Gln
            420                 425                 430

Met Ala Thr Gly Glu Thr Leu Asp Pro Arg Arg Val Glu Arg Ala Leu
        435                 440                 445

Arg Phe Ala Ser Gly Ile Asn Asp Ala Cys Ile Ile Gly Asn Ala Phe
    450                 455                 460

Leu Asn Gly Ser Ser Thr Ala Ile Tyr Ala Ile Ile Glu Leu Ala Pro
465                 470                 475                 480

Arg Thr Val Asn Ile Asn Asn Asp Ser Asn Val Ser His Leu Gln Val
                485                 490                 495

Val Ala Arg Ala Leu Ser Pro Ile Asn Arg Asp Leu Pro Pro Ala Leu
            500                 505                 510

Arg Ile Val Leu Ser Ser Val Leu Ile Leu Ala Glu Gly Met Lys Ile
        515                 520                 525

Pro Arg Thr Lys Lys Gly Glu Ile Phe Arg Lys Lys Ile Asp Glu Val
    530                 535                 540

Phe Gly Ala Ala Leu Arg Ala Leu Gly His Ser Ala Thr Pro Thr Glu
545                 550                 555                 560

Val Val Leu Glu Gln Glu Pro Ala Ala Ala Ser Lys Pro Ile Phe Asp
                565                 570                 575

Lys Asn Lys Leu Gln Thr Ala Ile Ala His Ser Leu Gly Leu Asp Ile
            580                 585                 590

Leu Glu Ile Asp Leu Leu Asp Lys Leu Thr Phe Ala Glu Leu Gly Met
        595                 600                 605

Thr Ser Ile Leu Ala Ile Arg Val Ala Glu Asp Leu Asn Lys Leu Leu
    610                 615                 620

Gln Gly Gln Val Thr Leu Pro Val Asn Ile Cys Tyr Leu Tyr Pro Asp
625                 630                 635                 640

Ala Gln Leu Leu Phe Ala Ala Val Gln Glu Gln Leu Leu Lys Gln Gln
                645                 650                 655

His Pro Ser Thr Pro Thr Ala Pro Ser Val Pro Ala Leu Leu Ser Ala
            660                 665                 670

Thr Ser Ser Val Pro Ile Leu Leu Gln Glu Leu Asp Asp Val Val Ile
        675                 680                 685

Val Gly Lys Ser Phe Arg Leu Pro Gly Gly Ile Tyr Asp Asp Arg Ala
    690                 695                 700

Leu Trp Ala Ala Leu Thr Asn Gln Ala Thr Arg Asn Pro Ile Ser Tyr
705                 710                 715                 720

Ile Ser Gly Gln Arg Trp Asp His Thr Ser Phe Tyr Pro Ala Asp Ile
                725                 730                 735

Ala Phe Leu Gln Ala Gly Leu Leu Asp Ser Asp His Phe Thr Asp Phe
            740                 745                 750

Asp Ala Ala Phe Phe Gly Met Thr Glu Lys Glu Ala Tyr Tyr Leu Ser
        755                 760                 765

Pro Thr Met Arg Leu Ala Leu Glu Val Ala Phe Glu Ala Leu Glu Asp
    770                 775                 780

Ala Asn Ile Pro Val Gly Gln Val Lys Gly Thr Ser Met Gly Val Tyr
785                 790                 795                 800

Ala Ala Val Lys Asp Asp Gly Phe Glu Thr Leu Leu Asn Ala Ala His
                805                 810                 815

Gly Tyr Asp Ala Tyr Thr Arg Phe Tyr Gly Thr Gly Arg Ala Pro Ser
            820                 825                 830

Thr Thr Ser Gly Arg Ile Ser Gln Gly Glu Ser Ala Ile Val Cys Ser
        835                 840                 845

Ser Asn Thr His Cys Trp Pro Gly Ser Phe Met Phe Leu Thr Ala Gln
    850                 855                 860

Gly Met Val Ser Pro His Gly Arg Cys Ala Ser Phe Ser Ala Gln Ala
865                 870                 875                 880

Asp Gly Tyr Val Pro Ser Glu Gly Ala Val Ala Phe Ile Leu Lys Thr
                885                 890                 895

Arg Lys Ala Ala Val Arg Asp Gly Asn Gln Ile Phe Ala Thr Ile Arg
            900                 905                 910

Ala Ala Val Val Ser His Asn Gly Arg Ser Gln Gly Leu Ala Ala Pro
        915                 920                 925

Asn Ile Gln Ala Gln Ser Glu Leu His Gln Gln Ala Leu Gln Lys Ala
    930                 935                 940

Asn Ile Gln Pro Thr Asp Ile His Phe Val Glu Thr His Gly Thr Gly
945                 950                 955                 960

Thr Ser Leu Gly Asp Val Cys Glu Ile His Gly Ile Asn Ala Ala Phe
                965                 970                 975

Ala Ala Gly His Arg Pro Ser Gly Pro Leu Ile Ile Ser Ala Ser Lys
            980                 985                 990

Gly Thr Ile Gly His Thr Glu Pro  Ser Ala Gly Leu Val  Gly Ile Met
        995                 1000                1005

Ala Ala  Leu Leu Ser Phe Lys  His Gly Leu Val Pro  Gly Leu Ile
    1010                 1015                 1020

His Thr  Ser His Gly Gln Leu  Asn Pro Ala Leu Asp  Gln Ser Lys
    1025                 1030                 1035

Val Pro  Leu Ile Phe Ser Pro  Gln Thr Ile Ser Leu  Gly Gly Glu
    1040                 1045                 1050

Lys Pro  Tyr Arg Ser Val Val  Met Ser Tyr Gly Phe  Ala Gly Thr
    1055                 1060                 1065

Leu Ala  Asp Ile Val Leu Glu  Gly Pro Ala Glu Glu  Ala Phe Ser
    1070                 1075                 1080

Gly Pro  Gly Lys Asn Ser Ser  Ala Pro Pro Pro Met  Ile Phe Ala
    1085                 1090                 1095

Leu Ser Ala Lys Ser Ala Ser Ala Leu Gln Glu Tyr Lys Gln Lys
    1100                1105                1110

Tyr Ile Thr Phe Leu Gln Asn Val Gly Ser Gly Gly Gln Leu Phe
    1115                1120                1125

Ser Lys Ile Cys Leu Thr Ser Cys Ile Ala Arg Glu His Tyr Lys
    1130                1135                1140

His Arg Phe Ser Cys Ala Ala Gln Asn Thr Leu Asp Leu Leu Leu
    1145                1150                1155

Gln Leu Glu His Ser Val Ala Ala Ser His Lys Pro Pro Thr Thr
    1160                1165                1170

Arg Thr Gly Thr Val Thr Phe Ala Phe Ser Gly Gln Gly Ala Gln
    1175                1180                1185

Phe Pro Ser Met Asp Ala Ala Leu Ala Gln Gly Tyr Ser Ala Phe
    1190                1195                1200

Lys Ser Ile Leu Leu Glu Leu Gly Asn Lys Ala Ala Lys Leu Ser
    1205                1210                1215

Gly Phe Pro Ile Thr Asp Cys Leu Leu Ala Thr Thr Ala Ser Ala
    1220                1225                1230

Asp Glu Glu Ala Val His Ser Glu Val Asp Gln Ile Cys Ile Phe
    1235                1240                1245

Val His Gln Tyr Ala Met Ala Leu Phe Leu Glu Met Leu Gly Ile
    1250                1255                1260

Val Pro Gly Ala Ala Ile Gly His Ser Leu Gly Glu Ile Thr Ala
    1265                1270                1275

Ala Val Val Ala Gly Gly Leu Ser Phe Glu Leu Gly Leu Glu Leu
    1280                1285                1290

Val Ile Leu Arg Ala His Leu Leu Arg Pro Glu Gln Asn Lys Pro
    1295                1300                1305

Ala Gly Met Ala Ala Leu Ala Cys Ser Glu Ala Asp Phe Leu Lys
    1310                1315                1320

Phe Pro Ser Thr Asp Ala Thr Ile Ser Val Phe Asn Ser Pro Arg
    1325                1330                1335

Ser Ile Ala Val Ser Gly Ala Ala Ser Ser Ile Glu Thr Val Leu
    1340                1345                1350

Thr Ala Ala Lys Glu Gln Asn Ile Lys Ala Thr Lys Leu Arg Val
    1355                1360                1365

Asp Gln Gly Phe His Ser Ser Tyr Val Glu His Ala Leu Pro Gly
    1370                1375                1380

Leu Lys His Trp Ser Ala Met Asn Ser Gly Ser Phe Gln Ala Leu
    1385                1390                1395

Arg Ile Pro Leu Tyr Ser Thr Ala Leu Gly His Val Val Pro Ala
    1400                1405                1410

Gly Glu Thr Leu Gln Pro Asp His Trp Met Asn His Thr Arg Asn
    1415                1420                1425

Ala Val His Phe Thr Gln Thr Ala Gln Ala Leu Lys Glu Ser Leu
    1430                1435                1440

Pro His Gly Ile Thr Leu Asp Leu Gly Pro Gln Ala Val Ala Gln
    1445                1450                1455

Thr Leu Leu Leu Ala Asn Asp His Pro Val Gly Arg Thr Ile Gly
    1460                1465                1470

Leu Cys Gly Lys Arg Thr Gly Asp Gln Arg His Ala Phe Leu Leu
    1475                1480                1485

Ala Leu Ala Glu Leu Tyr Gln Gln His Gly Leu Val Pro Asn Phe
    1490                1495                1500

His Ala Leu Tyr Gly Val Ala Ala Gln Asp Leu Lys Asp His Leu
    1505                1510                1515

Thr Ser Leu Pro Thr Tyr Pro Phe Gln Arg Val Arg Cys Tyr Pro
    1520                1525                1530

Ser Tyr Ile Pro Ser Arg His Ser Asn Thr Pro Gly Thr Thr Val
    1535                1540                1545

Val Ile Asp Ala Lys Pro Arg Asp Glu Val Lys Pro Val Ala Glu
    1550                1555                1560

Val Ser Lys Ser Asp Thr Asp Ser Ser Thr Ser Phe Ser Ser Thr
    1565                1570                1575

Ile Leu Phe His Ile Arg Ser Ile Leu Glu Leu Arg Pro His Glu
    1580                1585                1590

Val Leu Asp Thr Ser Glu Ser Leu Leu Thr Tyr Gly Val Asp Ser
    1595                1600                1605

Ile Gly Phe Ala Ala Leu Gln Lys Ala Leu Glu Gln Gln His Gly
    1610                1615                1620

Leu Asn Leu Ser Ile Val Phe Trp Ser Asp Val Phe Ser Ile Ala
    1625                1630                1635

Asp Ile Val Lys Asn Leu Glu Glu Gln Lys Ser Leu Lys Met
    1640                1645                1650

<210> 48
<211> 5313
<212> DNA
<213> Armillaria gallica

<400> 48
atggaggccg acggtcacca ctctcttctc gatgtctttc tcagcgttgc acatgattct 60

gagaagtcca aacgtaatgt cttggaatgc ggccaggata cctggacata ctcagatttg 120

gacatcatct cgtcggcctt ggcacaggat ctcaaagcta ccttgggttg ttttcccaaa 180

gttgcagtcg tcagcgagaa ccatccctac gtgtttgctc tcatgctggc cgtttggaag 240

cttgaaggga tattcatccc catcgacgtc catattacag ctgaccttct aaagggcatg 300

ctacgcattg ttgcccccac ttgtctggcg atcccagaga ccgatatttc caaccagcgt 360

gttgcctctg cgattggtat acatgttctc cccttcaacg tgaatgcgtc gaccatgaat 420

gcacttcgac agaaatacga cccatttact cagaatgcct cgctatctgg atgcgcactt 480

ccttacgttg atcgcgcatg cctctatctc tttacatcct ccgcgtcctc tactgccaac 540

ttgaaatgtg tacctttgac ccatactctt atcctcagta actgccgttc caagctcgca 600

tggtggcggc gcgttcgtcc ggaaggcgaa atggatggga tacgtgttct agggtgggca 660

ccttggtcgc atatccttgc ctacatgcaa gatattggca cggcaacgtt cctgaatgcc 720

ggctgctatg tctttgcgtc cgttccatcc acatatcctt cacagctggc agcgaatggc 780

ctacaaggcc ccaccatgaa tatcatcgat tcacttcttg aacggcgagt cgccgcattt 840

gcttgcgtac cgttcatttt gagcgaacta aaagctatgt gcgagacggc tgccagtcca 900

gatgacaagc atctcatgtg cttgagagct gaggagaaag ttcgccttgt cagtgcgctg 960

cagcggctta tgatgctcga gtgcggaggc gctgcgctcg agtcggatgt cacacgttgg 1020

gccgtcgaaa atggcatatc ggtcatggtc ggcatcggga tgacggagac agtcggtacg 1080

ctgtttgcag agcgcgcgca agacgcctgt tccaatggct attctgcgca ggacgccctc 1140

attgctgatg gcatcatgtc actggtcggg tctgacaacg aggaagccac cttcgaaggg 1200

gaactagtcg tgaagagcaa gctcatccca cacggataca tcaactaccg tgattcgtcg 1260

ttttcggtgg actcggacgg ctgggtaacg ttcaaaacgg gagacaaata tcagcgcacg 1320

ccagatggac gattcaaatg gcttggaaga aagaccgatt ttattcagat gacaagcagc 1380

gaaacattgg atcccagacc cattgagcaa gccctctgtg cgaatccaag tatcgcaaat 1440

gcatgcgtca ttggtgacag gttcctgaga gagcctgcga ctagcgtatg cgccattgtc 1500

gagatcgggc ctgaagtgga catgccttcg tccaagatcg acagagaaat tgcgaatgcc 1560

ctcgctccaa tcaatcgcga ccttcctcct gctcttcgca tatcatggtc tcgcgtactc 1620

ataattcgac ctcctcagaa aataccagtt acgaggaaag gcgatgtgtt ccgtaagaag 1680

attgaagata tgtttggccc tttccttggt gtcggcgttt ctaccgaggt cgaagccggc 1740

catgaaacta aagaagatga cacggaacac atcgtgagac aggttgtgag caatcttctt 1800

ggcgtccatg atcctgagct attgtctgct ttatctttcg ctgagctggg catgacctca 1860

tttatggctg ttagcatcgt caacgctctc aacaaacgca tcggcggcct cacccttccg 1920

tctaatgcat gctacatcca tattgatctt gattctcttg tggacgcgat ttcacttgaa 1980

tatggtcatg gaagtatccc tgcagagttg ccttctaacc ctttccccga catcgagtcc 2040

catcagcata atgataagga cattgtgata gtcggcaagg cattccgttt acccggctca 2100

ctcaacagta ctgcaactct ctgggaagct ttgttatcga ataacaattc ggtcatcagt 2160

gatatcccat ctgatcgctg ggatcatgca agcttttacc cccacaatat atgtttcacg 2220

aaagcaggcc tcgtcgatgt tgcacattac gactacagat tttttggcct catggcgaca 2280

gaggcgttgt acgtatctcc gacgatgcgc ctcgccttgg aagtgtcatt tgaagccctg 2340

gagaacgcaa atattccgct atccaagctg aaggggacac aaaccgctgt ctatgtcgcc 2400

actaaagacg atggcttcga gacactctta aatgccgagc aaggttacga tgcgtacacg 2460

cgattctacg gcacgggtcg cgctccgagt accgcaagcg gtcgcataag ctatcttctt 2520

gatattcatg ggccatctat taccgttgat acagcatgca gcggaggcat tgtatgtatg 2580

gatcaagcca tcactttctt gcaatccgga ggggccgata ccgcaattgt ctgttcgacc 2640

aatacgcact gttggccggg atcattcatg ttcctgacgg cacaaggcat ggtttctcca 2700

aatggaagat gcgctacatt cactaccgat gcagacggat atgtgccttc ggagggtgca 2760

gtggctttca ttctcaagac gcgcagcgct gcaatacgcg acaatgacaa tatactcgcc 2820

gtgatcaaat caacagatgt gtcccataac ggccgttctc aagggctagt tgcaccaaac 2880

gtaaaggcgc aggcaaacct acaccggtcg ttgctacgaa aagctgggct gtttcctgat 2940

caaattaact ttatcgaagc tcatggaaca ggtacatctc taggagacct ttcggaaatc 3000

cagggcatta ataacgccta catctcatca cgacctcgtc tggccggtcc ccttatcatt 3060

agtgcatcga aaacagtttt aggacacagt gaaccaacag cagggatggc cggcatcctc 3120

acagccttgc ttgcccttga gaaagagaca gttcctggtt taaatcactt aacggatcac 3180

aacctcaacc cttcgcttga ttgcagcgta gttcctctcc tgattcctca cgagtctatt 3240

cacattggtg gtgcaaagcc acatcgagct gcggttctgt catacggctt cgcgggtacg 3300

ctggccggtg ctatcttaga aggaccacct tctggtgtac catggccgtc gtcaaatgat 3360

atacaagaac accctatgat tttcgtcgtc agtgggaaaa ccgtgcctac actggaagcg 3420

tacctgggac ggtatttgac atttttgcgg gtcgcaaaaa cacacgactt ccaggacatc 3480

tgctacacca cttgcgtcgg gagggagcac tacaaatacc ggttctcctg cgttgcccga 3540

aacatggcag accttatttc tcaaattgaa catcgactga caactctttc cacttcgaaa 3600

cagaagcctc gcggctcgtt agggttcatg ttctcaggac aaggcacttg tttccctggt 3660

atggcttcag cacttgctga acaatattcg gggttccgaa tgctcgtctc taagtttggg 3720

caggctgccc aagagcggtc cggttatccg atcgataggc tgttgcttga agtttctgat 3780

acattaccag aaacaaacag cgaggtcgac caaatttgca tttttgtcta ccaatattct 3840

gttctgcaat ggttgcaatg tctaggcatt caaccgaaag cagtcctcgg tcacagcctg 3900

ggagaaatta ctgccgcagt cgcagctggc gccctttcgt tcgaatctgc gttggatctt 3960

gtggtcaccc gtgctcgtct tctccgtccc agaacaaaag actctgcagg aatggccgca 4020

gtagcagcgt ccaaggaaga agttgaagga gttatagaaa ccctccaact tgcaaactcg 4080

ctaagcgttg cggttcacaa cggtccgcgg agtgttgttg tgtcaggcgc atcagcagaa 4140

atcgatgccc tagttgtcgc agctaaagaa cggggcttga aagcctcccg cttaaaggtt 4200

gaccaaggct tccacagccc ttacgttgac tctgcggttc cgggtttact cgactggtca 4260

aacaagcatc gttcgacctt ctttccttta aacattcctc tatactcgac tttgaccggc 4320

gagcttattc cgaagggacg gaggttcgtc tcggatcact gggtaaacca tgctcgaaaa 4380

cctgttcagt ttgcggcggc agcggcagcg gtggatgaag atcgatccat tggtgtgctc 4440

gttgacgtcg gaccccaacc cgttgcgtgg accctccttc aagcaaacaa ccttctcaat 4500

acctctgcag ttgcgctatc cgcaaaggcc ggaaaggacc aggagatggc gctgctcact 4560

gctttgagct acctcttcca agagcacaac ctttctccca acttccacga gctttactct 4620

cagcgtcatg ggactctgca gaagacggac attcccacct acccattcca acgtgtccac 4680

cgctatccga ccttcatacc gtcacgaaat caaagtcctg ctattgcaac ggtagttata 4740

ccgccacctc gcttctctgt ccaaaaggct gcggatgtag catcacagtc gaaggaatca 4800

gactgtcgag ctggtttgat cagttgcctt agagccatcc tcgaattaac accggaagag 4860

gagtttgacc tttctgagac tctcaacgct cgtggtatgg attcgatcat gtttgcgcag 4920

ctacggaagc gggttgggga agaattcaac ctcgatatac ccatgatcta tctatcagac 4980

gtgttcacga tggaacagat ggtcgactac ctcgtcgaac agtccggatc cacacccgcg 5040

tcaaagcacg tagaaacttc agctaatcaa ccattagacg aagaagatct ccggacgggg 5100

ctcttgtcat gcctgaggaa cgtgctagaa attacccccg atgaagaact tgacctatct 5160

gaaactttga atgctcgtgg tgttgactcg atcatgttcg ctcagctgcg gaaacgcgtt 5220

ggggaaggtt ttggtgtgga aattccgatg atatatctgt ctgacgtgtt taccatggaa 5280

gacatgatca atttcctcgt ctccgagcgg tcg 5313

<210> 49
<211> 1771
<212> PRT
<213> Armillaria gallica

<400> 49

Met Glu Ala Asp Gly His His Ser Leu Leu Asp Val Phe Leu Ser Val
1               5                   10                  15

Ala His Asp Ser Glu Lys Ser Lys Arg Asn Val Leu Glu Cys Gly Gln
            20                  25                  30

Asp Thr Trp Thr Tyr Ser Asp Leu Asp Ile Ile Ser Ser Ala Leu Ala
        35                  40                  45

Gln Asp Leu Lys Ala Thr Leu Gly Cys Phe Pro Lys Val Ala Val Val
    50                  55                  60

Ser Glu Asn His Pro Tyr Val Phe Ala Leu Met Leu Ala Val Trp Lys
65                  70                  75                  80

Leu Glu Gly Ile Phe Ile Pro Ile Asp Val His Ile Thr Ala Asp Leu
                85                  90                  95

Leu Lys Gly Met Leu Arg Ile Val Ala Pro Thr Cys Leu Ala Ile Pro
            100                 105                 110

Glu Thr Asp Ile Ser Asn Gln Arg Val Ala Ser Ala Ile Gly Ile His
        115                 120                 125

Val Leu Pro Phe Asn Val Asn Ala Ser Thr Met Asn Ala Leu Arg Gln
    130                 135                 140

Lys Tyr Asp Pro Phe Thr Gln Asn Ala Ser Leu Ser Gly Cys Ala Leu
145                 150                 155                 160

Pro Tyr Val Asp Arg Ala Cys Leu Tyr Leu Phe Thr Ser Ser Ala Ser
                165                 170                 175

Ser Thr Ala Asn Leu Lys Cys Val Pro Leu Thr His Thr Leu Ile Leu
            180                 185                 190

Ser Asn Cys Arg Ser Lys Leu Ala Trp Trp Arg Arg Val Arg Pro Glu
        195                 200                 205

Gly Glu Met Asp Gly Ile Arg Val Leu Gly Trp Ala Pro Trp Ser His
    210                 215                 220

Ile Leu Ala Tyr Met Gln Asp Ile Gly Thr Ala Thr Phe Leu Asn Ala
225                 230                 235                 240

Gly Cys Tyr Val Phe Ala Ser Val Pro Ser Thr Tyr Pro Ser Gln Leu
                245                 250                 255

Ala Ala Asn Gly Leu Gln Gly Pro Thr Met Asn Ile Ile Asp Ser Leu
            260                 265                 270

Leu Glu Arg Arg Val Ala Ala Phe Ala Cys Val Pro Phe Ile Leu Ser
        275                 280                 285

Glu Leu Lys Ala Met Cys Glu Thr Ala Ala Ser Pro Asp Asp Lys His
    290                 295                 300

Leu Met Cys Leu Arg Ala Glu Glu Lys Val Arg Leu Val Ser Ala Leu
305                 310                 315                 320

Gln Arg Leu Met Met Leu Glu Cys Gly Gly Ala Ala Leu Glu Ser Asp
                325                 330                 335

Val Thr Arg Trp Ala Val Glu Asn Gly Ile Ser Val Met Val Gly Ile
            340                 345                 350

Gly Met Thr Glu Thr Val Gly Thr Leu Phe Ala Glu Arg Ala Gln Asp
        355                 360                 365

Ala Cys Ser Asn Gly Tyr Ser Ala Gln Asp Ala Leu Ile Ala Asp Gly
    370                 375                 380

Ile Met Ser Leu Val Gly Ser Asp Asn Glu Glu Ala Thr Phe Glu Gly
385                 390                 395                 400

Glu Leu Val Val Lys Ser Lys Leu Ile Pro His Gly Tyr Ile Asn Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Gln Ala Leu Cys Ala Asn Pro Ser Ile Ala Asn
465                 470                 475                 480

Ala Cys Val Ile Gly Asp Arg Phe Leu Arg Glu Pro Ala Thr Ser Val
                485                 490                 495

Cys Ala Ile Val Glu Ile Gly Pro Glu Val Asp Met Pro Ser Ser Lys
            500                 505                 510

Ile Asp Arg Glu Ile Ala Asn Ala Leu Ala Pro Ile Asn Arg Asp Leu
        515                 520                 525

Pro Pro Ala Leu Arg Ile Ser Trp Ser Arg Val Leu Ile Ile Arg Pro
    530                 535                 540

Pro Gln Lys Ile Pro Val Thr Arg Lys Gly Asp Val Phe Arg Lys Lys
545                 550                 555                 560

Ile Glu Asp Met Phe Gly Pro Phe Leu Gly Val Gly Val Ser Thr Glu
                565                 570                 575

Val Glu Ala Gly His Glu Thr Lys Glu Asp Asp Thr Glu His Ile Val
            580                 585                 590

Arg Gln Val Val Ser Asn Leu Leu Gly Val His Asp Pro Glu Leu Leu
        595                 600                 605

Ser Ala Leu Ser Phe Ala Glu Leu Gly Met Thr Ser Phe Met Ala Val
    610                 615                 620

Ser Ile Val Asn Ala Leu Asn Lys Arg Ile Gly Gly Leu Thr Leu Pro
625                 630                 635                 640

Ser Asn Ala Cys Tyr Ile His Ile Asp Leu Asp Ser Leu Val Asp Ala
                645                 650                 655

Ile Ser Leu Glu Tyr Gly His Gly Ser Ile Pro Ala Glu Leu Pro Ser
            660                 665                 670

Asn Pro Phe Pro Asp Ile Glu Ser His Gln His Asn Asp Lys Asp Ile
        675                 680                 685

Val Ile Val Gly Lys Ala Phe Arg Leu Pro Gly Ser Leu Asn Ser Thr
    690                 695                 700

Ala Thr Leu Trp Glu Ala Leu Leu Ser Asn Asn Asn Ser Val Ile Ser
705                 710                 715                 720

Asp Ile Pro Ser Asp Arg Trp Asp His Ala Ser Phe Tyr Pro His Asn
                725                 730                 735

Ile Cys Phe Thr Lys Ala Gly Leu Val Asp Val Ala His Tyr Asp Tyr
            740                 745                 750

Arg Phe Phe Gly Leu Met Ala Thr Glu Ala Leu Tyr Val Ser Pro Thr
        755                 760                 765

Met Arg Leu Ala Leu Glu Val Ser Phe Glu Ala Leu Glu Asn Ala Asn
    770                 775                 780

Ile Pro Leu Ser Lys Leu Lys Gly Thr Gln Thr Ala Val Tyr Val Ala
785                 790                 795                 800

Thr Lys Asp Asp Gly Phe Glu Thr Leu Leu Asn Ala Glu Gln Gly Tyr
                805                 810                 815

Asp Ala Tyr Thr Arg Phe Tyr Gly Thr Gly Arg Ala Pro Ser Thr Ala
            820                 825                 830

Ser Gly Arg Ile Ser Tyr Leu Leu Asp Ile His Gly Pro Ser Ile Thr
        835                 840                 845

Val Asp Thr Ala Cys Ser Gly Gly Ile Val Cys Met Asp Gln Ala Ile
    850                 855                 860

Thr Phe Leu Gln Ser Gly Gly Ala Asp Thr Ala Ile Val Cys Ser Thr
865                 870                 875                 880

Asn Thr His Cys Trp Pro Gly Ser Phe Met Phe Leu Thr Ala Gln Gly
                885                 890                 895

Met Val Ser Pro Asn Gly Arg Cys Ala Thr Phe Thr Thr Asp Ala Asp
            900                 905                 910

Gly Tyr Val Pro Ser Glu Gly Ala Val Ala Phe Ile Leu Lys Thr Arg
        915                 920                 925

Ser Ala Ala Ile Arg Asp Asn Asp Asn Ile Leu Ala Val Ile Lys Ser
    930                 935                 940

Thr Asp Val Ser His Asn Gly Arg Ser Gln Gly Leu Val Ala Pro Asn
945                 950                 955                 960

Val Lys Ala Gln Ala Asn Leu His Arg Ser Leu Leu Arg Lys Ala Gly
                965                 970                 975

Leu Phe Pro Asp Gln Ile Asn Phe Ile Glu Ala His Gly Thr Gly Thr
            980                 985                 990

Ser Leu Gly Asp Leu Ser Glu Ile Gln Gly Ile Asn Asn Ala Tyr Ile
        995                 1000                1005

Ser Ser Arg Pro Arg Leu Ala Gly Pro Leu Ile Ile Ser Ala Ser
    1010                1015                1020

Lys Thr Val Leu Gly His Ser Glu Pro Thr Ala Gly Met Ala Gly
    1025                1030                1035

Ile Leu Thr Ala Leu Leu Ala Leu Glu Lys Glu Thr Val Pro Gly
    1040                1045                1050

Leu Asn His Leu Thr Asp His Asn Leu Asn Pro Ser Leu Asp Cys
    1055                1060                1065

Ser Val Val Pro Leu Leu Ile Pro His Glu Ser Ile His Ile Gly
    1070                1075                1080

Gly Ala Lys Pro His Arg Ala Ala Val Leu Ser Tyr Gly Phe Ala
    1085                1090                1095

Gly Thr Leu Ala Gly Ala Ile Leu Glu Gly Pro Pro Ser Gly Val
    1100                1105                1110

Pro Trp Pro Ser Ser Asn Asp Ile Gln Glu His Pro Met Ile Phe
    1115                1120                1125

Val Val Ser Gly Lys Thr Val Pro Thr Leu Glu Ala Tyr Leu Gly
    1130                1135                1140

Arg Tyr Leu Thr Phe Leu Arg Val Ala Lys Thr His Asp Phe Gln
    1145                1150                1155

Asp Ile Cys Tyr Thr Thr Cys Val Gly Arg Glu His Tyr Lys Tyr
    1160                1165                1170

Arg Phe Ser Cys Val Ala Arg Asn Met Ala Asp Leu Ile Ser Gln
    1175                1180                1185

Ile Glu His Arg Leu Thr Thr Leu Ser Thr Ser Lys Gln Lys Pro
    1190                1195                1200

Arg Gly Ser Leu Gly Phe Met Phe Ser Gly Gln Gly Thr Cys Phe
    1205                1210                1215

Pro Gly Met Ala Ser Ala Leu Ala Glu Gln Tyr Ser Gly Phe Arg
    1220                1225                1230

Met Leu Val Ser Lys Phe Gly Gln Ala Ala Gln Glu Arg Ser Gly
    1235                1240                1245

Tyr Pro Ile Asp Arg Leu Leu Leu Glu Val Ser Asp Thr Leu Pro
    1250                1255                1260

Glu Thr Asn Ser Glu Val Asp Gln Ile Cys Ile Phe Val Tyr Gln
    1265                1270                1275

Tyr Ser Val Leu Gln Trp Leu Gln Cys Leu Gly Ile Gln Pro Lys
    1280                1285                1290

Ala Val Leu Gly His Ser Leu Gly Glu Ile Thr Ala Ala Val Ala
    1295                1300                1305

Ala Gly Ala Leu Ser Phe Glu Ser Ala Leu Asp Leu Val Val Thr
    1310                1315                1320

Arg Ala Arg Leu Leu Arg Pro Arg Thr Lys Asp Ser Ala Gly Met
    1325                1330                1335

Ala Ala Val Ala Ala Ser Lys Glu Glu Val Glu Gly Val Ile Glu
    1340                1345                1350

Thr Leu Gln Leu Ala Asn Ser Leu Ser Val Ala Val His Asn Gly
    1355                1360                1365

Pro Arg Ser Val Val Val Ser Gly Ala Ser Ala Glu Ile Asp Ala
    1370                1375                1380

Leu Val Val Ala Ala Lys Glu Arg Gly Leu Lys Ala Ser Arg Leu
    1385                1390                1395

Lys Val Asp Gln Gly Phe His Ser Pro Tyr Val Asp Ser Ala Val
    1400                1405                1410

Pro Gly Leu Leu Asp Trp Ser Asn Lys His Arg Ser Thr Phe Phe
    1415                1420                1425

Pro Leu Asn Ile Pro Leu Tyr Ser Thr Leu Thr Gly Glu Leu Ile
    1430                1435                1440

Pro Lys Gly Arg Arg Phe Val Ser Asp His Trp Val Asn His Ala
    1445                1450                1455

Arg Lys Pro Val Gln Phe Ala Ala Ala Ala Ala Ala Val Asp Glu
    1460                1465                1470

Asp Arg Ser Ile Gly Val Leu Val Asp Val Gly Pro Gln Pro Val
    1475                1480                1485

Ala Trp Thr Leu Leu Gln Ala Asn Asn Leu Leu Asn Thr Ser Ala
    1490                1495                1500

Val Ala Leu Ser Ala Lys Ala Gly Lys Asp Gln Glu Met Ala Leu
    1505                1510                1515

Leu Thr Ala Leu Ser Tyr Leu Phe Gln Glu His Asn Leu Ser Pro
    1520                1525                1530

Asn Phe His Glu Leu Tyr Ser Gln Arg His Gly Thr Leu Gln Lys
    1535                1540                1545

Thr Asp Ile Pro Thr Tyr Pro Phe Gln Arg Val His Arg Tyr Pro
    1550                1555                1560

Thr Phe Ile Pro Ser Arg Asn Gln Ser Pro Ala Ile Ala Thr Val
    1565                1570                1575

Val Ile Pro Pro Pro Arg Phe Ser Val Gln Lys Ala Ala Asp Val
    1580                1585                1590

Ala Ser Gln Ser Lys Glu Ser Asp Cys Arg Ala Gly Leu Ile Ser
    1595                1600                1605

Cys Leu Arg Ala Ile Leu Glu Leu Thr Pro Glu Glu Glu Phe Asp
    1610                1615                1620

Leu Ser Glu Thr Leu Asn Ala Arg Gly Met Asp Ser Ile Met Phe
    1625                1630                1635

Ala Gln Leu Arg Lys Arg Val Gly Glu Glu Phe Asn Leu Asp Ile
    1640                1645                1650

Pro Met Ile Tyr Leu Ser Asp Val Phe Thr Met Glu Gln Met Val
    1655                1660                1665

Asp Tyr Leu Val Glu Gln Ser Gly Ser Thr Pro Ala Ser Lys His
    1670                1675                1680

Val Glu Thr Ser Ala Asn Gln Pro Leu Asp Glu Glu Asp Leu Arg
    1685                1690                1695

Thr Gly Leu Leu Ser Cys Leu Arg Asn Val Leu Glu Ile Thr Pro
    1700                1705                1710

Asp Glu Glu Leu Asp Leu Ser Glu Thr Leu Asn Ala Arg Gly Val
    1715                1720                1725

Asp Ser Ile Met Phe Ala Gln Leu Arg Lys Arg Val Gly Glu Gly
    1730                1735                1740

Phe Gly Val Glu Ile Pro Met Ile Tyr Leu Ser Asp Val Phe Thr
    1745                1750                1755

Met Glu Asp Met Ile Asn Phe Leu Val Ser Glu Arg Ser
    1760                1765                1770

<210> 50
<211> 5310
<212> DNA
<213> Armillaria ostoyae

<400> 50
atggaggccg acggtcacta ctctcttctc gatgtctttc tcagcgttgc acatgattct 60

gagaagtcca aacgtaatgt cttggaatgc ggccaggata cttggacata ctcggatttg 120

gacattatct cgtcggccct ggcacaggat ctcaaagcta tcttgggttg ttttcccaaa 180

gttgcagtcg tcagcgagaa ccatccctac gtatttgctc tcatgctggc cgtatggaag 240

cttgaaggga tattcatccc tatcgacgtc cacgttacag ctgaccttct aaagggcatg 300

ctacgcattg ttgctcccac ttgtctggtg atcccagaga ccgatatttt taaccagcgt 360

gttgcctctg caattggtat acatgttctc cccttcaacg tgaatgcgtc gaccatgact 420

gcacttcgac agaaatacca cccatttact cagaaagcct cgctatctgg gtgcgcactg 480

ccttacgttg atcgcgcatg cctctatctc tttacatcct ccgcgtcctc tactgccaat 540

ttgaaatgcg tacctttgac ccatactctt atcctcagta actgccgttc caagctcgca 600

tggtggcggc gcgttcgtcc ggaaggcgaa atggatgaga tacgtgttct agggtgggca 660

ccttggtcgc atatccttgc ctacatgcaa gatattggca cggcaacgct cctgaacgcc 720

ggttgctatg tctttgcgtc cgttccatcc acatatccta cacaactggc agcgaatggc 780

ttacaaggcc ctatcatgaa tatcatcgat tcacttcttg aacgacgagt cgccgcattt 840

gcttgcgtac cgttcatttt gagcgaacta aaagctatgt gcgagacggc ttccagtcca 900

gacgacaagc atcaaatgtg cttgagagct gaggagaaag ttcgccttgt cagtgcgctg 960

cagcggctta taatgctcga gtgcggaggc gctgcgcttg agtcaggtgt cacacgttgg 1020

gccgtcgaga atggcatatc agtcatggtc ggcatcggga tgacggagac agtcggtacg 1080

ctgtttgcag agcgcgcgca agacgcccgt tccaacggct attctgcgca ggacgccctc 1140

atttctgatg ggatcatgtc actggtcggg tctgacaacg aggaagccac cctcgaaggg 1200

gaactagtcg tcaagagcaa gctcatccct catggataca tcaagtaccg tgattcgtcg 1260

ttttcggtgg actcggacgg ctgggtaact ttcaaaacgg gagacaaata tcagcgcacg 1320

ccagatggac gattcaaatg gcttggaaga aagaccgatt ttattcagat gacaagcagc 1380

gaaacactgg atcccagacc cattgaggaa gccctctgtg cgaatccaag tatcgcaaat 1440

gcatgcgtca ttggtgacag gttcctgagg gagcctgcga ctagcgtatg cgccattgtc 1500

gagatcgggc cggaagtgga catgccttcg tccaagatcg acagagaaat tgcgaatacc 1560

ctcactccaa tcaatcgcgg ccttcctcct gctcttcgca tatcatggtc tcgcgtactc 1620

ataattcgac ctcctcagaa aataccagtt acgaggaaag gtgatgtgtt ccgtaagaag 1680

attgaagata tgtttgggtc tttccttggt gtcggcgttt ctaccgaggt cgaagtcgac 1740

catgaaacta aagaagatga tacgaaacac gtcgtgagac aggttgtaag caatcttctt 1800

ggagtccatg atcttgagtt attgtctgct ttatccttcg ccgagctggg catgacatca 1860

tttatggctg ttagcatcgt caacactcta aacaaacgca tcgacggcct cacccttcca 1920

cctaatgcat gctacatcca tattgatctt gattctcttg tggacgcgat ttcacttgaa 1980

catggtcatg aaagtatccc tgcagagttg ccttctaacc ctttccccgt tatcgagtcc 2040

catcaacata acgataagga cattgtgata gtcggcaagg cattccgttt acccggctca 2100

ctcaacaata ctgcatctct ctgggaagct ttgttatcga agaacagttc agtcatcagt 2160

gacatcccat ccgatcgctg ggatcacgca agcttttacc cccacgatat atgcttcacg 2220

aaagcaggcc tcgtcgatgt tgcacattac gactacagat tttttggcct cacggcgaca 2280

gaggcgttgt acgtatctcc gacgatgcgc ctcgccttgg aagtgtcatt tgaggccctg 2340

gagaacgcaa atattccact atccaagctg aaggggacgc aaaccgccgt ctatgtcgcc 2400

actaaagacg atggcttcga gacactctta aatgccgagc aaggctacga tgcatacacg 2460

cgattctacg gtacgggtcg tgctccgagt accgcaagcg gccgtataag ctatcttctt 2520

gatattcatg ggccatctgt caccgttgat acagcatgca gcggaggcat tgtatgtatg 2580

gatcaagcca tcactttctt gcaatccgga ggggccgata ccgctattgt ctgttcgagc 2640

aatacgcact gttggccggg atcattcatg ttcctgacgg cgcaaggcat ggtttctcaa 2700

aatggaagat gcgctacatt tactaccgat gcagacggat atgtaccttc ggagggtgca 2760

gtggctttca ttctcaagac gcgcagcgct gcgatacgcg acaacgacaa tatactcgcc 2820

gtgatcagat caacagacgt gtcccataac ggccgttctc aagggctagt tgcaccaaac 2880

gtaaaggcgc agacaaacct acaccggtcg ttgctacgaa aagctgggct gtttcctgat 2940

caaattaact ttatcgaagc tcatgggaca ggtacgtctc taggagacct ttcggaaatc 3000

cagggcatta ataacgccta cacctcaaca cgacctcgtc tggccggtcc ccttatcatt 3060

agcgcatcga aaacagtttt aggacacagt gaaccaacag cagggatggc cggcatcctc 3120

acagccttgc ttgcccttga gaaagagaca gttcctggtc taaatcactt aacggagcac 3180

aaccttaacc cttcgcttga ttgcagcgta gttcctctcc tgattcctca cgagtctatt 3240

cacattggtg gtgcaaagcc acatcgagct gcggttctgt catacggctt cgcgggtacg 3300

ctggccggtg ccatcttaga gggaccacct tctgatgtac caaggccgtc gtcaaataat 3360

atacaagaac accctatgat tttcgtcgtc agtgggaaaa ccgtgcctgc actggaagcg 3420

tacctaggac ggtatttggc atttttgcgg gtcgcaaaaa cacacgactt ccatgacatc 3480

tgctacacta cttgcgtcgg gagggagcac tacaaatacc ggttctcctg cgttgcccga 3540

aacatggcag accttatttc tcaaattgaa catcgactga cagctctttc cacttcgaaa 3600

cagaagcctc gcggctcgct agggttcata ttctcaggac aaggcactta tttccctggt 3660

atggctgcag cacttgccga acaatattcg gggttccgag tgctcgtctc taagtttggg 3720

caggctgccc aagagcggtc gggttatccg atcgataggc tgttgcttga agtttctgat 3780

acattgccag aaacaaacag cgaggtcgat caaatttgca tttttgtcta ccaatattct 3840

gttctgcaat ggctgcagag tctaggcatt caaccgaaag cagtcctcgg tcacagtctg 3900

ggagaaatta ctgcagcagt cgcagctggt gccctttcgt tcgaatctgc gttggacctt 3960

gtggtcaccc gtgctcgtct tctccgtcct agagcaaaag attctgcagg aatggccgca 4020

gtagcagcat ccaaggaaga agtcaaaggg cttatagaaa ccctccaact tgcggactcg 4080

ctgagcgttg cggttcataa cggtccgcgg agtgttgttg tgtcaggcgc atcagccgaa 4140

atcgacgccc tggttgtcgc agctaaagaa cggggcttga aggcctcccg cttaaaggtt 4200

gaccaaggct tccacagccc ttacgttgat tctgcggttc caggtttact cgactggtca 4260

aataagcatc gttcgacctt ccttcctttg aacattcctt tatactcgac tttgactggc 4320

gagcttattc cgaagggacg gaggttcgtc tcggatcact gggtaaacca tgctcgaaaa 4380

cctgtccagt ttgcggcggc agcggcagcc gtggatgaag accgatccat tggtgtgctc 4440

gttgacgttg gaccccaacc cgtcgcgtgg accctccttc aagcaaacaa ccttctcaat 4500

acctctgcag ttgcactatt cgcaaaggct ggaaaggatc aggagatggc gctgcttact 4560

gctttgagct acctcgtcca agagcacaac ctttctccca acttccatga gctttactct 4620

cagcgtcatg gtgctctgaa gaagacagac gttcccacct acccattccg ccgtgtccac 4680

cgctatccga ccttcatacc gtcacgaaat caaagtcctg ctgctgcgac ggtagctatg 4740

ccgccacccc gcttctctgt ccaaaagaat gcggatgtag catcacagtc gaaggaatca 4800

gactgtcgag ctggtttgat cagttgcctt agagccatcc tcgaattaac accggaagag 4860

gagtttgacc tttctgagac tctcaacgct cgtggtatgg attcgatcat gtttgcgcag 4920

ctacggaagc gggttgggga agaattcgac cttgacatac ccatgatcta tttatcagat 4980

gtgttcacga tggaacagat ggttgattac ctcgtcaaac agtccggatc cagacccgca 5040

ttaaaacacg cagaaattcc agttaatcaa ccattagacg aagatctccg gacgaggctc 5100

gtttcatgcc tgaggaacgt gctagaaatc acccccgatg aagaacttga cctatctgaa 5160

actttgaacg ctcgtggtgt tgactcgatc atgttcgctc agctacgaaa acgcgttggg 5220

gaaggatttg gtgtggaaat tccgatgata tatctgtccg acgtgtttac catggaagac 5280

atgatcaatt tcctcgtctc tgagcgctcg 5310

<210> 51
<211> 1770
<212> PRT
<213> Armillaria ostoyae

<400> 51

Met Glu Ala Asp Gly His Tyr Ser Leu Leu Asp Val Phe Leu Ser Val
1               5                   10                  15

Ala His Asp Ser Glu Lys Ser Lys Arg Asn Val Leu Glu Cys Gly Gln
            20                  25                  30

Asp Thr Trp Thr Tyr Ser Asp Leu Asp Ile Ile Ser Ser Ala Leu Ala
        35                  40                  45

Gln Asp Leu Lys Ala Ile Leu Gly Cys Phe Pro Lys Val Ala Val Val
    50                  55                  60

Ser Glu Asn His Pro Tyr Val Phe Ala Leu Met Leu Ala Val Trp Lys
65                  70                  75                  80

Leu Glu Gly Ile Phe Ile Pro Ile Asp Val His Val Thr Ala Asp Leu
                85                  90                  95

Leu Lys Gly Met Leu Arg Ile Val Ala Pro Thr Cys Leu Val Ile Pro
            100                 105                 110

Glu Thr Asp Ile Phe Asn Gln Arg Val Ala Ser Ala Ile Gly Ile His
        115                 120                 125

Val Leu Pro Phe Asn Val Asn Ala Ser Thr Met Thr Ala Leu Arg Gln
    130                 135                 140

Lys Tyr His Pro Phe Thr Gln Lys Ala Ser Leu Ser Gly Cys Ala Leu
145                 150                 155                 160

Pro Tyr Val Asp Arg Ala Cys Leu Tyr Leu Phe Thr Ser Ser Ala Ser
                165                 170                 175

Ser Thr Ala Asn Leu Lys Cys Val Pro Leu Thr His Thr Leu Ile Leu
            180                 185                 190

Ser Asn Cys Arg Ser Lys Leu Ala Trp Trp Arg Arg Val Arg Pro Glu
        195                 200                 205

Gly Glu Met Asp Glu Ile Arg Val Leu Gly Trp Ala Pro Trp Ser His
    210                 215                 220

Ile Leu Ala Tyr Met Gln Asp Ile Gly Thr Ala Thr Leu Leu Asn Ala
225                 230                 235                 240

Gly Cys Tyr Val Phe Ala Ser Val Pro Ser Thr Tyr Pro Thr Gln Leu
                245                 250                 255

Ala Ala Asn Gly Leu Gln Gly Pro Ile Met Asn Ile Ile Asp Ser Leu
            260                 265                 270

Leu Glu Arg Arg Val Ala Ala Phe Ala Cys Val Pro Phe Ile Leu Ser
        275                 280                 285

Glu Leu Lys Ala Met Cys Glu Thr Ala Ser Ser Pro Asp Asp Lys His
    290                 295                 300

Gln Met Cys Leu Arg Ala Glu Glu Lys Val Arg Leu Val Ser Ala Leu
305                 310                 315                 320

Gln Arg Leu Ile Met Leu Glu Cys Gly Gly Ala Ala Leu Glu Ser Gly
                325                 330                 335

Val Thr Arg Trp Ala Val Glu Asn Gly Ile Ser Val Met Val Gly Ile
            340                 345                 350

Gly Met Thr Glu Thr Val Gly Thr Leu Phe Ala Glu Arg Ala Gln Asp
        355                 360                 365

Ala Arg Ser Asn Gly Tyr Ser Ala Gln Asp Ala Leu Ile Ser Asp Gly
    370                 375                 380

Ile Met Ser Leu Val Gly Ser Asp Asn Glu Glu Ala Thr Leu Glu Gly
385                 390                 395                 400

Glu Leu Val Val Lys Ser Lys Leu Ile Pro His Gly Tyr Ile Lys Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Glu Ala Leu Cys Ala Asn Pro Ser Ile Ala Asn
465                 470                 475                 480

Ala Cys Val Ile Gly Asp Arg Phe Leu Arg Glu Pro Ala Thr Ser Val
                485                 490                 495

Cys Ala Ile Val Glu Ile Gly Pro Glu Val Asp Met Pro Ser Ser Lys
            500                 505                 510

Ile Asp Arg Glu Ile Ala Asn Thr Leu Thr Pro Ile Asn Arg Gly Leu
        515                 520                 525

Pro Pro Ala Leu Arg Ile Ser Trp Ser Arg Val Leu Ile Ile Arg Pro
    530                 535                 540

Pro Gln Lys Ile Pro Val Thr Arg Lys Gly Asp Val Phe Arg Lys Lys
545                 550                 555                 560

Ile Glu Asp Met Phe Gly Ser Phe Leu Gly Val Gly Val Ser Thr Glu
                565                 570                 575

Val Glu Val Asp His Glu Thr Lys Glu Asp Asp Thr Lys His Val Val
            580                 585                 590

Arg Gln Val Val Ser Asn Leu Leu Gly Val His Asp Leu Glu Leu Leu
        595                 600                 605

Ser Ala Leu Ser Phe Ala Glu Leu Gly Met Thr Ser Phe Met Ala Val
    610                 615                 620

Ser Ile Val Asn Thr Leu Asn Lys Arg Ile Asp Gly Leu Thr Leu Pro
625                 630                 635                 640

Pro Asn Ala Cys Tyr Ile His Ile Asp Leu Asp Ser Leu Val Asp Ala
                645                 650                 655

Ile Ser Leu Glu His Gly His Glu Ser Ile Pro Ala Glu Leu Pro Ser
            660                 665                 670

Asn Pro Phe Pro Val Ile Glu Ser His Gln His Asn Asp Lys Asp Ile
        675                 680                 685

Val Ile Val Gly Lys Ala Phe Arg Leu Pro Gly Ser Leu Asn Asn Thr
    690                 695                 700

Ala Ser Leu Trp Glu Ala Leu Leu Ser Lys Asn Ser Ser Val Ile Ser
705                 710                 715                 720

Asp Ile Pro Ser Asp Arg Trp Asp His Ala Ser Phe Tyr Pro His Asp
                725                 730                 735

Ile Cys Phe Thr Lys Ala Gly Leu Val Asp Val Ala His Tyr Asp Tyr
            740                 745                 750

Arg Phe Phe Gly Leu Thr Ala Thr Glu Ala Leu Tyr Val Ser Pro Thr
        755                 760                 765

Met Arg Leu Ala Leu Glu Val Ser Phe Glu Ala Leu Glu Asn Ala Asn
    770                 775                 780

Ile Pro Leu Ser Lys Leu Lys Gly Thr Gln Thr Ala Val Tyr Val Ala
785                 790                 795                 800

Thr Lys Asp Asp Gly Phe Glu Thr Leu Leu Asn Ala Glu Gln Gly Tyr
                805                 810                 815

Asp Ala Tyr Thr Arg Phe Tyr Gly Thr Gly Arg Ala Pro Ser Thr Ala
            820                 825                 830

Ser Gly Arg Ile Ser Tyr Leu Leu Asp Ile His Gly Pro Ser Val Thr
        835                 840                 845

Val Asp Thr Ala Cys Ser Gly Gly Ile Val Cys Met Asp Gln Ala Ile
    850                 855                 860

Thr Phe Leu Gln Ser Gly Gly Ala Asp Thr Ala Ile Val Cys Ser Ser
865                 870                 875                 880

Asn Thr His Cys Trp Pro Gly Ser Phe Met Phe Leu Thr Ala Gln Gly
                885                 890                 895

Met Val Ser Gln Asn Gly Arg Cys Ala Thr Phe Thr Thr Asp Ala Asp
            900                 905                 910

Gly Tyr Val Pro Ser Glu Gly Ala Val Ala Phe Ile Leu Lys Thr Arg
        915                 920                 925

Ser Ala Ala Ile Arg Asp Asn Asp Asn Ile Leu Ala Val Ile Arg Ser
    930                 935                 940

Thr Asp Val Ser His Asn Gly Arg Ser Gln Gly Leu Val Ala Pro Asn
945                 950                 955                 960

Val Lys Ala Gln Thr Asn Leu His Arg Ser Leu Leu Arg Lys Ala Gly
                965                 970                 975

Leu Phe Pro Asp Gln Ile Asn Phe Ile Glu Ala His Gly Thr Gly Thr
            980                 985                 990

Ser Leu Gly Asp Leu Ser Glu Ile  Gln Gly Ile Asn Asn  Ala Tyr Thr
        995                 1000                1005

Ser Thr  Arg Pro Arg Leu Ala  Gly Pro Leu Ile Ile  Ser Ala Ser
    1010                1015                1020

Lys Thr Val Leu Gly His Ser Glu Pro Thr Ala Gly Met Ala Gly
    1025                1030                1035

Ile Leu Thr Ala Leu Leu Ala Leu Glu Lys Glu Thr Val Pro Gly
    1040                1045                1050

Leu Asn His Leu Thr Glu His Asn Leu Asn Pro Ser Leu Asp Cys
    1055                1060                1065

Ser Val Val Pro Leu Leu Ile Pro His Glu Ser Ile His Ile Gly
    1070                1075                1080

Gly Ala Lys Pro His Arg Ala Ala Val Leu Ser Tyr Gly Phe Ala
    1085                1090                1095

Gly Thr Leu Ala Gly Ala Ile Leu Glu Gly Pro Pro Ser Asp Val
    1100                1105                1110

Pro Arg Pro Ser Ser Asn Asn Ile Gln Glu His Pro Met Ile Phe
    1115                1120                1125

Val Val Ser Gly Lys Thr Val Pro Ala Leu Glu Ala Tyr Leu Gly
    1130                1135                1140

Arg Tyr Leu Ala Phe Leu Arg Val Ala Lys Thr His Asp Phe His
    1145                1150                1155

Asp Ile Cys Tyr Thr Thr Cys Val Gly Arg Glu His Tyr Lys Tyr
    1160                1165                1170

Arg Phe Ser Cys Val Ala Arg Asn Met Ala Asp Leu Ile Ser Gln
    1175                1180                1185

Ile Glu His Arg Leu Thr Ala Leu Ser Thr Ser Lys Gln Lys Pro
    1190                1195                1200

Arg Gly Ser Leu Gly Phe Ile Phe Ser Gly Gln Gly Thr Tyr Phe
    1205                1210                1215

Pro Gly Met Ala Ala Ala Leu Ala Glu Gln Tyr Ser Gly Phe Arg
    1220                1225                1230

Val Leu Val Ser Lys Phe Gly Gln Ala Ala Gln Glu Arg Ser Gly
    1235                1240                1245

Tyr Pro Ile Asp Arg Leu Leu Leu Glu Val Ser Asp Thr Leu Pro
    1250                1255                1260

Glu Thr Asn Ser Glu Val Asp Gln Ile Cys Ile Phe Val Tyr Gln
    1265                1270                1275

Tyr Ser Val Leu Gln Trp Leu Gln Ser Leu Gly Ile Gln Pro Lys
    1280                1285                1290

Ala Val Leu Gly His Ser Leu Gly Glu Ile Thr Ala Ala Val Ala
    1295                1300                1305

Ala Gly Ala Leu Ser Phe Glu Ser Ala Leu Asp Leu Val Val Thr
    1310                1315                1320

Arg Ala Arg Leu Leu Arg Pro Arg Ala Lys Asp Ser Ala Gly Met
    1325                1330                1335

Ala Ala Val Ala Ala Ser Lys Glu Glu Val Lys Gly Leu Ile Glu
    1340                1345                1350

Thr Leu Gln Leu Ala Asp Ser Leu Ser Val Ala Val His Asn Gly
    1355                1360                1365

Pro Arg Ser Val Val Val Ser Gly Ala Ser Ala Glu Ile Asp Ala
    1370                1375                1380

Leu Val Val Ala Ala Lys Glu Arg Gly Leu Lys Ala Ser Arg Leu
    1385                1390                1395

Lys Val Asp Gln Gly Phe His Ser Pro Tyr Val Asp Ser Ala Val
    1400                1405                1410

Pro Gly Leu Leu Asp Trp Ser Asn Lys His Arg Ser Thr Phe Leu
    1415                1420                1425

Pro Leu Asn Ile Pro Leu Tyr Ser Thr Leu Thr Gly Glu Leu Ile
    1430                1435                1440

Pro Lys Gly Arg Arg Phe Val Ser Asp His Trp Val Asn His Ala
    1445                1450                1455

Arg Lys Pro Val Gln Phe Ala Ala Ala Ala Ala Ala Val Asp Glu
    1460                1465                1470

Asp Arg Ser Ile Gly Val Leu Val Asp Val Gly Pro Gln Pro Val
    1475                1480                1485

Ala Trp Thr Leu Leu Gln Ala Asn Asn Leu Leu Asn Thr Ser Ala
    1490                1495                1500

Val Ala Leu Phe Ala Lys Ala Gly Lys Asp Gln Glu Met Ala Leu
    1505                1510                1515

Leu Thr Ala Leu Ser Tyr Leu Val Gln Glu His Asn Leu Ser Pro
    1520                1525                1530

Asn Phe His Glu Leu Tyr Ser Gln Arg His Gly Ala Leu Lys Lys
    1535                1540                1545

Thr Asp Val Pro Thr Tyr Pro Phe Arg Arg Val His Arg Tyr Pro
    1550                1555                1560

Thr Phe Ile Pro Ser Arg Asn Gln Ser Pro Ala Ala Ala Thr Val
    1565                1570                1575

Ala Met Pro Pro Pro Arg Phe Ser Val Gln Lys Asn Ala Asp Val
    1580                1585                1590

Ala Ser Gln Ser Lys Glu Ser Asp Cys Arg Ala Gly Leu Ile Ser
    1595                1600                1605

Cys Leu Arg Ala Ile Leu Glu Leu Thr Pro Glu Glu Glu Phe Asp
    1610                1615                1620

Leu Ser Glu Thr Leu Asn Ala Arg Gly Met Asp Ser Ile Met Phe
    1625                1630                1635

Ala Gln Leu Arg Lys Arg Val Gly Glu Glu Phe Asp Leu Asp Ile
    1640                1645                1650

Pro Met Ile Tyr Leu Ser Asp Val Phe Thr Met Glu Gln Met Val
    1655                1660                1665

Asp Tyr Leu Val Lys Gln Ser Gly Ser Arg Pro Ala Leu Lys His
    1670                1675                1680

Ala Glu Ile Pro Val Asn Gln Pro Leu Asp Glu Asp Leu Arg Thr
    1685                1690                1695

Arg Leu Val Ser Cys Leu Arg Asn Val Leu Glu Ile Thr Pro Asp
    1700                1705                1710

Glu Glu Leu Asp Leu Ser Glu Thr Leu Asn Ala Arg Gly Val Asp
    1715                1720                1725

Ser Ile Met Phe Ala Gln Leu Arg Lys Arg Val Gly Glu Gly Phe
    1730                1735                1740

Gly Val Glu Ile Pro Met Ile Tyr Leu Ser Asp Val Phe Thr Met
    1745                1750                1755

Glu Asp Met Ile Asn Phe Leu Val Ser Glu Arg Ser
    1760                1765                1770

<210> 52
<211> 5322
<212> DNA
<213> Armillaria fuscipes

<400> 52
atgaccatgg aggtcgacag ccactattct ctgctcgatg tctttctcag cattgcacat 60

gattctgaca agtccaaacg caatgtcttg gaatgcggcc tggaggcctg gacatactcg 120

gatttagaca tcatctcgtc ggctctggca caggatctca aagctacctt gggatgtttt 180

cccaaagttg cagtcgtcag cgagaaccat ccctacgtgt ttgctctcat gctggccgtc 240

tggaagctcg aagggatctt catccccata gacgtccacg ttacagctga ccttttaaag 300

ggcatgctac gcattgttgc tcctacttgt ctagtgatcc ctgagagcga tgtttccaac 360

cggcgtgttg cctctgcgat tggtatacgt gttctcccat ttgatgcgaa ttcgtcaacc 420

atgacggcac ttcgacaaaa gtacgaatca ttcactcaga aagcctcgcc atctgagtgc 480

acacttgccc acgccgatcg tacatgcctc tatcttttta catcctctgc atcctctacc 540

gccaacttga aatgtgtgcc tttgacccat actcttatcc tcaataactg ccgtaccaag 600

ctcgcatggt ggcagcgctt tcgtccagaa agcgaaatgg atgggatgcg tgttctaggg 660

tgggcacctt ggtcgcatat ccttgcctac atgcaagata tcggcacagc gacgctcctg 720

aacgccggtt gctatgtctt tgcgtccatt ccatccacat accctacaca actagcagca 780

aatggcttac aaggccccac tatgaatatc atcaacgcac ttcttgaacg acaaattgcc 840

gcatttgctt gcgtgccgtt cattttgagc gaactcaaag ctatgtgcga gatgacttcc 900

tgtacaggaa accaaatgtc cctaagagcc gaggagaaag ttcgcctggt cagggtgctg 960

caggggcttg taatgctcga gtgtggaggc gcggcacttg agtcagatgt tacgcgttgg 1020

gtcgttgaga atgacatacc agtcatggtt ggcattggga tgacggaaac agtcggtacg 1080

ctgttcgcag agcgcgccca agacgtccgt tccagcggat attccgccca agacgctctc 1140

attgctgatg gcattatgtc actggtcggt tctgacaacg aggaagccac tttcgaaggg 1200

gaactagtcg tgaagagcaa gctcatccca caggatacat agggtaccg tgattcatcg 1260

ttctcggtgg actcagatgg ctgggtaacg ttcaaaactg gagataaata tcagcgcact 1320

ccagatggac gattaaaatg gcttgggaga aagaccgact ttatccagat gacaagcagc 1380

gaaacactgg atcccaggcc cattgagcaa actctttgtg cgaatccata cgtcgcaaaa 1440

gcatgcgtca ttggtgacag attcctgaga gatcccgcga ccagcgtatg tgccataatt 1500

gagatcaggc cggaagtgga catgccttcg tccaagatcg acagagaaat tgcgaatgcc 1560

cttgctccaa tcaatcgcga cctccctcct gctcttcgca tatcatggtc tcgcgtactc 1620

atgatcagac cccctcagaa aatcccagta acgaggaaag gcgatgtgtt ccgtaagaag 1680

attgaagata tgtttgggtc tttcctcggt gtcggtgttt ctactgaggt cggagtcgac 1740

catgaaactg aaaaagatga tacggaacac attgtgagac aggttgttac taatcttctt 1800

ggagtccatg atcccgagct attgtctgct ttatctttcg ctgagcttgg aatgacttca 1860

tttatggctg tcagcatcgt caactctcta aacaagtaca tcgatggcct cacccttcca 1920

cctaatgcat gttacatcca tattgatctt gattctcttg tggaggccat ttcacgtgaa 1980

cggggtcatg gaagtaacgc cacagagttg ccttctcaac ccgtccctgt tgagttacat 2040

caacctaacg ataaggacgt tgtgatagtt ggcaaggcat tccgtttacc tggctcactc 2100

gacagcactg catctctatg ggaagctttg ttatcaaaga acaattcagt cgtcagtgaa 2160

atcccatccg atcgctggga tcacgcaagc ttttaccccc acgacatttg cttcacgaaa 2220

gcaggcctcg tcgatgttgc ccattacgat tacagattct ttggcctcac ggctacagag 2280

gcattgtatg tatctccgac gatgcgtctc gccttggaag tgtcatttga agccctggag 2340

aatgcgaata ttccgttatc caatctgaag ggaacacaaa ccgctgtcta tgtcgccacc 2400

aaagacgatg gtttcgagac acttttaaat gccgagcagg gctatgatgc ctacacacga 2460

ttctatggca cgggccgcgc tccaagcacc gcaagtggcc gtataagcta tctacttgat 2520

attcatgggc catctgtcac cgttgataca gcatgcagcg gaggcattgt gtgtatggat 2580

caagccatca ctttcttgca gtccggaggg gcagatacag ctattgtctg ttcgagcaat 2640

acgcattgtt ggcctggatc atttatgttc ctgacggcgc aaggcatggt ttctccaaat 2700

ggaagatgcg ctacatttag taccgatgca gacggatatg tgccttcaga gggcgcagta 2760

gctttcgttc tcaagacgcg tagcgcagca atacgcgata atgacaatat cctcgccgta 2820

atcaaatcaa cagatgtgtc tcataacggc cgttctcaag ggctggttgc acctaacgtg 2880

aaggcgcaga caaacctgca tcaatcgttg ttacgaaaag ctgggctgtt tcctgatcaa 2940

atcaacttta tcgaagccca tggaacaggt acatctctag gagacctctc agaaatccag 3000

ggcatcaata acgcctacac ctcaacacga cctcgtctag acggtcccct tatcatcagc 3060

gcatcgaaaa cagtgatagg acacagcgaa ccaactgcag ggatggcggg catcctcaca 3120

gccttgcttg ctcttgagaa agaaacagtt cctggtctca atcacttaac ggagcacagc 3180

cttaaccctt cgcttgattg cagcatagtc ccgctcctga ttcctcacga gtctattcac 3240

attggtgggg taaagccaca tcgagctgcg gttctgtcat acggcttcgc gggtacactg 3300

gcgggtgcta tcttagaggg accgccttca gatgcaccaa ggccgtcgtc aaataatgtg 3360

caagatcacc ctatgatttt tgccctcagt gggaaaagcg cgtccgcact ggaagcatac 3420

ctaaggcggt atttggcatt cctgcggatt gcagatccac acgacttcca taacatctgc 3480

tacacttcct gtgtcgggag ggagcactac aaatatcggt tctcctgtgt tgcccgaaac 3540

atggcagacc ttatatctca aattgaacat cgactgacaa ctgtttccat tccgaaaccg 3600

aaacctcgtg gctcaatagg attcacgttc tcaggacaag gcacttattt ccctggcatg 3660

gccgcagcac tcactgaaca atattctgga ttccggacgc tcgtctctaa gcttgggcag 3720

gctgcgcaag agcggtcggg tcatccgatt gacaggctgt tacttgaagt ttccggtaca 3780

tcaccagaaa caaacagtga ggtcgagcaa atttgcacat ttatctacca atatgccgtt 3840

ctgcaatggt tgcagagcct aggcgttcaa ccgaaagcag tcctcggtca cagcctggga 3900

gaaattactg ccgcagtcgc agctggtgcc ctgtcgttcg aatccgcgtt ggaccttgtg 3960

gtgacccgtg ctcgtcttct ccgtcccgaa acaaaagatt ctgctgggat ggtcgcggta 4020

gcaacgtcca aggatgaagt tgaaggactt atagaaacac tccaagttgc ggacgcgcta 4080

agcgttgccg ttcacaacgg ttcacggagt gttgtggttt caggcacatc agcggaagtt 4140

gatgccctgg tcgtcgcagc taaagaacgg ggcttaaagg cttcccgctt aagagtcgac 4200

caaggtttcc acagcccttg cgttgattct gccgttcctg gtttactcga ctggtcaaat 4260

gagcatcgtt ccaccttcct tcctttgaat atgcctttat actcgacttt gaccggcgag 4320

gtcattccca agggacggaa attcgtctgg gatcactggg taaaccatgc tcgaaaacct 4380

gttcagtttg caccggcagc aaaagcggtg gacgaagacc gatccatcgg tgtgctcgtt 4440

gatgtaggac ctcaacctgt cgcttggacc cttttgcaag caaacaacct ttccaacacc 4500

tctacggttg cgctattcgc gaaagccgga aaggatcagg agatggcact gctcactgct 4560

ttgagctacc tcttccaaga gcacaacctt tctcccaagt ttcacgacct ttatactggg 4620

tataatggtg ctctgaagaa gacggacatt cccacgtacc cattccaacg tgtccatcgc 4680

tatcccacct tcataccatc acgaaatcag agtcctgctg tcgcgaaagc agtcgtgcag 4740

ccgccccgct tctctatcca aaggaatcga gaagccacat tacagtcgaa ggaaccagat 4800

caccgagctt gtttagtcac ttgccttaga gccatcctcg aattaacatc agaggaagaa 4860

cttgacctct ctgagaccct caacgctcgt ggcgtggact cgatcatgtt ttcacagcta 4920

cggaagcggg ttggagaaga attcaatctc gagataccca tgatctattt atcagacgta 4980

ttcacgatgg agcagatgat tgactacctc gtcgaacagt ccggatccaa tcccgcatca 5040

aagcaagtag gaactccggt taaccgacta tcaggcgaag aagatcttcg gacggggctc 5100

atctcatgcc tgagggacgt gctagaaatc actcctgatg atgaacttga tcacccaaaa     5160

gacctatctg aaactttaaa tgctcgtggt gttgattcga taatgttcgc tcagctacga     5220

aaacgcgtcg gggaaggatt tggtgtggaa attccgatga tatatctgtc tgatgtgttt     5280

accatggaag acatgattaa tttcctcgtt tctgagcgct ca                        5322

<210>  53
<211>  1774
<212>  PRT
<213>  Armillaria fuscipes

<400>  53

Met Thr Met Glu Val Asp Ser His Tyr Ser Leu Leu Asp Val Phe Leu
1               5                   10                  15

Ser Ile Ala His Asp Ser Asp Lys Ser Lys Arg Asn Val Leu Glu Cys
            20                  25                  30

Gly Leu Glu Ala Trp Thr Tyr Ser Asp Leu Asp Ile Ile Ser Ser Ala
        35                  40                  45

Leu Ala Gln Asp Leu Lys Ala Thr Leu Gly Cys Phe Pro Lys Val Ala
    50                  55                  60

Val Val Ser Glu Asn His Pro Tyr Val Phe Ala Leu Met Leu Ala Val
65                  70                  75                  80

Trp Lys Leu Glu Gly Ile Phe Ile Pro Ile Asp Val His Val Thr Ala
                85                  90                  95

Asp Leu Leu Lys Gly Met Leu Arg Ile Val Ala Pro Thr Cys Leu Val
            100                 105                 110

Ile Pro Glu Ser Asp Val Ser Asn Arg Arg Val Ala Ser Ala Ile Gly
        115                 120                 125

Ile Arg Val Leu Pro Phe Asp Ala Asn Ser Ser Thr Met Thr Ala Leu
    130                 135                 140

Arg Gln Lys Tyr Glu Ser Phe Thr Gln Lys Ala Ser Pro Ser Glu Cys
145                 150                 155                 160

Thr Leu Ala His Ala Asp Arg Thr Cys Leu Tyr Leu Phe Thr Ser Ser
                165                 170                 175

Ala Ser Ser Thr Ala Asn Leu Lys Cys Val Pro Leu Thr His Thr Leu
            180                 185                 190

Ile Leu Asn Asn Cys Arg Thr Lys Leu Ala Trp Trp Gln Arg Phe Arg
        195                 200                 205

Pro Glu Ser Glu Met Asp Gly Met Arg Val Leu Gly Trp Ala Pro Trp
    210                 215                 220

Ser His Ile Leu Ala Tyr Met Gln Asp Ile Gly Thr Ala Thr Leu Leu
225                 230                 235                 240

Asn Ala Gly Cys Tyr Val Phe Ala Ser Ile Pro Ser Thr Tyr Pro Thr
                245                 250                 255

Gln Leu Ala Ala Asn Gly Leu Gln Gly Pro Thr Met Asn Ile Ile Asn
            260                 265                 270

Ala Leu Leu Glu Arg Gln Ile Ala Ala Phe Ala Cys Val Pro Phe Ile
        275                 280                 285

Leu Ser Glu Leu Lys Ala Met Cys Glu Met Thr Ser Cys Thr Gly Asn
    290                 295                 300

Gln Met Ser Leu Arg Ala Glu Glu Lys Val Arg Leu Val Arg Val Leu
305                 310                 315                 320

Gln Gly Leu Val Met Leu Glu Cys Gly Gly Ala Ala Leu Glu Ser Asp
                325                 330                 335

Val Thr Arg Trp Val Val Glu Asn Asp Ile Pro Val Met Val Gly Ile
            340                 345                 350

Gly Met Thr Glu Thr Val Gly Thr Leu Phe Ala Glu Arg Ala Gln Asp
        355                 360                 365

Val Arg Ser Ser Gly Tyr Ser Ala Gln Asp Ala Leu Ile Ala Asp Gly
    370                 375                 380

Ile Met Ser Leu Val Gly Ser Asp Asn Glu Glu Ala Thr Phe Glu Gly
385                 390                 395                 400

Glu Leu Val Val Lys Ser Lys Leu Ile Pro Gln Gly Tyr Ile Gly Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Leu Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Gln Thr Leu Cys Ala Asn Pro Tyr Val Ala Lys
465                 470                 475                 480

Ala Cys Val Ile Gly Asp Arg Phe Leu Arg Asp Pro Ala Thr Ser Val
                485                 490                 495

Cys Ala Ile Ile Glu Ile Arg Pro Glu Val Asp Met Pro Ser Ser Lys
            500                 505                 510

Ile Asp Arg Glu Ile Ala Asn Ala Leu Ala Pro Ile Asn Arg Asp Leu
        515                 520                 525

Pro Pro Ala Leu Arg Ile Ser Trp Ser Arg Val Leu Met Ile Arg Pro
    530                 535                 540

Pro Gln Lys Ile Pro Val Thr Arg Lys Gly Asp Val Phe Arg Lys Lys
545                 550                 555                 560

Ile Glu Asp Met Phe Gly Ser Phe Leu Gly Val Gly Val Ser Thr Glu
                565                 570                 575

Val Gly Val Asp His Glu Thr Glu Lys Asp Asp Thr Glu His Ile Val
            580                 585                 590

Arg Gln Val Val Thr Asn Leu Leu Gly Val His Asp Pro Glu Leu Leu
        595                 600                 605

Ser Ala Leu Ser Phe Ala Glu Leu Gly Met Thr Ser Phe Met Ala Val
    610                 615                 620

Ser Ile Val Asn Ser Leu Asn Lys Tyr Ile Asp Gly Leu Thr Leu Pro
625                 630                 635                 640

Pro Asn Ala Cys Tyr Ile His Ile Asp Leu Asp Ser Leu Val Glu Ala
                645                 650                 655

Ile Ser Arg Glu Arg Gly His Gly Ser Asn Ala Thr Glu Leu Pro Ser
            660                 665                 670

Gln Pro Val Pro Val Glu Leu His Gln Pro Asn Asp Lys Asp Val Val
        675                 680                 685

Ile Val Gly Lys Ala Phe Arg Leu Pro Gly Ser Leu Asp Ser Thr Ala
    690                 695                 700

Ser Leu Trp Glu Ala Leu Leu Ser Lys Asn Asn Ser Val Val Ser Glu
705                 710                 715                 720

Ile Pro Ser Asp Arg Trp Asp His Ala Ser Phe Tyr Pro His Asp Ile
                725                 730                 735

Cys Phe Thr Lys Ala Gly Leu Val Asp Val Ala His Tyr Asp Tyr Arg
            740                 745                 750

Phe Phe Gly Leu Thr Ala Thr Glu Ala Leu Tyr Val Ser Pro Thr Met
        755                 760                 765

Arg Leu Ala Leu Glu Val Ser Phe Glu Ala Leu Glu Asn Ala Asn Ile
    770                 775                 780

Pro Leu Ser Asn Leu Lys Gly Thr Gln Thr Ala Val Tyr Val Ala Thr
785                 790                 795                 800

Lys Asp Asp Gly Phe Glu Thr Leu Leu Asn Ala Glu Gln Gly Tyr Asp
                805                 810                 815

Ala Tyr Thr Arg Phe Tyr Gly Thr Gly Arg Ala Pro Ser Thr Ala Ser
            820                 825                 830

Gly Arg Ile Ser Tyr Leu Leu Asp Ile His Gly Pro Ser Val Thr Val
        835                 840                 845

Asp Thr Ala Cys Ser Gly Gly Ile Val Cys Met Asp Gln Ala Ile Thr
    850                 855                 860

Phe Leu Gln Ser Gly Gly Ala Asp Thr Ala Ile Val Cys Ser Ser Asn
865                 870                 875                 880

Thr His Cys Trp Pro Gly Ser Phe Met Phe Leu Thr Ala Gln Gly Met
                885                 890                 895

Val Ser Pro Asn Gly Arg Cys Ala Thr Phe Ser Thr Asp Ala Asp Gly
            900                 905                 910

Tyr Val Pro Ser Glu Gly Ala Val Ala Phe Val Leu Lys Thr Arg Ser
        915                 920                 925

Ala Ala Ile Arg Asp Asn Asp Asn Ile Leu Ala Val Ile Lys Ser Thr
    930                 935                 940

Asp Val Ser His Asn Gly Arg Ser Gln Gly Leu Val Ala Pro Asn Val
945                 950                 955                 960

Lys Ala Gln Thr Asn Leu His Gln Ser Leu Leu Arg Lys Ala Gly Leu
                965                 970                 975

Phe Pro Asp Gln Ile Asn Phe Ile Glu Ala His Gly Thr Gly Thr Ser
            980                 985                 990

Leu Gly Asp Leu Ser Glu Ile Gln  Gly Ile Asn Asn Ala  Tyr Thr Ser
        995                 1000                1005

Thr Arg  Pro Arg Leu Asp Gly  Pro Leu Ile Ile Ser  Ala Ser Lys
    1010                1015                1020

Thr Val Ile Gly His Ser Glu Pro Thr Ala Gly Met Ala Gly Ile
    1025                1030                1035

Leu Thr Ala Leu Leu Ala Leu Glu Lys Glu Thr Val Pro Gly Leu
    1040                1045                1050

Asn His Leu Thr Glu His Ser Leu Asn Pro Ser Leu Asp Cys Ser
    1055                1060                1065

Ile Val Pro Leu Leu Ile Pro His Glu Ser Ile His Ile Gly Gly
    1070                1075                1080

Val Lys Pro His Arg Ala Ala Val Leu Ser Tyr Gly Phe Ala Gly
    1085                1090                1095

Thr Leu Ala Gly Ala Ile Leu Glu Gly Pro Pro Ser Asp Ala Pro
    1100                1105                1110

Arg Pro Ser Ser Asn Asn Val Gln Asp His Pro Met Ile Phe Ala
    1115                1120                1125

Leu Ser Gly Lys Ser Ala Ser Ala Leu Glu Ala Tyr Leu Arg Arg
    1130                1135                1140

Tyr Leu Ala Phe Leu Arg Ile Ala Asp Pro His Asp Phe His Asn
    1145                1150                1155

Ile Cys Tyr Thr Ser Cys Val Gly Arg Glu His Tyr Lys Tyr Arg
    1160                1165                1170

Phe Ser Cys Val Ala Arg Asn Met Ala Asp Leu Ile Ser Gln Ile
    1175                1180                1185

Glu His Arg Leu Thr Thr Val Ser Ile Pro Lys Pro Lys Pro Arg
    1190                1195                1200

Gly Ser Ile Gly Phe Thr Phe Ser Gly Gln Gly Thr Tyr Phe Pro
    1205                1210                1215

Gly Met Ala Ala Ala Leu Thr Glu Gln Tyr Ser Gly Phe Arg Thr
    1220                1225                1230

Leu Val Ser Lys Leu Gly Gln Ala Ala Gln Glu Arg Ser Gly His
    1235                1240                1245

Pro Ile Asp Arg Leu Leu Leu Glu Val Ser Gly Thr Ser Pro Glu
    1250                1255                1260

Thr Asn Ser Glu Val Glu Gln Ile Cys Thr Phe Ile Tyr Gln Tyr
    1265                1270                1275

Ala Val Leu Gln Trp Leu Gln Ser Leu Gly Val Gln Pro Lys Ala
    1280                1285                1290

Val Leu Gly His Ser Leu Gly Glu Ile Thr Ala Ala Val Ala Ala
    1295                1300                1305

Gly Ala Leu Ser Phe Glu Ser Ala Leu Asp Leu Val Val Thr Arg
    1310                1315                1320

Ala Arg Leu Leu Arg Pro Glu Thr Lys Asp Ser Ala Gly Met Val
    1325                1330                1335

Ala Val Ala Thr Ser Lys Asp Glu Val Glu Gly Leu Ile Glu Thr
    1340                1345                1350

Leu Gln Val Ala Asp Ala Leu Ser Val Ala Val His Asn Gly Ser
    1355                1360                1365

Arg Ser Val Val Val Ser Gly Thr Ser Ala Glu Val Asp Ala Leu
    1370                1375                1380

Val Val Ala Ala Lys Glu Arg Gly Leu Lys Ala Ser Arg Leu Arg
    1385                1390                1395

Val Asp Gln Gly Phe His Ser Pro Cys Val Asp Ser Ala Val Pro
    1400                1405                1410

Gly Leu Leu Asp Trp Ser Asn Glu His Arg Ser Thr Phe Leu Pro
    1415                1420                1425

Leu Asn Met Pro Leu Tyr Ser Thr Leu Thr Gly Glu Val Ile Pro
    1430                1435                1440

Lys Gly Arg Lys Phe Val Trp Asp His Trp Val Asn His Ala Arg
    1445                1450                1455

Lys Pro Val Gln Phe Ala Pro Ala Ala Lys Ala Val Asp Glu Asp
    1460                1465                1470

Arg Ser Ile Gly Val Leu Val Asp Val Gly Pro Gln Pro Val Ala
    1475                1480                1485

Trp Thr Leu Leu Gln Ala Asn Asn Leu Ser Asn Thr Ser Thr Val
    1490                1495                1500

Ala Leu Phe Ala Lys Ala Gly Lys Asp Gln Glu Met Ala Leu Leu
    1505                1510                1515

Thr Ala Leu Ser Tyr Leu Phe Gln Glu His Asn Leu Ser Pro Lys
    1520                1525                1530

Phe His Asp Leu Tyr Thr Gly Tyr Asn Gly Ala Leu Lys Lys Thr
    1535                1540                1545

Asp Ile Pro Thr Tyr Pro Phe Gln Arg Val His Arg Tyr Pro Thr
    1550                1555                1560

Phe Ile Pro Ser Arg Asn Gln Ser Pro Ala Val Ala Lys Ala Val
    1565                1570                1575

Val Gln Pro Pro Arg Phe Ser Ile Gln Arg Asn Arg Glu Ala Thr
    1580                1585                1590

Leu Gln Ser Lys Glu Pro Asp His Arg Ala Cys Leu Val Thr Cys
    1595                1600                1605

Leu Arg Ala Ile Leu Glu Leu Thr Ser Glu Glu Glu Leu Asp Leu
    1610                1615                1620

Ser Glu Thr Leu Asn Ala Arg Gly Val Asp Ser Ile Met Phe Ser
    1625                1630                1635

Gln Leu Arg Lys Arg Val Gly Glu Glu Phe Asn Leu Glu Ile Pro
    1640                1645                1650

Met Ile Tyr Leu Ser Asp Val Phe Thr Met Glu Gln Met Ile Asp
    1655                1660                1665

Tyr Leu Val Glu Gln Ser Gly Ser Asn Pro Ala Ser Lys Gln Val
    1670                1675                1680

Gly Thr Pro Val Asn Arg Leu Ser Gly Glu Glu Asp Leu Arg Thr
    1685                1690                1695

Gly Leu Ile Ser Cys Leu Arg Asp Val Leu Glu Ile Thr Pro Asp
    1700                1705                1710

Asp Glu Leu Asp His Pro Lys Asp Leu Ser Glu Thr Leu Asn Ala
    1715                1720                1725

Arg Gly Val Asp Ser Ile Met Phe Ala Gln Leu Arg Lys Arg Val
    1730                1735                1740

Gly Glu Gly Phe Gly Val Glu Ile Pro Met Ile Tyr Leu Ser Asp
    1745                1750                1755

Val Phe Thr Met Glu Asp Met Ile Asn Phe Leu Val Ser Glu Arg
    1760                1765                1770

Ser

<210> 54
<211> 5304
<212> DNA
<213> Armillaria mellea

<400> 54
atggaggcca acggtcacta ctctcttctc gatgtctttc tcagcattgc acacgattct 60

gacaagtcca aacgtaatgt cttggaatgc ggtcaggata cctggacata ctcagatttg 120

gacattatct cgtcggccct ggcacaggat ctccaagcta cgctgggttg ttttcccaaa 180

gttgcagtcg tcagcgagaa ccatccctac atttttgctc tcatgctggc cgtttggaag 240

ctcgaaggga tattcatccc tgtcgacgtc catgttacag ctgaccttct aaagggcatg 300

ttacatatcg tcgctcccac ttgtctggtg atccctgaga ccgatatttc caaccagcgt 360

attgcttccg cgattggtat acatgttctc cccttcagtg cgaatgcgtc gaccatgact 420

gcacttcgac agaaatacga cctatgcatt cagaaagcct cgctatctga gcgcgcactt 480

cctcacgttg atcgcgcttg cctctatctc tttacatcct ctgcgtcctc tactgccaac 540

ttgaaatgcg tacctttgac ccatagtctt atcctcagta actgccgttc caaactcgca 600

tggtggcggc gcgttcgtcc agaggaagaa atggatggga tacgtgttct agggtgggca 660

ccttggtccc atatccttgc ctacatgcaa gatattggca cagcgacgct cctgaacgcc 720

ggttgctacg tctttgcgtc aattccgtcc acatatccta cacaactggt ggtgaatggc 780

ttacaaggcc ccaccatgaa tatcatcgac tcacttctta aacggcgaat cgtcgcattt 840

gcttgtgtcc cgttcatttt gggcgaacta aaagctatgt gcgagacggc ttcgggtcca 900

gatgtcaagc atcatatggg cttgagagct gaggagaagg ttcgccttgt tagggcactg 960

cagcagctta tgatgctcga gtgcggaggc gctgcgctcg agtcagatgt cacgcgttgg 1020

gttgtcgaaa atggcatatc ggtcatggtt ggcatcggga tgacggagac agttggtacg 1080

ctgtttgcag agcgcgcgca agacgcccgt tccaacggct actctgcgca gaacgccctc 1140

attactgatg gcattatgtc actggtcggg cctgacaacg aggaagtcac ctttgaaggg 1200

gaactagtcg tgaagagcaa gctcatccca cacgaataca tcaattaccg tgattcgtcg 1260

ttctcggtgg actcggatgg ctgggtaacg tttaaaacgg gagataaata tcagcgcaca 1320

ccagatggac gattcaaatg gcttggaaga aagaccgact ttattcagat gacaagcagt 1380

gaaacactgg atcccagacc cattgagaaa accctctgtg cgaatccaag tattgtaaat 1440

gcatgcgtca ttggtgacag attcctgagg gagcctgcaa ctagcgtatg cgccattgtc 1500

gagatcaggc cggaagtgga catcccttcg tccaagatcg acagggaaat tgcgaatgcc 1560

cttgctccaa tcaatcgcga cctccctcct gctcttcgca tatcatggtc tcgcgtactc 1620

ataattcgac ctcctcagaa aataccagtt acaaggaaag gcgatgtgtt ccgtaagaag 1680

attgaagatg tatttgggtc tttccttggt gtcggttcta ccgagatcaa agtcgaccat 1740

gaatctaaag aagatgatac ggaacacatt gtgagacagg ttgtcagcaa tcttcttgga 1800

gtccatgatc ctgagctatt gtctgcttta tctttcgctg agctgggaat gacctcgttt 1860

atagctgtta gtattgtcaa cgctctaaac aagcgcatca gcggcctcac ccttccacct 1920

aatgcgtgct acctccatat tgatcttgat tctcttgtga acgccatttc acgtgaacat 1980

ggtcatggaa ctaaccccgc agagttgcct tctaaacttt tccccgtcac cgagtcctat 2040

caacgtaatg ataaggacgt tgtgataatc ggcaaagcat tccgtttacc aggctcactc 2100

gacaatactg catctctctg ggaagctttg ttatcgaaga acaattcagt cgtcagtgac 2160

atcccatccg atcgctggga tcacgcaagc ttttaccccc acgatatatg cttcacgaaa 2220

gcaggcctcg tcgatgttgc acattacgat tacagattct ttgggctcac agcgacagag 2280

gcattgtacg tatctccgac gatgcgcctc gccttggaag tgtcatttga agcccttgag 2340

aacgcgaata ttccgctatc caagctgaag gggacacaaa cccctgtcta tgtcgccact 2400

aaagacgatg gctttgagac actcttaaat gctgagcaag gctacgatgc ttacacgcga 2460

ttctatggta caggtcgcgc tccgagcacc gcaagtggtc gtataagcta tctactcgat 2520

attcatgggc catctgttac cgttgataca gcatgcagcg gaggcattgt atgtatggat 2580

caagccatca ctttcttgca atccggaagg gccgataccg ctattgtctg ttcgagcaat 2640

acgcactgtt ggccgggatc atttatgttc ctgacggcgc agggcatggt ttctccacat 2700

ggaagatgcg ctacatttac taccgatgca gacggttatg taccttcgga gggtgctgtg 2760

gctttcattc tcaagacgcg cagtgctgca atacgcgata atgacaatat actcgccgtg 2820

atcaaatcaa cagatgtgtc ccataacggc cgttctcaag ggctagttgc accaaacgta 2880

aaggcgcaga caagcctaca ccgatcgttg ctacgaaaag ctggactatt tcctgatcaa 2940

atcaatttta tcgaagccca tggaacaggt acatctctag gagacctctc ggaaatccag 3000

ggcatcaaca acgcctacac ctcaacacga cctcgtttgg acggtcccct tatcattagc 3060

gcgtcgaaaa cagtgttggg acacagcgaa ccaattgcgg ggatggccgg catcctcaca 3120

gccttgcttt ccctcgagaa agaaacagtt tttggtttaa atcacttaac agagcacaac 3180

cttaaccctt cgcttgattg cagcctagtt cctctcctga ttcctcacga gtctattcac 3240

attggtggtg aaaaaccaca tcgagctgcg gttctgtcat acggtttcgc gggtacgctg 3300

gccggtgcca tcttagaggg accaccttca gatgtaccaa ggccgtcgtc aagcgatatg 3360

caagaacacc ctatggtttt cgtcctcagt gggaaaagcg tgcctgcact ggaaacgtac 3420

ctaagacggt atttggcatt tttgcgcgcc gcaaaaacaa acgacttcca tagcatctgc 3480

tacaccactt gcgtcgggag ggagcattac aaataccggt tctcctgcgt tgcccaaaat 3540

atggcagacc ttgtgtctca aattgaacat cgactgacaa ctctttccaa ttcgaaacag 3600

aaacctcgtg gctcgccagg gtttatgttc tcaggacaag gcacttattt ccctggtatg 3660

gctgcagcgc ttgctgaaca atatttgggg tttcgagtgc tagtctctag gtttgggaag 3720

gctgctcaag agcggtcggg ttatccgatc gataggctgt tgcttgaagt ttctgataca 3780

tcatcagaaa caaacagcga ggctgaccaa atttgcattt ttgtctacca atattccgtt 3840

ctgcaatggc tgcagagtct aggcattcaa ccgaaagcag tcctcggtca cagcctggga 3900

gaaattaccg ccgcagtcgc agctggtgcc ctttcattcg aatctgcgtt ggaccttgta 3960

gtcacccgtg ctcgtcttct ccgtcctgaa acaaaagatt cagcaggaat ggccgcagta 4020

gcagcatcca aggaagaagt tgaagaactt atagaaaacc tccaacttgc gcatgcgtta 4080

agtgttgcgg ttcacaacgg tccacggagt gttgttgtgt caggcgcatc gaccgaaatt 4140

gatgccctgg tcgtcgcagc taaagaacgg ggcttgaagg cttcccgctt aagagttgac 4200

caaggcttcc atagccctta cgttgattct gccgttccgg gtttactcga ctggtcaagt 4260

gaacattgtt cgaccttcct tcctttaaat attcctttat actcgacttt gaccggcgaa 4320

gttattccaa agggacggag gttcgcctgg gatcactggg taaaccatgc tcgaaaacct 4380

gttcagtttg cggcggcggc agcagcggtg gacgaagacc gatccatcgg cgtgctcctt 4440

gatgttggac cccaacccgt tgcgtggacc atccttcaag caagcagcct tttcaacacc 4500

tctgcagttg cgctatttgc gaaggctgga aaggatcagg agatggcgct gcttactact 4560

ttgagctacc tcttccaaga gcacaatctt tgtcccaact ttcacgagct ttactctcag 4620

cgtcatggtg ctcttaagaa gacggacatt cccacctacc cattccaacg tgtccaccgc 4680

tatccaacct tcataccatc acgaaatcaa agtcctgctg tcgcgaaggt agtagtccca 4740

tcgcgctttc ctgtccaaag gaaaggggaa gcaatatcac aatcgaacga atcagattac 4800

cgagctggtt tgatcacttg cctcagaacc atcctcgaat taacatcaga agaagagttt 4860

gacctttctg agaccctcaa cgctcgtggt gtggattcga tcatgttttc acagctacgg 4920

aagcgggttg gggaagaatt cgatctcgat atacccatga tctatttatc agacgtgttc 4980

acgatggaac agatgatcga ctacctcgtc gaacagtccg gatccagacc cgcgccaaag 5040

cacgtagaaa ctccggttaa cgaaccttta ggcaaagatc tccggacggg gctcgtttca 5100

tgcctgagga atgtactaga aatcaccccc gatgaagaac tcgacctatc tgaaactttg 5160

aacgctcgtg gtgtcgactc gatcatgttc gctcagctac gaaaacgcgt cggggaagga 5220

tttggcgtgg aaattccgat gatatatctg tctgacgtgt ttactatgga agacatgatc 5280

aatttcctcg tctctgagca cgcg 5304

<210> 55
<211> 1768
<212> PRT
<213> Armillaria mellea

<400> 55

Met Glu Ala Asn Gly His Tyr Ser Leu Leu Asp Val Phe Leu Ser Ile
1               5                   10                  15

Ala His Asp Ser Asp Lys Ser Lys Arg Asn Val Leu Glu Cys Gly Gln
            20                  25                  30

Asp Thr Trp Thr Tyr Ser Asp Leu Asp Ile Ile Ser Ser Ala Leu Ala
        35                  40                  45

Gln Asp Leu Gln Ala Thr Leu Gly Cys Phe Pro Lys Val Ala Val Val
    50                  55                  60

Ser Glu Asn His Pro Tyr Ile Phe Ala Leu Met Leu Ala Val Trp Lys
65                  70                  75                  80

Leu Glu Gly Ile Phe Ile Pro Val Asp Val His Val Thr Ala Asp Leu
                85                  90                  95

Leu Lys Gly Met Leu His Ile Val Ala Pro Thr Cys Leu Val Ile Pro
            100                 105                 110

Glu Thr Asp Ile Ser Asn Gln Arg Ile Ala Ser Ala Ile Gly Ile His
        115                 120                 125

Val Leu Pro Phe Ser Ala Asn Ala Ser Thr Met Thr Ala Leu Arg Gln
    130                 135                 140

Lys Tyr Asp Leu Cys Ile Gln Lys Ala Ser Leu Ser Glu Arg Ala Leu
145                 150                 155                 160

Pro His Val Asp Arg Ala Cys Leu Tyr Leu Phe Thr Ser Ser Ala Ser
                165                 170                 175

Ser Thr Ala Asn Leu Lys Cys Val Pro Leu Thr His Ser Leu Ile Leu
            180                 185                 190

Ser Asn Cys Arg Ser Lys Leu Ala Trp Trp Arg Arg Val Arg Pro Glu
        195                 200                 205

Glu Glu Met Asp Gly Ile Arg Val Leu Gly Trp Ala Pro Trp Ser His
    210                 215                 220

Ile Leu Ala Tyr Met Gln Asp Ile Gly Thr Ala Thr Leu Leu Asn Ala
225                 230                 235                 240

Gly Cys Tyr Val Phe Ala Ser Ile Pro Ser Thr Tyr Pro Thr Gln Leu
                245                 250                 255

Val Val Asn Gly Leu Gln Gly Pro Thr Met Asn Ile Ile Asp Ser Leu
            260                 265                 270

Leu Lys Arg Arg Ile Val Ala Phe Ala Cys Val Pro Phe Ile Leu Gly
        275                 280                 285

Glu Leu Lys Ala Met Cys Glu Thr Ala Ser Gly Pro Asp Val Lys His
    290                 295                 300

His Met Gly Leu Arg Ala Glu Glu Lys Val Arg Leu Val Arg Ala Leu
305                 310                 315                 320

Gln Gln Leu Met Met Leu Glu Cys Gly Gly Ala Ala Leu Glu Ser Asp
                325                 330                 335

Val Thr Arg Trp Val Val Glu Asn Gly Ile Ser Val Met Val Gly Ile
            340                 345                 350

Gly Met Thr Glu Thr Val Gly Thr Leu Phe Ala Glu Arg Ala Gln Asp
        355                 360                 365

Ala Arg Ser Asn Gly Tyr Ser Ala Gln Asn Ala Leu Ile Thr Asp Gly
    370                 375                 380

Ile Met Ser Leu Val Gly Pro Asp Asn Glu Glu Val Thr Phe Glu Gly
385                 390                 395                 400

Glu Leu Val Val Lys Ser Lys Leu Ile Pro His Glu Tyr Ile Asn Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Lys Thr Leu Cys Ala Asn Pro Ser Ile Val Asn
465                 470                 475                 480

Ala Cys Val Ile Gly Asp Arg Phe Leu Arg Glu Pro Ala Thr Ser Val
                485                 490                 495

Cys Ala Ile Val Glu Ile Arg Pro Glu Val Asp Ile Pro Ser Ser Lys
            500                 505                 510

Ile Asp Arg Glu Ile Ala Asn Ala Leu Ala Pro Ile Asn Arg Asp Leu
        515                 520                 525

Pro Pro Ala Leu Arg Ile Ser Trp Ser Arg Val Leu Ile Ile Arg Pro
    530                 535                 540

Pro Gln Lys Ile Pro Val Thr Arg Lys Gly Asp Val Phe Arg Lys Lys
545                 550                 555                 560

Ile Glu Asp Val Phe Gly Ser Phe Leu Gly Val Gly Ser Thr Glu Ile
                565                 570                 575

Lys Val Asp His Glu Ser Lys Glu Asp Asp Thr Glu His Ile Val Arg
            580                 585                 590

Gln Val Val Ser Asn Leu Leu Gly Val His Asp Pro Glu Leu Leu Ser
        595                 600                 605

Ala Leu Ser Phe Ala Glu Leu Gly Met Thr Ser Phe Ile Ala Val Ser
    610                 615                 620

Ile Val Asn Ala Leu Asn Lys Arg Ile Ser Gly Leu Thr Leu Pro Pro
625                 630                 635                 640

Asn Ala Cys Tyr Leu His Ile Asp Leu Asp Ser Leu Val Asn Ala Ile
                645                 650                 655

Ser Arg Glu His Gly His Gly Thr Asn Pro Ala Glu Leu Pro Ser Lys
            660                 665                 670

Leu Phe Pro Val Thr Glu Ser Tyr Gln Arg Asn Asp Lys Asp Val Val
        675                 680                 685

Ile Ile Gly Lys Ala Phe Arg Leu Pro Gly Ser Leu Asp Asn Thr Ala
    690                 695                 700

Ser Leu Trp Glu Ala Leu Leu Ser Lys Asn Asn Ser Val Val Ser Asp
705                 710                 715                 720

Ile Pro Ser Asp Arg Trp Asp His Ala Ser Phe Tyr Pro His Asp Ile
                725                 730                 735

Cys Phe Thr Lys Ala Gly Leu Val Asp Val Ala His Tyr Asp Tyr Arg
            740                 745                 750

Phe Phe Gly Leu Thr Ala Thr Glu Ala Leu Tyr Val Ser Pro Thr Met
        755                 760                 765

Arg Leu Ala Leu Glu Val Ser Phe Glu Ala Leu Glu Asn Ala Asn Ile
    770                 775                 780

Pro Leu Ser Lys Leu Lys Gly Thr Gln Thr Pro Val Tyr Val Ala Thr
785                 790                 795                 800

Lys Asp Asp Gly Phe Glu Thr Leu Leu Asn Ala Glu Gln Gly Tyr Asp
                805                 810                 815

Ala Tyr Thr Arg Phe Tyr Gly Thr Gly Arg Ala Pro Ser Thr Ala Ser
            820                 825                 830

Gly Arg Ile Ser Tyr Leu Leu Asp Ile His Gly Pro Ser Val Thr Val
        835                 840                 845

Asp Thr Ala Cys Ser Gly Gly Ile Val Cys Met Asp Gln Ala Ile Thr
    850                 855                 860

Phe Leu Gln Ser Gly Arg Ala Asp Thr Ala Ile Val Cys Ser Ser Asn
865                 870                 875                 880

Thr His Cys Trp Pro Gly Ser Phe Met Phe Leu Thr Ala Gln Gly Met
                885                 890                 895

Val Ser Pro His Gly Arg Cys Ala Thr Phe Thr Thr Asp Ala Asp Gly
            900                 905                 910

Tyr Val Pro Ser Glu Gly Ala Val Ala Phe Ile Leu Lys Thr Arg Ser
        915                 920                 925

Ala Ala Ile Arg Asp Asn Asp Asn Ile Leu Ala Val Ile Lys Ser Thr
    930                 935                 940

Asp Val Ser His Asn Gly Arg Ser Gln Gly Leu Val Ala Pro Asn Val
945                 950                 955                 960

Lys Ala Gln Thr Ser Leu His Arg Ser Leu Leu Arg Lys Ala Gly Leu
                965                 970                 975

Phe Pro Asp Gln Ile Asn Phe Ile Glu Ala His Gly Thr Gly Thr Ser
            980                 985                 990

Leu Gly Asp Leu Ser Glu Ile Gln  Gly Ile Asn Asn Ala  Tyr Thr Ser
        995                 1000                1005

Thr Arg Pro Arg Leu Asp Gly Pro Leu Ile Ile Ser Ala Ser Lys
    1010                1015                1020

Thr Val Leu Gly His Ser Glu Pro Ile Ala Gly Met Ala Gly Ile
    1025                1030                1035

Leu Thr Ala Leu Leu Ser Leu Glu Lys Glu Thr Val Phe Gly Leu
    1040                1045                1050

Asn His Leu Thr Glu His Asn Leu Asn Pro Ser Leu Asp Cys Ser
    1055                1060                1065

Leu Val Pro Leu Leu Ile Pro His Glu Ser Ile His Ile Gly Gly
    1070                1075                1080

Glu Lys Pro His Arg Ala Ala Val Leu Ser Tyr Gly Phe Ala Gly
    1085                1090                1095

Thr Leu Ala Gly Ala Ile Leu Glu Gly Pro Pro Ser Asp Val Pro
    1100                1105                1110

Arg Pro Ser Ser Ser Asp Met Gln Glu His Pro Met Val Phe Val
    1115                1120                1125

Leu Ser Gly Lys Ser Val Pro Ala Leu Glu Thr Tyr Leu Arg Arg
    1130                1135                1140

Tyr Leu Ala Phe Leu Arg Ala Ala Lys Thr Asn Asp Phe His Ser
    1145                1150                1155

Ile Cys Tyr Thr Thr Cys Val Gly Arg Glu His Tyr Lys Tyr Arg
    1160                1165                1170

Phe Ser Cys Val Ala Gln Asn Met Ala Asp Leu Val Ser Gln Ile
    1175                1180                1185

Glu His Arg Leu Thr Thr Leu Ser Asn Ser Lys Gln Lys Pro Arg
    1190                1195                1200

Gly Ser Pro Gly Phe Met Phe Ser Gly Gln Gly Thr Tyr Phe Pro
    1205                1210                1215

Gly Met Ala Ala Ala Leu Ala Glu Gln Tyr Leu Gly Phe Arg Val
    1220                1225                1230

Leu Val Ser Arg Phe Gly Lys Ala Ala Gln Glu Arg Ser Gly Tyr
    1235                1240                1245

Pro Ile Asp Arg Leu Leu Leu Glu Val Ser Asp Thr Ser Ser Glu
    1250                1255                1260

Thr Asn Ser Glu Ala Asp Gln Ile Cys Ile Phe Val Tyr Gln Tyr
    1265                1270                1275

Ser Val Leu Gln Trp Leu Gln Ser Leu Gly Ile Gln Pro Lys Ala
    1280                1285                1290

Val Leu Gly His Ser Leu Gly Glu Ile Thr Ala Ala Val Ala Ala
    1295                1300                1305

Gly Ala Leu Ser Phe Glu Ser Ala Leu Asp Leu Val Val Thr Arg
    1310                1315                1320

Ala Arg Leu Leu Arg Pro Glu Thr Lys Asp Ser Ala Gly Met Ala
    1325                1330                1335

Ala Val Ala Ala Ser Lys Glu Glu Val Glu Glu Leu Ile Glu Asn
    1340                1345                1350

Leu Gln Leu Ala His Ala Leu Ser Val Ala Val His Asn Gly Pro
    1355                1360                1365

Arg Ser Val Val Val Ser Gly Ala Ser Thr Glu Ile Asp Ala Leu
    1370                1375                1380

Val Val Ala Ala Lys Glu Arg Gly Leu Lys Ala Ser Arg Leu Arg
    1385                1390                1395

Val Asp Gln Gly Phe His Ser Pro Tyr Val Asp Ser Ala Val Pro
    1400                1405                1410

Gly Leu Leu Asp Trp Ser Ser Glu His Cys Ser Thr Phe Leu Pro
    1415                1420                1425

Leu Asn Ile Pro Leu Tyr Ser Thr Leu Thr Gly Glu Val Ile Pro
    1430                1435                1440

Lys Gly Arg Arg Phe Ala Trp Asp His Trp Val Asn His Ala Arg
    1445                1450                1455

Lys Pro Val Gln Phe Ala Ala Ala Ala Ala Ala Val Asp Glu Asp
    1460                1465                1470

Arg Ser Ile Gly Val Leu Leu Asp Val Gly Pro Gln Pro Val Ala
    1475                1480                1485

Trp Thr Ile Leu Gln Ala Ser Ser Leu Phe Asn Thr Ser Ala Val
    1490                1495                1500

Ala Leu Phe Ala Lys Ala Gly Lys Asp Gln Glu Met Ala Leu Leu
    1505                1510                1515

Thr Thr Leu Ser Tyr Leu Phe Gln Glu His Asn Leu Cys Pro Asn
    1520                1525                1530

Phe His Glu Leu Tyr Ser Gln Arg His Gly Ala Leu Lys Lys Thr
    1535                1540                1545

Asp Ile Pro Thr Tyr Pro Phe Gln Arg Val His Arg Tyr Pro Thr
    1550                1555                1560

Phe Ile Pro Ser Arg Asn Gln Ser Pro Ala Val Ala Lys Val Val
    1565                1570                1575

Val Pro Ser Arg Phe Pro Val Gln Arg Lys Gly Glu Ala Ile Ser
    1580                1585                1590

Gln Ser Asn Glu Ser Asp Tyr Arg Ala Gly Leu Ile Thr Cys Leu
    1595                1600                1605

Arg Thr Ile Leu Glu Leu Thr Ser Glu Glu Glu Phe Asp Leu Ser
    1610                1615                1620

Glu Thr Leu Asn Ala Arg Gly Val Asp Ser Ile Met Phe Ser Gln
    1625                1630                1635

Leu Arg Lys Arg Val Gly Glu Glu Phe Asp Leu Asp Ile Pro Met
    1640                1645                1650

Ile Tyr Leu Ser Asp Val Phe Thr Met Glu Gln Met Ile Asp Tyr
    1655                1660                1665

Leu Val Glu Gln Ser Gly Ser Arg Pro Ala Pro Lys His Val Glu
    1670                1675                1680

Thr Pro Val Asn Glu Pro Leu Gly Lys Asp Leu Arg Thr Gly Leu
    1685                1690                1695

Val Ser Cys Leu Arg Asn Val Leu Glu Ile Thr Pro Asp Glu Glu
    1700                1705                1710

Leu Asp Leu Ser Glu Thr Leu Asn Ala Arg Gly Val Asp Ser Ile
    1715                1720                1725

Met Phe Ala Gln Leu Arg Lys Arg Val Gly Glu Gly Phe Gly Val
    1730                1735                1740

Glu Ile Pro Met Ile Tyr Leu Ser Asp Val Phe Thr Met Glu Asp
    1745                1750                1755

Met Ile Asn Phe Leu Val Ser Glu His Ala
    1760                1765

<210>  56
<211>  40
<212>  PRT
<213>  Artificial sequence

<220>
<223>  Consensus amino acid sequence of 1 hispidin synthases

<220>

<221>  MISC_FEATURE
<223>  X - any amino acid

<220>
<221>  MISC_FEATURE
<222>  (1)..(40)
<223>  X - any amino acid

<400>  56

Val Ala Xaa Xaa Xaa Glu Asn His Pro Xaa Xaa Xaa Ala Leu Xaa Xaa
1               5                   10                  15

Ala Val Trp Lys Xaa Xaa Gly Xaa Phe Xaa Pro Xaa Asp Xaa His Xaa
            20                  25                  30

Xaa Xaa Xaa Xaa Xaa Xaa Gly Met
        35                  40

<210>  57
<211>  36
<212>  PRT
<213>  Artificial sequence

<220>
<223>  Consensus amino acid sequence of 2 hispidin synthases

<220>
<221>  MISC_FEATURE
<222>  (1)..(36)
<223>  X - any amino acid

<400>  57

Leu Gly Trp Ala Pro Xaa Ser His Xaa Leu Xaa Xaa Met Gln Asp Ile
1               5                   10                  15

Gly Xaa Xaa Xaa Xaa Leu Xaa Ala Gly Cys Tyr Val Phe Xaa Xaa Xaa
            20                  25                  30

Pro Xaa Xaa Tyr
        35

<210>  58
<211>  33
<212>  PRT
<213>  Artificial sequence

<220>
<223>  Consensus amino acid sequence of 3 hispidin synthases

<220>
<221>  MISC_FEATURE
<222>  (6)..(33)
<223>  X - any amino acid

<400>  58

Glu Cys Gly Gly Ala Xaa Leu Xaa Xaa Xaa Xaa Xaa Xaa Trp Xaa Xaa
1               5                   10                  15

Xaa Xaa Xaa Xaa Xaa Xaa Xaa Xaa Gly Xaa Gly Met Thr Glu Thr Xaa
            20                  25                  30

Gly

<210>  59
<211>  8
<212>  PRT
<213>  Artificial sequence

<220>
<223>  Consensus amino acid sequence of 4 hispidin synthases

<400>  59

Phe Ala Glu Leu Gly Met Thr Ser
1               5

<210>  60
<211>  25
<212>  PRT
<213>  Artificial sequence

<220>
<223>  Consensus amino acid sequence of 5 hispidin synthases

<220>
<221>  MISC_FEATURE
<222>  (2)..(24)
<223>  X - any amino acid

<400>  60

Ile Xaa Xaa Xaa Arg Trp Asp His Xaa Ser Phe Tyr Pro Xaa Xaa Ile
1               5                   10                  15

Xaa Xaa Xaa Xaa Ala Gly Leu Xaa Asp
            20                  25

<210>  61
<211>  65
<212>  PRT
<213>  Artificial sequence

<220>
<223>  Consensus amino acid sequence of 6 hispidin synthases

<220>
<221>  MISC_FEATURE
<222>  (2)..(64)
<223>  X - any amino acid

<400>  61

Asp Xaa Xaa Phe Phe Gly Xaa Xaa Xaa Xaa Glu Ala Xaa Xaa Xaa Ser
1               5                   10                  15

Pro Thr Met Arg Xaa Ala Leu Glu Val Xaa Xaa Glu Ala Leu Glu Xaa
            20                  25                  30

Ala Asn Ile Pro Xaa Xaa Xaa Xaa Lys Gly Xaa Xaa Xaa Xaa Val Xaa
        35                  40                  45

Xaa Ala Xaa Xaa Asp Asp Gly Phe Glu Thr Leu Leu Xaa Ala Xaa Xaa
    50                  55                  60

Gly
65

<210>  62
<211>  50
<212>  PRT
<213>  Artificial sequence

<220>
<223>  Consensus amino acid sequence of 7 hispidin synthases

<220>

<221>  MISC_FEATURE
<222>  (5)..(47)
<223>  X - any amino acid

<400>  62

Ala Asp Gly Tyr Xaa Pro Ser Glu Gly Ala Val Xaa Phe Xaa Leu Lys
1               5                   10                  15

Thr Xaa Xaa Ala Ala Xaa Arg Asp Xaa Xaa Xaa Ile Xaa Ala Xaa Ile
            20                  25                  30

Xaa Xaa Xaa Xaa Xaa Xaa His Asn Gly Arg Ser Gln Gly Leu Xaa Ala
        35                  40                  45

Pro Asn
    50

<210>  63
<211>  9
<212>  PRT
<213>  Artificial sequence

<220>
<223>  Consensus amino acid sequence of 8 hispidin synthases

<220>
<221>  MISC_FEATURE
<222>  (8)..(8)
<223>  X - any amino acid

<400>  63

Gly His Ser Leu Gly Glu Ile Xaa Ala
1               5

<210>  64
<211>  867
<212>  DNA
<213>  Neonothopanus nambi

<400>  64
atggcgccaa tttcttcaac ttggtctcgt ctcattcgat ttgtggctat tgaaacgtcc      60

ctcgtgcata tcggtgaacc gatagacgcc accatggacg tcggtctggc gagacgagaa     120

ggcaagacga tccaagcata cgagattatt ggatcaggct cggctctaga cctctcagcc     180

caagtatcga agaatgtgct gactgtaagg gaactcctga tgccgctttc aagagaggaa 240

attaaaactg tacgatgctt ggggttgaac taccctgttc atgccaccga agcgaacgtt 300

gctgttccaa aattcccgaa tttgttctac aaaccagtga cctcgctcat tggccccgat 360

ggactcatta ccatcccttc cgttgtccaa cccccgaagg agcatcagtc cgattatgaa 420

gcggaacttg tcattgtcat cgggaaagca gcaaagaatg tatcggagga tgaggctttg 480

gattatgtat tgggatacac tgccgcgaac gatatttcgt ttaggaaaca ccagctagca 540

gtctcacaat ggtctttctc gaaaggattt ggtagccttc tactcactat ccgtatggca 600

caaacccact cgggtaacat taatcgcttc tccagagacc agattttcaa tgtcaagaag 660

acaatttcct tcctgtcaca aggcactaca ctggaaccag gttctatcat tttgactggt 720

acacctgacg gagtgggctt tgtgcgcaat ccaccacttt accttaaaga tggagatgaa 780

gtaatgacct ggattggaag tggaatcgga acattagcca atacagtgca agaagagaag 840

acttgcttcg ctagtggcgg acacgag 867

<210> 65
<211> 289
<212> PRT
<213> Neonothopanus nambi

<400> 65

Met Ala Pro Ile Ser Ser Thr Trp Ser Arg Leu Ile Arg Phe Val Ala
1               5                   10                  15

Ile Glu Thr Ser Leu Val His Ile Gly Glu Pro Ile Asp Ala Thr Met
            20                  25                  30

Asp Val Gly Leu Ala Arg Arg Glu Gly Lys Thr Ile Gln Ala Tyr Glu
        35                  40                  45

Ile Ile Gly Ser Gly Ser Ala Leu Asp Leu Ser Ala Gln Val Ser Lys
    50                  55                  60

Asn Val Leu Thr Val Arg Glu Leu Leu Met Pro Leu Ser Arg Glu Glu
65                  70                  75                  80

Ile Lys Thr Val Arg Cys Leu Gly Leu Asn Tyr Pro Val His Ala Thr
                85                  90                  95

Glu Ala Asn Val Ala Val Pro Lys Phe Pro Asn Leu Phe Tyr Lys Pro
            100                 105                 110

Val Thr Ser Leu Ile Gly Pro Asp Gly Leu Ile Thr Ile Pro Ser Val
        115                 120                 125

Val Gln Pro Pro Lys Glu His Gln Ser Asp Tyr Glu Ala Glu Leu Val
    130                 135                 140

Ile Val Ile Gly Lys Ala Ala Lys Asn Val Ser Glu Asp Glu Ala Leu
145                 150                 155                 160

Asp Tyr Val Leu Gly Tyr Thr Ala Ala Asn Asp Ile Ser Phe Arg Lys
                165                 170                 175

His Gln Leu Ala Val Ser Gln Trp Ser Phe Ser Lys Gly Phe Gly Ser
            180                 185                 190

Leu Leu Leu Thr Ile Arg Met Ala Gln Thr His Ser Gly Asn Ile Asn
        195                 200                 205

Arg Phe Ser Arg Asp Gln Ile Phe Asn Val Lys Lys Thr Ile Ser Phe
    210                 215                 220

Leu Ser Gln Gly Thr Thr Leu Glu Pro Gly Ser Ile Ile Leu Thr Gly
225                 230                 235                 240

Thr Pro Asp Gly Val Gly Phe Val Arg Asn Pro Pro Leu Tyr Leu Lys
                245                 250                 255

Asp Gly Asp Glu Val Met Thr Trp Ile Gly Ser Gly Ile Gly Thr Leu
            260                 265                 270

Ala Asn Thr Val Gln Glu Glu Lys Thr Cys Phe Ala Ser Gly Gly His
        275                 280                 285

Glu

<210>  66
<211>  963
<212>  DNA
<213>  Neonothopanus gardneri

<400>  66
atggcgccga ttttgacagt gagttccagt tcaatggtgc tgaatagctc aaaacaggct      60

atattgaata tttctgaata tcaccagccc tggactcgtc tcattcgatt tgtagccgtt     120

gagacgtcac tcgtgcatat tggtgaaccc atcgaggtga ctttggacgt cgggcaggca     180

aaatgtgaag gcaagacgat caaagcgtac gagattattg gatcagggtc ggccttggac     240

ctctcagctc aggtatcgaa gaatgtgcta accgtaaagg aactcttgat gccgctttcg     300

agagaagagg tcaagactgt gcggtgcttg ggactgaact attttactca tgcttccacc     360

gggcgcccgc tgtcgactag attaccgact ttgttctata agccagtgac ttcactcatc     420

ggacctgagg cgttcattaa tattccttcc gctgttcaac caccgaagga gcatcagtcc     480

gattatgaag cggagttggt aattattatt gggagagcgg cgaaggatgt accggaagag     540

gaggctttga attatgtttt gggatacacc gccgccaacg acatttcatt taggaaatat     600

caatttgcag tttcccagtg gtgtttttcg aaaggtttcg ataatacaaa cccaatcggt     660

ccgtgcatcg tttccgcatc ttccattccg aacccgcaag acatccagat ccaatgcaaa     720

ctgaacggga atgtcgttca gaatggaaac accagtgatc aaatttttaa tatcaagaaa     780

acagtcgctt ttttgtcgca aggaacaaca cttgagtcag gatcaatcat cctgaccggt     840

acgcctggcg gagtgggatt tgtgcgcgat ccaccgcttt accttaaaga tggagatgaa     900

gtagtgactt ggattggaag tggggttgga agtttagtca acgtagtaaa agaagagaag     960

act                                                                   963


<210>  67
<211>  321
<212>  PRT
<213>  Neonothopanus gardneri

<400>  67

Met Ala Pro Ile Leu Thr Val Ser Ser Ser Ser Met Val Leu Asn Ser
1               5                   10                  15


Ser Lys Gln Ala Ile Leu Asn Ile Ser Glu Tyr His Gln Pro Trp Thr
            20                  25                  30

Arg Leu Ile Arg Phe Val Ala Val Glu Thr Ser Leu Val His Ile Gly
        35                  40                  45

Glu Pro Ile Glu Val Thr Leu Asp Val Gly Gln Ala Lys Cys Glu Gly
    50                  55                  60

Lys Thr Ile Lys Ala Tyr Glu Ile Ile Gly Ser Gly Ser Ala Leu Asp
65                  70                  75                  80

Leu Ser Ala Gln Val Ser Lys Asn Val Leu Thr Val Lys Glu Leu Leu
                85                  90                  95

Met Pro Leu Ser Arg Glu Glu Val Lys Thr Val Arg Cys Leu Gly Leu
            100                 105                 110

Asn Tyr Phe Thr His Ala Ser Thr Gly Arg Pro Leu Ser Thr Arg Leu
        115                 120                 125

Pro Thr Leu Phe Tyr Lys Pro Val Thr Ser Leu Ile Gly Pro Glu Ala
    130                 135                 140

Phe Ile Asn Ile Pro Ser Ala Val Gln Pro Pro Lys Glu His Gln Ser
145                 150                 155                 160

Asp Tyr Glu Ala Glu Leu Val Ile Ile Ile Gly Arg Ala Ala Lys Asp
                165                 170                 175

Val Pro Glu Glu Glu Ala Leu Asn Tyr Val Leu Gly Tyr Thr Ala Ala
            180                 185                 190

Asn Asp Ile Ser Phe Arg Lys Tyr Gln Phe Ala Val Ser Gln Trp Cys
        195                 200                 205

Phe Ser Lys Gly Phe Asp Asn Thr Asn Pro Ile Gly Pro Cys Ile Val
    210                 215                 220

Ser Ala Ser Ser Ile Pro Asn Pro Gln Asp Ile Gln Ile Gln Cys Lys
225                 230                 235                 240

Leu Asn Gly Asn Val Val Gln Asn Gly Asn Thr Ser Asp Gln Ile Phe
                245                 250                 255

Asn Ile Lys Lys Thr Val Ala Phe Leu Ser Gln Gly Thr Thr Leu Glu
            260                 265                 270

Ser Gly Ser Ile Ile Leu Thr Gly Thr Pro Gly Gly Val Gly Phe Val
        275                 280                 285

Arg Asp Pro Pro Leu Tyr Leu Lys Asp Gly Asp Glu Val Val Thr Trp
    290                 295                 300

Ile Gly Ser Gly Val Gly Ser Leu Val Asn Val Val Lys Glu Glu Lys
305                 310                 315                 320

Thr

<210>  68
<211>  906
<212>  DNA
<213>  Armillaria gallica

<400>  68
atggcgccta ttatcactca atggtccaga cttatccgct ttgtcgccgt cgaaacctct      60

cgtgtacata ttggacagcc cgtagattct aacctggatg ttggtctagc ggcgtaccag     120

ggaatgctaa tcaaggctta cgaaatactt ggttctgctc tcgatccatc cgcccaaatg     180

actagcaaga tcctcactgt caaacaacta ttaaccccgc tgtctggcga agatgtcaag     240

gtcgttagat gcttgggtct caactaccca gcacatgcga atgaagggaa agtagaagca     300

cctaagtttc ctaacctttt ctataagcca gtgacgtcgc ttatcgggcc cctcgcacct     360

gtgatcattc ctgcagtcgc acaaccttct gcaatacatc aatccgatta tgaggctgaa     420

tttactgtcg tcataggcag ggcagctaag aatatcactg aagcggaagc tttagactat     480

gttctcggct acaccggcgg caatgacgtg tcttttcgtc agcatcaatt tgcggtctct     540

caatggtgtt tctccaaaag ttttgacaac acaaatccct ttgggccatg cttagttgcc     600

gcatcgtcta ttcccgaccc tcaaactgtg gccattaagt ttacactgaa tggtcaaact     660

gtccaagacg gaactactgc cgatcaactt ttcagcgtca aaaagaccat agcttatctt     720

tctcaaggca cgacgttaca gccgggctcc ataattatga ctggtactcc cagtggcgtt 780

ggattcgtcc gaaacccacc tctctacctc aaagatggag accatatgtt gacttggata 840

agcggtggaa tcggtacgct tgcaaacagc gtcgtggagg agaagactcc gactcctggt 900

ttagat 906

<210> 69
<211> 302
<212> PRT
<213> Armillaria gallica

<400> 69

Met Ala Pro Ile Ile Thr Gln Trp Ser Arg Leu Ile Arg Phe Val Ala
1               5                   10                  15

Val Glu Thr Ser Arg Val His Ile Gly Gln Pro Val Asp Ser Asn Leu
            20                  25                  30

Asp Val Gly Leu Ala Ala Tyr Gln Gly Met Leu Ile Lys Ala Tyr Glu
        35                  40                  45

Ile Leu Gly Ser Ala Leu Asp Pro Ser Ala Gln Met Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ser Ala Ile His Gln Ser Asp Tyr Glu Ala Glu Phe Thr Val Val
    130                 135                 140

Ile Gly Arg Ala Ala Lys Asn Ile Thr Glu Ala Glu Ala Leu Asp Tyr
145                 150                 155                 160

Val Leu Gly Tyr Thr Gly Gly Asn Asp Val Ser Phe Arg Gln His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Thr Val Ala Ile Lys Phe Thr Leu Asn Gly Gln Thr Val Gln Asp Gly
    210                 215                 220

Thr Thr Ala Asp Gln Leu Phe Ser Val Lys Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Ser Gly Val Gly Phe Val Arg Asn Pro Pro Leu Tyr Leu Lys Asp
            260                 265                 270

Gly Asp His Met Leu Thr Trp Ile Ser Gly Gly Ile Gly Thr Leu Ala
        275                 280                 285

Asn Ser Val Val Glu Glu Lys Thr Pro Thr Pro Gly Leu Asp
    290                 295                 300

<210>  70
<211>  906
<212>  DNA
<213>  Armillaria ostoyae

<400>  70
atggcaccta ttattactca atggtccaga cttattcgct ttgttgccgt cgagacctct      60

cgtgtacata ttggacagcc catagattct accctggata ttggtctagc ggcgtaccag     120

ggaatgctaa tcaaggctta tgaaatactt ggttctgctc tcgatccatc cgcccaaatg     180

accagcaaga tcctcaccgt taaacagcta ttaactccgc tgtctggcga agatgtcaag     240

gtcgtccgat gcttgggtct taactatcca gctcatgcga atgaagggaa agtagaagcg     300

cctaagtttc ctaacctttt ctataagcca gtgacatcgc ttatcgggcc cctcgcacct 360

gtgatcattc ctgcagtcgc acagccttct gcaatacatc aatccgatta tgaggctgaa 420

tttactgtcg tcataggcag ggcagctaag aatgtcactg aagcggaagc tttagactat 480

gttctcgggt acaccggcgg caatgatgtg tcttttcgtc agcatcaatt tgcggtctct 540

caatggtgtt tctctaaaag ttttgacaat acaaatccct tcggtccatg cttagttgcc 600

gcatcgtcta ttcctgatcc tcaaactgtg gccattaagt ttacattgaa tggtgacacc 660

gtccaagacg gaactactgc tgatcaactt ttcagcgtca aaaagaccat cgcttatctt 720

tctcagggca cgacgttaca gccgggctcc ataattatga ctggcactcc cagtggtgtt 780

gggttcgtcc aaaacccacc tctctacctc aaagatgggg atcaaatgtt gacttggata 840

agcggtggaa tcggtacgct tgcaaacaac gtcgtagagg agaagactcc gactcctcgt 900

ttagac 906

<210> 71
<211> 302
<212> PRT
<213> Armillaria ostoyae

<400> 71

Met Ala Pro Ile Ile Thr Gln Trp Ser Arg Leu Ile Arg Phe Val Ala
1               5                   10                  15

Val Glu Thr Ser Arg Val His Ile Gly Gln Pro Ile Asp Ser Thr Leu
            20                  25                  30

Asp Ile Gly Leu Ala Ala Tyr Gln Gly Met Leu Ile Lys Ala Tyr Glu
        35                  40                  45

Ile Leu Gly Ser Ala Leu Asp Pro Ser Ala Gln Met Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ser Ala Ile His Gln Ser Asp Tyr Glu Ala Glu Phe Thr Val Val
    130                 135                 140

Ile Gly Arg Ala Ala Lys Asn Val Thr Glu Ala Glu Ala Leu Asp Tyr
145                 150                 155                 160

Val Leu Gly Tyr Thr Gly Gly Asn Asp Val Ser Phe Arg Gln His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Thr Val Ala Ile Lys Phe Thr Leu Asn Gly Asp Thr Val Gln Asp Gly
    210                 215                 220

Thr Thr Ala Asp Gln Leu Phe Ser Val Lys Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Ser Gly Val Gly Phe Val Gln Asn Pro Pro Leu Tyr Leu Lys Asp
            260                 265                 270

Gly Asp Gln Met Leu Thr Trp Ile Ser Gly Gly Ile Gly Thr Leu Ala
        275                 280                 285

Asn Asn Val Val Glu Glu Lys Thr Pro Thr Pro Arg Leu Asp
    290                 295                 300

<210> 72
<211> 891

<212>  DNA
<213>  Armillaria mellea

<400>  72
atggcgccta ttatcactca gtggtccaga cttattcgct ttgttgccgt cgagacttct      60

cgtgtacatt ttggacagcc cgtagattct accctggatg ttggtctagc ggcgtaccag     120

ggtgtgttga tcaaggctta tgaaatactt ggttctgctc ttgatccatc cgcccaaatg     180

accagcaaga tcctcaccgt gaaacagcta ttaactccgc tgtctggcga ggatgtcaaa     240

gtcgtccgat gcttgggtct taactatcca gcacatgcga atgaagggaa agtagaagca     300

cccaagtttc ctaacctgtt ctataagcca gtgacatcgc ttatcgggcc cctcgcgcct     360

gtgatcattc ctgcagtcgc acagccttct gcaatacatc aatctgatta tgaggctgaa     420

tttactgttg tcctaggcag ggcagctaag aatgtcactg aagctgaagc cttggactat     480

gttctcggtt acaccggcgg caatgatgtg tcttttcggc agcatcaatt tgctgtctct     540

caatggtgtt tctccaaaag ttttgacaat acaaatccct tcggtccatg cttagttgcc     600

gcgtcgtcta ttcctgatcc tcaaactgtg gccattaagt ttacattgaa tggcaacacc     660

gtccaagatg gaactactgc tgatcaactt ttcagcgtca gaaagaccat cgcttatctt     720

tctcaaggca cgacgttaca gcctggctcc ataattatga ccggtactcc cagtggcgtt     780

gggttcgtcc gaaacccacc tctctacctc aaagatgggg atcaaatgtt gacttggatt     840

agcggtggaa tcggtacgct tgcaaacagc gtcatagagg agaagacccc a              891


<210>  73
<211>  297
<212>  PRT
<213>  Armillaria mellea

<400>  73

Met Ala Pro Ile Ile Thr Gln Trp Ser Arg Leu Ile Arg Phe Val Ala
1               5                   10                  15


Val Glu Thr Ser Arg Val His Phe Gly Gln Pro Val Asp Ser Thr Leu
            20                  25                  30


Asp Val Gly Leu Ala Ala Tyr Gln Gly Val Leu Ile Lys Ala Tyr Glu
        35                  40                  45

Ile Leu Gly Ser Ala Leu Asp Pro Ser Ala Gln Met Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ser Ala Ile His Gln Ser Asp Tyr Glu Ala Glu Phe Thr Val Val
    130                 135                 140

Leu Gly Arg Ala Ala Lys Asn Val Thr Glu Ala Glu Ala Leu Asp Tyr
145                 150                 155                 160

Val Leu Gly Tyr Thr Gly Gly Asn Asp Val Ser Phe Arg Gln His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Thr Val Ala Ile Lys Phe Thr Leu Asn Gly Asn Thr Val Gln Asp Gly
    210                 215                 220

Thr Thr Ala Asp Gln Leu Phe Ser Val Arg Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Ser Gly Val Gly Phe Val Arg Asn Pro Pro Leu Tyr Leu Lys Asp
            260                 265                 270

Gly Asp Gln Met Leu Thr Trp Ile Ser Gly Gly Ile Gly Thr Leu Ala
        275                 280                 285

Asn Ser Val Ile Glu Glu Lys Thr Pro
    290                 295

<210>  74
<211>  891
<212>  DNA
<213>  Armillaria fuscipes

<400>  74
atggcaccta ttatcactca atggtccaga cttattcgct ttgtctccat tgagacttct      60

ggtgttcata ttggacaacc cgtagatcct acccttgacg tcggtctagc ggcgtcccag     120

ggagtggcaa tcaaggttta tgaaataatt ggttctgcgc ttgatccatc cgcccaagtg     180

accagcaaaa tccttaccgt caaacagcta ttaactccgc tgtctggtga ggatgtcaag     240

gtcgtccggt gcttgggtct gaactatcca gcacatgcca atgaaggaaa agtagaagca     300

cccaagtttc ctaacctttt ctataagcca gtgacatcgc ttattgggcc cctcgcgcct     360

gtgatcattc ctgcagtcgc acagccggcc gcaatacatc aatctgatta tgaggctgaa     420

tttactgttg tcattggcag ggcagccaag aatgtcacgg aagctgaagc cctggactat     480

gttcttggct acaccggcgg taatgatgtg tcttttcgga agcatcaatt tgcagtctct     540

caatggtgtt tctccaaaag ttttgacaat acaaatccct ttggaccatg cttagttgcc     600

gcttcgtcta tccctgatcc tcagaatgtg gccattaagt tcacgttgaa tggtaacacc     660

gttcaagatg gaactactgc tgaccaaatt ttcagcgtta gaaagactat cgcttatctt     720

tctcaaggca cgacgttaca gccaggctcc atcattatga ctggcactcc caatggcgtt     780

gggtttgtcc gagacccacc tctctacctc aaagacgggg atcaaatgct gacttggatt     840

agcggtggaa ttggtacgct tgcgaatggc gtcgtagaag agaagacccg g              891

<210>  75
<211>  297
<212>  PRT
<213>  Armillaria fuscipes

<400>  75

Met Ala Pro Ile Ile Thr Gln Trp Ser Arg Leu Ile Arg Phe Val Ser
1               5                   10                  15

Ile Glu Thr Ser Gly Val His Ile Gly Gln Pro Val Asp Pro Thr Leu
            20                  25                  30

Asp Val Gly Leu Ala Ala Ser Gln Gly Val Ala Ile Lys Val Tyr Glu
        35                  40                  45

Ile Ile Gly Ser Ala Leu Asp Pro Ser Ala Gln Val Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ala Ala Ile His Gln Ser Asp Tyr Glu Ala Glu Phe Thr Val Val
    130                 135                 140

Ile Gly Arg Ala Ala Lys Asn Val Thr Glu Ala Glu Ala Leu Asp Tyr
145                 150                 155                 160

Val Leu Gly Tyr Thr Gly Gly Asn Asp Val Ser Phe Arg Lys His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Asn Val Ala Ile Lys Phe Thr Leu Asn Gly Asn Thr Val Gln Asp Gly
    210                 215                 220

Thr Thr Ala Asp Gln Ile Phe Ser Val Arg Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Asn Gly Val Gly Phe Val Arg Asp Pro Pro Leu Tyr Leu Lys Asp
            260                 265                 270

Gly Asp Gln Met Leu Thr Trp Ile Ser Gly Gly Ile Gly Thr Leu Ala
        275                 280                 285

Asn Gly Val Val Glu Glu Lys Thr Arg
    290                 295

<210>  76
<211>  42
<212>  PRT
<213>  Artificial sequence

<220>
<223>  Consensus amino acid sequence 1
       caffeylpyruvate hydrolase

<220>
<221>  MISC_FEATURE
<222>  (2)..(39)
<223>  X - any amino acid

<400>  76

Trp Xaa Arg Leu Ile Arg Phe Val Xaa Xaa Glu Thr Ser Xaa Val His
1               5                   10                  15

Xaa Gly Xaa Pro Xaa Xaa Xaa Xaa Xaa Asp Xaa Gly Xaa Ala Xaa Xaa
            20                  25                  30

Xaa Gly Xaa Xaa Ile Xaa Xaa Tyr Glu Ile
        35                  40

<210>  77
<211>  137
<212>  PRT

<213>  Artificial sequence

<220>
<223>  Consensus amino acid sequence 2
       caffeylpyruvate hydrolase

<220>
<221>  MISC_FEATURE
<222>  (5)..(136)
<223>  X - any amino acid

<400>  77

Ser Ala Leu Asp Xaa Ser Ala Gln Xaa Xaa Xaa Xaa Xaa Leu Thr Val
1               5                   10                  15

Xaa Xaa Leu Leu Xaa Pro Leu Ser Xaa Glu Xaa Xaa Lys Xaa Val Arg
            20                  25                  30

Cys Leu Gly Leu Asn Tyr Xaa Xaa His Ala Xaa Xaa Xaa Xaa Xaa Xaa
        35                  40                  45

Xaa Xaa Xaa Xaa Pro Xaa Leu Phe Tyr Lys Pro Val Thr Ser Leu Ile
    50                  55                  60

Gly Pro Xaa Xaa Xaa Xaa Xaa Ile Pro Xaa Xaa Xaa Gln Pro Xaa Xaa
65                  70                  75                  80

Xaa His Gln Ser Asp Tyr Glu Ala Glu Xaa Xaa Xaa Xaa Xaa Gly Xaa
                85                  90                  95

Ala Ala Lys Xaa Xaa Xaa Glu Xaa Glu Ala Leu Xaa Tyr Val Leu Gly
            100                 105                 110

Tyr Thr Xaa Xaa Asn Asp Xaa Ser Phe Arg Xaa Xaa Gln Xaa Ala Val
        115                 120                 125

Ser Gln Trp Xaa Phe Ser Lys Xaa Phe
    130                 135

<210>  78
<211>  69
<212>  PRT
<213>  Artificial sequence

<220>
<223>  Consensus amino acid sequence 3
       caffeylpyruvate hydrolase

<220>
<221>  MISC_FEATURE
<222>  (3)..(65)
<223>  X - any amino acid

<400>  78

Asp Gln Xaa Phe Xaa Xaa Xaa Lys Thr Xaa Xaa Xaa Leu Ser Gln Gly
1               5                   10                  15

Thr Thr Leu Xaa Xaa Gly Ser Ile Ile Xaa Thr Gly Thr Pro Xaa Gly
            20                  25                  30

Val Gly Phe Val Xaa Xaa Pro Pro Leu Tyr Leu Lys Asp Gly Asp Xaa
        35                  40                  45

Xaa Xaa Thr Trp Ile Xaa Xaa Gly Xaa Gly Xaa Leu Xaa Asn Xaa Val
    50                  55                  60

Xaa Glu Glu Lys Thr
65

<210>  79
<211>  801
<212>  DNA
<213>  Neonothopanus nambi

<400>  79
atgcgcatta acattagcct ctcgtctctc ttcgaacgtc tctccaaact tagcagtcgc      60

agcatagcga ttacatgtgg agttgttctc gcctccgcaa tcgcctttcc catcatccgc     120

agagactacc agactttcct agaagtggga ccctcgtacg ctccgcagaa ctttagagga     180

tacatcatcg tctgtgtcct ctcgctattc cgccaagagc agaaagggct cgccatctat     240

gatcgtcttc ccgagaaacg caggtggttg gccgaccttc cctttcgtga aggaaccaga     300

cccagcatta ccagccatat cattcagcga cagcgcactc aactggtcga tcaggagttt     360

gccaccaggg agctcataga caaggtcatc cctcgcgtgc aagcacgaca caccgacaaa     420

acgttcctca gcacatcaaa gttcgagttt catgcgaagg ccatatttct cttgccttct     480

atcccaatca acgaccctct gaatatccct agccacgaca ctgtccgccg aacgaagcgc 540

gagattgcac atatgcatga ttatcatgat tgcacacttc atcttgctct cgctgcgcag 600

gatggaaagg aggtgctgaa gaaaggttgg ggacaacgac atcctttggc tggtcctgga 660

gttcctggtc caccaacgga atggactttt ctttatgcgc ctcgcaacga agaagaggct 720

cgagtagtgg agatgatcgt tgaggcttcc atagggtata tgacgaacga tcctgcagga 780

aagattgtag aaaacgccaa g 801

<210> 80
<211> 267
<212> PRT
<213> Neonothopanus nambi

<400> 80

Met Arg Ile Asn Ile Ser Leu Ser Ser Leu Phe Glu Arg Leu Ser Lys
1               5                   10                  15

Leu Ser Ser Arg Ser Ile Ala Ile Thr Cys Gly Val Val Leu Ala Ser
            20                  25                  30

Ala Ile Ala Phe Pro Ile Ile Arg Arg Asp Tyr Gln Thr Phe Leu Glu
        35                  40                  45

Val Gly Pro Ser Tyr Ala Pro Gln Asn Phe Arg Gly Tyr Ile Ile Val
    50                  55                  60

Cys Val Leu Ser Leu Phe Arg Gln Glu Gln Lys Gly Leu Ala Ile Tyr
65                  70                  75                  80

Asp Arg Leu Pro Glu Lys Arg Arg Trp Leu Ala Asp Leu Pro Phe Arg
                85                  90                  95

Glu Gly Thr Arg Pro Ser Ile Thr Ser His Ile Ile Gln Arg Gln Arg
            100                 105                 110

Thr Gln Leu Val Asp Gln Glu Phe Ala Thr Arg Glu Leu Ile Asp Lys
        115                 120                 125

Val Ile Pro Arg Val Gln Ala Arg His Thr Asp Lys Thr Phe Leu Ser

130                 135                 140

Thr Ser Lys Phe Glu Phe His Ala Lys Ala Ile Phe Leu Leu Pro Ser
145                 150                 155                 160

Ile Pro Ile Asn Asp Pro Leu Asn Ile Pro Ser His Asp Thr Val Arg
                165                 170                 175

Arg Thr Lys Arg Glu Ile Ala His Met His Asp Tyr His Asp Cys Thr
            180                 185                 190

Leu His Leu Ala Leu Ala Ala Gln Asp Gly Lys Glu Val Leu Lys Lys
        195                 200                 205

Gly Trp Gly Gln Arg His Pro Leu Ala Gly Pro Gly Val Pro Gly Pro
    210                 215                 220

Pro Thr Glu Trp Thr Phe Leu Tyr Ala Pro Arg Asn Glu Glu Glu Ala
225                 230                 235                 240

Arg Val Val Glu Met Ile Val Glu Ala Ser Ile Gly Tyr Met Thr Asn
                245                 250                 255

Asp Pro Ala Gly Lys Ile Val Glu Asn Ala Lys
            260                 265

<210>  81
<211>  783
<212>  DNA
<213>  Omphalotus olearius

<400>  81
atgctcccag ctttcatcta caaaccaagg ctagtgatca cttgtgtatt cgttctggcc      60

tccgcactcg catttccctt catacgcaaa gattaccaga ctttcctgga ggtgggaccc     120

tcgtacgccc cgcagaacct ccaaggatac atcatcgtct gtgtactctc tctgttccgg     180

caagaacaga aagacgtagc gatttatgat cgccttcctg agaaaaggag gtggttagga     240

gacctcccgt ttcgcgaggg gccaagaccg agtatcacta gccatatcat ccagcgacag     300

cgcacccaat tggctgacgc cgagttcgct accaaagagc tgataggcaa aatcatccct     360

cgcgtccaag cccgacacac caacacaaca ttcctcagca catctaaatt cgaattccac     420

gcccaggcca tcttcctttt gccctctatc ccaatcaacg accctcaaaa cattccaagc 480

cacgataccg ttcgtcgcac gaaacgcgag atcgcgcata tgcatgatta tcacgactgt 540

acgttgcatc tcgcacttgc tgctcaagat gggaaggagg ttttagagaa aggatggggt 600

cagcgacatc ctcttgctgg acctggtgtt cctggcccgc cgacggagtg gacgtttctt 660

tatgcaccgc gcagcgaaga ggaggttcgg gttgtggaga tgattgttga ggcatcagtt 720

gtgtatatga cgaatgatcc tgcggataaa atcgtagaag ctactgtgca gggtactgaa 780

gaa 783

<210> 82
<211> 261
<212> PRT
<213> Omphalotus olearius

<400> 82

Met Leu Pro Ala Phe Ile Tyr Lys Pro Arg Leu Val Ile Thr Cys Val
1               5                   10                  15

Phe Val Leu Ala Ser Ala Leu Ala Phe Pro Phe Ile Arg Lys Asp Tyr
            20                  25                  30

Gln Thr Phe Leu Glu Val Gly Pro Ser Tyr Ala Pro Gln Asn Leu Gln
        35                  40                  45

Gly Tyr Ile Ile Val Cys Val Leu Ser Leu Phe Arg Gln Glu Gln Lys
    50                  55                  60

Asp Val Ala Ile Tyr Asp Arg Leu Pro Glu Lys Arg Arg Trp Leu Gly
65                  70                  75                  80

Asp Leu Pro Phe Arg Glu Gly Pro Arg Pro Ser Ile Thr Ser His Ile
                85                  90                  95

Ile Gln Arg Gln Arg Thr Gln Leu Ala Asp Ala Glu Phe Ala Thr Lys
            100                 105                 110

Glu Leu Ile Gly Lys Ile Ile Pro Arg Val Gln Ala Arg His Thr Asn
        115                 120                 125

Thr Thr Phe Leu Ser Thr Ser Lys Phe Glu Phe His Ala Gln Ala Ile
    130                 135                 140

Phe Leu Leu Pro Ser Ile Pro Ile Asn Asp Pro Gln Asn Ile Pro Ser
145                 150                 155                 160

His Asp Thr Val Arg Arg Thr Lys Arg Glu Ile Ala His Met His Asp
                165                 170                 175

Tyr His Asp Cys Thr Leu His Leu Ala Leu Ala Ala Gln Asp Gly Lys
            180                 185                 190

Glu Val Leu Glu Lys Gly Trp Gly Gln Arg His Pro Leu Ala Gly Pro
        195                 200                 205

Gly Val Pro Gly Pro Pro Thr Glu Trp Thr Phe Leu Tyr Ala Pro Arg
    210                 215                 220

Ser Glu Glu Glu Val Arg Val Val Glu Met Ile Val Glu Ala Ser Val
225                 230                 235                 240

Val Tyr Met Thr Asn Asp Pro Ala Asp Lys Ile Val Glu Ala Thr Val
                245                 250                 255

Gln Gly Thr Glu Glu
            260

<210>  83
<211>  798
<212>  DNA
<213>  Armillaria gallica

<400>  83
atgtccttca tcgacagcat gaaacttgac ctcgtcggac acctctttgg catcaggaat      60

cgcggcttag ccgccgcttg ttgtgctcta gcagtcgcct ctactatcgc cttcccttac     120

attcgtaggg actaccagac atttttatct ggcggtccct cttacgctcc ccagaatatc     180

agaggatatt tcatcgtctg cgttctggcc ttgttccgtc aggagcaaaa gggccttgcg     240

atatatgatc gccttcccga gaagcgcagg tggctgcctg acttgcctcc tcgcaatggc     300

ccgcggccga tcacgaccag ccatataatc caaagacagc gcaaccaggc gccggacccc     360

aagttcgccc tcgaggaact caaggccacg gttattccac gggtgcaggc tcgccatact 420

gacctcaccc atctcagcct atccaaattc gagttccatg ctgaagcaat tttcctgctc 480

ccctctgtac ccatcgatga tccaaaaaat gttccaagtc acgacacggt gcgcaggacg 540

aaaagggaga tcgcgcatat gcacgactac catgacttca cgctgcatct tgcactggcc 600

gcccaagacg ggaaggaagt cgtgtcgaag ggatgggggc agcgacaccc cctagcaggc 660

cctggcgttc ctggtccacc tacggagtgg acatttattt atgcgccacg taacgaagag 720

gaactggcag tggtggaaat gattatcgag gcatcaatag gctatatgac caatgaccct 780

gctggagtag ttatcgca 798

<210> 84
<211> 266
<212> PRT
<213> Armillaria gallica

<400> 84

Met Ser Phe Ile Asp Ser Met Lys Leu Asp Leu Val Gly His Leu Phe
1               5                   10                  15

Gly Ile Arg Asn Arg Gly Leu Ala Ala Ala Cys Cys Ala Leu Ala Val
            20                  25                  30

Ala Ser Thr Ile Ala Phe Pro Tyr Ile Arg Arg Asp Tyr Gln Thr Phe
        35                  40                  45

Leu Ser Gly Gly Pro Ser Tyr Ala Pro Gln Asn Ile Arg Gly Tyr Phe
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Pro Asp Leu Pro
                85                  90                  95

Pro Arg Asn Gly Pro Arg Pro Ile Thr Thr Ser His Ile Ile Gln Arg
            100                 105                 110

Gln Arg Asn Gln Ala Pro Asp Pro Lys Phe Ala Leu Glu Glu Leu Lys

115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Val Pro Ser His Asp Thr
                165                 170                 175

Val Arg Arg Thr Lys Arg Glu Ile Ala His Met His Asp Tyr His Asp
            180                 185                 190

Phe Thr Leu His Leu Ala Leu Ala Ala Gln Asp Gly Lys Glu Val Val
        195                 200                 205

Ser Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Val Val Ile Ala
            260                 265

<210>  85
<211>  798
<212>  DNA
<213>  Armillaria ostoyae

<400>  85
atgtccttca tcgacagcat gaaacttgac ttcgtcggac acctctttgg catcaggaat        60

cgcggcttag ccaccgcttg ttgtgctgtg gcagtcgctt ctgccatcgc cttcccttac       120

attcgtaggg actaccagac attcttatct ggcggtccct cttacgctcc ccagaacatc       180

aaaggatatc tcatcgtctg cgtcctggcc ttgttccgtc aggagcaaaa gggccttgcg       240

atatatgacc gccttcccga gaagcgcagg tggctacctg acttgcctcc tcgcaatggc       300

ccgcggccca tcacgaccag ccatataatc caaagacagc gcaaccaggc gccagactcc 360

aagttcgccc tcgaggaact caaggctacg gtcattccac gggtgcaggc tcgccacact 420

gacctcaccc atctcagcct atccaagttc gagttccatg ctgaagcaat cttcctgctc 480

ccctctgtac ccatcgatga tccaaaaaat gttccaagtc atgacacggt gcgcaggacg 540

aagagggaga tcgcgcatat gcacgactac catgacttta cgttgcatct tgcactggcc 600

gcccaagacg ggaaggaagt cgtggcgaag ggatgggggc agcgacaccc gctggcaggc 660

cctggcgttc ctggtccacc tacggagtgg acgtttattt atgcgccacg taacgaagag 720

gaactggcag tggtggaaat gattatcgag gcatcaatag gctatatgac caatgaccct 780

gctggaacag ttatcgta 798

<210> 86
<211> 266
<212> PRT
<213> Armillaria ostoyae

<400> 86

Met Ser Phe Ile Asp Ser Met Lys Leu Asp Phe Val Gly His Leu Phe
1               5                   10                  15

Gly Ile Arg Asn Arg Gly Leu Ala Thr Ala Cys Cys Ala Val Ala Val
            20                  25                  30

Ala Ser Ala Ile Ala Phe Pro Tyr Ile Arg Arg Asp Tyr Gln Thr Phe
        35                  40                  45

Leu Ser Gly Gly Pro Ser Tyr Ala Pro Gln Asn Ile Lys Gly Tyr Leu
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Pro Asp Leu Pro
                85                  90                  95

Pro Arg Asn Gly Pro Arg Pro Ile Thr Thr Ser His Ile Ile Gln Arg
            100                 105                 110

Gln Arg Asn Gln Ala Pro Asp Ser Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Val Pro Ser His Asp Thr
                165                 170                 175

Val Arg Arg Thr Lys Arg Glu Ile Ala His Met His Asp Tyr His Asp
            180                 185                 190

Phe Thr Leu His Leu Ala Leu Ala Ala Gln Asp Gly Lys Glu Val Val
        195                 200                 205

Ala Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Thr Val Ile Val
            260                 265

<210>  87
<211>  798
<212>  DNA
<213>  Armillaria mellea

<400>  87
atgtccttct tcgacagcgt gaaacttgac ctcgtcggac gcctctttgg catcaggaat      60

cgcggcttag ctgttacttg ttgtgctgtg gcagtcgcct ctatcatcgc gttcccttac     120

attcgtaggg actaccagac atttttatct gggggtccct cctacgctcc ccagaacatc     180

agaggatacc tcattgtctg cgtcctggcc ttgttccgtc aggagcaaaa aggccttgcg     240

atatacgacc gccttcccga gaagcgcagg tggctacctg acttgcctcc tcgcgatggc 300

ccacggccca tcacgaccag ccatataatc caaagacagc gcaaccaggc gccggacctc 360

aagttcgccc tcgaggaact caaggccacg gtcattccac gggtgcaggc tcgccacact 420

gacctcaccc atctcagcct atccaagttc gagttccatg ctgaagcaat cttcctgctc 480

ccctctgtac ccatcgatga tccaaagaat gtgccaagtc acgacacggt gcgcaggacg 540

aagagggaaa ttgcgcatat gcacgactac catgactaca cgctgcatct tgcgttggcc 600

gcccaagacg ggaaggaagt cgtatcaaag ggatgggggc agcgacaccc gctggcaggc 660

cctggcgttc ctggtccacc gacggagtgg acgtttattt atgcgccacg taacgaagag 720

gagctggcag tggtggaaat gattatcgag gcatcgatag gctatatgac caatgaccct 780

gcaggaaaaa ctatcgca 798

<210> 88
<211> 266
<212> PRT
<213> Armillaria mellea

<400> 88

Met Ser Phe Phe Asp Ser Val Lys Leu Asp Leu Val Gly Arg Leu Phe
1               5                   10                  15

Gly Ile Arg Asn Arg Gly Leu Ala Val Thr Cys Cys Ala Val Ala Val
            20                  25                  30

Ala Ser Ile Ile Ala Phe Pro Tyr Ile Arg Arg Asp Tyr Gln Thr Phe
        35                  40                  45

Leu Ser Gly Gly Pro Ser Tyr Ala Pro Gln Asn Ile Arg Gly Tyr Leu
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Pro Asp Leu Pro
                85                  90                  95

Pro Arg Asp Gly Pro Arg Pro Ile Thr Thr Ser His Ile Ile Gln Arg

100                 105                 110

Gln Arg Asn Gln Ala Pro Asp Leu Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Val Pro Ser His Asp Thr
                165                 170                 175

Val Arg Arg Thr Lys Arg Glu Ile Ala His Met His Asp Tyr His Asp
            180                 185                 190

Tyr Thr Leu His Leu Ala Leu Ala Ala Gln Asp Gly Lys Glu Val Val
        195                 200                 205

Ser Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Lys Thr Ile Ala
            260                 265

<210>  89
<211>  798
<212>  DNA
<213>  Armillaria fuscipes

<400>  89
atgaccttct tggacagtat caaacttgac ctcgttgggc gcctctttgg catcaggaat      60

cacggcgtag ccgctgcctg ttgtgctgca gcagttgcct ctgccatcgt gttcccttat     120

attcgtaggg actaccagac atttctatct ggcggccctt cctacgctcc ccagaacatc     180

agaggataca tcattgtctg cgtcctagcc ttattccgcc aggagcaaaa aggccttgcg     240

atatatgacc gccttcccga gaagcgcagg tggttagctg acttgcctcc tcgcaatggc     300

ccacggccca tcacaaccag tcatataatt caaagacagc gcaaccaggc gccagacccc     360

aagttcgccc tcgaagaact caaggccaca gtcattccac gggtacaggc tcgccacact     420

gacctcaccc atctcagcct gtccaaattc gagtttcacg ctgaagcaat cttcctgctc     480

ccctctgtac ccatcgacga cccaaagaat atcccaagcc atgacacagt gcgcaggacg     540

aaaagggaga tcgcgcatat gcacgactat catgatttca cgctgcatct tgcactggct     600

gcccaagacg ggaaggaagt cgtatcaaag ggatgggggc agcggcaccc gctggcaggc     660

cctggtgtcc ctggtccacc aacggagtgg acgtttattt acgcgccacg gaacgaagag     720

gagctggcag tagtggaaat gataattgag gcatcaatag gctacatgac caatgaccct     780

gcaggatcag ttattcca                                                   798

<210>  90
<211>  266
<212>  PRT
<213>  Armillaria fuscipes

<400>  90

Met Thr Phe Leu Asp Ser Ile Lys Leu Asp Leu Val Gly Arg Leu Phe
1               5                   10                  15

Gly Ile Arg Asn His Gly Val Ala Ala Ala Cys Cys Ala Ala Ala Val
            20                  25                  30

Ala Ser Ala Ile Val Phe Pro Tyr Ile Arg Arg Asp Tyr Gln Thr Phe
        35                  40                  45

Leu Ser Gly Gly Pro Ser Tyr Ala Pro Gln Asn Ile Arg Gly Tyr Ile
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Ala Asp Leu Pro
                85                  90                  95

Pro Arg Asn Gly Pro Arg Pro Ile Thr Thr Ser His Ile Ile Gln Arg
            100                 105                 110

Gln Arg Asn Gln Ala Pro Asp Pro Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Ile Pro Ser His Asp Thr
                165                 170                 175

Val Arg Arg Thr Lys Arg Glu Ile Ala His Met His Asp Tyr His Asp
            180                 185                 190

Phe Thr Leu His Leu Ala Leu Ala Ala Gln Asp Gly Lys Glu Val Val
        195                 200                 205

Ser Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Ser Val Ile Pro
            260                 265

<210>  91
<211>  738
<212>  DNA
<213>  Mycena citricolor

<400>  91
atggcttatc agctcacttg gattcagact ctcgtgctgg gtgcccttgt ggcaatggca        60

gtagcgttcc ccttcatcaa gaaagactac gagacgttcc tgaagggcgg cccctcctat       120

gcgccccaaa acgttcgcgg atacatcatc gtgctcgtgc tcgcgctctt ccgccaagag 180

cagctcgggc tggagatcta cgaccgcatg cccgagaaac gtcgctggct cgcgaatctc 240

cctcagcgcg agggcccccg ccccaagacc acaagtcaca tcatccagcg gcagctcagc 300

cagcacacgg accccgcatt cggcgccgcg tacctcaaag acaccgtcat tccgcgcgtc 360

caggcgcggc acgcagccaa cacgcacatc gcgcgctcga cgttcgagtt ccacgccgcc 420

gcgatcttcc tgaacgcgga cgtgccgctg cccgagggcc tgcccgcaag cgagacggtg 480

cggcggacca agggcgagat cgcgcacatg cacgactacc acgacttcac gctgcacctc 540

gcgctcgcgg cggcggatgg gaaggaggtg gtcggcaagg gctgggggca gcgccatccg 600

ctggcgggac ccggtgtgcc gggtccgccg aacgagtgga cctttgtgta tgcgccgagg 660

aatgaagagg agatgggcgt ggtcgagcag atcgtagagg cggcgattgg gtacatgtcg 720

aacgtgcctg cgctggaa 738

<210> 92
<211> 246
<212> PRT
<213> Mycena citricolor

<400> 92

Met Ala Tyr Gln Leu Thr Trp Ile Gln Thr Leu Val Leu Gly Ala Leu
1               5                   10                  15

Val Ala Met Ala Val Ala Phe Pro Phe Ile Lys Lys Asp Tyr Glu Thr
            20                  25                  30

Phe Leu Lys Gly Gly Pro Ser Tyr Ala Pro Gln Asn Val Arg Gly Tyr
        35                  40                  45

Ile Ile Val Leu Val Leu Ala Leu Phe Arg Gln Glu Gln Leu Gly Leu
    50                  55                  60

Glu Ile Tyr Asp Arg Met Pro Glu Lys Arg Arg Trp Leu Ala Asn Leu
65                  70                  75                  80

Pro Gln Arg Glu Gly Pro Arg Pro Lys Thr Thr Ser His Ile Ile Gln
                85                  90                  95

Arg Gln Leu Ser Gln His Thr Asp Pro Ala Phe Gly Ala Ala Tyr Leu
            100                 105                 110

Lys Asp Thr Val Ile Pro Arg Val Gln Ala Arg His Ala Ala Asn Thr
        115                 120                 125

His Ile Ala Arg Ser Thr Phe Glu Phe His Ala Ala Ala Ile Phe Leu
    130                 135                 140

Asn Ala Asp Val Pro Leu Pro Glu Gly Leu Pro Ala Ser Glu Thr Val
145                 150                 155                 160

Arg Arg Thr Lys Gly Glu Ile Ala His Met His Asp Tyr His Asp Phe
                165                 170                 175

Thr Leu His Leu Ala Leu Ala Ala Ala Asp Gly Lys Glu Val Val Gly
            180                 185                 190

Lys Gly Trp Gly Gln Arg His Pro Leu Ala Gly Pro Gly Val Pro Gly
        195                 200                 205

Pro Pro Asn Glu Trp Thr Phe Val Tyr Ala Pro Arg Asn Glu Glu Glu
    210                 215                 220

Met Gly Val Val Glu Gln Ile Val Glu Ala Ala Ile Gly Tyr Met Ser
225                 230                 235                 240

Asn Val Pro Ala Leu Glu
                245

<210>  93
<211>  747
<212>  DNA
<213>  Panellus stipticus

<400>  93
atgaacatca acctgaaagc tctgatcgga gtctgtgccg tgctcatcac cgctgcagtg      60

ttccccttcg ttcgtaaaga ctatcacacc tttcttgaag gtggaccatc ctacgcgccg     120

cagaatttgc aaggctatat catcgtgttg gtgctctcac tctttcgagg ggaggagacg     180

ggattggaaa tatacgaccg cttgcccgaa aaacgccgct ggctcgagga gctgcctgtt     240

cgcgaaggcc cgcgcccaaa gacaaccagc cacatcattc agagacaatt gaatcagcac 300

gttgacccgg acttcggaat gaactctttg aaaggctccg tcatccggcg ccttcaatcc 360

cgccaccagg acataactca actcgcactc tcgaaattcg aattccacgc cgaggccata 420

tttctgcgcc ccgatatcgc gatcaacgat cccaaacacg tcccgagcca cgacacggtg 480

cgccgcacaa agcgcgagat agctcacatg cacgactacc atgattacac gtgtcatttg 540

gcgctcgcag cgcaggatgg gaagcaagtg attgcaaaag ggtggggcca gagacatccg 600

ctcgcgggac cgggcatgcc ggggccgccg acggagtgga catttttgta tgcgccgagg 660

aatgaggcgg aggttcaagt gttggagacg attatcgaag cgtcaatcgg gtacatgtcg 720

aacgcaccag ccttgggtgg gagcgag 747

<210> 94
<211> 249
<212> PRT
<213> Panellus stipticus

<400> 94

Met Asn Ile Asn Leu Lys Ala Leu Ile Gly Val Cys Ala Val Leu Ile
1               5                   10                  15

Thr Ala Ala Val Phe Pro Phe Val Arg Lys Asp Tyr His Thr Phe Leu
            20                  25                  30

Glu Gly Gly Pro Ser Tyr Ala Pro Gln Asn Leu Gln Gly Tyr Ile Ile
        35                  40                  45

Val Leu Val Leu Ser Leu Phe Arg Gly Glu Glu Thr Gly Leu Glu Ile
    50                  55                  60

Tyr Asp Arg Leu Pro Glu Lys Arg Arg Trp Leu Glu Glu Leu Pro Val
65                  70                  75                  80

Arg Glu Gly Pro Arg Pro Lys Thr Thr Ser His Ile Ile Gln Arg Gln
                85                  90                  95

Leu Asn Gln His Val Asp Pro Asp Phe Gly Met Asn Ser Leu Lys Gly
            100                 105                 110

Ser Val Ile Arg Arg Leu Gln Ser Arg His Gln Asp Ile Thr Gln Leu
        115                 120                 125

Ala Leu Ser Lys Phe Glu Phe His Ala Glu Ala Ile Phe Leu Arg Pro
    130                 135                 140

Asp Ile Ala Ile Asn Asp Pro Lys His Val Pro Ser His Asp Thr Val
145                 150                 155                 160

Arg Arg Thr Lys Arg Glu Ile Ala His Met His Asp Tyr His Asp Tyr
                165                 170                 175

Thr Cys His Leu Ala Leu Ala Ala Gln Asp Gly Lys Gln Val Ile Ala
            180                 185                 190

Lys Gly Trp Gly Gln Arg His Pro Leu Ala Gly Pro Gly Met Pro Gly
        195                 200                 205

Pro Pro Thr Glu Trp Thr Phe Leu Tyr Ala Pro Arg Asn Glu Ala Glu
    210                 215                 220

Val Gln Val Leu Glu Thr Ile Ile Glu Ala Ser Ile Gly Tyr Met Ser
225                 230                 235                 240

Asn Ala Pro Ala Leu Gly Gly Ser Glu
                245

<210>  95
<211>  792
<212>  DNA
<213>  Neonothopanus gardneri

<400>  95
atgaatcttc cgtctttcgt ccaacgtctc tccacagcaa gcagtcgcag tatagcgatt      60

acttgcgtag ttgtccttgc ctctgcaatc gcctttccct tcatccgcag agactaccag     120

accttcctgg aagtgggacc ctcgtacgcc ccgcagaact ttagaggata catcatcgtc     180

tgtgtcctct cgttgttccg ccaagaacaa aaaggactcg aaatctacga tcggctccca     240

gagaaacgaa ggtggttgtc cgaccttccc tttcgtgacg ggcccagacc cagcatcaca     300

agccatatca ttcaacgaca gcgtacccaa ctagttgatc cggacttcgc tacccaggag     360

ctcataggca aagtcatccc tcgtgtgcaa gcacgacaca ccgacaaaac attcctcagc 420

acctccaaat tcgaatttca cgcaaaagcc atattcctcc tgccttccat cccaatcaac 480

gaccctctga acgttccaag ccacgacact gtccgacgaa cgaagcgcga gatcgcgcat 540

atgcatgatt atcatgattg cactcttcac atcgctctcg ctgctcagga cggaaaggag 600

gttttgaaga agggatgggg gcaacgacac ccactcgctg gacctggagt gcccggccca 660

ccgacggagt ggacgtttct ctatgcgcct cgaaacgaag aagaggttcg agttgtggag 720

atgattattg aggctgccat aggttacatg acgaatgatc cggcaggaaa agttgtagaa 780

gccactggaa ag 792

<210> 96
<211> 264
<212> PRT
<213> Neonothopanus gardneri

<400> 96

Met Asn Leu Pro Ser Phe Val Gln Arg Leu Ser Thr Ala Ser Ser Arg
1               5                   10                  15

Ser Ile Ala Ile Thr Cys Val Val Val Leu Ala Ser Ala Ile Ala Phe
            20                  25                  30

Pro Phe Ile Arg Arg Asp Tyr Gln Thr Phe Leu Glu Val Gly Pro Ser
        35                  40                  45

Tyr Ala Pro Gln Asn Phe Arg Gly Tyr Ile Ile Val Cys Val Leu Ser
    50                  55                  60

Leu Phe Arg Gln Glu Gln Lys Gly Leu Glu Ile Tyr Asp Arg Leu Pro
65                  70                  75                  80

Glu Lys Arg Arg Trp Leu Ser Asp Leu Pro Phe Arg Asp Gly Pro Arg
                85                  90                  95

Pro Ser Ile Thr Ser His Ile Ile Gln Arg Gln Arg Thr Gln Leu Val
            100                 105                 110

Asp Pro Asp Phe Ala Thr Gln Glu Leu Ile Gly Lys Val Ile Pro Arg

115                 120                 125

Val Gln Ala Arg His Thr Asp Lys Thr Phe Leu Ser Thr Ser Lys Phe
    130                 135                 140

Glu Phe His Ala Lys Ala Ile Phe Leu Leu Pro Ser Ile Pro Ile Asn
145                 150                 155                 160

Asp Pro Leu Asn Val Pro Ser His Asp Thr Val Arg Arg Thr Lys Arg
                165                 170                 175

Glu Ile Ala His Met His Asp Tyr His Asp Cys Thr Leu His Ile Ala
            180                 185                 190

Leu Ala Ala Gln Asp Gly Lys Glu Val Leu Lys Lys Gly Trp Gly Gln
        195                 200                 205

Arg His Pro Leu Ala Gly Pro Gly Val Pro Gly Pro Pro Thr Glu Trp
    210                 215                 220

Thr Phe Leu Tyr Ala Pro Arg Asn Glu Glu Glu Val Arg Val Val Glu
225                 230                 235                 240

Met Ile Ile Glu Ala Ala Ile Gly Tyr Met Thr Asn Asp Pro Ala Gly
                245                 250                 255

Lys Val Val Glu Ala Thr Gly Lys
            260

<210>  97
<211>  753
<212>  DNA
<213>  Mycena chlorophos

<400>  97
atggtccaac tcacccgaac ctccggattc atcgccgctg cggccattgt tgctgccatc      60

gccttcccgt tcattcgtcg agactaccag acgttccttc gtggtgggcc gtcctatgcc     120

ccacagaaca tccgcggcta tatcatcgtc ctggttctgt ccctcttccg cggcgaggag     180

aagggtcttg caatctacga gccccttcct gagaagcgca catggctgcc ggagcttccg     240

cggcgcgcgg gagaccggcc caagacgacg agccacatca tccaacggca gctcgaccag     300

taccccgacc cggactttgt cctcaaagcc ctgaaagcga cggtcatccc gcgtgtccaa 360

gcccggcaca cagacaagac tcacctcgcg ctgtccaagt tcgagttcca tgctgaggcc 420

atcttcgtgc gcccggaaat cgccatcgac gacccgaagc atatccccag ccacgacacg 480

gtgcgacgga cgaagcgcga gattgcgcac atgcacgact atcacgactg cacgctgcat 540

ttggcgctag cggcgcagga cgcgaagcag gtgctgcaga agggctgggg ccagcgccat 600

ccgctggcag ggcctgggat gcccgggccg cccacggagt ggacgttctt gtatgccccg 660

aggaccgagg aggaagtgaa ggttgtggag accattgtcg aggcctctat cgcgtacatg 720

acgaacgcgg agaagccggt cgagctggtg cag 753

<210> 98
<211> 251
<212> PRT
<213> Mycena chlorophos

<400> 98

Met Val Gln Leu Thr Arg Thr Ser Gly Phe Ile Ala Ala Ala Ala Ile
1               5                   10                  15

Val Ala Ala Ile Ala Phe Pro Phe Ile Arg Arg Asp Tyr Gln Thr Phe
            20                  25                  30

Leu Arg Gly Gly Pro Ser Tyr Ala Pro Gln Asn Ile Arg Gly Tyr Ile
        35                  40                  45

Ile Val Leu Val Leu Ser Leu Phe Arg Gly Glu Glu Lys Gly Leu Ala
    50                  55                  60

Ile Tyr Glu Pro Leu Pro Glu Lys Arg Thr Trp Leu Pro Glu Leu Pro
65                  70                  75                  80

Arg Arg Ala Gly Asp Arg Pro Lys Thr Thr Ser His Ile Ile Gln Arg
                85                  90                  95

Gln Leu Asp Gln Tyr Pro Asp Pro Asp Phe Val Leu Lys Ala Leu Lys
            100                 105                 110

Ala Thr Val Ile Pro Arg Val Gln Ala Arg His Thr Asp Lys Thr His

115                 120                 125

Leu Ala Leu Ser Lys Phe Glu Phe His Ala Glu Ala Ile Phe Val Arg
    130                 135                 140

Pro Glu Ile Ala Ile Asp Asp Pro Lys His Ile Pro Ser His Asp Thr
145                 150                 155                 160

Val Arg Arg Thr Lys Arg Glu Ile Ala His Met His Asp Tyr His Asp
                165                 170                 175

Cys Thr Leu His Leu Ala Leu Ala Ala Gln Asp Ala Lys Gln Val Leu
            180                 185                 190

Gln Lys Gly Trp Gly Gln Arg His Pro Leu Ala Gly Pro Gly Met Pro
        195                 200                 205

Gly Pro Pro Thr Glu Trp Thr Phe Leu Tyr Ala Pro Arg Thr Glu Glu
    210                 215                 220

Glu Val Lys Val Val Glu Thr Ile Val Glu Ala Ser Ile Ala Tyr Met
225                 230                 235                 240

Thr Asn Ala Glu Lys Pro Val Glu Leu Val Gln
                245                 250

<210>  99
<211>  801
<212>  DNA
<213>  Artificial sequence

<220>
<223>  Optimized for mammalian expression
       (humanized) nucleic acid, encoding protein SEQ ID
       No:80

<400>  99
atgcgcatta acatctccct ttcatctctt ttcgagcgat tgagcaaact gagttccagg      60

agtattgcaa tcacttgtgg ggttgtcctc gcgagcgcca tcgcattccc catcatccgg     120

agagattatc agacgtttct tgaggtgggc cctagctatg caccacagaa cttccgagga     180

tatatcatcg tgtgtgtact gtcactgttt aggcaagaac aaaagggatt ggctatctat     240

gataggttgc ctgagaaacg gcggtggctc gctgatctcc catttagaga ggggacacga 300

ccgagcatca cttcacacat catacaaaga cagcgaacgc agctcgttga ccaagagttc 360

gcaactaggg aactgattga taaggtgata cccagagtac aggcgcgaca caccgataaa 420

acttttcttt ccacctctaa attcgagttc catgccaaag ctattttctt gttgccttcc 480

ataccgatta atgatcctct gaatattcca tcccacgaca cagttcgacg gacgaaacgc 540

gaaattgcgc acatgcacga ctatcacgat tgcactttgc acctggcact ggctgctcaa 600

gacggaaaag aagttctgaa aaagggttgg gggcaaagac atccgctggc gggacccggt 660

gtacctgggc cgcctacgga atggacattt ttgtacgcac cgaggaacga agaggaggcc 720

agggtcgttg agatgattgt tgaggctagt attgggtaca tgacgaatga tccggctggt 780

aaaattgttg aaaatgcaaa g 801

<210> 100
<211> 1266
<212> DNA
<213> Artificial sequence

<220>
<223> Optimized for mammalian expression
 (humanized) nucleic acid, encoding protein SEQ ID
 No:2

<400> 100
atggcgtcat tcgagaacag tcttagcgtg ctgatcgtcg gcgctgggct cggcggtctt 60

gctgcggcaa tcgccctcag gcgacaagga cacgttgtta aaatctatga cagctcatca 120

tttaaggcag agttgggcgc aggcctcgcg gtccccccaa acactttgag atcactgcaa 180

caactgggtt gtaatactga gaaccttaac ggcgtggata acctctgctt cactgcaatg 240

ggttacgatg gcagtgtggg tatgatgaac aatatgaccg attataggga ggcgtacggc 300

actagctgga taatggtcca tcgggttgat ctccacaatg agcttatgcg cgtagcgttg 360

gatccgggcg gattgggacc cccagctacc ttgcacttga atcaccgcgt gactttttgt 420

gatgtcgacg catgcacagt aaccttcacc aatgggacga ctcagtcagc ggatctcatc 480

gtcggcgccg acggtatacg atccactatc cgcagattcg tcctggagga agatgtcaca 540

gttccggcat ccggaatcgt tggtttccgc tggctcgtcc aggctgatgc tttggatcct 600

taccctgaac ttgactggat tgttaaaaag ccccctctcg gcgctaggtt gataagtacg 660

cctcaaaacc cgcagtctgg ggtaggtctc gcggatcgca gaacgatcat tatatacgcg 720

tgtcgaggag gtactatggt aaacgtactt gccgtccatg acgatgagag ggatcagaat 780

acggcagatt ggtccgtgcc agctagcaag gatgatcttt tcagagtttt tcacgactat 840

catcctcgat ttcggcggct gttggagttg gcgcaagaca tcaatctgtg gcagatgcgg 900

gtggtccccg ttctgaagaa atgggtgaac aaaagagtct gtctcttggg ggatgcagcg 960

catgcgtccc tccctacctt ggggcagggt ttcggcatgg ggttggagga cgccgtagcc 1020

cttgggactt tgcttccaaa ggggacgaca gcatcccaaa tagaaacaag acttgccgta 1080

tatgagcagc tccgaaaaga tcgcgccgag ttcgtcgcgg ctgagtccta cgaagaacaa 1140

tatgtaccag aaatgagggg actttacctg cgatccaaag aattgcgcga ccgggtaatg 1200

ggctatgaca taaaggtgga gtccgaaaag gtcttggaga cactgttgcg gtcaagcaat 1260

tccgcc 1266

<210> 101
<211> 2112
<212> DNA
<213> Artificial sequence

<220>
<223> Nucleic acid encoding the fusion protein
      hispidin-hydroxylase and luciferase

<400> 101
atggcatcgt ttgagaattc tctaagcgtt ttgattgtcg gggccggact tggtgggctt 60

gctgctgcca tcgcgctgcg tcgccaaggg catgtcgtga aaatatacga ctcctctagc 120

ttcaaagccg aacttggtgc gggactcgct gtgccgccta acaccttgcg cagtctacag 180

caacttggtt gcaataccga gaacctcaat ggtgtggata atctttgctt cactgcgatg 240

gggtatgacg ggagtgtagg gatgatgaac aacatgactg actatcgaga ggcatacggt 300

acttcttgga tcatggtcca ccgcgttgac ttgcataacg agctgatgcg cgtagcactt 360

gatccaggtg ggctcggacc tcctgcgaca ctccatctta atcatcgtgt cacattctgc 420

gatgtcgacg cttgcaccgt gacattcacc aacgggacca ctcaatcagc tgatctcatc 480

gttggtgcag acggtatacg ctctaccatt cggcggtttg tcttagaaga agacgtgact 540

gtgcctgcgt caggaatcgt cgggtttcga tggcttgtac aagctgacgc gctggaccca 600

tatcctgaac tcgactggat tgttaaaaag cctcctctag gcgcgcgact gatctccact 660

cctcagaatc cacagtctgg tgttggcttg gctgacaggc gcactatcat catctacgca 720

tgtcgtggcg gcaccatggt caatgtcctt gcagtgcatg atgacgaacg tgaccagaac 780

accgcagatt ggagtgtacc ggcttccaaa gacgatctat ttcgtgtttt ccacgattac 840

catccacgct ttcggcggct tttagagctt gcgcaggata ttaatctctg gcaaatgcgt 900

gttgtacctg ttttgaaaaa atgggttaac aagcgggttt gcttgttagg agatgctgcg 960

cacgcttctt taccgacgtt gggtcaaggt tttggtatgg gcctggaaga tgccgtagca 1020

cttggtacac tccttccaaa gggtaccact gcatctcaga tcgagactcg acttgcggtg 1080

tacgaacagc tacgtaagga tcgtgcggaa tttgttgcgg ctgaatcata tgaagagcaa 1140

tatgttcctg aaatgcgggg actttatctg aggtcaaagg aactgcgtga tagagtcatg 1200

ggttatgata tcaaagtgga gagcgagaag gttctcgaga cgctcctaag aagttctaat 1260

tctgcctccg gaactggggg caacgcctct gacggtggtg ggtctggtgg tatgcgcatt 1320

aacattagcc tctcgtctct cttcgaacgt ctctccaaac ttagcagtcg cagcatagcg 1380

attacatgtg gagttgttct cgcctccgca atcgcctttc ccatcatccg cagagactac 1440

cagactttcc tagaagtggg accctcgtac gctccgcaga actttagagg atacatcatc 1500

gtctgtgtcc tctcgctatt ccgccaagag cagaaagggc tcgccatcta tgatcgtctt 1560

cccgagaaac gcaggtggtt ggccgacctt ccctttcgtg aaggaaccag acccagcatt 1620

accagccata tcattcagcg acagcgcact caactggtcg atcaggagtt tgccaccagg 1680

gagctcatag acaaggtcat ccctcgcgtg caagcacgac acaccgacaa aacgttcctc 1740

agcacatcaa agttcgagtt tcatgcgaag gccatatttc tcttgccttc tatcccaatc 1800

aacgaccctc tgaatatccc tagccacgac actgtccgcc gaacgaagcg cgagattgca 1860

catatgcatg attatcatga ttgcacactt catcttgctc tcgctgcgca ggatggaaag 1920

gaggtgctga agaaaggttg gggacaacga catcctttgg ctggtcctgg agttcctggt 1980

ccaccaacgg aatggacttt tctttatgcg cctcgcaacg aagaagaggc tcgagtagtg 2040

gagatgatcg ttgaggcttc catagggtat atgacgaacg atcctgcagg aaagattgta 2100

gaaaacgcca ag 2112

<210> 102
<211> 704

<212>  PRT
<213>  Artificial sequence

<220>
<223>  Fusion protein amino acid sequence
      hispidin-hydroxylase and luciferase

<400>  102

Met Ala Ser Phe Glu Asn Ser Leu Ser Val Leu Ile Val Gly Ala Gly
1               5                   10                  15

Leu Gly Gly Leu Ala Ala Ala Ile Ala Leu Arg Arg Gln Gly His Val
            20                  25                  30

Val Lys Ile Tyr Asp Ser Ser Ser Phe Lys Ala Glu Leu Gly Ala Gly
        35                  40                  45

Leu Ala Val Pro Pro Asn Thr Leu Arg Ser Leu Gln Gln Leu Gly Cys
    50                  55                  60

Asn Thr Glu Asn Leu Asn Gly Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly Thr Ser Trp Ile Met Val His Arg Val Asp Leu His
            100                 105                 110

Asn Glu Leu Met Arg Val Ala Leu Asp Pro Gly Gly Leu Gly Pro Pro
        115                 120                 125

Ala Thr Leu His Leu Asn His Arg Val Thr Phe Cys Asp Val Asp Ala
    130                 135                 140

Cys Thr Val Thr Phe Thr Asn Gly Thr Thr Gln Ser Ala Asp Leu Ile
145                 150                 155                 160

Val Gly Ala Asp Gly Ile Arg Ser Thr Ile Arg Arg Phe Val Leu Glu
                165                 170                 175

Glu Asp Val Thr Val Pro Ala Ser Gly Ile Val Gly Phe Arg Trp Leu

            180                 185                 190

Val Gln Ala Asp Ala Leu Asp Pro Tyr Pro Glu Leu Asp Trp Ile Val
        195                 200                 205

Lys Lys Pro Pro Leu Gly Ala Arg Leu Ile Ser Thr Pro Gln Asn Pro
    210                 215                 220

Gln Ser Gly Val Gly Leu Ala Asp Arg Arg Thr Ile Ile Ile Tyr Ala
225                 230                 235                 240

Cys Arg Gly Gly Thr Met Val Asn Val Leu Ala Val His Asp Asp Glu
                245                 250                 255

Arg Asp Gln Asn Thr Ala Asp Trp Ser Val Pro Ala Ser Lys Asp Asp
            260                 265                 270

Leu Phe Arg Val Phe His Asp Tyr His Pro Arg Phe Arg Arg Leu Leu
        275                 280                 285

Glu Leu Ala Gln Asp Ile Asn Leu Trp Gln Met Arg Val Val Pro Val
    290                 295                 300

Leu Lys Lys Trp Val Asn Lys Arg Val Cys Leu Leu Gly Asp Ala Ala
305                 310                 315                 320

His Ala Ser Leu Pro Thr Leu Gly Gln Gly Phe Gly Met Gly Leu Glu
                325                 330                 335

Asp Ala Val Ala Leu Gly Thr Leu Leu Pro Lys Gly Thr Thr Ala Ser
            340                 345                 350

Gln Ile Glu Thr Arg Leu Ala Val Tyr Glu Gln Leu Arg Lys Asp Arg
        355                 360                 365

Ala Glu Phe Val Ala Ala Glu Ser Tyr Glu Glu Gln Tyr Val Pro Glu
    370                 375                 380

Met Arg Gly Leu Tyr Leu Arg Ser Lys Glu Leu Arg Asp Arg Val Met
385                 390                 395                 400

Gly Tyr Asp Ile Lys Val Glu Ser Glu Lys Val Leu Glu Thr Leu Leu
                405                 410                 415

Arg Ser Ser Asn Ser Ala Ser Gly Thr Gly Gly Asn Ala Ser Asp Gly
            420                 425                 430

Gly Gly Ser Gly Gly Met Arg Ile Asn Ile Ser Leu Ser Ser Leu Phe
        435                 440                 445

Glu Arg Leu Ser Lys Leu Ser Ser Arg Ser Ile Ala Ile Thr Cys Gly
    450                 455                 460

Val Val Leu Ala Ser Ala Ile Ala Phe Pro Ile Ile Arg Arg Asp Tyr
465                 470                 475                 480

Gln Thr Phe Leu Glu Val Gly Pro Ser Tyr Ala Pro Gln Asn Phe Arg
                485                 490                 495

Gly Tyr Ile Ile Val Cys Val Leu Ser Leu Phe Arg Gln Glu Gln Lys
            500                 505                 510

Gly Leu Ala Ile Tyr Asp Arg Leu Pro Glu Lys Arg Arg Trp Leu Ala
        515                 520                 525

Asp Leu Pro Phe Arg Glu Gly Thr Arg Pro Ser Ile Thr Ser His Ile
    530                 535                 540

Ile Gln Arg Gln Arg Thr Gln Leu Val Asp Gln Glu Phe Ala Thr Arg
545                 550                 555                 560

Glu Leu Ile Asp Lys Val Ile Pro Arg Val Gln Ala Arg His Thr Asp
                565                 570                 575

Lys Thr Phe Leu Ser Thr Ser Lys Phe Glu Phe His Ala Lys Ala Ile
            580                 585                 590

Phe Leu Leu Pro Ser Ile Pro Ile Asn Asp Pro Leu Asn Ile Pro Ser
        595                 600                 605

His Asp Thr Val Arg Arg Thr Lys Arg Glu Ile Ala His Met His Asp

610                 615                 620

Tyr His Asp Cys Thr Leu His Leu Ala Leu Ala Ala Gln Asp Gly Lys
625                 630                 635                 640

Glu Val Leu Lys Lys Gly Trp Gly Gln Arg His Pro Leu Ala Gly Pro
                645                 650                 655

Gly Val Pro Gly Pro Pro Thr Glu Trp Thr Phe Leu Tyr Ala Pro Arg
            660                 665                 670

Asn Glu Glu Glu Ala Arg Val Val Glu Met Ile Val Glu Ala Ser Ile
        675                 680                 685

Gly Tyr Met Thr Asn Asp Pro Ala Gly Lys Ile Val Glu Asn Ala Lys
    690                 695                 700

<210>  103
<211>  1266
<212>  DNA
<213>  Artificial sequence

<220>
<223>  Nucleic acid optimized for expression in plants,
       encoding protein SEQ ID No:2

<400>  103
atggcatctt ttgagaattc tctgagcgtt ttgattgtcg gggccggact tggtgggctt      60

gctgctgcca tcgccctgcg tcgacaaggg catgtcgtga aaatatacga ctcctctagc     120

ttcaaagccg aacttggtgc tggactcgct gtgccgccta acaccttgcg tagtctccag     180

caacttggtt gcaataccga gaacctcaat ggtgtggata atctttgctt cactgctatg     240

gggtatgacg ggagtgtagg gatgatgaac aacatgactg actatcgaga ggcatacggt     300

acttcttgga tcatggtcca cagagttgac ttgcataacg agctgatgag ggtagcactt     360

gatccaggtg ggctcggacc tcctgcaaca ctccatctta atcatcgtgt cacattctgc     420

gatgtcgacg cttgcaccgt gacattcacc aacggaccca ctcaatcagc tgatctcatc     480

gttggtgcag acggtataag atctaccatt cgaaggtttg tcctggaaga agacgtgact     540

gtgcctgcat caggaatcgt cgggtttcga tggcttgtac aagctgacgc tctggaccca     600

tatcctgaac tcgactggat tgttaaaaag cctcctcttg gcgcacgact gatctccact     660

cctcagaatc cacagtctgg tgttggcttg gctgacagga ggactatcat catctacgca 720

tgtcgtggcg gcaccatggt caatgtcctt gcagtgcatg atgacgaacg tgaccagaac 780

accgcagatt ggagtgtacc ggcttccaaa gacgatctct ttcgtgtttt ccacgattac 840

catccacgat ttaggaggct tctcgagctt gctcaggata ttaatctctg gcaaatgcgt 900

gttgtacctg ttttgaaaaa atgggttaac aagagggttt gcttgctcgg agatgctgca 960

cacgcttctc tgccgacgtt gggtcaaggt tttggtatgg gtctggaaga tgccgtagca 1020

cttggtacac tccttccaaa gggtaccact gcatctcaga tcgagactcg acttgctgtg 1080

tacgaacagt tgcgtaagga tcgtgccgaa tttgttgccg ctgaatcata tgaagagcaa 1140

tatgttcctg aaatgcgagg actttatctg aggtcaaagg aactgcgtga tagagtcatg 1200

ggttatgata tcaaagtgga gagcgagaag gttctcgaga cgctccttag aagttctaat 1260

tctgcc 1266

<210> 104
<211> 1032
<212> DNA
<213> Aspergillus nidulans

<400> 104
atggtgcagg acacgtcatc tgcttctact tcccccattt tgacacgttg gtacatcgac 60

acaagaccct taacagcatc cacggcagca cttcctcttc tggaaacgtt gcagcctgca 120

gatcagatta gtgtacagaa gtattatcat ttaaaggata aacatatgag tcttgcttct 180

aacttattga agtatctgtt tgttcatcgt aattgtcgta ttccctggag ttctatcgtg 240

atctcccgta cacctgatcc tcaccgtcgt ccatgctaca ttcctccctc aggctctcag 300

gaagattcct tcaaagacgg atacactggt atcaatgttg agttcaatgt atcacaccaa 360

gccagtatgg tcgccattgc tggaaccgca tttactccca attctggtgg cgattctaaa 420

ctgaagcctg aggtgggaat cgacatcaca tgtgtaaacg agcgtcaggg ccgtaacggt 480

gaggagcgtt cattggaatc tcttcgtcaa tatatcgaca tcttctctga agtattctcc 540

acggctgaga tggcaaacat ccgtcgtctt gatggcgtta gtagttcttc tttaagtgct 600

gatcgtcttg ttgactatgg ttaccgtctt ttttatacat actgggccct gaaggaagcc 660

tatatcaaga tgactggtga agcattgctt gcaccatggt tacgtgagct ggagttctcc 720

aatgttgttg cccctgccgc tgttgctgaa tccggtgact ctgccggcga cttcggtgag 780

ccatacacag gagtccgtac gactttatat aaaaatttag ttgaggatgt gcgtatcgag 840

gtagccgccc ttggtggcga ttatcttttt gcaaccgcag ctcgtggagg aggaattgga 900

gccagttcaa ggcccggagg cggccctgat ggtagtggta tccgttcaca ggacccttgg 960

cgtccattta agaaacttga tatcgagcgt gacattcagc catgtgcaac aggtgtatgc 1020

aactgcctta gt 1032

<210> 105
<211> 344
<212> PRT
<213> Aspergillus nidulans

<400> 105

Met Val Gln Asp Thr Ser Ser Ala Ser Thr Ser Pro Ile Leu Thr Arg
1               5                   10                  15

Trp Tyr Ile Asp Thr Arg Pro Leu Thr Ala Ser Thr Ala Ala Leu Pro
            20                  25                  30

Leu Leu Glu Thr Leu Gln Pro Ala Asp Gln Ile Ser Val Gln Lys Tyr
        35                  40                  45

Tyr His Leu Lys Asp Lys His Met Ser Leu Ala Ser Asn Leu Leu Lys
    50                  55                  60

Tyr Leu Phe Val His Arg Asn Cys Arg Ile Pro Trp Ser Ser Ile Val
65                  70                  75                  80

Ile Ser Arg Thr Pro Asp Pro His Arg Arg Pro Cys Tyr Ile Pro Pro
                85                  90                  95

Ser Gly Ser Gln Glu Asp Ser Phe Lys Asp Gly Tyr Thr Gly Ile Asn
            100                 105                 110

Val Glu Phe Asn Val Ser His Gln Ala Ser Met Val Ala Ile Ala Gly
        115                 120                 125

Thr Ala Phe Thr Pro Asn Ser Gly Gly Asp Ser Lys Leu Lys Pro Glu
    130                 135                 140

Val Gly Ile Asp Ile Thr Cys Val Asn Glu Arg Gln Gly Arg Asn Gly
145                 150                 155                 160

Glu Glu Arg Ser Leu Glu Ser Leu Arg Gln Tyr Ile Asp Ile Phe Ser
            165                 170                 175

Glu Val Phe Ser Thr Ala Glu Met Ala Asn Ile Arg Arg Leu Asp Gly
        180                 185                 190

Val Ser Ser Ser Ser Leu Ser Ala Asp Arg Leu Val Asp Tyr Gly Tyr
    195                 200                 205

Arg Leu Phe Tyr Thr Tyr Trp Ala Leu Lys Glu Ala Tyr Ile Lys Met
    210                 215                 220

Thr Gly Glu Ala Leu Leu Ala Pro Trp Leu Arg Glu Leu Glu Phe Ser
225                 230                 235                 240

Asn Val Val Ala Pro Ala Ala Val Ala Glu Ser Gly Asp Ser Ala Gly
            245                 250                 255

Asp Phe Gly Glu Pro Tyr Thr Gly Val Arg Thr Thr Leu Tyr Lys Asn
        260                 265                 270

Leu Val Glu Asp Val Arg Ile Glu Val Ala Ala Leu Gly Gly Asp Tyr
    275                 280                 285

Leu Phe Ala Thr Ala Ala Arg Gly Gly Gly Ile Gly Ala Ser Ser Arg
    290                 295                 300

Pro Gly Gly Gly Pro Asp Gly Ser Gly Ile Arg Ser Gln Asp Pro Trp
305                 310                 315                 320

Arg Pro Phe Lys Lys Leu Asp Ile Glu Arg Asp Ile Gln Pro Cys Ala
            325                 330                 335

Thr Gly Val Cys Asn Cys Leu Ser
        340

<210>  106
<211>  1593
<212>  DNA
<213>  Rhodobacter capsulatus

<400>  106
atgactcttc aatcccaaac tgctaaggat tgcttggcac tggatggtgc actgactttg      60

gtgcagtgcg aggcaatcgc cacgcatcgt agtcgtattt ccgttacgcc agccttgcgt     120

gaacgttgtg ctcgtgccca cgcacgtttg gagcacgcaa tcgcagagca gcgtcatatt     180

tacggtatca ccactggatt tggacccctg gctaaccgtc ttattggagc cgatcagggc     240

gctgagctgc aacagaatct gatttaccat cttgcaacgg gcgttggacc caagctgtcc     300

tgggcagagg cccgtgcact tatgttggca cgtcttaatt ccattttgca gggcgctagt     360

ggtgcttctc ctgaaacgat cgaccgtatc gtcgcagtac ttaacgctgg ctttgctcct     420

gaagttcccg cacaaggtac tgttggcgca agtggcgact tgactcccct tgcccacatg     480

gtactggccc tgcaaggccg tggacgtatg atcgatccta gtggacgtgt gcaagaggct     540

ggagccgtca tggatcgtct ttgcggaggc cctcttacgt tggccgcacg tgacggcctt     600

gctctggtca acggtacaag tgctatgact gctatcgctg ctcttactgg agtcgaggcc     660

gctcgtgcta tcgatgctgc acttcgtcac tccgccgttt tgatggaagt cctgtctgga     720

catgcagaag cctggcaccc cgcctttgca gagttgagac cccacccagg ccagttgcgt     780

gctactgaac gtttggccca ggccctggat ggcgcaggcc gtgtatgccg tactctgacg     840

gctgcccgtc gtcttactgc agccgacctg cgtccagagg accaccccgc acaagacgct     900

tattctctgc gtgtcgttcc tcaactggtt ggtgccgtat gggatactct tgactggcac     960

gaccgtgtcg tgacctgtga gcttaattca gtaaccgata accccatttt tccagagggt    1020

tgcgccgttc ctgcacttca cggaggaaac ttcatgggag tccacgtcgc actggcttca    1080

gacgctttga atgcagccct ggttaccctt gccggtcttg tagagcgtca aattgcccgt    1140

cttaccgacg agaaacttaa taaaggtctg cctgctttcc ttcacggtgg acaggcaggc    1200

cttcagagtg gcttcatggg cgctcaggtt acagcaactg ccctgttggc tgagatgcgt    1260

gcaaatgcaa ctccagtctc agtacaatcc ttgtccacaa acggtgcaaa ccaagacgtc    1320

gtatctatgg gaactatcgc cgcacgtcgt gctcgtgcac aattgttgcc actgtcacag    1380

attcaggcta tcttggcttt ggcattggcc caggctatgg atcttcttga cgatcccgag    1440

ggtcaagccg gatggtcttt gacagcccgt gatttgcgtg accgtatccg tgccgtttct 1500

cccggactgc gtgccgaccg tccactggca ggccatatcg aagctgttgc tcagggactt 1560

cgtcacccat ctgctgcagc cgatccacca gcc 1593

<210> 107
<211> 531
<212> PRT
<213> Rhodobacter capsulatus

<400> 107

Met Thr Leu Gln Ser Gln Thr Ala Lys Asp Cys Leu Ala Leu Asp Gly
1               5                   10                  15

Ala Leu Thr Leu Val Gln Cys Glu Ala Ile Ala Thr His Arg Ser Arg
            20                  25                  30

Ile Ser Val Thr Pro Ala Leu Arg Glu Arg Cys Ala Arg Ala His Ala
        35                  40                  45

Arg Leu Glu His Ala Ile Ala Glu Gln Arg His Ile Tyr Gly Ile Thr
    50                  55                  60

Thr Gly Phe Gly Pro Leu Ala Asn Arg Leu Ile Gly Ala Asp Gln Gly
65                  70                  75                  80

Ala Glu Leu Gln Gln Asn Leu Ile Tyr His Leu Ala Thr Gly Val Gly
                85                  90                  95

Pro Lys Leu Ser Trp Ala Glu Ala Arg Ala Leu Met Leu Ala Arg Leu
            100                 105                 110

Asn Ser Ile Leu Gln Gly Ala Ser Gly Ala Ser Pro Glu Thr Ile Asp
        115                 120                 125

Arg Ile Val Ala Val Leu Asn Ala Gly Phe Ala Pro Glu Val Pro Ala
    130                 135                 140

Gln Gly Thr Val Gly Ala Ser Gly Asp Leu Thr Pro Leu Ala His Met
145                 150                 155                 160

Val Leu Ala Leu Gln Gly Arg Gly Arg Met Ile Asp Pro Ser Gly Arg
                165                 170                 175

Val Gln Glu Ala Gly Ala Val Met Asp Arg Leu Cys Gly Gly Pro Leu
            180                 185                 190

Thr Leu Ala Ala Arg Asp Gly Leu Ala Leu Val Asn Gly Thr Ser Ala
        195                 200                 205

Met Thr Ala Ile Ala Ala Leu Thr Gly Val Glu Ala Ala Arg Ala Ile
    210                 215                 220

Asp Ala Ala Leu Arg His Ser Ala Val Leu Met Glu Val Leu Ser Gly
225                 230                 235                 240

His Ala Glu Ala Trp His Pro Ala Phe Ala Glu Leu Arg Pro His Pro
                245                 250                 255

Gly Gln Leu Arg Ala Thr Glu Arg Leu Ala Gln Ala Leu Asp Gly Ala
            260                 265                 270

Gly Arg Val Cys Arg Thr Leu Thr Ala Ala Arg Arg Leu Thr Ala Ala
        275                 280                 285

Asp Leu Arg Pro Glu Asp His Pro Ala Gln Asp Ala Tyr Ser Leu Arg
    290                 295                 300

Val Val Pro Gln Leu Val Gly Ala Val Trp Asp Thr Leu Asp Trp His
305                 310                 315                 320

Asp Arg Val Val Thr Cys Glu Leu Asn Ser Val Thr Asp Asn Pro Ile
                325                 330                 335

Phe Pro Glu Gly Cys Ala Val Pro Ala Leu His Gly Gly Asn Phe Met
            340                 345                 350

Gly Val His Val Ala Leu Ala Ser Asp Ala Leu Asn Ala Ala Leu Val
        355                 360                 365

Thr Leu Ala Gly Leu Val Glu Arg Gln Ile Ala Arg Leu Thr Asp Glu
    370                 375                 380

Lys Leu Asn Lys Gly Leu Pro Ala Phe Leu His Gly Gly Gln Ala Gly
385                 390                 395                 400

Leu Gln Ser Gly Phe Met Gly Ala Gln Val Thr Ala Thr Ala Leu Leu
                405                 410                 415

Ala Glu Met Arg Ala Asn Ala Thr Pro Val Ser Val Gln Ser Leu Ser
            420                 425                 430

Thr Asn Gly Ala Asn Gln Asp Val Val Ser Met Gly Thr Ile Ala Ala
        435                 440                 445

Arg Arg Ala Arg Ala Gln Leu Leu Pro Leu Ser Gln Ile Gln Ala Ile
    450                 455                 460

Leu Ala Leu Ala Leu Ala Gln Ala Met Asp Leu Leu Asp Asp Pro Glu
465                 470                 475                 480

Gly Gln Ala Gly Trp Ser Leu Thr Ala Arg Asp Leu Arg Asp Arg Ile
                485                 490                 495

Arg Ala Val Ser Pro Gly Leu Arg Ala Asp Arg Pro Leu Ala Gly His
            500                 505                 510

Ile Glu Ala Val Ala Gln Gly Leu Arg His Pro Ser Ala Ala Ala Asp
        515                 520                 525

Pro Pro Ala
    530

<210>  108
<211>  1560
<212>  DNA
<213>  Escherichia coli

<400>  108
atgaaacctg aagattttcg tgcttccacc cagcggccct ttactggaga agaatatctg        60

aagtctcttc aagacggccg tgagatctat atttacggcg agcgtgtaaa ggatgttaca       120

acgcaccctg cttttcgtaa tgctgctgcc tcagttgccc aactttatga cgccctgcat       180

aaacccgaaa tgcaggactc actttgttgg aatacggaca cggggtccgg tggttacaca 240

cacaagttct ttcgtgtcgc caaatctgcc gatgacttgc gtcaacaacg tgatgctatc 300

gctgaatggt cacgtctgag ttacggctgg atgggccgta cgcccgacta taaggccgcc 360

tttggttgcg cattgggtgc aaatcccggc ttttatggac agtttgaaca aaatgcacgt 420

aactggtata ctcgtatcca ggaaaccggc ttgtacttta atcatgctat tgttaaccca 480

cctatcgatc gtcatttgcc aaccgataag gtcaaggatg tatatattaa acttgaaaag 540

gagacggacg ctggtatcat tgtgtctggc gctaaagtgg tggctacgaa cagtgcattg 600

acgcattaca atatgattgg ttttggttcc gcccaagtga tgggcgagaa ccccgacttt 660

gccttgatgt ttgttgcccc tatggacgct gacggagtaa aacttatttc acgtgcctca 720

tatgaaatgg tagctggtgc cactggttct ccttatgatt atcctctttc atcccgtttt 780

gacgaaaacg acgccatcct ggtcatggac aatgttctta ttccctggga gaacgtgctg 840

atctaccgtg acttcgatcg ttgccgtcgt tggactatgg aaggaggttt cgctcgtatg 900

tatccactgc aggcctgtgt tcgtttggct gtgaagttgg actttatcac agcccttctt 960

aaaaagtcac ttgagtgtac tggaacgttg gagttccgtg gtgtacaggc agatctgggc 1020

gaagtagtag cctggcgtaa tactttctgg gcactgtctg actctatgtg ctccgaggca 1080

acaccatggg tgaacggcgc atacctgccc gatcacgccg ctcttcaaac atatcgtgtg 1140

cttgctccca tggcttatgc taaaatcaaa aatattatcg agcgtaatgt aacttctggt 1200

ctgatttacc tgccatccag tgctcgtgac cttaacaatc ctcaaatcga tcagtatctt 1260

gcaaaatatg tgcgtggctc aaacggaatg gaccatgtgc agcgtatcaa aatcctgaag 1320

ttgatgtggg atgccatcgg ttctgaattt ggcggacgtc atgaactgta tgagattaac 1380

tactctggat ctcaagacga aatcagactt cagtgtttgc gtcaggccca aaattctgga 1440

aatatggata aaatgatggc tatggtagac cgttgtctga gtgagtacga tcaagatggt 1500

tggacagttc ctcatcttca taataatgac gatattaata tgcttgataa gttgcttaaa 1560

<210> 109
<211> 520
<212> PRT
<213> Escherichia coli

<400> 109

Met Lys Pro Glu Asp Phe Arg Ala Ser Thr Gln Arg Pro Phe Thr Gly
1               5                   10                  15

Glu Glu Tyr Leu Lys Ser Leu Gln Asp Gly Arg Glu Ile Tyr Ile Tyr
            20                  25                  30

Gly Glu Arg Val Lys Asp Val Thr Thr His Pro Ala Phe Arg Asn Ala
        35                  40                  45

Ala Ala Ser Val Ala Gln Leu Tyr Asp Ala Leu His Lys Pro Glu Met
    50                  55                  60

Gln Asp Ser Leu Cys Trp Asn Thr Asp Thr Gly Ser Gly Gly Tyr Thr
65                  70                  75                  80

His Lys Phe Phe Arg Val Ala Lys Ser Ala Asp Asp Leu Arg Gln Gln
                85                  90                  95

Arg Asp Ala Ile Ala Glu Trp Ser Arg Leu Ser Tyr Gly Trp Met Gly
            100                 105                 110

Arg Thr Pro Asp Tyr Lys Ala Ala Phe Gly Cys Ala Leu Gly Ala Asn
        115                 120                 125

Pro Gly Phe Tyr Gly Gln Phe Glu Gln Asn Ala Arg Asn Trp Tyr Thr
    130                 135                 140

Arg Ile Gln Glu Thr Gly Leu Tyr Phe Asn His Ala Ile Val Asn Pro
145                 150                 155                 160

Pro Ile Asp Arg His Leu Pro Thr Asp Lys Val Lys Asp Val Tyr Ile
                165                 170                 175

Lys Leu Glu Lys Glu Thr Asp Ala Gly Ile Ile Val Ser Gly Ala Lys
            180                 185                 190

Val Val Ala Thr Asn Ser Ala Leu Thr His Tyr Asn Met Ile Gly Phe
        195                 200                 205

Gly Ser Ala Gln Val Met Gly Glu Asn Pro Asp Phe Ala Leu Met Phe
    210                 215                 220

Val Ala Pro Met Asp Ala Asp Gly Val Lys Leu Ile Ser Arg Ala Ser
225                 230                 235                 240

Tyr Glu Met Val Ala Gly Ala Thr Gly Ser Pro Tyr Asp Tyr Pro Leu
                245                 250                 255

Ser Ser Arg Phe Asp Glu Asn Asp Ala Ile Leu Val Met Asp Asn Val
            260                 265                 270

Leu Ile Pro Trp Glu Asn Val Leu Ile Tyr Arg Asp Phe Asp Arg Cys
        275                 280                 285

Arg Arg Trp Thr Met Glu Gly Gly Phe Ala Arg Met Tyr Pro Leu Gln
    290                 295                 300

Ala Cys Val Arg Leu Ala Val Lys Leu Asp Phe Ile Thr Ala Leu Leu
305                 310                 315                 320

Lys Lys Ser Leu Glu Cys Thr Gly Thr Leu Glu Phe Arg Gly Val Gln
                325                 330                 335

Ala Asp Leu Gly Glu Val Val Ala Trp Arg Asn Thr Phe Trp Ala Leu
            340                 345                 350

Ser Asp Ser Met Cys Ser Glu Ala Thr Pro Trp Val Asn Gly Ala Tyr
        355                 360                 365

Leu Pro Asp His Ala Ala Leu Gln Thr Tyr Arg Val Leu Ala Pro Met
    370                 375                 380

Ala Tyr Ala Lys Ile Lys Asn Ile Ile Glu Arg Asn Val Thr Ser Gly
385                 390                 395                 400

Leu Ile Tyr Leu Pro Ser Ser Ala Arg Asp Leu Asn Asn Pro Gln Ile
                405                 410                 415

Asp Gln Tyr Leu Ala Lys Tyr Val Arg Gly Ser Asn Gly Met Asp His
            420                 425                 430

Val Gln Arg Ile Lys Ile Leu Lys Leu Met Trp Asp Ala Ile Gly Ser
        435                 440                 445

Glu Phe Gly Gly Arg His Glu Leu Tyr Glu Ile Asn Tyr Ser Gly Ser
    450                 455                 460

Gln Asp Glu Ile Arg Leu Gln Cys Leu Arg Gln Ala Gln Asn Ser Gly
465                 470                 475                 480

Asn Met Asp Lys Met Met Ala Met Val Asp Arg Cys Leu Ser Glu Tyr
                485                 490                 495

Asp Gln Asp Gly Trp Thr Val Pro His Leu His Asn Asn Asp Asp Ile
            500                 505                 510

Asn Met Leu Asp Lys Leu Leu Lys
        515                 520

<210>  110
<211>  510
<212>  DNA
<213>  Escherichia coli

<400>  110
atgcagcttg atgagcagcg tttgcgtttt cgtgacgcta tggcatcact tagtgccgct      60

gtgaacatca tcacgactga gggagacgcc ggtcagtgcg gaatcacagc aaccgcagta     120

tgtagtgtta cggacactcc cccatctctg atggtctgta tcaatgccaa tagtgctatg     180

aatccagtct ttcagggtaa tggtaaactt tgtgtcaacg tcctgaatca cgagcaagag     240

ttgatggctc gtcattttgc aggaatgact ggtatggcta tggaagagcg tttttccttg     300

tcttgttggc aaaaaggacc actggcccag cctgtattga aaggttcttt ggcttccctg     360

gagggtgaga tccgtgacgt tcaagccatt ggaactcatc tggtatattt ggtagaaatc     420

aagaatatta tcctgtcagc cgagggccat ggtctgatct actttaaacg tcgttttcac     480

cccgtgatgc tggagatgga ggcagccatt                                      510

<210>  111
<211>  170
<212>  PRT
<213>  Escherichia coli

<400>  111

Met Gln Leu Asp Glu Gln Arg Leu Arg Phe Arg Asp Ala Met Ala Ser
1               5                   10                  15

Leu Ser Ala Ala Val Asn Ile Ile Thr Thr Glu Gly Asp Ala Gly Gln
            20                  25                  30

Cys Gly Ile Thr Ala Thr Ala Val Cys Ser Val Thr Asp Thr Pro Pro
        35                  40                  45

Ser Leu Met Val Cys Ile Asn Ala Asn Ser Ala Met Asn Pro Val Phe
    50                  55                  60

Gln Gly Asn Gly Lys Leu Cys Val Asn Val Leu Asn His Glu Gln Glu
65                  70                  75                  80

Leu Met Ala Arg His Phe Ala Gly Met Thr Gly Met Ala Met Glu Glu
                85                  90                  95

Arg Phe Ser Leu Ser Cys Trp Gln Lys Gly Pro Leu Ala Gln Pro Val
            100                 105                 110

Leu Lys Gly Ser Leu Ala Ser Leu Glu Gly Glu Ile Arg Asp Val Gln
        115                 120                 125

Ala Ile Gly Thr His Leu Val Tyr Leu Val Glu Ile Lys Asn Ile Ile
    130                 135                 140

Leu Ser Ala Glu Gly His Gly Leu Ile Tyr Phe Lys Arg Arg Phe His
145                 150                 155                 160

Pro Val Met Leu Glu Met Glu Ala Ala Ile
                165                 170

<210>  112
<211>  801
<212>  DNA
<213>  Artificial sequence

<220>
<223>  Nucleic acid optimized for expression in plants,
       encoding protein SEQ ID No:80

<400>  112
atgaggataa atatctcttt gtctagtctc tttgagagac tgagcaaatt gtcatcccga      60

tccatcgcaa ttacctgcgg cgtagtcttg gcaagtgcaa tagcattccc aattatccga     120

agagactatc aaacgttcct tgaagtcggt ccgagctatg ccccacagaa cttccgaggc     180

tatatcatcg tttgtgtctt gtcacttttt aggcaagagc agaaagggtt ggcaatctat     240

gacaggcttc cagaaaagag gcgttggctt gccgacttgc cgtttcgtga agggacgagg     300

ccatccataa cctcccacat tatacagcgt cagcgtactc agctggtaga tcaagagttt     360

gcaaccagag aacttatcga taaggtgatc ccacgtgtgc aagcaagaca cacagacaaa     420

acttttctca gtacgtcaaa atttgagttt catgcaaagg ccatcttcct tctcccctct     480

atccctatta atgatcctct taacattccc tcacacgata cggtaagaag aaccaagcgt     540

gagatcgctc acatgcatga ttaccatgat tgcactctgc acttggctct tgctgctcag     600

gatggtaagg aagttttgaa gaaggggtgg ggccagcgtc acccactggc cggaccagga     660

gtcccaggcc ctcctactga gtggaccttc ctttacgcac caaggaacga agaggaggcc     720

agagttgtcg agatgatagt cgaagctagt attggctaca tgactaacga tcctgctggt     780

aagattgtcg aaaatgccaa g                                               801


<210>  113
<211>  5034
<212>  DNA
<213>  Artificial squence

<220>
<223>  Optimized for expression in plants of a nucleic acid,
       encoding protein SEQ ID No:35

<400>  113
atgaattcca gcaagaatcc tccttccact cttcttgatg tttttctgga tactgccagg      60

aaccttgata ccgctttgag gaatgtcttg gaatgcggcg aacacagatg gtcctacaga     120

gagcttgata ctgtttcatc tgctctcgcc cagcatctta ggtacactgt cggtcttagt     180

cctactgtcg ccgtcatcag tgaaaaccat ccttatattc tcgctttgat gctggctgta     240

tggaaacttg gaggcacctt cgctcctatt gatgtccatt ctcctgccga attggtagct     300

ggcatgctga acatagtctc tccttcttgc ttggttattc cgagctcaga tgtaactaat     360

caaactcttg cctgcgatct taatatcccc gtcgttgcat ttcacccaca tcaatccact     420

attcctgagc tgaacaagaa gtacctcacc gattctcaaa tttctccgga tcttcctttt 480
tcagatccaa acagacctgc tctgtacctc ttcacttcat ccgccacttc tcgaagtaat 540
ctcaaatgcg tgcctctcac tcacaccttt atcctccgaa acagcctctc taagcgtgca 600
tggtgcaagc gtatgcgtcc agagacagac tttgacggca tacgtgttct tggatgggcc 660
ccgtggtctc acgtcctcgc acacatgcaa gacatcggac cactcaccct gcttaatgcc 720
ggatgctacg tttttgcaac tactccatcc acgtacccta cggaattgaa ggacgacagg 780
gacctgatat cttgcgccgc aaatgctatc atgtacaagg gcgtcaagtc atttgcttgt 840
cttccctttg tactcggagg gctgaaggca ctctgcgagt ctgagccatc cgtgaaggcc 900
catcttcagg tcgaggagag agctcaactc ctgaagtctc tgcaacacat ggaaattctt 960
gagtgtggag gtgccatgct cgaagcaagt gttgcctctt gggctattga gaactgcatt 1020
cccatttcta tcggtattgg tatgacggag actggtggag ccctctttgc aggccccgtt 1080
caggccatca aaaccgggtt ttcttcagag gataaattca ttgaagatgc tacttacttg 1140
ctcgttaagg atgatcatga gagtcatgct gaggaggata ttaacgaggg tgaactcgtt 1200
gtgaaaagta aaatgctccc acgaggctac cttggctata gtgatccttc cttctcagtc 1260
gacgatgctg gctgggttac atttagaaca ggagacagat acagcgttac acctgacgga 1320
aagttttcct ggctgggccg aaacactgat ttcattcaga tgaccagtgg tgagacgctg 1380
gatccccgac caattgagag cagtctctgc gaaagttctc ttatttctag agcatgcgtt 1440
atcggagata aatttctcaa cgggcctgct gctgctgttt gtgctatcat tgagcttgag 1500
cccacagctg tggaaaaagg acaagctcac agccgtgaga tagcaagagt tttcgcacct 1560
attaatcgag acttgccgcc tcctcttagg attgcatgga gtcacgtttt ggttctccag 1620
cccagtgaga agataccgat gacgaagaag ggtaccatct tcaggaagaa aattgagcag 1680
gtgtttggct ctgccttggg tggcagctct ggagataact ctcaagccac tgctgatgct 1740
ggcgttgttc gacgagacga gctttcaaac actgtcaagc acataattag ccgtgttctc 1800
ggagtttccg atgacgaact cctttggacg ttgtcatttg ccgagctcgg aatgacgtca 1860
gcattggcca ctcgaatcgc caacgagttg aacgaagttt tggttggagt taatctccct 1920
atcaacgctt gctatataca tgtcgacctt ccttctctga gcaatgccgt ctatgccaaa 1980
cttgcacacc tcaagctgcc agatcgtact cccgaaccca ggcaagcccc tgtcgaaaac 2040

tctggtggga aggagatcgt tgtcgttggc caggcctttc gtcttcctgg ctcaataaac 2100

gatgtcgcct ctcttcgaga cgcattcctg gccagacaag catcatccat tatcactgaa 2160

ataccatccg ataggtggga ccacgccagc ttctatccca aggatatacg tttcaacaag 2220

gctggccttg tggatatagc caattatgat catagctttt tcggactgac ggcaaccgaa 2280

gcactctatc tgtccccaac tatgcgtttg gcactcgaag tttcctttga agccttggag 2340

aatgctaata tcccggtgtc acaactcaag ggttctcaaa cagctgttta tgttgctact 2400

acagatgacg gatttgagac ccttttgaat gccgaggccg gctatgatgc ttatacaaga 2460

ttctatggca ctggtcgagc agcaagtaca gccagcgggc gtataagctg tcttcttgat 2520

gtccatggac cctctattac tgttgatacg gcatgcagtg gaggggctgt ttgtattgac 2580

caagcaatcg actatctgca atcaagcagt gcagcagaca ccgctatcat atgtgctagt 2640

aacacgcact gctggccagg cagcttcagg tttctttccg cacaagggat ggtatcccca 2700

ggaggacgat gcgcaacatt tacaactgat gctgatggct acgtgccctc tgagggcgct 2760

gtcgccttca tattgaaaac ccgagaagca gctatgcgtg acaaggacac tatcctcgcc 2820

acaatcaaag ctacacagat atcacacaat ggccgatctc aaggtcttgt ggcaccgaat 2880

gtcaacagcc aagctgacct tcatagaagc ttgcttcaaa aagctggcct tagcccggct 2940

gatatccgtt tcattgaagc tcatgggaca ggaacgtcac tgggagacct ctcagaaatt 3000

caagctataa atgatgctta tacctcctct cagccgcgta cgaccggccc actcatagtc 3060

agcgcttcca aaacggtcat tggtcatacc gaaccagctg gccccttggt cggtatgctg 3120

agtgtcttga actctttcaa agaaggcgcc gtccctggtc tcgcccatct taccgcagac 3180

aatttgaatc ccagtctgga ctgttcttct gtgccacttc tcattcccta tcaacctgtt 3240

cacctggctg cacccaagcc tcaccgagct gctgtaaggt catacggctt ttcaggtacc 3300

ctgggcggca tcgttctcga ggctcctgac gaagaaagac tggaagaaga gctgccaaat 3360

gacaagccca tgttgttcgt cgtcagcgca aagacacata cagcacttat cgaatacctg 3420

gggcgttatc tcgagttcct cttgcaggca aacccccaag atttttgtga catttgttat 3480

acaagctgcg ttgggcgtga gcactataga tatcgatatg cttgtgtagc aaatgatatg 3540

gaggacctca taggccaact ccagaaacgt ttgggcagca aggtgccgcc aaagccgtca 3600

tacaaacgtg gtgctttggc ctttgccttt tctggtcagg gtacacaatt ccgagggatg 3660

gccacagagc ttgcaaaagc atactccggc ttccgaaaga tcgtgtccga tctcgcaaag 3720

agagctagcg agttgtcagg tcatgccatt gaccgttttc ttcttgcata tgacataggc 3780

gctgaaaatg tagctcctga tagtgaggca gaccagattt gcatctttgt gtatcagtgt 3840

tctgtccttc gttggctgca gactatgggg attagaccca gtgcagtgat aggccatagc 3900

ctcggggaga tctcagcttc tgtggcagca ggagcacttt ctcttgactc cgctttggat 3960

cttgtcatct cacgagctcg tcttttgagg tcttcagcaa gtgctcctgc aggaatggca 4020

gctatgtctg cctctcaaga cgaggttgtg gagttgattg ggaaactcga cctcgacaag 4080

gctaattctc tcagcgtttc agtcataaat ggtccccaaa atactgtcgt gtccggctct 4140

tcagctgcta ttgaaagcat agtggctctt gccaaaggga gaaagatcaa agcctctgcc 4200

ctgaatatca atcaagcttt tcatagtcca tacgtcgaca gtgccgtccc tggtctccgt 4260

gcttggtcag aaaagcatat ctcctcagct agaccattgc aaattccgct gtattcaacg 4320

ttgttgggag cacaaatctc tgagggagag atgttgaatc cagatcactg ggtcgaccat 4380

gcacgaaagc ctgtacagtt cgcacaagca gccacaacca tgaaagaatc cttcaccgga 4440

gtcatcatag atatcggccc tcaagtagtg gcttggtcac ttctgctcag taacgggctc 4500

acgtccgtga ctgctctcgc tgcaaaaaga gggagaagtc aacaggtggc tttcctcagc 4560

gccttggccg atttgtatca agattacggt gttgttcctg attttgtcgg gctttatgct 4620

cagcaggaag atgcttcaag gttgaagaag acggatatct tgacgtatcc gttccagcgt 4680

ggcgaagaga ctctttctag tggttctagc actccgacat tggaaaacac ggatttggat 4740

tccggtaagg aactccttat gggaccgact agagggttgc tccgtgctga cgacttgcgt 4800

gacagtatcg tttcttctgt gaaggatgtt ctggaactca agtcaaatga agacctcgat 4860

ttgtctgaaa gtctgaatgc acttggtatg gacagcatca tgttcgctca gctccgaaag 4920

cgtattgggg aaggactcgg attgaatgtt ccgatggttt ttctgagcga cgccttttct 4980

attggtgaga tggttagtaa tcttgtggaa caggcagagg cttctgagga caat 5034

<210> 114
<211> 867
<212> DNA
<213> Artificial sequence

<220>

<223>  Optimized for expression in plants of a nucleic acid,
       encoding protein SEQ ID No:65

<400>  114
atggctccaa tttcttcaac ttggtctcgt ctcattcgat ttgtggctat tgaaacgtcc        60

ctcgtgcata tcggtgaacc gatagacgcc accatggacg tcggtctggc cagacgagaa       120

ggcaagacga tccaagcata cgagattatt ggatcaggca gtgctcttga cctctcagcc       180

caagtaagta agaatgtgct gactgtaagg gaactcctga tgccgctttc aagagaggaa       240

attaaaactg tacgatgctt ggggttgaac taccctgttc atgccaccga agcaaacgtt       300

gctgttccaa aattcccgaa tttgttctac aaaccagtga cctccctcat tggccccgat       360

ggactcatta ccatcccttc cgttgtccaa cccccgaagg agcatcagtc cgattatgaa       420

gcagaacttg tcattgtcat cgggaaagca gcaaagaatg taagtgagga tgaggctttg       480

gattatgtat tgggatacac tgccgctaac gatatttcct ttaggaaaca ccagcttgca       540

gtctcacaat ggtctttctc taaaggattt ggtagccttt tgctcactat ccgtatggca       600

caaacccact ctggtaacat taatagattc tccagagacc agattttcaa tgtcaagaag       660

acaatttcct tcctgtcaca aggcactaca ctggaaccag gttctatcat tttgactggt       720

acacctgacg gagtgggctt tgtgcgaaat ccaccacttt accttaaaga tggagatgaa       780

gtaatgacct ggattggaag tggaatcgga acattggcca atacagtgca agaagagaag       840

acttgcttcg ctagtggcgg acacgag                                           867


<210>  115
<211>  9
<212>  PRT
<213>  Artificial sequence

<220>
<223>  Amino acid linker sequence

<400>  115

Gly Gly Ser Gly Gly Ser Gly Gly Ser
1               5


<210>  116
<211>  1569
<212>  DNA
<213>  Artificial sequence

<220>
<223>  Optimized for expression in plants of a nucleic acid,
       encoding a phenylalanine-ammonium-lyase Streptomyces maritimus (SEQ
       ID No: 117)

<400>  116
atgacattcg tgatagagct ggacatgaat gtcactcttg atcagttaga ggatgctgca      60

agacaaagaa cacccgtgga attgtctgca cctgtacgtt caagggtccg tgcttcacgt     120

gatgttttag ttaaattcgt acaagatgag cgtgtgattt acggtgtaaa tacttcaatg     180

ggcggattcg tggatcacct agtacctgtg agtcaggccc gtcaattgca agagaattta     240

atcaatgcag ttgccacaaa tgttggagca tatttagatg atacaacggc caggacgatt     300

atgttgagta ggattgtatc cctggcaagg ggcaattctg ccataactcc cgccaacctt     360

gacaaattag tggcagtgtt gaacgcagga atcgtcccat gcatccctga aaaaggttca     420

ttaggtactt caggtgactt aggccctctg gcagctatcg ccttagtatg cgctggccag     480

tggaaggcac gttacaatgg acaaataatg ccaggccgtc aggctctatc agaggccggt     540

gtcgaaccta tggaactttc atataaggac ggcctagcac ttataaacgg tacgtctgga     600

atggtaggcc ttggaactat ggttttacaa gccgctagga ggttagtgga caggtatttg     660

caagtaagtg ccttatctgt agaaggcttg gcaggcatga ctaaaccttt tgaccctcgt     720

gtacacggag ttaagcccca tagaggtcaa agacaagtag cttcccgtct atgggaagga     780

ttggccgatt ctcaccttgc cgtgaacgag cttgatactg aacaaaccct tgcaggtgaa     840

atgggcacgg tggcaaaggc tggaagttta gccatagagg atgcctactc cattcgttgc     900

acccctcaga tcctgggtcc agtcgtagac gtattggata ggatcggtgc aacattacaa     960

gacgagctga attcttcaaa cgataacccc attgtccttc ctgaggaagc agaagtattc    1020

cataatggac actttcatgg acagtatgtc gccatggcaa tggatcacct aaatatggct    1080

cttgctactg ttacgaatct tgccaataga agagtcgaca gatttctaga taaatcaaat    1140

tccaatggac taccagcctt tctttgccgt gaagacccag gccttaggct tggtttgatg    1200

ggcggccagt tcatgactgc atcaatcacc gctgaaaccc gtacactgac aatcccaatg    1260

tctgtacaat cacttacttc cacggcagat tttcaagaca tagtatcctt tggcttcgta    1320

gccgcaagga gagctagaga ggtgttgaca aacgctgctt atgtggtagc ctttgaacta    1380

ctgtgtgcct gtcaggctgt ggacatcaga ggagcagaca agctatcttc tttcacccgt    1440

cctctatatg aaagaaccag gaaaattgtt ccctttttcg acagagatga aacaattact 1500

gactacgttg agaagttagc tgctgattta atagcaggcg agcctgtaga tgccgctgtg 1560

gctgcacat 1569

<210> 117
<211> 523
<212> PRT
<213> Streptomyces maritimus

<400> 117

Met Thr Phe Val Ile Glu Leu Asp Met Asn Val Thr Leu Asp Gln Leu
1               5                   10                  15

Glu Asp Ala Ala Arg Gln Arg Thr Pro Val Glu Leu Ser Ala Pro Val
            20                  25                  30

Arg Ser Arg Val Arg Ala Ser Arg Asp Val Leu Val Lys Phe Val Gln
        35                  40                  45

Asp Glu Arg Val Ile Tyr Gly Val Asn Thr Ser Met Gly Gly Phe Val
    50                  55                  60

Asp His Leu Val Pro Val Ser Gln Ala Arg Gln Leu Gln Glu Asn Leu
65                  70                  75                  80

Ile Asn Ala Val Ala Thr Asn Val Gly Ala Tyr Leu Asp Asp Thr Thr
                85                  90                  95

Ala Arg Thr Ile Met Leu Ser Arg Ile Val Ser Leu Ala Arg Gly Asn
            100                 105                 110

Ser Ala Ile Thr Pro Ala Asn Leu Asp Lys Leu Val Ala Val Leu Asn
        115                 120                 125

Ala Gly Ile Val Pro Cys Ile Pro Glu Lys Gly Ser Leu Gly Thr Ser
    130                 135                 140

Gly Asp Leu Gly Pro Leu Ala Ala Ile Ala Leu Val Cys Ala Gly Gln
145                 150                 155                 160

Trp Lys Ala Arg Tyr Asn Gly Gln Ile Met Pro Gly Arg Gln Ala Leu
                165                 170                 175

Ser Glu Ala Gly Val Glu Pro Met Glu Leu Ser Tyr Lys Asp Gly Leu
            180                 185                 190

Ala Leu Ile Asn Gly Thr Ser Gly Met Val Gly Leu Gly Thr Met Val
        195                 200                 205

Leu Gln Ala Ala Arg Arg Leu Val Asp Arg Tyr Leu Gln Val Ser Ala
    210                 215                 220

Leu Ser Val Glu Gly Leu Ala Gly Met Thr Lys Pro Phe Asp Pro Arg
225                 230                 235                 240

Val His Gly Val Lys Pro His Arg Gly Gln Arg Gln Val Ala Ser Arg
                245                 250                 255

Leu Trp Glu Gly Leu Ala Asp Ser His Leu Ala Val Asn Glu Leu Asp
            260                 265                 270

Thr Glu Gln Thr Leu Ala Gly Glu Met Gly Thr Val Ala Lys Ala Gly
        275                 280                 285

Ser Leu Ala Ile Glu Asp Ala Tyr Ser Ile Arg Cys Thr Pro Gln Ile
    290                 295                 300

Leu Gly Pro Val Val Asp Val Leu Asp Arg Ile Gly Ala Thr Leu Gln
305                 310                 315                 320

Asp Glu Leu Asn Ser Ser Asn Asp Asn Pro Ile Val Leu Pro Glu Glu
                325                 330                 335

Ala Glu Val Phe His Asn Gly His Phe His Gly Gln Tyr Val Ala Met
            340                 345                 350

Ala Met Asp His Leu Asn Met Ala Leu Ala Thr Val Thr Asn Leu Ala
        355                 360                 365

Asn Arg Arg Val Asp Arg Phe Leu Asp Lys Ser Asn Ser Asn Gly Leu
    370                 375                 380

Pro Ala Phe Leu Cys Arg Glu Asp Pro Gly Leu Arg Leu Gly Leu Met
385                 390                 395                 400

Gly Gly Gln Phe Met Thr Ala Ser Ile Thr Ala Glu Thr Arg Thr Leu
                405                 410                 415

Thr Ile Pro Met Ser Val Gln Ser Leu Thr Ser Thr Ala Asp Phe Gln
            420                 425                 430

Asp Ile Val Ser Phe Gly Phe Val Ala Ala Arg Arg Ala Arg Glu Val
        435                 440                 445

Leu Thr Asn Ala Ala Tyr Val Val Ala Phe Glu Leu Leu Cys Ala Cys
    450                 455                 460

Gln Ala Val Asp Ile Arg Gly Ala Asp Lys Leu Ser Ser Phe Thr Arg
465                 470                 475                 480

Pro Leu Tyr Glu Arg Thr Arg Lys Ile Val Pro Phe Phe Asp Arg Asp
                485                 490                 495

Glu Thr Ile Thr Asp Tyr Val Glu Lys Leu Ala Ala Asp Leu Ile Ala
            500                 505                 510

Gly Glu Pro Val Asp Ala Ala Val Ala Ala His
        515                 520

<210>  118
<211>  1173
<212>  DNA
<213>  Artificial sequence

<220>
<223>  Nucleotide sequence PKS from Aquilaria sinensis,
       optimized for expression in Nicotiana benthamiana

<400>  118
atgggatctc aagatgttgc tggaggagct cttaagggag ttaatccagg aaaggctact      60

attcttgctc ttggaaaggc ttttccatat caacttgtta tgcaagaatt tcttgttgat     120

ggatatttta agaatacttc ttgtaaggat caagaactta agcaaaagct tgctagactt     180

tgtaagacta ctactgttaa gactagatat gttgttatgt ctgaagaaat tcttaataag 240

tatccagaac ttgctgttga aggaattcca actcttaagc aaagacttga tattggaaat 300

gaagctctta ctgaaatggc tattgaagct tctcaagctt gtattaagaa gtggggaaga 360

ccagcttctg aaattactca tcttgtttat gtttcttctt ctgaagctag acttccagga 420

ggagatcttt atcttgctca aggacttgga ctttctccaa gaactaagag agttgttctt 480

tattttatgg gatgttctgg aggagttgct ggacttagag ttgctaagga tattgctgaa 540

aataatccag gatctagagt tcttcttgct acttctgaaa ctactattgt tggatttaag 600

ccaccatctg ctcatagacc atatgatctt gttggagttg ctctttttgg agatggagct 660

ggagctatgg ttattggatc tgatccactt ccaggaactg aatctccact ttttgaactt 720

catactgcta ttcaaaattt tcttccaaat actgaaaaga ctattgatgg aagacttact 780

gaagaaggaa tttcttttaa gcttgctaga gaacttccac aaattgttga agatcatatt 840

gaaggatttt gtggacaact tactggagtt attggacttt ctcataagca atataataag 900

atgttttggg ctgttcatcc aggaggacca gctattctta atagagttga aaagagactt 960

gatcttcatc caaataagct tgatgcttct agaagagctc ttgaagatta tggaaatgct 1020

tcttctaatt ctattgttta tgttcttgat tatatgattg aagaaactct taagatgaag 1080

actgaatctc ttgaaccatc tgaatgggga cttattcttg cttttggacc aggagttact 1140

tttgaaggaa ttcttgctag aaatcttgct gtt 1173

<210> 119
<211> 391
<212> PRT
<213> Aquilaria sinensis

<400> 119

Met Gly Ser Gln Asp Val Ala Gly Gly Ala Leu Lys Gly Val Asn Pro
1               5                   10                  15

Gly Lys Ala Thr Ile Leu Ala Leu Gly Lys Ala Phe Pro Tyr Gln Leu
            20                  25                  30

Val Met Gln Glu Phe Leu Val Asp Gly Tyr Phe Lys Asn Thr Ser Cys
        35                  40                  45

Lys Asp Gln Glu Leu Lys Gln Lys Leu Ala Arg Leu Cys Lys Thr Thr
    50                  55                  60

Thr Val Lys Thr Arg Tyr Val Val Met Ser Glu Glu Ile Leu Asn Lys
65                  70                  75                  80

Tyr Pro Glu Leu Ala Val Glu Gly Ile Pro Thr Leu Lys Gln Arg Leu
                85                  90                  95

Asp Ile Gly Asn Glu Ala Leu Thr Glu Met Ala Ile Glu Ala Ser Gln
            100                 105                 110

Ala Cys Ile Lys Lys Trp Gly Arg Pro Ala Ser Glu Ile Thr His Leu
        115                 120                 125

Val Tyr Val Ser Ser Ser Glu Ala Arg Leu Pro Gly Gly Asp Leu Tyr
    130                 135                 140

Leu Ala Gln Gly Leu Gly Leu Ser Pro Arg Thr Lys Arg Val Val Leu
145                 150                 155                 160

Tyr Phe Met Gly Cys Ser Gly Gly Val Ala Gly Leu Arg Val Ala Lys
                165                 170                 175

Asp Ile Ala Glu Asn Asn Pro Gly Ser Arg Val Leu Leu Ala Thr Ser
            180                 185                 190

Glu Thr Thr Ile Val Gly Phe Lys Pro Pro Ser Ala His Arg Pro Tyr
        195                 200                 205

Asp Leu Val Gly Val Ala Leu Phe Gly Asp Gly Ala Gly Ala Met Val
    210                 215                 220

Ile Gly Ser Asp Pro Leu Pro Gly Thr Glu Ser Pro Leu Phe Glu Leu
225                 230                 235                 240

His Thr Ala Ile Gln Asn Phe Leu Pro Asn Thr Glu Lys Thr Ile Asp
                245                 250                 255

Gly Arg Leu Thr Glu Glu Gly Ile Ser Phe Lys Leu Ala Arg Glu Leu
            260                 265                 270

Pro Gln Ile Val Glu Asp His Ile Glu Gly Phe Cys Gly Gln Leu Thr
        275                 280                 285

Gly Val Ile Gly Leu Ser His Lys Gln Tyr Asn Lys Met Phe Trp Ala
    290                 295                 300

Val His Pro Gly Gly Pro Ala Ile Leu Asn Arg Val Glu Lys Arg Leu
305                 310                 315                 320

Asp Leu His Pro Asn Lys Leu Asp Ala Ser Arg Arg Ala Leu Glu Asp
                325                 330                 335

Tyr Gly Asn Ala Ser Ser Asn Ser Ile Val Tyr Val Leu Asp Tyr Met
            340                 345                 350

Ile Glu Glu Thr Leu Lys Met Lys Thr Glu Ser Leu Glu Pro Ser Glu
        355                 360                 365

Trp Gly Leu Ile Leu Ala Phe Gly Pro Gly Val Thr Phe Glu Gly Ile
    370                 375                 380

Leu Ala Arg Asn Leu Ala Val
385                 390

<210>  120
<211>  1164
<212>  DNA
<213>  Artificial sequence

<220>
<223>  Nucleotide sequence PKS2 from Aquilaria sinensis,
       optimized for expression in Nicotiana benthamiana

<400>  120
atgtctcaag ctattgctga taatgcttat agacatcatc ttaagagagc tccaactcca      60

ggaaaggcta ctgttcttgc tcttggaaag gcttttccaa agcaagttat tccacaagaa     120

aatcttgttg aaggatatat tagagatact aagtgtgaag atgtttctat taaggaaaag     180

cttgaaagac tttgtaagac tactactgtt aagactagat atactgttat gtctaaggaa     240

attcttgata attatccaga acttgttact gaaggatctc caactattag acaaagactt     300

gaaattgcta atccagctgt tgttgaaatg gctaaggaag cttctcttgc ttgtattaag 360

caatggggaa gaccagctgg agatattact catattgttt atgtttcttc ttctgaaatt 420

agacttccag gaggagatct ttatcttgct aatgaacttg gacttaagaa tgatattaat 480

agaattatgc tttattttct tggatgttat ggaggagtta ctggacttag agttgctaag 540

gatattgctg aaaataatcc aggatctaga attcttctta ctacttctga aactactatt 600

cttggattta gaccaccaaa taagtctaga ccatatgatc ttgttggagc tgctcttttt 660

ggagatggag ctgctgctgt tattattgga gctaatccag aaattggaag agaatctcca 720

tttatggaac ttaattttgc tcttcaacaa tttcttccag gaactcatgg agttattgat 780

ggaagacttt ctgaagaagg aattaatttt aagcttggaa gagatcttcc acaaaagatt 840

gaagataata ttgaagattt ttgtagaaag cttatgatta aggctgatgg agatcttaag 900

gaatttaatg aacttttttg ggctgttcat ccaggaggac cagctattct taatagactt 960

gaatctattc ttgatcttaa gaatggaaag cttgaatgtt ctagaagagc tcttatggat 1020

tatggaaatg tttcttctaa tactattttt tatgttatgg aatatatgag agaagaactt 1080

aagagagaag gatctgaaga atggggactt gctcttgctt ttggaccagg aattactttt 1140

gaaggaattc ttcttagatc tctt 1164

<210> 121
<211> 388
<212> PRT
<213> Aquilaria sinensis

<400> 121

Met Ser Gln Ala Ile Ala Asp Asn Ala Tyr Arg His His Leu Lys Arg
1               5                   10                  15

Ala Pro Thr Pro Gly Lys Ala Thr Val Leu Ala Leu Gly Lys Ala Phe
            20                  25                  30

Pro Lys Gln Val Ile Pro Gln Glu Asn Leu Val Glu Gly Tyr Ile Arg
        35                  40                  45

Asp Thr Lys Cys Glu Asp Val Ser Ile Lys Glu Lys Leu Glu Arg Leu
    50                  55                  60

Cys Lys Thr Thr Thr Val Lys Thr Arg Tyr Thr Val Met Ser Lys Glu
65                  70                  75                  80

Ile Leu Asp Asn Tyr Pro Glu Leu Val Thr Glu Gly Ser Pro Thr Ile
                85                  90                  95

Arg Gln Arg Leu Glu Ile Ala Asn Pro Ala Val Val Glu Met Ala Lys
            100                 105                 110

Glu Ala Ser Leu Ala Cys Ile Lys Gln Trp Gly Arg Pro Ala Gly Asp
        115                 120                 125

Ile Thr His Ile Val Tyr Val Ser Ser Ser Glu Ile Arg Leu Pro Gly
    130                 135                 140

Gly Asp Leu Tyr Leu Ala Asn Glu Leu Gly Leu Lys Asn Asp Ile Asn
145                 150                 155                 160

Arg Ile Met Leu Tyr Phe Leu Gly Cys Tyr Gly Gly Val Thr Gly Leu
                165                 170                 175

Arg Val Ala Lys Asp Ile Ala Glu Asn Asn Pro Gly Ser Arg Ile Leu
            180                 185                 190

Leu Thr Thr Ser Glu Thr Thr Ile Leu Gly Phe Arg Pro Pro Asn Lys
        195                 200                 205

Ser Arg Pro Tyr Asp Leu Val Gly Ala Ala Leu Phe Gly Asp Gly Ala
    210                 215                 220

Ala Ala Val Ile Ile Gly Ala Asn Pro Glu Ile Gly Arg Glu Ser Pro
225                 230                 235                 240

Phe Met Glu Leu Asn Phe Ala Leu Gln Gln Phe Leu Pro Gly Thr His
                245                 250                 255

Gly Val Ile Asp Gly Arg Leu Ser Glu Glu Gly Ile Asn Phe Lys Leu
            260                 265                 270

Gly Arg Asp Leu Pro Gln Lys Ile Glu Asp Asn Ile Glu Asp Phe Cys
        275                 280                 285

Arg Lys Leu Met Ile Lys Ala Asp Gly Asp Leu Lys Glu Phe Asn Glu
    290                 295                 300

Leu Phe Trp Ala Val His Pro Gly Gly Pro Ala Ile Leu Asn Arg Leu
305                 310                 315                 320

Glu Ser Ile Leu Asp Leu Lys Asn Gly Lys Leu Glu Cys Ser Arg Arg
                325                 330                 335

Ala Leu Met Asp Tyr Gly Asn Val Ser Ser Asn Thr Ile Phe Tyr Val
            340                 345                 350

Met Glu Tyr Met Arg Glu Glu Leu Lys Arg Glu Gly Ser Glu Glu Trp
        355                 360                 365

Gly Leu Ala Leu Ala Phe Gly Pro Gly Ile Thr Phe Glu Gly Ile Leu
    370                 375                 380

Leu Arg Ser Leu
385

<210>  122
<211>  1185
<212>  DNA
<213>  Artificial sequence

<220>
<223>  Nucleotide sequence PKS from Arabidopsis thaliana,
       optimized for expression in Nicotiana benthamiana

<400>  122
atgtctaatt ctagaatgaa tggagttgaa aagctttctt ctaagtctac tagaagagtt      60

gctaatgctg gaaaggctac tcttcttgct cttggaaagg cttttccatc tcaagttgtt     120

ccacaagaaa atcttgttga aggatttctt agagatacta agtgtgatga tgcttttatt     180

aaggaaaagc ttgaacatct ttgtaagact actactgtta agactagata tactgttctt     240

actagagaaa ttcttgctaa gtatccagaa cttactactg aaggatctcc aactattaag     300

caaagacttg aaattgctaa tgaagctgtt gttgaaatgg ctcttgaagc ttctcttgga     360

tgtattaagg aatggggaag accagttgaa gatattactc atattgttta tgtttcttct     420

tctgaaatta gacttccagg aggagatctt tatctttctg ctaagcttgg acttagaaat 480

gatgttaata gagttatgct ttattttctt ggatgttatg gaggagttac tggacttaga 540

gttgctaagg atattgctga aaataatcca ggatctagag ttcttcttac tacttctgaa 600

actactattc ttggatttag accaccaaat aaggctagac catatgatct tgttggagct 660

gctctttttg gagatggagc tgctgctgtt attattggag ctgatccaag agaatgtgaa 720

gctccattta tggaacttca ttatgctgtt caacaatttc ttccaggaac tcaaaatgtt 780

attgaaggaa gacttactga agaaggaatt aattttaagc ttggaagaga tcttccacaa 840

aagattgaag aaaatattga agaattttgt aagaagctta tgggaaaggc tggagatgaa 900

tctatggaat ttaatgatat gttttgggct gttcatccag gaggaccagc tattcttaat 960

agacttgaaa ctaagcttaa gcttgaaaag gaaaagcttg aatcttctag aagagctctt 1020

gttgattatg gaaatgtttc ttctaatact attctttatg ttatggaata tatgagagat 1080

gaacttaaga agaaggagga tgctgctcaa gaatggggac ttggacttgc ttttggacca 1140

ggaattactt ttgaaggact tcttattaga tctcttactt cttct 1185

<210> 123
<211> 395
<212> PRT
<213> Arabidopsis thaliana

<400> 123

Met Ser Asn Ser Arg Met Asn Gly Val Glu Lys Leu Ser Ser Lys Ser
1               5                   10                  15

Thr Arg Arg Val Ala Asn Ala Gly Lys Ala Thr Leu Leu Ala Leu Gly
            20                  25                  30

Lys Ala Phe Pro Ser Gln Val Val Pro Gln Glu Asn Leu Val Glu Gly
        35                  40                  45

Phe Leu Arg Asp Thr Lys Cys Asp Asp Ala Phe Ile Lys Glu Lys Leu
    50                  55                  60

Glu His Leu Cys Lys Thr Thr Thr Val Lys Thr Arg Tyr Thr Val Leu
65                  70                  75                  80

Thr Arg Glu Ile Leu Ala Lys Tyr Pro Glu Leu Thr Thr Glu Gly Ser
                85                  90                  95

Pro Thr Ile Lys Gln Arg Leu Glu Ile Ala Asn Glu Ala Val Val Glu
            100                 105                 110

Met Ala Leu Glu Ala Ser Leu Gly Cys Ile Lys Glu Trp Gly Arg Pro
        115                 120                 125

Val Glu Asp Ile Thr His Ile Val Tyr Val Ser Ser Ser Glu Ile Arg
    130                 135                 140

Leu Pro Gly Gly Asp Leu Tyr Leu Ser Ala Lys Leu Gly Leu Arg Asn
145                 150                 155                 160

Asp Val Asn Arg Val Met Leu Tyr Phe Leu Gly Cys Tyr Gly Gly Val
                165                 170                 175

Thr Gly Leu Arg Val Ala Lys Asp Ile Ala Glu Asn Asn Pro Gly Ser
            180                 185                 190

Arg Val Leu Leu Thr Thr Ser Glu Thr Thr Ile Leu Gly Phe Arg Pro
        195                 200                 205

Pro Asn Lys Ala Arg Pro Tyr Asp Leu Val Gly Ala Ala Leu Phe Gly
    210                 215                 220

Asp Gly Ala Ala Ala Val Ile Ile Gly Ala Asp Pro Arg Glu Cys Glu
225                 230                 235                 240

Ala Pro Phe Met Glu Leu His Tyr Ala Val Gln Gln Phe Leu Pro Gly
                245                 250                 255

Thr Gln Asn Val Ile Glu Gly Arg Leu Thr Glu Glu Gly Ile Asn Phe
            260                 265                 270

Lys Leu Gly Arg Asp Leu Pro Gln Lys Ile Glu Glu Asn Ile Glu Glu
        275                 280                 285

Phe Cys Lys Lys Leu Met Gly Lys Ala Gly Asp Glu Ser Met Glu Phe
    290                 295                 300

Asn Asp Met Phe Trp Ala Val His Pro Gly Gly Pro Ala Ile Leu Asn
305                 310                 315                 320

Arg Leu Glu Thr Lys Leu Lys Leu Glu Lys Glu Lys Leu Glu Ser Ser
                325                 330                 335

Arg Arg Ala Leu Val Asp Tyr Gly Asn Val Ser Ser Asn Thr Ile Leu
            340                 345                 350

Tyr Val Met Glu Tyr Met Arg Asp Glu Leu Lys Lys Lys Gly Asp Ala
        355                 360                 365

Ala Gln Glu Trp Gly Leu Gly Leu Ala Phe Gly Pro Gly Ile Thr Phe
    370                 375                 380

Glu Gly Leu Leu Ile Arg Ser Leu Thr Ser Ser
385                 390                 395

<210>  124
<211>  1197
<212>  DNA
<213>  Artificial sequence

<220>
<223>  Nucleotide sequence PKS from Hydrangea macrophylla,
       optimized for expression in Nicotiana benthamiana

<400>  124
atggctacta agtctgttgc tgttgaagaa atgtgtaagg ctcaaaaggc tggaggacca      60

gctactattc ttgctattgg aactgctgtt ccatctaatt gttattatca atctgaatat     120

ccagattttt attttagagt tactaagtct gatcatctta ctgatcttaa gtctaagttt     180

aagagaatgt gtgaaagatc ttctattaag aagagatata tgcatcttac tgaagaaatt     240

cttgaagaaa atccaaatat gtgtactttt gctgctccat ctattgatgg aagacaagat     300

attgttgtta aggaaattcc aaagcttgct aaggaagctg cttctaaggc tattaaggaa     360

tggggacaac caaagtctaa tattactcat cttgtttttt gtactacttc tggagttgat     420

atgccaggat gtgattatca acttactaga cttcttggac ttagaccatc tattaagaga     480

cttatgatgt atcaacaagg atgtcatgct ggaggaactg gacttagact tgctaaggat     540

cttgctgaaa ataataaggg agctagagtt cttgttgttt gttctgaaat gactgttatt 600

aattttagag gaccatctga agctcatatg gattctcttg ttggacaatc tctttttgga 660

gatggagctt ctgctgttat tgttggatct gatccagatc tttctactga acatccactt 720

tatcaaatta tgtctgcttc tcaaattatt gttgctgatt ctgaaggagc tattgatgga 780

catcttagac aagaaggact tacttttcat cttagaaagg atgttccatc tcttgtttct 840

gataatattg aaaatactct tgttgaagct tttactccaa ttcttatgga ttctattgat 900

tctattattg attggaattc tattttttgg attgctcatc caggaggacc agctattctt 960

aatcaagttc aagctaaggt tggacttaag gaagaaaagc ttagagtttc tagacatatt 1020

ctttctgaat atggaaatat gtcttctgct tgtgtttttt ttattatgga tgaaatgaga 1080

aagagatcta tggaagaagg aaagggaact actggagaag gacttgaatg gggagttctt 1140

tttggatttg gaccaggatt tactgttgaa actattgttc ttcattctgt tccaatt 1197

<210> 125
<211> 399
<212> PRT
<213> Hydrangea macrophylla

<400> 125

Met Ala Thr Lys Ser Val Ala Val Glu Glu Met Cys Lys Ala Gln Lys
1               5                   10                  15

Ala Gly Gly Pro Ala Thr Ile Leu Ala Ile Gly Thr Ala Val Pro Ser
            20                  25                  30

Asn Cys Tyr Tyr Gln Ser Glu Tyr Pro Asp Phe Tyr Phe Arg Val Thr
        35                  40                  45

Lys Ser Asp His Leu Thr Asp Leu Lys Ser Lys Phe Lys Arg Met Cys
    50                  55                  60

Glu Arg Ser Ser Ile Lys Lys Arg Tyr Met His Leu Thr Glu Glu Ile
65                  70                  75                  80

Leu Glu Glu Asn Pro Asn Met Cys Thr Phe Ala Ala Pro Ser Ile Asp
                85                  90                  95

Gly Arg Gln Asp Ile Val Val Lys Glu Ile Pro Lys Leu Ala Lys Glu
            100                 105                 110

Ala Ala Ser Lys Ala Ile Lys Glu Trp Gly Gln Pro Lys Ser Asn Ile
        115                 120                 125

Thr His Leu Val Phe Cys Thr Thr Ser Gly Val Asp Met Pro Gly Cys
    130                 135                 140

Asp Tyr Gln Leu Thr Arg Leu Leu Gly Leu Arg Pro Ser Ile Lys Arg
145                 150                 155                 160

Leu Met Met Tyr Gln Gln Gly Cys His Ala Gly Gly Thr Gly Leu Arg
                165                 170                 175

Leu Ala Lys Asp Leu Ala Glu Asn Asn Lys Gly Ala Arg Val Leu Val
            180                 185                 190

Val Cys Ser Glu Met Thr Val Ile Asn Phe Arg Gly Pro Ser Glu Ala
        195                 200                 205

His Met Asp Ser Leu Val Gly Gln Ser Leu Phe Gly Asp Gly Ala Ser
    210                 215                 220

Ala Val Ile Val Gly Ser Asp Pro Asp Leu Ser Thr Glu His Pro Leu
225                 230                 235                 240

Tyr Gln Ile Met Ser Ala Ser Gln Ile Ile Val Ala Asp Ser Glu Gly
                245                 250                 255

Ala Ile Asp Gly His Leu Arg Gln Glu Gly Leu Thr Phe His Leu Arg
            260                 265                 270

Lys Asp Val Pro Ser Leu Val Ser Asp Asn Ile Glu Asn Thr Leu Val
        275                 280                 285

Glu Ala Phe Thr Pro Ile Leu Met Asp Ser Ile Asp Ser Ile Ile Asp
    290                 295                 300

Trp Asn Ser Ile Phe Trp Ile Ala His Pro Gly Gly Pro Ala Ile Leu
305                 310                 315                 320

Asn Gln Val Gln Ala Lys Val Gly Leu Lys Glu Glu Lys Leu Arg Val
                325                 330                 335

Ser Arg His Ile Leu Ser Glu Tyr Gly Asn Met Ser Ser Ala Cys Val
            340                 345                 350

Phe Phe Ile Met Asp Glu Met Arg Lys Arg Ser Met Glu Glu Gly Lys
        355                 360                 365

Gly Thr Thr Gly Glu Gly Leu Glu Trp Gly Val Leu Phe Gly Phe Gly
    370                 375                 380

Pro Gly Phe Thr Val Glu Thr Ile Val Leu His Ser Val Pro Ile
385                 390                 395

<210>  126
<211>  1254
<212>  DNA
<213>  Artificial sequence

<220>
<223>  Nucleotide sequence PKS from Physcomitrella patens,
       optimized for expression in Nicotiana benthamiana

<400>  126
atggcttcta gaagagttga agctgctttt gatggacaag ctgttgaact tggagctact      60

attccagctg ctaatggaaa tggaactcat caatctatta aggttccagg acatagacaa     120

gttactccag gaaagactac tattatggct attggaagag ctgttccagc taatactact     180

tttaatgatg gacttgctga tcattatatt caagaattta atcttcaaga tccagttctt     240

caagctaagc ttagaagact ttgtgaaact actactgtta agactagata tcttgttgtt     300

aataaggaaa ttcttgatga acatccagaa tttcttgttg atggagctgc tactgtttct     360

caaagacttg ctattactgg agaagctgtt actcaacttg gacatgaagc tgctactgct     420

gctattaagg aatggggaag accagcttct gaaattactc atcttgttta tgtttcttct     480

tctgaaatta gacttccagg aggagatctt tatcttgctc aacttcttgg acttagatct     540

gatgttaata gagttatgct ttatatgctt ggatgttatg gaggagcttc tggaattaga     600

gttgctaagg atcttgctga aaataatcca ggatctagag ttcttcttat tacttctgaa     660

tgtactctta ttggatataa gtctctttct ccagatagac catatgatct tgttggagct 720

gctctttttg gagatggagc tgctgctatg attatgggaa aggatccaat tccagttctt 780

gaaagagctt tttttgaact tgattgggct ggacaatctt ttattccagg aactaataag 840

actattgatg gaagactttc tgaagaagga atttctttta agcttggaag agaacttcca 900

aagcttattg aatctaatat tcaaggattt tgtgatccaa ttcttaagag agctggagga 960

cttaagtata atgatatttt ttgggctgtt catccaggag gaccagctat tcttaatgct 1020

gttcaaaagc aacttgatct tgctccagaa aagcttcaaa ctgctagaca agttcttaga 1080

gattatggaa atatttcttc ttctacttgt atttatgttc ttgattatat gagacatcaa 1140

tctcttaagc ttaaggaagc taatgataat gttaatactg aaccagaatg ggacttcttt 1200

cttgcttttg gaccaggagt tactattgaa ggagctcttc ttagaaatct ttgt 1254

<210> 127
<211> 397
<212> PRT
<213> Physcomitrella patens

<400> 127

Met Ser Lys Thr Val Glu Asp Arg Ala Ala Gln Arg Ala Lys Gly Pro
1               5                   10                  15

Ala Thr Val Leu Ala Ile Gly Thr Ala Thr Pro Ala Asn Val Val Tyr
            20                  25                  30

Gln Thr Asp Tyr Pro Asp Tyr Tyr Phe Arg Val Thr Lys Ser Glu His
        35                  40                  45

Met Thr Lys Leu Lys Asn Lys Phe Gln Arg Met Cys Asp Arg Ser Thr
    50                  55                  60

Ile Lys Lys Arg Tyr Met Val Leu Thr Glu Glu Leu Leu Glu Lys Asn
65                  70                  75                  80

Leu Ser Leu Cys Thr Tyr Met Glu Pro Ser Leu Asp Ala Arg Gln Asp
                85                  90                  95

Ile Leu Val Pro Glu Val Pro Lys Leu Gly Lys Glu Ala Ala Asp Glu
            100                 105                 110

Ala Ile Ala Glu Trp Gly Arg Pro Lys Ser Glu Ile Thr His Leu Ile
        115                 120                 125

Phe Cys Thr Thr Cys Gly Val Asp Met Pro Gly Ala Asp Tyr Gln Leu
    130                 135                 140

Thr Lys Leu Leu Gly Leu Arg Ser Ser Val Arg Arg Thr Met Leu Tyr
145                 150                 155                 160

Gln Gln Gly Cys Phe Gly Gly Gly Thr Val Leu Arg Leu Ala Lys Asp
                165                 170                 175

Leu Ala Glu Asn Asn Ala Gly Ala Arg Val Leu Val Val Cys Ser Glu
            180                 185                 190

Ile Thr Thr Ala Val Asn Phe Arg Gly Pro Ser Asp Thr His Leu Asp
        195                 200                 205

Leu Leu Val Gly Leu Ala Leu Phe Gly Asp Gly Ala Ala Ala Val Ile
    210                 215                 220

Val Gly Ala Asp Pro Asp Pro Thr Leu Glu Arg Pro Leu Phe Gln Ile
225                 230                 235                 240

Val Ser Gly Ala Gln Thr Ile Leu Pro Asp Ser Glu Gly Ala Ile Asn
                245                 250                 255

Gly His Leu Arg Glu Val Gly Leu Thr Ile Arg Leu Leu Lys Asp Val
            260                 265                 270

Pro Gly Leu Val Ser Met Asn Ile Glu Lys Cys Leu Met Glu Ala Phe
        275                 280                 285

Ala Pro Met Gly Ile His Asp Trp Asn Ser Ile Phe Trp Ile Ala His
    290                 295                 300

Pro Gly Gly Pro Thr Ile Leu Asp Gln Val Glu Ala Lys Leu Gly Leu
305                 310                 315                 320

Lys Glu Glu Lys Leu Lys Ser Thr Arg Ala Val Leu Arg Glu Tyr Gly
                325                 330                 335

Asn Met Ser Ser Ala Cys Val Leu Phe Ile Leu Asp Glu Val Arg Lys
            340                 345                 350

Arg Ser Met Glu Glu Gly Lys Thr Thr Thr Gly Glu Gly Phe Asp Trp
        355                 360                 365

Gly Val Leu Phe Gly Phe Gly Pro Gly Phe Thr Val Glu Thr Val Val
    370                 375                 380

Leu His Ser Met Pro Ile Pro Lys Ala Asp Glu Gly Arg
385                 390                 395

<210>  128
<211>  1179
<212>  DNA
<213>  Artificial sequence

<220>
<223>  Nucleotide sequence PKS from Polygonum cuspidatum,
       optimized for expression in Nicotiana benthamiana

<400>  128
atggctccag ctgttgctga tattagaaag gctcaaagag ctgaaggacc agctactgtt      60

cttgctattg gaactgctac tccaccaaat tgtgtttatc aaaaggatta tccagattat     120

tattttagag ttactaattc tgatcatatg actgatctta aggaaaagtt tagaagaatg     180

tgtgaaaagt ctaatattga aaagagatat atgtatctta ctgaagaaat tcttaaggaa     240

aatccaaata tgtgttctta tatgcaaact tcttctcttg atactagaca agatatggtt     300

gtttctgaag ttccaagact tggaaaggaa gctgctcaaa aggctattaa ggaatgggga     360

caaccaaagt ctaagattac tcatgttatt atgtgtacta cttctggagt tgatatgcca     420

ggagctgatt atcaacttac taagcttctt ggacttcatc catctgttaa gagatttatg     480

atgtatcaac aaggatgttt tgctggagga actgttctta gacttgctaa ggatcttgct     540

gaaaataata gaggagctag agttcttgtt gtttgttctg aaattactgc tatttgtttt     600

agaggaccaa ctgatactca tccagattct atggttggac aagctctttt tggagatgga     660

tctggagctg ttattattgg agctgatcca gatctttcta ttgaaaagcc aatttttgaa     720

cttgtttgga ctgctcaaac tattcttcca gattctgaag gagctattga tggacatctt 780

agagaagttg gacttacttt tcatcttctt aaggatgttc caggacttat ttctaagaat 840

attgaaaaga atcttactga agctttttct ccacttaatg tttctgattg gaattctctt 900

ttttggattg ctcatccagg aggaccagct attcttgatc aagttgaaac taagcttgga 960

cttaaggaag aaaagcttaa ggctactaga caagttctta atgattatgg aaatatgtct 1020

tctgcttgtg ttctttttat tatggatgaa atgagaaaga agtctgttga aaatggacat 1080

gctactactg gagaaggact tgaatgggga gttctttttg gatttggacc aggacttact 1140

gttgaaactg ttgttcttca ttctgttcca gttgctaat 1179

<210> 129
<211> 393
<212> PRT
<213> Polygonum cuspidatum

<400> 129

Met Ala Pro Ala Val Ala Asp Ile Arg Lys Ala Gln Arg Ala Glu Gly
1               5                   10                  15

Pro Ala Thr Val Leu Ala Ile Gly Thr Ala Thr Pro Pro Asn Cys Val
            20                  25                  30

Tyr Gln Lys Asp Tyr Pro Asp Tyr Tyr Phe Arg Val Thr Asn Ser Asp
        35                  40                  45

His Met Thr Asp Leu Lys Glu Lys Phe Arg Arg Met Cys Glu Lys Ser
    50                  55                  60

Asn Ile Glu Lys Arg Tyr Met Tyr Leu Thr Glu Glu Ile Leu Lys Glu
65                  70                  75                  80

Asn Pro Asn Met Cys Ser Tyr Met Gln Thr Ser Ser Leu Asp Thr Arg
                85                  90                  95

Gln Asp Met Val Val Ser Glu Val Pro Arg Leu Gly Lys Glu Ala Ala
            100                 105                 110

Gln Lys Ala Ile Lys Glu Trp Gly Gln Pro Lys Ser Lys Ile Thr His
        115                 120                 125

Val Ile Met Cys Thr Thr Ser Gly Val Asp Met Pro Gly Ala Asp Tyr
    130                 135                 140

Gln Leu Thr Lys Leu Leu Gly Leu His Pro Ser Val Lys Arg Phe Met
145                 150                 155                 160

Met Tyr Gln Gln Gly Cys Phe Ala Gly Gly Thr Val Leu Arg Leu Ala
                165                 170                 175

Lys Asp Leu Ala Glu Asn Asn Arg Gly Ala Arg Val Leu Val Val Cys
            180                 185                 190

Ser Glu Ile Thr Ala Ile Cys Phe Arg Gly Pro Thr Asp Thr His Pro
        195                 200                 205

Asp Ser Met Val Gly Gln Ala Leu Phe Gly Asp Gly Ser Gly Ala Val
    210                 215                 220

Ile Ile Gly Ala Asp Pro Asp Leu Ser Ile Glu Lys Pro Ile Phe Glu
225                 230                 235                 240

Leu Val Trp Thr Ala Gln Thr Ile Leu Pro Asp Ser Glu Gly Ala Ile
                245                 250                 255

Asp Gly His Leu Arg Glu Val Gly Leu Thr Phe His Leu Leu Lys Asp
            260                 265                 270

Val Pro Gly Leu Ile Ser Lys Asn Ile Glu Lys Asn Leu Thr Glu Ala
        275                 280                 285

Phe Ser Pro Leu Asn Val Ser Asp Trp Asn Ser Leu Phe Trp Ile Ala
    290                 295                 300

His Pro Gly Gly Pro Ala Ile Leu Asp Gln Val Glu Thr Lys Leu Gly
305                 310                 315                 320

Leu Lys Glu Glu Lys Leu Lys Ala Thr Arg Gln Val Leu Asn Asp Tyr
                325                 330                 335

Gly Asn Met Ser Ser Ala Cys Val Leu Phe Ile Met Asp Glu Met Arg
            340                 345                 350

Lys Lys Ser Val Glu Asn Gly His Ala Thr Thr Gly Glu Gly Leu Glu
        355                 360                 365

Trp Gly Val Leu Phe Gly Phe Gly Pro Gly Leu Thr Val Glu Thr Val
    370                 375                 380

Val Leu His Ser Val Pro Val Ala Asn
385                 390

<210>  130
<211>  1152
<212>  DNA
<213>  Artificial squence

<220>
<223>  Nucleotide sequence of the mutant L132S PKS from Rheum
       palmatum, optimized for expression in Nicotiana benthamiana

<400>  130
atggctactg aagaaatgaa gaagcttgct actgttatgg ctattggaac tgctaatcca      60

ccaaattgtt attatcaagc tgattttcca gatttttatt ttagagttac taattctgat     120

catcttatta atcttaagca aaagtttaag agactttgtg aaaattctag aattgaaaag     180

agatatcttc atgttactga agaaattctt aaggaaaatc caaatattgc tgcttatgaa     240

gctacttctc ttaatgttag acataagatg caagttaagg gagttgctga acttggaaag     300

gaagctgctc ttaaggctat taaggaatgg ggacaaccaa agtctaagat tactcatctt     360

attgtttgtt gttctgctgg agttgatatg ccaggagctg attatcaact tactaagctt     420

cttgatcttg atccatctgt taagagattt atgttttatc atcttggatg ttatgctgga     480

ggaactgttc ttagacttgc taaggatatt gctgaaaata ataagggagc tagagttctt     540

attgtttgtt ctgaaatgac tactacttgt tttagaggac catctgaaac tcatcttgat     600

tctatgattg gacaagctat tcttggagat ggagctgctg ctgttattgt tggagctgat     660

ccagatctta ctgttgaaag accaattttt gaacttgttt ctactgctca aactattgtt     720

ccagaatctc atggagctat tgaaggacat cttcttgaat ctggactttc ttttcatctt     780

tataagactg ttccaactct tatttctaat aatattaaga cttgtctttc tgatgctttt     840

actccactta atatttctga ttggaattct cttttttgga ttgctcatcc aggaggacca 900

gctattcttg atcaagttac tgctaaggtt ggacttgaaa aggaaaagct taaggttact 960

agacaagttc ttaaggatta tggaaatatg tcttctgcta ctgttttttt tattatggat 1020

gaaatgagaa agaagtctct tgaaaatgga caagctacta ctggagaagg acttgaatgg 1080

ggagttcttt ttggatttgg accaggaatt actgttgaaa ctgttgttct tagatctgtt 1140

ccagttattt ct 1152

<210> 131
<211> 384
<212> PRT
<213> Artificial sequence

<220>
<223> Amino acid sequence of the mutant L132S PKS from Rheum
      palmatum, optimized for expression in Nicotiana benthamiana

<400> 131

Met Ala Thr Glu Glu Met Lys Lys Leu Ala Thr Val Met Ala Ile Gly
1               5                   10                  15

Thr Ala Asn Pro Pro Asn Cys Tyr Tyr Gln Ala Asp Phe Pro Asp Phe
            20                  25                  30

Tyr Phe Arg Val Thr Asn Ser Asp His Leu Ile Asn Leu Lys Gln Lys
        35                  40                  45

Phe Lys Arg Leu Cys Glu Asn Ser Arg Ile Glu Lys Arg Tyr Leu His
    50                  55                  60

Val Thr Glu Glu Ile Leu Lys Glu Asn Pro Asn Ile Ala Ala Tyr Glu
65                  70                  75                  80

Ala Thr Ser Leu Asn Val Arg His Lys Met Gln Val Lys Gly Val Ala
                85                  90                  95

Glu Leu Gly Lys Glu Ala Ala Leu Lys Ala Ile Lys Glu Trp Gly Gln
            100                 105                 110

Pro Lys Ser Lys Ile Thr His Leu Ile Val Cys Cys Ser Ala Gly Val
        115                 120                 125

Asp Met Pro Gly Ala Asp Tyr Gln Leu Thr Lys Leu Leu Asp Leu Asp
    130                 135                 140

Pro Ser Val Lys Arg Phe Met Phe Tyr His Leu Gly Cys Tyr Ala Gly
145                 150                 155                 160

Gly Thr Val Leu Arg Leu Ala Lys Asp Ile Ala Glu Asn Asn Lys Gly
                165                 170                 175

Ala Arg Val Leu Ile Val Cys Ser Glu Met Thr Thr Thr Cys Phe Arg
            180                 185                 190

Gly Pro Ser Glu Thr His Leu Asp Ser Met Ile Gly Gln Ala Ile Leu
        195                 200                 205

Gly Asp Gly Ala Ala Ala Val Ile Val Gly Ala Asp Pro Asp Leu Thr
    210                 215                 220

Val Glu Arg Pro Ile Phe Glu Leu Val Ser Thr Ala Gln Thr Ile Val
225                 230                 235                 240

Pro Glu Ser His Gly Ala Ile Glu Gly His Leu Leu Glu Ser Gly Leu
                245                 250                 255

Ser Phe His Leu Tyr Lys Thr Val Pro Thr Leu Ile Ser Asn Asn Ile
            260                 265                 270

Lys Thr Cys Leu Ser Asp Ala Phe Thr Pro Leu Asn Ile Ser Asp Trp
        275                 280                 285

Asn Ser Leu Phe Trp Ile Ala His Pro Gly Gly Pro Ala Ile Leu Asp
    290                 295                 300

Gln Val Thr Ala Lys Val Gly Leu Glu Lys Glu Lys Leu Lys Val Thr
305                 310                 315                 320

Arg Gln Val Leu Lys Asp Tyr Gly Asn Met Ser Ser Ala Thr Val Phe
                325                 330                 335

Phe Ile Met Asp Glu Met Arg Lys Lys Ser Leu Glu Asn Gly Gln Ala
            340                 345                 350

Thr Thr Gly Glu Gly Leu Glu Trp Gly Val Leu Phe Gly Phe Gly Pro
        355                 360                 365

Gly Ile Thr Val Glu Thr Val Val Leu Arg Ser Val Pro Val Ile Ser
    370                 375                 380

<210> 132
<211> 1173
<212> PRT
<213> Artificial sequence

<220>
<223> Nucleotide sequence PKS from Rheum tataricum,
optimized for expression in Nicotiana benthamiana

<400> 132

Ala Thr Gly Gly Cys Thr Cys Cys Ala Gly Ala Ala Gly Ala Ala Thr
1               5                   10                  15

Cys Thr Ala Gly Ala Cys Ala Thr Gly Cys Thr Gly Ala Ala Ala Cys
            20                  25                  30

Thr Gly Cys Thr Gly Thr Thr Ala Ala Thr Ala Gly Ala Gly Cys Thr
        35                  40                  45

Gly Cys Thr Ala Cys Thr Gly Thr Thr Cys Thr Thr Gly Cys Thr Ala
    50                  55                  60

Thr Thr Gly Gly Ala Ala Cys Thr Gly Cys Thr Ala Ala Thr Cys Cys
65                  70                  75                  80

Ala Cys Cys Ala Ala Ala Thr Thr Gly Thr Thr Ala Thr Thr Ala Thr
                85                  90                  95

Cys Ala Ala Gly Cys Thr Gly Ala Thr Thr Thr Cys Cys Ala Gly
            100                 105                 110

Ala Thr Thr Thr Thr Thr Ala Thr Thr Thr Thr Ala Gly Ala Gly Cys
            115                 120                 125

Thr Ala Cys Thr Ala Ala Thr Thr Cys Thr Gly Ala Thr Cys Ala Thr
    130                 135                 140

Cys Thr Thr Ala Cys Thr Cys Ala Thr Cys Thr Thr Ala Ala Gly Cys
145                 150                 155                 160

Ala Ala Ala Ala Gly Thr Thr Thr Ala Ala Gly Ala Gly Ala Ala Thr
                165                 170                 175

Thr Thr Gly Thr Gly Ala Ala Ala Ala Gly Thr Cys Thr Ala Thr Gly
            180                 185                 190

Ala Thr Thr Gly Ala Ala Ala Ala Gly Ala Gly Ala Thr Ala Thr Cys
        195                 200                 205

Thr Thr Cys Ala Thr Cys Thr Thr Ala Cys Thr Gly Ala Ala Gly Ala
    210                 215                 220

Ala Ala Thr Thr Cys Thr Thr Ala Ala Gly Gly Ala Ala Ala Ala Thr
225                 230                 235                 240

Cys Cys Ala Ala Ala Thr Ala Thr Thr Gly Cys Thr Thr Cys Thr Thr
                245                 250                 255

Thr Thr Gly Ala Ala Gly Cys Thr Cys Cys Ala Thr Cys Thr Cys Thr
            260                 265                 270

Thr Gly Ala Thr Gly Thr Thr Ala Gly Ala Cys Ala Thr Ala Ala Thr
        275                 280                 285

Ala Thr Thr Cys Ala Ala Gly Thr Thr Ala Ala Gly Gly Ala Ala Gly
    290                 295                 300

Thr Thr Gly Thr Thr Cys Thr Thr Cys Thr Thr Gly Gly Ala Ala Ala
305                 310                 315                 320

Gly Gly Ala Ala Gly Cys Thr Gly Cys Thr Cys Thr Thr Ala Ala Gly
                325                 330                 335

Gly Cys Thr Ala Thr Thr Ala Ala Thr Gly Ala Ala Thr Gly Gly Gly

340                 345                 350

Gly Ala Cys Ala Ala Cys Cys Ala Ala Ala Gly Thr Cys Thr Ala Ala
        355                 360                 365

Gly Ala Thr Thr Ala Cys Thr Ala Gly Ala Cys Thr Thr Ala Thr Thr
    370                 375                 380

Gly Thr Thr Thr Gly Thr Thr Gly Thr Ala Thr Thr Gly Cys Thr Gly
385                 390                 395                 400

Gly Ala Gly Thr Thr Gly Ala Thr Ala Thr Gly Cys Cys Ala Gly Gly
                405                 410                 415

Ala Gly Cys Thr Gly Ala Thr Thr Ala Thr Cys Ala Ala Cys Thr Thr
            420                 425                 430

Ala Cys Thr Ala Ala Gly Cys Thr Thr Cys Thr Thr Gly Gly Ala Cys
        435                 440                 445

Thr Thr Cys Ala Ala Cys Thr Thr Thr Cys Thr Gly Thr Thr Ala Ala
    450                 455                 460

Gly Ala Gly Ala Thr Thr Thr Ala Thr Gly Thr Thr Thr Thr Ala Thr
465                 470                 475                 480

Cys Ala Thr Cys Thr Thr Gly Gly Ala Thr Gly Thr Thr Ala Thr Gly
                485                 490                 495

Cys Thr Gly Gly Ala Gly Gly Ala Ala Cys Thr Gly Thr Thr Cys Thr
            500                 505                 510

Thr Ala Gly Ala Cys Thr Thr Gly Cys Thr Ala Ala Gly Gly Ala Thr
        515                 520                 525

Ala Thr Thr Gly Cys Thr Gly Ala Ala Ala Ala Thr Ala Ala Thr Ala
    530                 535                 540

Ala Gly Gly Ala Ala Gly Cys Thr Ala Gly Ala Gly Thr Thr Cys Thr
545                 550                 555                 560

Thr Ala Thr Thr Gly Thr Thr Ala Gly Ala Thr Cys Thr Gly Ala Ala
                565                 570                 575

Ala Thr Gly Ala Cys Thr Cys Cys Ala Ala Thr Thr Thr Gly Thr Thr
            580                 585                 590

Thr Thr Ala Gly Ala Gly Gly Ala Cys Cys Ala Thr Cys Thr Gly Ala
        595                 600                 605

Ala Ala Cys Thr Cys Ala Thr Ala Thr Thr Gly Ala Thr Thr Cys Thr
    610                 615                 620

Ala Thr Gly Gly Thr Thr Gly Gly Ala Cys Ala Ala Gly Cys Thr Ala
625                 630                 635                 640

Thr Thr Thr Thr Thr Gly Gly Ala Gly Ala Thr Gly Gly Ala Gly Cys
                645                 650                 655

Thr Gly Cys Thr Gly Cys Thr Gly Thr Thr Ala Thr Thr Gly Thr Thr
            660                 665                 670

Gly Gly Ala Gly Cys Thr Ala Ala Thr Cys Cys Ala Gly Ala Thr Cys
        675                 680                 685

Thr Thr Thr Cys Thr Ala Thr Thr Gly Ala Ala Ala Gly Ala Cys Cys
    690                 695                 700

Ala Ala Thr Thr Thr Thr Thr Gly Ala Ala Cys Thr Thr Ala Thr Thr
705                 710                 715                 720

Thr Cys Thr Ala Cys Thr Thr Cys Thr Cys Ala Ala Ala Cys Thr Ala
                725                 730                 735

Thr Thr Ala Thr Thr Cys Cys Ala Gly Ala Ala Thr Cys Thr Gly Ala
            740                 745                 750

Thr Gly Gly Ala Gly Cys Thr Ala Thr Thr Gly Ala Ala Gly Gly Ala
        755                 760                 765

Cys Ala Thr Cys Thr Thr Cys Thr Thr Gly Ala Ala Gly Thr Thr Gly

770                 775                 780

Gly Ala Cys Thr Thr Thr Cys Thr Thr Thr Thr Cys Ala Ala Cys Thr
785                 790                 795                 800

Thr Thr Ala Thr Cys Ala Ala Ala Cys Thr Gly Thr Thr Cys Cys Ala
                805                 810                 815

Thr Cys Thr Cys Thr Thr Ala Thr Thr Thr Cys Thr Ala Ala Thr Thr
            820                 825                 830

Gly Thr Ala Thr Thr Gly Ala Ala Ala Cys Thr Thr Gly Thr Cys Thr
        835                 840                 845

Thr Thr Cys Thr Ala Ala Gly Gly Cys Thr Thr Thr Thr Ala Cys Thr
    850                 855                 860

Cys Cys Ala Cys Thr Thr Ala Ala Thr Ala Thr Thr Thr Cys Thr Gly
865                 870                 875                 880

Ala Thr Thr Gly Gly Ala Ala Thr Thr Cys Thr Cys Thr Thr Thr Thr
                885                 890                 895

Thr Thr Gly Gly Ala Thr Thr Gly Cys Thr Cys Ala Thr Cys Cys Ala
            900                 905                 910

Gly Gly Ala Gly Gly Ala Ala Gly Ala Gly Cys Thr Ala Thr Thr Cys
        915                 920                 925

Thr Thr Gly Ala Thr Gly Ala Thr Ala Thr Thr Gly Ala Ala Gly Cys
    930                 935                 940

Thr Ala Cys Thr Gly Thr Thr Gly Gly Ala Cys Thr Thr Ala Ala Gly
945                 950                 955                 960

Ala Ala Gly Gly Ala Ala Ala Ala Gly Cys Thr Thr Ala Ala Gly Gly
                965                 970                 975

Cys Thr Ala Cys Thr Ala Gly Ala Cys Ala Ala Gly Thr Thr Cys Thr
            980                 985                 990

Thr Ala Ala Thr Gly Ala Thr Thr  Ala Thr Gly Gly Ala  Ala Ala Thr
        995                 1000                1005

Ala Thr  Gly Thr Cys Thr Thr  Cys Thr Gly Cys Thr  Thr Gly Thr
    1010                 1015                1020

Gly Thr  Thr Thr Thr Thr Thr  Thr Thr Ala Thr Thr  Ala Thr Gly
    1025                 1030                1035

Gly Ala  Thr Gly Ala Ala Ala  Thr Gly Ala Gly Ala  Ala Ala Gly
    1040                 1045                1050

Ala Ala  Gly Thr Cys Thr Cys  Thr Thr Gly Cys Thr  Ala Ala Thr
    1055                 1060                1065

Gly Gly  Ala Cys Ala Ala Gly  Thr Thr Ala Cys Thr  Ala Cys Thr
    1070                 1075                1080

Gly Gly  Ala Gly Ala Ala Gly  Gly Ala Cys Thr Thr  Ala Ala Gly
    1085                 1090                1095

Thr Gly  Gly Gly Gly Ala Gly  Thr Thr Cys Thr Thr  Thr Thr Thr
    1100                 1105                1110

Gly Gly  Ala Thr Thr Thr Gly  Gly Ala Cys Cys Ala  Gly Gly Ala
    1115                 1120                1125

Gly Thr  Thr Ala Cys Thr Gly  Thr Thr Gly Ala Ala  Ala Cys Thr
    1130                 1135                1140

Gly Thr  Thr Gly Thr Thr Cys  Thr Thr Thr Cys Thr  Thr Cys Thr
    1145                 1150                1155

Gly Thr  Thr Cys Cys Ala Cys  Thr Thr Ala Thr Thr  Ala Cys Thr
    1160                 1165                1170

<210>  133
<211>  391
<212>  PRT
<213>  Rheum tataricum

<400>  133

Met Ala Pro Glu Glu Ser Arg His Ala Glu Thr Ala Val Asn Arg Ala
1               5                   10                  15

Ala Thr Val Leu Ala Ile Gly Thr Ala Asn Pro Pro Asn Cys Tyr Tyr
            20                  25                  30

Gln Ala Asp Phe Pro Asp Phe Tyr Phe Arg Ala Thr Asn Ser Asp His
        35                  40                  45

Leu Thr His Leu Lys Gln Lys Phe Lys Arg Ile Cys Glu Lys Ser Met
    50                  55                  60

Ile Glu Lys Arg Tyr Leu His Leu Thr Glu Glu Ile Leu Lys Glu Asn
65                  70                  75                  80

Pro Asn Ile Ala Ser Phe Glu Ala Pro Ser Leu Asp Val Arg His Asn
                85                  90                  95

Ile Gln Val Lys Glu Val Val Leu Leu Gly Lys Glu Ala Ala Leu Lys
            100                 105                 110

Ala Ile Asn Glu Trp Gly Gln Pro Lys Ser Lys Ile Thr Arg Leu Ile
        115                 120                 125

Val Cys Cys Ile Ala Gly Val Asp Met Pro Gly Ala Asp Tyr Gln Leu
    130                 135                 140

Thr Lys Leu Leu Gly Leu Gln Leu Ser Val Lys Arg Phe Met Phe Tyr
145                 150                 155                 160

His Leu Gly Cys Tyr Ala Gly Gly Thr Val Leu Arg Leu Ala Lys Asp
                165                 170                 175

Ile Ala Glu Asn Asn Lys Glu Ala Arg Val Leu Ile Val Arg Ser Glu
            180                 185                 190

Met Thr Pro Ile Cys Phe Arg Gly Pro Ser Glu Thr His Ile Asp Ser
        195                 200                 205

Met Val Gly Gln Ala Ile Phe Gly Asp Gly Ala Ala Ala Val Ile Val
    210                 215                 220

Gly Ala Asn Pro Asp Leu Ser Ile Glu Arg Pro Ile Phe Glu Leu Ile
225                 230                 235                 240

Ser Thr Ser Gln Thr Ile Ile Pro Glu Ser Asp Gly Ala Ile Glu Gly
                245                 250                 255

His Leu Leu Glu Val Gly Leu Ser Phe Gln Leu Tyr Gln Thr Val Pro
            260                 265                 270

Ser Leu Ile Ser Asn Cys Ile Glu Thr Cys Leu Ser Lys Ala Phe Thr
        275                 280                 285

Pro Leu Asn Ile Ser Asp Trp Asn Ser Leu Phe Trp Ile Ala His Pro
    290                 295                 300

Gly Gly Arg Ala Ile Leu Asp Asp Ile Glu Ala Thr Val Gly Leu Lys
305                 310                 315                 320

Lys Glu Lys Leu Lys Ala Thr Arg Gln Val Leu Asn Asp Tyr Gly Asn
                325                 330                 335

Met Ser Ser Ala Cys Val Phe Phe Ile Met Asp Glu Met Arg Lys Lys
            340                 345                 350

Ser Leu Ala Asn Gly Gln Val Thr Thr Gly Glu Gly Leu Lys Trp Gly
        355                 360                 365

Val Leu Phe Gly Phe Gly Pro Gly Val Thr Val Glu Thr Val Val Leu
    370                 375                 380

Ser Ser Val Pro Leu Ile Thr
385                 390

<210>  134
<211>  1182
<212>  DNA
<213>  Artificial sequence

<220>

<223>  Nucleotide sequence PKS from Wachendorfia thyrsiflora,
       optimized for expression in Nicotiana benthamiana

<400>  134
atggcttcta ctgaaggaat tcaagcttat agaaataata tggctgaagg accagctact      60

attatggcta ttggaactgc taatccacca aatgttgttg atgcttctac ttttccagat     120

tattattgga gagttactaa ttctgaacat ctttctccag aatatagagt taagcttaag     180

agaatttgtg aaagatcttc tattagaaag agacatcttg ttcttactga acaacttctt     240

aaggaaaatc caactcttac tacttatgtt gatgcttctt atgatgaaag acaatctatt     300

gttcttgatg ctgttccaaa gcttgcttgt gaagctgctg ctaaggctat taaggaatgg     360

ggaagaccaa agactgatat tactcatatg gttgtttgta ctggagctgg agttgatgtt     420

ccaggagttg attataagat gatgaatctt cttggacttc caccaactgt taatagagtt     480

atgctttata atgttggatg tcatgcttct ggaactgttc ttagaattgc taaggatctt     540

gctgaaaata ataagggagc tagagttctt gttgtttctt ctgaagtttc tgttatgttt     600

tttagaggac cagctgaagg agatgttgaa attcttcttg gacaagctct ttttggagat     660

ggatctgctg ctattattgt tggagctgat ccaattgaag gagttgaaaa gccaattttt     720

caaatttttt ctgcttctca aatgactctt ccagaaggag aacatcttgt tgctggacat     780

cttagagaac ttggacttac ttttcatctt aagccacaac ttccaaatac tgtttcttct     840

aatattcata agccacttaa gaaggctttt gaaccactta atattactga ttggaattct     900

atttttggga ttgttcatcc aggaggaaga gctattcttg atcaagttca agaaaagatt     960

ggacttgaag aaaataagct tgatgtttct agatatgttc ttgctgaaaa tggaaatatg    1020

atgtctgctt ctgttttttt tattatggat gaaatgagaa agagatctgc tgctcaagga    1080

tgttctacta ctggagaagg acatgaatgg ggagttcttt ttggatttgg accaggactt    1140

tctattgaaa ctgttgttct tcattctgtt ccactttcta tt                       1182


<210>  135
<211>  394
<212>  PRT
<213>  Wachendorfia thyrsiflora

<400>  135

Met Ala Ser Thr Glu Gly Ile Gln Ala Tyr Arg Asn Asn Met Ala Glu
1               5                   10                  15

Gly Pro Ala Thr Ile Met Ala Ile Gly Thr Ala Asn Pro Pro Asn Val
            20                  25                  30

Val Asp Ala Ser Thr Phe Pro Asp Tyr Tyr Trp Arg Val Thr Asn Ser
        35                  40                  45

Glu His Leu Ser Pro Glu Tyr Arg Val Lys Leu Lys Arg Ile Cys Glu
    50                  55                  60

Arg Ser Ser Ile Arg Lys Arg His Leu Val Leu Thr Glu Gln Leu Leu
65                  70                  75                  80

Lys Glu Asn Pro Thr Leu Thr Thr Tyr Val Asp Ala Ser Tyr Asp Glu
                85                  90                  95

Arg Gln Ser Ile Val Leu Asp Ala Val Pro Lys Leu Ala Cys Glu Ala
            100                 105                 110

Ala Ala Lys Ala Ile Lys Glu Trp Gly Arg Pro Lys Thr Asp Ile Thr
        115                 120                 125

His Met Val Val Cys Thr Gly Ala Gly Val Asp Val Pro Gly Val Asp
    130                 135                 140

Tyr Lys Met Met Asn Leu Leu Gly Leu Pro Pro Thr Val Asn Arg Val
145                 150                 155                 160

Met Leu Tyr Asn Val Gly Cys His Ala Ser Gly Thr Val Leu Arg Ile
                165                 170                 175

Ala Lys Asp Leu Ala Glu Asn Asn Lys Gly Ala Arg Val Leu Val Val
            180                 185                 190

Ser Ser Glu Val Ser Val Met Phe Phe Arg Gly Pro Ala Glu Gly Asp
        195                 200                 205

Val Glu Ile Leu Leu Gly Gln Ala Leu Phe Gly Asp Gly Ser Ala Ala
    210                 215                 220

Ile Ile Val Gly Ala Asp Pro Ile Glu Gly Val Glu Lys Pro Ile Phe
225                 230                 235                 240

Gln Ile Phe Ser Ala Ser Gln Met Thr Leu Pro Glu Gly Glu His Leu
                245                 250                 255

Val Ala Gly His Leu Arg Glu Leu Gly Leu Thr Phe His Leu Lys Pro
            260                 265                 270

Gln Leu Pro Asn Thr Val Ser Ser Asn Ile His Lys Pro Leu Lys Lys
        275                 280                 285

Ala Phe Glu Pro Leu Asn Ile Thr Asp Trp Asn Ser Ile Phe Trp Ile
    290                 295                 300

Val His Pro Gly Gly Arg Ala Ile Leu Asp Gln Val Gln Glu Lys Ile
305                 310                 315                 320

Gly Leu Glu Glu Asn Lys Leu Asp Val Ser Arg Tyr Val Leu Ala Glu
                325                 330                 335

Asn Gly Asn Met Met Ser Ala Ser Val Phe Phe Ile Met Asp Glu Met
            340                 345                 350

Arg Lys Arg Ser Ala Ala Gln Gly Cys Ser Thr Thr Gly Glu Gly His
        355                 360                 365

Glu Trp Gly Val Leu Phe Gly Phe Gly Pro Gly Leu Ser Ile Glu Thr
    370                 375                 380

Val Val Leu His Ser Val Pro Leu Ser Ile
385                 390

<210>  136
<211>  1191
<212>  DNA
<213>  Artificial sequence

<220>
<223>  Nucleotide sequence PKS1 from Piper methysticum,
       optimized for expression in Nicotiana benthamiana

<400>  136

atgtctaaga ctgttgaaga tagagctgct caaagagcta agggaccagc tactgttctt 60

gctattggaa ctgctactcc agctaatgtt gtttatcaaa ctgattatcc agattattat 120

tttagagtta ctaagtctga acatatgact aagcttaaga ataagtttca aagaatgtgt 180

gatagatcta ctattaagaa gagatatatg gttcttactg aagaacttct tgaaaagaat 240

ctttctcttt gtacttatat ggaaccatct cttgatgcta gacaagatat tcttgttcca 300

gaagttccaa agcttggaaa ggaagctgct gatgaagcta ttgctgaatg gggaagacca 360

aagtctgaaa ttactcatct tattttttgt actacttgtg gagttgatat gccaggagct 420

gattatcaac ttactaagct tcttggactt agatcttctg ttagaagaac tatgctttat 480

caacaaggat gttttggagg aggaactgtt cttagacttg ctaaggatct tgctgaaaat 540

aatgctggag ctagagttct tgttgtttgt tctgaaatta ctactgctgt taattttaga 600

ggaccatctg atactcatct tgatcttctt gttggacttg ctctttttgg agatggagct 660

gctgctgtta ttgttggagc tgatccagat ccaactcttg aaagaccact ttttcaaatt 720

gtttctggag ctcaaactat tcttccagat tctgaaggag ctattaatgg acatcttaga 780

gaagttggac ttactattag acttcttaag gatgttccag gacttgtttc tatgaatatt 840

gaaaagtgtc ttatggaagc ttttgctcca atgggaattc atgattggaa ttctattttt 900

tggattgctc atccaggagg accaactatt cttgatcaag ttgaagctaa gcttggactt 960

aaggaagaaa agcttaagtc tactagagct gttcttagag aatatggaaa tatgtcttct 1020

gcttgtgttc tttttattct tgatgaagtt agaaagagat ctatggaaga aggaaagact 1080

actactggag aaggatttga ttggggagtt ctttttggat ttggaccagg atttactgtt 1140

gaaactgttg ttcttcattc tatgccaatt ccaaaggctg atgaaggaag a 1191

<210> 137
<211> 397
<212> PRT
<213> Piper methysticum

<400> 137

Met Ser Lys Thr Val Glu Asp Arg Ala Ala Gln Arg Ala Lys Gly Pro
1               5                   10                  15

Ala Thr Val Leu Ala Ile Gly Thr Ala Thr Pro Ala Asn Val Val Tyr
            20                  25                  30

Gln Thr Asp Tyr Pro Asp Tyr Tyr Phe Arg Val Thr Lys Ser Glu His
        35                  40                  45

Met Thr Lys Leu Lys Asn Lys Phe Gln Arg Met Cys Asp Arg Ser Thr
    50                  55                  60

Ile Lys Lys Arg Tyr Met Val Leu Thr Glu Glu Leu Leu Glu Lys Asn
65                  70                  75                  80

Leu Ser Leu Cys Thr Tyr Met Glu Pro Ser Leu Asp Ala Arg Gln Asp
                85                  90                  95

Ile Leu Val Pro Glu Val Pro Lys Leu Gly Lys Glu Ala Ala Asp Glu
            100                 105                 110

Ala Ile Ala Glu Trp Gly Arg Pro Lys Ser Glu Ile Thr His Leu Ile
        115                 120                 125

Phe Cys Thr Thr Cys Gly Val Asp Met Pro Gly Ala Asp Tyr Gln Leu
    130                 135                 140

Thr Lys Leu Leu Gly Leu Arg Ser Ser Val Arg Arg Thr Met Leu Tyr
145                 150                 155                 160

Gln Gln Gly Cys Phe Gly Gly Gly Thr Val Leu Arg Leu Ala Lys Asp
                165                 170                 175

Leu Ala Glu Asn Asn Ala Gly Ala Arg Val Leu Val Val Cys Ser Glu
            180                 185                 190

Ile Thr Thr Ala Val Asn Phe Arg Gly Pro Ser Asp Thr His Leu Asp
        195                 200                 205

Leu Leu Val Gly Leu Ala Leu Phe Gly Asp Gly Ala Ala Ala Val Ile
    210                 215                 220

Val Gly Ala Asp Pro Asp Pro Thr Leu Glu Arg Pro Leu Phe Gln Ile
225                 230                 235                 240

Val Ser Gly Ala Gln Thr Ile Leu Pro Asp Ser Glu Gly Ala Ile Asn
                245                 250                 255

Gly His Leu Arg Glu Val Gly Leu Thr Ile Arg Leu Leu Lys Asp Val
            260                 265                 270

Pro Gly Leu Val Ser Met Asn Ile Glu Lys Cys Leu Met Glu Ala Phe
        275                 280                 285

Ala Pro Met Gly Ile His Asp Trp Asn Ser Ile Phe Trp Ile Ala His
    290                 295                 300

Pro Gly Gly Pro Thr Ile Leu Asp Gln Val Glu Ala Lys Leu Gly Leu
305                 310                 315                 320

Lys Glu Glu Lys Leu Lys Ser Thr Arg Ala Val Leu Arg Glu Tyr Gly
                325                 330                 335

Asn Met Ser Ser Ala Cys Val Leu Phe Ile Leu Asp Glu Val Arg Lys
            340                 345                 350

Arg Ser Met Glu Glu Gly Lys Thr Thr Thr Gly Glu Gly Phe Asp Trp
        355                 360                 365

Gly Val Leu Phe Gly Phe Gly Pro Gly Phe Thr Val Glu Thr Val Val
    370                 375                 380

Leu His Ser Met Pro Ile Pro Lys Ala Asp Glu Gly Arg
385                 390                 395

<210>  138
<211>  1182
<212>  DNA
<213>  Artificial sequence

<220>
<223>  Nucleotide sequence of PKS2 from Piper methysticum,
       optimized for expression in Nicotiana benthamiana

<400>  138
atgtctaaga tggttgaaga acattgggct gctcaaagag ctagaggacc agctactgtt      60

cttgctattg gaactgctaa tccaccaaat gttctttatc aagctgatta tccagatttt     120

tattttagag ttactaagtc tgaacatatg actcaactta aggaaaagtt taagagaatt 180

tgtgataagt ctgctattag aaagagacat cttcatctta ctgaagaact tcttgaaaag 240

aatccaaata tttgtgctca tatggctcca tctcttgatg ctagacaaga tattgctgtt 300

gttgaagttc caaagcttgc taaggaagct gctactaagg ctattaagga atggggaaga 360

ccaaagtctg atattactca tcttattttt tgtactactt gtggagttga tatgccagga 420

gctgattatc aacttactac tcttcttgga cttagaccaa ctgttagaag aactatgctt 480

tatcaacaag gatgttttgc tggaggaact gttcttagac atgctaagga ttttgctgaa 540

aataatagag gagctagagt tcttgctgtt tgttctgaat ttactgttat gaatttttct 600

ggaccatctg aagctcatct tgattctatg gttggaatgg ctctttttgg agatggagct 660

tctgctgtta ttgttggagc tgatccagat tttgctattg aaagaccact tttcaacttt 720

gtttctacta ctcaaactat tgttccagat tctgatggag ctattaagtg tcatcttaag 780

gaagttggac ttactcttca tcttgttaag aatgttccag atcttatttc taataatatg 840

gataagattc ttgaagaagc ttttgctcca cttggaatta gagattggaa ttctattttt 900

tggactgctc atccaggagg agctgctatt cttgatcaac ttgaagctaa gcttggactt 960

aataaggaaa agcttaagac tactagaact gttcttagag aatatggaaa tatgtcttct 1020

gcttgtgttt gttttgttct tgatgaaatg agaagatctt ctcttgaaga aggaaagact 1080

acttctggag aaggacttga atggggaatt cttcttggat ttggaccagg acttactgtt 1140

gaaactgttg ttcttagatc tgttccaatt tctactgcta at 1182

<210> 139
<211> 394
<212> PRT
<213> Piper methysticum

<400> 139

Met Ser Lys Met Val Glu Glu His Trp Ala Ala Gln Arg Ala Arg Gly
1               5                   10                  15

Pro Ala Thr Val Leu Ala Ile Gly Thr Ala Asn Pro Pro Asn Val Leu
            20                  25                  30

Tyr Gln Ala Asp Tyr Pro Asp Phe Tyr Phe Arg Val Thr Lys Ser Glu
        35                  40                  45

His Met Thr Gln Leu Lys Glu Lys Phe Lys Arg Ile Cys Asp Lys Ser
    50                  55                  60

Ala Ile Arg Lys Arg His Leu His Leu Thr Glu Glu Leu Leu Glu Lys
65                  70                  75                  80

Asn Pro Asn Ile Cys Ala His Met Ala Pro Ser Leu Asp Ala Arg Gln
                85                  90                  95

Asp Ile Ala Val Val Glu Val Pro Lys Leu Ala Lys Glu Ala Ala Thr
            100                 105                 110

Lys Ala Ile Lys Glu Trp Gly Arg Pro Lys Ser Asp Ile Thr His Leu
        115                 120                 125

Ile Phe Cys Thr Thr Cys Gly Val Asp Met Pro Gly Ala Asp Tyr Gln
    130                 135                 140

Leu Thr Thr Leu Leu Gly Leu Arg Pro Thr Val Arg Arg Thr Met Leu
145                 150                 155                 160

Tyr Gln Gln Gly Cys Phe Ala Gly Gly Thr Val Leu Arg His Ala Lys
                165                 170                 175

Asp Phe Ala Glu Asn Asn Arg Gly Ala Arg Val Leu Ala Val Cys Ser
            180                 185                 190

Glu Phe Thr Val Met Asn Phe Ser Gly Pro Ser Glu Ala His Leu Asp
        195                 200                 205

Ser Met Val Gly Met Ala Leu Phe Gly Asp Gly Ala Ser Ala Val Ile
    210                 215                 220

Val Gly Ala Asp Pro Asp Phe Ala Ile Glu Arg Pro Leu Phe Gln Leu
225                 230                 235                 240

Val Ser Thr Thr Gln Thr Ile Val Pro Asp Ser Asp Gly Ala Ile Lys
                245                 250                 255

Cys His Leu Lys Glu Val Gly Leu Thr Leu His Leu Val Lys Asn Val
            260                 265                 270

Pro Asp Leu Ile Ser Asn Asn Met Asp Lys Ile Leu Glu Glu Ala Phe
        275                 280                 285

Ala Pro Leu Gly Ile Arg Asp Trp Asn Ser Ile Phe Trp Thr Ala His
    290                 295                 300

Pro Gly Gly Ala Ala Ile Leu Asp Gln Leu Glu Ala Lys Leu Gly Leu
305                 310                 315                 320

Asn Lys Glu Lys Leu Lys Thr Thr Arg Thr Val Leu Arg Glu Tyr Gly
                325                 330                 335

Asn Met Ser Ser Ala Cys Val Cys Phe Val Leu Asp Glu Met Arg Arg
            340                 345                 350

Ser Ser Leu Glu Glu Gly Lys Thr Thr Ser Gly Glu Gly Leu Glu Trp
        355                 360                 365

Gly Ile Leu Leu Gly Phe Gly Pro Gly Leu Thr Val Glu Thr Val Val
    370                 375                 380

Leu Arg Ser Val Pro Ile Ser Thr Ala Asn
385                 390

<210>  140
<211>  1734
<212>  DNA
<213>  Arabidopsis thaliana

<400>  140
atggaagaag attataagat ggctccacaa gaacaagctg tttctcaagt tatggaaaag      60

caatctaata ataataattc tgatgttatt tttagatcta agcttccaga tatttatatt     120

ccaaatcatc tttctcttca tgattatatt tttcaaaata tttctgaatt tgctactaag     180

ccatgtctta ttaatggacc aactggacat gtttatactt attctgatgt tcatgttatt     240

tctagacaaa ttgctgctaa ttttcataag cttggagtta atcaaaatga tgttgttatg     300

cttcttcttc caaattgtcc agaatttgtt ctttcttttc ttgctgcttc ttttagagga     360

gctactgcta ctgctgctaa tccatttttt actccagctg aaattgctaa gcaagctaag 420

gcttctaata ctaagcttat tattactgaa gctagatatg ttgataagat taagccactt 480

caaaatgatg atggagttgt tattgtttgt attgatgata atgaatctgt tccaattcca 540

gaaggatgtc ttagatttac tgaacttact caatctacta ctgaagcttc tgaagttatt 600

gattctgttg aaatttctcc agatgatgtt gttgctcttc catattcttc tggaactact 660

ggacttccaa agggagttat gcttactcat aagggacttg ttacttctgt tgctcaacaa 720

gttgatggag aaaatccaaa tctttatttt cattctgatg atgttattct ttgtgttctt 780

ccaatgtttc atatttatgc tcttaattct attatgcttt gtggacttag agttggagct 840

gctattctta ttatgccaaa gtttgaaatt aatcttcttc ttgaacttat tcaaagatgt 900

aaggttactg ttgctccaat ggttccacca attgttcttg ctattgctaa gtcttctgaa 960

actgaaaagt atgatctttc ttctattaga gttgttaagt ctggagctgc tccacttgga 1020

aaggaacttg aagatgctgt taatgctaag tttccaaatg ctaagcttgg acaaggatat 1080

ggaatgactg aagctggacc agttcttgct atgtctcttg gatttgctaa ggaaccattt 1140

ccagttaagt ctggagcttg tggaactgtt gttagaaatg ctgaaatgaa gattgttgat 1200

ccagatactg gagattctct ttctagaaat caaccaggag aaatttgtat tagaggacat 1260

caaattatga agggatatct taataatcca gctgctactg ctgaaactat tgataaggat 1320

ggatggcttc atactggaga tattggactt attgatgatg atgatgaact ttttattgtt 1380

gatagactta aggaacttat taagtataag ggatttcaag ttgctccagc tgaacttgaa 1440

gctcttctta ttggacatcc agatattact gatgttgctg ttgttgctat gaaggaagaa 1500

gctgctggag aagttccagt tgcttttgtt gttaagtcta aggattctga actttctgaa 1560

gatgatgtta agcaatttgt ttctaagcaa gttgtttttt ataagagaat taataaggtt 1620

ttttttactg aatctattcc aaaggctcca tctggaaaga ttcttagaaa ggatcttaga 1680

gctaagcttg ctaatggact ttctggatat ccatatgatg ttccagatta tgct 1734

<210> 141
<211> 578
<212> PRT
<213> Arabidopsis thaliana

<400> 141

Met Glu Glu Asp Tyr Lys Met Ala Pro Gln Glu Gln Ala Val Ser Gln
1               5                   10                  15

Val Met Glu Lys Gln Ser Asn Asn Asn Asn Ser Asp Val Ile Phe Arg
            20                  25                  30

Ser Lys Leu Pro Asp Ile Tyr Ile Pro Asn His Leu Ser Leu His Asp
        35                  40                  45

Tyr Ile Phe Gln Asn Ile Ser Glu Phe Ala Thr Lys Pro Cys Leu Ile
    50                  55                  60

Asn Gly Pro Thr Gly His Val Tyr Thr Tyr Ser Asp Val His Val Ile
65                  70                  75                  80

Ser Arg Gln Ile Ala Ala Asn Phe His Lys Leu Gly Val Asn Gln Asn
                85                  90                  95

Asp Val Val Met Leu Leu Leu Pro Asn Cys Pro Glu Phe Val Leu Ser
            100                 105                 110

Phe Leu Ala Ala Ser Phe Arg Gly Ala Thr Ala Thr Ala Ala Asn Pro
        115                 120                 125

Phe Phe Thr Pro Ala Glu Ile Ala Lys Gln Ala Lys Ala Ser Asn Thr
    130                 135                 140

Lys Leu Ile Ile Thr Glu Ala Arg Tyr Val Asp Lys Ile Lys Pro Leu
145                 150                 155                 160

Gln Asn Asp Asp Gly Val Val Ile Val Cys Ile Asp Asp Asn Glu Ser
                165                 170                 175

Val Pro Ile Pro Glu Gly Cys Leu Arg Phe Thr Glu Leu Thr Gln Ser
            180                 185                 190

Thr Thr Glu Ala Ser Glu Val Ile Asp Ser Val Glu Ile Ser Pro Asp
        195                 200                 205

Asp Val Val Ala Leu Pro Tyr Ser Ser Gly Thr Thr Gly Leu Pro Lys
    210                 215                 220

Gly Val Met Leu Thr His Lys Gly Leu Val Thr Ser Val Ala Gln Gln
225                 230                 235                 240

Val Asp Gly Glu Asn Pro Asn Leu Tyr Phe His Ser Asp Asp Val Ile
                245                 250                 255

Leu Cys Val Leu Pro Met Phe His Ile Tyr Ala Leu Asn Ser Ile Met
            260                 265                 270

Leu Cys Gly Leu Arg Val Gly Ala Ala Ile Leu Ile Met Pro Lys Phe
        275                 280                 285

Glu Ile Asn Leu Leu Leu Glu Leu Ile Gln Arg Cys Lys Val Thr Val
    290                 295                 300

Ala Pro Met Val Pro Pro Ile Val Leu Ala Ile Ala Lys Ser Ser Glu
305                 310                 315                 320

Thr Glu Lys Tyr Asp Leu Ser Ser Ile Arg Val Val Lys Ser Gly Ala
                325                 330                 335

Ala Pro Leu Gly Lys Glu Leu Glu Asp Ala Val Asn Ala Lys Phe Pro
            340                 345                 350

Asn Ala Lys Leu Gly Gln Gly Tyr Gly Met Thr Glu Ala Gly Pro Val
        355                 360                 365

Leu Ala Met Ser Leu Gly Phe Ala Lys Glu Pro Phe Pro Val Lys Ser
    370                 375                 380

Gly Ala Cys Gly Thr Val Val Arg Asn Ala Glu Met Lys Ile Val Asp
385                 390                 395                 400

Pro Asp Thr Gly Asp Ser Leu Ser Arg Asn Gln Pro Gly Glu Ile Cys
                405                 410                 415

Ile Arg Gly His Gln Ile Met Lys Gly Tyr Leu Asn Asn Pro Ala Ala
            420                 425                 430

Thr Ala Glu Thr Ile Asp Lys Asp Gly Trp Leu His Thr Gly Asp Ile
        435                 440                 445

Gly Leu Ile Asp Asp Asp Asp Glu Leu Phe Ile Val Asp Arg Leu Lys
    450                 455                 460

Glu Leu Ile Lys Tyr Lys Gly Phe Gln Val Ala Pro Ala Glu Leu Glu
465                 470                 475                 480

Ala Leu Leu Ile Gly His Pro Asp Ile Thr Asp Val Ala Val Val Ala
                485                 490                 495

Met Lys Glu Glu Ala Ala Gly Glu Val Pro Val Ala Phe Val Val Lys
            500                 505                 510

Ser Lys Asp Ser Glu Leu Ser Glu Asp Asp Val Lys Gln Phe Val Ser
        515                 520                 525

Lys Gln Val Val Phe Tyr Lys Arg Ile Asn Lys Val Phe Phe Thr Glu
    530                 535                 540

Ser Ile Pro Lys Ala Pro Ser Gly Lys Ile Leu Arg Lys Asp Leu Arg
545                 550                 555                 560

Ala Lys Leu Ala Asn Gly Leu Ser Gly Tyr Pro Tyr Asp Val Pro Asp
                565                 570                 575

Tyr Ala

LIST OF SEQUENCES

<110>  Light Bio, Inc

<120>  ENZYMES OF LUCIFERIN BIOSYNTHESIS AND USE THEREOF

<130> 413825

<150> RU 2018123601
<151> 2018-06-28

<160>  141

<170>  PatentIn version 3.5

<210>  1
<211>  1266
<212>  DNA
<213>  Neonothopanus nambi

<400>  1
atggcatcgt ttgagaattc tctaagcgtt ttgattgtcg gggccggact tggtgggctt      60

gctgctgcca tcgcgctgcg tcgccaaggg catgtcgtga aaatatacga ctcctctagc     120

ttcaaagccg aacttggtgc gggactcgct gtgccgccta acaccttgcg cagtctacag     180

caacttggtt gcaataccga gaacctcaat ggtgtggata atctttgctt cactgcgatg     240

gggtatgacg ggagtgtagg gatgatgaac aacatgactg actatcgaga ggcatacggt     300

acttcttgga tcatggtcca ccgcgttgac ttgcataacg agctgatgcg cgtagcactt     360

gatccaggtg ggctcggacc tcctgcgaca ctccatctta atcatcgtgt cacattctgc     420

gatgtcgacg cttgcaccgt gacattcacc aacggaccca ctcaatcagc tgatctcatc     480

gttggtgcag acggtatacg ctctaccatt cggcggtttg tcttagaaga agacgtgact     540

gtgcctgcgt caggaatcgt cgggtttcga tggcttgtac aagctgacgc gctggaccca     600

tatcctgaac tcgactggat tgttaaaaag cctcctctag gcgcgcgact gatctccact     660

cctcagaatc cacagtctgg tgttggcttg gctgacaggc gcactatcat catctacgca     720

tgtcgtggcg gcaccatggt caatgtcctt gcagtgcatg atgacgaacg tgaccagaac     780

accgcagatt ggagtgtacc ggcttccaaa gacgatctat ttcgtgtttt ccacgattac     840

catccacgct ttcggcggct tttagagctt gcgcaggata ttaatctctg gcaaatgcgt     900

gttgtacctg ttttgaaaaa atgggttaac aagcgggttt gcttgttagg agatgctgcg     960

cacgcttctt taccgacgtt gggtcaaggt tttggtatgg gtctggaaga tgccgtagca    1020

cttggtacac tccttccaaa gggtaccact gcatctcaga tcgagactcg acttgcggtg    1080

tacgaacagc tacgtaagga tcgtgcggaa tttgttgcgg ctgaatcata tgaagagcaa    1140

tatgttcctg aaatgcgggg actttatctg aggtcaaagg aactgcgtga tagagtcatg    1200

ggttatgata tcaaagtgga gagcgagaag gttctcgaga cgctcctaag aagttctaat    1260

tctgcc                                                                1266

<210>  2
<211>  422
<212>  PRT
<213>  Neonothopanus nambi

<400>  2

Met Ala Ser Phe Glu Asn Ser Leu Ser Val Leu Ile Val Gly Ala Gly
1               5                   10                  15

Leu Gly Gly Leu Ala Ala Ala Ile Ala Leu Arg Arg Gln Gly His Val
            20                  25                  30

Val Lys Ile Tyr Asp Ser Ser Ser Phe Lys Ala Glu Leu Gly Ala Gly
        35                  40                  45

Leu Ala Val Pro Pro Asn Thr Leu Arg Ser Leu Gln Gln Leu Gly Cys
    50                  55                  60

Asn Thr Glu Asn Leu Asn Gly Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly Thr Ser Trp Ile Met Val His Arg Val Asp Leu His
            100                 105                 110

Asn Glu Leu Met Arg Val Ala Leu Asp Pro Gly Gly Leu Gly Pro Pro
        115                 120                 125

Ala Thr Leu His Leu Asn His Arg Val Thr Phe Cys Asp Val Asp Ala
    130                 135                 140

Cys Thr Val Thr Phe Thr Asn Gly Thr Thr Gln Ser Ala Asp Leu Ile
145                 150                 155                 160

Val Gly Ala Asp Gly Ile Arg Ser Thr Ile Arg Arg Phe Val Leu Glu
                165                 170                 175

Glu Asp Val Thr Val Pro Ala Ser Gly Ile Val Gly Phe Arg Trp Leu
            180                 185                 190

Val Gln Ala Asp Ala Leu Asp Pro Tyr Pro Glu Leu Asp Trp Ile Val
        195                 200                 205

Lys Lys Pro Pro Leu Gly Ala Arg Leu Ile Ser Thr Pro Gln Asn Pro

210                 215                 220

Gln Ser Gly Val Gly Leu Ala Asp Arg Arg Thr Ile Ile Ile Tyr Ala
225                 230                 235                 240

Cys Arg Gly Gly Thr Met Val Asn Val Leu Ala Val His Asp Asp Glu
                245                 250                 255

Arg Asp Gln Asn Thr Ala Asp Trp Ser Val Pro Ala Ser Lys Asp Asp
            260                 265                 270

Leu Phe Arg Val Phe His Asp Tyr His Pro Arg Phe Arg Arg Leu Leu
        275                 280                 285

Glu Leu Ala Gln Asp Ile Asn Leu Trp Gln Met Arg Val Val Pro Val
    290                 295                 300

Leu Lys Lys Trp Val Asn Lys Arg Val Cys Leu Leu Gly Asp Ala Ala
305                 310                 315                 320

His Ala Ser Leu Pro Thr Leu Gly Gln Gly Phe Gly Met Gly Leu Glu
                325                 330                 335

Asp Ala Val Ala Leu Gly Thr Leu Leu Pro Lys Gly Thr Thr Ala Ser
            340                 345                 350

Gln Ile Glu Thr Arg Leu Ala Val Tyr Glu Gln Leu Arg Lys Asp Arg
        355                 360                 365

Ala Glu Phe Val Ala Ala Glu Ser Tyr Glu Glu Gln Tyr Val Pro Glu
    370                 375                 380

Met Arg Gly Leu Tyr Leu Arg Ser Lys Glu Leu Arg Asp Arg Val Met
385                 390                 395                 400

Gly Tyr Asp Ile Lys Val Glu Ser Glu Lys Val Leu Glu Thr Leu Leu
                405                 410                 415

Arg Ser Ser Asn Ser Ala
            420

<210>  3
<211>  1620
<212>  DNA
<213>  Omphalotus olearius

<400>  3
atgacgccct ccgagagtcc tttgaatatc tcgattgttg gtgctgggct cgggggcctt        60

gctgcagcta ttgcgctgcg tcgtcaaggt catatcatca gaatcttcga ctcgtcaagt 120

tttaaaacgg aactgggtgc tggacttgct gttccaccca atacattacg cagtcttcag 180

gaacttggct gtgatattca gaacttcaat gccgtggaca atctttgttt caccgcgatg 240

ggctacgacg ggagtgtagg gatgatgaac aatatgactg actatcgtga ggcgtatggt 300

gttccctggg tcatggtcca ccgcgttgac ctacataatg aactgcgacg tgtggcactc 360

gatccagatg gccttggacc tcctgcagca ttgcacctca atcatcgtgt gacatcctgc 420

gatgtcgatt cctgcaccgt cacattcgct aacggaaccg ctcatacagc ggatctcatc 480

gttggcgcgg atggtatacg ctcttccatc cgacccttcg tgttgggaga agacgtaatc 540

gtacctgcaa caggaatcgc aggatttcga tggctcatac aagccgaccg gctagatgcg 600

tatcctgaac tcgactggat tgtcaagaac cctcctctcg gcgcgcgatt gatttctgct 660

ccggctcgga aggaacgttc tgtaatcagc gaagcccggc ctgatcgacg tacgattata 720

ttatatgcgt gtcgtggtgg tactattgtc aatgtccttg cggtgcacga cgacgaacgt 780

gatcaggaca ccgtagaatg gagcgtgcca gctaccaaag acgacctatt tcgcgttttc 840

aacgattatc acccaagatt tcggcgactt ctggacctgg cggaggatgt taatctctgg 900

cagatgcgtg ttgtgcctgt tttgagacga tgggttaata aacgggtttg cttgctggga 960

gatgcagcac atgcttcttt accgacattg ggtcaaggtt ttggtatggg tctcgaggat 1020

gcggtggcac ttggtacgct tcttccgagc gggactactg tgtcacagat tgaaatccga 1080

ctttgggtgt atgaaaaact gcgcaaggag cgtgctgaat ttgtttcggc tgaatcgtat 1140

gaagaacact gctctgtgga ttgctataaa tctcataaag cccagtcgac atccaataca 1200

gtgacagaag cagacgacat cttggaagaa ccaaagcctc tgaagccttt atcgtctctg 1260

aaatggccgt acgttcccga agaaccttcg tatcctgatc ccctccaaag aaacgacccc 1320

aaacccctac aactcagaca ctacgaagca atagctacat ctcctgcagt acgggaggtc 1380

ctatcgagtc atccgaatct ccctgcttta ttgacgtcta tcgacaaact gagaggtttt 1440

gatcgcgaac gagctttaga aaaggcgttg gaggttactg cgcctgcgct tgttgatgat 1500

tcaagggctg tagcgctgga ggacgatgta ctcgcaatga gagcattggt agaagcgatt 1560

gaaggtgctg ttaggggcaa taaagaagac gcattaggtc tggattggac tggtagtact 1620

<210> 4
<211> 540
<212> PRT
<213> Omphalotus olearius

<400> 4

Met Thr Pro Ser Glu Ser Pro Leu Asn Ile Ser Ile Val Gly Ala Gly
1               5                   10                  15

Leu Gly Gly Leu Ala Ala Ala Ile Ala Leu Arg Arg Gln Gly His Ile
        20                  25                  30

Ile Arg Ile Phe Asp Ser Ser Ser Phe Lys Thr Glu Leu Gly Ala Gly
        35                  40                  45

Leu Ala Val Pro Pro Asn Thr Leu Arg Ser Leu Gln Glu Leu Gly Cys
    50                  55                  60

Asp Ile Gln Asn Phe Asn Ala Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly Val Pro Trp Val Met Val His Arg Val Asp Leu His
            100                 105                 110

Asn Glu Leu Arg Arg Val Ala Leu Asp Pro Asp Gly Leu Gly Pro Pro
        115                 120                 125

Ala Ala Leu His Leu Asn His Arg Val Thr Ser Cys Asp Val Asp Ser
    130                 135                 140

Cys Thr Val Thr Phe Ala Asn Gly Thr Ala His Thr Ala Asp Leu Ile
145                 150                 155                 160

Val Gly Ala Asp Gly Ile Arg Ser Ser Ile Arg Pro Phe Val Leu Gly
                165                 170                 175

Glu Asp Val Ile Val Pro Ala Thr Gly Ile Ala Gly Phe Arg Trp Leu
            180                 185                 190

Ile Gln Ala Asp Arg Leu Asp Ala Tyr Pro Glu Leu Asp Trp Ile Val
        195                 200                 205

Lys Asn Pro Pro Leu Gly Ala Arg Leu Ile Ser Ala Pro Ala Arg Lys
    210                 215                 220

Glu Arg Ser Val Ile Ser Glu Ala Arg Pro Asp Arg Arg Thr Ile Ile
225                 230                 235                 240

Leu Tyr Ala Cys Arg Gly Gly Thr Ile Val Asn Val Leu Ala Val His
                245                 250                 255

Asp Asp Glu Arg Asp Gln Asp Thr Val Glu Trp Ser Val Pro Ala Thr
            260                 265                 270

Lys Asp Asp Leu Phe Arg Val Phe Asn Asp Tyr His Pro Arg Phe Arg
        275                 280                 285

Arg Leu Leu Asp Leu Ala Glu Asp Val Asn Leu Trp Gln Met Arg Val
    290                 295                 300

Val Pro Val Leu Arg Arg Trp Val Asn Lys Arg Val Cys Leu Leu Gly
305                 310                 315                 320

Asp Ala Ala His Ala Ser Leu Pro Thr Leu Gly Gln Gly Phe Gly Met
                325                 330                 335

Gly Leu Glu Asp Ala Val Ala Leu Gly Thr Leu Leu Pro Ser Gly Thr
            340                 345                 350

Thr Val Ser Gln Ile Glu Ile Arg Leu Trp Val Tyr Glu Lys Leu Arg
        355                 360                 365

Lys Glu Arg Ala Glu Phe Val Ser Ala Glu Ser Tyr Glu Glu His Cys
    370                 375                 380

Ser Val Asp Cys Tyr Lys Ser His Lys Ala Gln Ser Thr Ser Asn Thr
385                 390                 395                 400

Val Thr Glu Ala Asp Asp Ile Leu Glu Glu Pro Lys Pro Leu Lys Pro
                405                 410                 415

Leu Ser Ser Leu Lys Trp Pro Tyr Val Pro Glu Glu Pro Ser Tyr Pro
            420                 425                 430

Asp Pro Leu Gln Arg Asn Asp Pro Lys Pro Leu Gln Leu Arg His Tyr
        435                 440                 445

Glu Ala Ile Ala Thr Ser Pro Ala Val Arg Glu Val Leu Ser Ser His
    450                 455                 460

Pro Asn Leu Pro Ala Leu Leu Thr Ser Ile Asp Lys Leu Arg Gly Phe
465                 470                 475                 480

Asp Arg Glu Arg Ala Leu Glu Lys Ala Leu Glu Val Thr Ala Pro Ala
                485                 490                 495

Leu Val Asp Asp Ser Arg Ala Val Ala Leu Glu Asp Asp Val Leu Ala
            500                 505                 510

Met Arg Ala Leu Val Glu Ala Ile Glu Gly Ala Val Arg Gly Asn Lys

515                 520                 525

Glu Asp Ala Leu Gly Leu Asp Trp Thr Gly Ser Thr
    530                 535                 540

<210>  5
<211>  1329
<212>  DNA
<213>  Guyanagaster necrorhiza

<400>  5
atgcaacaaa tcgacgaagt gtgcccattg aaagtgatcg ttgtaggtgc tggacttggg      60

ggcctttctg ctgccattgc ccttcgtagg caaggccatt gtgtccatat actggaatcg     120

tcaagtttca agagcgaact tggcgcaggt ctcgcagtac cgcccaatac tgtacgctca     180

cttcgaggcc taggctgtaa catcgacaat ctcaagcctg tggataatct ttgtttcact     240

gccatggcgc atgacggaag tcctggtatg atgaataaca tgacggacta tggccaggcg     300

tatggagatc cttgggtaat ggcgcatcgt gttgaccttc acaatgagct catgcgagtg     360

gcccttgaac ccgaaaaaac gggacctcct gcccagcttc gtctggacag ccaggtggca     420

tcttgcaatg tagatgcctg taccgtttct cttgtcgacg gaacaattta ttccgctgat     480

cttatcgttg gtgcagacgg aattaggtct accatacgct cctatgtttt ggacgcagaa     540

atagacatac ctcctaccgg tatcgctgga taccgttggc tcacacctgc agaagctttg     600

gagccatatc ccgaactcga ctggatcatc aagaacccac ccctaggagc acgtttaatc     660

acagctcctg tacgccgaaa cgacagcatt gagcagtcgg gtcctgctcc tatttctgag     720

aaggctgaca agcgtacgat catcatctac gcgtgccgga atggtactat gcttaacgtt     780

ctcggtgtac acgatgaccc tcgcaaccag aacgaagttg gatggaacgt gccagttacc     840

caagaaagtt tgctggattt ttttaaagac tatcatcccc gattcaagcg tctgcttgag     900

ctggctgaca atgttcatct gtggcaaatg cgcgtcgtcc cgcggcttga gacttggatc     960

aataaacgcg tgtgtttgtt gggcgattct gcgcatgcat cattaccaac actcggtcaa    1020

ggcttcggga tgggacttga ggatgccgta gcccttgcaa ccctccttcc gatgggaacc    1080

aaagtgtctg acatcgagaa ccgcctcgtc gcctacgaaa gcttgcgtaa ggggcgcgct    1140

gagtatgtgg ccatggaatc gttcgaacaa cagaatatcc cggaaaagcg aggcttgtat    1200

ctcaggtctt ctatgatgcg tgacgaaatc atgggttatg atgtcaaagc cgaggctgag    1260

aaggttttta aagaattaat gacctcgact gataaggtta cataccgtcc ccatgtggac    1320

tgtctatgg                                                            1329

<210>  6
<211>  443
<212>  PRT

<213>  Guyanagaster necrorhiza

<400>  6

Met Gln Gln Ile Asp Glu Val Cys Pro Leu Lys Val Ile Val Val Gly
1               5                   10                  15

Ala Gly Leu Gly Gly Leu Ser Ala Ala Ile Ala Leu Arg Arg Gln Gly
            20                  25                  30

His Cys Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Thr
65                  70                  75                  80

Ala Met Ala His Asp Gly Ser Pro Gly Met Met Asn Asn Met Thr Asp
                85                  90                  95

Tyr Gly Gln Ala Tyr Gly Asp Pro Trp Val Met Ala His Arg Val Asp
            100                 105                 110

Leu His Asn Glu Leu Met Arg Val Ala Leu Glu Pro Glu Lys Thr Gly
        115                 120                 125

Pro Pro Ala Gln Leu Arg Leu Asp Ser Gln Val Ala Ser Cys Asn Val
    130                 135                 140

Asp Ala Cys Thr Val Ser Leu Val Asp Gly Thr Ile Tyr Ser Ala Asp
145                 150                 155                 160

Leu Ile Val Gly Ala Asp Gly Ile Arg Ser Thr Ile Arg Ser Tyr Val
                165                 170                 175

Leu Asp Ala Glu Ile Asp Ile Pro Pro Thr Gly Ile Ala Gly Tyr Arg
            180                 185                 190

Trp Leu Thr Pro Ala Glu Ala Leu Glu Pro Tyr Pro Glu Leu Asp Trp
        195                 200                 205

Ile Ile Lys Asn Pro Pro Leu Gly Ala Arg Leu Ile Thr Ala Pro Val
    210                 215                 220

Arg Arg Asn Asp Ser Ile Glu Gln Ser Gly Pro Ala Pro Ile Ser Glu
225                 230                 235                 240

Lys Ala Asp Lys Arg Thr Ile Ile Ile Tyr Ala Cys Arg Asn Gly Thr
                245                 250                 255

Met Leu Asn Val Leu Gly Val His Asp Asp Pro Arg Asn Gln Asn Glu
            260                 265                 270

Val Gly Trp Asn Val Pro Val Thr Gln Glu Ser Leu Leu Asp Phe Phe
        275                 280                 285

Lys Asp Tyr His Pro Arg Phe Lys Arg Leu Leu Glu Leu Ala Asp Asn
    290                 295                 300

Val His Leu Trp Gln Met Arg Val Val Pro Arg Leu Glu Thr Trp Ile
305                 310                 315                 320

Asn Lys Arg Val Cys Leu Leu Gly Asp Ser Ala His Ala Ser Leu Pro
                325                 330                 335

Thr Leu Gly Gln Gly Phe Gly Met Gly Leu Glu Asp Ala Val Ala Leu
            340                 345                 350

Ala Thr Leu Leu Pro Met Gly Thr Lys Val Ser Asp Ile Glu Asn Arg
        355                 360                 365

Leu Val Ala Tyr Glu Ser Leu Arg Lys Gly Arg Ala Glu Tyr Val Ala
    370                 375                 380

Met Glu Ser Phe Glu Gln Gln Asn Ile Pro Glu Lys Arg Gly Leu Tyr
385                 390                 395                 400

Leu Arg Ser Ser Met Met Arg Asp Glu Ile Met Gly Tyr Asp Val Lys
                405                 410                 415

Ala Glu Ala Glu Lys Val Phe Lys Glu Leu Met Thr Ser Thr Asp Lys
            420                 425                 430

Val Thr Tyr Arg Pro His Val Asp Cys Leu Trp
        435                 440

<210>  7
<211>  1284
<212>  DNA
<213>  Panellus stipticus

<400>  7
atgtcacaag gcactgaaga ctcgccctca ctcttcgcaa tagttgtcgg tgctggtctg        60

gtcggctttg ccgctgccgt cgcgctgcgc cgtcaaggtc accgtgtcac gatctacgaa       120

tcttcaagct ttaagacaga acttggggcg ggcctcgcga tcccttcaaa cacattgcga 180

tgcttggttg gccttggatg tactgtcgcc aacatggatc ccgtcaataa tctttgtttt 240

acatcgatgg catacgatgg taccgcgggg atgaaaagcg acagctccga ctacgaggcg 300

cagtatggca ctccctggat catggcccac cgcgtcgatc tgcacaagga gcttcgtcgc 360

ctggcagtgg atcccgaggg caccggtccc cccgcagaac tgcaccttag ccaccgggtt 420

gtctcctgcg acgtcgaatc cggctccgtc acgctcctgg acggctccgt tcagtcagca 480

gacttgataa ttggggctga tggaattcgt tcaaccgttc gcaaatttgt cgtaggcgaa 540

gaaatagcaa tccccccatc tcgtacggcg ggcttccgct ggctcacaca agcaagcgct 600

cttgacccct accccgaact ggattggatc gtgaaaacgc cgccgttggg tgctcgggta 660

atctccgctc cgattcaacc tccagaagtc acccacatcg accaccgtac gatcgtcatc 720

tatgcctgtc gcggaggtcg tctcgtaaat gttctaggaa tacatgagga tctgcgcgac 780

caggattctg tcgactggaa cgtccccata acgcgagaag cgctgctcca ctttttcgga 840

gactaccacc cacggttcaa gcggctcttg gagctcgcgg aggacgtaca cgtctggcag 900

atgcgcgtgg tccccccact gccgacatgg gtgaacggcc gcgtatgcat catgggcgac 960

gcagcgcatg catcgcttcc cacactggga caaggtttgg gcatgggcct cgaagacgcg 1020

gtcgcgctcg ggacgctgct ttctagttcg acgccgtcaa gcgacattcc aagccgtctc 1080

gtcgcgtacg agaagcttcg caaagcgcgc gctgagtatg tttccaaaga gtcgtacgaa 1140

cagcagcatg tccgagaaaa gagagggctg tatctccggt cccgagaaat gcgggatgtg 1200

atcatgggat acgacgtgaa gaaggaggca gaacggatct tgagcgagat cagcattgca 1260

caagaacagt gtgctgttca tgat 1284

<210> 8
<211> 428
<212> PRT
<213> Panellus stipticus

<400> 8

Met Ser Gln Gly Thr Glu Asp Ser Pro Ser Leu Phe Ala Ile Val Val
1               5                   10                  15

Gly Ala Gly Leu Val Gly Phe Ala Ala Ala Val Ala Leu Arg Arg Gln
            20                  25                  30

Gly His Arg Val Thr Ile Tyr Glu Ser Ser Ser Phe Lys Thr Glu Leu
        35                  40                  45

Gly Ala Gly Leu Ala Ile Pro Ser Asn Thr Leu Arg Cys Leu Val Gly
    50                  55                  60

Leu Gly Cys Thr Val Ala Asn Met Asp Pro Val Asn Asn Leu Cys Phe
65                  70                  75                  80

Thr Ser Met Ala Tyr Asp Gly Thr Ala Gly Met Lys Ser Asp Ser Ser
                85                  90                  95

Asp Tyr Glu Ala Gln Tyr Gly Thr Pro Trp Ile Met Ala His Arg Val
            100                 105                 110

Asp Leu His Lys Glu Leu Arg Arg Leu Ala Val Asp Pro Glu Gly Thr
        115                 120                 125

Gly Pro Pro Ala Glu Leu His Leu Ser His Arg Val Val Ser Cys Asp
    130                 135                 140

Val Glu Ser Gly Ser Val Thr Leu Leu Asp Gly Ser Val Gln Ser Ala
145                 150                 155                 160

Asp Leu Ile Ile Gly Ala Asp Gly Ile Arg Ser Thr Val Arg Lys Phe
                165                 170                 175

Val Val Gly Glu Glu Ile Ala Ile Pro Pro Ser Arg Thr Ala Gly Phe
            180                 185                 190

Arg Trp Leu Thr Gln Ala Ser Ala Leu Asp Pro Tyr Pro Glu Leu Asp
        195                 200                 205

Trp Ile Val Lys Thr Pro Pro Leu Gly Ala Arg Val Ile Ser Ala Pro
    210                 215                 220

Ile Gln Pro Pro Glu Val Thr His Ile Asp His Arg Thr Ile Val Ile
225                 230                 235                 240

Tyr Ala Cys Arg Gly Gly Arg Leu Val Asn Val Leu Gly Ile His Glu
                245                 250                 255

Asp Leu Arg Asp Gln Asp Ser Val Asp Trp Asn Val Pro Ile Thr Arg
            260                 265                 270

Glu Ala Leu Leu His Phe Phe Gly Asp Tyr His Pro Arg Phe Lys Arg
        275                 280                 285

Leu Leu Glu Leu Ala Glu Asp Val His Val Trp Gln Met Arg Val Val
    290                 295                 300

Pro Pro Leu Pro Thr Trp Val Asn Gly Arg Val Cys Ile Met Gly Asp
305                 310                 315                 320

Ala Ala His Ala Ser Leu Pro Thr Leu Gly Gln Gly Phe Gly Met Gly
                325                 330                 335

Leu Glu Asp Ala Val Ala Leu Gly Thr Leu Leu Ser Ser Ser Thr Pro
            340                 345                 350

Ser Ser Asp Ile Pro Ser Arg Leu Val Ala Tyr Glu Lys Leu Arg Lys
        355                 360                 365

Ala Arg Ala Glu Tyr Val Ser Lys Glu Ser Tyr Glu Gln Gln His Val
    370                 375                 380

Arg Glu Lys Arg Gly Leu Tyr Leu Arg Ser Arg Glu Met Arg Asp Val
385                 390                 395                 400

Ile Met Gly Tyr Asp Val Lys Lys Glu Ala Glu Arg Ile Leu Ser Glu
                405                 410                 415

Ile Ser Ile Ala Gln Glu Gln Cys Ala Val His Asp
            420                 425

<210>  9
<211>  1284
<212>  DNA
<213>  Panellus stipticus

<400>  9
atgtcacaag gcactgaaga ctcgccctca ctcttcgcaa tagttgtcgg tgctggtctg      60

gtcggctttg ccgctgccgt cgcgctgcgc cgtcaaggtc accgtgtcac gatctacgaa     120

tcttcaagct ttaagacaga acttggggcg ggcctcgcga tcccttcaaa cacattgcga     180

tgcttggttg gccttggatg tactgtcgcc aacatggatc ccgtcaataa tctttgtttt     240

acatcgatgg catacgatgg taccgcgggg atgaaaagcg acagctccga ctacgaggcg     300

cagtatggca ctccctggat catggcccac cgcgtcgatc tgcacaagga gcttcgtcgc     360

ctggcagtgg atcccgaggg caccggtccc cccgcagaac tgcaccttag ccaccgggtt     420

gtctcctgcg acgtcgaatc cggctccgtc acgctcctgg acggctccgt tcagtcagca     480

gacttgataa ttggggctga tggaattcgt tcaaccgttc gcaaatttgt cgtaggcgaa     540

gaaatagcaa tccccccatc tcgtacggcg ggcttccgct ggctcacaca agcaagcgct     600

cttgacccct accccgaact ggattggatc gtgaaaacgc cgccgttggg tgctcgggta     660

atctccgctc cgattcaacc tccagaagtc acccacatcg accaccgtac gatcgtcatc     720

tatgcctgtc gcggaggtcg tctcgtaaat gttctaggaa tacatgagga tctgcgcgac     780

caggattctg tcgactggaa cgtccccata acgcgagaag cgctgctcca ctttttcgga     840

gactaccacc cacggttcaa gcggctcttg gagctcgcgg aggacgtaca cgtctggcag 900

atgcgcgtgg tccccccact gccgacatgg gtgaacggcc gcgtatgcat catgggcgac 960

gcagcgcatg catcgcttcc cacactggga caagggtttg gcatgggcct cgaagacgcg 1020

gtcgcgctcg ggacgctgct tcctagttcg acgccgtcaa gcgacattcc aagccgtctc 1080

gtcgcgtacg agaagcttcg caaagcgcgc gctgagtatg tttccaaaga gtcgtacgaa 1140

cagcagcatg tccgagaaaa gagagggctg tatctccggt cccgagaaat gcgggatgtg 1200

atcatgggat acgacgtgaa gaaggaggca gaacggatct tgagcgagat cagcattgca 1260

caagaacagt gtgctgttca tgat 1284

<210> 10
<211> 428
<212> PRT
<213> Panellus stipticus

<400> 10

Met Ser Gln Gly Thr Glu Asp Ser Pro Ser Leu Phe Ala Ile Val Val
1               5                   10                  15

Gly Ala Gly Leu Val Gly Phe Ala Ala Ala Val Ala Leu Arg Arg Gln
            20                  25                  30

Gly His Arg Val Thr Ile Tyr Glu Ser Ser Ser Phe Lys Thr Glu Leu
        35                  40                  45

Gly Ala Gly Leu Ala Ile Pro Ser Asn Thr Leu Arg Cys Leu Val Gly
    50                  55                  60

Leu Gly Cys Thr Val Ala Asn Met Asp Pro Val Asn Asn Leu Cys Phe
65                  70                  75                  80

Thr Ser Met Ala Tyr Asp Gly Thr Ala Gly Met Lys Ser Asp Ser Ser
                85                  90                  95

Asp Tyr Glu Ala Gln Tyr Gly Thr Pro Trp Ile Met Ala His Arg Val
            100                 105                 110

Asp Leu His Lys Glu Leu Arg Arg Leu Ala Val Asp Pro Glu Gly Thr
        115                 120                 125

Gly Pro Pro Ala Glu Leu His Leu Ser His Arg Val Val Ser Cys Asp
    130                 135                 140

Val Glu Ser Gly Ser Val Thr Leu Leu Asp Gly Ser Val Gln Ser Ala
145                 150                 155                 160

Asp Leu Ile Ile Gly Ala Asp Gly Ile Arg Ser Thr Val Arg Lys Phe
                165                 170                 175

Val Val Gly Glu Glu Ile Ala Ile Pro Pro Ser Arg Thr Ala Gly Phe
            180                 185                 190

Arg Trp Leu Thr Gln Ala Ser Ala Leu Asp Pro Tyr Pro Glu Leu Asp
        195                 200                 205

Trp Ile Val Lys Thr Pro Pro Leu Gly Ala Arg Val Ile Ser Ala Pro
    210                 215                 220

Ile Gln Pro Pro Glu Val Thr His Ile Asp His Arg Thr Ile Val Ile
225                 230                 235                 240

Tyr Ala Cys Arg Gly Gly Arg Leu Val Asn Val Leu Gly Ile His Glu
                245                 250                 255

Asp Leu Arg Asp Gln Asp Ser Val Asp Trp Asn Val Pro Ile Thr Arg
            260                 265                 270

Glu Ala Leu Leu His Phe Phe Gly Asp Tyr His Pro Arg Phe Lys Arg
        275                 280                 285

Leu Leu Glu Leu Ala Glu Asp Val His Val Trp Gln Met Arg Val Val
    290                 295                 300

Pro Pro Leu Pro Thr Trp Val Asn Gly Arg Val Cys Ile Met Gly Asp
305                 310                 315                 320

Ala Ala His Ala Ser Leu Pro Thr Leu Gly Gln Gly Phe Gly Met Gly
                325                 330                 335

Leu Glu Asp Ala Val Ala Leu Gly Thr Leu Leu Pro Ser Ser Thr Pro
            340                 345                 350

Ser Ser Asp Ile Pro Ser Arg Leu Val Ala Tyr Glu Lys Leu Arg Lys
        355                 360                 365

Ala Arg Ala Glu Tyr Val Ser Lys Glu Ser Tyr Glu Gln Gln His Val
    370                 375                 380

Arg Glu Lys Arg Gly Leu Tyr Leu Arg Ser Arg Glu Met Arg Asp Val
385                 390                 395                 400

Ile Met Gly Tyr Asp Val Lys Lys Glu Ala Glu Arg Ile Leu Ser Glu

405                 410                 415

Ile Ser Ile Ala Gln Glu Gln Cys Ala Val His Asp
            420                 425

<210>  11
<211>  1284
<212>  DNA
<213>  Panellus stipticus

<400>  11
atgtcacaag gcactgaaga ctcgccctca ctcttcgcaa tagttgtcgg tgctggtctg      60

gtcggctttg ccgctgccgt cgcgctgcgc cgtcaaggtc accgtgtcac tatctacgaa     120

tcttcaagct ttaagacaga acttggggcg ggcctcgcga tcccttcaaa cacattgcga     180

tgcttggttg gccttggatg tactgtcgcc aatctggatc ccgtcaataa tctttgtttt     240

acatcgatgg cttacgatgg taccgcgggg atgaaaagcg acagctccga ctacgaggcg     300

cagtatggca ctccctggat catggcccac cgcgtcgatc tgcacaagga gcttcgtcgc     360

ctggcagtgg atcccgaggg caccggtccc cccgcagaac tgcaccttag ccaccgggtt     420

gtctcctgcg acgtcgaatc cggctccgtc acgctcctgg acggctccat tcagtcagca     480

gacttgataa ttggggctga tggaattcgt tcaaccgttc gcaaatttgt cgtaggcgaa     540

gaaatagcaa tccccccatc tcgtacggcg ggcttccgtt ggctcacaca agcaagcgct     600

cttgacccct accccgaact ggattggatc gtgaaaacgc cgccgttggg tgctcgggta     660

atctccgctc cgattcaacc tccagaagtc acccacatcg accaccgtac gatcgtcatc     720

tatgcctgtc gcggaggtcg tctcgtaaat gttctaggaa tacatgagga tctgcgcgac     780

caggattctg tcgactggaa cgtccccata acgcgagaag cgctgctcca ctttttcgga     840

gactaccacc cacggttcaa gcggctcttg gagctcgcgg aggacgtaca cgtctggcag     900

atgcgcgtgg tccccccact gccgacatgg gtgaacggcc gcgtatgcat catgggcgac     960

gcagcgcatg catcgcttcc cacactggga caagggtttg gcatgggcct cgaagacgcg    1020

gtcgcgctcg ggacgctgct tcctagttcg acgccgtcag gcgacattcc aagccgtctc    1080

gtcgcgtacg agaagcttcg caaagcgcgc gctgagtatg tttccaaaga gtcgtacgaa    1140

cagcagcatg tccgagaaaa gagagggctg tatctccggt cccgagaaat gcgggatgtg    1200

atcatgggat acgacgtgaa gaaggaggca gaacggatct ttagcgagat cagcattgca    1260

caagaacagc gtgctgttca tgat                                          1284

<210>  12
<211>  428
<212>  PRT
<213>  Panellus stipticus

<400>  12

Met Ser Gln Gly Thr Glu Asp Ser Pro Ser Leu Phe Ala Ile Val Val
1               5                   10                  15

Gly Ala Gly Leu Val Gly Phe Ala Ala Ala Val Ala Leu Arg Arg Gln
            20                  25                  30

Gly His Arg Val Thr Ile Tyr Glu Ser Ser Ser Phe Lys Thr Glu Leu
        35                  40                  45

Gly Ala Gly Leu Ala Ile Pro Ser Asn Thr Leu Arg Cys Leu Val Gly
    50                  55                  60

Leu Gly Cys Thr Val Ala Asn Leu Asp Pro Val Asn Asn Leu Cys Phe
65                  70                  75                  80

Thr Ser Met Ala Tyr Asp Gly Thr Ala Gly Met Lys Ser Asp Ser Ser
                85                  90                  95

Asp Tyr Glu Ala Gln Tyr Gly Thr Pro Trp Ile Met Ala His Arg Val
            100                 105                 110

Asp Leu His Lys Glu Leu Arg Arg Leu Ala Val Asp Pro Glu Gly Thr
        115                 120                 125

Gly Pro Pro Ala Glu Leu His Leu Ser His Arg Val Val Ser Cys Asp
    130                 135                 140

Val Glu Ser Gly Ser Val Thr Leu Leu Asp Gly Ser Ile Gln Ser Ala
145                 150                 155                 160

Asp Leu Ile Ile Gly Ala Asp Gly Ile Arg Ser Thr Val Arg Lys Phe
                165                 170                 175

Val Val Gly Glu Glu Ile Ala Ile Pro Pro Ser Arg Thr Ala Gly Phe
            180                 185                 190

Arg Trp Leu Thr Gln Ala Ser Ala Leu Asp Pro Tyr Pro Glu Leu Asp
        195                 200                 205

Trp Ile Val Lys Thr Pro Pro Leu Gly Ala Arg Val Ile Ser Ala Pro
    210                 215                 220

Ile Gln Pro Pro Glu Val Thr His Ile Asp His Arg Thr Ile Val Ile
225                 230                 235                 240

Tyr Ala Cys Arg Gly Gly Arg Leu Val Asn Val Leu Gly Ile His Glu

245                 250                 255

Asp Leu Arg Asp Gln Asp Ser Val Asp Trp Asn Val Pro Ile Thr Arg
            260                 265                 270

Glu Ala Leu Leu His Phe Phe Gly Asp Tyr His Pro Arg Phe Lys Arg
        275                 280                 285

Leu Leu Glu Leu Ala Glu Asp Val His Val Trp Gln Met Arg Val Val
    290                 295                 300

Pro Pro Leu Pro Thr Trp Val Asn Gly Arg Val Cys Ile Met Gly Asp
305                 310                 315                 320

Ala Ala His Ala Ser Leu Pro Thr Leu Gly Gln Gly Phe Gly Met Gly
                325                 330                 335

Leu Glu Asp Ala Val Ala Leu Gly Thr Leu Leu Pro Ser Ser Thr Pro
            340                 345                 350

Ser Gly Asp Ile Pro Ser Arg Leu Val Ala Tyr Glu Lys Leu Arg Lys
        355                 360                 365

Ala Arg Ala Glu Tyr Val Ser Lys Glu Ser Tyr Glu Gln Gln His Val
    370                 375                 380

Arg Glu Lys Arg Gly Leu Tyr Leu Arg Ser Arg Glu Met Arg Asp Val
385                 390                 395                 400

Ile Met Gly Tyr Asp Val Lys Lys Glu Ala Glu Arg Ile Phe Ser Glu
                405                 410                 415

Ile Ser Ile Ala Gln Glu Gln Arg Ala Val His Asp
            420                 425

<210>  13
<211>  1248
<212>  DNA
<213>  Neonothopanus gardneri

<400>  13
atggcactat ctgagagtcc tttgaacgtc ttgatagtag gagcggggct cgggggcctt      60

gctgctgcca tagcactacg tcgtcaaggg catatcgtga aaatattcga ttcctccagt     120

ttcaaaaccg aacttggtgc aggacttgct gtcccgccta ataccctgcg tagtctgcag     180

gaactcgggt gcagtgtcga gaacctcaat gctgtggata atctttgctt cactgcgatg     240

gggtatgacg ggagtgtagg aatgatgaac aatatgaccg actatcgaga ggcgtacggt     300

catccttggg tcatggttca ccgtgtcgac ttgcataatg agctgaagcg cgtggcgctt 360

gatccagacg gcctcggacc tcctgcaact ttgcatctca accatcgtgt cacattctgc 420

gacatcgact cttgcactgt cacgttcgct aatggacttt ctaaatcggc agatcttatc 480

gtaggcgcag acggtatacg ctctaccatt cgcaagttca ttcttggaga agacgtcgtt 540

atacccgcgt caggaatagc agggtttcga tggcttgtgc aagctgacgc gctggatccg 600

tatcctgaac tcgactggat cgtgaagaac cctcctctag gagcccgact gatttccgct 660

cctaggaatc aacagtctac tgataggcgc actatcatca tctatgcgtg tcgtagcggc 720

accatggtca acgtactcgc agtacatgat gatgatcgtg accagaacgc cgtagattgg 780

agtgcaccag cttccaaaga tgatttattc cacatcttcc acgactacca cccacgattc 840

cagcggcttc tggagctggc gcaagatatc aatctctggc aaatgcgtgt tgttcctgtt 900

ctgaaacaat gggttaacaa acgtgtttgc ttgttaggag atgcggcaca cgcttcttta 960

cctacattag ggcagggatt tggtatgggt ctagaagatg ccgtagcact gggtacgctt 1020

cttccaaagg gggccacagt atctcagatc gagagccgac tcgcggtgta tgaaattctg 1080

cgcaaggagc gtgctgaatt tgtttcggct gagtcatatg aagagcagta cgttccagaa 1140

aaacgcgggc tttacttaag atcgaaggaa ttgcgcgaca gtgtcatggg ttacgatatt 1200

aaaatggaga gcgagaaggt tctcgtggca ttacttagcg gttcttca 1248

<210> 14
<211> 416
<212> PRT
<213> Neonothopanus gardneri

<400> 14

Met Ala Leu Ser Glu Ser Pro Leu Asn Val Leu Ile Val Gly Ala Gly
1               5                   10                  15

Leu Gly Gly Leu Ala Ala Ala Ile Ala Leu Arg Arg Gln Gly His Ile
            20                  25                  30

Val Lys Ile Phe Asp Ser Ser Ser Phe Lys Thr Glu Leu Gly Ala Gly
        35                  40                  45

Leu Ala Val Pro Pro Asn Thr Leu Arg Ser Leu Gln Glu Leu Gly Cys
    50                  55                  60

Ser Val Glu Asn Leu Asn Ala Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly His Pro Trp Val Met Val His Arg Val Asp Leu His
            100                 105                 110

Asn Glu Leu Lys Arg Val Ala Leu Asp Pro Asp Gly Leu Gly Pro Pro
        115                 120                 125

Ala Thr Leu His Leu Asn His Arg Val Thr Phe Cys Asp Ile Asp Ser
    130                 135                 140

Cys Thr Val Thr Phe Ala Asn Gly Thr Ser Lys Ser Ala Asp Leu Ile
145                 150                 155                 160

Val Gly Ala Asp Gly Ile Arg Ser Thr Ile Arg Lys Phe Ile Leu Gly
                165                 170                 175

Glu Asp Val Val Ile Pro Ala Ser Gly Ile Ala Gly Phe Arg Trp Leu
            180                 185                 190

Val Gln Ala Asp Ala Leu Asp Pro Tyr Pro Glu Leu Asp Trp Ile Val
        195                 200                 205

Lys Asn Pro Pro Leu Gly Ala Arg Leu Ile Ser Ala Pro Arg Asn Gln
    210                 215                 220

Gln Ser Thr Asp Arg Arg Thr Ile Ile Ile Tyr Ala Cys Arg Ser Gly
225                 230                 235                 240

Thr Met Val Asn Val Leu Ala Val His Asp Asp Asp Arg Asp Gln Asn
                245                 250                 255

Ala Val Asp Trp Ser Ala Pro Ala Ser Lys Asp Asp Leu Phe His Ile
            260                 265                 270

Phe His Asp Tyr His Pro Arg Phe Gln Arg Leu Leu Glu Leu Ala Gln
        275                 280                 285

Asp Ile Asn Leu Trp Gln Met Arg Val Val Pro Val Leu Lys Gln Trp
    290                 295                 300

Val Asn Lys Arg Val Cys Leu Leu Gly Asp Ala Ala His Ala Ser Leu
305                 310                 315                 320

Pro Thr Leu Gly Gln Gly Phe Gly Met Gly Leu Glu Asp Ala Val Ala
                325                 330                 335

Leu Gly Thr Leu Leu Pro Lys Gly Ala Thr Val Ser Gln Ile Glu Ser
            340                 345                 350

Arg Leu Ala Val Tyr Glu Ile Leu Arg Lys Glu Arg Ala Glu Phe Val
        355                 360                 365

Ser Ala Glu Ser Tyr Glu Glu Gln Tyr Val Pro Glu Lys Arg Gly Leu
    370                 375                 380

Tyr Leu Arg Ser Lys Glu Leu Arg Asp Ser Val Met Gly Tyr Asp Ile
385                 390                 395                 400

Lys Met Glu Ser Glu Lys Val Leu Val Ala Leu Leu Ser Gly Ser Ser
                405                 410                 415

<210>  15
<211>  1257
<212>  DNA
<213>  Mycena citricolor

<400>  15
atgaacaccc ccaataacgc tctcgatgtt attgttgtcg gtgctggcct ggttggtttc      60

gcggccgctg ctgctctacg ccgacaaggt catcgcgtga ctatctacga gacttccagc     120

ttcaagaacg agctaggagc tggccttgct attccaccca acactgtccg tggcctaatt     180

ggcttgggat gtgtgattga gaacttggac ccggtggaga atctatgcgt ttccgtcgct     240

tttgacggaa gtgctggtat gcgcagtgac cagaccaact acgaagcaag ctacggcctt     300

ccctggatca tggtgcatcg cgtcgatttg cacaatgagc ttcgtcgggt tgctctcagt     360

gccgagggga acattggtcc cccagccgag ctacgcctgg accaccgagt cagctcgtgt     420

gatgtcgaga aatgcactgt gacgctgagc aatggcgata cccaccacgc ggatctgatc     480

attggagcag acgggatcca ttctacaatc cgatccttcg tcgtgggcga ggaaatcgtc     540

attccgccct ccaagacagc cggtttccgc tggctcacag agagtactgc gttggagccc     600

tatccggaat tggactggat tgtgaagatc ccaccacttg gcgcccggct gatctctgcg     660

ccaatgaacc ctgcgccacc gcaggtcgac caccggacga tcatcatcta cgcctgtcgt     720

ggcagtacac tgataaatgt actcggagtc catgaggatc tccgcgatca agatacagtt     780

ccctggaatg cacccgtaac ccaatcggag ctgctccagt tctttggcga ttaccatccg     840

cgattcaaac gattgttgga gcttgcaaat gatgttcatg tgtggcagat gcgagtagtg     900

ccccgcttgg agacctgggt caatcgtcgg gtttgcatta tgggcgatgc tgcgcatgca     960

tcactcccca cgctgggtca aggtttcgga atggggctcg aggatgcagt cgctcttgga    1020

acactccttc cgcttgggac aactcccgaa gagatcccgg accgtctcac cctctggcag    1080

gatctcgtca aacctcgggc tgagtttgtc gcgactgaat cctacgaaca gcagcatatt    1140

cctgcgaaac ggggactcta tcttcgctcg caggagatgc gcgactgggt catgggatac    1200

gatgtccagg ctgaggcaca gaaggtcttg gcgggagctg tgaatagatc caaggga          1257

<210>  16
<211>  419
<212>  PRT
<213>  Mycena citricolor

<400>  16

Met Asn Thr Pro Asn Asn Ala Leu Asp Val Ile Val Val Gly Ala Gly
1               5                   10                  15

Leu Val Gly Phe Ala Ala Ala Ala Ala Leu Arg Arg Gln Gly His Arg
            20                  25                  30

Val Thr Ile Tyr Glu Thr Ser Ser Phe Lys Asn Glu Leu Gly Ala Gly
        35                  40                  45

Leu Ala Ile Pro Pro Asn Thr Val Arg Gly Leu Ile Gly Leu Gly Cys
    50                  55                  60

Val Ile Glu Asn Leu Asp Pro Val Glu Asn Leu Cys Val Ser Val Ala
65                  70                  75                  80

Phe Asp Gly Ser Ala Gly Met Arg Ser Asp Gln Thr Asn Tyr Glu Ala
                85                  90                  95

Ser Tyr Gly Leu Pro Trp Ile Met Val His Arg Val Asp Leu His Asn
            100                 105                 110

Glu Leu Arg Arg Val Ala Leu Ser Ala Glu Gly Asn Ile Gly Pro Pro
        115                 120                 125

Ala Glu Leu Arg Leu Asp His Arg Val Ser Ser Cys Asp Val Glu Lys
    130                 135                 140

Cys Thr Val Thr Leu Ser Asn Gly Asp Thr His His Ala Asp Leu Ile
145                 150                 155                 160

Ile Gly Ala Asp Gly Ile His Ser Thr Ile Arg Ser Phe Val Val Gly
                165                 170                 175

Glu Glu Ile Val Ile Pro Pro Ser Lys Thr Ala Gly Phe Arg Trp Leu
            180                 185                 190

Thr Glu Ser Thr Ala Leu Glu Pro Tyr Pro Glu Leu Asp Trp Ile Val
        195                 200                 205

Lys Ile Pro Pro Leu Gly Ala Arg Leu Ile Ser Ala Pro Met Asn Pro
    210                 215                 220

Ala Pro Pro Gln Val Asp His Arg Thr Ile Ile Ile Tyr Ala Cys Arg
225                 230                 235                 240

Gly Ser Thr Leu Ile Asn Val Leu Gly Val His Glu Asp Leu Arg Asp
                245                 250                 255

Gln Asp Thr Val Pro Trp Asn Ala Pro Val Thr Gln Ser Glu Leu Leu
            260                 265                 270

Gln Phe Phe Gly Asp Tyr His Pro Arg Phe Lys Arg Leu Leu Glu Leu
        275                 280                 285

Ala Asn Asp Val His Val Trp Gln Met Arg Val Val Pro Arg Leu Glu
    290                 295                 300

Thr Trp Val Asn Arg Arg Val Cys Ile Met Gly Asp Ala Ala His Ala
305                 310                 315                 320

Ser Leu Pro Thr Leu Gly Gln Gly Phe Gly Met Gly Leu Glu Asp Ala
                325                 330                 335

Val Ala Leu Gly Thr Leu Leu Pro Leu Gly Thr Thr Pro Glu Glu Ile
            340                 345                 350

Pro Asp Arg Leu Thr Leu Trp Gln Asp Leu Val Lys Pro Arg Ala Glu
        355                 360                 365

Phe Val Ala Thr Glu Ser Tyr Glu Gln Gln His Ile Pro Ala Lys Arg
    370                 375                 380

Gly Leu Tyr Leu Arg Ser Gln Glu Met Arg Asp Trp Val Met Gly Tyr
385                 390                 395                 400

Asp Val Gln Ala Glu Ala Gln Lys Val Leu Ala Gly Ala Val Asn Arg
                405                 410                 415

Ser Lys Gly

<210>  17
<211>  1260
<212>  DNA
<213>  Mycena citricolor

<400>  17
atgaacaccc ccaataacgc tctcgatgtt attgttgtcg gtgctggcct ggttggtttc        60

gcggccgctg ctgctctacg ccgacaaggt catcgcgtga ctatctatga gacttccagc 120

ttcaagaacg agttaggagc tggcctggct attccaccca acactgtccg tggtctaatt 180

ggcttgggat gtgtgattga gaacttggac ccggtggaga atctatgctt cactgccgtc 240

gcgtttgacg gaagtgctgg tatgcgcagc gaccaaacca actacgaagc aagttacggc 300

cttccctgga tcatggtgca tcgtgtcgat ttgcacaacg agcttcgtcg ggttgctctc 360

agcgccgagg ggaacactgg tcccccagcg gagctacgcc tggaccaccg agtcagctcg 420

tgtgatgtcg agaaatgcac tgtgacgctg agcaatgggg atacccacca cgcagatctg 480

atcattggag cagacgggat ccattctaca atccgatcct tcgttgtggg cgaggaaatc 540

gtcattccgc cctccaagac agccggtttc cgctggctca cagagagtac tgcgttggag 600

ccctatccgg aattggactg gattgtgaag atcccaccac ttggcgcccg gctgatctct 660

gcgccaatga accctgcgcc accgcaggtc gaccaccgga cgatcatcat ctacgcctgt 720

cgtggcagta cactgataaa tgtactcgga gtccatgagg atctccgcga tcaagataca 780

gtcccctgga atgcacccgt aacccaatcg gagctgctcc agttctttgg cgattaccat 840

ccgcggttca aacgattgtt ggagcttgca aatgatgttc atgtgtggca gatgcgagta 900

gtgccccgct tggagacctg ggtcaatcgt cgggtttgca ttatgggcga tgctgcgcat 960

gcatcactcc ccacgctggg tcaaggtttc ggaatggggc tcgaggatgc agtcgctctt 1020

ggaacactcc ttccgcttgg gacaactccc gaagagatcc cggaccgtct caccctctgg 1080

caggatctcg tcaaacctcg ggctgagttt gtcgcgactg aatcctacga acagcagcat 1140

attcctgcga aacggggact ctatcttcgc tcgcaggaga tgcgcgactg ggtcatggga 1200

tacgatgtcc aggctgaggc acagaaggtc ttggcgggag ctgtgaatag atccaaggga 1260

<210> 18
<211> 420
<212> PRT
<213> Mycena citricolor

<400> 18

Met Asn Thr Pro Asn Asn Ala Leu Asp Val Ile Val Val Gly Ala Gly
1               5                   10                  15

Leu Val Gly Phe Ala Ala Ala Ala Ala Leu Arg Arg Gln Gly His Arg
            20                  25                  30

Val Thr Ile Tyr Glu Thr Ser Ser Phe Lys Asn Glu Leu Gly Ala Gly
        35                  40                  45

Leu Ala Ile Pro Pro Asn Thr Val Arg Gly Leu Ile Gly Leu Gly Cys
    50                  55                  60

Val Ile Glu Asn Leu Asp Pro Val Glu Asn Leu Cys Phe Thr Ala Val
65                  70                  75                  80

Ala Phe Asp Gly Ser Ala Gly Met Arg Ser Asp Gln Thr Asn Tyr Glu
                85                  90                  95

Ala Ser Tyr Gly Leu Pro Trp Ile Met Val His Arg Val Asp Leu His
            100                 105                 110

Asn Glu Leu Arg Arg Val Ala Leu Ser Ala Glu Gly Asn Thr Gly Pro
        115                 120                 125

Pro Ala Glu Leu Arg Leu Asp His Arg Val Ser Ser Cys Asp Val Glu
    130                 135                 140

Lys Cys Thr Val Thr Leu Ser Asn Gly Asp Thr His His Ala Asp Leu
145                 150                 155                 160

Ile Ile Gly Ala Asp Gly Ile His Ser Thr Ile Arg Ser Phe Val Val
                165                 170                 175

Gly Glu Glu Ile Val Ile Pro Pro Ser Lys Thr Ala Gly Phe Arg Trp
            180                 185                 190

Leu Thr Glu Ser Thr Ala Leu Glu Pro Tyr Pro Glu Leu Asp Trp Ile
        195                 200                 205

Val Lys Ile Pro Pro Leu Gly Ala Arg Leu Ile Ser Ala Pro Met Asn
    210                 215                 220

Pro Ala Pro Pro Gln Val Asp His Arg Thr Ile Ile Ile Tyr Ala Cys
225                 230                 235                 240

Arg Gly Ser Thr Leu Ile Asn Val Leu Gly Val His Glu Asp Leu Arg
                245                 250                 255

Asp Gln Asp Thr Val Pro Trp Asn Ala Pro Val Thr Gln Ser Glu Leu
            260                 265                 270

Leu Gln Phe Phe Gly Asp Tyr His Pro Arg Phe Lys Arg Leu Leu Glu
        275                 280                 285

Leu Ala Asn Asp Val His Val Trp Gln Met Arg Val Val Pro Arg Leu
    290                 295                 300

Glu Thr Trp Val Asn Arg Arg Val Cys Ile Met Gly Asp Ala Ala His

305                 310                 315                 320

Ala Ser Leu Pro Thr Leu Gly Gln Gly Phe Gly Met Gly Leu Glu Asp
                325                 330                 335

Ala Val Ala Leu Gly Thr Leu Leu Pro Leu Gly Thr Thr Pro Glu Glu
            340                 345                 350

Ile Pro Asp Arg Leu Thr Leu Trp Gln Asp Leu Val Lys Pro Arg Ala
        355                 360                 365

Glu Phe Val Ala Thr Glu Ser Tyr Glu Gln Gln His Ile Pro Ala Lys
    370                 375                 380

Arg Gly Leu Tyr Leu Arg Ser Gln Glu Met Arg Asp Trp Val Met Gly
385                 390                 395                 400

Tyr Asp Val Gln Ala Glu Ala Gln Lys Val Leu Ala Gly Ala Val Asn
                405                 410                 415

Arg Ser Lys Gly
            420

<210>  19
<211>  1287
<212>  DNA
<213>  Armillaria mellea

<400>  19
atgcaacaaa tcgacgaagc gcgcccattg aaagtgatag tagtgggtgc tggactttgt      60

gggctttccg ccgccattgc acttcgtagg caagggcatc atgttcatat acttgaatct     120

tcaagtttta agagcgagct tggcgcaggt ctcgccgtcc cacccaatac tgtacgctct     180

cttcgaggcc taggttgtaa catcgacaat ctcaagcccg tggataattt gtgtttctct     240

gccatggcgc atgacggaag cccaggcatg atgaataaca tgacagacta tcacaaggcg     300

tacggtgatc cttgggtaat ggcacatcgt gtcgacctcc ataacgagct cttgcgagtg     360

gctttcgacc ccgaaggaac agggcctcct gctcaacttc gtttgggcgt ccaggtagtg     420

acttgcgata tggaagcttg tacaatttcc cttgtcgatg gaacagtctg ttccgccgat     480

cttatcgtag gagctgacgg tattaagtcg accatacgct cctgtgttct aggcaaagaa     540

atagacatac ctcctaccgg tatcgccgga taccgctggc tcataccggc agaagctttg     600

gagccctatc ccgagctcga ctggattatc aagaacccac ccctaggagc acgtttaatc     660

acggatcccg tacgccgaac tgaacaaacg gatgacggta agaaggctga caagcgcacg     720

atcataatct atgcgtgccg cagtggcacg atgatcaacg ttcttggtgt gcacgatgac     780

ctgcgcaacc agaatgaagt cggatggaac gtaccagtca cacgggaaaa cttgctggag 840

tttttcgggg actaccaccc acggtttaag cgtttactcc agctagccga tagtattcat 900

ttgtggcaaa tgcgtgttgt cccacggctt gacacatgga ttaatagatg cgtgtgtttg 960

ctgggcgatt ctgcacatgc gtcattacca actctcgggc aaggcttcgg aatgggtctt 1020

gaggatgccg tagctctcgc agccctcctt ccgatgggaa ccaatgcgtc tgacgttgag 1080

aaccgcctta tcgcctacga aagcttgcgt aaggagcgtg cagagtatgt agccacggaa 1140

tcattagaac agcaggatat tgcaggaaag cgaggcttgt atctcaggtc tcctatgatg 1200

cgcgataaaa taatgggtta tgatattaaa gcggaagctg agaaggtttt aatcgaatta 1260

aaaaattcga cagctcagca ggtcact 1287

<210> 20
<211> 429
<212> PRT
<213> Armillaria mellea

<400> 20

Met Gln Gln Ile Asp Glu Ala Arg Pro Leu Lys Val Ile Val Val Gly
1               5                   10                  15

Ala Gly Leu Cys Gly Leu Ser Ala Ala Ile Ala Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Ser
65                  70                  75                  80

Ala Met Ala His Asp Gly Ser Pro Gly Met Met Asn Asn Met Thr Asp
                85                  90                  95

Tyr His Lys Ala Tyr Gly Asp Pro Trp Val Met Ala His Arg Val Asp
            100                 105                 110

Leu His Asn Glu Leu Leu Arg Val Ala Phe Asp Pro Glu Gly Thr Gly
        115                 120                 125

Pro Pro Ala Gln Leu Arg Leu Gly Val Gln Val Val Thr Cys Asp Met
    130                 135                 140

Glu Ala Cys Thr Ile Ser Leu Val Asp Gly Thr Val Cys Ser Ala Asp

145                 150                 155                 160

Leu Ile Val Gly Ala Asp Gly Ile Lys Ser Thr Ile Arg Ser Cys Val
                165                 170                 175

Leu Gly Lys Glu Ile Asp Ile Pro Pro Thr Gly Ile Ala Gly Tyr Arg
            180                 185                 190

Trp Leu Ile Pro Ala Glu Ala Leu Glu Pro Tyr Pro Glu Leu Asp Trp
        195                 200                 205

Ile Ile Lys Asn Pro Pro Leu Gly Ala Arg Leu Ile Thr Asp Pro Val
    210                 215                 220

Arg Arg Thr Glu Gln Thr Asp Asp Gly Lys Lys Ala Asp Lys Arg Thr
225                 230                 235                 240

Ile Ile Ile Tyr Ala Cys Arg Ser Gly Thr Met Ile Asn Val Leu Gly
                245                 250                 255

Val His Asp Asp Leu Arg Asn Gln Asn Glu Val Gly Trp Asn Val Pro
            260                 265                 270

Val Thr Arg Glu Asn Leu Leu Glu Phe Phe Gly Asp Tyr His Pro Arg
        275                 280                 285

Phe Lys Arg Leu Leu Gln Leu Ala Asp Ser Ile His Leu Trp Gln Met
    290                 295                 300

Arg Val Val Pro Arg Leu Asp Thr Trp Ile Asn Arg Cys Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Val Ala Leu Ala Ala Leu Leu Pro Met
            340                 345                 350

Gly Thr Asn Ala Ser Asp Val Glu Asn Arg Leu Ile Ala Tyr Glu Ser
        355                 360                 365

Leu Arg Lys Glu Arg Ala Glu Tyr Val Ala Thr Glu Ser Leu Glu Gln
    370                 375                 380

Gln Asp Ile Ala Gly Lys Arg Gly Leu Tyr Leu Arg Ser Pro Met Met
385                 390                 395                 400

Arg Asp Lys Ile Met Gly Tyr Asp Ile Lys Ala Glu Ala Glu Lys Val
                405                 410                 415

Leu Ile Glu Leu Lys Asn Ser Thr Ala Gln Gln Val Thr
            420                 425

<210>  21
<211>  1278
<212>  DNA
<213>  Armillaria fuscipes

<400>  21
atgcaacaaa tcgacgaagc gcgtccattg aaggtgctag tcgtgggtgc tggactctgt      60

gggctttccg ccgccatcgc acttcgtaga caagggcatc acgtccatat acttgaatct     120

tcaagtttca agagcgaact tggggcaggt ctcgccgtgc cacctaatac tgtgcgctct     180

cttcgaggtc tcggctgtga catagacaat ctcaagcccg tggataatct ttgtttcact     240

gctatgtcgc atgacggaag cccaggcatg atgaataaca tgacggacta tcgcaaggcg     300

tatggtgatc cttgggtgat ggcacatcgt gtcgaccttc ataacgagct tatgcgagtg     360

gctctcgacc ctgatgggac aggtcctcct gcccaacttc gtttgggcgt ccaggttgtg     420

tcttgcgatg ttgaagcttg tactgtttcc cttgtcgatg gagaggtctg ttccgccgat     480

cttatcgttg gagctgatgg tatcaggtca accatacgct cctatgttct aggcaaagaa     540

atagatatac ctcctaccgg cattgccgga taccgctggc ttacaccatc agaggctttg     600

gagccttttc ccgaacttga ttggattatc aagaacccac ctctaggagc acgtctaatc     660

accgctccca tacgccggaa cgaacaaatg aatgacggtg agatggctga caagcgtacg     720

atcatcatct acgcgtgccg caacggcaca atgattaacg ttctcggtgt gcacgatgac     780

ccgcgcaacc agaatgaagt cggatggaac gtgccagtaa cccaagaaaa attgctcgaa     840

tttttcggag actaccaccc acggttcaaa agtttacttc agctatctga tagtattcat     900

ttgtggcaaa tgcgtgttgt tccacggctt gacacatgga tcaatcaacg tgtgtgtttg     960

ctgggcgatt ctgcacatgc gtcattacca acgctcgggc aaggcttcgg gatgggtctt    1020

gaggacgcca tagctcttgc aaccctcctt ccgatgggcg ccaaagtgtc ggacattgag    1080

aatcgcctta tcgcctacga aagcctgcgt aaggagcgtg cagagtttgt agccacggaa    1140

tcattagaac agcaagatat tcccgaaaag cgaggcttgt atctcagatc ccctatgatg    1200

cgcgataaaa taatgggtta cgatatcaaa gccgaagctg aaaaggtttt aatggagtta    1260

ttgagctcga aagctcaa                                                  1278

<210>  22
<211>  426
<212>  PRT
<213>  Armillaria fuscipes

<400>  22

Met Gln Gln Ile Asp Glu Ala Arg Pro Leu Lys Val Leu Val Val Gly
1               5                   10                  15

Ala Gly Leu Cys Gly Leu Ser Ala Ala Ile Ala Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asp Ile Asp Asn Leu Lys Pro Val Asp Asn Leu Cys Phe Thr
65                  70                  75                  80

Ala Met Ser His Asp Gly Ser Pro Gly Met Met Asn Asn Met Thr Asp
                85                  90                  95

Tyr Arg Lys Ala Tyr Gly Asp Pro Trp Val Met Ala His Arg Val Asp
            100                 105                 110

Leu His Asn Glu Leu Met Arg Val Ala Leu Asp Pro Asp Gly Thr Gly
        115                 120                 125

Pro Pro Ala Gln Leu Arg Leu Gly Val Gln Val Val Ser Cys Asp Val
    130                 135                 140

Glu Ala Cys Thr Val Ser Leu Val Asp Gly Glu Val Cys Ser Ala Asp
145                 150                 155                 160

Leu Ile Val Gly Ala Asp Gly Ile Arg Ser Thr Ile Arg Ser Tyr Val
                165                 170                 175

Leu Gly Lys Glu Ile Asp Ile Pro Pro Thr Gly Ile Ala Gly Tyr Arg
            180                 185                 190

Trp Leu Thr Pro Ser Glu Ala Leu Glu Pro Phe Pro Glu Leu Asp Trp
        195                 200                 205

Ile Ile Lys Asn Pro Pro Leu Gly Ala Arg Leu Ile Thr Ala Pro Ile
    210                 215                 220

Arg Arg Asn Glu Gln Met Asn Asp Gly Glu Met Ala Asp Lys Arg Thr
225                 230                 235                 240

Ile Ile Ile Tyr Ala Cys Arg Asn Gly Thr Met Ile Asn Val Leu Gly
                245                 250                 255

Val His Asp Asp Pro Arg Asn Gln Asn Glu Val Gly Trp Asn Val Pro
            260                 265                 270

Val Thr Gln Glu Lys Leu Leu Glu Phe Phe Gly Asp Tyr His Pro Arg
        275                 280                 285

Phe Lys Ser Leu Leu Gln Leu Ser Asp Ser Ile His Leu Trp Gln Met
    290                 295                 300

Arg Val Val Pro Arg Leu Asp Thr Trp Ile Asn Gln Arg Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Ile Ala Leu Ala Thr Leu Leu Pro Met
            340                 345                 350

Gly Ala Lys Val Ser Asp Ile Glu Asn Arg Leu Ile Ala Tyr Glu Ser
        355                 360                 365

Leu Arg Lys Glu Arg Ala Glu Phe Val Ala Thr Glu Ser Leu Glu Gln
    370                 375                 380

Gln Asp Ile Pro Glu Lys Arg Gly Leu Tyr Leu Arg Ser Pro Met Met
385                 390                 395                 400

Arg Asp Lys Ile Met Gly Tyr Asp Ile Lys Ala Glu Ala Glu Lys Val
                405                 410                 415

Leu Met Glu Leu Leu Ser Ser Lys Ala Gln
            420                 425

<210>  23
<211>  1266
<212>  DNA
<213>  Armillaria gallica

<400>  23
atgcaacaaa tcgacgaagc gcgcgcattg aaagtgatag tcgtgggtgc tggactttgc      60

gggctttccg ccgccattgt acttcgtagg caagggcatc acgtccatat acttgaatct     120

tcaagtttca agagcgaact tggtgcaggt cttgccgtgc cgcccaacac tgtacgctct     180

cttcgaggtc taggttgtaa catcgacaat ctcaagcccg tggataatct ttgtttcact     240

gccatggctc atgacggaag cccaggcatg atgaataaca tgacagacta tcagaaggcg     300

tacggcgatc cttgggtaat ggcgcatcgt gtcgacctcc ataacgagct catgcgagtg 360

gctctcgacc ctgaaggaac aggccctgct gcccagcttc gtttgggcgt ccaggtggtg 420

tcttgtgatg tggaagcttg taccatttct cttgtcgatg gatcaatctg taccgccgat 480

cttatcgtcg gagctgatgg tattaggtca accatacggt cctatgtttt gggcaaagaa 540

atagacatac ctcctaccgg tattgctgga taccgctggc tcacaccggc agaagctttg 600

gacccatatc ccgaactcga ctggattatc aagaacccac ccctgggagc acgtttaatc 660

acagctcccg ttcgccgaaa cgataaggcg gatgacggtg agaaagctga caagcgcacg 720

atcataatct acgcgtgccg cagtggcact atgatcaacg ttctcggtgt gcacgatgac 780

ccgcgcaacc agaatgaagt cggatggaac gtaccagtta cccaggaaaa tttgttggag 840

tttttcgaag actaccaccc acggtttaag cgtttacttc agctgaccga taacattcat 900

ttgtggcaaa tgcgtgttgt cccgcggctt gacacatgga ttaataaacg cgtgtgtttg 960

ctgggcgatt ctgcacatgc gtcattacca acactcgggc aaggcttcgg gatgggtctt 1020

gaggacgccg tagctctcgc aaccctcctt ccgatgggaa ccaaattgtc tgacattgaa 1080

aaccgtcttg tcgcctacga aagcttgcgt aaggagcgtg ctgagtatgt agccacggaa 1140

tcattagaac agcaggatat tccgggaaag cgaggcttgt atctcaggtc tcctgtgatg 1200

cgcgataaaa taatgggtta tgatatcaaa gccgaagctg agaaggtttt aatggaattg 1260

ataacc 1266

<210> 24
<211> 422
<212> PRT
<213> Armillaria gallica

<400> 24

Met Gln Gln Ile Asp Glu Ala Arg Ala Leu Lys Val Ile Val Val Gly
1               5                   10                  15

Ala Gly Leu Cys Gly Leu Ser Ala Ala Ile Val Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Thr
65                  70                  75                  80

Ala Met Ala His Asp Gly Ser Pro Gly Met Met Asn Asn Met Thr Asp
                85                  90                  95

Tyr Gln Lys Ala Tyr Gly Asp Pro Trp Val Met Ala His Arg Val Asp
            100                 105                 110

Leu His Asn Glu Leu Met Arg Val Ala Leu Asp Pro Glu Gly Thr Gly
        115                 120                 125

Pro Ala Ala Gln Leu Arg Leu Gly Val Gln Val Val Ser Cys Asp Val
    130                 135                 140

Glu Ala Cys Thr Ile Ser Leu Val Asp Gly Ser Ile Cys Thr Ala Asp
145                 150                 155                 160

Leu Ile Val Gly Ala Asp Gly Ile Arg Ser Thr Ile Arg Ser Tyr Val
                165                 170                 175

Leu Gly Lys Glu Ile Asp Ile Pro Pro Thr Gly Ile Ala Gly Tyr Arg
            180                 185                 190

Trp Leu Thr Pro Ala Glu Ala Leu Asp Pro Tyr Pro Glu Leu Asp Trp
        195                 200                 205

Ile Ile Lys Asn Pro Pro Leu Gly Ala Arg Leu Ile Thr Ala Pro Val
    210                 215                 220

Arg Arg Asn Asp Lys Ala Asp Asp Gly Glu Lys Ala Asp Lys Arg Thr
225                 230                 235                 240

Ile Ile Ile Tyr Ala Cys Arg Ser Gly Thr Met Ile Asn Val Leu Gly
                245                 250                 255

Val His Asp Asp Pro Arg Asn Gln Asn Glu Val Gly Trp Asn Val Pro
            260                 265                 270

Val Thr Gln Glu Asn Leu Leu Glu Phe Phe Glu Asp Tyr His Pro Arg
        275                 280                 285

Phe Lys Arg Leu Leu Gln Leu Thr Asp Asn Ile His Leu Trp Gln Met
    290                 295                 300

Arg Val Val Pro Arg Leu Asp Thr Trp Ile Asn Lys Arg Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Val Ala Leu Ala Thr Leu Leu Pro Met
            340                 345                 350

Gly Thr Lys Leu Ser Asp Ile Glu Asn Arg Leu Val Ala Tyr Glu Ser
        355                 360                 365

Leu Arg Lys Glu Arg Ala Glu Tyr Val Ala Thr Glu Ser Leu Glu Gln
    370                 375                 380

Gln Asp Ile Pro Gly Lys Arg Gly Leu Tyr Leu Arg Ser Pro Val Met
385                 390                 395                 400

Arg Asp Lys Ile Met Gly Tyr Asp Ile Lys Ala Glu Ala Glu Lys Val
                405                 410                 415

Leu Met Glu Leu Ile Thr
            420

<210>  25
<211>  1278
<212>  DNA
<213>  Armillaria ostoyae

<400>  25
atgcaacaaa tcgacgaagc gcacccattg acagtgatag tcgtgggtgc tggactttgc      60

gggctttccg ccgccattgc acttcgtagg caagggcatc acgtccatat acttgaatct     120

tcaagtttca aaagcgaact tggcgcaggt ctcgccgtgc cgcccaacac tgtacgctct     180

cttcgaggtc taggttgtaa catcgacaat ctcaagcccg tggataatct ttgtttcact     240

gccatggcgc atgacggaag cccaggcatg atgaataaca tgacagacta tcacaaggcg     300

tacggcgagc cttgggtaat ggcgcatcgt gtcgacctcc ataacgagct catgcgagtg     360

gctctcgacc ctgaaggaac aggtcctcct gctcagcttc gtttgggtgt ccaggtggtg     420

tcttgcgatg tggaagcttg taccatttct cttgtcgatg gatcaatctg ttccgccgat     480

cttatcgtcg gagctgatgg tattaggtca accatacgct cctatgtttt gggcaaagaa     540

atagacatac ctcctaccgg tattgctgga taccgttggc tcacaccggc agaagctttg     600

gagccatatc ccgaactcga ctggattatc aagaacccac ccctgggagc acgtttaatc     660

acggctcccg tacgccgaaa cgataagacg gatgacggtg agaagactga caagcgcacg     720

atcataatct acgcgtgccg caatggcact atgatcaacg ttctcggtgt gcacgatgac     780

ccgcgcaacc agaatgaagt cggatggaac gtaccagtta cccaggaaaa tttgttggag     840

tttttcgaag actaccaccc acggtttaag cgtttacttc agttggccga tagtattcat     900

ttgtggcaaa tgcgtgttgt cccacggctt gacacatgga ttaataaacg cgtgtgtttg     960

ctgggcgatt ctgcacatgc gtcattacca acactcgggc aaggcttcgg gatgggtctt 1020

gaggacgccg tagctctcgc agccctcctt ccgatgggaa ccaaagtgtc tgacgttgag 1080

agtcgtctta tcgcctacga aagcttgcgt aaggagcgtg ctgagtatgt agccacggaa 1140

tcattagaac agcagaatat tccgggaaag cgaggcttgt atctcaggtc tcctatgatg 1200

cgcgataaaa taatgggtta tgatatcaaa gccgaagctg agaaggtttt aatggaatta 1260

ataacctcga cagctcag 1278

<210> 26
<211> 426
<212> PRT
<213> Armillaria ostoyae

<400> 26

Met Gln Gln Ile Asp Glu Ala His Pro Leu Thr Val Ile Val Val Gly
1               5                   10                  15

Ala Gly Leu Cys Gly Leu Ser Ala Ala Ile Ala Leu Arg Arg Gln Gly
            20                  25                  30

His His Val His Ile Leu Glu Ser Ser Ser Phe Lys Ser Glu Leu Gly
        35                  40                  45

Ala Gly Leu Ala Val Pro Pro Asn Thr Val Arg Ser Leu Arg Gly Leu
    50                  55                  60

Gly Cys Asn Ile Asp Asn Leu Lys Pro Val Asp Asn Leu Cys Phe Thr
65                  70                  75                  80

Ala Met Ala His Asp Gly Ser Pro Gly Met Met Asn Asn Met Thr Asp
                85                  90                  95

Tyr His Lys Ala Tyr Gly Glu Pro Trp Val Met Ala His Arg Val Asp
            100                 105                 110

Leu His Asn Glu Leu Met Arg Val Ala Leu Asp Pro Glu Gly Thr Gly
        115                 120                 125

Pro Pro Ala Gln Leu Arg Leu Gly Val Gln Val Val Ser Cys Asp Val
    130                 135                 140

Glu Ala Cys Thr Ile Ser Leu Val Asp Gly Ser Ile Cys Ser Ala Asp
145                 150                 155                 160

Leu Ile Val Gly Ala Asp Gly Ile Arg Ser Thr Ile Arg Ser Tyr Val
                165                 170                 175

Leu Gly Lys Glu Ile Asp Ile Pro Pro Thr Gly Ile Ala Gly Tyr Arg
            180                 185                 190

Trp Leu Thr Pro Ala Glu Ala Leu Glu Pro Tyr Pro Glu Leu Asp Trp
        195                 200                 205

Ile Ile Lys Asn Pro Pro Leu Gly Ala Arg Leu Ile Thr Ala Pro Val
    210                 215                 220

Arg Arg Asn Asp Lys Thr Asp Asp Gly Glu Lys Thr Asp Lys Arg Thr
225                 230                 235                 240

Ile Ile Ile Tyr Ala Cys Arg Asn Gly Thr Met Ile Asn Val Leu Gly
                245                 250                 255

Val His Asp Asp Pro Arg Asn Gln Asn Glu Val Gly Trp Asn Val Pro
            260                 265                 270

Val Thr Gln Glu Asn Leu Leu Glu Phe Phe Glu Asp Tyr His Pro Arg
        275                 280                 285

Phe Lys Arg Leu Leu Gln Leu Ala Asp Ser Ile His Leu Trp Gln Met
    290                 295                 300

Arg Val Val Pro Arg Leu Asp Thr Trp Ile Asn Lys Arg Val Cys Leu
305                 310                 315                 320

Leu Gly Asp Ser Ala His Ala Ser Leu Pro Thr Leu Gly Gln Gly Phe
                325                 330                 335

Gly Met Gly Leu Glu Asp Ala Val Ala Leu Ala Ala Leu Leu Pro Met
            340                 345                 350

Gly Thr Lys Val Ser Asp Val Glu Ser Arg Leu Ile Ala Tyr Glu Ser
        355                 360                 365

Leu Arg Lys Glu Arg Ala Glu Tyr Val Ala Thr Glu Ser Leu Glu Gln
    370                 375                 380

Gln Asn Ile Pro Gly Lys Arg Gly Leu Tyr Leu Arg Ser Pro Met Met
385                 390                 395                 400

Arg Asp Lys Ile Met Gly Tyr Asp Ile Lys Ala Glu Ala Glu Lys Val
                405                 410                 415

Leu Met Glu Leu Ile Thr Ser Thr Ala Gln
            420                 425

<210>  27
<211>  1266
<212>  DNA
<213>  Mycena chlorophos

<400>  27
atgccctcca ccgccgaatc tccgcagccg ctcaagatcg tcatcgtcgg tgctgggctc      60

gttggtctcg ctgctgccat tgcgcttcgt cgcgagggtc atcatgtaga gatatacgaa     120

tcgtcgacgt tcaagaccga actcggcgcc ggtctcgcga taccgtgcaa taccctccgt     180

agcctcattg agctggggtg cattgtggct aacttgaacc cggtggagaa cctctgtttc     240

acgtctatgg cgcacgacgg aagcgagtca ggcatgcgaa gcgaccacac cgactacgag     300

gcgcgttatg ggaccccttg ggtcatggcg catcgcgtcg acatacacgc agagctgctc     360

cgaatggcca ccacctccga tattcccggc ccaccggcga cactgcatct cggccaacgc     420

gtccttgcct gcaatgtgtc cgactgctcc attgcactgg ccaccggcaa aacgatctca     480

gcggatctcg tcgttggtgc cgatgggatt cgctcgacca ttcgagctgc tgttcttggc     540

gaagatatcc acattccggc atcgggcact gccggcttcc gatggctcgt agattccgcc     600

gcgctggatc cctatcccga gctggactgg attgtgaaag cccgtccgct tggcgctcgc     660

gttatttctg ccccgatggg cctcgcactc gaagatcatc gtaccattgt gatctatgcg     720

tgtcgcggcg gtaacttgat caacgttctt gcggtccacg aagacaagcg cgaccaggag     780

gctgtccctt ggaatgtccc tttgacgcgc gaagccctct tggacttctt tagcgactac     840

cacccgcgtt tccgccgtct cttcgagctc gcgccggtcg acggaattca cgtctggcag     900

atgcgggtcg taccaccttt ggccaactgg atccgtgacc gcgtttgcat tctcggcgac     960

gcggcgcatg cgtctcttcc tactatgggc cagggctttg gccaaggtct cgaagacgcc    1020

gttgcgctag cgactttgct cccgctagga acgcgtagaa cggatatccc cgctcgtcta    1080

gtggcgtatg aggggatgcg caagcctcgg accgagtgga ttgcacgcga atcgtttgag    1140

cagcaggccg tcgcggaaaa gcgcggcatt tacttgcgct ctatcgaaat gcgcgatgcg    1200

gttatggggt ataatgttcg cgaggaggct aagcgcgtct tgtccgagct cactaaatct    1260

gattgt                                                                1266


<210>  28
<211>  422
<212>  PRT
<213>  Mycena chlorophos

<400>  28

Met Pro Ser Thr Ala Glu Ser Pro Gln Pro Leu Lys Ile Val Ile Val
1               5                   10                  15

Gly Ala Gly Leu Val Gly Leu Ala Ala Ala Ile Ala Leu Arg Arg Glu
        20                  25                  30

Gly His His Val Glu Ile Tyr Glu Ser Ser Thr Phe Lys Thr Glu Leu
    35                  40                  45

Gly Ala Gly Leu Ala Ile Pro Cys Asn Thr Leu Arg Ser Leu Ile Glu
    50                  55                  60

Leu Gly Cys Ile Val Ala Asn Leu Asn Pro Val Glu Asn Leu Cys Phe
65                  70                  75                  80

Thr Ser Met Ala His Asp Gly Ser Glu Ser Gly Met Arg Ser Asp His
                85                  90                  95

Thr Asp Tyr Glu Ala Arg Tyr Gly Thr Pro Trp Val Met Ala His Arg
            100                 105                 110

Val Asp Ile His Ala Glu Leu Leu Arg Met Ala Thr Thr Ser Asp Ile
        115                 120                 125

Pro Gly Pro Pro Ala Thr Leu His Leu Gly Gln Arg Val Leu Ala Cys
    130                 135                 140

Asn Val Ser Asp Cys Ser Ile Ala Leu Ala Thr Gly Lys Thr Ile Ser
145                 150                 155                 160

Ala Asp Leu Val Val Gly Ala Asp Gly Ile Arg Ser Thr Ile Arg Ala
                165                 170                 175

Ala Val Leu Gly Glu Asp Ile His Ile Pro Ala Ser Gly Thr Ala Gly
            180                 185                 190

Phe Arg Trp Leu Val Asp Ser Ala Ala Leu Asp Pro Tyr Pro Glu Leu
        195                 200                 205

Asp Trp Ile Val Lys Ala Arg Pro Leu Gly Ala Arg Val Ile Ser Ala
    210                 215                 220

Pro Met Gly Leu Ala Leu Glu Asp His Arg Thr Ile Val Ile Tyr Ala
225                 230                 235                 240

Cys Arg Gly Gly Asn Leu Ile Asn Val Leu Ala Val His Glu Asp Lys
                245                 250                 255

Arg Asp Gln Glu Ala Val Pro Trp Asn Val Pro Leu Thr Arg Glu Ala
            260                 265                 270

Leu Leu Asp Phe Phe Ser Asp Tyr His Pro Arg Phe Arg Arg Leu Phe
        275                 280                 285

Glu Leu Ala Pro Val Asp Gly Ile His Val Trp Gln Met Arg Val Val
    290                 295                 300

Pro Pro Leu Ala Asn Trp Ile Arg Asp Arg Val Cys Ile Leu Gly Asp
305                 310                 315                 320

Ala Ala His Ala Ser Leu Pro Thr Met Gly Gln Gly Phe Gly Gln Gly
                325                 330                 335

Leu Glu Asp Ala Val Ala Leu Ala Thr Leu Leu Pro Leu Gly Thr Arg
            340                 345                 350

Arg Thr Asp Ile Pro Ala Arg Leu Val Ala Tyr Glu Gly Met Arg Lys
        355                 360                 365

Pro Arg Thr Glu Trp Ile Ala Arg Glu Ser Phe Glu Gln Gln Ala Val
    370                 375                 380

Ala Glu Lys Arg Gly Ile Tyr Leu Arg Ser Ile Glu Met Arg Asp Ala
385                 390                 395                 400

Val Met Gly Tyr Asn Val Arg Glu Glu Ala Lys Arg Val Leu Ser Glu
                405                 410                 415

Leu Thr Lys Ser Asp Cys
            420

<210>  29
<211>  72
<212>  PRT
<213>  Artificial sequence

<220>
<223>  Consensus amino acid sequence 1 for
       hispidin hydroxylase


<220>
<221>  MISC_FEATURE
<222>  (1)..(72)
<223>  X - any amino acid

<400>  29

Val Gly Ala Gly Leu Xaa Gly Xaa Xaa Ala Ala Xaa Xaa Leu Arg Arg
1               5                   10                  15

Xaa Gly His Xaa Xaa Xaa Xaa Xaa Xaa Xaa Ser Xaa Phe Lys Xaa Glu
            20                  25                  30

Xaa Gly Ala Gly Xaa Ala Xaa Pro Xaa Asn Thr Xaa Xaa Xaa Leu Xaa
        35                  40                  45

Xaa Leu Gly Cys Xaa Xaa Xaa Asn Xaa Xaa Xaa Val Xaa Asn Leu Cys
    50                  55                  60

Xaa Xaa Xaa Xaa Xaa Xaa Asp Gly
65                  70

<210> 30
<211> 33
<212> PRT
<213> Artificial sequence

<220>
<223> Consensus amino acid sequence 2 for
hispidin hydroxylase

<220>
<221> MISC_FEATURE
<222> (1)..(33)
<223> X - any amino acid

<400> 30

Gly Met Xaa Xaa Xaa Xaa Xaa Xaa Tyr Xaa Xaa Xaa Tyr Gly Xaa Xaa
1               5                   10                  15

Trp Xaa Met Xaa His Arg Val Asp Xaa His Xaa Glu Leu Xaa Arg Xaa
            20                  25                  30

Ala

<210> 31
<211> 96
<212> PRT
<213> Artificial sequence

<220>
<223> Consensus amino acid sequence 3 for
hispidin hydroxylase

<220>
<221> MISC_FEATURE
<222> (1)..(96)
<223> X - any amino acid

<400> 31

Gly Pro Xaa Ala Xaa Leu Xaa Leu Xaa Xaa Xaa Val Xaa Xaa Cys Xaa

1                   5                   10                  15

Xaa Xaa Xaa Xaa Xaa Xaa Xaa Xaa Xaa Xaa Gly Xaa Xaa Xaa Xaa Ala
            20                  25                  30

Asp Leu Xaa Xaa Gly Ala Asp Gly Ile Xaa Ser Xaa Xaa Arg Xaa Xaa
        35                  40                  45

Xaa Xaa Xaa Xaa Xaa Xaa Xaa Xaa Pro Xaa Xaa Xaa Xaa Xaa Gly Xaa
    50                  55                  60

Arg Trp Xaa Xaa Xaa Xaa Xaa Xaa Leu Xaa Xaa Xaa Pro Glu Leu Asp
65                  70                  75                  80

Trp Xaa Xaa Lys Xaa Xaa Pro Leu Gly Ala Arg Xaa Xaa Xaa Xaa Pro
                85                  90                  95

<210>  32
<211>  57
<212>  PRT
<213>  Artificial sequence

<220>
<223>  Amino acid consensus sequence 4 for
       hispidin hydroxylase

<220>
<221>  MISC_FEATURE
<222>  (1)..(57)
<223>  X - any amino acid

<400>  32

Arg Thr Ile Xaa Xaa Tyr Ala Cys Arg Xaa Xaa Xaa Xaa Xaa Asn Val
1               5                   10                  15

Leu Xaa Xaa His Xaa Asp Xaa Arg Xaa Gln Xaa Xaa Xaa Xaa Trp Xaa
            20                  25                  30

Xaa Pro Xaa Xaa Xaa Xaa Xaa Xaa Xaa Xaa Xaa Xaa Xaa Asp Tyr His
        35                  40                  45

Pro Arg Phe Xaa Xaa Leu Xaa Xaa Leu
    50                  55

<210>  33
<211>  83
<212>  PRT
<213>  Artificial sequence

<220>
<223>  Consensus amino acid sequence 5 for

hispidin hydroxylase

<220>
<221>  MISC_FEATURE
<222>  (1)..(82)
<223>  X - any amino acid

<400>  33

Trp Gln Xaa Arg Val Xaa Pro Xaa Leu Xaa Xaa Trp Xaa Xaa Xaa Xaa
1               5                   10                  15

Xaa Cys Xaa Xaa Gly Asp Xaa Ala His Ala Ser Leu Pro Thr Xaa Gly
            20                  25                  30

Gln Gly Phe Gly Xaa Gly Leu Glu Asp Ala Xaa Ala Leu Xaa Xaa Leu
        35                  40                  45

Leu Xaa Xaa Xaa Xaa Xaa Xaa Xaa Xaa Xaa Xaa Xaa Arg Leu Xaa Xaa
    50                  55                  60

Xaa Xaa Xaa Xaa Xaa Lys Xaa Arg Xaa Glu Xaa Xaa Xaa Xaa Glu Ser
65                  70                  75                  80

Xaa Glu Gln

<210>  34
<211>  4832
<212>  DNA
<213>  Neonothopanus nambi

<400>  34
aaaaccatcc ttatattctc gctttgatgc tggctgtatg gaaacttgga ggcaccttcg      60

ctcctattga tgtccattct cctgccgaat tggtagctgg catgctgaac atagtctctc     120

cttcttgctt ggttattccg agctcagatg taactaatca aactcttgcg tgcgatctta     180

atatccccgt cgttgcattt cacccacatc aatccactat tcctgagctg aacaagaagt     240

acctcaccga ttctcaaatt tctccggatc ttcctttttc agatccaaac cggcctgctc      300

tgtacctctt cacttcgtcc gccacttctc gaagtaatct caaatgcgtg cctctcactc     360

acacctttat cttacgcaac agcctctcga agcgtgcatg gtgcaagcgt atgcgtccag      420

agacagactt tgacggcata cgcgttcttg gatgggcccc gtggtctcac gtcctagcac     480

acatgcaaga catcggacca ctcaccttac ttaatgccgg atgctacgtt tttgcgacta     540

ctccatccac gtaccctacg gaattgaagg acgacaggga cctgatatct tgcgcggcaa     600

atgctatcat gtacaagggc gtcaagtcat ttgcttgtct tccctttgta ctcggagggc     660

tgaaggcatt atgcgagtct gagccatccg tgaaggcgca tctacaggtc gaggagagag     720

ctcaactcct gaagtctctg caacacatgg aaattcttga gtgtggaggt gccatgctcg 780

aagcaagtgt tgcgtcttgg gctattgaga actgcattcc catttcgatc ggtattggta 840

tgacggagac tggtggagcg ctctttgcag gccccgttca ggccatcaaa accgggtttt 900

cttcagagga taaattcatt gaagatgcta cttacttgct cgttaaggat gatcatgaga 960

gtcatgctga ggaggatatt aacgagggtg aactagttgt gaaaagtaaa atgctcccac 1020

gaggctacct tggctatagt gatccttcct tctcagtcga cgatgctggc tgggttacat 1080

ttagaacagg agacagatac agcgttacac ctgacggaaa gttttcctgg ctgggccgga 1140

acactgattt cattcagatg accagtggtg agacgctgga tccccgacca attgagagct 1200

cgctctgcga aagttctctt atttctagag catgcgttat cggagataaa tttctcaacg 1260

ggcctgctgc tgctgtttgt gcgatcattg agcttgagcc cacagcggtg gaaaaaggac 1320

aagctcactc gcgtgagata gcaagagttt tcgcacctat taatcgagac ctaccgcctc 1380

ctcttaggat tgcatggtcg cacgttttgg ttctccagcc ctcggagaag ataccgatga 1440

cgaagaaggg taccatcttc cgcaagaaaa ttgagcaggt gtttggctct gcgttgggtg 1500

gcagctctgg agataactct caagccactg cggatgctgg cgttgttcga cgagacgagt 1560

tatcgaacac tgtcaagcac ataattagcc gtgttttagg agtttccgat gacgaattac 1620

tttggacgct atcatttgcg gagttaggaa tgacgtcagc actagccact cgcatcgcca 1680

acgagttgaa cgaagtttta gttggagtta atctccctat caacgcttgc tatatacatg 1740

tcgaccttcc ttctctaagc aatgccgtct atgcgaaact tgcacacctc aagttaccag 1800

atcgtactcc cgaacccagg caagcccctg tcgaaaactc tggtgggaag gagatcgttg 1860

tcgttggcca ggcctttcgt cttcctggct caataaacga tgtcgcctct cttcgagacg 1920

cattcctggc gagacaagca tcatccatta tcactgaaat accatccgat cgctgggacc 1980

acgccagctt ctatcccaag gatatacgtt tcaacaaggc tggccttgtg gatatagcca 2040

attatgatca tagctttttc ggactgacgg caaccgaagc gctctatctg tcgccaacta 2100

tgcgtctagc attagaagtt tcgtttgaag cgctagagaa tgctaatatc ccggtgtcac 2160

aactcaaggg ttcgcaaaca gcggtttatg ttgctactac agatgacgga tttgagaccc 2220

ttttgaatgc cgaggccggc tatgatgctt atacaagatt ctatggcact ggtcgagcag 2280

caagtacagc gagcgggcgc ataagctgtc ttcttgatgt ccatggaccc tctattactg 2340

ttgatacggc atgcagtgga ggggctgttt gtattgacca agcaatcgac tatctacaat 2400

catcgagtgc agcagacacc gctatcatat gtgctagtaa cacgcactgc tggccaggct 2460

cgttcaggtt tctttccgca caaggatggt tatccccagg aggacgatgc gcgacattta 2520

caactgatgc tgatggctac gtgccctctg agggcgcggt cgccttcata ttgaaaaccc 2580

gagaagcagc tatgcgtgac aaggacacta tcctcgcgac aatcaaagcg acacagatat 2640

cgcacaatgg ccgatctcaa ggtcttgtgg caccgaatgt caactcgcaa gctgaccttc 2700

atcgctcgtt gcttcaaaaa gctggcctta gcccggctga tatccgtttc attgaagctc 2760

atgggacagg aacgtcactg ggagacctct cagaaattca agctataaat gatgcttata 2820

cctcctctca gccgcgcacg accggcccac tcatagtcag cgcttccaaa acggtcattg 2880

gtcataccga accagctggc cccttggtcg gtatgctgtc ggtcttgaac tctttcaaag 2940

aaggcgccgt ccctggtctc gcccatctta ccgcagacaa tttgaatccc tcgctggact 3000

gttcttctgt gccacttctc attccctatc aacctgttca cctggctgca cccaagcctc 3060

accgagctgc tgtaaggtca tacggctttt caggtaccct gggcggcatc gttctagagg 3120

ctcctgacga agaaagatta gaagaagagc tgccaaatga caagcccatg ttgttcgtcg 3180

tcagcgcaaa gacacataca gcactaatcg aatacctggg gcggtatctc gagttcctct 3240

tgcaggcgaa cccccaagat ttttgtgaca tttgttatac aagctgcgtt gggcgggagc 3300

actatagata tcgctatgct tgtgtagcaa atgatatgga ggacctcata ggccaactcc 3360

agaaacgttt gggcagcaag gtgccgccaa agccgtcata caaacgcggt gctttggcct 3420

ttgccttttc tggtcagggt acacaattcc gagggatggc gacagagctt gcaaaagcgt 3480

actccggctt ccgaaagatc gtgtcggatc tcgcaaagag agctagcgag ttgtcaggtc 3540

atgccattga ccgtttcttt cttgcatatg acataggcgc tgaaaatgta gctcctgata 3600

gtgaggcaga ccagatttgc atctttgtgt atcagtgttc tgtccttcgc tggctgcaga 3660

ctatggggat tagacccagt gcagtgatag gccatagcct cggggagatc tcagcttctg 3720

tggcggcagg agcactttct cttgactccg ctttggatct tgtcatctca cgagctcgcc 3780

ttttgcgctc ttcggcaagt gctcctgcag gaatggcagc tatgtctgcc tcgcaagacg 3840

aggttgtgga gttgattggg aaactagacc tcgacaaggc taattcgctc agcgtttcgg 3900

tcataaatgg tccccaaaat actgtcgtgt ccggctcttc agcggctatt gaaagcatag 3960

tggctttagc gaaagggaga aagatcaaag cgtctgccct gaatatcaat caagcttttc 4020

atagtccata cgtcgacagt gccgtccctg gtctccgtgc ttggtcagaa aagcatatct 4080

cctcagctcg gccattgcaa attccgctgt attcaacgtt gttgggagca caaatctctg 4140

agggagagat gttgaatcca gatcactggg tcgaccatgc acggaagcct gtacagttcg 4200

cacaagcagc cacaaccatg aaagaatcct tcaccggagt catcatagat atcggccctc 4260

aagtagtggc ttggtcactt ctgctctcga acgggctcac gtccgtgact gcgctcgctg 4320

cgaaaagagg gagaagtcaa caggtggctt tcttaagcgc cttggcggat ttgtatcaag 4380

attacggtgt tgttcctgat tttgtcgggc tttatgctca gcaggaagat gcttcgaggt 4440

tgaagaagac ggatatcttg acgtatccgt tccagcgggg cgaagagact ctttctagtg 4500

gttctagcac tccgacattg gaaaacacgg atttggattc cggtaaggaa ttacttatgg 4560

gaccgactcg ggggttgtta cgcgcggacg acttgcgtga cagtatcgtt tcttctgtga 4620

aggatgttct ggaactcaag tcaaatgaag acctcgattt gtctgaaagt ctgaatgcgc 4680

ttggtatgga ctcgatcatg ttcgctcagt tacggaagcg tattggggaa ggactcggat 4740

tgaatgttcc gatggttttt ctgtcggacg cgttttctat tggtgagatg gttagtaatc 4800

ttgtggaaca ggcggaggcg tctgaggaca at 4832

<210> 35
<211> 1678
<212> PRT
<213> Neonothopanus nambi

<400> 35

Met Asn Ser Ser Lys Asn Pro Pro Ser Thr Leu Leu Asp Val Phe Leu
1               5                   10                  15

Asp Thr Ala Arg Asn Leu Asp Thr Ala Leu Arg Asn Val Leu Glu Cys
            20                  25                  30

Gly Glu His Arg Trp Ser Tyr Arg Glu Leu Asp Thr Val Ser Ser Ala
        35                  40                  45

Leu Ala Gln His Leu Arg Tyr Thr Val Gly Leu Ser Pro Thr Val Ala
    50                  55                  60

Val Ile Ser Glu Asn His Pro Tyr Ile Leu Ala Leu Met Leu Ala Val
65                  70                  75                  80

Trp Lys Leu Gly Gly Thr Phe Ala Pro Ile Asp Val His Ser Pro Ala
                85                  90                  95

Glu Leu Val Ala Gly Met Leu Asn Ile Val Ser Pro Ser Cys Leu Val
            100                 105                 110

Ile Pro Ser Ser Asp Val Thr Asn Gln Thr Leu Ala Cys Asp Leu Asn
        115                 120                 125

Ile Pro Val Val Ala Phe His Pro His Gln Ser Thr Ile Pro Glu Leu
    130                 135                 140

Asn Lys Lys Tyr Leu Thr Asp Ser Gln Ile Ser Pro Asp Leu Pro Phe
145                 150                 155                 160

Ser Asp Pro Asn Arg Pro Ala Leu Tyr Leu Phe Thr Ser Ser Ala Thr
                165                 170                 175

Ser Arg Ser Asn Leu Lys Cys Val Pro Leu Thr His Thr Phe Ile Leu
        180                 185                 190

Arg Asn Ser Leu Ser Lys Arg Ala Trp Cys Lys Arg Met Arg Pro Glu
    195                 200                 205

Thr Asp Phe Asp Gly Ile Arg Val Leu Gly Trp Ala Pro Trp Ser His
    210                 215                 220

Val Leu Ala His Met Gln Asp Ile Gly Pro Leu Thr Leu Leu Asn Ala
225                 230                 235                 240

Gly Cys Tyr Val Phe Ala Thr Thr Pro Ser Thr Tyr Pro Thr Glu Leu
                245                 250                 255

Lys Asp Asp Arg Asp Leu Ile Ser Cys Ala Ala Asn Ala Ile Met Tyr
            260                 265                 270

Lys Gly Val Lys Ser Phe Ala Cys Leu Pro Phe Val Leu Gly Gly Leu
        275                 280                 285

Lys Ala Leu Cys Glu Ser Glu Pro Ser Val Lys Ala His Leu Gln Val
    290                 295                 300

Glu Glu Arg Ala Gln Leu Leu Lys Ser Leu Gln His Met Glu Ile Leu
305                 310                 315                 320

Glu Cys Gly Gly Ala Met Leu Glu Ala Ser Val Ala Ser Trp Ala Ile
                325                 330                 335

Glu Asn Cys Ile Pro Ile Ser Ile Gly Ile Gly Met Thr Glu Thr Gly
            340                 345                 350

Gly Ala Leu Phe Ala Gly Pro Val Gln Ala Ile Lys Thr Gly Phe Ser
        355                 360                 365

Ser Glu Asp Lys Phe Ile Glu Asp Ala Thr Tyr Leu Leu Val Lys Asp
    370                 375                 380

Asp His Glu Ser His Ala Glu Glu Asp Ile Asn Glu Gly Glu Leu Val
385                 390                 395                 400

Val Lys Ser Lys Met Leu Pro Arg Gly Tyr Leu Gly Tyr Ser Asp Pro
                405                 410                 415

Ser Phe Ser Val Asp Asp Ala Gly Trp Val Thr Phe Arg Thr Gly Asp

            420                 425                 430

Arg Tyr Ser Val Thr Pro Asp Gly Lys Phe Ser Trp Leu Gly Arg Asn
        435                 440                 445

Thr Asp Phe Ile Gln Met Thr Ser Gly Glu Thr Leu Asp Pro Arg Pro
    450                 455                 460

Ile Glu Ser Ser Leu Cys Glu Ser Ser Leu Ile Ser Arg Ala Cys Val
465                 470                 475                 480

Ile Gly Asp Lys Phe Leu Asn Gly Pro Ala Ala Ala Val Cys Ala Ile
                485                 490                 495

Ile Glu Leu Glu Pro Thr Ala Val Glu Lys Gly Gln Ala His Ser Arg
            500                 505                 510

Glu Ile Ala Arg Val Phe Ala Pro Ile Asn Arg Asp Leu Pro Pro Pro
        515                 520                 525

Leu Arg Ile Ala Trp Ser His Val Leu Val Leu Gln Pro Ser Glu Lys
    530                 535                 540

Ile Pro Met Thr Lys Lys Gly Thr Ile Phe Arg Lys Lys Ile Glu Gln
545                 550                 555                 560

Val Phe Gly Ser Ala Leu Gly Gly Ser Ser Gly Asp Asn Ser Gln Ala
                565                 570                 575

Thr Ala Asp Ala Gly Val Val Arg Arg Asp Glu Leu Ser Asn Thr Val
            580                 585                 590

Lys His Ile Ile Ser Arg Val Leu Gly Val Ser Asp Asp Glu Leu Leu
        595                 600                 605

Trp Thr Leu Ser Phe Ala Glu Leu Gly Met Thr Ser Ala Leu Ala Thr
    610                 615                 620

Arg Ile Ala Asn Glu Leu Asn Glu Val Leu Val Gly Val Asn Leu Pro
625                 630                 635                 640

Ile Asn Ala Cys Tyr Ile His Val Asp Leu Pro Ser Leu Ser Asn Ala
                645                 650                 655

Val Tyr Ala Lys Leu Ala His Leu Lys Leu Pro Asp Arg Thr Pro Glu
            660                 665                 670

Pro Arg Gln Ala Pro Val Glu Asn Ser Gly Gly Lys Glu Ile Val Val
        675                 680                 685

Val Gly Gln Ala Phe Arg Leu Pro Gly Ser Ile Asn Asp Val Ala Ser
    690                 695                 700

Leu Arg Asp Ala Phe Leu Ala Arg Gln Ala Ser Ser Ile Ile Thr Glu
705                 710                 715                 720

Ile Pro Ser Asp Arg Trp Asp His Ala Ser Phe Tyr Pro Lys Asp Ile
                725                 730                 735

Arg Phe Asn Lys Ala Gly Leu Val Asp Ile Ala Asn Tyr Asp His Ser
            740                 745                 750

Phe Phe Gly Leu Thr Ala Thr Glu Ala Leu Tyr Leu Ser Pro Thr Met
        755                 760                 765

Arg Leu Ala Leu Glu Val Ser Phe Glu Ala Leu Glu Asn Ala Asn Ile
    770                 775                 780

Pro Val Ser Gln Leu Lys Gly Ser Gln Thr Ala Val Tyr Val Ala Thr
785                 790                 795                 800

Thr Asp Asp Gly Phe Glu Thr Leu Leu Asn Ala Glu Ala Gly Tyr Asp
                805                 810                 815

Ala Tyr Thr Arg Phe Tyr Gly Thr Gly Arg Ala Ala Ser Thr Ala Ser
            820                 825                 830

Gly Arg Ile Ser Cys Leu Leu Asp Val His Gly Pro Ser Ile Thr Val
        835                 840                 845

Asp Thr Ala Cys Ser Gly Gly Ala Val Cys Ile Asp Gln Ala Ile Asp
    850                 855                 860

Tyr Leu Gln Ser Ser Ser Ala Ala Asp Thr Ala Ile Ile Cys Ala Ser
865                 870                 875                 880

Asn Thr His Cys Trp Pro Gly Ser Phe Arg Phe Leu Ser Ala Gln Gly
                885                 890                 895

Met Val Ser Pro Gly Gly Arg Cys Ala Thr Phe Thr Thr Asp Ala Asp
            900                 905                 910

Gly Tyr Val Pro Ser Glu Gly Ala Val Ala Phe Ile Leu Lys Thr Arg
        915                 920                 925

Glu Ala Ala Met Arg Asp Lys Asp Thr Ile Leu Ala Thr Ile Lys Ala
    930                 935                 940

Thr Gln Ile Ser His Asn Gly Arg Ser Gln Gly Leu Val Ala Pro Asn
945                 950                 955                 960

Val Asn Ser Gln Ala Asp Leu His Arg Ser Leu Leu Gln Lys Ala Gly
                965                 970                 975

Leu Ser Pro Ala Asp Ile Arg Phe Ile Glu Ala His Gly Thr Gly Thr
            980                 985                 990

Ser Leu Gly Asp Leu Ser Glu Ile  Gln Ala Ile Asn Asp  Ala Tyr Thr
        995                 1000                 1005

Ser Ser  Gln Pro Arg Thr Thr  Gly Pro Leu Ile Val  Ser Ala Ser
    1010                 1015                 1020

Lys Thr  Val Ile Gly His Thr  Glu Pro Ala Gly Pro  Leu Val Gly
    1025                 1030                 1035

Met Leu  Ser Val Leu Asn Ser  Phe Lys Glu Gly Ala  Val Pro Gly
    1040                 1045                 1050

Leu Ala  His Leu Thr Ala Asp  Asn Leu Asn Pro Ser  Leu Asp Cys
    1055                 1060                 1065

Ser Ser  Val Pro Leu Leu Ile  Pro Tyr Gln Pro Val  His Leu Ala
    1070                 1075                 1080

Ala Pro  Lys Pro His Arg Ala  Ala Val Arg Ser Tyr  Gly Phe Ser
    1085                 1090                 1095

Gly Thr  Leu Gly Gly Ile Val  Leu Glu Ala Pro Asp  Glu Glu Arg
    1100                 1105                 1110

Leu Glu  Glu Glu Leu Pro Asn  Asp Lys Pro Met Leu  Phe Val Val
    1115                 1120                 1125

Ser Ala  Lys Thr His Thr Ala  Leu Ile Glu Tyr Leu  Gly Arg Tyr
    1130                 1135                 1140

Leu Glu  Phe Leu Leu Gln Ala  Asn Pro Gln Asp Phe  Cys Asp Ile
    1145                 1150                 1155

Cys Tyr  Thr Ser Cys Val Gly  Arg Glu His Tyr Arg  Tyr Arg Tyr
    1160                 1165                 1170

Ala Cys Val Ala Asn Asp Met Glu Asp Leu Ile Gly Gln Leu Gln
    1175                1180                1185

Lys Arg Leu Gly Ser Lys Val Pro Pro Lys Pro Ser Tyr Lys Arg
    1190                1195                1200

Gly Ala Leu Ala Phe Ala Phe Ser Gly Gln Gly Thr Gln Phe Arg
    1205                1210                1215

Gly Met Ala Thr Glu Leu Ala Lys Ala Tyr Ser Gly Phe Arg Lys
    1220                1225                1230

Ile Val Ser Asp Leu Ala Lys Arg Ala Ser Glu Leu Ser Gly His
    1235                1240                1245

Ala Ile Asp Arg Phe Leu Leu Ala Tyr Asp Ile Gly Ala Glu Asn
    1250                1255                1260

Val Ala Pro Asp Ser Glu Ala Asp Gln Ile Cys Ile Phe Val Tyr
    1265                1270                1275

Gln Cys Ser Val Leu Arg Trp Leu Gln Thr Met Gly Ile Arg Pro
    1280                1285                1290

Ser Ala Val Ile Gly His Ser Leu Gly Glu Ile Ser Ala Ser Val
    1295                1300                1305

Ala Ala Gly Ala Leu Ser Leu Asp Ser Ala Leu Asp Leu Val Ile
    1310                1315                1320

Ser Arg Ala Arg Leu Leu Arg Ser Ser Ala Ser Ala Pro Ala Gly
    1325                1330                1335

Met Ala Ala Met Ser Ala Ser Gln Asp Glu Val Val Glu Leu Ile
    1340                1345                1350

Gly Lys Leu Asp Leu Asp Lys Ala Asn Ser Leu Ser Val Ser Val
    1355                1360                1365

Ile Asn Gly Pro Gln Asn Thr Val Val Ser Gly Ser Ser Ala Ala
    1370                1375                1380

Ile Glu Ser Ile Val Ala Leu Ala Lys Gly Arg Lys Ile Lys Ala
    1385                1390                1395

Ser Ala Leu Asn Ile Asn Gln Ala Phe His Ser Pro Tyr Val Asp

1400                 1405                 1410

Ser Ala  Val Pro Gly Leu Arg  Ala Trp Ser Glu Lys  His Ile Ser
    1415                 1420                 1425

Ser Ala  Arg Pro Leu Gln Ile  Pro Leu Tyr Ser Thr  Leu Leu Gly
    1430                 1435                 1440

Ala Gln  Ile Ser Glu Gly Glu  Met Leu Asn Pro Asp  His Trp Val
    1445                 1450                 1455

Asp His  Ala Arg Lys Pro Val  Gln Phe Ala Gln Ala  Ala Thr Thr
    1460                 1465                 1470

Met Lys  Glu Ser Phe Thr Gly  Val Ile Ile Asp Ile  Gly Pro Gln
    1475                 1480                 1485

Val Val  Ala Trp Ser Leu Leu  Leu Ser Asn Gly Leu  Thr Ser Val
    1490                 1495                 1500

Thr Ala  Leu Ala Ala Lys Arg  Gly Arg Ser Gln Gln  Val Ala Phe
    1505                 1510                 1515

Leu Ser  Ala Leu Ala Asp Leu  Tyr Gln Asp Tyr Gly  Val Val Pro
    1520                 1525                 1530

Asp Phe  Val Gly Leu Tyr Ala  Gln Gln Glu Asp Ala  Ser Arg Leu
    1535                 1540                 1545

Lys Lys  Thr Asp Ile Leu Thr  Tyr Pro Phe Gln Arg  Gly Glu Glu
    1550                 1555                 1560

Thr Leu  Ser Ser Gly Ser Ser  Thr Pro Thr Leu Glu  Asn Thr Asp
    1565                 1570                 1575

Leu Asp  Ser Gly Lys Glu Leu  Leu Met Gly Pro Thr  Arg Gly Leu
    1580                 1585                 1590

Leu Arg  Ala Asp Asp Leu Arg  Asp Ser Ile Val Ser  Ser Val Lys
    1595                 1600                 1605

Asp Val  Leu Glu Leu Lys Ser  Asn Glu Asp Leu Asp  Leu Ser Glu
    1610                 1615                 1620

Ser Leu  Asn Ala Leu Gly Met  Asp Ser Ile Met Phe  Ala Gln Leu
    1625                 1630                 1635

Arg Lys Arg Ile Gly Glu Gly Leu Gly Leu Asn Val Pro Met Val
    1640                1645                1650

Phe Leu Ser Asp Ala Phe Ser Ile Gly Glu Met Val Ser Asn Leu
    1655                1660                1665

Val Glu Gln Ala Glu Ala Ser Glu Asp Asn
    1670                1675

<210> 36
<211> 4512
<212> DNA
<213> Panellus stipticus

<400> 36
atgcctcctg ctccttcctc catccttgat gtgttcaccg agactgcata caatcctcgc 60

acctccaacc gtcctgtagt agaatgtggc gagcacctct ggacatactc acaacttgaa 120

gcagtttcca atgccatgtc gcaagacctg gagaactcga tcggtcttta cgcgaaggtc 180

gcttttgtcg gtgaaaacca tccatttgtt ttcgctctca tgcttgcggt ctggaaaatt 240

gctggcacat tcatccccat tgatgcgcac attcctcccg ccctcctaga tggcatggtc 300

gacattgtga agccgatgcg catctatgta tcatcggccg atacctctaa ttcctcctgg 360

gcctcagaac tcgcggtcga gtccaaactc gcgtggtggc gacgcatgca gccaagcatc 420

aacctagaca acacccgagt cctcggctgg gccccgtggt cgcatgtatt atcccacatg 480

caggacattg gcaccgctac cattctcacc gcaggctgtt acgtctttgc ctctatccct 540

tccacatatc agcttcagca ggcgccaaca gatcttacga cccaagtcat caacggtatc 600

ctcaataaaa acatctcggc gcttgctgcc cttccatttg ttttcggcgg tatcaaagca 660

gcgtgcgagt cgggcgatct ggatgtggag gcacttctgg gtgccctgcg ccgcatgacg 720

atgctcgaat gtggcggggc tgcgctcgac cctgcaattg caaattggac ggatataaat 780

ggtgtatcgc tcatggttgg gattggaatg acggaaacgg gtggtgcaat cttcgcgggg 840

agggcgaaag actcgctctc cgggtttctt gctgagggcc tgatctcaga tgccagaatt 900

gagcttgaca agggtaaatc tgatggctcg gatgagggag agcttgttgt cacaagcaag 960

ctacttccac atggctacat tggcttcgat gacgggtcat tttccgtgga tgcgcaaggg 1020

tgggtgacgt tcaagacaag ggattgctat cgtgtcaagg actcgaagtt tatttggcta 1080

ggccgggcca ctgattacat tcaggcgagc acatctctcc ttcctcagcg ctttaaacat 1140

gaactgcgct atcagatgac aagcggcgaa tcccttgacc cgcgtcccct cgaaaatttc 1200

ttacgctccg ccgagttcat ctccaatgtg tgcgtcatag gtgacaactt cctccggggt 1260

gcctccactt ctgtgtgggc cgttatcgag ctcacggaca gcgcgcaccg tctggatgcc 1320

gcttcagcga gaaagcaggt ggcgcgcgtg ctggcgccgc tcaatggtgg cctcccaccc 1380

gctctccgga tctcgatgtc ctcggtgttg atattgagcg gatcacagaa aattccgcgc 1440

acgaagaagg gcgaaatctt ccgtaagcag attgaggatc tgtttggtgc cgcgatgagc 1500

atgccgcctg aggctgagcc ggctctggag gatgaactaa caagtattgt tggaaatgtc 1560

ctcaacatat cggacagcga catgttgacc tcgatgactt ttgccgaact tggaatgacc 1620

tctgttctcg cagtaaaaat ctcagcaagg ctcaacgagc accttgctgg gcgggccgtc 1680

ctcccggcca acatctgtta catctacatc gatactccat cactgattgc cggcatccgg 1740

aacttccttt cgccggcatc ttctgatcaa gagccatcga actttgccac cacggcagac 1800

aagaaggacg aaatcgtcat cataggcaaa gccttccggc ttcccggcgg gatcaacgac 1860

gactctgctc tctgggctgc tcttatgggc aagaacgact ctgttattgc agacatcccg 1920

ccagaccgtt gggatcatgc tagcttctat cccgcccaca tctgcttgcg caaggccggg 1980

cttatagata tgtccagcta tgactatggg ttctttggcc tttcggcgac tgaggcgtac 2040

tatgtgtcgc ccaccatgcg cgcagcgctc gaggtggcgt tcgaagcgct ggaggatgcg 2100

aacatcccgg tgtcaagaat caaggggaca aatacgagtg tttttgttgc aacgaaagat 2160

gatggatttg agacattgct gaatgcggcg catggatttg atgatgctga cggatacgtc 2220

ccttcagaag gcgccgtcgc ttttatactg aagacacgga cggcggcgga gagggatggg 2280

gatcgaatta tggccatcat caaagccacg gaagtctcgc ataatggaag atctcaggga 2340

ctcgccgcgc cgaacgtcaa ggcgcaagcc gctcttcata gagcagtctt gcgcaaagct 2400

aagctcgacc ctctggacat ccatttcatt gaagcccacg gaactggaac accgctgggc 2460

gatctttgtg aaatacaagg cataaatgaa gcctttgtct ccgcacgtcc acgtgcggag 2520

gatccactca ttgtcagcgc ctcaaagtct tcccttgggc acaccgaacc ttcggctgga 2580

ttggttggga tgctatctgt gttgatggcc ttgaagcacg gcatcgtgcc tgggttgctc 2640

catctccgag cggacaatgc taaccaccaa ctggacctga cacaagttcc acttcgtata 2700

tcaccggaac ctgtagtcat cgccgcatcc aagccgcatc acgccatggt gctatcctat 2760

ggattttcag gtacattggc agacattgtt ttggagagtc cggaagaacc atcgtcccca 2820

aacccgggag ccgcgggccc aatgatattt gtcctcagcg cgaagacttc cgcggctctg 2880

tcggcatata tcaaggctta cattgcattt ctacagaatg cagacgcgca cgagttttac 2940

aacatctgct acaccgcctg cgttggaaga gaacattaca aacacagatt tgcttgcgtt 3000

gcaaatgacc ttgctgatct gattcgccaa ttacaagact gtgcgagtgc gctggctcca 3060

accaaaagta gtactggtgc cttggcgttc gcgttccctg gccaaggcgt gcaatttcca 3120

ggcatggccg ctgcacttgc taaaaggcat tccatatttc gcgactatgt catggaattc 3180

ggtgatagag cacaagatct ttgtggcttg cccatcgcaa agatgttgct ggacgtggat 3240

gcagcagagg aagaagatat ccacagtgac gtagaccaga tttgcgtctt tgtctatcag 3300

tattcgatgt gtcgatggct tagggagctc gggctcgagg caagtgcggt tatcgggcac 3360

agtttgggtg aaataacggc cgcacttatc ggggacgcat ttacatttga agtggctctc 3420

gatctcgttg tcactcgagg acggctactc cgcccttctc aagggaatcc aggcgggatg 3480

gctgcgctgg catgccccga ggagaatgtc ccggcgattt tggaccaatg tcgtgtggac 3540

agtaccatta gcgtctccgt tatcaatggt ccgaggagcc tttgtgtatc cggagcttcc 3600

aacgacatcg acgagtttgt caagatggca aaacggcaaa acatcaaagc gactcgactg 3660

agggtggacc aaggatttca tagccctcga gttgatagtg ccgcggttgg actgcgtgca 3720

tggtcaggtt ctttttccaa atcatttcag ccgttgcgca tcacattata ctctacttct 3780

ctgggtgctg caatctcgaa aggagagatt ttgaatcaga cgcattgggc cgatcacgtc 3840

cgccgtccgg tcatattctc aaaagcagca gcagccatcc tcgaggacaa gtccattggc 3900

gcgatcctgg atatcggacc acagacggtg gcatggtctc tccttctggc gaacggctgc 3960

aacgtcgcgt cagctgttgc cctgtccggc cgaagagtac aagatcagga aacagccttt 4020

ttatctgcac tggcgaatct gtatcaaaat cacggggtga cgccgaattt tcgcgtattt 4080

tatgctcacc aggcagtcca ggcgcgctat agaaccgtgg acatcccgaa gtatcccttc 4140

caacgccgac atcgatatcc atcctacatt ccatcgcgca atgccacggg tgccaacaga 4200

ctgaaagaac cattccgtag cgacctggat gaaccggctc aagacacgga gcacaccgcg 4260

gaactgagag tggacatgac tccggagaag ctccgggacg ccctgatgca ctgtgtgcgg 4320

gacacattgg aaggcgaaga ttttgatgaa tcggaatccc tcgtttcgcg tggaattgac 4380

tccattactt ttgcgggttt acggaagcgt gttcaagaac gacacggact taatctttcc 4440

atcattttct ggtctgatgg gttttctgtg aaagacatgg tcgacagcct catcgaacag 4500

catttgtcc ac 4512

<210> 37
<211> 1504
<212> PRT
<213> Panellus stipticus

<400> 37

Met Pro Pro Ala Pro Ser Ser Ile Leu Asp Val Phe Thr Glu Thr Ala
1               5                   10                  15

Tyr Asn Pro Arg Thr Ser Asn Arg Pro Val Val Glu Cys Gly Glu His
            20                  25                  30

Leu Trp Thr Tyr Ser Gln Leu Glu Ala Val Ser Asn Ala Met Ser Gln
        35                  40                  45

Asp Leu Glu Asn Ser Ile Gly Leu Tyr Ala Lys Val Ala Phe Val Gly
    50                  55                  60

Glu Asn His Pro Phe Val Phe Ala Leu Met Leu Ala Val Trp Lys Ile
65                  70                  75                  80

Ala Gly Thr Phe Ile Pro Ile Asp Ala His Ile Pro Pro Ala Leu Leu
                85                  90                  95

Asp Gly Met Val Asp Ile Val Lys Pro Met Arg Ile Tyr Val Ser Ser
            100                 105                 110

Ala Asp Thr Ser Asn Ser Ser Trp Ala Ser Glu Leu Ala Val Glu Ser
        115                 120                 125

Lys Leu Ala Trp Trp Arg Arg Met Gln Pro Ser Ile Asn Leu Asp Asn
    130                 135                 140

Thr Arg Val Leu Gly Trp Ala Pro Trp Ser His Val Leu Ser His Met
145                 150                 155                 160

Gln Asp Ile Gly Thr Ala Thr Ile Leu Thr Ala Gly Cys Tyr Val Phe
                165                 170                 175

Ala Ser Ile Pro Ser Thr Tyr Gln Leu Gln Gln Ala Pro Thr Asp Leu
            180                 185                 190

Thr Thr Gln Val Ile Asn Gly Ile Leu Asn Lys Asn Ile Ser Ala Leu
        195                 200                 205

Ala Ala Leu Pro Phe Val Phe Gly Gly Ile Lys Ala Ala Cys Glu Ser
    210                 215                 220

Gly Asp Leu Asp Val Glu Ala Leu Leu Gly Ala Leu Arg Arg Met Thr
225                 230                 235                 240

Met Leu Glu Cys Gly Gly Ala Ala Leu Asp Pro Ala Ile Ala Asn Trp
                245                 250                 255

Thr Asp Ile Asn Gly Val Ser Leu Met Val Gly Ile Gly Met Thr Glu
            260                 265                 270

Thr Gly Gly Ala Ile Phe Ala Gly Arg Ala Lys Asp Ser Leu Ser Gly
        275                 280                 285

Phe Leu Ala Glu Gly Leu Ile Ser Asp Ala Arg Ile Glu Leu Asp Lys
    290                 295                 300

Gly Lys Ser Asp Gly Ser Asp Glu Gly Glu Leu Val Val Thr Ser Lys
305                 310                 315                 320

Leu Leu Pro His Gly Tyr Ile Gly Phe Asp Asp Gly Ser Phe Ser Val
                325                 330                 335

Asp Ala Gln Gly Trp Val Thr Phe Lys Thr Arg Asp Cys Tyr Arg Val
            340                 345                 350

Lys Asp Ser Lys Phe Ile Trp Leu Gly Arg Ala Thr Asp Tyr Ile Gln
        355                 360                 365

Ala Ser Thr Ser Leu Leu Pro Gln Arg Phe Lys His Glu Leu Arg Tyr
    370                 375                 380

Gln Met Thr Ser Gly Glu Ser Leu Asp Pro Arg Pro Leu Glu Asn Phe
385                 390                 395                 400

Leu Arg Ser Ala Glu Phe Ile Ser Asn Val Cys Val Ile Gly Asp Asn
                405                 410                 415

Phe Leu Arg Gly Ala Ser Thr Ser Val Trp Ala Val Ile Glu Leu Thr
            420                 425                 430

Asp Ser Ala His Arg Leu Asp Ala Ala Ser Ala Arg Lys Gln Val Ala
        435                 440                 445

Arg Val Leu Ala Pro Leu Asn Gly Gly Leu Pro Pro Ala Leu Arg Ile
    450                 455                 460

Ser Met Ser Ser Val Leu Ile Leu Ser Gly Ser Gln Lys Ile Pro Arg
465                 470                 475                 480

Thr Lys Lys Gly Glu Ile Phe Arg Lys Gln Ile Glu Asp Leu Phe Gly
                485                 490                 495

Ala Ala Met Ser Met Pro Pro Glu Ala Glu Pro Ala Leu Glu Asp Glu
            500                 505                 510

Leu Thr Ser Ile Val Gly Asn Val Leu Asn Ile Ser Asp Ser Asp Met
        515                 520                 525

Leu Thr Ser Met Thr Phe Ala Glu Leu Gly Met Thr Ser Val Leu Ala
    530                 535                 540

Val Lys Ile Ser Ala Arg Leu Asn Glu His Leu Ala Gly Arg Ala Val

545                 550                 555                 560

Leu Pro Ala Asn Ile Cys Tyr Ile Tyr Ile Asp Thr Pro Ser Leu Ile
                565                 570                 575

Ala Gly Ile Arg Asn Phe Leu Ser Pro Ala Ser Ser Asp Gln Glu Pro
            580                 585                 590

Ser Asn Phe Ala Thr Thr Ala Asp Lys Lys Asp Glu Ile Val Ile Ile
        595                 600                 605

Gly Lys Ala Phe Arg Leu Pro Gly Gly Ile Asn Asp Asp Ser Ala Leu
    610                 615                 620

Trp Ala Ala Leu Met Gly Lys Asn Asp Ser Val Ile Ala Asp Ile Pro
625                 630                 635                 640

Pro Asp Arg Trp Asp His Ala Ser Phe Tyr Pro Ala His Ile Cys Leu
                645                 650                 655

Arg Lys Ala Gly Leu Ile Asp Met Ser Ser Tyr Asp Tyr Gly Phe Phe
            660                 665                 670

Gly Leu Ser Ala Thr Glu Ala Tyr Tyr Val Ser Pro Thr Met Arg Ala
        675                 680                 685

Ala Leu Glu Val Ala Phe Glu Ala Leu Glu Asp Ala Asn Ile Pro Val
    690                 695                 700

Ser Arg Ile Lys Gly Thr Asn Thr Ser Val Phe Val Ala Thr Lys Asp
705                 710                 715                 720

Asp Gly Phe Glu Thr Leu Leu Asn Ala Ala His Gly Phe Asp Asp Ala
                725                 730                 735

Asp Gly Tyr Val Pro Ser Glu Gly Ala Val Ala Phe Ile Leu Lys Thr
            740                 745                 750

Arg Thr Ala Ala Glu Arg Asp Gly Asp Arg Ile Met Ala Ile Ile Lys
        755                 760                 765

Ala Thr Glu Val Ser His Asn Gly Arg Ser Gln Gly Leu Ala Ala Pro
    770                 775                 780

Asn Val Lys Ala Gln Ala Ala Leu His Arg Ala Val Leu Arg Lys Ala
785                 790                 795                 800

Lys Leu Asp Pro Leu Asp Ile His Phe Ile Glu Ala His Gly Thr Gly
                805                 810                 815

Thr Pro Leu Gly Asp Leu Cys Glu Ile Gln Gly Ile Asn Glu Ala Phe
            820                 825                 830

Val Ser Ala Arg Pro Arg Ala Glu Asp Pro Leu Ile Val Ser Ala Ser
        835                 840                 845

Lys Ser Ser Leu Gly His Thr Glu Pro Ser Ala Gly Leu Val Gly Met
    850                 855                 860

Leu Ser Val Leu Met Ala Leu Lys His Gly Ile Val Pro Gly Leu Leu
865                 870                 875                 880

His Leu Arg Ala Asp Asn Ala Asn His Gln Leu Asp Leu Thr Gln Val
                885                 890                 895

Pro Leu Arg Ile Ser Pro Glu Pro Val Val Ile Ala Ala Ser Lys Pro
            900                 905                 910

His His Ala Met Val Leu Ser Tyr Gly Phe Ser Gly Thr Leu Ala Asp
        915                 920                 925

Ile Val Leu Glu Ser Pro Glu Glu Pro Ser Ser Pro Asn Pro Gly Ala
    930                 935                 940

Ala Gly Pro Met Ile Phe Val Leu Ser Ala Lys Thr Ser Ala Ala Leu
945                 950                 955                 960

Ser Ala Tyr Ile Lys Ala Tyr Ile Ala Phe Leu Gln Asn Ala Asp Ala
                965                 970                 975

His Glu Phe Tyr Asn Ile Cys Tyr Thr Ala Cys Val Gly Arg Glu His
            980                 985                 990

Tyr Lys His Arg Phe Ala Cys Val  Ala Asn Asp Leu Ala  Asp Leu Ile
        995                 1000                 1005

Arg Gln  Leu Gln Asp Cys Ala  Ser Ala Leu Ala Pro  Thr Lys Ser
    1010                 1015                 1020

Ser Thr  Gly Ala Leu Ala Phe  Ala Phe Pro Gly Gln  Gly Val Gln
    1025                 1030                 1035

Phe Pro  Gly Met Ala Ala Ala  Leu Ala Lys Arg His  Ser Ile Phe
    1040                 1045                 1050

Arg Asp Tyr Val Met Glu Phe Gly Asp Arg Ala Gln Asp Leu Cys
    1055                1060                1065

Gly Leu Pro Ile Ala Lys Met Leu Leu Asp Val Asp Ala Ala Glu
    1070                1075                1080

Glu Glu Asp Ile His Ser Asp Val Asp Gln Ile Cys Val Phe Val
    1085                1090                1095

Tyr Gln Tyr Ser Met Cys Arg Trp Leu Arg Glu Leu Gly Leu Glu
    1100                1105                1110

Ala Ser Ala Val Ile Gly His Ser Leu Gly Glu Ile Thr Ala Ala
    1115                1120                1125

Leu Ile Gly Asp Ala Phe Thr Phe Glu Val Ala Leu Asp Leu Val
    1130                1135                1140

Val Thr Arg Gly Arg Leu Leu Arg Pro Ser Gln Gly Asn Pro Gly
    1145                1150                1155

Gly Met Ala Ala Leu Ala Cys Pro Glu Glu Asn Val Pro Ala Ile
    1160                1165                1170

Leu Asp Gln Cys Arg Val Asp Ser Thr Ile Ser Val Ser Val Ile
    1175                1180                1185

Asn Gly Pro Arg Ser Leu Cys Val Ser Gly Ala Ser Asn Asp Ile
    1190                1195                1200

Asp Glu Phe Val Lys Met Ala Lys Arg Gln Asn Ile Lys Ala Thr
    1205                1210                1215

Arg Leu Arg Val Asp Gln Gly Phe His Ser Pro Arg Val Asp Ser
    1220                1225                1230

Ala Ala Val Gly Leu Arg Ala Trp Ser Gly Ser Phe Ser Lys Ser
    1235                1240                1245

Phe Gln Pro Leu Arg Ile Thr Leu Tyr Ser Thr Ser Leu Gly Ala
    1250                1255                1260

Ala Ile Ser Lys Gly Glu Ile Leu Asn Gln Thr His Trp Ala Asp
    1265                1270                1275

His Val Arg Arg Pro Val Ile Phe Ser Lys Ala Ala Ala Ala Ile
    1280                1285                1290

Leu Glu Asp Lys Ser Ile Gly Ala Ile Leu Asp Ile Gly Pro Gln
    1295                1300                1305

Thr Val Ala Trp Ser Leu Leu Leu Ala Asn Gly Cys Asn Val Ala
    1310                1315                1320

Ser Ala Val Ala Leu Ser Gly Arg Arg Val Gln Asp Gln Glu Thr
    1325                1330                1335

Ala Phe Leu Ser Ala Leu Ala Asn Leu Tyr Gln Asn His Gly Val
    1340                1345                1350

Thr Pro Asn Phe Arg Val Phe Tyr Ala His Gln Ala Val Gln Ala
    1355                1360                1365

Arg Tyr Arg Thr Val Asp Ile Pro Lys Tyr Pro Phe Gln Arg Arg
    1370                1375                1380

His Arg Tyr Pro Ser Tyr Ile Pro Ser Arg Asn Ala Thr Gly Ala
    1385                1390                1395

Asn Arg Leu Lys Glu Pro Phe Arg Ser Asp Leu Asp Glu Pro Ala
    1400                1405                1410

Gln Asp Thr Glu His Thr Ala Glu Leu Arg Val Asp Met Thr Pro
    1415                1420                1425

Glu Lys Leu Arg Asp Ala Leu Met His Cys Val Arg Asp Thr Leu
    1430                1435                1440

Glu Gly Glu Asp Phe Asp Glu Ser Glu Ser Leu Val Ser Arg Gly
    1445                1450                1455

Ile Asp Ser Ile Thr Phe Ala Gly Leu Arg Lys Arg Val Gln Glu
    1460                1465                1470

Arg His Gly Leu Asn Leu Ser Ile Ile Phe Trp Ser Asp Gly Phe
    1475                1480                1485

Ser Val Lys Asp Met Val Asp Ser Leu Ile Glu Gln His Phe Val
    1490                1495                1500

His

<210> 38

<211>  4803
<212>  DNA
<213>  Panellus stipticus

<400>  38
atggcactgg tctctcccac ttgttcgtct cagatgcctc ctgctccttc ctccatcctt      60

gatgtgttca ccgagactgc atacaatcct cgcacctcca accgtcctgt agtagaatgt     120

ggcgagcacg tctggaccta ctcacaactt gaagcagttt ccaatgccat gtcgcaagac     180

ctggagaact cgatcggtct ttacgcgaag gtcgcttttg tcggtgaaaa ccatccattt     240

gttttcgctc tcatgcttgc agtctggaaa attgctggca cattcatccc cattgatgcg     300

cacattcctc ccgccctcct agatggcatg gtcgacattg tgaagccgat gtgcatctat     360

gtatcatcgg ccgatacctc taattcctcc tgggcctcag aactcgcggt cgaggttcgg     420

gtattccgtc cggaagaatc tacgattccg gctttgaacg aacactatgg aagatccagc     480

atcactcccg ctcagcatcg gccaatactc aacgtggtcc aggcagttcc cctgacacac     540

aaatttatcc tcagcaactg tcagtccaaa ctcgcgtggt ggcgacgcat gcagccaagc     600

atcaacctag acaacacccg agtcctcggc tgggccccgt ggtcgcatgt attatcccac     660

atgcaggaca ttggcaccgc taccattctc accgcaggct gttacgtctt tgcctctatc     720

ccttccacat atcagcttca gcaggcgcca acagatctta cgacccaagt catcaacggt     780

atcctcaata aaaacatctc ggcgcttgct gcccttccat ttgttttcgg cggtatcaaa     840

gcagcgtgcg agtcgggcga tctggatgtg gaggcacttc tgggtgccct gcgccgcatg     900

acgatgctcg aatgtggcgg ggctgcgctc gaccctgcaa ttgcaaattg gacggatata     960

aacggtgtat cgctcatggt tgggattgga atgacagaaa cgggcggtgc aatcttcgcg    1020

gggagggcga aagactcgct ctccgggttt cttgctgagg gtctgatctc agacgccaga    1080

attgagcttg acaagggtga atctgatggc tcggatggta cgacgttctc catcttctcg    1140

accactgccg gaattaaacc tgattgtcag agggagagct tgttgtcaca agcaagctac    1200

ttccacatgg ctacattggc ttcgatgacg ggtcattttc cgtggatgcg caagggtgac    1260

aacttcctcc ggggtgcctc cacttctgtg tgggccatta tcgagctcac ggacagcgcg    1320

caccgcctgg atgccgcttc agcgagaaag caggtggcgc gcgtactggc gccgctcaat    1380

ggtggcctcc cacccgctct ccggatctca atgtcctcgg tgttgatatt gagcggatca    1440

cagaaaattc cgcgcacgaa gaagggcgag atcttccgta agcagattga ggatctgttt    1500

ggtgccgcga tgagcatgcc gcctgaggct gagccggctc tggaggatga actaacaagt    1560

attgttggaa atgtcctcaa catatcggac agcgacatgt tgacctcgat gacttttgcc    1620

gaacttggaa tgacctctgt tctggcagta aaaatctcag caaggctcaa cgagcacctt    1680

gctgggcggg ccgtcctccc ggccaacatc tgttacatct acatcgatac tccatcactg    1740

attgccggca tccggaactt cctttcgccg acatcttctg atcaagagcc atcgaacttt 1800

gccaccacgg cagacaagaa ggacgagatc gtcatcatag gcaaagcctt tcggcttccc 1860

ggcgggatca acgacgactc tgctctctgg gctgctctta tgggcaagaa cgactctgtt 1920

attgcagaca tcccgccaga ccgttgggat catgctagct tctatcccgc ccacatctgc 1980

ttgcgcaagg ctgggcttat agatatgtcc agctatgact atgggttctt tggcctttcg 2040

gcgactgagg cgtactatgt gtcgcccacc atgcgcgcag cgctcgaggt ggcgttcgaa 2100

gcgctggagg atgcgaacat cccggtgtca agaattaagg ggacaaatac gagtgttttt 2160

gttgcaacga aagatgatgg atttgagaca ttgctgaatg cggcgcatgg atttgatgcg 2220

tatacccggt tctacgggac tggcgggctc ccaagtactg ccagtgggcg cataagctac 2280

cttcttgaca tccatgggcc ctcactcaca gtagatacgg cctgtagcgg aggaattgtc 2340

tgcattgatc aagatatacc agcgaattta tgtatcatag cgatcgcata cctccagtct 2400

ggcgccggtg aatcagccat cgaacgacaa tacaggtttc tcacggcgca gaacatggca 2460

tcgcccacta gccgctgttc caccttcact gcagatgctg acggatatgt cccttcagaa 2520

ggcgccgtcg cttttatact gaagacacgg actgcggcgg agaggatggg ggatcgaatt 2580

atggccatca tcaaagccac agaagtctcg cataatggaa gatctcaggg actcgccgcg 2640

ccgaacgtca aagcgcaagc cgctcttcat agagcagtct tgcgcaaagc taagctcgac 2700

cctctggaca tccatttcat tgaagcccac ggaactgttt taggaacacc gctgggtgat 2760

ctttgtgaaa tacaaggcat aaatgaagcc tttgtctccg cacgtccacg tgcggaggat 2820

ccactcattg tcagcgcctc caagtcttcc cttgggcaca ccgaaccttc ggctggattg 2880

gttgggatgc tatctgtgtt gatggccttg aagcacggca tcgtgcctgg gttgctccat 2940

ctccgagcgg acaatgctaa ccaccaactg gacctgacac gagtcccact gcgtatatca 3000

ccggaacctg tagtcatcgc cgcatccaag ccgcatcacg ccatggtgct gctcacagtc 3060

cgtacattgg cagacattgt tttggagagt ccggaagaac caccgtccca gaacccggga 3120

gccgcgggcc caatgatatt tgtcctcagc gcgaagactt ccgcggctct gtcggcatat 3180

atcaaggctt acattgcatt tctacagaat gcagacgcgc acgagtttta caacatctgc 3240

tacaccgcct gtgttggaag agaacattac aaacacagat ttgcttgcgt tgcaaatgac 3300

cttgctgatc tgattcgcca attacaagac tgtgcgagtg cgctggctcc aaccaaaagt 3360

agtactggtg ccttggcgtt cgcgttccct ggccaaggcg tgcaatttcc aggcatggcc 3420

gctgcacttg ctaaaaggca ttccatattt cgcgactatg tcatggaatt tggtcataga 3480

gcacaagatc tttgtggctt gcccatcgca aagatgttgc tggacgtgga tgcagcagag 3540

gaagaagata tccacagtga cgtagaccag atttgcgtct ttgtctatca gtattcgatg 3600

tgtcgatggc ttagggagct cgggctcgag gcaagtgcgg ttatcgggca cagtttgggc 3660

gaaataacag ccgcacttat cggggacgca tttacatttg aagtggctct cgatctcgtt 3720

gtcactcgag gacggctact ccgcccttct caagggaatc caggcgggat ggctgcgctg 3780

gcatgccccg aggagaatgt cccagcgatt ttggaccaat gtcgtgtgga cagtaccatt 3840

agcgtctccg ttatcaatgg tccgaggagc ctttgtgtat ccggagcttc caacgacatc 3900

gacgagtttg tcaagatggc aaaacggcaa aacatcaaag cgactcgact gagggtggac 3960

caaggatttc atagccctcg agttgatagt gccgcggttg gactgcgtga atggtcaggt 4020

tctttttcga aatcatttca gccgttgcgc atcacattat actctacgtc tctgggtgct 4080

gcaatctcga aaggagagat tttgactcag acacattggg ccgatcacgt ccgccgtccg 4140

gtcatattct caaaagcagc agcagccatc ctcgaggaca agtccattgg cgcgatcctg 4200

gatatcggac cacagacggt ggcatggtct ctccttctgg cgaacggctg caacgtcgcg 4260

tcagctgttg ccctgtccgg ccgaagagta caagatcagg aaacggcctt tttatctgca 4320

ctggcgaatc tgtatcaaaa tcacggggtg acgccgaatt ttcgcgtatt ttacgctcac 4380

caggcagtcc aggcgcgcta tagaaccgtg gacattccga agtatccctt ccaacgccga 4440

catcgatatc catcctacat tccatcgcgc aatgccacgg gtgccaacag actgaaagaa 4500

ccattccgta gcgacctgga tgaaccggct caagacacgg agcacaccgc ggaaatgaga 4560

gtggacatga ctccggagaa gctccgggac gccctgatgc actgtgtgcg ggacacactg 4620

gaaggcgaag atttgatgaa atcggaatcc ctcgtttcgc gtggaattga ctccattact 4680

tttgcgggtc tacggaagcg tgttcaagaa cgacacggac ttaatctttc catcattttc 4740

tggtctgatg ggttttctgt gaaagacatg gtcgacagcc tcatcgaaca gcattttgtc 4800

cac 4803

<210> 39
<211> 1601
<212> PRT
<213> Panellus stipticus

<400> 39

Met Ala Leu Val Ser Pro Thr Cys Ser Ser Gln Met Pro Pro Ala Pro
1               5                   10                  15

Ser Ser Ile Leu Asp Val Phe Thr Glu Thr Ala Tyr Asn Pro Arg Thr
            20                  25                  30

Ser Asn Arg Pro Val Val Glu Cys Gly Glu His Val Trp Thr Tyr Ser
        35                  40                  45

Gln Leu Glu Ala Val Ser Asn Ala Met Ser Gln Asp Leu Glu Asn Ser
    50                  55                  60

Ile Gly Leu Tyr Ala Lys Val Ala Phe Val Gly Glu Asn His Pro Phe
65                  70                  75                  80

Val Phe Ala Leu Met Leu Ala Val Trp Lys Ile Ala Gly Thr Phe Ile
                85                  90                  95

Pro Ile Asp Ala His Ile Pro Pro Ala Leu Leu Asp Gly Met Val Asp
            100                 105                 110

Ile Val Lys Pro Met Cys Ile Tyr Val Ser Ser Ala Asp Thr Ser Asn
        115                 120                 125

Ser Ser Trp Ala Ser Glu Leu Ala Val Glu Val Arg Val Phe Arg Pro
    130                 135                 140

Glu Glu Ser Thr Ile Pro Ala Leu Asn Glu His Tyr Gly Arg Ser Ser
145                 150                 155                 160

Ile Thr Pro Ala Gln His Arg Pro Ile Leu Asn Val Val Gln Ala Val
                165                 170                 175

Pro Leu Thr His Lys Phe Ile Leu Ser Asn Cys Gln Ser Lys Leu Ala
            180                 185                 190

Trp Trp Arg Arg Met Gln Pro Ser Ile Asn Leu Asp Asn Thr Arg Val
        195                 200                 205

Leu Gly Trp Ala Pro Trp Ser His Val Leu Ser His Met Gln Asp Ile
    210                 215                 220

Gly Thr Ala Thr Ile Leu Thr Ala Gly Cys Tyr Val Phe Ala Ser Ile
225                 230                 235                 240

Pro Ser Thr Tyr Gln Leu Gln Gln Ala Pro Thr Asp Leu Thr Thr Gln
                245                 250                 255

Val Ile Asn Gly Ile Leu Asn Lys Asn Ile Ser Ala Leu Ala Ala Leu
            260                 265                 270

Pro Phe Val Phe Gly Gly Ile Lys Ala Ala Cys Glu Ser Gly Asp Leu
        275                 280                 285

Asp Val Glu Ala Leu Leu Gly Ala Leu Arg Arg Met Thr Met Leu Glu
    290                 295                 300

Cys Gly Gly Ala Ala Leu Asp Pro Ala Ile Ala Asn Trp Thr Asp Ile

305                 310                 315                 320

Asn Gly Val Ser Leu Met Val Gly Ile Gly Met Thr Glu Thr Gly Gly
                325                 330                 335

Ala Ile Phe Ala Gly Arg Ala Lys Asp Ser Leu Ser Gly Phe Leu Ala
            340                 345                 350

Glu Gly Leu Ile Ser Asp Ala Arg Ile Glu Leu Asp Lys Gly Glu Ser
        355                 360                 365

Asp Gly Ser Asp Gly Thr Thr Phe Ser Ile Phe Ser Thr Thr Ala Gly
    370                 375                 380

Ile Lys Pro Asp Cys Gln Arg Glu Ser Leu Leu Ser Gln Ala Ser Tyr
385                 390                 395                 400

Phe His Met Ala Thr Leu Ala Ser Met Thr Gly His Phe Pro Trp Met
                405                 410                 415

Arg Lys Gly Asp Asn Phe Leu Arg Gly Ala Ser Thr Ser Val Trp Ala
            420                 425                 430

Ile Ile Glu Leu Thr Asp Ser Ala His Arg Leu Asp Ala Ala Ser Ala
        435                 440                 445

Arg Lys Gln Val Ala Arg Val Leu Ala Pro Leu Asn Gly Gly Leu Pro
    450                 455                 460

Pro Ala Leu Arg Ile Ser Met Ser Ser Val Leu Ile Leu Ser Gly Ser
465                 470                 475                 480

Gln Lys Ile Pro Arg Thr Lys Lys Gly Glu Ile Phe Arg Lys Gln Ile
                485                 490                 495

Glu Asp Leu Phe Gly Ala Ala Met Ser Met Pro Pro Glu Ala Glu Pro
            500                 505                 510

Ala Leu Glu Asp Glu Leu Thr Ser Ile Val Gly Asn Val Leu Asn Ile
        515                 520                 525

Ser Asp Ser Asp Met Leu Thr Ser Met Thr Phe Ala Glu Leu Gly Met
    530                 535                 540

Thr Ser Val Leu Ala Val Lys Ile Ser Ala Arg Leu Asn Glu His Leu
545                 550                 555                 560

Ala Gly Arg Ala Val Leu Pro Ala Asn Ile Cys Tyr Ile Tyr Ile Asp
                565                 570                 575

Thr Pro Ser Leu Ile Ala Gly Ile Arg Asn Phe Leu Ser Pro Thr Ser
            580                 585                 590

Ser Asp Gln Glu Pro Ser Asn Phe Ala Thr Thr Ala Asp Lys Lys Asp
        595                 600                 605

Glu Ile Val Ile Ile Gly Lys Ala Phe Arg Leu Pro Gly Gly Ile Asn
    610                 615                 620

Asp Asp Ser Ala Leu Trp Ala Ala Leu Met Gly Lys Asn Asp Ser Val
625                 630                 635                 640

Ile Ala Asp Ile Pro Pro Asp Arg Trp Asp His Ala Ser Phe Tyr Pro
                645                 650                 655

Ala His Ile Cys Leu Arg Lys Ala Gly Leu Ile Asp Met Ser Ser Tyr
            660                 665                 670

Asp Tyr Gly Phe Phe Gly Leu Ser Ala Thr Glu Ala Tyr Tyr Val Ser
        675                 680                 685

Pro Thr Met Arg Ala Ala Leu Glu Val Ala Phe Glu Ala Leu Glu Asp
    690                 695                 700

Ala Asn Ile Pro Val Ser Arg Ile Lys Gly Thr Asn Thr Ser Val Phe
705                 710                 715                 720

Val Ala Thr Lys Asp Asp Gly Phe Glu Thr Leu Leu Asn Ala Ala His
                725                 730                 735

Gly Phe Asp Ala Tyr Thr Arg Phe Tyr Gly Thr Gly Arg Ala Pro Ser
            740                 745                 750

Thr Ala Ser Gly Arg Ile Ser Tyr Leu Leu Asp Ile His Gly Pro Ser
        755                 760                 765

Leu Thr Val Asp Thr Ala Cys Ser Gly Gly Ile Val Cys Ile Asp Gln
    770                 775                 780

Asp Ile Pro Ala Asn Leu Cys Ile Ile Ala Ile Ala Tyr Leu Gln Ser
785                 790                 795                 800

Gly Ala Gly Glu Ser Ala Ile Glu Arg Gln Tyr Arg Phe Leu Thr Ala
                805                 810                 815

Gln Asn Met Ala Ser Pro Thr Ser Arg Cys Ser Thr Phe Thr Ala Asp
            820                 825                 830

Ala Asp Gly Tyr Val Pro Ser Glu Gly Ala Val Ala Phe Ile Leu Lys
        835                 840                 845

Thr Arg Thr Ala Ala Glu Arg Asp Gly Asp Arg Ile Met Ala Ile Ile
    850                 855                 860

Lys Ala Thr Glu Val Ser His Asn Gly Arg Ser Gln Gly Leu Ala Ala
865                 870                 875                 880

Pro Asn Val Lys Ala Gln Ala Ala Leu His Arg Ala Val Leu Arg Lys
                885                 890                 895

Ala Lys Leu Asp Pro Leu Asp Ile His Phe Ile Glu Ala His Gly Thr
            900                 905                 910

Val Leu Gly Thr Pro Leu Gly Asp Leu Cys Glu Ile Gln Gly Ile Asn
        915                 920                 925

Glu Ala Phe Val Ser Ala Arg Pro Arg Ala Glu Asp Pro Leu Ile Val
    930                 935                 940

Ser Ala Ser Lys Ser Ser Leu Gly His Thr Glu Pro Ser Ala Gly Leu
945                 950                 955                 960

Val Gly Met Leu Ser Val Leu Met Ala Leu Lys His Gly Ile Val Pro
                965                 970                 975

Gly Leu Leu His Leu Arg Ala Asp Asn Ala Asn His Gln Leu Asp Leu
            980                 985                 990

Thr Arg Val Pro Leu Arg Ile Ser Pro Glu Pro Val Val Ile Ala Ala
        995                 1000                1005

Ser Lys Pro His His Ala Met Val Leu Leu Thr Val Arg Thr Leu
    1010                1015                1020

Ala Asp Ile Val Leu Glu Ser Pro Glu Glu Pro Pro Ser Gln Asn
    1025                1030                1035

Pro Gly Ala Ala Gly Pro Met Ile Phe Val Leu Ser Ala Lys Thr
    1040                1045                1050

Ser Ala Ala Leu Ser Ala Tyr Ile Lys Ala Tyr Ile Ala Phe Leu
    1055                1060                1065

Gln Asn Ala Asp Ala His Glu Phe Tyr Asn Ile Cys Tyr Thr Ala
    1070                1075                1080

Cys Val Gly Arg Glu His Tyr Lys His Arg Phe Ala Cys Val Ala
    1085                1090                1095

Asn Asp Leu Ala Asp Leu Ile Arg Gln Leu Gln Asp Cys Ala Ser
    1100                1105                1110

Ala Leu Ala Pro Thr Lys Ser Ser Thr Gly Ala Leu Ala Phe Ala
    1115                1120                1125

Phe Pro Gly Gln Gly Val Gln Phe Pro Gly Met Ala Ala Ala Leu
    1130                1135                1140

Ala Lys Arg His Ser Ile Phe Arg Asp Tyr Val Met Glu Phe Gly
    1145                1150                1155

His Arg Ala Gln Asp Leu Cys Gly Leu Pro Ile Ala Lys Met Leu
    1160                1165                1170

Leu Asp Val Asp Ala Ala Glu Glu Glu Asp Ile His Ser Asp Val
    1175                1180                1185

Asp Gln Ile Cys Val Phe Val Tyr Gln Tyr Ser Met Cys Arg Trp
    1190                1195                1200

Leu Arg Glu Leu Gly Leu Glu Ala Ser Ala Val Ile Gly His Ser
    1205                1210                1215

Leu Gly Glu Ile Thr Ala Ala Leu Ile Gly Asp Ala Phe Thr Phe
    1220                1225                1230

Glu Val Ala Leu Asp Leu Val Val Thr Arg Gly Arg Leu Leu Arg
    1235                1240                1245

Pro Ser Gln Gly Asn Pro Gly Gly Met Ala Ala Leu Ala Cys Pro
    1250                1255                1260

Glu Glu Asn Val Pro Ala Ile Leu Asp Gln Cys Arg Val Asp Ser
    1265                1270                1275

Thr Ile Ser Val Ser Val Ile Asn Gly Pro Arg Ser Leu Cys Val
    1280                1285                1290

Ser Gly Ala Ser Asn Asp Ile Asp Glu Phe Val Lys Met Ala Lys

1295                1300                1305

Arg Gln Asn Ile Lys Ala Thr Arg Leu Arg Val Asp Gln Gly Phe
    1310                1315                1320

His Ser Pro Arg Val Asp Ser Ala Ala Val Gly Leu Arg Glu Trp
    1325                1330                1335

Ser Gly Ser Phe Ser Lys Ser Phe Gln Pro Leu Arg Ile Thr Leu
    1340                1345                1350

Tyr Ser Thr Ser Leu Gly Ala Ala Ile Ser Lys Gly Glu Ile Leu
    1355                1360                1365

Thr Gln Thr His Trp Ala Asp His Val Arg Arg Pro Val Ile Phe
    1370                1375                1380

Ser Lys Ala Ala Ala Ala Ile Leu Glu Asp Lys Ser Ile Gly Ala
    1385                1390                1395

Ile Leu Asp Ile Gly Pro Gln Thr Val Ala Trp Ser Leu Leu Leu
    1400                1405                1410

Ala Asn Gly Cys Asn Val Ala Ser Ala Val Ala Leu Ser Gly Arg
    1415                1420                1425

Arg Val Gln Asp Gln Glu Thr Ala Phe Leu Ser Ala Leu Ala Asn
    1430                1435                1440

Leu Tyr Gln Asn His Gly Val Thr Pro Asn Phe Arg Val Phe Tyr
    1445                1450                1455

Ala His Gln Ala Val Gln Ala Arg Tyr Arg Thr Val Asp Ile Pro
    1460                1465                1470

Lys Tyr Pro Phe Gln Arg Arg His Arg Tyr Pro Ser Tyr Ile Pro
    1475                1480                1485

Ser Arg Asn Ala Thr Gly Ala Asn Arg Leu Lys Glu Pro Phe Arg
    1490                1495                1500

Ser Asp Leu Asp Glu Pro Ala Gln Asp Thr Glu His Thr Ala Glu
    1505                1510                1515

Met Arg Val Asp Met Thr Pro Glu Lys Leu Arg Asp Ala Leu Met
    1520                1525                1530

His Cys Val Arg Asp Thr Leu Glu Gly Glu Asp Phe Asp Glu Ser
    1535                1540                1545

Glu Ser Leu Val Ser Arg Gly Ile Asp Ser Ile Thr Phe Ala Gly
    1550                1555                1560

Leu Arg Lys Arg Val Gln Glu Arg His Gly Leu Asn Leu Ser Ile
    1565                1570                1575

Ile Phe Trp Ser Asp Gly Phe Ser Val Lys Asp Met Val Asp Ser
    1580                1585                1590

Leu Ile Glu Gln His Phe Val His
    1595                1600

<210>  40
<211>  5073
<212>  DNA
<213>  Neonothopanus gardneri

<400>  40
atgaattcca aagtgaatct tccctccact ttgcttgatg ttttcctcga gatcgctggc      60

gagccatcta ccgcttcgcg tgatgttttg gaatgtggcg agcacagatg gacttaccag     120

cagcttgacg ttgtttcatc tgctttagcc cagcatctca agtacactgt cggtctatct     180

cctacagtcg cagtgatcag cgaaaatcat ccttatgttt ttgccttgat cctggctgtg     240

tggaaagttg gggcattttt cgcgcccctc gacgcacatg ctcctgctga gttggtagct     300

ggcatgttaa gcataatctc tccttcgtgc ttagtacttc agagcacaga tgtagctaat     360

caaactcttg cgtgtgatct cgatattcct gttgaggtat tccactcgcg tcaatccact     420

attcctgaac taaacaagaa atatctcacc gattctgggt taccggcggg ttttccgctc     480

tcagattcaa acaaaccggc tctgtatctc ttcacctcgt ctgccacttc tcggagcaat     540

cttaaatgcg tgcctctcac tcacgctttc atcctgagca atagcctctc gaaacgcgca     600

tggtgccaac gtatgcggcc agagacagac ttcgatggca tacgcgttct tggatgggct     660

ccatggtctc atgtcttggc gcacatgcag gacatcgggc ccgtcacttt actcaatgct     720

ggatgctacg tctttgctac tatcccttcc tcgtacccca cggatgtgca gagtgacagc     780

aatttgatat ctcatgtcgc aaatgctatc atacacaagg gtgtaaaatc gtttgcttgt     840

ctcccttttg tactcggagg gctgaaagca ttatgcgagt ccaagccatc cgtcaaagca     900

gatctacagg tcgaagagca agctcagctt ttgatctctc taaggcgcat ggaaattctt     960

gaatgcggag gtgcgatgct cgaagcgaat gttgcgtctt gggctatcga gaatcatatt    1020

ccggtctcta ttggtatcgg tatgacagaa actggtggcg cgcttttcgc tggacctgtt    1080

caggatatcc ggaccggttt ttctgcggac aataaattca tcaaggacgc tacctacttc    1140

ctagttgcaa atggagatga atctgggaac gatgtaactg aaggggagct agttgtgaaa 1200

agtaaaatgc tcccgcgagg ctatcttggc tacaatgatt cttccttttc cgttgacgat 1260

gacggattgg ttacgttcaa gacaggcgac agatacagtg ttacacgcga cggaagattc 1320

tcttggctag gcaggaatac tgatttcatt cagatgacta gtggagagac attagatccc 1380

cgacctgtcg agagttcgct ctgccaaagt cctctcatct ctcaagcatg cgttattgga 1440

gacaagtttc tcaacgggcc tgccactgct gtttgcgcga tcgttgagct tgagccgaca 1500

atggtagaaa cgggagaggc taactcgcgg gacatagtcc aagtctttgc acccatcaac 1560

cgagacctgc ctcctccttt aaggatagca tggtcgcaca ttttaattct caagcattcc 1620

gagaagatac cgatgacgaa gaagggaacg attttccgta agaagattga gcagatgttt 1680

ggcgctgcat tgggcattgc ttccatccct acaaacagtg tcaatggatc cgttggctcc 1740

ggagaagagc ctcaagctac tgcagatgag actgttctac aagatgaact atcaaatacc 1800

gtgaagaaaa taatcagccg tgttctagga gtaactgatg aggaattgct ttggacactg 1860

tcattcgcag agctgggaat gacctcaaca ctggctattc gtatcgtaaa cgagctgaac 1920

gaaactctcg ttgggggcga tctccctatc aatgcttgct acatttatgt cgacctttcc 1980

tctctgagca aggctgtgta tgcgaaactg gctcatctcg agctgtcaga tcatgttcct 2040

gagctcaaac aagctccctt caagtcttct ggcgggaaag agattgtcat tgtcggccaa 2100

gcgtttcgtc tacccggctc aatcaatgac gttgcctctc ttcgggatgc attcctagca 2160

agacagacat cgtccatcat cgctggaata ccttccgatc gctgggacca tgccagcttc 2220

tatcccaagg acatatgttt cgacaaggct ggtcttgtgg atatagctca ttatgatcat 2280

agcttcttcg gaatcacagc aacggaagcg ctccatctgt ctccaaccat gcgtcttgca 2340

ctggaagttt cgtttgaggc gcttgagaat gctaatatcc caacgtcgcg attgaaaggc 2400

tcgcagacgg cagtttatgt tgcaacaaca gatgacggat ttgagaccct gttgaatgct 2460

gaggctggct atgaagctta tacacggttt tatggtactg gtcgggcagc aagtacggcg 2520

agtgggcgca taagctatct tcttgatata catggaccct ccgtcactat tgacacggca 2580

tgcagtggtg gagttgtttg tatcgatcaa gcaatcaact atttacaatc gtcgagttca 2640

gcggacaccg ctatcgtgtt cctttctgct caagggatgg tctccccgag gggacgatgt 2700

gcgacattta cggctgatgc tgacggctat gtcccttctg agggtgcagt tgctttcgta 2760

ttgaaaactc gcgaagccgc tatacgcgac aaagacaaca ttctcgctac aatcaaagcg 2820

acacagattt cgcacaacgg ccgctcacaa ggacttgtag caccgaacgt aaactcgcaa 2880

gttgaccttc atcgttcgtt gctcgagaaa gctcgtctta gtcctgctga tgtccaattc 2940

attgaagctc atgggacagg aacgtcattg ggagatctct cagagattca agctataaac 3000

gctgcttaca cttcctctca gccacgtacg accggcccac tcatagtcag cgcttccaag 3060

actgtcgttg gccataccga accagctggt cctttggttg gtatgttgtc ggtcttgctg 3120

tctttccagg aaggcgcagt ccctggtctc gctcatctta ccacacgtaa cctcaatcct 3180

acgttggact attcttcagt gccgcttctc attccctctg aacctgttcg tctacaaaca 3240

ccaaagcctt atagagctgc cgtaatgtcc tacggctttt cgggtaccct ggccggcctc 3300

gttctagaga gccctgacga acgtagctca gaagaagagc cgcctgatga caagccgatg 3360

ctgttcgtcg ttagcgcaaa gacacacacg gcactaattg aatacctgca acggtatctc 3420

gagttcctct tacatgcgaa tccccgcgat ttctgtgata tctgctacac aagctgtgtc 3480

gggagggaac actatagata tcggtttgct tgtgttgcaa atgacatgga agacctcatt 3540

ggccaacttc aaagacgttt gtctagcaag ttaccatcga agccgctata caaacgcggt 3600

gctctggcct ttgcgttttc tggacagggg acacagtttc aaggaatggc gacagatctt 3660

gcgaagaggt actctggctt ccgaaaaatc gtttccggac tcgcaaagag tgctggcgag 3720

ctctcgggtt acgctattga ccgttatctt ctcgcgtatg acgttggtag cagtattgct 3780

actcctaata gtgaggtgga ccagatctgc attttcgtat accaatgctc tgtccttcgc 3840

tggttgggga gtattggaat taaaccaaac gtggtaatcg gccatagcct tggagagatt 3900

tcggcttctg tggcggcagg ggcactttct cttgacattg ctctggatct tgtcatctca 3960

cgagctgggt tgttgcgccc ctcgacagat gttcctgcgg gaatggctgc tgtggccgct 4020

tcacaacagg aggtcattga gttgattgat gcgctggacc ccgacaaggc aaattcgctc 4080

agtgtttcgg ttataaatgg acctcaaaat atcgttgtgt caggcgcttc agcagctatt 4140

gagagaatgg tcgcttctgc gaaggagaag aagatcaaag cttctgttct gaatattagc 4200

caagcttttc acagttcgta tgttgacagt gccattacgg gtcttcgagc ttggtcagaa 4260

aaacatattt cctcagcgat accactgcag attccgctgt attcgacatt gctgggagct 4320

cggatatcaa agggccaaaa actgaaccca gaccactggg tcgaccacgc acggaagccc 4380

gtacagttcg cacaagcagc tacggcgatg aaagaaacct tcaccggagt catcatggat 4440

atcggacccc aagcagtagc atggtcactt ctgcttgcaa acggactcac atccgtaacc 4500

gcgcttgctg cgaagagagg gaggagtcag caggtggctt tcttgagcgc cttggcggag 4560

ttgtatcagg attatggcat tgttcctgat tttgtggcgc tttatgctca ggaggaggaa 4620

atagacaagt tgaggaagac ggatattttg acatatccgt ttcagcgtgt taggaggtat 4680

ccgagtttta taccttcaag gcgtgtgaga ggtggagaaa ttccttcgag ccatcccagc 4740

gagtctgcga cgttggagaa cacgaatgag ggtacggctt tgcgtgctga gtcgagggtg 4800

ttgtgcaggg aggatctgca tgatagcatc gttacctctg tgaaggatgt tctagagctc 4860

aaaccaaatg aagatctaga tttgtctgaa agcctgaacg cgcttggtgt ggactctata 4920

atgtttgctc agttaaggaa acgtattggg gagggactcg ggttgagtat cccgacagtg 4980

ttcctttcgg atgccttttc tattaatgag atggttaata atcttatgga acaggcggag 5040

acgcctggtg aagagggcgt aatgcaggag aat 5073

<210> 41
<211> 1691
<212> PRT
<213> Neonothopanus gardneri

<400> 41

Met Asn Ser Lys Val Asn Leu Pro Ser Thr Leu Leu Asp Val Phe Leu
1               5                   10                  15

Glu Ile Ala Gly Glu Pro Ser Thr Ala Ser Arg Asp Val Leu Glu Cys
            20                  25                  30

Gly Glu His Arg Trp Thr Tyr Gln Gln Leu Asp Val Val Ser Ser Ala
        35                  40                  45

Leu Ala Gln His Leu Lys Tyr Thr Val Gly Leu Ser Pro Thr Val Ala
    50                  55                  60

Val Ile Ser Glu Asn His Pro Tyr Val Phe Ala Leu Ile Leu Ala Val
65                  70                  75                  80

Trp Lys Val Gly Gly Ile Phe Ala Pro Leu Asp Ala His Ala Pro Ala
                85                  90                  95

Glu Leu Val Ala Gly Met Leu Ser Ile Ile Ser Pro Ser Cys Leu Val
            100                 105                 110

Leu Gln Ser Thr Asp Val Ala Asn Gln Thr Leu Ala Cys Asp Leu Asp
        115                 120                 125

Ile Pro Val Glu Val Phe His Ser Arg Gln Ser Thr Ile Pro Glu Leu
    130                 135                 140

Asn Lys Lys Tyr Leu Thr Asp Ser Gly Leu Pro Ala Gly Phe Pro Leu
145                 150                 155                 160

Ser Asp Ser Asn Lys Pro Ala Leu Tyr Leu Phe Thr Ser Ser Ala Thr
                165                 170                 175

Ser Arg Ser Asn Leu Lys Cys Val Pro Leu Thr His Ala Phe Ile Leu
            180                 185                 190

Ser Asn Ser Leu Ser Lys Arg Ala Trp Cys Gln Arg Met Arg Pro Glu
        195                 200                 205

Thr Asp Phe Asp Gly Ile Arg Val Leu Gly Trp Ala Pro Trp Ser His
    210                 215                 220

Val Leu Ala His Met Gln Asp Ile Gly Pro Val Thr Leu Leu Asn Ala
225                 230                 235                 240

Gly Cys Tyr Val Phe Ala Thr Ile Pro Ser Ser Tyr Pro Thr Asp Val
                245                 250                 255

Gln Ser Asp Ser Asn Leu Ile Ser His Val Ala Asn Ala Ile Ile His
            260                 265                 270

Lys Gly Val Lys Ser Phe Ala Cys Leu Pro Phe Val Leu Gly Gly Leu
        275                 280                 285

Lys Ala Leu Cys Glu Ser Lys Pro Ser Val Lys Ala Asp Leu Gln Val
    290                 295                 300

Glu Glu Gln Ala Gln Leu Leu Ile Ser Leu Arg Arg Met Glu Ile Leu
305                 310                 315                 320

Glu Cys Gly Gly Ala Met Leu Glu Ala Asn Val Ala Ser Trp Ala Ile
                325                 330                 335

Glu Asn His Ile Pro Val Ser Ile Gly Ile Gly Met Thr Glu Thr Gly
            340                 345                 350

Gly Ala Leu Phe Ala Gly Pro Val Gln Asp Ile Arg Thr Gly Phe Ser
        355                 360                 365

Ala Asp Asn Lys Phe Ile Lys Asp Ala Thr Tyr Phe Leu Val Ala Asn
    370                 375                 380

Gly Asp Glu Ser Gly Asn Asp Val Thr Glu Gly Glu Leu Val Val Lys
385                 390                 395                 400

Ser Lys Met Leu Pro Arg Gly Tyr Leu Gly Tyr Asn Asp Ser Ser Phe
                405                 410                 415

Ser Val Asp Asp Asp Gly Leu Val Thr Phe Lys Thr Gly Asp Arg Tyr
            420                 425                 430

Ser Val Thr Arg Asp Gly Arg Phe Ser Trp Leu Gly Arg Asn Thr Asp
        435                 440                 445

Phe Ile Gln Met Thr Ser Gly Glu Thr Leu Asp Pro Arg Pro Val Glu
    450                 455                 460

Ser Ser Leu Cys Gln Ser Pro Leu Ile Ser Gln Ala Cys Val Ile Gly
465                 470                 475                 480

Asp Lys Phe Leu Asn Gly Pro Ala Thr Ala Val Cys Ala Ile Val Glu
                485                 490                 495

Leu Glu Pro Thr Met Val Glu Thr Gly Glu Ala Asn Ser Arg Asp Ile
            500                 505                 510

Val Gln Val Phe Ala Pro Ile Asn Arg Asp Leu Pro Pro Pro Leu Arg
        515                 520                 525

Ile Ala Trp Ser His Ile Leu Ile Leu Lys His Ser Glu Lys Ile Pro
    530                 535                 540

Met Thr Lys Lys Gly Thr Ile Phe Arg Lys Lys Ile Glu Gln Met Phe
545                 550                 555                 560

Gly Ala Ala Leu Gly Ile Ala Ser Ile Pro Thr Asn Ser Val Asn Gly
                565                 570                 575

Ser Val Gly Ser Gly Glu Glu Pro Gln Ala Thr Ala Asp Glu Thr Val
            580                 585                 590

Leu Gln Asp Glu Leu Ser Asn Thr Val Lys Lys Ile Ile Ser Arg Val
        595                 600                 605

Leu Gly Val Thr Asp Glu Glu Leu Leu Trp Thr Leu Ser Phe Ala Glu
    610                 615                 620

Leu Gly Met Thr Ser Thr Leu Ala Ile Arg Ile Val Asn Glu Leu Asn
625                 630                 635                 640

Glu Thr Leu Val Gly Gly Asp Leu Pro Ile Asn Ala Cys Tyr Ile Tyr
                645                 650                 655

Val Asp Leu Ser Ser Leu Ser Lys Ala Val Tyr Ala Lys Leu Ala His
            660                 665                 670

Leu Glu Leu Ser Asp His Val Pro Glu Leu Lys Gln Ala Pro Phe Lys
        675                 680                 685

Ser Ser Gly Gly Lys Glu Ile Val Ile Val Gly Gln Ala Phe Arg Leu
    690                 695                 700

Pro Gly Ser Ile Asn Asp Val Ala Ser Leu Arg Asp Ala Phe Leu Ala
705                 710                 715                 720

Arg Gln Thr Ser Ser Ile Ile Ala Gly Ile Pro Ser Asp Arg Trp Asp
                725                 730                 735

His Ala Ser Phe Tyr Pro Lys Asp Ile Cys Phe Asp Lys Ala Gly Leu
            740                 745                 750

Val Asp Ile Ala His Tyr Asp His Ser Phe Phe Gly Ile Thr Ala Thr
        755                 760                 765

Glu Ala Leu His Leu Ser Pro Thr Met Arg Leu Ala Leu Glu Val Ser
    770                 775                 780

Phe Glu Ala Leu Glu Asn Ala Asn Ile Pro Thr Ser Arg Leu Lys Gly
785                 790                 795                 800

Ser Gln Thr Ala Val Tyr Val Ala Thr Thr Asp Asp Gly Phe Glu Thr
                805                 810                 815

Leu Leu Asn Ala Glu Ala Gly Tyr Glu Ala Tyr Thr Arg Phe Tyr Gly
            820                 825                 830

Thr Gly Arg Ala Ala Ser Thr Ala Ser Gly Arg Ile Ser Tyr Leu Leu
        835                 840                 845

Asp Ile His Gly Pro Ser Val Thr Ile Asp Thr Ala Cys Ser Gly Gly
    850                 855                 860

Val Val Cys Ile Asp Gln Ala Ile Asn Tyr Leu Gln Ser Ser Ser Ser
865                 870                 875                 880

Ala Asp Thr Ala Ile Val Phe Leu Ser Ala Gln Gly Met Val Ser Pro
                885                 890                 895

Arg Gly Arg Cys Ala Thr Phe Thr Ala Asp Ala Asp Gly Tyr Val Pro
            900                 905                 910

Ser Glu Gly Ala Val Ala Phe Val Leu Lys Thr Arg Glu Ala Ala Ile
        915                 920                 925

Arg Asp Lys Asp Asn Ile Leu Ala Thr Ile Lys Ala Thr Gln Ile Ser
    930                 935                 940

His Asn Gly Arg Ser Gln Gly Leu Val Ala Pro Asn Val Asn Ser Gln

945                    950                    955                    960

Val Asp Leu His Arg Ser Leu Leu Glu Lys Ala Arg Leu Ser Pro Ala
                965                    970                    975

Asp Val Gln Phe Ile Glu Ala His Gly Thr Gly Thr Ser Leu Gly Asp
            980                    985                    990

Leu Ser Glu Ile Gln Ala Ile Asn  Ala Ala Tyr Thr Ser  Ser Gln Pro
        995                    1000                    1005

Arg Thr  Thr Gly Pro Leu Ile  Val Ser Ala Ser Lys  Thr Val Val
    1010                    1015                    1020

Gly His  Thr Glu Pro Ala Gly  Pro Leu Val Gly Met  Leu Ser Val
    1025                    1030                    1035

Leu Leu  Ser Phe Gln Glu Gly  Ala Val Pro Gly Leu  Ala His Leu
    1040                    1045                    1050

Thr Thr  Arg Asn Leu Asn Pro  Thr Leu Asp Tyr Ser  Ser Val Pro
    1055                    1060                    1065

Leu Leu  Ile Pro Ser Glu Pro  Val Arg Leu Gln Thr  Pro Lys Pro
    1070                    1075                    1080

Tyr Arg  Ala Ala Val Met Ser  Tyr Gly Phe Ser Gly  Thr Leu Ala
    1085                    1090                    1095

Gly Leu  Val Leu Glu Ser Pro  Asp Glu Arg Ser Ser  Glu Glu Glu
    1100                    1105                    1110

Pro Pro  Asp Asp Lys Pro Met  Leu Phe Val Val Ser  Ala Lys Thr
    1115                    1120                    1125

His Thr  Ala Leu Ile Glu Tyr  Leu Gln Arg Tyr Leu  Glu Phe Leu
    1130                    1135                    1140

Leu His  Ala Asn Pro Arg Asp  Phe Cys Asp Ile Cys  Tyr Thr Ser
    1145                    1150                    1155

Cys Val  Gly Arg Glu His Tyr  Arg Tyr Arg Phe Ala  Cys Val Ala
    1160                    1165                    1170

Asn Asp  Met Glu Asp Leu Ile  Gly Gln Leu Gln Arg  Arg Leu Ser
    1175                    1180                    1185

Ser Lys Leu Pro Ser Lys Pro Leu Tyr Lys Arg Gly Ala Leu Ala
    1190                1195                1200

Phe Ala Phe Ser Gly Gln Gly Thr Gln Phe Gln Gly Met Ala Thr
    1205                1210                1215

Asp Leu Ala Lys Arg Tyr Ser Gly Phe Arg Lys Ile Val Ser Gly
    1220                1225                1230

Leu Ala Lys Ser Ala Gly Glu Leu Ser Gly Tyr Ala Ile Asp Arg
    1235                1240                1245

Tyr Leu Leu Ala Tyr Asp Val Gly Ser Ser Ile Ala Thr Pro Asn
    1250                1255                1260

Ser Glu Val Asp Gln Ile Cys Ile Phe Val Tyr Gln Cys Ser Val
    1265                1270                1275

Leu Arg Trp Leu Gly Ser Ile Gly Ile Lys Pro Asn Val Val Ile
    1280                1285                1290

Gly His Ser Leu Gly Glu Ile Ser Ala Ser Val Ala Ala Gly Ala
    1295                1300                1305

Leu Ser Leu Asp Ile Ala Leu Asp Leu Val Ile Ser Arg Ala Gly
    1310                1315                1320

Leu Leu Arg Pro Ser Thr Asp Val Pro Ala Gly Met Ala Ala Val
    1325                1330                1335

Ala Ala Ser Gln Gln Glu Val Ile Glu Leu Ile Asp Ala Leu Asp
    1340                1345                1350

Pro Asp Lys Ala Asn Ser Leu Ser Val Ser Val Ile Asn Gly Pro
    1355                1360                1365

Gln Asn Ile Val Val Ser Gly Ala Ser Ala Ala Ile Glu Arg Met
    1370                1375                1380

Val Ala Ser Ala Lys Glu Lys Lys Ile Lys Ala Ser Val Leu Asn
    1385                1390                1395

Ile Ser Gln Ala Phe His Ser Ser Tyr Val Asp Ser Ala Ile Thr
    1400                1405                1410

Gly Leu Arg Ala Trp Ser Glu Lys His Ile Ser Ser Ala Ile Pro
    1415                1420                1425

Leu Gln Ile Pro Leu Tyr Ser Thr Leu Leu Gly Ala Arg Ile Ser
    1430                1435                1440

Lys Gly Gln Lys Leu Asn Pro Asp His Trp Val Asp His Ala Arg
    1445                1450                1455

Lys Pro Val Gln Phe Ala Gln Ala Ala Thr Ala Met Lys Glu Thr
    1460                1465                1470

Phe Thr Gly Val Ile Met Asp Ile Gly Pro Gln Ala Val Ala Trp
    1475                1480                1485

Ser Leu Leu Leu Ala Asn Gly Leu Thr Ser Val Thr Ala Leu Ala
    1490                1495                1500

Ala Lys Arg Gly Arg Ser Gln Gln Val Ala Phe Leu Ser Ala Leu
    1505                1510                1515

Ala Glu Leu Tyr Gln Asp Tyr Gly Ile Val Pro Asp Phe Val Ala
    1520                1525                1530

Leu Tyr Ala Gln Glu Glu Glu Ile Asp Lys Leu Arg Lys Thr Asp
    1535                1540                1545

Ile Leu Thr Tyr Pro Phe Gln Arg Val Arg Arg Tyr Pro Ser Phe
    1550                1555                1560

Ile Pro Ser Arg Arg Val Arg Gly Gly Glu Ile Pro Ser Ser His
    1565                1570                1575

Pro Ser Glu Ser Ala Thr Leu Glu Asn Thr Asn Glu Gly Thr Ala
    1580                1585                1590

Leu Arg Ala Glu Ser Arg Val Leu Cys Arg Glu Asp Leu His Asp
    1595                1600                1605

Ser Ile Val Thr Ser Val Lys Asp Val Leu Glu Leu Lys Pro Asn
    1610                1615                1620

Glu Asp Leu Asp Leu Ser Glu Ser Leu Asn Ala Leu Gly Val Asp
    1625                1630                1635

Ser Ile Met Phe Ala Gln Leu Arg Lys Arg Ile Gly Glu Gly Leu
    1640                1645                1650

Gly Leu Ser Ile Pro Thr Val Phe Leu Ser Asp Ala Phe Ser Ile
    1655                1660                1665

Asn Glu Met Val Asn Asn Leu Met Glu Gln Ala Glu Thr Pro Gly
    1670                1675                1680

Glu Glu Gly Val Met Gln Glu Asn
    1685                1690

<210> 42
<211> 5298
<212> DNA
<213> Guyanagaster necrorhiza

<400> 42
atggcggccg ataggcactt ttctcttctc gatgtctttc tcgacgttgc ccataatgct 60

gagacgtcac aacgcaatat tttggaatgc ggcctggaca cctggacata ctcagatttg 120

gacatcatct cgtcggccct ggcccaggat ctcagtacta ccttgggttg ctctcccagg 180

gttgcagtcg tcagcgagaa ccatccctat gtatttgctc tcatgctggc cgtatggaag 240

cttggaggaa tattcatccc catcgacgtc catgttcccg ccgagctttt aacgggcatg 300

ctacgcatcg ttgctccaga ctgcgtggtg attcctgaga ctgatctttc taatcagcgc 360

gtcatctctg cactttacct ccacgttatt cccttcaatg tcaatgcgtc gacaatgtct 420

acacttcgac agaaatatgt cctatctact cagaaaccct tgctgtctga gttccctctt 480

cctcatgttg atcgtgcatg cctctatctc tttacgtcct ccgcgtcttc caccgccaac 540

ttgaaatgcg tacctttgac tcatgcactt atcctcagca actgtcgttc caagcttgca 600

tggtggcggc gtgttcgtcc agaagaaaac atggatggga tgcgtgtttt ggggtgggcg 660

ccttggtcac atattctcgc gtacatgcag gatattggga cagcaacgct cctgaatgca 720

ggctgctatg tctttgcatc tgttccgtcc acatatccta cgcagcacgt agccaatggc 780

ctgcaagacc ccacttcaaa tataatcaag tcgcttctca accgccgtat cacggcgttc 840

gcatgtgtgc cgttcattct tagtgaactg aaagctatgt gcgacccagc ttccggtccg 900

gacgccaagg atcaaatgtg cttgggagct ggggagaaag tgcgtcttat aagcacactg 960

cagaatctca tcatgttcga gtgtggaggc gctgcgctcg aggcagatat cacggattgg 1020

accgtcgaaa atggtatatc agtcatggtc ggtattggaa tgacggagac cgccggcacg 1080

gttttggcag cgcgtgcaca agacgctcgt tcgaatggct attctgccga agaggctctc 1140

atcgctgatg gcatactatc attggtcgga cctgacgagg aggaaatggc ctttgacgga 1200

gaacttgtcg tgaaaagcaa gctcatccca catggataca tcaagtacca cgattcggca 1260

ttttcagtgg actcagatga ctgggtgacg ttcaaaactg gggacaaata tcagcgtaca 1320

ccagatggac atttcaagtg gcttggaaga aagaccgatt ttattcagat gacaagcagc 1380

gaaaccttag atcccagacc tatcgaaaaa gccctctgca tgaaccccat tatcgcaaat 1440

gcgtgtgtca ttggtgacag gtttctaagg gaacctgcga cgggtgtatg tgccattgtc 1500

gagatcaggt cagatgtgga tatggcttcc gccgaggttg acagggagat tgcgaatgtc 1560

ctcgctccaa tcaatcgtga tcttcctccg gctcttcgaa tagcatggtc tcgcgtactc 1620

ataatccgac ctcctcagaa aataccactc acaaagaaag gtgaggtgtt tcgcaagaag 1680

attgaagata tatttggcac cttcttgggt gtcggtgtta ctaccaaggt ggaagtggac 1740

catgaaagta aagaagatga tacggaacac atcgtgagac aggttgtgag caatcttctt 1800

ggagtccatg atcctgagct attgtctact ttatctttcg ctgagctcgg gatgacctca 1860

tttatggccg ttagcatcgt taacacttta aacaagcata ttggcggcct cgcacttcca 1920

cttaactcat gctacattca tattgatctt gattctcttg tagatgccat ttcacttgaa 1980

cgtggtcatc gaaagaaccc tacgcccttt tcttctaacc ctttcctcgc ctttgaatcc 2040

agccagcaaa aggatatgga gattgtgatt gttggtaaag cattccgttt gcccggctca 2100

ctcgacaata gcacctctct ctgggaagct ttgttgtcaa agagtgattc agtcatcggt 2160

gacatcccac ccgatcgctg ggatcatgca agtttttacc cccacgatat atgctttacg 2220

aaagcaggcc ttgtcgatgt ttcccattat gactatagat ttttcggtct cactgctaca 2280

gaggcgttgt acgtatctcc gacaatgcgc ctcgccttgg aagtgtcatt tgaggctctg 2340

gagaatgcaa atattccctt atccaagttg aaggggacac gaactgctgt ttatgttgcc 2400

actaaagacg atgggttcga gacactctta aatgctgaac aaggctacga tgcctacacg 2460

cgattctacg gcacgggacg tgctccgagt accgcaagtg gccgtataag ctatcttctt 2520

gatattcatg gaccttctgt taccgttgac acagcatgca gcggaggcat tgtatgtata 2580

gaccaagcca tcacttttct gcaatccgga ggggcagata ctgctattgt ttgttcgagc 2640

aatacgcact gttggccggg atcatttatg ttcctgacag cgcaaggcat ggtctctcca 2700

aatggaagat gtgccacatt cactacaaat gcagatggat atgtaccttc agaaggcgca 2760

gtggctttcg ttctcaagac acacagcgca gcaacacgcg acaaagacaa tatactggcc 2820

gtgataaagt caacagacgt gtctcataac ggccgttctc aagggctagt tgcaccaaac 2880

gtaaaggcac agacaaacct acaccagtcg ctgttacgaa aagctgggct gcatcctgat 2940

ctaattaact ttatcgaagc tcatggaaca ggtacatctt taggagacct ttcagaaatc 3000

cagggtatca ataatgccta cacctcatct cgacctcgtc cagccggtcc tctgatcatt 3060

agcgcgtcaa aaacggtttt gggacatagt gaaccaactg caggaatggc cggcatcctc 3120

acaaccttgc tcgcctttga gaaagagacg gttcctggtc tcaaccactt aacggaacat 3180

aatttaaacc cttcgcttga ttgctctgca gtgcctctcc tgattcctca cgagcctgtt 3240

catatcagtg gtgcaaagcc ccatcgagct gcggttctat catacggatt cgcggggaca 3300

ctggctggta ccatcttaga gagtccacct tgcgacctta caaggccctt gtcaaacgac 3360

atacaagaac atcctatgat tttcgtcgtc agtgggaaaa cggtgccctc tctggaagca 3420

tatctagagc ggtatttggt atttttacgg gtcgcaaaac caagcgaatt ccatgacatc 3480

tgctacacca cttgcatcgg gagggagctg tataaatacc ggttctcctg cgttgcccga 3540

aacatggccg acctcatttc tcaaattgaa catcgactga cgacttgttc cacttcgaaa 3600

gagaagcccc gtggctcgtt aggattcgtg ttctcgggtc aaggtaccca gttccccggc 3660

atggcagcag cacttgccga acgatattca gggttccgag cgctcgtctc caagtttggg 3720

cagatcgccc aagagcagtc gggctacccg atcgataggc tgctcctcga agttaccgat 3780

acattaccag aagcaaacag cgaggtcgac caaatttgca tttttgtcta ccaatattct 3840

gttctgcaat ggctgcaacg tctaggcatt caaccgaaaa cagtccttgg tcacagtcta 3900

ggagaaatta ccgcctcagt cgcagttggc gccttctctt ttaggtctgc attggacctt 3960

gtggtcaccc gcgctcgtct tcttcgtcct caaccaaaat tctctgcggg aatggctgca 4020

gtagcagcgt ccaaggaaga agttgaagga cttatagata tgctcaaact tgcggagtcg 4080

ctgagcgttg cggttcataa cagtccacgg agtatcgttg tatcaggcgc atcagctgcg 4140

gtcgatgcca tggttgtcgc tgctaaaaag cagggcttga aggcctcccg cttaaaggtt 4200

gaccaagcct ttcacagccc ttacgttgat tctgctgtat cggggttgct cgactggtcc 4260

aataagcatc gttcgacctt cctcccattg aacatccctt tatactcaac tttgactggc 4320

gcgcgtattc cgaaaggagg gaagttctgc tgggatcact gggttaatca tgctcgaaag 4380

cccgtccagt ttgcggcggc agctgcagca atggacgaag atcaatccat cggtgttctt 4440

gttgatgttg gaccccaacc tgttgcgtgg accctccttc aagcgaataa cctcctcaat 4500

acctcttcga ttgctctatc agcaaaaatt ggaaaggatc aggagatggc gttgctctct 4560

gctttgagct acctcgtcca agagcacaac ctttctctca gcttttatga gctttactct 4620

cagcgtcacg gtactctgaa gaagacagac gttcctacct acccgttccg tcgcgtccac 4680

cgctacccga ctttcatacc gtcacggaat agaagtcctg ctgacatgag ggtagctata 4740

ccacctaccg acctctctgt ccgaaagaat gtggatgcaa caccgcagtc tcgtcgtgct 4800

ggcctgatag cctgtcttaa agttatcctc gagttaacac caggagagga atttgacctt 4860

tctgagactc tcaatgctcg tggggtcgat tcagttatgt tcgcacagtt gcggaagcgt 4920

gttggggaag aattcgacct agatatacca atgatctatt tatcagacgt gttcacgatg 4980

gaacagatga ttgactacct cgtcgaacag tccagcccca catcaaagtt ggtagagatt 5040

tcggttaatc aatcattaga cggagaaggc ctccggacag ggcttgtatc atgccttagg 5100

gacgtactgg aaatctcctc cgacgaagaa cttgactttt ccgaaacttt gaacgctcgt 5160

ggtacggact caattatgtt cgctcagcta cgaaaacgtg tcggggaagg atttggtctt 5220

gaaattccga tgatatatct gtctgatgtg tttaccatgg aagacatgat caatttcctt     5280

gtctcggagc gctcgtgc                                                  5298

<210>  43
<211>  1766
<212>  PRT
<213>  Guyanagaster necrorhiza

<400>  43

Met Ala Ala Asp Arg His Phe Ser Leu Leu Asp Val Phe Leu Asp Val
1               5                   10                  15

Ala His Asn Ala Glu Thr Ser Gln Arg Asn Ile Leu Glu Cys Gly Leu
            20                  25                  30

Asp Thr Trp Thr Tyr Ser Asp Leu Asp Ile Ile Ser Ser Ala Leu Ala
        35                  40                  45

Gln Asp Leu Ser Thr Thr Leu Gly Cys Ser Pro Arg Val Ala Val Val
    50                  55                  60

Ser Glu Asn His Pro Tyr Val Phe Ala Leu Met Leu Ala Val Trp Lys
65                  70                  75                  80

Leu Gly Gly Ile Phe Ile Pro Ile Asp Val His Val Pro Ala Glu Leu
                85                  90                  95

Leu Thr Gly Met Leu Arg Ile Val Ala Pro Asp Cys Val Val Ile Pro
            100                 105                 110

Glu Thr Asp Leu Ser Asn Gln Arg Val Ile Ser Ala Leu Tyr Leu His
        115                 120                 125

Val Ile Pro Phe Asn Val Asn Ala Ser Thr Met Ser Thr Leu Arg Gln
    130                 135                 140

Lys Tyr Val Leu Ser Thr Gln Lys Pro Leu Leu Ser Glu Phe Pro Leu
145                 150                 155                 160

Pro His Val Asp Arg Ala Cys Leu Tyr Leu Phe Thr Ser Ser Ala Ser
                165                 170                 175

Ser Thr Ala Asn Leu Lys Cys Val Pro Leu Thr His Ala Leu Ile Leu
            180                 185                 190

Ser Asn Cys Arg Ser Lys Leu Ala Trp Trp Arg Arg Val Arg Pro Glu
        195                 200                 205

Glu Asn Met Asp Gly Met Arg Val Leu Gly Trp Ala Pro Trp Ser His
    210                 215                 220

Ile Leu Ala Tyr Met Gln Asp Ile Gly Thr Ala Thr Leu Leu Asn Ala
225                 230                 235                 240

Gly Cys Tyr Val Phe Ala Ser Val Pro Ser Thr Tyr Pro Thr Gln His
                245                 250                 255

Val Ala Asn Gly Leu Gln Asp Pro Thr Ser Asn Ile Ile Lys Ser Leu
            260                 265                 270

Leu Asn Arg Arg Ile Thr Ala Phe Ala Cys Val Pro Phe Ile Leu Ser
        275                 280                 285

Glu Leu Lys Ala Met Cys Asp Pro Ala Ser Gly Pro Asp Ala Lys Asp
    290                 295                 300

Gln Met Cys Leu Gly Ala Gly Glu Lys Val Arg Leu Ile Ser Thr Leu
305                 310                 315                 320

Gln Asn Leu Ile Met Phe Glu Cys Gly Gly Ala Ala Leu Glu Ala Asp
                325                 330                 335

Ile Thr Asp Trp Thr Val Glu Asn Gly Ile Ser Val Met Val Gly Ile
            340                 345                 350

Gly Met Thr Glu Thr Ala Gly Thr Val Leu Ala Ala Arg Ala Gln Asp
        355                 360                 365

Ala Arg Ser Asn Gly Tyr Ser Ala Glu Glu Ala Leu Ile Ala Asp Gly
    370                 375                 380

Ile Leu Ser Leu Val Gly Pro Asp Glu Glu Glu Met Ala Phe Asp Gly
385                 390                 395                 400

Glu Leu Val Val Lys Ser Lys Leu Ile Pro His Gly Tyr Ile Lys Tyr
                405                 410                 415

His Asp Ser Ala Phe Ser Val Asp Ser Asp Asp Trp Val Thr Phe Lys
            420                 425                 430

Thr Gly Asp Lys Tyr Gln Arg Thr Pro Asp Gly His Phe Lys Trp Leu
        435                 440                 445

Gly Arg Lys Thr Asp Phe Ile Gln Met Thr Ser Ser Glu Thr Leu Asp

450                 455                 460

Pro Arg Pro Ile Glu Lys Ala Leu Cys Met Asn Pro Ile Ile Ala Asn
465                 470                 475                 480

Ala Cys Val Ile Gly Asp Arg Phe Leu Arg Glu Pro Ala Thr Gly Val
                485                 490                 495

Cys Ala Ile Val Glu Ile Arg Ser Asp Val Asp Met Ala Ser Ala Glu
            500                 505                 510

Val Asp Arg Glu Ile Ala Asn Val Leu Ala Pro Ile Asn Arg Asp Leu
        515                 520                 525

Pro Pro Ala Leu Arg Ile Ala Trp Ser Arg Val Leu Ile Ile Arg Pro
    530                 535                 540

Pro Gln Lys Ile Pro Leu Thr Lys Lys Gly Glu Val Phe Arg Lys Lys
545                 550                 555                 560

Ile Glu Asp Ile Phe Gly Thr Phe Leu Gly Val Gly Val Thr Thr Lys
                565                 570                 575

Val Glu Val Asp His Glu Ser Lys Glu Asp Asp Thr Glu His Ile Val
            580                 585                 590

Arg Gln Val Val Ser Asn Leu Leu Gly Val His Asp Pro Glu Leu Leu
        595                 600                 605

Ser Thr Leu Ser Phe Ala Glu Leu Gly Met Thr Ser Phe Met Ala Val
    610                 615                 620

Ser Ile Val Asn Thr Leu Asn Lys His Ile Gly Gly Leu Ala Leu Pro
625                 630                 635                 640

Leu Asn Ser Cys Tyr Ile His Ile Asp Leu Asp Ser Leu Val Asp Ala
                645                 650                 655

Ile Ser Leu Glu Arg Gly His Arg Lys Asn Pro Thr Pro Phe Ser Ser
            660                 665                 670

Asn Pro Phe Leu Ala Phe Glu Ser Ser Gln Gln Lys Asp Met Glu Ile
        675                 680                 685

Val Ile Val Gly Lys Ala Phe Arg Leu Pro Gly Ser Leu Asp Asn Ser
    690                 695                 700

Thr Ser Leu Trp Glu Ala Leu Leu Ser Lys Ser Asp Ser Val Ile Gly
705                 710                 715                 720

Asp Ile Pro Pro Asp Arg Trp Asp His Ala Ser Phe Tyr Pro His Asp
                725                 730                 735

Ile Cys Phe Thr Lys Ala Gly Leu Val Asp Val Ser His Tyr Asp Tyr
            740                 745                 750

Arg Phe Phe Gly Leu Thr Ala Thr Glu Ala Leu Tyr Val Ser Pro Thr
        755                 760                 765

Met Arg Leu Ala Leu Glu Val Ser Phe Glu Ala Leu Glu Asn Ala Asn
    770                 775                 780

Ile Pro Leu Ser Lys Leu Lys Gly Thr Arg Thr Ala Val Tyr Val Ala
785                 790                 795                 800

Thr Lys Asp Asp Gly Phe Glu Thr Leu Leu Asn Ala Glu Gln Gly Tyr
                805                 810                 815

Asp Ala Tyr Thr Arg Phe Tyr Gly Thr Gly Arg Ala Pro Ser Thr Ala
            820                 825                 830

Ser Gly Arg Ile Ser Tyr Leu Leu Asp Ile His Gly Pro Ser Val Thr
        835                 840                 845

Val Asp Thr Ala Cys Ser Gly Gly Ile Val Cys Ile Asp Gln Ala Ile
    850                 855                 860

Thr Phe Leu Gln Ser Gly Gly Ala Asp Thr Ala Ile Val Cys Ser Ser
865                 870                 875                 880

Asn Thr His Cys Trp Pro Gly Ser Phe Met Phe Leu Thr Ala Gln Gly
                885                 890                 895

Met Val Ser Pro Asn Gly Arg Cys Ala Thr Phe Thr Thr Asn Ala Asp
            900                 905                 910

Gly Tyr Val Pro Ser Glu Gly Ala Val Ala Phe Val Leu Lys Thr His
        915                 920                 925

Ser Ala Ala Thr Arg Asp Lys Asp Asn Ile Leu Ala Val Ile Lys Ser
    930                 935                 940

Thr Asp Val Ser His Asn Gly Arg Ser Gln Gly Leu Val Ala Pro Asn
945                 950                 955                 960

Val Lys Ala Gln Thr Asn Leu His Gln Ser Leu Leu Arg Lys Ala Gly
                965                 970                 975

Leu His Pro Asp Leu Ile Asn Phe Ile Glu Ala His Gly Thr Gly Thr
            980                 985                 990

Ser Leu Gly Asp Leu Ser Glu Ile Gln Gly Ile Asn Asn Ala Tyr Thr
        995                 1000                1005

Ser Ser Arg Pro Arg Pro Ala Gly Pro Leu Ile Ile Ser Ala Ser
    1010                1015                1020

Lys Thr Val Leu Gly His Ser Glu Pro Thr Ala Gly Met Ala Gly
    1025                1030                1035

Ile Leu Thr Thr Leu Leu Ala Phe Glu Lys Glu Thr Val Pro Gly
    1040                1045                1050

Leu Asn His Leu Thr Glu His Asn Leu Asn Pro Ser Leu Asp Cys
    1055                1060                1065

Ser Ala Val Pro Leu Leu Ile Pro His Glu Pro Val His Ile Ser
    1070                1075                1080

Gly Ala Lys Pro His Arg Ala Ala Val Leu Ser Tyr Gly Phe Ala
    1085                1090                1095

Gly Thr Leu Ala Gly Thr Ile Leu Glu Ser Pro Pro Cys Asp Leu
    1100                1105                1110

Thr Arg Pro Leu Ser Asn Asp Ile Gln Glu His Pro Met Ile Phe
    1115                1120                1125

Val Val Ser Gly Lys Thr Val Pro Ser Leu Glu Ala Tyr Leu Glu
    1130                1135                1140

Arg Tyr Leu Val Phe Leu Arg Val Ala Lys Pro Ser Glu Phe His
    1145                1150                1155

Asp Ile Cys Tyr Thr Thr Cys Ile Gly Arg Glu Leu Tyr Lys Tyr
    1160                1165                1170

Arg Phe Ser Cys Val Ala Arg Asn Met Ala Asp Leu Ile Ser Gln
    1175                1180                1185

Ile Glu His Arg Leu Thr Thr Cys Ser Thr Ser Lys Glu Lys Pro
    1190                1195                1200

Arg Gly Ser Leu Gly Phe Val Phe Ser Gly Gln Gly Thr Gln Phe
    1205                1210                1215

Pro Gly Met Ala Ala Ala Leu Ala Glu Arg Tyr Ser Gly Phe Arg
    1220                1225                1230

Ala Leu Val Ser Lys Phe Gly Gln Ile Ala Gln Glu Gln Ser Gly
    1235                1240                1245

Tyr Pro Ile Asp Arg Leu Leu Leu Glu Val Thr Asp Thr Leu Pro
    1250                1255                1260

Glu Ala Asn Ser Glu Val Asp Gln Ile Cys Ile Phe Val Tyr Gln
    1265                1270                1275

Tyr Ser Val Leu Gln Trp Leu Gln Arg Leu Gly Ile Gln Pro Lys
    1280                1285                1290

Thr Val Leu Gly His Ser Leu Gly Glu Ile Thr Ala Ser Val Ala
    1295                1300                1305

Val Gly Ala Phe Ser Phe Arg Ser Ala Leu Asp Leu Val Val Thr
    1310                1315                1320

Arg Ala Arg Leu Leu Arg Pro Gln Pro Lys Phe Ser Ala Gly Met
    1325                1330                1335

Ala Ala Val Ala Ala Ser Lys Glu Glu Val Glu Gly Leu Ile Asp
    1340                1345                1350

Met Leu Lys Leu Ala Glu Ser Leu Ser Val Ala Val His Asn Ser
    1355                1360                1365

Pro Arg Ser Ile Val Val Ser Gly Ala Ser Ala Ala Val Asp Ala
    1370                1375                1380

Met Val Val Ala Ala Lys Lys Gln Gly Leu Lys Ala Ser Arg Leu
    1385                1390                1395

Lys Val Asp Gln Ala Phe His Ser Pro Tyr Val Asp Ser Ala Val
    1400                1405                1410

Ser Gly Leu Leu Asp Trp Ser Asn Lys His Arg Ser Thr Phe Leu
    1415                1420                1425

Pro Leu Asn Ile Pro Leu Tyr Ser Thr Leu Thr Gly Ala Arg Ile

1430                1435                1440

Pro Lys Gly Gly Lys Phe Cys Trp Asp His Trp Val Asn His Ala
    1445                1450                1455

Arg Lys Pro Val Gln Phe Ala Ala Ala Ala Ala Ala Met Asp Glu
    1460                1465                1470

Asp Gln Ser Ile Gly Val Leu Val Asp Val Gly Pro Gln Pro Val
    1475                1480                1485

Ala Trp Thr Leu Leu Gln Ala Asn Asn Leu Leu Asn Thr Ser Ser
    1490                1495                1500

Ile Ala Leu Ser Ala Lys Ile Gly Lys Asp Gln Glu Met Ala Leu
    1505                1510                1515

Leu Ser Ala Leu Ser Tyr Leu Val Gln Glu His Asn Leu Ser Leu
    1520                1525                1530

Ser Phe Tyr Glu Leu Tyr Ser Gln Arg His Gly Thr Leu Lys Lys
    1535                1540                1545

Thr Asp Val Pro Thr Tyr Pro Phe Arg Arg Val His Arg Tyr Pro
    1550                1555                1560

Thr Phe Ile Pro Ser Arg Asn Arg Ser Pro Ala Asp Met Arg Val
    1565                1570                1575

Ala Ile Pro Pro Thr Asp Leu Ser Val Arg Lys Asn Val Asp Ala
    1580                1585                1590

Thr Pro Gln Ser Arg Arg Ala Gly Leu Ile Ala Cys Leu Lys Val
    1595                1600                1605

Ile Leu Glu Leu Thr Pro Gly Glu Glu Phe Asp Leu Ser Glu Thr
    1610                1615                1620

Leu Asn Ala Arg Gly Val Asp Ser Val Met Phe Ala Gln Leu Arg
    1625                1630                1635

Lys Arg Val Gly Glu Glu Phe Asp Leu Asp Ile Pro Met Ile Tyr
    1640                1645                1650

Leu Ser Asp Val Phe Thr Met Glu Gln Met Ile Asp Tyr Leu Val
    1655                1660                1665

Glu Gln Ser Ser Pro Thr Ser Lys Leu Val Glu Ile Ser Val Asn
    1670                1675                1680

Gln Ser Leu Asp Gly Glu Gly Leu Arg Thr Gly Leu Val Ser Cys
    1685                1690                1695

Leu Arg Asp Val Leu Glu Ile Ser Ser Asp Glu Glu Leu Asp Phe
    1700                1705                1710

Ser Glu Thr Leu Asn Ala Arg Gly Thr Asp Ser Ile Met Phe Ala
    1715                1720                1725

Gln Leu Arg Lys Arg Val Gly Glu Gly Phe Gly Leu Glu Ile Pro
    1730                1735                1740

Met Ile Tyr Leu Ser Asp Val Phe Thr Met Glu Asp Met Ile Asn
    1745                1750                1755

Phe Leu Val Ser Glu Arg Ser Cys
    1760                1765

<210> 44
<211> 5034
<212> DNA
<213> Mycena citricolor

<400> 44
atgtccgcct cttcctccta ttctgaactg gagtctccga cgtcgctgct tgacgtattc 60

gtgcacgccg cgcgagaccc ccacaccgcg tcgcgtcgcg tgctggagtg cggctcggac 120

acatggacat acgcggcctt ggatgcggta tccgatggca tagcgaggga gctcgcgccc 180

ttcggtctgg cgcccaaggt cgctgtggtc agcgagaacc atccatttgt gttcgcgctg 240

ctgttcgcag tctggaagct cgggggaacg ttcatcccca tcgacgcgca tgtccccttt 300

gccatgctca cggggatggt gaacatcgtg aagccgacgt gtctctacct ccccgcctcc 360

gccacatcca acatatctct cgtgaaggcc ttcgacatac ggaccgtcgt atttgggcac 420

aaggagaatt ccatgcaagc tttgttcgac aagtactcat tgcacgcggc gccgttgcac 480

gcggcaccat tgcattacgc gcctcccagt gccgaccatg cctgtctgta tctcttcacg 540

tcgtcagcgt cctcgaccaa aaacctcaag gcggtcccgc tgacgcatac actcgtcctg 600

cgggggtgtc aatccaaact cgcttggtgg cgccgagtcc aacccggaaa gaatctcgac 660

gctatccgga tcctcggctg ggcaccctgg tctcacgttc tcgcctatat gcaggatatc 720

gggacagcga cactgctcaa cgcgggctgc tacgtctttg cgaccatccc ttcgtcctac 780

ccgcagctgc ctaccactac ccccgtcgat ctgacgactt cgctcatcga cgcgctcgtc 840

acgcggcgcg tcgcggcatt cgcctgtgtc cccttcgtcc tggcgaacct caaagccgcg 900

tgccagagca accaccccgc gcgctcccag ctgctcgagg cgctgcagaa gacgatcatg 960

ctcgagtgcg gtggggcggt gctggacgac gcgacggtgg actgggccga gcgcaacggc 1020

atccgcatct tcacgggaat cgggatgacc gagactggcg gggccgtttt cgtcggcctc 1080

gcgagcgaat ctagacgcgg gtttctaccg gaaggcctac tcggtgatgc atccttctcg 1140

atctcgagtg acacggacgc tcttgacgaa ggggaacttg tcgtgaaaag taaactcatc 1200

gcgcccggat atgtcggcta cgacgacgga gcgcactccg tggactgcga cggctgggtc 1260

acattcagga caggagatcg atatcgccag acccagccag acgggcgctt tacgtggcta 1320

ggaaggatca cagacttcat tcagatggtc agcggcgagt acctcgatcc gcggcccctc 1380

gaggagagtc tgcgtgcgtc cccgctgatc gccaacgcct gcatcgtggg cgacgcgttc 1440

ctcagcagcg cctcgacgag catcctcgcc atcatcgagc tcgcgacccc ggatctggcg 1500

cacacggcct ccatccgggc gcagctcgcg cgcgtcctcg cgcccctcaa ccgcgatctg 1560

cctccaccgc ttcggatcgc agcgtcttcg atcctggttc tggacgggat gcggaagatc 1620

ccgaagacga agaagggcga catcttccgc aaaaagctgg aagacacgtt cggtgcggag 1680

tttgagcaga tgctgcggac cgagaaagtc gggctgggtg acttggcgga tgtggatgcc 1740

ggtatcactc gtatcatcgg caacttgctc ggcatctccg acgacgagct tctgtcgacc 1800

atgtcgtttg ctgagctcgg gatgacctcg cttctcgccg tcaagatcgc gagcgaacta 1860

aacaagtttc tggacggccg ggctgtgctg cccacgaaca tctgttacat acactttgac 1920

gtgccctcgc tcacgagtag cgtccgagaa aggctatcct ccgcgccctc ctcgatcaca 1980

tccgccgcca gcgagcccgc ggcctcgtcc cccggcaccc cccgcgccga cgaaatcgtg 2040

atcgtgggca aggccttccg gctgccggac ggcgtgaacg acgacgccgc gctgtgggac 2100

gtcctgacgg gcgagtccgc gtcgatcatc aaggacatcc ccgccgaccg ctgggaccac 2160

gcgagcttct acccgaagga catccacttt ggcagagctg gtctggtgga tgtcgcgcgg 2220

ttcgactacg gctttttcgg catgacggcg agcgaggcgt attcgctgtc gccgacgatg 2280

cgcttggcgc tcgaagtcgc gtatgaggcg ttagaggacg cgaacattcc gttccgggcc 2340

gtcaagggct cgcgcatggg tgtgttcgtc gctgtgaaag acgatggatt cgagaccctg 2400

ttgcatgcgg ggcagggtta tgacgcttac acgcggttct atggaactgg aagggcgccg 2460

agcaccgcca gcggccgcat caactatctg ctcgatctcc acgggccgtc gatcaccgtc 2520

gacacggcct gcagcggcgg catcgtgtgc atcgaccagg ctgtcaccta tcttcaatcg 2580

ggcgctgcag agacggcgat cgtgtgttcg agcaacacac actgctggcc cggatccttc 2640

atgttcttga ccgcgcaagg catggcctcc cccaacggcc ggtgcgcatc cttcacatca 2700

gacgccgacg gatacgcgcc gtcggagggc gcggtgggct tcgtgctgaa gacgcggtct 2760

gccgctgtgc gcgacggcga tcggatactg gctacgatca gggccacgga gatcggacac 2820

aatggacggt ctcagggact cgctgcgccg aatgtcaggt cccaggcggc tgctcatcgg 2880

gcggtgctgc ggagagcgag gctggacccg tccgagattg acttcatcga agcgcatggg 2940

actggcacca cattgggcga tctgtgcgaa gtgcagggca tcaacgacag ctttgtctcg 3000

cccaagaaac gcgccaaccc tcttgtggtc agcgcgtcca agagcaccat cggccacacc 3060

gagccgtcgg cgggtctcgt cgggatcctg tccgcgctga tgtcgttcga gaagcgcatc 3120

gtgccgagat tggcatatct gactgagagc aatgtgaacc cggcgctcga cgcgagtgtc 3180

gttccgctgc actttcccac aaagcacatc gagctgcgcg ctgatgtgcc gtacaaggcc 3240

gtagtgatgt cgtacggatt cgcaggtacc ctagccgaca tcgtcctcga gagcgaggta 3300

ccccaaccca cgcccgcggt cgctcaggac acggccggcc aacagccaat gctcttcgtg 3360

ctcagcgcca aaaccccacg cgcactcgca gcctacatcg agctgtacct aggcttcctg 3420

cggcacgcgg acccgggcct cttcgcgcgc atctgctaca cggcgtgtgt cgcgcgcgag 3480

cactacaagc accgcgtcgc gtgcgttgcg acggacctcg tcgacctcat cgcgcagctc 3540

gagacgcgtc tggtgcagac ggcgtatgcg ggcggcggcg gggcccgggc cgcgcgcacc 3600

gggccgctgg tgtttgcgtt ttcggggcag ggcacgcagt tcccggcgat ggcggcgccg 3660

ctggcgcggc ggtacgcgcg cttcggggag atcgtggggg gctgtgcgcg catggcgcgc 3720

gagctgagcg ggttccccgt ggatggcatt ctcctcggag acgatgtgac gcccgtgaag 3780

gacaacagcg ctgcggcgga ggtgcacagc gaggtggacc agatctgcat atttgtgtac 3840

cagtatgcga tgtgtcggtg gttgggcgag ctgggtgtgg agcccaaggc tgccataggc 3900

cacagcttgg gagagatcac agcagcggtc atcgcaggag cactcccttt cgaagccgcc 3960

ctcgatctgg tcgtcacgcg cgcccggctg ctcaagccgt gcgccgagca accgagcggc 4020

atggcggcgc tcgcctgcac cccagacgtc gcatcgaagc tcacactcgg cgcgtcggtg 4080

tcggtgtcgg tctacaacgg cccgcagagc atctgcctgt ccggcgcgtc ggccgagctc 4140

gacgatgcgg tgcgggccgc gaaggcgcgg aacatcaagg cgacgcggct gcaggtcgac 4200

cagggcttcc actcgccgtg cgtcgacgcc gcggtgccgg ggctgcaggc gtggtgcgcg 4260

gcgcaccgtg cgtcagctgc gccgctgaag atgccgctgt actcgaccgt gcgcggggac 4320

gtcgttccga agggagcggc gctggatccg gagcactggg tggcgcacgc gcggaacccg 4380

gtgctgttcg cgcagacggc gcaggctctg aaagagtccc ttccgcatgg gatcactttg 4440

gacgtcggcc cgcaggcggt ggcgtggtcg ctgctgctgc tgaacgggct cagcgcgacg 4500

cgcacggtcg ctgccggggc gaagaagggc gcagaccagg agcgcgcgct gctgggggcg 4560

ctgggggcgc tgttcgagca gcacaaggtc acgccggact ttgggcggct gtacgcgccg 4620

ctcgagaaga cgcggatccc gacgtacccc tttgagcgcg cgcggtgcta cccgacgttc 4680

ataccctcgc gcttcgcgca cggggctgcg gctgctgcga agacagatgg cgaggtcctg 4740

tcggccgaag aagaaaatgt ggcacccgtg ggattgctga caaaggagga tctgcgtgcg 4800

gctctcgtcg cgtgtctccg agcgacgctc gagctgcgcc ccgacgaaga gctggacgaa 4860

gcagagccgc tgaccgtgca cggcgtggac tcgatcgggt ttgcgaagct gcgcaagcac 4920

gtcgaggacc gctgggggct ggacatcccc gtcgtgtact ggtccgacgc gttcaccgtc 4980

ggcgagatgc tcggcaactt ggtcggccag tatgacgtag tgtctactgc tgcg 5034

<210> 45
<211> 1678
<212> PRT
<213> Mycena citricolor

<400> 45

Met Ser Ala Ser Ser Ser Tyr Ser Glu Leu Glu Ser Pro Thr Ser Leu
1               5                   10                  15

Leu Asp Val Phe Val His Ala Ala Arg Asp Pro His Thr Ala Ser Arg
            20                  25                  30

Arg Val Leu Glu Cys Gly Ser Asp Thr Trp Thr Tyr Ala Ala Leu Asp
        35                  40                  45

Ala Val Ser Asp Gly Ile Ala Arg Glu Leu Ala Pro Phe Gly Leu Ala
    50                  55                  60

Pro Lys Val Ala Val Val Ser Glu Asn His Pro Phe Val Phe Ala Leu
65                  70                  75                  80

Leu Phe Ala Val Trp Lys Leu Gly Gly Thr Phe Ile Pro Ile Asp Ala
                85                  90                  95

His Val Pro Phe Ala Met Leu Thr Gly Met Val Asn Ile Val Lys Pro
            100                 105                 110

Thr Cys Leu Tyr Leu Pro Ala Ser Ala Thr Ser Asn Ile Ser Leu Val
        115                 120                 125

Lys Ala Phe Asp Ile Arg Thr Val Val Phe Gly His Lys Glu Asn Ser
    130                 135                 140

Met Gln Ala Leu Phe Asp Lys Tyr Ser Leu His Ala Ala Pro Leu His
145                 150                 155                 160

Ala Ala Pro Leu His Tyr Ala Pro Pro Ser Ala Asp His Ala Cys Leu
                165                 170                 175

Tyr Leu Phe Thr Ser Ser Ala Ser Ser Thr Lys Asn Leu Lys Ala Val
            180                 185                 190

Pro Leu Thr His Thr Leu Val Leu Arg Gly Cys Gln Ser Lys Leu Ala
        195                 200                 205

Trp Trp Arg Arg Val Gln Pro Gly Lys Asn Leu Asp Ala Ile Arg Ile
    210                 215                 220

Leu Gly Trp Ala Pro Trp Ser His Val Leu Ala Tyr Met Gln Asp Ile
225                 230                 235                 240

Gly Thr Ala Thr Leu Leu Asn Ala Gly Cys Tyr Val Phe Ala Thr Ile
                245                 250                 255

Pro Ser Ser Tyr Pro Gln Leu Pro Thr Thr Thr Pro Val Asp Leu Thr
            260                 265                 270

Thr Ser Leu Ile Asp Ala Leu Val Thr Arg Arg Val Ala Ala Phe Ala
        275                 280                 285

Cys Val Pro Phe Val Leu Ala Asn Leu Lys Ala Ala Cys Gln Ser Asn
    290                 295                 300

His Pro Ala Arg Ser Gln Leu Leu Glu Ala Leu Gln Lys Thr Ile Met
305                 310                 315                 320

Leu Glu Cys Gly Gly Ala Val Leu Asp Asp Ala Thr Val Asp Trp Ala
                325                 330                 335

Glu Arg Asn Gly Ile Arg Ile Phe Thr Gly Ile Gly Met Thr Glu Thr
            340                 345                 350

Gly Gly Ala Val Phe Val Gly Leu Ala Ser Glu Ser Arg Arg Gly Phe
        355                 360                 365

Leu Pro Glu Gly Leu Leu Gly Asp Ala Ser Phe Ser Ile Ser Ser Asp
    370                 375                 380

Thr Asp Ala Leu Asp Glu Gly Glu Leu Val Val Lys Ser Lys Leu Ile
385                 390                 395                 400

Ala Pro Gly Tyr Val Gly Tyr Asp Asp Gly Ala His Ser Val Asp Cys
                405                 410                 415

Asp Gly Trp Val Thr Phe Arg Thr Gly Asp Arg Tyr Arg Gln Thr Gln

420                 425                 430

Pro Asp Gly Arg Phe Thr Trp Leu Gly Arg Ile Thr Asp Phe Ile Gln
        435                 440                 445

Met Val Ser Gly Glu Tyr Leu Asp Pro Arg Pro Leu Glu Glu Ser Leu
    450                 455                 460

Arg Ala Ser Pro Leu Ile Ala Asn Ala Cys Ile Val Gly Asp Ala Phe
465                 470                 475                 480

Leu Ser Ser Ala Ser Thr Ser Ile Leu Ala Ile Ile Glu Leu Ala Thr
                485                 490                 495

Pro Asp Leu Ala His Thr Ala Ser Ile Arg Ala Gln Leu Ala Arg Val
            500                 505                 510

Leu Ala Pro Leu Asn Arg Asp Leu Pro Pro Pro Leu Arg Ile Ala Ala
        515                 520                 525

Ser Ser Ile Leu Val Leu Asp Gly Met Arg Lys Ile Pro Lys Thr Lys
    530                 535                 540

Lys Gly Asp Ile Phe Arg Lys Lys Leu Glu Asp Thr Phe Gly Ala Glu
545                 550                 555                 560

Phe Glu Gln Met Leu Arg Thr Glu Lys Val Gly Leu Gly Asp Leu Ala
                565                 570                 575

Asp Val Asp Ala Gly Ile Thr Arg Ile Ile Gly Asn Leu Leu Gly Ile
            580                 585                 590

Ser Asp Asp Glu Leu Leu Ser Thr Met Ser Phe Ala Glu Leu Gly Met
        595                 600                 605

Thr Ser Leu Leu Ala Val Lys Ile Ala Ser Glu Leu Asn Lys Phe Leu
    610                 615                 620

Asp Gly Arg Ala Val Leu Pro Thr Asn Ile Cys Tyr Ile His Phe Asp
625                 630                 635                 640

Val Pro Ser Leu Thr Ser Ser Val Arg Glu Arg Leu Ser Ser Ala Pro
                645                 650                 655

Ser Ser Ile Thr Ser Ala Ala Ser Glu Pro Ala Ala Ser Ser Pro Gly
            660                 665                 670

Thr Pro Arg Ala Asp Glu Ile Val Ile Val Gly Lys Ala Phe Arg Leu
        675                 680                 685

Pro Asp Gly Val Asn Asp Asp Ala Ala Leu Trp Asp Val Leu Thr Gly
    690                 695                 700

Glu Ser Ala Ser Ile Ile Lys Asp Ile Pro Ala Asp Arg Trp Asp His
705                 710                 715                 720

Ala Ser Phe Tyr Pro Lys Asp Ile His Phe Gly Arg Ala Gly Leu Val
                725                 730                 735

Asp Val Ala Arg Phe Asp Tyr Gly Phe Phe Gly Met Thr Ala Ser Glu
            740                 745                 750

Ala Tyr Ser Leu Ser Pro Thr Met Arg Leu Ala Leu Glu Val Ala Tyr
        755                 760                 765

Glu Ala Leu Glu Asp Ala Asn Ile Pro Phe Arg Ala Val Lys Gly Ser
    770                 775                 780

Arg Met Gly Val Phe Val Ala Val Lys Asp Asp Gly Phe Glu Thr Leu
785                 790                 795                 800

Leu His Ala Gly Gln Gly Tyr Asp Ala Tyr Thr Arg Phe Tyr Gly Thr
                805                 810                 815

Gly Arg Ala Pro Ser Thr Ala Ser Gly Arg Ile Asn Tyr Leu Leu Asp
            820                 825                 830

Leu His Gly Pro Ser Ile Thr Val Asp Thr Ala Cys Ser Gly Gly Ile
        835                 840                 845

Val Cys Ile Asp Gln Ala Val Thr Tyr Leu Gln Ser Gly Ala Ala Glu
    850                 855                 860

Thr Ala Ile Val Cys Ser Ser Asn Thr His Cys Trp Pro Gly Ser Phe
865                 870                 875                 880

Met Phe Leu Thr Ala Gln Gly Met Ala Ser Pro Asn Gly Arg Cys Ala
                885                 890                 895

Ser Phe Thr Ser Asp Ala Asp Gly Tyr Ala Pro Ser Glu Gly Ala Val
            900                 905                 910

Gly Phe Val Leu Lys Thr Arg Ser Ala Ala Val Arg Asp Gly Asp Arg
        915                 920                 925

Ile Leu Ala Thr Ile Arg Ala Thr Glu Ile Gly His Asn Gly Arg Ser
    930                 935                 940

Gln Gly Leu Ala Ala Pro Asn Val Arg Ser Gln Ala Ala Ala His Arg
945                 950                 955                 960

Ala Val Leu Arg Arg Ala Arg Leu Asp Pro Ser Glu Ile Asp Phe Ile
                965                 970                 975

Glu Ala His Gly Thr Gly Thr Thr Leu Gly Asp Leu Cys Glu Val Gln
            980                 985                 990

Gly Ile Asn Asp Ser Phe Val Ser  Pro Lys Lys Arg Ala  Asn Pro Leu
        995                 1000                1005

Val Val  Ser Ala Ser Lys Ser  Thr Ile Gly His Thr  Glu Pro Ser
    1010                1015                1020

Ala Gly  Leu Val Gly Ile Leu  Ser Ala Leu Met Ser  Phe Glu Lys
    1025                1030                1035

Arg Ile  Val Pro Arg Leu Ala  Tyr Leu Thr Glu Ser  Asn Val Asn
    1040                1045                1050

Pro Ala  Leu Asp Ala Ser Val  Val Pro Leu His Phe  Pro Thr Lys
    1055                1060                1065

His Ile  Glu Leu Arg Ala Asp  Val Pro Tyr Lys Ala  Val Val Met
    1070                1075                1080

Ser Tyr  Gly Phe Ala Gly Thr  Leu Ala Asp Ile Val  Leu Glu Ser
    1085                1090                1095

Glu Val  Pro Gln Pro Thr Pro  Ala Val Ala Gln Asp  Thr Ala Gly
    1100                1105                1110

Gln Gln  Pro Met Leu Phe Val  Leu Ser Ala Lys Thr  Pro Arg Ala
    1115                1120                1125

Leu Ala  Ala Tyr Ile Glu Leu  Tyr Leu Gly Phe Leu  Arg His Ala
    1130                1135                1140

Asp Pro  Gly Leu Phe Ala Arg  Ile Cys Tyr Thr Ala  Cys Val Ala
    1145                1150                1155

Arg Glu  His Tyr Lys His Arg  Val Ala Cys Val Ala  Thr Asp Leu
    1160                1165                1170

Val Asp Leu Ile Ala Gln Leu Glu Thr Arg Leu Val Gln Thr Ala
    1175                1180                1185

Tyr Ala Gly Gly Gly Gly Ala Arg Ala Ala Arg Thr Gly Pro Leu
    1190                1195                1200

Val Phe Ala Phe Ser Gly Gln Gly Thr Gln Phe Pro Ala Met Ala
    1205                1210                1215

Ala Pro Leu Ala Arg Arg Tyr Ala Arg Phe Gly Glu Ile Val Gly
    1220                1225                1230

Gly Cys Ala Arg Met Ala Arg Glu Leu Ser Gly Phe Pro Val Asp
    1235                1240                1245

Gly Ile Leu Leu Gly Asp Asp Val Thr Pro Val Lys Asp Asn Ser
    1250                1255                1260

Ala Ala Ala Glu Val His Ser Glu Val Asp Gln Ile Cys Ile Phe
    1265                1270                1275

Val Tyr Gln Tyr Ala Met Cys Arg Trp Leu Gly Glu Leu Gly Val
    1280                1285                1290

Glu Pro Lys Ala Ala Ile Gly His Ser Leu Gly Glu Ile Thr Ala
    1295                1300                1305

Ala Val Ile Ala Gly Ala Leu Pro Phe Glu Ala Ala Leu Asp Leu
    1310                1315                1320

Val Val Thr Arg Ala Arg Leu Leu Lys Pro Cys Ala Glu Gln Pro
    1325                1330                1335

Ser Gly Met Ala Ala Leu Ala Cys Thr Pro Asp Val Ala Ser Lys
    1340                1345                1350

Leu Thr Leu Gly Ala Ser Val Ser Val Ser Val Tyr Asn Gly Pro
    1355                1360                1365

Gln Ser Ile Cys Leu Ser Gly Ala Ser Ala Glu Leu Asp Asp Ala
    1370                1375                1380

Val Arg Ala Ala Lys Ala Arg Asn Ile Lys Ala Thr Arg Leu Gln
    1385                1390                1395

Val Asp Gln Gly Phe His Ser Pro Cys Val Asp Ala Ala Val Pro

1400                1405                1410

Gly Leu Gln Ala Trp Cys Ala Ala His Arg Ala Ser Ala Ala Pro
    1415                1420                1425

Leu Lys Met Pro Leu Tyr Ser Thr Val Arg Gly Asp Val Val Pro
    1430                1435                1440

Lys Gly Ala Ala Leu Asp Pro Glu His Trp Val Ala His Ala Arg
    1445                1450                1455

Asn Pro Val Leu Phe Ala Gln Thr Ala Gln Ala Leu Lys Glu Ser
    1460                1465                1470

Leu Pro His Gly Ile Thr Leu Asp Val Gly Pro Gln Ala Val Ala
    1475                1480                1485

Trp Ser Leu Leu Leu Leu Asn Gly Leu Ser Ala Thr Arg Thr Val
    1490                1495                1500

Ala Ala Gly Ala Lys Lys Gly Ala Asp Gln Glu Arg Ala Leu Leu
    1505                1510                1515

Gly Ala Leu Gly Ala Leu Phe Glu Gln His Lys Val Thr Pro Asp
    1520                1525                1530

Phe Gly Arg Leu Tyr Ala Pro Leu Glu Lys Thr Arg Ile Pro Thr
    1535                1540                1545

Tyr Pro Phe Glu Arg Ala Arg Cys Tyr Pro Thr Phe Ile Pro Ser
    1550                1555                1560

Arg Phe Ala His Gly Ala Ala Ala Ala Ala Lys Thr Asp Gly Glu
    1565                1570                1575

Val Leu Ser Ala Glu Glu Glu Asn Val Ala Pro Val Gly Leu Leu
    1580                1585                1590

Thr Lys Glu Asp Leu Arg Ala Ala Leu Val Ala Cys Leu Arg Ala
    1595                1600                1605

Thr Leu Glu Leu Arg Pro Asp Glu Glu Leu Asp Glu Ala Glu Pro
    1610                1615                1620

Leu Thr Val His Gly Val Asp Ser Ile Gly Phe Ala Lys Leu Arg
    1625                1630                1635

Lys His Val Glu Asp Arg Trp Gly Leu Asp Ile Pro Val Val Tyr
    1640                1645                1650

Trp Ser Asp Ala Phe Thr Val Gly Glu Met Leu Gly Asn Leu Val
    1655                1660                1665

Gly Gln Tyr Asp Val Val Ser Thr Ala Ala
    1670                1675

<210> 46
<211> 4956
<212> DNA
<213> Mycena chlorophos

<400> 46
atgaatccgc cctcgtctat cctcgaagtc ttccagcgta ccgccctcga cccctcgggc 60

gccgaccggc gcgttctgga atgcgggccc gacttttgga cctacgcggg tctcgacgcg 120

gtttccacag gccttgcggc ggacttggct gctctcggag attctcccat cgtggctgtc 180

gtcgctgaga accatccctt cgtcttcgca ctcatgtttg ccgtctggaa attgcacggg 240

acgtttgttc ccatcgacgc gcatattccg tggaacctgc tcgacggcat gttggacatc 300

gtcaagccga cttgcatgtt tctcgtcgag tcggatacca acaatatctc gaacaccaag 360

gcccagggag tcgacttcgc tgtgcgcctc ttcggaggag aaggattcac catcccggcg 420

ctctcggcca aatatgcggg gaacgtctcg aatggcgccc ccgagtcact cccttctcca 480

gacgcgactg ctctgtatct gttcacgtcg tctgcctcgt cgcggcacaa tttgaaggcc 540

gttccgctca cgcatcgatt cattgccgct ggctgcgaag cgaaactcgc cttctggcac 600

cgtcttcatc ctcacaaccc caccgatgcg attcgcgtgc tgggatgggc tccgttgtcg 660

cacgtcctcg cgcatatgca ggatatcggc accgcggccc tcctcaacgc cggctgctat 720

gtcttttcga cgatcccctt gtcttacacc tcagcagaaa ctcagcccgc gcaagatatc 780

acctcggctc tcattcactc cgtgctccat tacgaggtca aagcatttgc gggcctgcct 840

tttgttattg cggcattcaa ggctgcttgt gaaggcggga acgaccgtct cctagcgcaa 900

ctacgctcca tgaccatgct cgagtgtggc ggggcgcagt tggacaagga catcgtggat 960

tgggcagtca agcaagcgat cccgctcgtg gttggggtcg ggatgacgga aacgggtggc 1020

gcgatactgg cgggccccgt cggggatgcg tcggatgggt ttcaccccca agggctgctg 1080

ctagatgcac agttctccct tatcggcaat gacgatgaat cggaaggcga gctggtcatc 1140

aagagtccca atcttccgcg cggatatctg aagtacgagg acggctcgtt cgacatcgac 1200

gcgcagggtg tcgtcacgtt caagactgga gacatctacc gcaaaagtgt cgagggtaaa 1260

ctcctttggg ttgggcgtag tacggatttc atccagatgg ctaccggcga gacgctcgat 1320

ccccgtcgtg ttgaacgggc gctacgcttc gcatcgggga tcaacgatgc ctgcatcatc 1380

gggaatgcgt tcctgaatgg ctcctctacc gcaatttacg ccatcatcga gctcgctccg 1440

cgcaccgtca acatcaataa tgattcaaat gtctcccatc tgcaggtggt tgcccgcgcc 1500

ctgtctccta tcaaccgcga ccttcctccc gcattacgca ttgtgttgtc ttctgtattg 1560

attctggctg aaggcatgaa gattccgagg acaaagaaag gcgaaatctt ccggaagaag 1620

attgatgaag ttttcggagc tgctctccgg gctttaggtc actcggcaac tccaacagag 1680

gttgttcttg agcaggaacc agcggcagcc agcaaaccca tttttgacaa gaacaagctg 1740

cagactgcta ttgcgcatag cttgggtctg gatattctgg agattgacct gctggacaag 1800

ctgacctttg ctgagcttgg catgacatct attcttgcaa ttcgggttgc agaagatctc 1860

aacaaattgc tgcagggaca agttaccctg ccagtcaata tctgctacct ctatccagat 1920

gcccagctgt tgtttgcagc agttcaggaa cagctgctca agcagcagca cccttcaacc 1980

ccaactgctc cctccgtgcc ggctttgctg tcagcaactt cctctgttcc aattctcttg 2040

caggaactag atgatgttgt cattgttggc aaatcattcc ggttgcctgg cggaatctac 2100

gatgatcgag cactctgggc agctctcacc aatcaagcta cccgaaaccc catctcatat 2160

atttcgggcc agcgctggga ccatacaagc ttttacccag ctgatattgc attcttgcag 2220

gcagggttgc ttgactccga ccactttacg gattttgatg cagctttctt cggatgaccc 2280

gagaaagagg catactatct gtccccgacc atgcggcttg ctcttgaagt agcctttgag 2340

gctctagaag atgcaaatat tcctgttggt caggtgaaag gcactagcat gggagtatat 2400

gcagctgtca aggatgatgg attcgaaacc cttttgaatg ctgctcatgg gtatgatgcc 2460

tacacacgat tctacggaac tggacgggca ccaagtacca ctagcgggcg gatcagtcaa 2520

ggagaatcag cgattgtctg ctccagcaat acacactgtt ggcctggctc cttcatgttc 2580

ttgactgccc aaggaatggt gtctcctcat ggacgttgtg cctccttcag tgctcaagca 2640

gatggatatg ttccttcaga gggtgctgtt gcatttatcc tgaagacccg caaagcagca 2700

gttcgggatg gaaaccaaat ttttgccaca attcgggctg cggtggtatc acacaatggt 2760

cgatcacaag gtcttgcagc accaaacatt caagcccaat ccgagttgca tcaacaagca 2820

ttgcagaagg caaatatcca acccactgat attcattttg tggaaactca tggaacaggg 2880

acttcgcttg gggatgtctg tgagattcat gggataaatg ctgcttttgc agcaggtcac 2940

cgtccctctg gacctctcat cattagtgca agcaaaggca ctattggaca tacagagcct 3000

tctgcaggtc ttgtgggcat catggcggca ctgctctcct tcaagcatgg ccttgttcct 3060

gggctgatcc atacatctca tgggcaactc aacccggcac ttgatcaatc caaagttccg 3120

cttatcttca gcccacaaac aatttccctg ggcggagaaa agccttacag atctgtggtc 3180

atgtcatatg gctttgcagg cacactagcg gatattgtgc ttgaaggccc tgctgaggag 3240

gctttttccg ggccaggcaa aaacagcagt gctcctccgc ctatgatctt tgccctcagt 3300

gccaaatctg catcagccct ccaagaatac aagcagaagt acatcacctt cctgcagaat 3360

gttggctctg gaggccaact gttcagcaag atctgtttga cttcttgcat tgcccgagag 3420

cactacaagc atagattctc ctgtgctgct cagaacacac tggatcttct tctgcagcta 3480

gagcactctg ttgctgccag ccacaaacct ccaacaactc gtaccggaac agtcaccttt 3540

gctttctctg gacagggagc ccaattcccc agcatggatg cagctctggc tcaaggctac 3600

tctgccttca aatccatctt gctggaactt ggaaacaagg ctgccaaact ctctggattc 3660

cccatcactg attgcctgtt ggcaacaaca gcatcagctg atgaagaagc cgtccatagt 3720

gaggtggacc aaatttgcat ttttgttcat caatatgcaa tggctctttt cctcgagatg 3780

ctaggaattg tccccggtgc tgccataggc cacagcttgg gagagatcac agcagcggtc 3840

gttgctggtg gactttcgtt tgaacttggc ctagagttgg tcatcctccg tgcacatctg 3900

ctccgtccag agcagaacaa gcccgctggc atggctgcct tggcatgctc agaagcggac 3960

ttcctcaagt ttccgtccac cgatgcaact atttctgttt tcaactctcc tcggagcatt 4020

gcagtctctg gagcagcaag ctccattgag acagttctta ctgctgccaa agagcagaat 4080

atcaaggcca cgaagctcag ggttgatcaa ggattccata gcagctatgt ggagcatgcg 4140

cttcccgggc tcaagcactg gtcagcaatg aattcaggct ccttccaggc actcaggatt 4200

ccactctatt caactgcact tggccatgtt gttcctgctg gagagaccct tcagccagat 4260

cactggatga accatacccg caatgctgtt cattttacgc aaactgcgca ggctctgaaa 4320

gagtcccttc cgcatgggat cactttggat cttggtcctc aggctgtagc tcaaactctc 4380

ctgctggcca atgaccatcc tgttggccgc accattggat tgtgtggcaa acgcacagga 4440

gatcaaagac atgcattcct gctcgctctt gctgagcttt accagcagca tggtcttgtg 4500

cccaactttc atgcacttta tggcgtagct gcccaggatc tcaaggacca tctcaccagc 4560

ctgccaacat atccattcca acgtgtccgc tgctatccca gctacattcc atcccgtcac 4620

tccaacactc ccgggaccac cgtggtgatt gatgcaaagc cgcgggatga agtgaaacct 4680

gtggcagagg tctcaaagtc ggacacggat tcttccacat cattttcttc gaccattctc 4740

ttccacattc gctccatcct tgagcttcgc ccccatgagg ttctggatac gtctgaatcc 4800

ctcttgacgt acggggtcga ctcgattggg tttgcagcac tgcagaaggc cctggagcag 4860

cagcatgggc taaacttgtc gattgtgttc tggagcgacg tgttttccat tgccgacatt 4920

gtgaagaatc ttgaggagca gaagagcttg aagatg 4956

<210> 47
<211> 1652
<212> PRT
<213> Mycena chlorophos

<400> 47

Met Asn Pro Pro Ser Ser Ile Leu Glu Val Phe Gln Arg Thr Ala Leu
1               5                   10                  15

Asp Pro Ser Gly Ala Asp Arg Arg Val Leu Glu Cys Gly Pro Asp Phe
            20                  25                  30

Trp Thr Tyr Ala Gly Leu Asp Ala Val Ser Thr Gly Leu Ala Ala Asp
        35                  40                  45

Leu Ala Ala Leu Gly Asp Ser Pro Ile Val Ala Val Val Ala Glu Asn
    50                  55                  60

His Pro Phe Val Phe Ala Leu Met Phe Ala Val Trp Lys Leu His Gly
65                  70                  75                  80

Thr Phe Val Pro Ile Asp Ala His Ile Pro Trp Asn Leu Leu Asp Gly
                85                  90                  95

Met Leu Asp Ile Val Lys Pro Thr Cys Met Phe Leu Val Glu Ser Asp
            100                 105                 110

Thr Asn Asn Ile Ser Asn Thr Lys Ala Gln Gly Val Asp Phe Ala Val
        115                 120                 125

Arg Leu Phe Gly Gly Glu Gly Phe Thr Ile Pro Ala Leu Ser Ala Lys
    130                 135                 140

Tyr Ala Gly Asn Val Ser Asn Gly Ala Pro Glu Ser Leu Pro Ser Pro
145                 150                 155                 160

Asp Ala Thr Ala Leu Tyr Leu Phe Thr Ser Ser Ala Ser Ser Arg His
                165                 170                 175

Asn Leu Lys Ala Val Pro Leu Thr His Arg Phe Ile Ala Ala Gly Cys
            180                 185                 190

Glu Ala Lys Leu Ala Phe Trp His Arg Leu His Pro His Asn Pro Thr
        195                 200                 205

Asp Ala Ile Arg Val Leu Gly Trp Ala Pro Leu Ser His Val Leu Ala
    210                 215                 220

His Met Gln Asp Ile Gly Thr Ala Ala Leu Leu Asn Ala Gly Cys Tyr
225                 230                 235                 240

Val Phe Ser Thr Ile Pro Leu Ser Tyr Thr Ser Ala Glu Thr Gln Pro
                245                 250                 255

Ala Gln Asp Ile Thr Ser Ala Leu Ile His Ser Val Leu His Tyr Glu
            260                 265                 270

Val Lys Ala Phe Ala Gly Leu Pro Phe Val Ile Ala Ala Phe Lys Ala
        275                 280                 285

Ala Cys Glu Gly Gly Asn Asp Arg Leu Leu Ala Gln Leu Arg Ser Met
    290                 295                 300

Thr Met Leu Glu Cys Gly Gly Ala Gln Leu Asp Lys Asp Ile Val Asp
305                 310                 315                 320

Trp Ala Val Lys Gln Ala Ile Pro Leu Val Val Gly Val Gly Met Thr
                325                 330                 335

Glu Thr Gly Gly Ala Ile Leu Ala Gly Pro Val Gly Asp Ala Ser Asp
            340                 345                 350

Gly Phe His Pro Gln Gly Leu Leu Leu Asp Ala Gln Phe Ser Leu Ile
        355                 360                 365

Gly Asn Asp Asp Glu Ser Glu Gly Glu Leu Val Ile Lys Ser Pro Asn
    370                 375                 380

Leu Pro Arg Gly Tyr Leu Lys Tyr Glu Asp Gly Ser Phe Asp Ile Asp
385                 390                 395                 400

Ala Gln Gly Val Val Thr Phe Lys Thr Gly Asp Ile Tyr Arg Lys Ser
                405                 410                 415

Val Glu Gly Lys Leu Leu Trp Val Gly Arg Ser Thr Asp Phe Ile Gln
            420                 425                 430

Met Ala Thr Gly Glu Thr Leu Asp Pro Arg Arg Val Glu Arg Ala Leu
        435                 440                 445

Arg Phe Ala Ser Gly Ile Asn Asp Ala Cys Ile Ile Gly Asn Ala Phe
    450                 455                 460

Leu Asn Gly Ser Ser Thr Ala Ile Tyr Ala Ile Ile Glu Leu Ala Pro
465                 470                 475                 480

Arg Thr Val Asn Ile Asn Asn Asp Ser Asn Val Ser His Leu Gln Val
                485                 490                 495

Val Ala Arg Ala Leu Ser Pro Ile Asn Arg Asp Leu Pro Pro Ala Leu
            500                 505                 510

Arg Ile Val Leu Ser Ser Val Leu Ile Leu Ala Glu Gly Met Lys Ile
        515                 520                 525

Pro Arg Thr Lys Lys Gly Glu Ile Phe Arg Lys Lys Ile Asp Glu Val
    530                 535                 540

Phe Gly Ala Ala Leu Arg Ala Leu Gly His Ser Ala Thr Pro Thr Glu
545                 550                 555                 560

Val Val Leu Glu Gln Glu Pro Ala Ala Ala Ser Lys Pro Ile Phe Asp
                565                 570                 575

Lys Asn Lys Leu Gln Thr Ala Ile Ala His Ser Leu Gly Leu Asp Ile
            580                 585                 590

Leu Glu Ile Asp Leu Leu Asp Lys Leu Thr Phe Ala Glu Leu Gly Met
        595                 600                 605

Thr Ser Ile Leu Ala Ile Arg Val Ala Glu Asp Leu Asn Lys Leu Leu
    610                 615                 620

Gln Gly Gln Val Thr Leu Pro Val Asn Ile Cys Tyr Leu Tyr Pro Asp
625                 630                 635                 640

Ala Gln Leu Leu Phe Ala Ala Val Gln Glu Gln Leu Leu Lys Gln Gln
                645                 650                 655

His Pro Ser Thr Pro Thr Ala Pro Ser Val Pro Ala Leu Leu Ser Ala
            660                 665                 670

Thr Ser Ser Val Pro Ile Leu Leu Gln Glu Leu Asp Asp Val Val Ile
        675                 680                 685

Val Gly Lys Ser Phe Arg Leu Pro Gly Gly Ile Tyr Asp Asp Arg Ala
    690                 695                 700

Leu Trp Ala Ala Leu Thr Asn Gln Ala Thr Arg Asn Pro Ile Ser Tyr
705                 710                 715                 720

Ile Ser Gly Gln Arg Trp Asp His Thr Ser Phe Tyr Pro Ala Asp Ile
                725                 730                 735

Ala Phe Leu Gln Ala Gly Leu Leu Asp Ser Asp His Phe Thr Asp Phe
            740                 745                 750

Asp Ala Ala Phe Phe Gly Met Thr Glu Lys Glu Ala Tyr Tyr Leu Ser
        755                 760                 765

Pro Thr Met Arg Leu Ala Leu Glu Val Ala Phe Glu Ala Leu Glu Asp
    770                 775                 780

Ala Asn Ile Pro Val Gly Gln Val Lys Gly Thr Ser Met Gly Val Tyr
785                 790                 795                 800

Ala Ala Val Lys Asp Asp Gly Phe Glu Thr Leu Leu Asn Ala Ala His
                805                 810                 815

Gly Tyr Asp Ala Tyr Thr Arg Phe Tyr Gly Thr Gly Arg Ala Pro Ser
            820                 825                 830

Thr Thr Ser Gly Arg Ile Ser Gln Gly Glu Ser Ala Ile Val Cys Ser
        835                 840                 845

Ser Asn Thr His Cys Trp Pro Gly Ser Phe Met Phe Leu Thr Ala Gln
    850                 855                 860

Gly Met Val Ser Pro His Gly Arg Cys Ala Ser Phe Ser Ala Gln Ala
865                 870                 875                 880

Asp Gly Tyr Val Pro Ser Glu Gly Ala Val Ala Phe Ile Leu Lys Thr
                885                 890                 895

Arg Lys Ala Ala Val Arg Asp Gly Asn Gln Ile Phe Ala Thr Ile Arg
            900                 905                 910

Ala Ala Val Val Ser His Asn Gly Arg Ser Gln Gly Leu Ala Ala Pro
        915                 920                 925

Asn Ile Gln Ala Gln Ser Glu Leu His Gln Gln Ala Leu Gln Lys Ala
    930                 935                 940

Asn Ile Gln Pro Thr Asp Ile His Phe Val Glu Thr His Gly Thr Gly
945                 950                 955                 960

Thr Ser Leu Gly Asp Val Cys Glu Ile His Gly Ile Asn Ala Ala Phe
                965                 970                 975

Ala Ala Gly His Arg Pro Ser Gly Pro Leu Ile Ile Ser Ala Ser Lys
            980                 985                 990

Gly Thr Ile Gly His Thr Glu Pro Ser Ala Gly Leu Val Gly Ile Met

995                 1000                1005

Ala Ala  Leu Leu Ser Phe Lys  His Gly Leu Val Pro  Gly Leu Ile
    1010                 1015                 1020

His Thr  Ser His Gly Gln Leu  Asn Pro Ala Leu Asp  Gln Ser Lys
    1025                 1030                 1035

Val Pro  Leu Ile Phe Ser Pro  Gln Thr Ile Ser Leu  Gly Gly Glu
    1040                 1045                 1050

Lys Pro  Tyr Arg Ser Val Val  Met Ser Tyr Gly Phe  Ala Gly Thr
    1055                 1060                 1065

Leu Ala  Asp Ile Val Leu Glu  Gly Pro Ala Glu Glu  Ala Phe Ser
    1070                 1075                 1080

Gly Pro  Gly Lys Asn Ser Ser  Ala Pro Pro Pro Met  Ile Phe Ala
    1085                 1090                 1095

Leu Ser  Ala Lys Ser Ala Ser  Ala Leu Gln Glu Tyr  Lys Gln Lys
    1100                 1105                 1110

Tyr Ile  Thr Phe Leu Gln Asn  Val Gly Ser Gly Gly  Gln Leu Phe
    1115                 1120                 1125

Ser Lys  Ile Cys Leu Thr Ser  Cys Ile Ala Arg Glu  His Tyr Lys
    1130                 1135                 1140

His Arg  Phe Ser Cys Ala Ala  Gln Asn Thr Leu Asp  Leu Leu Leu
    1145                 1150                 1155

Gln Leu  Glu His Ser Val Ala  Ala Ser His Lys Pro  Pro Thr Thr
    1160                 1165                 1170

Arg Thr  Gly Thr Val Thr Phe  Ala Phe Ser Gly Gln  Gly Ala Gln
    1175                 1180                 1185

Phe Pro  Ser Met Asp Ala Ala  Leu Ala Gln Gly Tyr  Ser Ala Phe
    1190                 1195                 1200

Lys Ser  Ile Leu Leu Glu Leu  Gly Asn Lys Ala Ala  Lys Leu Ser
    1205                 1210                 1215

Gly Phe  Pro Ile Thr Asp Cys  Leu Leu Ala Thr Thr  Ala Ser Ala
    1220                 1225                 1230

Asp Glu Glu Ala Val His Ser Glu Val Asp Gln Ile Cys Ile Phe
    1235                1240                1245

Val His Gln Tyr Ala Met Ala Leu Phe Leu Glu Met Leu Gly Ile
    1250                1255                1260

Val Pro Gly Ala Ala Ile Gly His Ser Leu Gly Glu Ile Thr Ala
    1265                1270                1275

Ala Val Val Ala Gly Gly Leu Ser Phe Glu Leu Gly Leu Glu Leu
    1280                1285                1290

Val Ile Leu Arg Ala His Leu Leu Arg Pro Glu Gln Asn Lys Pro
    1295                1300                1305

Ala Gly Met Ala Ala Leu Ala Cys Ser Glu Ala Asp Phe Leu Lys
    1310                1315                1320

Phe Pro Ser Thr Asp Ala Thr Ile Ser Val Phe Asn Ser Pro Arg
    1325                1330                1335

Ser Ile Ala Val Ser Gly Ala Ala Ser Ser Ile Glu Thr Val Leu
    1340                1345                1350

Thr Ala Ala Lys Glu Gln Asn Ile Lys Ala Thr Lys Leu Arg Val
    1355                1360                1365

Asp Gln Gly Phe His Ser Ser Tyr Val Glu His Ala Leu Pro Gly
    1370                1375                1380

Leu Lys His Trp Ser Ala Met Asn Ser Gly Ser Phe Gln Ala Leu
    1385                1390                1395

Arg Ile Pro Leu Tyr Ser Thr Ala Leu Gly His Val Val Pro Ala
    1400                1405                1410

Gly Glu Thr Leu Gln Pro Asp His Trp Met Asn His Thr Arg Asn
    1415                1420                1425

Ala Val His Phe Thr Gln Thr Ala Gln Ala Leu Lys Glu Ser Leu
    1430                1435                1440

Pro His Gly Ile Thr Leu Asp Leu Gly Pro Gln Ala Val Ala Gln
    1445                1450                1455

Thr Leu Leu Leu Ala Asn Asp His Pro Val Gly Arg Thr Ile Gly
    1460                1465                1470

Leu Cys Gly Lys Arg Thr Gly Asp Gln Arg His Ala Phe Leu Leu
    1475                1480                1485

Ala Leu Ala Glu Leu Tyr Gln Gln His Gly Leu Val Pro Asn Phe
    1490                1495                1500

His Ala Leu Tyr Gly Val Ala Ala Gln Asp Leu Lys Asp His Leu
    1505                1510                1515

Thr Ser Leu Pro Thr Tyr Pro Phe Gln Arg Val Arg Cys Tyr Pro
    1520                1525                1530

Ser Tyr Ile Pro Ser Arg His Ser Asn Thr Pro Gly Thr Thr Val
    1535                1540                1545

Val Ile Asp Ala Lys Pro Arg Asp Glu Val Lys Pro Val Ala Glu
    1550                1555                1560

Val Ser Lys Ser Asp Thr Asp Ser Ser Thr Ser Phe Ser Ser Thr
    1565                1570                1575

Ile Leu Phe His Ile Arg Ser Ile Leu Glu Leu Arg Pro His Glu
    1580                1585                1590

Val Leu Asp Thr Ser Glu Ser Leu Leu Thr Tyr Gly Val Asp Ser
    1595                1600                1605

Ile Gly Phe Ala Ala Leu Gln Lys Ala Leu Glu Gln Gln His Gly
    1610                1615                1620

Leu Asn Leu Ser Ile Val Phe Trp Ser Asp Val Phe Ser Ile Ala
    1625                1630                1635

Asp Ile Val Lys Asn Leu Glu Glu Gln Lys Ser Leu Lys Met
    1640                1645                1650

<210> 48
<211> 5313
<212> DNA
<213> Armillaria gallica

<400> 48
atggaggccg acggtcacca ctctcttctc gatgtctttc tcagcgttgc acatgattct 60

gagaagtcca aacgtaatgt cttggaatgc ggccaggata cctggacata ctcagatttg 120

gacatcatct cgtcggcctt ggcacaggat ctcaaagcta ccttgggttg ttttcccaaa 180

gttgcagtcg tcagcgagaa ccatccctac gtgtttgctc tcatgctggc cgtttggaag 240

cttgaaggga tattcatccc catcgacgtc catattacag ctgaccttct aaagggcatg 300

ctacgcattg ttgcccccac ttgtctggcg atcccagaga ccgatatttc caaccagcgt 360

gttgcctctg cgattggtat acatgttctc cccttcaacg tgaatgcgtc gaccatgaat 420

gcacttcgac agaaatacga cccatttact cagaatgcct cgctatctgg atgcgcactt 480

ccttacgttg atcgcgcatg cctctatctc tttacatcct ccgcgtcctc tactgccaac 540

ttgaaatgtg tacctttgac ccatactctt atcctcagta actgccgttc caagctcgca 600

tggtggcggc gcgttcgtcc ggaaggcgaa atggatggga tacgtgttct agggtgggca 660

ccttggtcgc atatccttgc ctacatgcaa gatattggca cggcaacgtt cctgaatgcc 720

ggctgctatg tctttgcgtc cgttccatcc acatatcctt cacagctggc agcgaatggc 780

ctacaaggcc ccaccatgaa tatcatcgat tcacttcttg aacggcgagt cgccgcattt 840

gcttgcgtac cgttcatttt gagcgaacta aaagctatgt gcgagacggc tgccagtcca 900

gatgacaagc atctcatgtg cttgagagct gaggagaaag ttcgccttgt cagtgcgctg 960

cagcggctta tgatgctcga gtgcggaggc gctgcgctcg agtcggatgt cacacgttgg 1020

gccgtcgaaa atggcatatc ggtcatggtc ggcatcggga tgacggagac agtcggtacg 1080

ctgtttgcag agcgcgcgca agacgcctgt tccaatggct attctgcgca ggacgccctc 1140

attgctgatg gcatcatgtc actggtcggg tctgacaacg aggaagccac cttcgaaggg 1200

gaactagtcg tgaagagcaa gctcatccca cacggataca tcaactaccg tgattcgtcg 1260

ttttcggtgg actcggacgg ctgggtaacg ttcaaaacgg gagacaaata tcagcgcacg 1320

ccagatggac gattcaaatg gcttggaaga aagaccgatt ttattcagat gacaagcagc 1380

gaaacattgg atcccagacc cattgagcaa gccctctgtg cgaatccaag tatcgcaaat 1440

gcatgcgtca ttggtgacag gttcctgaga gagcctgcga ctagcgtatg cgccattgtc 1500

gagatcgggc ctgaagtgga catgccttcg tccaagatcg acagagaaat tgcgaatgcc 1560

ctcgctccaa tcaatcgcga ccttcctcct gctcttcgca tatcatggtc tcgcgtactc 1620

ataattcgac ctcctcagaa aataccagtt acgaggaaag gcgatgtgtt ccgtaagaag 1680

attgaagata tgtttgggcc tttccttggt gtcggcgttt ctaccgaggt cgaagccggc 1740

catgaaacta aagaagatga cacggaacac atcgtgagac aggttgtgag caatcttctt 1800

ggcgtccatg atcctgagct attgtctgct ttatctttcg ctgagctggg catgacctca 1860

tttatggctg ttagcatcgt caacgctctc aacaaacgca tcggcggcct cacccttccg 1920

tctaatgcat gctacatcca tattgatctt gattctcttg tggacgcgat ttcacttgaa 1980

tatggtcatg gaagtatccc tgcagagttg ccttctaacc ctttccccga catcgagtcc 2040

catcagcata atgataagga cattgtgata gtcggcaagg cattccgttt acccggctca 2100

ctcaacagta ctgcaactct ctgggaagct ttgttatcga ataacaattc ggtcatcagt 2160

gatatcccat ctgatcgctg ggatcatgca agcttttacc cccacaatat atgtttcacg 2220

aaagcaggcc tcgtcgatgt tgcacattac gactacagat tttttggcct catggcgaca 2280

gaggcgttgt acgtatctcc gacgatgcgc ctcgccttgg aagtgtcatt tgaagccctg 2340

gagaacgcaa atattccgct atccaagctg aaggggacac aaaccgctgt ctatgtcgcc 2400

actaaagacg atggcttcga gacactctta aatgccgagc aaggttacga tgcgtacacg 2460

cgattctacg gcacgggtcg cgctccgagt accgcaagcg gtcgcataag ctatcttctt 2520

gatattcatg ggccatctat taccgttgat acagcatgca gcggaggcat tgtatgtatg 2580

gatcaagcca tcactttctt gcaatccgga ggggccgata ccgcaattgt ctgttcgacc 2640

aatacgcact gttggccggg atcattcatg ttcctgacgg cacaaggcat ggtttctcca 2700

aatggaagat gcgctacatt cactaccgat gcagacggat atgtgccttc ggagggtgca 2760

gtggctttca ttctcaagac gcgcagcgct gcaatacgcg acaatgacaa tatactcgcc 2820

gtgatcaaat caacagatgt gtcccataac ggccgttctc aagggctagt tgcaccaaac 2880

gtaaaggcgc aggcaaacct acaccggtcg ttgctacgaa aagctgggct gtttcctgat 2940

caaattaact ttatcgaagc tcatggaaca ggtacatctc taggagacct ttcggaaatc 3000

cagggcatta ataacgccta catctcatca cgacctcgtc tggccggtcc ccttatcatt 3060

agtgcatcga aaacagtttt aggacacagt gaaccaacag cagggatggc cggcatcctc 3120

acagccttgc ttgcccttga gaaagagaca gttcctggtt taaatcactt aacggatcac 3180

aacctcaacc cttcgcttga ttgcagcgta gttcctctcc tgattcctca cgagtctatt 3240

cacattggtg gtgcaaagcc acatcgagct gcggttctgt catacggctt cgcgggtacg 3300

ctggccggtg ctatcttaga aggaccacct tctggtgtac catggccgtc gtcaaatgat 3360

atacaagaac accctatgat tttcgtcgtc agtgggaaaa ccgtgcctac actggaagcg 3420

tacctgggac ggtatttgac atttttgcgg gtcgcaaaaa cacacgactt ccaggacatc 3480

tgctacacca cttgcgtcgg gagggagcac tacaaatacc ggttctcctg cgttgcccga 3540

aacatggcag accttatttc tcaaattgaa catcgactga caactctttc cacttcgaaa 3600

cagaagcctc gcggctcgtt agggttcatg ttctcaggac aaggcacttg tttccctggt 3660

atggcttcag cacttgctga acaatattcg gggttccgaa tgctcgtctc taagtttggg 3720

caggctgccc aagagcggtc cggttatccg atcgataggc tgttgcttga agtttctgat 3780

acattaccag aaacaaacag cgaggtcgac caaatttgca tttttgtcta ccaatattct 3840

gttctgcaat ggttgcaatg tctaggcatt caaccgaaag cagtcctcgg tcacagcctg 3900

ggagaaatta ctgccgcagt cgcagctggc gccctttcgt tcgaatctgc gttggatctt 3960

gtggtcaccc gtgctcgtct tctccgtccc agaacaaaag actctgcagg aatggccgca 4020

gtagcagcgt ccaaggaaga agttgaagga gttatagaaa ccctccaact tgcaaactcg 4080

ctaagcgttg cggttcacaa cggtccgcgg agtgttgttg tgtcaggcgc atcagcagaa 4140

atcgatgccc tagttgtcgc agctaaagaa cggggcttga aagcctcccg cttaaaggtt 4200

gaccaaggct tccacagccc ttacgttgac tctgcggttc cgggtttact cgactggtca 4260

aacaagcatc gttcgacctt ctttccttta aacattcctc tatactcgac tttgaccggc 4320

gagcttattc cgaagggacg gaggttcgtc tcggatcact gggtaaacca tgctcgaaaa 4380

cctgttcagt ttgcggcggc agcggcagcg gtggatgaag atcgatccat tggtgtgctc 4440

gttgacgtcg gaccccaacc cgttgcgtgg accctccttc aagcaaacaa ccttctcaat 4500

acctctgcag ttgcgctatc cgcaaaggcc ggaaaggacc aggagatggc gctgctcact 4560

gctttgagct acctcttcca agagcacaac ctttctccca acttccacga gctttactct 4620

cagcgtcatg ggactctgca gaagacggac attcccacct acccattcca acgtgtccac 4680

cgctatccga ccttcatacc gtcacgaaat caaagtcctg ctattgcaac ggtagttata 4740

ccgccacctc gcttctctgt ccaaaaggct gcggatgtag catcacagtc gaaggaatca 4800

gactgtcgag ctggtttgat cagttgcctt agagccatcc tcgaattaac accggaagag 4860

gagtttgacc tttctgagac tctcaacgct cgtggtatgg attcgatcat gtttgcgcag 4920

ctacggaagc gggttgggga agaattcaac ctcgatatac ccatgatcta tctatcagac 4980

gtgttcacga tggaacagat ggtcgactac ctcgtcgaac agtccggatc cacacccgcg 5040

tcaaagcacg tagaaacttc agctaatcaa ccattagacg aagaagatct ccggacgggg 5100

ctcttgtcat gcctgaggaa cgtgctagaa attacccccg atgaagaact tgacctatct 5160

gaaactttga atgctcgtgg tgttgactcg atcatgttcg ctcagctgcg gaaacgcgtt 5220

ggggaaggtt ttggtgtgga aattccgatg atatatctgt ctgacgtgtt taccatggaa 5280

gacatgatca atttcctcgt ctccgagcgg tcg 5313

<210> 49
<211> 1771
<212> PRT
<213> Armillaria gallica

<400> 49

Met Glu Ala Asp Gly His His Ser Leu Leu Asp Val Phe Leu Ser Val
1               5                   10                  15

Ala His Asp Ser Glu Lys Ser Lys Arg Asn Val Leu Glu Cys Gly Gln
            20                  25                  30

Asp Thr Trp Thr Tyr Ser Asp Leu Asp Ile Ile Ser Ser Ala Leu Ala
        35                  40                  45

Gln Asp Leu Lys Ala Thr Leu Gly Cys Phe Pro Lys Val Ala Val Val
    50                  55                  60

Ser Glu Asn His Pro Tyr Val Phe Ala Leu Met Leu Ala Val Trp Lys
65                  70                  75                  80

Leu Glu Gly Ile Phe Ile Pro Ile Asp Val His Ile Thr Ala Asp Leu
                85                  90                  95

Leu Lys Gly Met Leu Arg Ile Val Ala Pro Thr Cys Leu Ala Ile Pro
            100                 105                 110

Glu Thr Asp Ile Ser Asn Gln Arg Val Ala Ser Ala Ile Gly Ile His
        115                 120                 125

Val Leu Pro Phe Asn Val Asn Ala Ser Thr Met Asn Ala Leu Arg Gln
    130                 135                 140

Lys Tyr Asp Pro Phe Thr Gln Asn Ala Ser Leu Ser Gly Cys Ala Leu
145                 150                 155                 160

Pro Tyr Val Asp Arg Ala Cys Leu Tyr Leu Phe Thr Ser Ser Ala Ser
                165                 170                 175

Ser Thr Ala Asn Leu Lys Cys Val Pro Leu Thr His Thr Leu Ile Leu
            180                 185                 190

Ser Asn Cys Arg Ser Lys Leu Ala Trp Trp Arg Arg Val Arg Pro Glu
        195                 200                 205

Gly Glu Met Asp Gly Ile Arg Val Leu Gly Trp Ala Pro Trp Ser His
    210                 215                 220

Ile Leu Ala Tyr Met Gln Asp Ile Gly Thr Ala Thr Phe Leu Asn Ala
225                 230                 235                 240

Gly Cys Tyr Val Phe Ala Ser Val Pro Ser Thr Tyr Pro Ser Gln Leu
                245                 250                 255

Ala Ala Asn Gly Leu Gln Gly Pro Thr Met Asn Ile Ile Asp Ser Leu
            260                 265                 270

Leu Glu Arg Arg Val Ala Ala Phe Ala Cys Val Pro Phe Ile Leu Ser
        275                 280                 285

Glu Leu Lys Ala Met Cys Glu Thr Ala Ala Ser Pro Asp Asp Lys His
    290                 295                 300

Leu Met Cys Leu Arg Ala Glu Glu Lys Val Arg Leu Val Ser Ala Leu
305                 310                 315                 320

Gln Arg Leu Met Met Leu Glu Cys Gly Gly Ala Ala Leu Glu Ser Asp
                325                 330                 335

Val Thr Arg Trp Ala Val Glu Asn Gly Ile Ser Val Met Val Gly Ile
            340                 345                 350

Gly Met Thr Glu Thr Val Gly Thr Leu Phe Ala Glu Arg Ala Gln Asp
        355                 360                 365

Ala Cys Ser Asn Gly Tyr Ser Ala Gln Asp Ala Leu Ile Ala Asp Gly
    370                 375                 380

Ile Met Ser Leu Val Gly Ser Asp Asn Glu Glu Ala Thr Phe Glu Gly
385                 390                 395                 400

Glu Leu Val Val Lys Ser Lys Leu Ile Pro His Gly Tyr Ile Asn Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Gln Ala Leu Cys Ala Asn Pro Ser Ile Ala Asn
465                 470                 475                 480

Ala Cys Val Ile Gly Asp Arg Phe Leu Arg Glu Pro Ala Thr Ser Val
                485                 490                 495

Cys Ala Ile Val Glu Ile Gly Pro Glu Val Asp Met Pro Ser Ser Lys
            500                 505                 510

Ile Asp Arg Glu Ile Ala Asn Ala Leu Ala Pro Ile Asn Arg Asp Leu
        515                 520                 525

Pro Pro Ala Leu Arg Ile Ser Trp Ser Arg Val Leu Ile Ile Arg Pro
    530                 535                 540

Pro Gln Lys Ile Pro Val Thr Arg Lys Gly Asp Val Phe Arg Lys Lys

545                 550                 555                 560

Ile Glu Asp Met Phe Gly Pro Phe Leu Gly Val Gly Val Ser Thr Glu
                565                 570                 575

Val Glu Ala Gly His Glu Thr Lys Glu Asp Asp Thr Glu His Ile Val
            580                 585                 590

Arg Gln Val Val Ser Asn Leu Leu Gly Val His Asp Pro Glu Leu Leu
        595                 600                 605

Ser Ala Leu Ser Phe Ala Glu Leu Gly Met Thr Ser Phe Met Ala Val
    610                 615                 620

Ser Ile Val Asn Ala Leu Asn Lys Arg Ile Gly Gly Leu Thr Leu Pro
625                 630                 635                 640

Ser Asn Ala Cys Tyr Ile His Ile Asp Leu Asp Ser Leu Val Asp Ala
                645                 650                 655

Ile Ser Leu Glu Tyr Gly His Gly Ser Ile Pro Ala Glu Leu Pro Ser
            660                 665                 670

Asn Pro Phe Pro Asp Ile Glu Ser His Gln His Asn Asp Lys Asp Ile
        675                 680                 685

Val Ile Val Gly Lys Ala Phe Arg Leu Pro Gly Ser Leu Asn Ser Thr
    690                 695                 700

Ala Thr Leu Trp Glu Ala Leu Leu Ser Asn Asn Asn Ser Val Ile Ser
705                 710                 715                 720

Asp Ile Pro Ser Asp Arg Trp Asp His Ala Ser Phe Tyr Pro His Asn
                725                 730                 735

Ile Cys Phe Thr Lys Ala Gly Leu Val Asp Val Ala His Tyr Asp Tyr
            740                 745                 750

Arg Phe Phe Gly Leu Met Ala Thr Glu Ala Leu Tyr Val Ser Pro Thr
        755                 760                 765

Met Arg Leu Ala Leu Glu Val Ser Phe Glu Ala Leu Glu Asn Ala Asn
    770                 775                 780

Ile Pro Leu Ser Lys Leu Lys Gly Thr Gln Thr Ala Val Tyr Val Ala
785                 790                 795                 800

Thr Lys Asp Asp Gly Phe Glu Thr Leu Leu Asn Ala Glu Gln Gly Tyr
                805                 810                 815

Asp Ala Tyr Thr Arg Phe Tyr Gly Thr Gly Arg Ala Pro Ser Thr Ala
            820                 825                 830

Ser Gly Arg Ile Ser Tyr Leu Leu Asp Ile His Gly Pro Ser Ile Thr
        835                 840                 845

Val Asp Thr Ala Cys Ser Gly Gly Ile Val Cys Met Asp Gln Ala Ile
    850                 855                 860

Thr Phe Leu Gln Ser Gly Gly Ala Asp Thr Ala Ile Val Cys Ser Thr
865                 870                 875                 880

Asn Thr His Cys Trp Pro Gly Ser Phe Met Phe Leu Thr Ala Gln Gly
                885                 890                 895

Met Val Ser Pro Asn Gly Arg Cys Ala Thr Phe Thr Thr Asp Ala Asp
            900                 905                 910

Gly Tyr Val Pro Ser Glu Gly Ala Val Ala Phe Ile Leu Lys Thr Arg
        915                 920                 925

Ser Ala Ala Ile Arg Asp Asn Asp Asn Ile Leu Ala Val Ile Lys Ser
    930                 935                 940

Thr Asp Val Ser His Asn Gly Arg Ser Gln Gly Leu Val Ala Pro Asn
945                 950                 955                 960

Val Lys Ala Gln Ala Asn Leu His Arg Ser Leu Leu Arg Lys Ala Gly
                965                 970                 975

Leu Phe Pro Asp Gln Ile Asn Phe Ile Glu Ala His Gly Thr Gly Thr
            980                 985                 990

Ser Leu Gly Asp Leu Ser Glu Ile  Gln Gly Ile Asn Asn  Ala Tyr Ile
        995                 1000                 1005

Ser Ser  Arg Pro Arg Leu Ala  Gly Pro Leu Ile Ile  Ser Ala Ser
    1010                 1015                 1020

Lys Thr  Val Leu Gly His Ser  Glu Pro Thr Ala Gly  Met Ala Gly
    1025                 1030                 1035

Ile Leu  Thr Ala Leu Leu Ala  Leu Glu Lys Glu Thr  Val Pro Gly
    1040                 1045                 1050

Leu Asn His Leu Thr Asp His Asn Leu Asn Pro Ser Leu Asp Cys
    1055                1060                1065

Ser Val Val Pro Leu Leu Ile Pro His Glu Ser Ile His Ile Gly
    1070                1075                1080

Gly Ala Lys Pro His Arg Ala Ala Val Leu Ser Tyr Gly Phe Ala
    1085                1090                1095

Gly Thr Leu Ala Gly Ala Ile Leu Glu Gly Pro Pro Ser Gly Val
    1100                1105                1110

Pro Trp Pro Ser Ser Asn Asp Ile Gln Glu His Pro Met Ile Phe
    1115                1120                1125

Val Val Ser Gly Lys Thr Val Pro Thr Leu Glu Ala Tyr Leu Gly
    1130                1135                1140

Arg Tyr Leu Thr Phe Leu Arg Val Ala Lys Thr His Asp Phe Gln
    1145                1150                1155

Asp Ile Cys Tyr Thr Thr Cys Val Gly Arg Glu His Tyr Lys Tyr
    1160                1165                1170

Arg Phe Ser Cys Val Ala Arg Asn Met Ala Asp Leu Ile Ser Gln
    1175                1180                1185

Ile Glu His Arg Leu Thr Thr Leu Ser Thr Ser Lys Gln Lys Pro
    1190                1195                1200

Arg Gly Ser Leu Gly Phe Met Phe Ser Gly Gln Gly Thr Cys Phe
    1205                1210                1215

Pro Gly Met Ala Ser Ala Leu Ala Glu Gln Tyr Ser Gly Phe Arg
    1220                1225                1230

Met Leu Val Ser Lys Phe Gly Gln Ala Ala Gln Glu Arg Ser Gly
    1235                1240                1245

Tyr Pro Ile Asp Arg Leu Leu Leu Glu Val Ser Asp Thr Leu Pro
    1250                1255                1260

Glu Thr Asn Ser Glu Val Asp Gln Ile Cys Ile Phe Val Tyr Gln
    1265                1270                1275

Tyr Ser Val Leu Gln Trp Leu Gln Cys Leu Gly Ile Gln Pro Lys
    1280                1285                1290

Ala Val Leu Gly His Ser Leu Gly Glu Ile Thr Ala Ala Val Ala
    1295                1300                1305

Ala Gly Ala Leu Ser Phe Glu Ser Ala Leu Asp Leu Val Val Thr
    1310                1315                1320

Arg Ala Arg Leu Leu Arg Pro Arg Thr Lys Asp Ser Ala Gly Met
    1325                1330                1335

Ala Ala Val Ala Ala Ser Lys Glu Glu Val Glu Gly Val Ile Glu
    1340                1345                1350

Thr Leu Gln Leu Ala Asn Ser Leu Ser Val Ala Val His Asn Gly
    1355                1360                1365

Pro Arg Ser Val Val Val Ser Gly Ala Ser Ala Glu Ile Asp Ala
    1370                1375                1380

Leu Val Val Ala Ala Lys Glu Arg Gly Leu Lys Ala Ser Arg Leu
    1385                1390                1395

Lys Val Asp Gln Gly Phe His Ser Pro Tyr Val Asp Ser Ala Val
    1400                1405                1410

Pro Gly Leu Leu Asp Trp Ser Asn Lys His Arg Ser Thr Phe Phe
    1415                1420                1425

Pro Leu Asn Ile Pro Leu Tyr Ser Thr Leu Thr Gly Glu Leu Ile
    1430                1435                1440

Pro Lys Gly Arg Arg Phe Val Ser Asp His Trp Val Asn His Ala
    1445                1450                1455

Arg Lys Pro Val Gln Phe Ala Ala Ala Ala Ala Val Asp Glu
    1460                1465                1470

Asp Arg Ser Ile Gly Val Leu Val Asp Val Gly Pro Gln Pro Val
        1475                1480                1485

Ala Trp Thr Leu Leu Gln Ala Asn Asn Leu Leu Asn Thr Ser Ala
        1490                1495                1500

Val Ala Leu Ser Ala Lys Ala Gly Lys Asp Gln Glu Met Ala Leu
        1505                1510                1515

Leu Thr Ala Leu Ser Tyr Leu Phe Gln Glu His Asn Leu Ser Pro

1520                1525                1530

Asn Phe His Glu Leu Tyr Ser Gln Arg His Gly Thr Leu Gln Lys
    1535                1540                1545

Thr Asp Ile Pro Thr Tyr Pro Phe Gln Arg Val His Arg Tyr Pro
    1550                1555                1560

Thr Phe Ile Pro Ser Arg Asn Gln Ser Pro Ala Ile Ala Thr Val
    1565                1570                1575

Val Ile Pro Pro Pro Arg Phe Ser Val Gln Lys Ala Ala Asp Val
    1580                1585                1590

Ala Ser Gln Ser Lys Glu Ser Asp Cys Arg Ala Gly Leu Ile Ser
    1595                1600                1605

Cys Leu Arg Ala Ile Leu Glu Leu Thr Pro Glu Glu Glu Phe Asp
    1610                1615                1620

Leu Ser Glu Thr Leu Asn Ala Arg Gly Met Asp Ser Ile Met Phe
    1625                1630                1635

Ala Gln Leu Arg Lys Arg Val Gly Glu Glu Phe Asn Leu Asp Ile
    1640                1645                1650

Pro Met Ile Tyr Leu Ser Asp Val Phe Thr Met Glu Gln Met Val
    1655                1660                1665

Asp Tyr Leu Val Glu Gln Ser Gly Ser Thr Pro Ala Ser Lys His
    1670                1675                1680

Val Glu Thr Ser Ala Asn Gln Pro Leu Asp Glu Glu Asp Leu Arg
    1685                1690                1695

Thr Gly Leu Leu Ser Cys Leu Arg Asn Val Leu Glu Ile Thr Pro
    1700                1705                1710

Asp Glu Glu Leu Asp Leu Ser Glu Thr Leu Asn Ala Arg Gly Val
    1715                1720                1725

Asp Ser Ile Met Phe Ala Gln Leu Arg Lys Arg Val Gly Glu Gly
    1730                1735                1740

Phe Gly Val Glu Ile Pro Met Ile Tyr Leu Ser Asp Val Phe Thr
    1745                1750                1755

Met Glu Asp Met Ile Asn Phe Leu Val Ser Glu Arg Ser
        1760                1765                1770

<210> 50
<211> 5310
<212> DNA
<213> Armillaria ostoyae

<400> 50
atggaggccg acggtcacta ctctcttctc gatgtctttc tcagcgttgc acatgattct 60

gagaagtcca aacgtaatgt cttggaatgc ggccaggata cttggacata ctcggatttg 120

gacattatct cgtcggccct ggcacaggat ctcaaagcta tcttgggttg ttttcccaaa 180

gttgcagtcg tcagcgagaa ccatccctac gtatttgctc tcatgctggc cgtatggaag 240

cttgaaggga tattcatccc tatcgacgtc cacgttacag ctgaccttct aaagggcatg 300

ctacgcattg ttgctcccac ttgtctggtg atcccagaga ccgatatttt taaccagcgt 360

gttgcctctg caattggtat acatgttctc cccttcaacg tgaatgcgtc gaccatgact 420

gcacttcgac agaaatacca cccatttact cagaaagcct cgctatctgg gtgcgcactg 480

ccttacgttg atcgcgcatg cctctatctc tttacatcct ccgcgtcctc tactgccaat 540

ttgaaatgcg tacctttgac ccatactctt atcctcagta actgccgttc caagctcgca 600

tggtggcggc gcgttcgtcc ggaaggcgaa atggatgaga tacgtgttct agggtgggca 660

ccttggtcgc atatccttgc ctacatgcaa gatattggca cggcaacgct cctgaacgcc 720

ggttgctatg tctttgcgtc cgttccatcc acatatccta cacaactggc agcgaatggc 780

ttacaaggcc ctatcatgaa tatcatcgat tcacttcttg aacgacgagt cgccgcattt 840

gcttgcgtac cgttcatttt gagcgaacta aaagctatgt gcgagacggc ttccagtcca 900

gacgacaagc atcaaatgtg cttgagagct gaggagaaag ttcgccttgt cagtgcgctg 960

cagcggctta taatgctcga gtgcggaggc gctgcgcttg agtcaggtgt cacacgttgg 1020

gccgtcgaga atggcatatc agtcatggtc ggcatcggga tgacggagac agtcggtacg 1080

ctgtttgcag agcgcgcgca agacgcccgt tccaacggct attctgcgca ggacgccctc 1140

atttctgatg ggatcatgtc actggtcggg tctgacaacg aggaagccac cctcgaaggg 1200

gaactagtcg tcaagagcaa gctcatccct catggataca tcaagtaccg tgattcgtcg 1260

ttttcggtgg actcggacgg ctgggtaact ttcaaaacgg gagacaaata tcagcgcacg 1320

ccagatggac gattcaaatg gcttggaaga aagaccgatt ttattcagat gacaagcagc 1380

gaaacactgg atcccagacc cattgaggaa gccctctgtg cgaatccaag tatcgcaaat 1440

gcatgcgtca ttggtgacag gttcctgagg gagcctgcga ctagcgtatg cgccattgtc 1500

gagatcgggc cggaagtgga catgccttcg tccaagatcg acagagaaat tgcgaatacc 1560

ctcactccaa tcaatcgcgg ccttcctcct gctcttcgca tatcatggtc tcgcgtactc 1620

ataattcgac ctcctcagaa aataccagtt acgaggaaag gtgatgtgtt ccgtaagaag 1680

attgaagata tgtttgggtc tttccttggt gtcggcgttt ctaccgaggt cgaagtcgac 1740

catgaaacta aagaagatga tacgaaacac gtcgtgagac aggttgtaag caatcttctt 1800

ggagtccatg atcttgagtt attgtctgct ttatccttcg ccgagctggg catgacatca 1860

tttatggctg ttagcatcgt caacactcta aacaaacgca tcgacggcct cacccttcca 1920

cctaatgcat gctacatcca tattgatctt gattctcttg tggacgcgat ttcacttgaa 1980

catggtcatg aaagtatccc tgcagagttg ccttctaacc ctttccccgt tatcgagtcc 2040

catcaacata acgataagga cattgtgata gtcggcaagg cattccgttt acccggctca 2100

ctcaacaata ctgcatctct ctgggaagct ttgttatcga agaacagttc agtcatcagt 2160

gacatcccat ccgatcgctg ggatcacgca agcttttacc cccacgatat atgcttcacg 2220

aaagcaggcc tcgtcgatgt tgcacattac gactacagat tttttggcct cacggcgaca 2280

gaggcgttgt acgtatctcc gacgatgcgc ctcgccttgg aagtgtcatt tgaggccctg 2340

gagaacgcaa atattccact atccaagctg aaggggacgc aaaccgccgt ctatgtcgcc 2400

actaaagacg atggcttcga gacactctta aatgccgagc aaggctacga tgcatacacg 2460

cgattctacg gtacgggtcg tgctccgagt accgcaagcg gccgtataag ctatcttctt 2520

gatattcatg ggccatctgt caccgttgat acagcatgca gcggaggcat tgtatgtatg 2580

gatcaagcca tcactttctt gcaatccgga ggggccgata ccgctattgt ctgttcgagc 2640

aatacgcact gttggccggg atcattcatg ttcctgacgg cgcaaggcat ggtttctcaa 2700

aatggaagat gcgctacatt tactaccgat gcagacggat atgtaccttc ggagggtgca 2760

gtggctttca ttctcaagac gcgcagcgct gcgatacgcg acaacgacaa tatactcgcc 2820

gtgatcagat caacagacgt gtcccataac ggccgttctc aagggctagt tgcaccaaac 2880

gtaaaggcgc agacaaacct acaccggtcg ttgctacgaa aagctgggct gtttcctgat 2940

caaattaact ttatcgaagc tcatgggaca ggtacgtctc taggagacct ttcggaaatc 3000

cagggcatta ataacgccta cacctcaaca cgacctcgtc tggccggtcc ccttatcatt 3060

agcgcatcga aaacagtttt aggacacagt gaaccaacag cagggatggc cggcatcctc 3120

acagccttgc ttgcccttga gaaagagaca gttcctggtc taaatcactt aacggagcac 3180

aaccttaacc cttcgcttga ttgcagcgta gttcctctcc tgattcctca cgagtctatt 3240

cacattggtg gtgcaaagcc acatcgagct gcggttctgt catacggctt cgcgggtacg 3300

ctggccggtg ccatcttaga gggaccacct tctgatgtac caaggccgtc gtcaaataat 3360

atacaagaac accctatgat tttcgtcgtc agtgggaaaa ccgtgcctgc actggaagcg 3420

tacctaggac ggtatttggc atttttgcgg gtcgcaaaaa cacacgactt ccatgacatc 3480

tgctacacta cttgcgtcgg gagggagcac tacaaatacc ggttctcctg cgttgcccga 3540

aacatggcag accttatttc tcaaattgaa catcgactga cagctctttc cacttcgaaa 3600

cagaagcctc gcggctcgct agggttcata ttctcaggac aaggcactta tttccctggt 3660

atggctgcag cacttgccga acaatattcg gggttccgag tgctcgtctc taagtttggg 3720

caggctgccc aagagcggtc gggttatccg atcgataggc tgttgcttga agtttctgat 3780

acattgccag aaacaaacag cgaggtcgat caaatttgca tttttgtcta ccaatattct 3840

gttctgcaat ggctgcagag tctaggcatt caaccgaaag cagtcctcgg tcacagtctg 3900

ggagaaatta ctgcagcagt cgcagctggt gccctttcgt tcgaatctgc gttggacctt 3960

gtggtcaccc gtgctcgtct tctccgtcct agagcaaaag attctgcagg aatggccgca 4020

gtagcagcat ccaaggaaga agtcaaaggg cttatagaaa ccctccaact tgcggactcg 4080

ctgagcgttg cggttcataa cggtccgcgg agtgttgttg tgtcaggcgc atcagccgaa 4140

atcgacgccc tggttgtcgc agctaaagaa cggggcttga aggcctcccg cttaaaggtt 4200

gaccaaggct tccacagccc ttacgttgat tctgcggttc caggtttact cgactggtca 4260

aataagcatc gttcgacctt ccttcctttg aacattcctt tatactcgac tttgactggc 4320

gagcttattc cgaagggacg gaggttcgtc tcggatcact gggtaaacca tgctcgaaaa 4380

cctgtccagt ttgcggcggc agcggcagcc gtggatgaag accgatccat tggtgtgctc 4440

gttgacgttg gaccccaacc cgtcgcgtgg accctccttc aagcaaacaa ccttctcaat 4500

acctctgcag ttgcactatt cgcaaaggct ggaaaggatc aggagatggc gctgcttact 4560

gctttgagct acctcgtcca agagcacaac ctttctccca acttccatga gctttactct 4620

cagcgtcatg gtgctctgaa gaagacagac gttcccacct acccattccg ccgtgtccac 4680

cgctatccga ccttcatacc gtcacgaaat caaagtcctg ctgctgcgac ggtagctatg 4740

ccgccacccc gcttctctgt ccaaaagaat gcggatgtag catcacagtc gaaggaatca 4800

gactgtcgag ctggtttgat cagttgcctt agagccatcc tcgaattaac accggaagag 4860

gagtttgacc tttctgagac tctcaacgct cgtggtatgg attcgatcat gtttgcgcag 4920

ctacggaagc gggttgggga agaattcgac cttgacatac ccatgatcta tttatcagat 4980

gtgttcacga tggaacagat ggttgattac ctcgtcaaac agtccggatc cagacccgca 5040

ttaaaacacg cagaaattcc agttaatcaa ccattagacg aagatctccg gacgaggctc 5100

gtttcatgcc tgaggaacgt gctagaaatc acccccgatg aagaacttga cctatctgaa 5160

actttgaacg ctcgtggtgt tgactcgatc atgttcgctc agctacgaaa acgcgttggg 5220

gaaggatttg gtgtggaaat tccgatgata tatctgtccg acgtgtttac catggaagac 5280

atgatcaatt tcctcgtctc tgagcgctcg 5310

<210>  51
<211>  1770
<212>  PRT
<213>  Armillaria ostoyae

<400>  51

Met Glu Ala Asp Gly His Tyr Ser Leu Leu Asp Val Phe Leu Ser Val
1               5                   10                  15

Ala His Asp Ser Glu Lys Ser Lys Arg Asn Val Leu Glu Cys Gly Gln
            20                  25                  30

Asp Thr Trp Thr Tyr Ser Asp Leu Asp Ile Ile Ser Ser Ala Leu Ala
        35                  40                  45

Gln Asp Leu Lys Ala Ile Leu Gly Cys Phe Pro Lys Val Ala Val Val
    50                  55                  60

Ser Glu Asn His Pro Tyr Val Phe Ala Leu Met Leu Ala Val Trp Lys
65                  70                  75                  80

Leu Glu Gly Ile Phe Ile Pro Ile Asp Val His Val Thr Ala Asp Leu
                85                  90                  95

Leu Lys Gly Met Leu Arg Ile Val Ala Pro Thr Cys Leu Val Ile Pro
            100                 105                 110

Glu Thr Asp Ile Phe Asn Gln Arg Val Ala Ser Ala Ile Gly Ile His
        115                 120                 125

Val Leu Pro Phe Asn Val Asn Ala Ser Thr Met Thr Ala Leu Arg Gln
    130                 135                 140

Lys Tyr His Pro Phe Thr Gln Lys Ala Ser Leu Ser Gly Cys Ala Leu
145                 150                 155                 160

Pro Tyr Val Asp Arg Ala Cys Leu Tyr Leu Phe Thr Ser Ser Ala Ser
                165                 170                 175

Ser Thr Ala Asn Leu Lys Cys Val Pro Leu Thr His Thr Leu Ile Leu
            180                 185                 190

Ser Asn Cys Arg Ser Lys Leu Ala Trp Trp Arg Arg Val Arg Pro Glu
        195                 200                 205

Gly Glu Met Asp Glu Ile Arg Val Leu Gly Trp Ala Pro Trp Ser His
    210                 215                 220

Ile Leu Ala Tyr Met Gln Asp Ile Gly Thr Ala Thr Leu Leu Asn Ala
225                 230                 235                 240

Gly Cys Tyr Val Phe Ala Ser Val Pro Ser Thr Tyr Pro Thr Gln Leu
                245                 250                 255

Ala Ala Asn Gly Leu Gln Gly Pro Ile Met Asn Ile Ile Asp Ser Leu
            260                 265                 270

Leu Glu Arg Arg Val Ala Ala Phe Ala Cys Val Pro Phe Ile Leu Ser
        275                 280                 285

Glu Leu Lys Ala Met Cys Glu Thr Ala Ser Ser Pro Asp Asp Lys His
    290                 295                 300

Gln Met Cys Leu Arg Ala Glu Glu Lys Val Arg Leu Val Ser Ala Leu
305                 310                 315                 320

Gln Arg Leu Ile Met Leu Glu Cys Gly Gly Ala Ala Leu Glu Ser Gly
                325                 330                 335

Val Thr Arg Trp Ala Val Glu Asn Gly Ile Ser Val Met Val Gly Ile
            340                 345                 350

Gly Met Thr Glu Thr Val Gly Thr Leu Phe Ala Glu Arg Ala Gln Asp
        355                 360                 365

Ala Arg Ser Asn Gly Tyr Ser Ala Gln Asp Ala Leu Ile Ser Asp Gly
    370                 375                 380

Ile Met Ser Leu Val Gly Ser Asp Asn Glu Glu Ala Thr Leu Glu Gly
385                 390                 395                 400

Glu Leu Val Val Lys Ser Lys Leu Ile Pro His Gly Tyr Ile Lys Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Glu Ala Leu Cys Ala Asn Pro Ser Ile Ala Asn
465                 470                 475                 480

Ala Cys Val Ile Gly Asp Arg Phe Leu Arg Glu Pro Ala Thr Ser Val
                485                 490                 495

Cys Ala Ile Val Glu Ile Gly Pro Glu Val Asp Met Pro Ser Ser Lys
            500                 505                 510

Ile Asp Arg Glu Ile Ala Asn Thr Leu Thr Pro Ile Asn Arg Gly Leu
        515                 520                 525

Pro Pro Ala Leu Arg Ile Ser Trp Ser Arg Val Leu Ile Ile Arg Pro
    530                 535                 540

Pro Gln Lys Ile Pro Val Thr Arg Lys Gly Asp Val Phe Arg Lys Lys
545                 550                 555                 560

Ile Glu Asp Met Phe Gly Ser Phe Leu Gly Val Gly Val Ser Thr Glu
                565                 570                 575

Val Glu Val Asp His Glu Thr Lys Glu Asp Asp Thr Lys His Val Val
            580                 585                 590

Arg Gln Val Val Ser Asn Leu Leu Gly Val His Asp Leu Glu Leu Leu
        595                 600                 605

Ser Ala Leu Ser Phe Ala Glu Leu Gly Met Thr Ser Phe Met Ala Val
    610                 615                 620

Ser Ile Val Asn Thr Leu Asn Lys Arg Ile Asp Gly Leu Thr Leu Pro
625                 630                 635                 640

Pro Asn Ala Cys Tyr Ile His Ile Asp Leu Asp Ser Leu Val Asp Ala
                645                 650                 655

Ile Ser Leu Glu His Gly His Glu Ser Ile Pro Ala Glu Leu Pro Ser
            660                 665                 670

Asn Pro Phe Pro Val Ile Glu Ser His Gln His Asn Asp Lys Asp Ile
        675                 680                 685

Val Ile Val Gly Lys Ala Phe Arg Leu Pro Gly Ser Leu Asn Asn Thr
    690                 695                 700

Ala Ser Leu Trp Glu Ala Leu Leu Ser Lys Asn Ser Ser Val Ile Ser
705                 710                 715                 720

Asp Ile Pro Ser Asp Arg Trp Asp His Ala Ser Phe Tyr Pro His Asp
                725                 730                 735

Ile Cys Phe Thr Lys Ala Gly Leu Val Asp Val Ala His Tyr Asp Tyr
            740                 745                 750

Arg Phe Phe Gly Leu Thr Ala Thr Glu Ala Leu Tyr Val Ser Pro Thr
        755                 760                 765

Met Arg Leu Ala Leu Glu Val Ser Phe Glu Ala Leu Glu Asn Ala Asn
    770                 775                 780

Ile Pro Leu Ser Lys Leu Lys Gly Thr Gln Thr Ala Val Tyr Val Ala
785                 790                 795                 800

Thr Lys Asp Asp Gly Phe Glu Thr Leu Leu Asn Ala Glu Gln Gly Tyr
                805                 810                 815

Asp Ala Tyr Thr Arg Phe Tyr Gly Thr Gly Arg Ala Pro Ser Thr Ala
            820                 825                 830

Ser Gly Arg Ile Ser Tyr Leu Leu Asp Ile His Gly Pro Ser Val Thr
        835                 840                 845

Val Asp Thr Ala Cys Ser Gly Gly Ile Val Cys Met Asp Gln Ala Ile
    850                 855                 860

Thr Phe Leu Gln Ser Gly Gly Ala Asp Thr Ala Ile Val Cys Ser Ser
865                 870                 875                 880

Asn Thr His Cys Trp Pro Gly Ser Phe Met Phe Leu Thr Ala Gln Gly
                885                 890                 895

Met Val Ser Gln Asn Gly Arg Cys Ala Thr Phe Thr Thr Asp Ala Asp
            900                 905                 910

Gly Tyr Val Pro Ser Glu Gly Ala Val Ala Phe Ile Leu Lys Thr Arg
        915                 920                 925

Ser Ala Ala Ile Arg Asp Asn Asp Asn Ile Leu Ala Val Ile Arg Ser
    930                 935                 940

Thr Asp Val Ser His Asn Gly Arg Ser Gln Gly Leu Val Ala Pro Asn
945                 950                 955                 960

Val Lys Ala Gln Thr Asn Leu His Arg Ser Leu Leu Arg Lys Ala Gly
                965                 970                 975

Leu Phe Pro Asp Gln Ile Asn Phe Ile Glu Ala His Gly Thr Gly Thr

            980                 985                 990

Ser Leu Gly Asp Leu Ser Glu Ile Gln Gly Ile Asn Asn Ala Tyr Thr
        995                 1000                1005

Ser Thr Arg Pro Arg Leu Ala Gly Pro Leu Ile Ile Ser Ala Ser
    1010                1015                1020

Lys Thr Val Leu Gly His Ser Glu Pro Thr Ala Gly Met Ala Gly
    1025                1030                1035

Ile Leu Thr Ala Leu Leu Ala Leu Glu Lys Glu Thr Val Pro Gly
    1040                1045                1050

Leu Asn His Leu Thr Glu His Asn Leu Asn Pro Ser Leu Asp Cys
    1055                1060                1065

Ser Val Val Pro Leu Leu Ile Pro His Glu Ser Ile His Ile Gly
    1070                1075                1080

Gly Ala Lys Pro His Arg Ala Ala Val Leu Ser Tyr Gly Phe Ala
    1085                1090                1095

Gly Thr Leu Ala Gly Ala Ile Leu Glu Gly Pro Pro Ser Asp Val
    1100                1105                1110

Pro Arg Pro Ser Ser Asn Asn Ile Gln Glu His Pro Met Ile Phe
    1115                1120                1125

Val Val Ser Gly Lys Thr Val Pro Ala Leu Glu Ala Tyr Leu Gly
    1130                1135                1140

Arg Tyr Leu Ala Phe Leu Arg Val Ala Lys Thr His Asp Phe His
    1145                1150                1155

Asp Ile Cys Tyr Thr Thr Cys Val Gly Arg Glu His Tyr Lys Tyr
    1160                1165                1170

Arg Phe Ser Cys Val Ala Arg Asn Met Ala Asp Leu Ile Ser Gln
    1175                1180                1185

Ile Glu His Arg Leu Thr Ala Leu Ser Thr Ser Lys Gln Lys Pro
    1190                1195                1200

Arg Gly Ser Leu Gly Phe Ile Phe Ser Gly Gln Gly Thr Tyr Phe
    1205                1210                1215

Pro Gly Met Ala Ala Ala Leu Ala Glu Gln Tyr Ser Gly Phe Arg
    1220                1225                1230

Val Leu Val Ser Lys Phe Gly Gln Ala Ala Gln Glu Arg Ser Gly
    1235                1240                1245

Tyr Pro Ile Asp Arg Leu Leu Leu Glu Val Ser Asp Thr Leu Pro
    1250                1255                1260

Glu Thr Asn Ser Glu Val Asp Gln Ile Cys Ile Phe Val Tyr Gln
    1265                1270                1275

Tyr Ser Val Leu Gln Trp Leu Gln Ser Leu Gly Ile Gln Pro Lys
    1280                1285                1290

Ala Val Leu Gly His Ser Leu Gly Glu Ile Thr Ala Ala Val Ala
    1295                1300                1305

Ala Gly Ala Leu Ser Phe Glu Ser Ala Leu Asp Leu Val Val Thr
    1310                1315                1320

Arg Ala Arg Leu Leu Arg Pro Arg Ala Lys Asp Ser Ala Gly Met
    1325                1330                1335

Ala Ala Val Ala Ala Ser Lys Glu Glu Val Lys Gly Leu Ile Glu
    1340                1345                1350

Thr Leu Gln Leu Ala Asp Ser Leu Ser Val Ala Val His Asn Gly
    1355                1360                1365

Pro Arg Ser Val Val Val Ser Gly Ala Ser Ala Glu Ile Asp Ala
    1370                1375                1380

Leu Val Val Ala Ala Lys Glu Arg Gly Leu Lys Ala Ser Arg Leu
    1385                1390                1395

Lys Val Asp Gln Gly Phe His Ser Pro Tyr Val Asp Ser Ala Val
    1400                1405                1410

Pro Gly Leu Leu Asp Trp Ser Asn Lys His Arg Ser Thr Phe Leu
    1415                1420                1425

Pro Leu Asn Ile Pro Leu Tyr Ser Thr Leu Thr Gly Glu Leu Ile
    1430                1435                1440

Pro Lys Gly Arg Arg Phe Val Ser Asp His Trp Val Asn His Ala
    1445                1450                1455

Arg Lys Pro Val Gln Phe Ala Ala Ala Ala Ala Ala Val Asp Glu
    1460                1465                1470

Asp Arg Ser Ile Gly Val Leu Val Asp Val Gly Pro Gln Pro Val
    1475                1480                1485

Ala Trp Thr Leu Leu Gln Ala Asn Asn Leu Leu Asn Thr Ser Ala
    1490                1495                1500

Val Ala Leu Phe Ala Lys Ala Gly Lys Asp Gln Glu Met Ala Leu
    1505                1510                1515

Leu Thr Ala Leu Ser Tyr Leu Val Gln Glu His Asn Leu Ser Pro
    1520                1525                1530

Asn Phe His Glu Leu Tyr Ser Gln Arg His Gly Ala Leu Lys Lys
    1535                1540                1545

Thr Asp Val Pro Thr Tyr Pro Phe Arg Arg Val His Arg Tyr Pro
    1550                1555                1560

Thr Phe Ile Pro Ser Arg Asn Gln Ser Pro Ala Ala Ala Thr Val
    1565                1570                1575

Ala Met Pro Pro Pro Arg Phe Ser Val Gln Lys Asn Ala Asp Val
    1580                1585                1590

Ala Ser Gln Ser Lys Glu Ser Asp Cys Arg Ala Gly Leu Ile Ser
    1595                1600                1605

Cys Leu Arg Ala Ile Leu Glu Leu Thr Pro Glu Glu Glu Phe Asp
    1610                1615                1620

Leu Ser Glu Thr Leu Asn Ala Arg Gly Met Asp Ser Ile Met Phe
    1625                1630                1635

Ala Gln Leu Arg Lys Arg Val Gly Glu Glu Phe Asp Leu Asp Ile
    1640                1645                1650

Pro Met Ile Tyr Leu Ser Asp Val Phe Thr Met Glu Gln Met Val
    1655                1660                1665

Asp Tyr Leu Val Lys Gln Ser Gly Ser Arg Pro Ala Leu Lys His
    1670                1675                1680

Ala Glu Ile Pro Val Asn Gln Pro Leu Asp Glu Asp Leu Arg Thr
    1685                1690                1695

Arg Leu Val Ser Cys Leu Arg Asn Val Leu Glu Ile Thr Pro Asp
    1700                1705                1710

Glu Glu Leu Asp Leu Ser Glu Thr Leu Asn Ala Arg Gly Val Asp
    1715                1720                1725

Ser Ile Met Phe Ala Gln Leu Arg Lys Arg Val Gly Glu Gly Phe
    1730                1735                1740

Gly Val Glu Ile Pro Met Ile Tyr Leu Ser Asp Val Phe Thr Met
    1745                1750                1755

Glu Asp Met Ile Asn Phe Leu Val Ser Glu Arg Ser
    1760                1765                1770

<210> 52
<211> 5322
<212> DNA
<213> Armillaria fuscipes

<400> 52
   atgaccatgg aggtcgacag ccactattct ctgctcgatg tctttctcag cattgcacat 60
   gattctgaca agtccaaacg caatgtcttg gaatgcggcc tggaggcctg gacatactcg 120
   gatttagaca tcatctcgtc ggctctggca caggatctca aagctacctt gggatgtttt 180
   cccaaagttg cagtcgtcag cgagaaccat ccctacgtgt ttgctctcat gctggccgtc 240
   tggaagctcg aaggatcttt catccccata gacgtccacg ttacagctga ccttttaaag 300
   ggcatgctac gcattgttgc tcctacttgt ctagtgatcc ctgagagcga tgtttccaac 360
   cggcgtgttg cctctgcgat tggtatacgt gttctcccat ttgatgcgaa ttcgtcaacc 420
   atgacggcac ttcgacaaaa gtacgaatca ttcactcaga aagcctcgcc atctgagtgc 480
   acacttgccc acgccgatcg tacatgcctc tatcttttta catcctctgc atcctctacc 540
   gccaacttga aatgtgtgcc tttgacccat actcttatcc tcaataactg ccgtaccaag 600
   ctcgcatggt ggcagcgctt tcgtccagaa agcgaaatgg atgggatgcg tgttctaggg 660
   tgggcacctt ggtcgcatat ccttgcctac atgcaagata tcggcacagc gacgctcctg 720
   aacgccggtt gctatgtctt tgcgtccatt ccatccacat accctacaca actagcagca 780
   aatggcttac aaggccccac tatgaatatc atcaacgcac ttcttgaacg acaaattgcc 840
   gcatttgctt gcgtgccgtt cattttgagc gaactcaaag ctatgtgcga gatgacttcc 900
   tgtacaggaa accaaatgtc cctaagagcc gaggagaaag ttcgcctggt cagggtgctg 960
   caggggcttg taatgctcga gtgtggaggc gcggcacttg agtcagatgt tacgcgttgg 1020
   gtcgttgaga atgacatacc agtcatggtt ggcattggga tgacggaaac agtcggtacg 1080
   ctgttcgcag agcgcgccca agacgtccgt tccagcggat attccgccca agacgctctc 1140
   attgctgatg gcattatgtc actggtcggt tctgacaacg aggaagccac tttcgaaggg 1200
   gaactagtcg tgaagagcaa gctcatccca caggatacaa tagggtaccg tgattcatcg 1260
   ttctcggtgg actcagatgg ctgggtaacg ttcaaaactg gagataaata tcagcgcact 1320
   ccagatggac gattaaaatg gcttgggaga aagaccgact ttatccagat gacaagcagc 1380
   gaaacactgg atcccaggcc cattgagcaa actctttgtg cgaatccata cgtcgcaaaa 1440
   gcatgcgtca ttggtgacag attcctgaga gatcccgcga ccagcgtatg tgccataatt 1500
   gagatcaggc cggaagtgga catgccttcg tccaagatcg acagagaaat tgcgaatgcc 1560
   cttgctccaa tcaatcgcga cctccctcct gctcttcgca tatcatggtc tcgcgtactc 1620
   atgatcagac cccctcagaa aatcccagta acgaggaaag gcgatgtgtt ccgtaagaag 1680
   attgaagata tgtttgggtc tttcctcggt gtcggtgttt ctactgaggt cggagtcgac 1740
   catgaaactg aaaaagatga tacggaacac attgtgagac aggttgttac taatcttctt 1800
   ggagtccatg atcccgagct attgtctgct ttatctttcg ctgagcttgg aatgacttca 1860
   tttatggctg tcagcatcgt caactctcta aacaagtaca tcgatggcct cacccttcca 1920
   cctaatgcat gttacatcca tattgatctt gattctcttg tggaggccat ttcacgtgaa 1980
   cggggtcatg gaagtaacgc cacagagttg ccttctcaac ccgtccctgt tgagttacat 2040
   caacctaacg ataaggacgt tgtgatagtt ggcaaggcat tccgtttacc tggctcactc 2100
   gacagcactg catctctatg ggaagctttg ttatcaaaga acaattcagt cgtcagtgaa 2160
   atcccatccg atcgctggga tcacgcaagc ttttaccccc acgacatttg cttcacgaaa 2220
   gcaggcctcg tcgatgttgc ccattacgat tacagattct ttggcctcac ggctacagag 2280
   gcattgtatg tatctccgac gatgcgtctc gccttggaag tgtcatttga agccctggag 2340
   aatgcgaata ttccgttatc caatctgaag ggaacacaaa ccgctgtcta tgtcgccacc 2400
   aaagacgatg gtttcgagac acttttaaat gccgagcagg gctatgatgc ctacacacga 2460
   ttctatggca cgggccgcgc tccaagcacc gcaagtggcc gtataagcta tctacttgat 2520
   attcatgggc catctgtcac cgttgataca gcatgcagcg gaggcattgt gtgtatggat 2580
   caagccatca ctttcttgca gtccggaggg gcagatacag ctattgtctg ttcgagcaat 2640
   acgcattgtt ggcctggatc atttatgttc ctgacggcgc aaggcatggt ttctccaaat 2700
   ggaagatgcg ctacatttag taccgatgca gacggatatg tgccttcaga gggcgcagta 2760
   gctttcgttc tcaagacgcg tagcgcagca atacgcgata atgacaatat cctcgccgta 2820
   atcaaatcaa cagatgtgtc tcataacggc cgttctcaag ggctggttgc acctaacgtg 2880
   aaggcgcaga caaacctgca tcaatcgttg ttacgaaaag ctgggctgtt tcctgatcaa 2940
   atcaacttta tcgaagccca tggaacaggt acatctctag gagacctctc agaaatccag 3000
   ggcatcaata acgcctacac ctcaacacga cctcgtctag acggtcccct tatcatcagc 3060
   gcatcgaaaa cagtgatagg acacagcgaa ccaactgcag ggatggcggg catcctcaca 3120
   gccttgcttg ctcttgagaa agaaacagtt cctggtctca atcacttaac ggagcacagc 3180
   cttaaccctt cgcttgattg cagcatagtc ccgctcctga ttcctcacga gtctattcac 3240
   attggtgggg taaagccaca tcgagctgcg gttctgtcat acggcttcgc gggtacactg 3300
   gcgggtgcta tcttagaggg accgccttca gatgcaccaa ggccgtcgtc aaataatgtg 3360
   caagatcacc ctatgatttt tgccctcagt gggaaaagcg cgtccgcact ggaagcatac 3420
   ctaaggcggt atttggcatt cctgcggatt gcagatccac acgacttcca taacatctgc 3480
   tacacttcct gtgtcgggag ggagcactac aaatatcggt tctcctgtgt tgcccgaaac 3540
   atggcagacc ttatatctca aattgaacat cgactgacaa ctgtttccat tccgaaaccg 3600
   aaacctcgtg gctcaatagg attcacgttc tcaggacaag gcacttattt ccctggcatg 3660
   gccgcagcac tcactgaaca atattctgga ttccggacgc tcgtctctaa gcttgggcag 3720
   gctgcgcaag agcggtcggg tcatccgatt gacaggctgt tacttgaagt ttccggtaca 3780
   tcaccagaaa caaacagtga ggtcgagcaa atttgcacat ttatctacca atatgccgtt 3840
   ctgcaatggt tgcagagcct aggcgttcaa ccgaaagcag tcctcggtca cagcctggga 3900
   gaaattactg ccgcagtcgc agctggtgcc ctgtcgttcg aatccgcgtt ggaccttgtg 3960
   gtgacccgtg ctcgtcttct ccgtcccgaa acaaaagatt ctgctgggat ggtcgcggta 4020
   gcaacgtcca aggatgaagt tgaaggactt atagaaacac tccaagttgc ggacgcgcta 4080
   agcgttgccg ttcacaacgg ttcacggagt gttgtggttt caggcacatc agcggaagtt 4140
   gatgccctgg tcgtcgcagc taaagaacgg ggcttaaagg cttcccgctt aagagtcgac 4200
   caaggtttcc acagcccttg cgttgattct gccgttcctg gtttactcga ctggtcaaat 4260
   gagcatcgtt ccaccttcct tcctttgaat atgcctttat actcgacttt gaccggcgag 4320
   gtcattccca agggacggaa attcgtctgg gatcactggg taaaccatgc tcgaaaacct 4380
   gttcagtttg caccggcagc aaaagcggtg gacgaagacc gatccatcgg tgtgctcgtt 4440
   gatgtaggac ctcaacctgt cgcttggacc cttttgcaag caaacaacct ttccaacacc 4500
   tctacggttg cgctattcgc gaaagccgga aaggatcagg agatggcact gctcactgct 4560
   ttgagctacc tcttccaaga gcacaacctt tctcccaagt ttcacgacct ttatactggg 4620
   tataatggtg ctctgaagaa gacggacatt cccacgtacc cattccaacg tgtccatcgc 4680
   tatcccacct tcataccatc acgaaatcag agtcctgctg tcgcgaaagc agtcgtgcag 4740
   ccgccccgct tctctatcca aaggaatcga gaagccacat tacagtcgaa ggaaccagat 4800
   caccgagctt gtttagtcac ttgccttaga gccatcctcg aattaacatc agaggaagaa 4860
   cttgacctct ctgagaccct caacgctcgt ggcgtggact cgatcatgtt ttcacagcta 4920
   cggaagcggg ttggagaaga attcaatctc gagataccca tgatctattt atcagacgta 4980
   ttcacgatgg agcagatgat tgactacctc gtcgaacagt ccggatccaa tcccgcatca 5040
   aagcaagtag gaactccggt taaccgacta tcaggcgaag aagatcttcg gacggggctc 5100
   atctcatgcc tgagggacgt gctagaaatc actcctgatg atgaacttga tcacccaaaa 5160
   gacctatctg aaactttaaa tgctcgtggt gttgattcga taatgttcgc tcagctacga 5220
   aaacgcgtcg gggaaggatt tggtgtggaa attccgatga tatatctgtc tgatgtgttt 5280
   accatggaag acatgattaa tttcctcgtt tctgagcgct ca 5322

<210> 53
<211> 1774
<212> PRT
<213> Armillaria fuscipes

<400> 53

Met Thr Met Glu Val Asp Ser His Tyr Ser Leu Leu Asp Val Phe Leu
1               5                   10                  15

Ser Ile Ala His Asp Ser Asp Lys Ser Lys Arg Asn Val Leu Glu Cys
            20                  25                  30

Gly Leu Glu Ala Trp Thr Tyr Ser Asp Leu Asp Ile Ile Ser Ser Ala
        35                  40                  45

Leu Ala Gln Asp Leu Lys Ala Thr Leu Gly Cys Phe Pro Lys Val Ala
    50                  55                  60

Val Val Ser Glu Asn His Pro Tyr Val Phe Ala Leu Met Leu Ala Val
65                  70                  75                  80

Trp Lys Leu Glu Gly Ile Phe Ile Pro Ile Asp Val His Val Thr Ala
                85                  90                  95

Asp Leu Leu Lys Gly Met Leu Arg Ile Val Ala Pro Thr Cys Leu Val
            100                 105                 110

Ile Pro Glu Ser Asp Val Ser Asn Arg Arg Val Ala Ser Ala Ile Gly
        115                 120                 125

Ile Arg Val Leu Pro Phe Asp Ala Asn Ser Ser Thr Met Thr Ala Leu
    130                 135                 140

Arg Gln Lys Tyr Glu Ser Phe Thr Gln Lys Ala Ser Pro Ser Glu Cys
145                 150                 155                 160

Thr Leu Ala His Ala Asp Arg Thr Cys Leu Tyr Leu Phe Thr Ser Ser
                165                 170                 175

Ala Ser Ser Thr Ala Asn Leu Lys Cys Val Pro Leu Thr His Thr Leu
            180                 185                 190

Ile Leu Asn Asn Cys Arg Thr Lys Leu Ala Trp Trp Gln Arg Phe Arg
        195                 200                 205

Pro Glu Ser Glu Met Asp Gly Met Arg Val Leu Gly Trp Ala Pro Trp
    210                 215                 220

Ser His Ile Leu Ala Tyr Met Gln Asp Ile Gly Thr Ala Thr Leu Leu
225                 230                 235                 240

Asn Ala Gly Cys Tyr Val Phe Ala Ser Ile Pro Ser Thr Tyr Pro Thr
                245                 250                 255

Gln Leu Ala Ala Asn Gly Leu Gln Gly Pro Thr Met Asn Ile Ile Asn
            260                 265                 270

Ala Leu Leu Glu Arg Gln Ile Ala Ala Phe Ala Cys Val Pro Phe Ile
        275                 280                 285

Leu Ser Glu Leu Lys Ala Met Cys Glu Met Thr Ser Cys Thr Gly Asn
    290                 295                 300

Gln Met Ser Leu Arg Ala Glu Glu Lys Val Arg Leu Val Arg Val Leu
305                 310                 315                 320

Gln Gly Leu Val Met Leu Glu Cys Gly Gly Ala Ala Leu Glu Ser Asp
                325                 330                 335

Val Thr Arg Trp Val Val Glu Asn Asp Ile Pro Val Met Val Gly Ile
            340                 345                 350

Gly Met Thr Glu Thr Val Gly Thr Leu Phe Ala Glu Arg Ala Gln Asp
        355                 360                 365

Val Arg Ser Ser Gly Tyr Ser Ala Gln Asp Ala Leu Ile Ala Asp Gly
    370                 375                 380

Ile Met Ser Leu Val Gly Ser Asp Asn Glu Glu Ala Thr Phe Glu Gly
385                 390                 395                 400

Glu Leu Val Val Lys Ser Lys Leu Ile Pro Gln Gly Tyr Ile Gly Tyr

                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Leu Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Gln Thr Leu Cys Ala Asn Pro Tyr Val Ala Lys
465                 470                 475                 480

Ala Cys Val Ile Gly Asp Arg Phe Leu Arg Asp Pro Ala Thr Ser Val
                485                 490                 495

Cys Ala Ile Ile Glu Ile Arg Pro Glu Val Asp Met Pro Ser Ser Lys
            500                 505                 510

Ile Asp Arg Glu Ile Ala Asn Ala Leu Ala Pro Ile Asn Arg Asp Leu
        515                 520                 525

Pro Pro Ala Leu Arg Ile Ser Trp Ser Arg Val Leu Met Ile Arg Pro
    530                 535                 540

Pro Gln Lys Ile Pro Val Thr Arg Lys Gly Asp Val Phe Arg Lys Lys
545                 550                 555                 560

Ile Glu Asp Met Phe Gly Ser Phe Leu Gly Val Gly Val Ser Thr Glu
                565                 570                 575

Val Gly Val Asp His Glu Thr Glu Lys Asp Asp Thr Glu His Ile Val
            580                 585                 590

Arg Gln Val Val Thr Asn Leu Leu Gly Val His Asp Pro Glu Leu Leu
        595                 600                 605

Ser Ala Leu Ser Phe Ala Glu Leu Gly Met Thr Ser Phe Met Ala Val
    610                 615                 620

Ser Ile Val Asn Ser Leu Asn Lys Tyr Ile Asp Gly Leu Thr Leu Pro
625                 630                 635                 640

Pro Asn Ala Cys Tyr Ile His Ile Asp Leu Asp Ser Leu Val Glu Ala
                645                 650                 655

Ile Ser Arg Glu Arg Gly His Gly Ser Asn Ala Thr Glu Leu Pro Ser
        660                 665                 670

Gln Pro Val Pro Val Glu Leu His Gln Pro Asn Asp Lys Asp Val Val
    675                 680                 685

Ile Val Gly Lys Ala Phe Arg Leu Pro Gly Ser Leu Asp Ser Thr Ala
    690                 695                 700

Ser Leu Trp Glu Ala Leu Leu Ser Lys Asn Asn Ser Val Val Ser Glu
705                 710                 715                 720

Ile Pro Ser Asp Arg Trp Asp His Ala Ser Phe Tyr Pro His Asp Ile
                725                 730                 735

Cys Phe Thr Lys Ala Gly Leu Val Asp Val Ala His Tyr Asp Tyr Arg
            740                 745                 750

Phe Phe Gly Leu Thr Ala Thr Glu Ala Leu Tyr Val Ser Pro Thr Met
        755                 760                 765

Arg Leu Ala Leu Glu Val Ser Phe Glu Ala Leu Glu Asn Ala Asn Ile
    770                 775                 780

Pro Leu Ser Asn Leu Lys Gly Thr Gln Thr Ala Val Tyr Val Ala Thr
785                 790                 795                 800

Lys Asp Asp Gly Phe Glu Thr Leu Leu Asn Ala Glu Gln Gly Tyr Asp
                805                 810                 815

Ala Tyr Thr Arg Phe Tyr Gly Thr Gly Arg Ala Pro Ser Thr Ala Ser
            820                 825                 830

Gly Arg Ile Ser Tyr Leu Leu Asp Ile His Gly Pro Ser Val Thr Val
        835                 840                 845

Asp Thr Ala Cys Ser Gly Gly Ile Val Cys Met Asp Gln Ala Ile Thr
    850                 855                 860

Phe Leu Gln Ser Gly Gly Ala Asp Thr Ala Ile Val Cys Ser Ser Asn
865                 870                 875                 880

Thr His Cys Trp Pro Gly Ser Phe Met Phe Leu Thr Ala Gln Gly Met
                885                 890                 895

Val Ser Pro Asn Gly Arg Cys Ala Thr Phe Ser Thr Asp Ala Asp Gly
            900                 905                 910

Tyr Val Pro Ser Glu Gly Ala Val Ala Phe Val Leu Lys Thr Arg Ser
        915                 920                 925

Ala Ala Ile Arg Asp Asn Asp Asn Ile Leu Ala Val Ile Lys Ser Thr
    930                 935                 940

Asp Val Ser His Asn Gly Arg Ser Gln Gly Leu Val Ala Pro Asn Val
945                 950                 955                 960

Lys Ala Gln Thr Asn Leu His Gln Ser Leu Leu Arg Lys Ala Gly Leu
                965                 970                 975

Phe Pro Asp Gln Ile Asn Phe Ile Glu Ala His Gly Thr Gly Thr Ser
            980                 985                 990

Leu Gly Asp Leu Ser Glu Ile Gln Gly Ile Asn Asn Ala Tyr Thr Ser
        995                 1000                1005

Thr Arg Pro Arg Leu Asp Gly Pro Leu Ile Ile Ser Ala Ser Lys
    1010                1015                1020

Thr Val Ile Gly His Ser Glu Pro Thr Ala Gly Met Ala Gly Ile
    1025                1030                1035

Leu Thr Ala Leu Leu Ala Leu Glu Lys Glu Thr Val Pro Gly Leu
    1040                1045                1050

Asn His Leu Thr Glu His Ser Leu Asn Pro Ser Leu Asp Cys Ser
    1055                1060                1065

Ile Val Pro Leu Leu Ile Pro His Glu Ser Ile His Ile Gly Gly
    1070                1075                1080

Val Lys Pro His Arg Ala Ala Val Leu Ser Tyr Gly Phe Ala Gly
    1085                1090                1095

Thr Leu Ala Gly Ala Ile Leu Glu Gly Pro Pro Ser Asp Ala Pro
    1100                1105                1110

Arg Pro Ser Ser Asn Asn Val Gln Asp His Pro Met Ile Phe Ala
    1115                1120                1125

Leu Ser Gly Lys Ser Ala Ser Ala Leu Glu Ala Tyr Leu Arg Arg
    1130                1135                1140

Tyr Leu Ala Phe Leu Arg Ile Ala Asp Pro His Asp Phe His Asn
    1145                1150                1155

Ile Cys Tyr Thr Ser Cys Val Gly Arg Glu His Tyr Lys Tyr Arg
    1160                1165                1170

Phe Ser Cys Val Ala Arg Asn Met Ala Asp Leu Ile Ser Gln Ile
    1175                1180                1185

Glu His Arg Leu Thr Thr Val Ser Ile Pro Lys Pro Lys Pro Arg
    1190                1195                1200

Gly Ser Ile Gly Phe Thr Phe Ser Gly Gln Gly Thr Tyr Phe Pro
    1205                1210                1215

Gly Met Ala Ala Ala Leu Thr Glu Gln Tyr Ser Gly Phe Arg Thr
    1220                1225                1230

Leu Val Ser Lys Leu Gly Gln Ala Ala Gln Glu Arg Ser Gly His
    1235                1240                1245

Pro Ile Asp Arg Leu Leu Leu Glu Val Ser Gly Thr Ser Pro Glu
    1250                1255                1260

Thr Asn Ser Glu Val Glu Gln Ile Cys Thr Phe Ile Tyr Gln Tyr
    1265                1270                1275

Ala Val Leu Gln Trp Leu Gln Ser Leu Gly Val Gln Pro Lys Ala
    1280                1285                1290

Val Leu Gly His Ser Leu Gly Glu Ile Thr Ala Ala Val Ala Ala
    1295                1300                1305

Gly Ala Leu Ser Phe Glu Ser Ala Leu Asp Leu Val Val Thr Arg
    1310                1315                1320

Ala Arg Leu Leu Arg Pro Glu Thr Lys Asp Ser Ala Gly Met Val
    1325                1330                1335

Ala Val Ala Thr Ser Lys Asp Glu Val Glu Gly Leu Ile Glu Thr
    1340                1345                1350

Leu Gln Val Ala Asp Ala Leu Ser Val Ala Val His Asn Gly Ser
    1355                1360                1365

Arg Ser Val Val Val Ser Gly Thr Ser Ala Glu Val Asp Ala Leu
    1370                1375                1380

Val Val Ala Ala Lys Glu Arg Gly Leu Lys Ala Ser Arg Leu Arg

1385                1390                1395

Val Asp  Gln Gly Phe His Ser  Pro Cys Val Asp Ser  Ala Val Pro
    1400                1405                1410

Gly Leu  Leu Asp Trp Ser Asn  Glu His Arg Ser Thr  Phe Leu Pro
    1415                1420                1425

Leu Asn  Met Pro Leu Tyr Ser  Thr Leu Thr Gly Glu  Val Ile Pro
    1430                1435                1440

Lys Gly  Arg Lys Phe Val Trp  Asp His Trp Val Asn  His Ala Arg
    1445                1450                1455

Lys Pro  Val Gln Phe Ala Pro  Ala Ala Lys Ala Val  Asp Glu Asp
    1460                1465                1470

Arg Ser  Ile Gly Val Leu Val  Asp Val Gly Pro Gln  Pro Val Ala
    1475                1480                1485

Trp Thr  Leu Leu Gln Ala Asn  Asn Leu Ser Asn Thr  Ser Thr Val
    1490                1495                1500

Ala Leu  Phe Ala Lys Ala Gly  Lys Asp Gln Glu Met  Ala Leu Leu
    1505                1510                1515

Thr Ala  Leu Ser Tyr Leu Phe  Gln Glu His Asn Leu  Ser Pro Lys
    1520                1525                1530

Phe His  Asp Leu Tyr Thr Gly  Tyr Asn Gly Ala Leu  Lys Lys Thr
    1535                1540                1545

Asp Ile  Pro Thr Tyr Pro Phe  Gln Arg Val His Arg  Tyr Pro Thr
    1550                1555                1560

Phe Ile  Pro Ser Arg Asn Gln  Ser Pro Ala Val Ala  Lys Ala Val
    1565                1570                1575

Val Gln  Pro Pro Arg Phe Ser  Ile Gln Arg Asn Arg  Glu Ala Thr
    1580                1585                1590

Leu Gln  Ser Lys Glu Pro Asp  His Arg Ala Cys Leu  Val Thr Cys
    1595                1600                1605

Leu Arg  Ala Ile Leu Glu Leu  Thr Ser Glu Glu Glu  Leu Asp Leu
    1610                1615                1620

Ser Glu Thr Leu Asn Ala Arg Gly Val Asp Ser Ile Met Phe Ser
    1625                1630                1635

Gln Leu Arg Lys Arg Val Gly Glu Glu Phe Asn Leu Glu Ile Pro
    1640                1645                1650

Met Ile Tyr Leu Ser Asp Val Phe Thr Met Glu Gln Met Ile Asp
    1655                1660                1665

Tyr Leu Val Glu Gln Ser Gly Ser Asn Pro Ala Ser Lys Gln Val
    1670                1675                1680

Gly Thr Pro Val Asn Arg Leu Ser Gly Glu Glu Asp Leu Arg Thr
    1685                1690                1695

Gly Leu Ile Ser Cys Leu Arg Asp Val Leu Glu Ile Thr Pro Asp
    1700                1705                1710

Asp Glu Leu Asp His Pro Lys Asp Leu Ser Glu Thr Leu Asn Ala
    1715                1720                1725

Arg Gly Val Asp Ser Ile Met Phe Ala Gln Leu Arg Lys Arg Val
    1730                1735                1740

Gly Glu Gly Phe Gly Val Glu Ile Pro Met Ile Tyr Leu Ser Asp
    1745                1750                1755

Val Phe Thr Met Glu Asp Met Ile Asn Phe Leu Val Ser Glu Arg
    1760                1765                1770

Ser

<210>  54
<211>  5304
<212>  DNA
<213>  Armillaria mellea

<400>  54
atggaggcca acggtcacta ctctcttctc gatgtctttc tcagcattgc acacgattct      60

gacaagtcca aacgtaatgt cttggaatgc ggtcaggata cctggacata ctcagatttg     120

gacattatct cgtcggccct ggcacaggat ctccaagcta cgctgggttg ttttcccaaa     180

gttgcagtcg tcagcgagaa ccatccctac atttttgctc tcatgctggc cgtttggaag     240

ctcgaaggga tattcatccc tgtcgacgtc catgttacag ctgaccttct aaagggcatg     300

ttacatatcg tcgctcccac ttgtctggtg atccctgaga ccgatatttc caaccagcgt     360

attgcttccg cgattggtat acatgttctc cccttcagtg cgaatgcgtc gaccatgact     420

gcacttcgac agaaatacga cctatgcatt cagaaagcct cgctatctga gcgcgcactt 480

cctcacgttg atcgcgcttg cctctatctc tttacatcct ctgcgtcctc tactgccaac 540

ttgaaatgcg tacctttgac ccatagtctt atcctcagta actgccgttc caaactcgca 600

tggtggcggc gcgttcgtcc agaggaagaa atggatggga tacgtgttct agggtgggca 660

ccttggtccc atatccttgc ctacatgcaa gatattggca cagcgacgct cctgaacgcc 720

ggttgctacg tctttgcgtc aattccgtcc acatatccta cacaactggt ggtgaatggc 780

ttacaaggcc ccaccatgaa tatcatcgac tcacttctta aacggcgaat cgtcgcattt 840

gcttgtgtcc cgttcatttt gggcgaacta aaagctatgt gcgagacggc ttcgggtcca 900

gatgtcaagc atcatatggg cttgagagct gaggagaagg ttcgccttgt tagggcactg 960

cagcagctta tgatgctcga gtgcggaggc gctgcgctcg agtcagatgt cacgcgttgg 1020

gttgtcgaaa atggcatatc ggtcatggtt ggcatcggga tgacggagac agttggtacg 1080

ctgtttgcag agcgcgcgca agacgcccgt tccaacggct actctgcgca gaacgccctc 1140

attactgatg gcattatgtc actggtcggg cctgacaacg aggaagtcac ctttgaaggg 1200

gaactagtcg tgaagagcaa gctcatccca cacgaataca tcaattaccg tgattcgtcg 1260

ttctcggtgg actcggatgg ctgggtaacg tttaaaacgg gagataaata tcagcgcaca 1320

ccagatggac gattcaaatg gcttggaaga aagaccgact ttattcagat gacaagcagt 1380

gaaacactgg atcccagacc cattgagaaa accctctgtg cgaatccaag tattgtaaat 1440

gcatgcgtca ttggtgacag attcctgagg gagcctgcaa ctagcgtatg cgccattgtc 1500

gagatcaggc cggaagtgga catcccttcg tccaagatcg acagggaaat tgcgaatgcc 1560

cttgctccaa tcaatcgcga cctccctcct gctcttcgca tatcatggtc tcgcgtactc 1620

ataattcgac ctcctcagaa aataccagtt acaaggaaag gcgatgtgtt ccgtaagaag 1680

attgaagatg tatttgggtc tttccttggt gtcggttcta ccgagatcaa agtcgaccat 1740

gaatctaaag aagatgatac ggaacacatt gtgagacagg ttgtcagcaa tcttcttgga 1800

gtccatgatc ctgagctatt gtctgcttta tctttcgctg agctgggaat gacctcgttt 1860

atagctgtta gtattgtcaa cgctctaaac aagcgcatca gcggcctcac ccttccacct 1920

aatgcgtgct acctccatat tgatcttgat tctcttgtga acgccatttc acgtgaacat 1980

ggtcatggaa ctaaccccgc agagttgcct tctaaacttt tccccgtcac cgagtcctat 2040

caacgtaatg ataaggacgt tgtgataatc ggcaaagcat tccgtttacc aggctcactc 2100

gacaatactg catctctctg ggaagctttg ttatcgaaga acaattcagt cgtcagtgac 2160

atcccatccg atcgctggga tcacgcaagc ttttaccccc acgatatatg cttcacgaaa 2220

gcaggcctcg tcgatgttgc acattacgat tacagattct ttgggctcac agcgacagag 2280

gcattgtacg tatctccgac gatgcgcctc gccttggaag tgtcatttga agcccttgag 2340

aacgcgaata ttccgctatc caagctgaag gggacacaaa cccctgtcta tgtcgccact 2400

aaagacgatg gctttgagac actcttaaat gctgagcaag gctacgatgc ttacacgcga 2460

ttctatggta caggtcgcgc tccgagcacc gcaagtggtc gtataagcta tctactcgat 2520

attcatgggc catctgttac cgttgataca gcatgcagcg gaggcattgt atgtatggat 2580

caagccatca ctttcttgca atccggaagg gccgataccg ctattgtctg ttcgagcaat 2640

acgcactgtt ggccgggatc atttatgttc ctgacggcgc agggcatggt ttctccacat 2700

ggaagatgcg ctacatttac taccgatgca gacggttatg taccttcgga gggtgctgtg 2760

gctttcattc tcaagacgcg cagtgctgca atacgcgata atgacaatat actcgccgtg 2820

atcaaatcaa cagatgtgtc ccataacggc cgttctcaag ggctagttgc accaaacgta 2880

aaggcgcaga caagcctaca ccgatcgttg ctacgaaaag ctggactatt tcctgatcaa 2940

atcaatttta tcgaagccca tggaacaggt acatctctag gagacctctc ggaaatccag 3000

ggcatcaaca acgcctacac ctcaacacga cctcgtttgg acggtcccct tatcattagc 3060

gcgtcgaaaa cagtgttggg acacagcgaa ccaattgcgg ggatggccgg catcctcaca 3120

gccttgcttt ccctcgagaa agaaacagtt tttggtttaa atcacttaac agagcacaac 3180

cttaaccctt cgcttgattg cagcctagtt cctctcctga ttcctcacga gtctattcac 3240

attggtggtg aaaaaccaca tcgagctgcg gttctgtcat acggtttcgc gggtacgctg 3300

gccggtgcca tcttagaggg accaccttca gatgtaccaa ggccgtcgtc aagcgatatg 3360

caagaacacc ctatggtttt cgtcctcagt gggaaaagcg tgcctgcact ggaaacgtac 3420

ctaagacggt atttggcatt tttgcgcgcc gcaaaaacaa acgacttcca tagcatctgc 3480

tacaccactt gcgtcgggag ggagcattac aaataccggt tctcctgcgt tgcccaaaat 3540

atggcagacc ttgtgtctca aattgaacat cgactgacaa ctctttccaa ttcgaaacag 3600

aaacctcgtg gctcgccagg gtttatgttc tcaggacaag gcacttattt ccctggtatg 3660

gctgcagcgc ttgctgaaca atatttgggg tttcgagtgc tagtctctag gtttgggaag 3720

gctgctcaag agcggtcggg ttatccgatc gataggctgt tgcttgaagt ttctgataca 3780

tcatcagaaa caaacagcga ggctgaccaa atttgcattt ttgtctacca atattccgtt 3840

ctgcaatggc tgcagagtct aggcattcaa ccgaaagcag tcctcggtca cagcctggga 3900

gaaattaccg ccgcagtcgc agctggtgcc ctttcattcg aatctgcgtt ggaccttgta 3960

gtcacccgtg ctcgtcttct ccgtcctgaa acaaaagatt cagcaggaat ggccgcagta 4020

gcagcatcca aggaagaagt tgaagaactt atagaaaacc tccaacttgc gcatgcgtta 4080

agtgttgcgg ttcacaacgg tccacggagt gttgttgtgt caggcgcatc gaccgaaatt 4140

gatgccctgg tcgtcgcagc taaagaacgg ggcttgaagg cttcccgctt aagagttgac 4200

caaggcttcc atagccctta cgttgattct gccgttccgg gtttactcga ctggtcaagt 4260

gaacattgtt cgaccttcct tcctttaaat attcctttat actcgacttt gaccggcgaa 4320

gttattccaa agggacggag gttcgcctgg gatcactggg taaaccatgc tcgaaaacct 4380

gttcagtttg cggcggcggc agcagcggtg gacgaagacc gatccatcgg cgtgctcctt 4440

gatgttggac cccaacccgt tgcgtggacc atccttcaag caagcagcct tttcaacacc 4500

tctgcagttg cgctatttgc gaaggctgga aaggatcagg agatggcgct gcttactact 4560

ttgagctacc tcttccaaga gcacaatctt tgtcccaact ttcacgagct ttactctcag 4620

cgtcatggtg ctcttaagaa gacggacatt cccacctacc cattccaacg tgtccaccgc 4680

tatccaacct tcataccatc acgaaatcaa agtcctgctg tcgcgaaggt agtagtccca 4740

tcgcgctttc ctgtccaaag gaaaggggaa gcaatatcac aatcgaacga atcagattac 4800

cgagctggtt tgatcacttg cctcagaacc atcctcgaat taacatcaga agaagagttt 4860

gacctttctg agaccctcaa cgctcgtggt gtggattcga tcatgttttc acagctacgg 4920

aagcgggttg gggaagaatt cgatctcgat atacccatga tctatttatc agacgtgttc 4980

acgatggaac agatgatcga ctacctcgtc gaacagtccg gatccagacc cgcgccaaag 5040

cacgtagaaa ctccggttaa cgaaccttta ggcaaagatc tccggacggg gctcgtttca 5100

tgcctgagga atgtactaga aatcaccccc gatgaagaac tcgacctatc tgaaactttg 5160

aacgctcgtg gtgtcgactc gatcatgttc gctcagctac gaaaacgcgt cggggaagga 5220

tttggcgtgg aaattccgat gatatatctg tctgacgtgt ttactatgga agacatgatc 5280

aatttcctcg tctctgagca cgcg 5304

<210> 55
<211> 1768
<212> PRT
<213> Armillaria mellea

<400> 55

Met Glu Ala Asn Gly His Tyr Ser Leu Leu Asp Val Phe Leu Ser Ile
1               5                   10                  15

Ala His Asp Ser Asp Lys Ser Lys Arg Asn Val Leu Glu Cys Gly Gln
            20                  25                  30

Asp Thr Trp Thr Tyr Ser Asp Leu Asp Ile Ile Ser Ser Ala Leu Ala
        35                  40                  45

Gln Asp Leu Gln Ala Thr Leu Gly Cys Phe Pro Lys Val Ala Val Val
    50                  55                  60

Ser Glu Asn His Pro Tyr Ile Phe Ala Leu Met Leu Ala Val Trp Lys
65                  70                  75                  80

Leu Glu Gly Ile Phe Ile Pro Val Asp Val His Val Thr Ala Asp Leu
                85                  90                  95

Leu Lys Gly Met Leu His Ile Val Ala Pro Thr Cys Leu Val Ile Pro
            100                 105                 110

Glu Thr Asp Ile Ser Asn Gln Arg Ile Ala Ser Ala Ile Gly Ile His
        115                 120                 125

Val Leu Pro Phe Ser Ala Asn Ala Ser Thr Met Thr Ala Leu Arg Gln
    130                 135                 140

Lys Tyr Asp Leu Cys Ile Gln Lys Ala Ser Leu Ser Glu Arg Ala Leu
145                 150                 155                 160

Pro His Val Asp Arg Ala Cys Leu Tyr Leu Phe Thr Ser Ser Ala Ser
                165                 170                 175

Ser Thr Ala Asn Leu Lys Cys Val Pro Leu Thr His Ser Leu Ile Leu
            180                 185                 190

Ser Asn Cys Arg Ser Lys Leu Ala Trp Trp Arg Arg Val Arg Pro Glu
        195                 200                 205

Glu Glu Met Asp Gly Ile Arg Val Leu Gly Trp Ala Pro Trp Ser His
    210                 215                 220

Ile Leu Ala Tyr Met Gln Asp Ile Gly Thr Ala Thr Leu Leu Asn Ala
225                 230                 235                 240

Gly Cys Tyr Val Phe Ala Ser Ile Pro Ser Thr Tyr Pro Thr Gln Leu
                245                 250                 255

Val Val Asn Gly Leu Gln Gly Pro Thr Met Asn Ile Ile Asp Ser Leu
            260                 265                 270

Leu Lys Arg Arg Ile Val Ala Phe Ala Cys Val Pro Phe Ile Leu Gly
        275                 280                 285

Glu Leu Lys Ala Met Cys Glu Thr Ala Ser Gly Pro Asp Val Lys His
    290                 295                 300

His Met Gly Leu Arg Ala Glu Glu Lys Val Arg Leu Val Arg Ala Leu
305                 310                 315                 320

Gln Gln Leu Met Met Leu Glu Cys Gly Gly Ala Ala Leu Glu Ser Asp
                325                 330                 335

Val Thr Arg Trp Val Val Glu Asn Gly Ile Ser Val Met Val Gly Ile
            340                 345                 350

Gly Met Thr Glu Thr Val Gly Thr Leu Phe Ala Glu Arg Ala Gln Asp
        355                 360                 365

Ala Arg Ser Asn Gly Tyr Ser Ala Gln Asn Ala Leu Ile Thr Asp Gly
    370                 375                 380

Ile Met Ser Leu Val Gly Pro Asp Asn Glu Glu Val Thr Phe Glu Gly
385                 390                 395                 400

Glu Leu Val Val Lys Ser Lys Leu Ile Pro His Glu Tyr Ile Asn Tyr
                405                 410                 415

Arg Asp Ser Ser Phe Ser Val Asp Ser Asp Gly Trp Val Thr Phe Lys
            420                 425                 430

Thr Gly Asp Lys Tyr Gln Arg Thr Pro Asp Gly Arg Phe Lys Trp Leu
        435                 440                 445

Gly Arg Lys Thr Asp Phe Ile Gln Met Thr Ser Ser Glu Thr Leu Asp
    450                 455                 460

Pro Arg Pro Ile Glu Lys Thr Leu Cys Ala Asn Pro Ser Ile Val Asn
465                 470                 475                 480

Ala Cys Val Ile Gly Asp Arg Phe Leu Arg Glu Pro Ala Thr Ser Val
                485                 490                 495

Cys Ala Ile Val Glu Ile Arg Pro Glu Val Asp Ile Pro Ser Ser Lys
            500                 505                 510

Ile Asp Arg Glu Ile Ala Asn Ala Leu Ala Pro Ile Asn Arg Asp Leu
        515                 520                 525

Pro Pro Ala Leu Arg Ile Ser Trp Ser Arg Val Leu Ile Ile Arg Pro
    530                 535                 540

Pro Gln Lys Ile Pro Val Thr Arg Lys Gly Asp Val Phe Arg Lys Lys
545                 550                 555                 560

Ile Glu Asp Val Phe Gly Ser Phe Leu Gly Val Gly Ser Thr Glu Ile
                565                 570                 575

Lys Val Asp His Glu Ser Lys Glu Asp Asp Thr Glu His Ile Val Arg
            580                 585                 590

Gln Val Val Ser Asn Leu Leu Gly Val His Asp Pro Glu Leu Leu Ser
        595                 600                 605

Ala Leu Ser Phe Ala Glu Leu Gly Met Thr Ser Phe Ile Ala Val Ser
    610                 615                 620

Ile Val Asn Ala Leu Asn Lys Arg Ile Ser Gly Leu Thr Leu Pro Pro
625                 630                 635                 640

Asn Ala Cys Tyr Leu His Ile Asp Leu Asp Ser Leu Val Asn Ala Ile
                645                 650                 655

Ser Arg Glu His Gly His Gly Thr Asn Pro Ala Glu Leu Pro Ser Lys
            660                 665                 670

Leu Phe Pro Val Thr Glu Ser Tyr Gln Arg Asn Asp Lys Asp Val Val
        675                 680                 685

Ile Ile Gly Lys Ala Phe Arg Leu Pro Gly Ser Leu Asp Asn Thr Ala
    690                 695                 700

Ser Leu Trp Glu Ala Leu Leu Ser Lys Asn Asn Ser Val Val Ser Asp
705                 710                 715                 720

Ile Pro Ser Asp Arg Trp Asp His Ala Ser Phe Tyr Pro His Asp Ile
                725                 730                 735

Cys Phe Thr Lys Ala Gly Leu Val Asp Val Ala His Tyr Asp Tyr Arg
            740                 745                 750

Phe Phe Gly Leu Thr Ala Thr Glu Ala Leu Tyr Val Ser Pro Thr Met
        755                 760                 765

Arg Leu Ala Leu Glu Val Ser Phe Glu Ala Leu Glu Asn Ala Asn Ile
    770                 775                 780

Pro Leu Ser Lys Leu Lys Gly Thr Gln Thr Pro Val Tyr Val Ala Thr
785                 790                 795                 800

Lys Asp Asp Gly Phe Glu Thr Leu Leu Asn Ala Glu Gln Gly Tyr Asp
                805                 810                 815

Ala Tyr Thr Arg Phe Tyr Gly Thr Gly Arg Ala Pro Ser Thr Ala Ser

            820                 825                 830

Gly Arg Ile Ser Tyr Leu Leu Asp Ile His Gly Pro Ser Val Thr Val
        835                 840                 845

Asp Thr Ala Cys Ser Gly Gly Ile Val Cys Met Asp Gln Ala Ile Thr
    850                 855                 860

Phe Leu Gln Ser Gly Arg Ala Asp Thr Ala Ile Val Cys Ser Ser Asn
865                 870                 875                 880

Thr His Cys Trp Pro Gly Ser Phe Met Phe Leu Thr Ala Gln Gly Met
                885                 890                 895

Val Ser Pro His Gly Arg Cys Ala Thr Phe Thr Thr Asp Ala Asp Gly
            900                 905                 910

Tyr Val Pro Ser Glu Gly Ala Val Ala Phe Ile Leu Lys Thr Arg Ser
        915                 920                 925

Ala Ala Ile Arg Asp Asn Asp Asn Ile Leu Ala Val Ile Lys Ser Thr
    930                 935                 940

Asp Val Ser His Asn Gly Arg Ser Gln Gly Leu Val Ala Pro Asn Val
945                 950                 955                 960

Lys Ala Gln Thr Ser Leu His Arg Ser Leu Leu Arg Lys Ala Gly Leu
                965                 970                 975

Phe Pro Asp Gln Ile Asn Phe Ile Glu Ala His Gly Thr Gly Thr Ser
            980                 985                 990

Leu Gly Asp Leu Ser Glu Ile Gln Gly Ile Asn Asn Ala Tyr Thr Ser
        995                 1000                1005

Thr Arg Pro Arg Leu Asp Gly Pro Leu Ile Ile Ser Ala Ser Lys
    1010                1015                1020

Thr Val Leu Gly His Ser Glu Pro Ile Ala Gly Met Ala Gly Ile
    1025                1030                1035

Leu Thr Ala Leu Leu Ser Leu Glu Lys Glu Thr Val Phe Gly Leu
    1040                1045                1050

Asn His Leu Thr Glu His Asn Leu Asn Pro Ser Leu Asp Cys Ser
    1055                1060                1065

Leu Val Pro Leu Leu Ile Pro His Glu Ser Ile His Ile Gly Gly
    1070                1075                1080

Glu Lys Pro His Arg Ala Ala Val Leu Ser Tyr Gly Phe Ala Gly
    1085                1090                1095

Thr Leu Ala Gly Ala Ile Leu Glu Gly Pro Pro Ser Asp Val Pro
    1100                1105                1110

Arg Pro Ser Ser Ser Asp Met Gln Glu His Pro Met Val Phe Val
    1115                1120                1125

Leu Ser Gly Lys Ser Val Pro Ala Leu Glu Thr Tyr Leu Arg Arg
    1130                1135                1140

Tyr Leu Ala Phe Leu Arg Ala Ala Lys Thr Asn Asp Phe His Ser
    1145                1150                1155

Ile Cys Tyr Thr Thr Cys Val Gly Arg Glu His Tyr Lys Tyr Arg
    1160                1165                1170

Phe Ser Cys Val Ala Gln Asn Met Ala Asp Leu Val Ser Gln Ile
    1175                1180                1185

Glu His Arg Leu Thr Thr Leu Ser Asn Ser Lys Gln Lys Pro Arg
    1190                1195                1200

Gly Ser Pro Gly Phe Met Phe Ser Gly Gln Gly Thr Tyr Phe Pro
    1205                1210                1215

Gly Met Ala Ala Ala Leu Ala Glu Gln Tyr Leu Gly Phe Arg Val
    1220                1225                1230

Leu Val Ser Arg Phe Gly Lys Ala Ala Gln Glu Arg Ser Gly Tyr
    1235                1240                1245

Pro Ile Asp Arg Leu Leu Leu Glu Val Ser Asp Thr Ser Ser Glu
    1250                1255                1260

Thr Asn Ser Glu Ala Asp Gln Ile Cys Ile Phe Val Tyr Gln Tyr
    1265                1270                1275

Ser Val Leu Gln Trp Leu Gln Ser Leu Gly Ile Gln Pro Lys Ala
    1280                1285                1290

Val Leu Gly His Ser Leu Gly Glu Ile Thr Ala Ala Val Ala Ala
    1295                1300                1305

Gly Ala Leu Ser Phe Glu Ser Ala Leu Asp Leu Val Val Thr Arg
    1310                1315                1320

Ala Arg Leu Leu Arg Pro Glu Thr Lys Asp Ser Ala Gly Met Ala
    1325                1330                1335

Ala Val Ala Ala Ser Lys Glu Glu Val Glu Glu Leu Ile Glu Asn
    1340                1345                1350

Leu Gln Leu Ala His Ala Leu Ser Val Ala Val His Asn Gly Pro
    1355                1360                1365

Arg Ser Val Val Val Ser Gly Ala Ser Thr Glu Ile Asp Ala Leu
    1370                1375                1380

Val Val Ala Ala Lys Glu Arg Gly Leu Lys Ala Ser Arg Leu Arg
    1385                1390                1395

Val Asp Gln Gly Phe His Ser Pro Tyr Val Asp Ser Ala Val Pro
    1400                1405                1410

Gly Leu Leu Asp Trp Ser Ser Glu His Cys Ser Thr Phe Leu Pro
    1415                1420                1425

Leu Asn Ile Pro Leu Tyr Ser Thr Leu Thr Gly Glu Val Ile Pro
    1430                1435                1440

Lys Gly Arg Arg Phe Ala Trp Asp His Trp Val Asn His Ala Arg
    1445                1450                1455

Lys Pro Val Gln Phe Ala Ala Ala Ala Ala Ala Val Asp Glu Asp
    1460                1465                1470

Arg Ser Ile Gly Val Leu Leu Asp Val Gly Pro Gln Pro Val Ala
    1475                1480                1485

Trp Thr Ile Leu Gln Ala Ser Ser Leu Phe Asn Thr Ser Ala Val
    1490                1495                1500

Ala Leu Phe Ala Lys Ala Gly Lys Asp Gln Glu Met Ala Leu Leu
    1505                1510                1515

Thr Thr Leu Ser Tyr Leu Phe Gln Glu His Asn Leu Cys Pro Asn
    1520                1525                1530

Phe His Glu Leu Tyr Ser Gln Arg His Gly Ala Leu Lys Lys Thr
    1535                1540                1545

Asp Ile Pro Thr Tyr Pro Phe Gln Arg Val His Arg Tyr Pro Thr
    1550                1555                1560

Phe Ile Pro Ser Arg Asn Gln Ser Pro Ala Val Ala Lys Val Val
    1565                1570                1575

Val Pro Ser Arg Phe Pro Val Gln Arg Lys Gly Glu Ala Ile Ser
    1580                1585                1590

Gln Ser Asn Glu Ser Asp Tyr Arg Ala Gly Leu Ile Thr Cys Leu
    1595                1600                1605

Arg Thr Ile Leu Glu Leu Thr Ser Glu Glu Glu Phe Asp Leu Ser
    1610                1615                1620

Glu Thr Leu Asn Ala Arg Gly Val Asp Ser Ile Met Phe Ser Gln
    1625                1630                1635

Leu Arg Lys Arg Val Gly Glu Glu Phe Asp Leu Asp Ile Pro Met
    1640                1645                1650

Ile Tyr Leu Ser Asp Val Phe Thr Met Glu Gln Met Ile Asp Tyr
    1655                1660                1665

Leu Val Glu Gln Ser Gly Ser Arg Pro Ala Pro Lys His Val Glu
    1670                1675                1680

Thr Pro Val Asn Glu Pro Leu Gly Lys Asp Leu Arg Thr Gly Leu
    1685                1690                1695

Val Ser Cys Leu Arg Asn Val Leu Glu Ile Thr Pro Asp Glu Glu
    1700                1705                1710

Leu Asp Leu Ser Glu Thr Leu Asn Ala Arg Gly Val Asp Ser Ile
    1715                1720                1725

Met Phe Ala Gln Leu Arg Lys Arg Val Gly Glu Gly Phe Gly Val
    1730                1735                1740

Glu Ile Pro Met Ile Tyr Leu Ser Asp Val Phe Thr Met Glu Asp
    1745                1750                1755

Met Ile Asn Phe Leu Val Ser Glu His Ala
    1760                1765

<210> 56

<211>  40
<212>  PRT
<213>  Artificial sequence

<220>
<223>  Consensus amino acid sequence of 1 hispidin synthases


<220>
<221>  MISC_FEATURE
<223>  X - any amino acid

<220>
<221>  MISC_FEATURE
<222>  (1)..(40)
<223>  X - any amino acid

<400>  56

Val Ala Xaa Xaa Xaa Glu Asn His Pro Xaa Xaa Xaa Ala Leu Xaa Xaa
1               5                   10                  15


Ala Val Trp Lys Xaa Xaa Gly Xaa Phe Xaa Pro Xaa Asp Xaa His Xaa
            20                  25                  30


Xaa Xaa Xaa Xaa Xaa Xaa Gly Met
        35                  40


<210>  57
<211>  36
<212>  PRT
<213>  Artificial sequence

<220>
<223>  Consensus amino acid sequence of 2 hispidin synthases


<220>
<221>  MISC_FEATURE
<222>  (1)..(36)
<223>  X - any amino acid

<400>  57

Leu Gly Trp Ala Pro Xaa Ser His Xaa Leu Xaa Xaa Met Gln Asp Ile
1               5                   10                  15


Gly Xaa Xaa Xaa Xaa Leu Xaa Ala Gly Cys Tyr Val Phe Xaa Xaa Xaa
            20                  25                  30


Pro Xaa Xaa Tyr
        35


<210>  58
<211>  33
<212>  PRT
<213>  Artificial sequence

<220>
<223>  Consensus amino acid sequence of 3 hispidin synthases


<220>
<221>  MISC_FEATURE
<222>  (6)..(33)
<223>  X - any amino acid

<400>  58

Glu Cys Gly Gly Ala Xaa Leu Xaa Xaa Xaa Xaa Xaa Xaa Trp Xaa Xaa
1               5                   10                  15


Xaa Xaa Xaa Xaa Xaa Xaa Xaa Xaa Gly Xaa Gly Met Thr Glu Thr Xaa
            20                  25                  30


Gly




<210>  59
<211>  8
<212>  PRT
<213>  Artificial sequence

<220>
<223>  Consensus amino acid sequence of 4 hispidin synthases

<400>  59

Phe Ala Glu Leu Gly Met Thr Ser
1               5


<210>  60
<211>  25
<212>  PRT
<213>  Artificial sequence

<220>
<223>  Consensus amino acid sequence of 5 hispidin synthases


<220>
<221>  MISC_FEATURE
<222>  (2)..(24)
<223>  X - any amino acid

<400>  60

Ile Xaa Xaa Xaa Arg Trp Asp His Xaa Ser Phe Tyr Pro Xaa Xaa Ile
1               5                   10                  15


Xaa Xaa Xaa Xaa Ala Gly Leu Xaa Asp
            20                  25


<210>  61

<211>  65
<212>  PRT
<213>  Artificial sequence

<220>
<223>  Consensus amino acid sequence of 6 hispidin synthases


<220>
<221>  MISC_FEATURE
<222>  (2)..(64)
<223>  X - any amino acid

<400>  61

Asp Xaa Xaa Phe Phe Gly Xaa Xaa Xaa Xaa Glu Ala Xaa Xaa Xaa Ser
1               5                   10                  15


Pro Thr Met Arg Xaa Ala Leu Glu Val Xaa Xaa Glu Ala Leu Glu Xaa
            20                  25                  30


Ala Asn Ile Pro Xaa Xaa Xaa Xaa Lys Gly Xaa Xaa Xaa Xaa Val Xaa
        35                  40                  45


Xaa Ala Xaa Xaa Asp Asp Gly Phe Glu Thr Leu Leu Xaa Ala Xaa Xaa
    50                  55                  60


Gly
65


<210>  62
<211>  50
<212>  PRT
<213>  Artificial sequence

<220>
<223>  Consensus amino acid sequence of 7 hispidin synthases


<220>
<221>  MISC_FEATURE
<222>  (5)..(47)
<223>  X - any amino acid

<400>  62

Ala Asp Gly Tyr Xaa Pro Ser Glu Gly Ala Val Xaa Phe Xaa Leu Lys
1               5                   10                  15


Thr Xaa Xaa Ala Ala Xaa Arg Asp Xaa Xaa Xaa Ile Xaa Ala Xaa Ile
            20                  25                  30


Xaa Xaa Xaa Xaa Xaa Xaa His Asn Gly Arg Ser Gln Gly Leu Xaa Ala
        35                  40                  45

Pro Asn
    50


<210>  63
<211>  9
<212>  PRT
<213>  Artificial sequence

<220>
<223>  Consensus amino acid sequence of 8 hispidin synthases


<220>
<221>  MISC_FEATURE
<222>  (8)..(8)
<223>  X - any amino acid

<400>  63

Gly His Ser Leu Gly Glu Ile Xaa Ala
1               5


<210>  64
<211>  867
<212>  DNA
<213>  Neonothopanus nambi

<400>  64
atggcgccaa tttcttcaac ttggtctcgt ctcattcgat ttgtggctat tgaaacgtcc      60

ctcgtgcata tcggtgaacc gatagacgcc accatggacg tcggtctggc gagacgagaa     120

ggcaagacga tccaagcata cgagattatt ggatcaggct cggctctaga cctctcagcc     180

caagtatcga agaatgtgct gactgtaagg gaactcctga tgccgctttc aagagaggaa     240

attaaaactg tacgatgctt ggggttgaac taccctgttc atgccaccga agcgaacgtt     300

gctgttccaa aattcccgaa tttgttctac aaaccagtga cctcgctcat tggccccgat     360

ggactcatta ccatcccttc cgttgtccaa cccccgaagg agcatcagtc cgattatgaa     420

gcggaacttg tcattgtcat cgggaaagca gcaaagaatg tatcggagga tgaggctttg     480

gattatgtat tgggatacac tgccgcgaac gatatttcgt ttaggaaaca ccagctagca     540

gtctcacaat ggtctttctc gaaaggattt ggtagccttc tactcactat ccgtatggca     600

caaacccact cgggtaacat taatcgcttc tccagagacc agattttcaa tgtcaagaag     660

acaatttcct tcctgtcaca aggcactaca ctggaaccag gttctatcat tttgactggt     720

acacctgacg gagtgggctt tgtgcgcaat ccaccacttt accttaaaga tggagatgaa     780

gtaatgacct ggattggaag tggaatcgga acattagcca atacagtgca agaagagaag     840

acttgcttcg ctagtggcgg acacgag                                         867


<210>  65
<211>  289

<212>  PRT
<213>  Neonothopanus nambi

<400>  65

Met Ala Pro Ile Ser Ser Thr Trp Ser Arg Leu Ile Arg Phe Val Ala
1               5                   10                  15

Ile Glu Thr Ser Leu Val His Ile Gly Glu Pro Ile Asp Ala Thr Met
            20                  25                  30

Asp Val Gly Leu Ala Arg Arg Glu Gly Lys Thr Ile Gln Ala Tyr Glu
        35                  40                  45

Ile Ile Gly Ser Gly Ser Ala Leu Asp Leu Ser Ala Gln Val Ser Lys
    50                  55                  60

Asn Val Leu Thr Val Arg Glu Leu Leu Met Pro Leu Ser Arg Glu Glu
65                  70                  75                  80

Ile Lys Thr Val Arg Cys Leu Gly Leu Asn Tyr Pro Val His Ala Thr
                85                  90                  95

Glu Ala Asn Val Ala Val Pro Lys Phe Pro Asn Leu Phe Tyr Lys Pro
            100                 105                 110

Val Thr Ser Leu Ile Gly Pro Asp Gly Leu Ile Thr Ile Pro Ser Val
        115                 120                 125

Val Gln Pro Pro Lys Glu His Gln Ser Asp Tyr Glu Ala Glu Leu Val
    130                 135                 140

Ile Val Ile Gly Lys Ala Ala Lys Asn Val Ser Glu Asp Glu Ala Leu
145                 150                 155                 160

Asp Tyr Val Leu Gly Tyr Thr Ala Ala Asn Asp Ile Ser Phe Arg Lys
                165                 170                 175

His Gln Leu Ala Val Ser Gln Trp Ser Phe Ser Lys Gly Phe Gly Ser
            180                 185                 190

Leu Leu Leu Thr Ile Arg Met Ala Gln Thr His Ser Gly Asn Ile Asn
        195                 200                 205

Arg Phe Ser Arg Asp Gln Ile Phe Asn Val Lys Lys Thr Ile Ser Phe
    210                 215                 220

Leu Ser Gln Gly Thr Thr Leu Glu Pro Gly Ser Ile Ile Leu Thr Gly
225                 230                 235                 240

Thr Pro Asp Gly Val Gly Phe Val Arg Asn Pro Pro Leu Tyr Leu Lys
                245                 250                 255

Asp Gly Asp Glu Val Met Thr Trp Ile Gly Ser Gly Ile Gly Thr Leu
            260                 265                 270

Ala Asn Thr Val Gln Glu Glu Lys Thr Cys Phe Ala Ser Gly Gly His
        275                 280                 285

Glu

<210>  66
<211>  963
<212>  DNA
<213>  Neonothopanus gardneri

<400>  66
atggcgccga ttttgacagt gagttccagt tcaatggtgc tgaatagctc aaaacaggct      60

atattgaata tttctgaata tcaccagccc tggactcgtc tcattcgatt tgtagccgtt     120

gagacgtcac tcgtgcatat tggtgaaccc atcgaggtga ctttggacgt cgggcaggca     180

aaatgtgaag gcaagacgat caaagcgtac gagattattg gatcagggtc ggccttggac     240

ctctcagctc aggtatcgaa gaatgtgcta accgtaaagg aactcttgat gccgctttcg     300

agagaagagg tcaagactgt gcggtgcttg ggactgaact attttactca tgcttccacc     360

gggcgcccgc tgtcgactag attaccgact ttgttctata agccagtgac ttcactcatc     420

ggacctgagg cgttcattaa tattccttcc gctgttcaac caccgaagga gcatcagtcc     480

gattatgaag cggagttggt aattattatt gggagagcgg cgaaggatgt accggaagag     540

gaggctttga attatgtttt gggatacacc gccgccaacg acatttcatt taggaaatat     600

caatttgcag tttcccagtg gtgtttttcg aaaggtttcg ataatacaaa cccaatcggt     660

ccgtgcatcg tttccgcatc ttccattccg aacccgcaag acatccagat ccaatgcaaa     720

ctgaacggga atgtcgttca gaatggaaac accagtgatc aaatttttaa tatcaagaaa     780

acagtcgctt ttttgtcgca aggaacaaca cttgagtcag gatcaatcat cctgaccggt     840

acgcctggcg gagtgggatt tgtgcgcgat ccaccgcttt accttaaaga tggagatgaa     900

gtagtgactt ggattggaag tggggttgga agtttagtca acgtagtaaa agaagagaag     960

act                                                                   963

<210>  67
<211>  321
<212>  PRT
<213>  Neonothopanus gardneri

<400>  67

Met Ala Pro Ile Leu Thr Val Ser Ser Ser Ser Met Val Leu Asn Ser
1               5                   10                  15

Ser Lys Gln Ala Ile Leu Asn Ile Ser Glu Tyr His Gln Pro Trp Thr
            20                  25                  30

Arg Leu Ile Arg Phe Val Ala Val Glu Thr Ser Leu Val His Ile Gly
        35                  40                  45

Glu Pro Ile Glu Val Thr Leu Asp Val Gly Gln Ala Lys Cys Glu Gly
    50                  55                  60

Lys Thr Ile Lys Ala Tyr Glu Ile Ile Gly Ser Gly Ser Ala Leu Asp
65                  70                  75                  80

Leu Ser Ala Gln Val Ser Lys Asn Val Leu Thr Val Lys Glu Leu Leu
                85                  90                  95

Met Pro Leu Ser Arg Glu Glu Val Lys Thr Val Arg Cys Leu Gly Leu
            100                 105                 110

Asn Tyr Phe Thr His Ala Ser Thr Gly Arg Pro Leu Ser Thr Arg Leu
        115                 120                 125

Pro Thr Leu Phe Tyr Lys Pro Val Thr Ser Leu Ile Gly Pro Glu Ala
    130                 135                 140

Phe Ile Asn Ile Pro Ser Ala Val Gln Pro Pro Lys Glu His Gln Ser
145                 150                 155                 160

Asp Tyr Glu Ala Glu Leu Val Ile Ile Ile Gly Arg Ala Ala Lys Asp
                165                 170                 175

Val Pro Glu Glu Glu Ala Leu Asn Tyr Val Leu Gly Tyr Thr Ala Ala
            180                 185                 190

Asn Asp Ile Ser Phe Arg Lys Tyr Gln Phe Ala Val Ser Gln Trp Cys
        195                 200                 205

Phe Ser Lys Gly Phe Asp Asn Thr Asn Pro Ile Gly Pro Cys Ile Val
    210                 215                 220

Ser Ala Ser Ser Ile Pro Asn Pro Gln Asp Ile Gln Ile Gln Cys Lys
225                 230                 235                 240

Leu Asn Gly Asn Val Val Gln Asn Gly Asn Thr Ser Asp Gln Ile Phe
                245                 250                 255

Asn Ile Lys Lys Thr Val Ala Phe Leu Ser Gln Gly Thr Thr Leu Glu
            260                 265                 270

Ser Gly Ser Ile Ile Leu Thr Gly Thr Pro Gly Gly Val Gly Phe Val
        275                 280                 285

Arg Asp Pro Pro Leu Tyr Leu Lys Asp Gly Asp Glu Val Val Thr Trp
    290                 295                 300

Ile Gly Ser Gly Val Gly Ser Leu Val Asn Val Val Lys Glu Glu Lys
305                 310                 315                 320

Thr

<210>  68
<211>  906
<212>  DNA
<213>  Armillaria gallica

<400>  68
atggcgccta ttatcactca atggtccaga cttatccgct ttgtcgccgt cgaaacctct      60

cgtgtacata ttggacagcc cgtagattct aacctggatg ttggtctagc ggcgtaccag     120

ggaatgctaa tcaaggctta cgaaatactt ggttctgctc tcgatccatc cgcccaaatg     180

actagcaaga tcctcactgt caaacaacta ttaaccccgc tgtctggcga agatgtcaag     240

gtcgttagat gcttgggtct caactaccca gcacatgcga atgaagggaa agtagaagca     300

cctaagtttc ctaacctttt ctataagcca gtgacgtcgc ttatcgggcc cctcgcacct     360

gtgatcattc ctgcagtcgc acaaccttct gcaatacatc aatccgatta tgaggctgaa     420

tttactgtcg tcataggcag ggcagctaag aatatcactg aagcggaagc tttagactat     480

gttctcggct acaccggcgg caatgacgtg tcttttcgtc agcatcaatt tgcggtctct     540

caatggtgtt tctccaaaag ttttgacaac acaaatccct ttgggccatg cttagttgcc     600

gcatcgtcta ttcccgaccc tcaaactgtg gccattaagt ttacactgaa tggtcaaact     660

gtccaagacg gaactactgc cgatcaactt ttcagcgtca aaaagaccat agcttatctt     720

tctcaaggca cgacgttaca gccgggctcc ataattatga ctggtactcc cagtggcgtt     780

ggattcgtcc gaaacccacc tctctacctc aaagatggag accatatgtt gacttggata     840

agcggtggaa tcggtacgct tgcaaacagc gtcgtggagg agaagactcc gactcctggt     900

ttagat                                                                906

<210>  69
<211>  302
<212>  PRT
<213>  Armillaria gallica

<400>  69

Met Ala Pro Ile Ile Thr Gln Trp Ser Arg Leu Ile Arg Phe Val Ala
1               5                   10                  15

Val Glu Thr Ser Arg Val His Ile Gly Gln Pro Val Asp Ser Asn Leu
            20                  25                  30

Asp Val Gly Leu Ala Ala Tyr Gln Gly Met Leu Ile Lys Ala Tyr Glu
        35                  40                  45

Ile Leu Gly Ser Ala Leu Asp Pro Ser Ala Gln Met Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ser Ala Ile His Gln Ser Asp Tyr Glu Ala Glu Phe Thr Val Val
    130                 135                 140

Ile Gly Arg Ala Ala Lys Asn Ile Thr Glu Ala Glu Ala Leu Asp Tyr
145                 150                 155                 160

Val Leu Gly Tyr Thr Gly Gly Asn Asp Val Ser Phe Arg Gln His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Thr Val Ala Ile Lys Phe Thr Leu Asn Gly Gln Thr Val Gln Asp Gly
    210                 215                 220

Thr Thr Ala Asp Gln Leu Phe Ser Val Lys Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Ser Gly Val Gly Phe Val Arg Asn Pro Pro Leu Tyr Leu Lys Asp
            260                 265                 270

Gly Asp His Met Leu Thr Trp Ile Ser Gly Gly Ile Gly Thr Leu Ala
        275                 280                 285

Asn Ser Val Val Glu Glu Lys Thr Pro Thr Pro Gly Leu Asp
    290                 295                 300

<210>  70
<211>  906
<212>  DNA
<213>  Armillaria ostoyae

<400>  70
atggcaccta ttattactca atggtccaga cttattcgct ttgttgccgt cgagacctct      60

cgtgtacata ttggacagcc catagattct accctggata ttggtctagc ggcgtaccag     120

ggaatgctaa tcaaggctta tgaaatactt ggttctgctc tcgatccatc cgcccaaatg     180

accagcaaga tcctcaccgt taaacagcta ttaactccgc tgtctggcga agatgtcaag     240

gtcgtccgat gcttgggtct taactatcca gctcatgcga atgaagggaa agtagaagcg     300

cctaagtttc ctaacctttt ctataagcca gtgacatcgc ttatcgggcc cctcgcacct     360

gtgatcattc ctgcagtcgc acagccttct gcaatacatc aatccgatta tgaggctgaa     420

tttactgtcg tcataggcag ggcagctaag aatgtcactg aagcggaagc tttagactat     480

gttctcgggt acaccggcgg caatgatgtg tcttttcgtc agcatcaatt tgcggtctct     540

caatggtgtt tctctaaaag ttttgacaat acaaatccct tcggtccatg cttagttgcc     600

gcatcgtcta ttcctgatcc tcaaactgtg gccattaagt ttacattgaa tggtgacacc     660

gtccaagacg gaactactgc tgatcaactt ttcagcgtca aaaagaccat cgcttatctt     720

tctcagggca cgacgttaca gccgggctcc ataattatga ctggcactcc cagtggtgtt     780

gggttcgtcc aaaacccacc tctctacctc aaagatgggg atcaaatgtt gacttggata     840

agcggtggaa tcggtacgct tgcaaacaac gtcgtagagg agaagactcc gactcctcgt     900

ttagac                                                                906

<210>  71
<211>  302
<212>  PRT
<213>  Armillaria ostoyae

<400>  71

Met Ala Pro Ile Ile Thr Gln Trp Ser Arg Leu Ile Arg Phe Val Ala
1               5                   10                  15

Val Glu Thr Ser Arg Val His Ile Gly Gln Pro Ile Asp Ser Thr Leu
            20                  25                  30

Asp Ile Gly Leu Ala Ala Tyr Gln Gly Met Leu Ile Lys Ala Tyr Glu
        35                  40                  45

Ile Leu Gly Ser Ala Leu Asp Pro Ser Ala Gln Met Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ser Ala Ile His Gln Ser Asp Tyr Glu Ala Glu Phe Thr Val Val
    130                 135                 140

Ile Gly Arg Ala Ala Lys Asn Val Thr Glu Ala Glu Ala Leu Asp Tyr
145                 150                 155                 160

Val Leu Gly Tyr Thr Gly Gly Asn Asp Val Ser Phe Arg Gln His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Thr Val Ala Ile Lys Phe Thr Leu Asn Gly Asp Thr Val Gln Asp Gly
    210                 215                 220

Thr Thr Ala Asp Gln Leu Phe Ser Val Lys Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Ser Gly Val Gly Phe Val Gln Asn Pro Pro Leu Tyr Leu Lys Asp
            260                 265                 270

Gly Asp Gln Met Leu Thr Trp Ile Ser Gly Gly Ile Gly Thr Leu Ala
        275                 280                 285

Asn Asn Val Val Glu Glu Lys Thr Pro Thr Pro Arg Leu Asp
    290                 295                 300

<210>  72
<211>  891
<212>  DNA
<213>  Armillaria mellea

<400>  72
atggcgccta ttatcactca gtggtccaga cttattcgct ttgttgccgt cgagacttct      60

cgtgtacatt ttggacagcc cgtagattct accctggatg ttggtctagc ggcgtaccag     120

ggtgtgttga tcaaggctta tgaaatactt ggttctgctc ttgatccatc cgcccaaatg     180

accagcaaga tcctcaccgt gaaacagcta ttaactccgc tgtctggcga ggatgtcaaa     240

gtcgtccgat gcttgggtct taactatcca gcacatgcga atgaagggaa agtagaagca     300

cccaagtttc ctaacctgtt ctataagcca gtgacatcgc ttatcgggcc cctcgcgcct     360

gtgatcattc ctgcagtcgc acagccttct gcaatacatc aatctgatta tgaggctgaa     420

tttactgttg tcctaggcag ggcagctaag aatgtcactg aagctgaagc cttggactat     480

gttctcggtt acaccggcgg caatgatgtg tcttttcggc agcatcaatt tgctgtctct     540

caatggtgtt tctccaaaag ttttgacaat acaaatccct tcggtccatg cttagttgcc     600

gcgtcgtcta ttcctgatcc tcaaactgtg gccattaagt ttacattgaa tggcaacacc     660

gtccaagatg gaactactgc tgatcaactt ttcagcgtca gaaagaccat cgcttatctt     720

tctcaaggca cgacgttaca gcctggctcc ataattatga ccggtactcc cagtggcgtt     780

gggttcgtcc gaaacccacc tctctacctc aaagatgggg atcaaatgtt gacttggatt     840

agcggtggaa tcggtacgct tgcaaacagc gtcatagagg agaagacccc a              891

<210>  73
<211>  297
<212>  PRT
<213>  Armillaria mellea

<400>  73

Met Ala Pro Ile Ile Thr Gln Trp Ser Arg Leu Ile Arg Phe Val Ala
1               5                   10                  15

Val Glu Thr Ser Arg Val His Phe Gly Gln Pro Val Asp Ser Thr Leu
            20                  25                  30

Asp Val Gly Leu Ala Ala Tyr Gln Gly Val Leu Ile Lys Ala Tyr Glu
        35                  40                  45

Ile Leu Gly Ser Ala Leu Asp Pro Ser Ala Gln Met Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ser Ala Ile His Gln Ser Asp Tyr Glu Ala Glu Phe Thr Val Val
    130                 135                 140

Leu Gly Arg Ala Ala Lys Asn Val Thr Glu Ala Glu Ala Leu Asp Tyr
145                 150                 155                 160

Val Leu Gly Tyr Thr Gly Gly Asn Asp Val Ser Phe Arg Gln His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Thr Val Ala Ile Lys Phe Thr Leu Asn Gly Asn Thr Val Gln Asp Gly
    210                 215                 220

Thr Thr Ala Asp Gln Leu Phe Ser Val Arg Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Ser Gly Val Gly Phe Val Arg Asn Pro Pro Leu Tyr Leu Lys Asp
            260                 265                 270

Gly Asp Gln Met Leu Thr Trp Ile Ser Gly Gly Ile Gly Thr Leu Ala
        275                 280                 285

Asn Ser Val Ile Glu Glu Lys Thr Pro
    290                 295

<210>  74
<211>  891
<212>  DNA
<213>  Armillaria fuscipes

<400>  74
atggcaccta ttatcactca atggtccaga cttattcgct ttgtctccat tgagacttct      60

ggtgttcata ttggacaacc cgtagatcct acccttgacg tcggtctagc ggcgtcccag     120

ggagtggcaa tcaaggttta tgaaataatt ggttctgcgc ttgatccatc cgcccaagtg     180

accagcaaaa tccttaccgt caaacagcta ttaactccgc tgtctggtga ggatgtcaag     240

gtcgtccggt gcttgggtct gaactatcca gcacatgcca atgaagggaa agtagaagca     300

cccaagtttc ctaacctttt ctataagcca gtgacatcgc ttattgggcc cctcgcgcct     360

gtgatcattc ctgcagtcgc acagccggcc gcaatacatc aatctgatta tgaggctgaa     420

tttactgttg tcattggcag ggcagccaag aatgtcacgg aagctgaagc cctggactat     480

gttcttggct acaccggcgg taatgatgtg tcttttcgga agcatcaatt tgcagtctct     540

caatggtgtt tctccaaaag ttttgacaat acaaatccct ttggaccatg cttagttgcc     600

gcttcgtcta tccctgatcc tcagaatgtg gccattaagt tcacgttgaa tggtaacacc     660

gttcaagatg gaactactgc tgaccaaatt ttcagcgtta gaaagactat cgcttatctt     720

tctcaaggca cgacgttaca gccaggctcc atcattatga ctggcactcc caatggcgtt     780

gggtttgtcc gagacccacc tctctacctc aaagacgggg atcaaatgct gacttggatt     840

agcggtggaa ttggtacgct tgcgaatggc gtcgtagaag agaagacccg g              891

<210>  75
<211>  297
<212>  PRT
<213>  Armillaria fuscipes

<400>  75

Met Ala Pro Ile Ile Thr Gln Trp Ser Arg Leu Ile Arg Phe Val Ser
1               5                   10                  15

Ile Glu Thr Ser Gly Val His Ile Gly Gln Pro Val Asp Pro Thr Leu
            20                  25                  30

Asp Val Gly Leu Ala Ala Ser Gln Gly Val Ala Ile Lys Val Tyr Glu

35                  40                  45

Ile Ile Gly Ser Ala Leu Asp Pro Ser Ala Gln Val Thr Ser Lys Ile
    50                  55                  60

Leu Thr Val Lys Gln Leu Leu Thr Pro Leu Ser Gly Glu Asp Val Lys
65                  70                  75                  80

Val Val Arg Cys Leu Gly Leu Asn Tyr Pro Ala His Ala Asn Glu Gly
                85                  90                  95

Lys Val Glu Ala Pro Lys Phe Pro Asn Leu Phe Tyr Lys Pro Val Thr
            100                 105                 110

Ser Leu Ile Gly Pro Leu Ala Pro Val Ile Ile Pro Ala Val Ala Gln
        115                 120                 125

Pro Ala Ala Ile His Gln Ser Asp Tyr Glu Ala Glu Phe Thr Val Val
    130                 135                 140

Ile Gly Arg Ala Ala Lys Asn Val Thr Glu Ala Glu Ala Leu Asp Tyr
145                 150                 155                 160

Val Leu Gly Tyr Thr Gly Gly Asn Asp Val Ser Phe Arg Lys His Gln
                165                 170                 175

Phe Ala Val Ser Gln Trp Cys Phe Ser Lys Ser Phe Asp Asn Thr Asn
            180                 185                 190

Pro Phe Gly Pro Cys Leu Val Ala Ala Ser Ser Ile Pro Asp Pro Gln
        195                 200                 205

Asn Val Ala Ile Lys Phe Thr Leu Asn Gly Asn Thr Val Gln Asp Gly
    210                 215                 220

Thr Thr Ala Asp Gln Ile Phe Ser Val Arg Lys Thr Ile Ala Tyr Leu
225                 230                 235                 240

Ser Gln Gly Thr Thr Leu Gln Pro Gly Ser Ile Ile Met Thr Gly Thr
                245                 250                 255

Pro Asn Gly Val Gly Phe Val Arg Asp Pro Pro Leu Tyr Leu Lys Asp
            260                 265                 270

Gly Asp Gln Met Leu Thr Trp Ile Ser Gly Gly Ile Gly Thr Leu Ala
        275                 280                 285

Asn Gly Val Val Glu Glu Lys Thr Arg
    290                 295

<210>  76
<211>  42
<212>  PRT
<213>  Artificial sequence

<220>
<223>  Consensus amino acid sequence 1
       caffeylpyruvate hydrolase

<220>
<221>  MISC_FEATURE
<222>  (2)..(39)
<223>  X - any amino acid

<400>  76

Trp Xaa Arg Leu Ile Arg Phe Val Xaa Xaa Glu Thr Ser Xaa Val His
1               5                   10                  15

Xaa Gly Xaa Pro Xaa Xaa Xaa Xaa Xaa Asp Xaa Gly Xaa Ala Xaa Xaa
            20                  25                  30

Xaa Gly Xaa Xaa Ile Xaa Xaa Tyr Glu Ile
        35                  40

<210>  77
<211>  137
<212>  PRT
<213>  Artificial sequence

<220>
<223>  Consensus amino acid sequence 2
       caffeylpyruvate hydrolase

<220>
<221>  MISC_FEATURE
<222>  (5)..(136)
<223>  X - any amino acid

<400>  77

Ser Ala Leu Asp Xaa Ser Ala Gln Xaa Xaa Xaa Xaa Xaa Leu Thr Val
1               5                   10                  15

Xaa Xaa Leu Leu Xaa Pro Leu Ser Xaa Glu Xaa Xaa Lys Xaa Val Arg
            20                  25                  30

Cys Leu Gly Leu Asn Tyr Xaa Xaa His Ala Xaa Xaa Xaa Xaa Xaa Xaa
        35                  40                  45

Xaa Xaa Xaa Xaa Pro Xaa Leu Phe Tyr Lys Pro Val Thr Ser Leu Ile

50                  55                  60

Gly Pro Xaa Xaa Xaa Xaa Xaa Ile Pro Xaa Xaa Xaa Gln Pro Xaa Xaa
65                  70                  75                  80

Xaa His Gln Ser Asp Tyr Glu Ala Glu Xaa Xaa Xaa Xaa Xaa Gly Xaa
                85                  90                  95

Ala Ala Lys Xaa Xaa Xaa Glu Xaa Glu Ala Leu Xaa Tyr Val Leu Gly
            100                 105                 110

Tyr Thr Xaa Xaa Asn Asp Xaa Ser Phe Arg Xaa Xaa Gln Xaa Ala Val
        115                 120                 125

Ser Gln Trp Xaa Phe Ser Lys Xaa Phe
    130                 135

<210>  78
<211>  69
<212>  PRT
<213>  Artificial sequence

<220>
<223>  Consensus amino acid sequence 3
       caffeylpyruvate hydrolase

<220>
<221>  MISC_FEATURE
<222>  (3)..(65)
<223>  X - any amino acid

<400>  78

Asp Gln Xaa Phe Xaa Xaa Xaa Lys Thr Xaa Xaa Xaa Leu Ser Gln Gly
1               5                   10                  15

Thr Thr Leu Xaa Xaa Gly Ser Ile Ile Xaa Thr Gly Thr Pro Xaa Gly
            20                  25                  30

Val Gly Phe Val Xaa Xaa Pro Pro Leu Tyr Leu Lys Asp Gly Asp Xaa
        35                  40                  45

Xaa Xaa Thr Trp Ile Xaa Xaa Gly Xaa Gly Xaa Leu Xaa Asn Xaa Val
    50                  55                  60

Xaa Glu Glu Lys Thr
65

<210>  79
<211>  801
<212>  DNA

<213>  Neonothopanus nambi

<400>  79
atgcgcatta acattagcct ctcgtctctc ttcgaacgtc tctccaaact tagcagtcgc      60

agcatagcga ttacatgtgg agttgttctc gcctccgcaa tcgcctttcc catcatccgc     120

agagactacc agactttcct agaagtggga ccctcgtacg ctccgcagaa ctttagagga     180

tacatcatcg tctgtgtcct ctcgctattc cgccaagagc agaaagggct cgccatctat     240

gatcgtcttc ccgagaaacg caggtggttg gccgaccttc cctttcgtga aggaaccaga     300

cccagcatta ccagccatat cattcagcga cagcgcactc aactggtcga tcaggagttt     360

gccaccaggg agctcataga caaggtcatc cctcgcgtgc aagcacgaca caccgacaaa     420

acgttcctca gcacatcaaa gttcgagttt catgcgaagg ccatatttct cttgccttct     480

atcccaatca acgaccctct gaatatccct agccacgaca ctgtccgccg aacgaagcgc     540

gagattgcac atatgcatga ttatcatgat tgcacacttc atcttgctct cgctgcgcag     600

gatggaaagg aggtgctgaa gaaaggttgg ggacaacgac atcctttggc tggtcctgga     660

gttcctggtc caccaacgga atggactttt ctttatgcgc ctcgcaacga agaagaggct     720

cgagtagtgg agatgatcgt tgaggcttcc atagggtata tgacgaacga tcctgcagga     780

aagattgtag aaaacgccaa g                                               801

<210>  80
<211>  267
<212>  PRT
<213>  Neonothopanus nambi

<400>  80

Met Arg Ile Asn Ile Ser Leu Ser Ser Leu Phe Glu Arg Leu Ser Lys
1               5                   10                  15

Leu Ser Ser Arg Ser Ile Ala Ile Thr Cys Gly Val Val Leu Ala Ser
            20                  25                  30

Ala Ile Ala Phe Pro Ile Ile Arg Arg Asp Tyr Gln Thr Phe Leu Glu
        35                  40                  45

Val Gly Pro Ser Tyr Ala Pro Gln Asn Phe Arg Gly Tyr Ile Ile Val
    50                  55                  60

Cys Val Leu Ser Leu Phe Arg Gln Glu Gln Lys Gly Leu Ala Ile Tyr
65                  70                  75                  80

Asp Arg Leu Pro Glu Lys Arg Arg Trp Leu Ala Asp Leu Pro Phe Arg
                85                  90                  95

Glu Gly Thr Arg Pro Ser Ile Thr Ser His Ile Ile Gln Arg Gln Arg
            100                 105                 110

Thr Gln Leu Val Asp Gln Glu Phe Ala Thr Arg Glu Leu Ile Asp Lys
        115                 120                 125

Val Ile Pro Arg Val Gln Ala Arg His Thr Asp Lys Thr Phe Leu Ser
    130                 135                 140

Thr Ser Lys Phe Glu Phe His Ala Lys Ala Ile Phe Leu Leu Pro Ser
145                 150                 155                 160

Ile Pro Ile Asn Asp Pro Leu Asn Ile Pro Ser His Asp Thr Val Arg
                165                 170                 175

Arg Thr Lys Arg Glu Ile Ala His Met His Asp Tyr His Asp Cys Thr
            180                 185                 190

Leu His Leu Ala Leu Ala Ala Gln Asp Gly Lys Glu Val Leu Lys Lys
        195                 200                 205

Gly Trp Gly Gln Arg His Pro Leu Ala Gly Pro Gly Val Pro Gly Pro
    210                 215                 220

Pro Thr Glu Trp Thr Phe Leu Tyr Ala Pro Arg Asn Glu Glu Glu Ala
225                 230                 235                 240

Arg Val Val Glu Met Ile Val Glu Ala Ser Ile Gly Tyr Met Thr Asn
                245                 250                 255

Asp Pro Ala Gly Lys Ile Val Glu Asn Ala Lys
            260                 265

<210>  81
<211>  783
<212>  DNA
<213>  Omphalotus olearius

<400>  81
atgctcccag ctttcatcta caaaccaagg ctagtgatca cttgtgtatt cgttctggcc      60

tccgcactcg catttccctt catacgcaaa gattaccaga ctttcctgga ggtgggaccc     120

tcgtacgccc cgcagaacct ccaaggatac atcatcgtct gtgtactctc tctgttccgg     180

caagaacaga aagacgtagc gatttatgat cgccttcctg agaaaaggag gtggttagga     240

gacctcccgt ttcgcgaggg gccaagaccg agtatcacta gccatatcat ccagcgacag     300

cgcacccaat tggctgacgc cgagttcgct accaaagagc tgataggcaa aatcatccct     360

cgcgtccaag cccgacacac caacacaaca ttcctcagca catctaaatt cgaattccac     420

gcccaggcca tcttcctttt gccctctatc ccaatcaacg accctcaaaa cattccaagc 480

cacgataccg ttcgtcgcac gaaacgcgag atcgcgcata tgcatgatta tcacgactgt 540

acgttgcatc tcgcacttgc tgctcaagat gggaaggagg ttttagagaa aggatggggt 600

cagcgacatc ctcttgctgg acctggtgtt cctggcccgc cgacggagtg gacgtttctt 660

tatgcaccgc gcagcgaaga ggaggttcgg gttgtggaga tgattgttga ggcatcagtt 720

gtgtatatga cgaatgatcc tgcggataaa atcgtagaag ctactgtgca gggtactgaa 780

gaa 783

<210> 82
<211> 261
<212> PRT
<213> Omphalotus olearius

<400> 82

Met Leu Pro Ala Phe Ile Tyr Lys Pro Arg Leu Val Ile Thr Cys Val
1               5                   10                  15

Phe Val Leu Ala Ser Ala Leu Ala Phe Pro Phe Ile Arg Lys Asp Tyr
            20                  25                  30

Gln Thr Phe Leu Glu Val Gly Pro Ser Tyr Ala Pro Gln Asn Leu Gln
        35                  40                  45

Gly Tyr Ile Ile Val Cys Val Leu Ser Leu Phe Arg Gln Glu Gln Lys
    50                  55                  60

Asp Val Ala Ile Tyr Asp Arg Leu Pro Glu Lys Arg Arg Trp Leu Gly
65                  70                  75                  80

Asp Leu Pro Phe Arg Glu Gly Pro Arg Pro Ser Ile Thr Ser His Ile
                85                  90                  95

Ile Gln Arg Gln Arg Thr Gln Leu Ala Asp Ala Glu Phe Ala Thr Lys
            100                 105                 110

Glu Leu Ile Gly Lys Ile Ile Pro Arg Val Gln Ala Arg His Thr Asn
        115                 120                 125

Thr Thr Phe Leu Ser Thr Ser Lys Phe Glu Phe His Ala Gln Ala Ile
    130                 135                 140

Phe Leu Leu Pro Ser Ile Pro Ile Asn Asp Pro Gln Asn Ile Pro Ser
145                 150                 155                 160

His Asp Thr Val Arg Arg Thr Lys Arg Glu Ile Ala His Met His Asp
                165                 170                 175

Tyr His Asp Cys Thr Leu His Leu Ala Leu Ala Ala Gln Asp Gly Lys
            180                 185                 190

Glu Val Leu Glu Lys Gly Trp Gly Gln Arg His Pro Leu Ala Gly Pro
        195                 200                 205

Gly Val Pro Gly Pro Pro Thr Glu Trp Thr Phe Leu Tyr Ala Pro Arg
    210                 215                 220

Ser Glu Glu Glu Val Arg Val Val Glu Met Ile Val Glu Ala Ser Val
225                 230                 235                 240

Val Tyr Met Thr Asn Asp Pro Ala Asp Lys Ile Val Glu Ala Thr Val
                245                 250                 255

Gln Gly Thr Glu Glu
            260

<210>  83
<211>  798
<212>  DNA
<213>  Armillaria gallica

<400>  83
atgtccttca tcgacagcat gaaacttgac ctcgtcggac acctctttgg catcaggaat      60

cgcggcttag ccgccgcttg ttgtgctcta gcagtcgcct ctactatcgc cttcccttac     120

attcgtaggg actaccagac atttttatct ggcggtccct cttacgctcc ccagaatatc     180

agaggatatt tcatcgtctg cgttctggcc ttgttccgtc aggagcaaaa gggccttgcg     240

atatatgatc gccttcccga gaagcgcagg tggctgcctg acttgcctcc tcgcaatggc     300

ccgcggccga tcacgaccag ccatataatc caaagacagc gcaaccaggc gccggacccc     360

aagttcgccc tcgaggaact caaggccacg gttattccac gggtgcaggc tcgccatact     420

gacctcaccc atctcagcct atccaaattc gagttccatg ctgaagcaat tttcctgctc     480

ccctctgtac ccatcgatga tccaaaaaat gttccaagtc acgacacggt gcgcaggacg     540

aaaagggaga tcgcgcatat gcacgactac catgacttca cgctgcatct tgcactggcc     600

gcccaagacg ggaaggaagt cgtgtcgaag ggatgggggc agcgacaccc cctagcaggc     660

cctggcgttc ctggtccacc tacggagtgg acatttattt atgcgccacg taacgaagag     720

gaactggcag tggtggaaat gattatcgag gcatcaatag gctatatgac caatgaccct     780

gctggagtag ttatcgca                                                   798

<210>  84
<211>  266
<212>  PRT
<213>  Armillaria gallica

<400>  84

Met Ser Phe Ile Asp Ser Met Lys Leu Asp Leu Val Gly His Leu Phe
1               5                   10                  15

Gly Ile Arg Asn Arg Gly Leu Ala Ala Ala Cys Cys Ala Leu Ala Val
            20                  25                  30

Ala Ser Thr Ile Ala Phe Pro Tyr Ile Arg Arg Asp Tyr Gln Thr Phe
        35                  40                  45

Leu Ser Gly Gly Pro Ser Tyr Ala Pro Gln Asn Ile Arg Gly Tyr Phe
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Pro Asp Leu Pro
                85                  90                  95

Pro Arg Asn Gly Pro Arg Pro Ile Thr Thr Ser His Ile Ile Gln Arg
            100                 105                 110

Gln Arg Asn Gln Ala Pro Asp Pro Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Val Pro Ser His Asp Thr
                165                 170                 175

Val Arg Arg Thr Lys Arg Glu Ile Ala His Met His Asp Tyr His Asp
            180                 185                 190

Phe Thr Leu His Leu Ala Leu Ala Ala Gln Asp Gly Lys Glu Val Val
        195                 200                 205

Ser Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Val Val Ile Ala
            260                 265

<210>  85
<211>  798
<212>  DNA
<213>  Armillaria ostoyae

<400>  85
atgtccttca tcgacagcat gaaacttgac ttcgtcggac acctctttgg catcaggaat      60

cgcggcttag ccaccgcttg ttgtgctgtg gcagtcgctt ctgccatcgc cttcccttac     120

attcgtaggg actaccagac attcttatct ggcggtccct cttacgctcc ccagaacatc     180

aaaggatatc tcatcgtctg cgtcctggcc ttgttccgtc aggagcaaaa gggccttgcg     240

atatatgacc gccttcccga gaagcgcagg tggctacctg acttgcctcc tcgcaatggc     300

ccgcggccca tcacgaccag ccatataatc caaagacagc gcaaccaggc gccagactcc     360

aagttcgccc tcgaggaact caaggctacg gtcattccac gggtgcaggc tcgccacact     420

gacctcaccc atctcagcct atccaagttc gagttccatg ctgaagcaat cttcctgctc     480

ccctctgtac ccatcgatga tccaaaaaat gttccaagtc atgacacggt gcgcaggacg     540

aagagggaga tcgcgcatat gcacgactac catgacttta cgttgcatct tgcactggcc     600

gcccaagacg ggaaggaagt cgtggcgaag ggatgggggc agcgacaccc gctggcaggc     660

cctggcgttc ctggtccacc tacggagtgg acgtttattt atgcgccacg taacgaagag     720

gaactggcag tggtggaaat gattatcgag gcatcaatag gctatatgac caatgaccct     780

gctggaacag ttatcgta                                                   798

<210>  86
<211>  266
<212>  PRT
<213>  Armillaria ostoyae

<400>  86

Met Ser Phe Ile Asp Ser Met Lys Leu Asp Phe Val Gly His Leu Phe
1               5                   10                  15

Gly Ile Arg Asn Arg Gly Leu Ala Thr Ala Cys Cys Ala Val Ala Val
            20                  25                  30

Ala Ser Ala Ile Ala Phe Pro Tyr Ile Arg Arg Asp Tyr Gln Thr Phe

35                  40                  45

Leu Ser Gly Gly Pro Ser Tyr Ala Pro Gln Asn Ile Lys Gly Tyr Leu
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Pro Asp Leu Pro
                85                  90                  95

Pro Arg Asn Gly Pro Arg Pro Ile Thr Thr Ser His Ile Ile Gln Arg
            100                 105                 110

Gln Arg Asn Gln Ala Pro Asp Ser Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Val Pro Ser His Asp Thr
                165                 170                 175

Val Arg Arg Thr Lys Arg Glu Ile Ala His Met His Asp Tyr His Asp
            180                 185                 190

Phe Thr Leu His Leu Ala Leu Ala Ala Gln Asp Gly Lys Glu Val Val
        195                 200                 205

Ala Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Thr Val Ile Val
            260                 265

<210>  87
<211>  798
<212>  DNA
<213>  Armillaria mellea

<400> 87
atgtccttct tcgacagcgt gaaacttgac ctcgtcggac gcctctttgg catcaggaat 60

cgcggcttag ctgttacttg ttgtgctgtg gcagtcgcct ctatcatcgc gttcccttac 120

attcgtaggg actaccagac atttttatct gggggtccct cctacgctcc ccagaacatc 180

agaggatacc tcattgtctg cgtcctggcc ttgttccgtc aggagcaaaa aggccttgcg 240

atatacgacc gccttcccga gaagcgcagg tggctacctg acttgcctcc tcgcgatggc 300

ccacggccca tcacgaccag ccatataatc caaagacagc gcaaccaggc gccggacctc 360

aagttcgccc tcgaggaact caaggccacg gtcattccac gggtgcaggc tcgccacact 420

gacctcaccc atctcagcct atccaagttc gagttccatg ctgaagcaat cttcctgctc 480

ccctctgtac ccatcgatga tccaaagaat gtgccaagtc acgacacggt gcgcaggacg 540

aagagggaaa ttgcgcatat gcacgactac catgactaca cgctgcatct tgcgttggcc 600

gcccaagacg ggaaggaagt cgtatcaaag ggatgggggc agcgacaccc gctggcaggc 660

cctggcgttc ctggtccacc gacggagtgg acgtttattt atgcgccacg taacgaagag 720

gagctggcag tggtggaaat gattatcgag gcatcgatag gctatatgac caatgaccct 780

gcaggaaaaa ctatcgca 798

<210> 88
<211> 266
<212> PRT
<213> Armillaria mellea

<400> 88

Met Ser Phe Phe Asp Ser Val Lys Leu Asp Leu Val Gly Arg Leu Phe
1               5                   10                  15

Gly Ile Arg Asn Arg Gly Leu Ala Val Thr Cys Cys Ala Val Ala Val
            20                  25                  30

Ala Ser Ile Ile Ala Phe Pro Tyr Ile Arg Arg Asp Tyr Gln Thr Phe
        35                  40                  45

Leu Ser Gly Gly Pro Ser Tyr Ala Pro Gln Asn Ile Arg Gly Tyr Leu
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Pro Asp Leu Pro
                85                  90                  95

Pro Arg Asp Gly Pro Arg Pro Ile Thr Thr Ser His Ile Ile Gln Arg

                100                 105                 110

Gln Arg Asn Gln Ala Pro Asp Leu Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Val Pro Ser His Asp Thr
                165                 170                 175

Val Arg Arg Thr Lys Arg Glu Ile Ala His Met His Asp Tyr His Asp
            180                 185                 190

Tyr Thr Leu His Leu Ala Leu Ala Ala Gln Asp Gly Lys Glu Val Val
        195                 200                 205

Ser Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Lys Thr Ile Ala
            260                 265

<210>  89
<211>  798
<212>  DNA
<213>  Armillaria fuscipes

<400>  89
atgaccttct tggacagtat caaacttgac ctcgttgggc gcctctttgg catcaggaat      60

cacggcgtag ccgctgcctg ttgtgctgca gcagttgcct ctgccatcgt gttcccttat     120

attcgtaggg actaccagac atttctatct ggcggccctt cctacgctcc ccagaacatc     180

agaggataca tcattgtctg cgtcctagcc ttattccgcc aggagcaaaa aggccttgcg     240

atatatgacc gccttcccga gaagcgcagg tggttagctg acttgcctcc tcgcaatggc     300

ccacggccca tcacaaccag tcatataatt caaagacagc gcaaccaggc gccagacccc     360

aagttcgccc tcgaagaact caaggccaca gtcattccac gggtacaggc tcgccacact     420

gacctcaccc atctcagcct gtccaaattc gagtttcacg ctgaagcaat cttcctgctc 480

ccctctgtac ccatcgacga cccaaagaat atcccaagcc atgacacagt gcgcaggacg 540

aaaagggaga tcgcgcatat gcacgactat catgatttca cgctgcatct tgcactggct 600

gcccaagacg ggaaggaagt cgtatcaaag ggatgggggc agcggcaccc gctggcaggc 660

cctggtgtcc ctggtccacc aacggagtgg acgtttattt acgcgccacg gaacgaagag 720

gagctggcag tagtggaaat gataattgag gcatcaatag gctacatgac caatgaccct 780

gcaggatcag ttattcca 798

<210> 90
<211> 266
<212> PRT
<213> Armillaria fuscipes

<400> 90

Met Thr Phe Leu Asp Ser Ile Lys Leu Asp Leu Val Gly Arg Leu Phe
1               5                   10                  15

Gly Ile Arg Asn His Gly Val Ala Ala Ala Cys Cys Ala Ala Ala Val
            20                  25                  30

Ala Ser Ala Ile Val Phe Pro Tyr Ile Arg Arg Asp Tyr Gln Thr Phe
        35                  40                  45

Leu Ser Gly Gly Pro Ser Tyr Ala Pro Gln Asn Ile Arg Gly Tyr Ile
    50                  55                  60

Ile Val Cys Val Leu Ala Leu Phe Arg Gln Glu Gln Lys Gly Leu Ala
65                  70                  75                  80

Ile Tyr Asp Arg Leu Pro Glu Lys Arg Arg Trp Leu Ala Asp Leu Pro
                85                  90                  95

Pro Arg Asn Gly Pro Arg Pro Ile Thr Thr Ser His Ile Ile Gln Arg
            100                 105                 110

Gln Arg Asn Gln Ala Pro Asp Pro Lys Phe Ala Leu Glu Glu Leu Lys
        115                 120                 125

Ala Thr Val Ile Pro Arg Val Gln Ala Arg His Thr Asp Leu Thr His
    130                 135                 140

Leu Ser Leu Ser Lys Phe Glu Phe His Ala Glu Ala Ile Phe Leu Leu
145                 150                 155                 160

Pro Ser Val Pro Ile Asp Asp Pro Lys Asn Ile Pro Ser His Asp Thr

165                 170                 175

Val Arg Arg Thr Lys Arg Glu Ile Ala His Met His Asp Tyr His Asp
            180                 185                 190

Phe Thr Leu His Leu Ala Leu Ala Ala Gln Asp Gly Lys Glu Val Val
        195                 200                 205

Ser Lys Gly Trp Gly Gln Arg His Pro Leu Ala Gly Pro Gly Val Pro
    210                 215                 220

Gly Pro Pro Thr Glu Trp Thr Phe Ile Tyr Ala Pro Arg Asn Glu Glu
225                 230                 235                 240

Glu Leu Ala Val Val Glu Met Ile Ile Glu Ala Ser Ile Gly Tyr Met
                245                 250                 255

Thr Asn Asp Pro Ala Gly Ser Val Ile Pro
            260                 265

<210>  91
<211>  738
<212>  DNA
<213>  Mycena citricolor

<400>  91
atggcttatc agctcacttg gattcagact ctcgtgctgg gtgcccttgt ggcaatggca      60

gtagcgttcc ccttcatcaa gaaagactac gagacgttcc tgaagggcgg cccctcctat     120

gcgccccaaa acgttcgcgg atacatcatc gtgctcgtgc tcgcgctctt ccgccaagag     180

cagctcgggc tggagatcta cgaccgcatg cccgagaaac gtcgctggct cgcgaatctc     240

cctcagcgcg agggcccccg ccccaagacc acaagtcaca tcatccagcg gcagctcagc     300

cagcacacgg accccgcatt cggcgccgcg tacctcaaag acaccgtcat tccgcgcgtc     360

caggcgcggc acgcagccaa cacgcacatc gcgcgctcga cgttcgagtt ccacgccgcc     420

gcgatcttcc tgaacgcgga cgtgccgctg cccgagggcc tgcccgcaag cgagacggtg     480

cggcggacca agggcgagat cgcgcacatg cacgactacc acgacttcac gctgcacctc     540

gcgctcgcgg cggcggatgg gaaggaggtg gtcggcaagg gctgggggca gcgccatccg     600

ctggcgggac ccggtgtgcc gggtccgccg aacgagtgga cctttgtgta tgcgccgagg     660

aatgaagagg agatgggcgt ggtcgagcag atcgtagagg cggcgattgg gtacatgtcg     720

aacgtgcctg cgctggaa                                                   738

<210>  92
<211>  246
<212>  PRT

<213>  Mycena citricolor

<400>  92

Met Ala Tyr Gln Leu Thr Trp Ile Gln Thr Leu Val Leu Gly Ala Leu
1               5                   10                  15

Val Ala Met Ala Val Ala Phe Pro Phe Ile Lys Lys Asp Tyr Glu Thr
            20                  25                  30

Phe Leu Lys Gly Gly Pro Ser Tyr Ala Pro Gln Asn Val Arg Gly Tyr
        35                  40                  45

Ile Ile Val Leu Val Leu Ala Leu Phe Arg Gln Glu Gln Leu Gly Leu
    50                  55                  60

Glu Ile Tyr Asp Arg Met Pro Glu Lys Arg Arg Trp Leu Ala Asn Leu
65                  70                  75                  80

Pro Gln Arg Glu Gly Pro Arg Pro Lys Thr Thr Ser His Ile Ile Gln
                85                  90                  95

Arg Gln Leu Ser Gln His Thr Asp Pro Ala Phe Gly Ala Ala Tyr Leu
            100                 105                 110

Lys Asp Thr Val Ile Pro Arg Val Gln Ala Arg His Ala Ala Asn Thr
        115                 120                 125

His Ile Ala Arg Ser Thr Phe Glu Phe His Ala Ala Ala Ile Phe Leu
    130                 135                 140

Asn Ala Asp Val Pro Leu Pro Glu Gly Leu Pro Ala Ser Glu Thr Val
145                 150                 155                 160

Arg Arg Thr Lys Gly Glu Ile Ala His Met His Asp Tyr His Asp Phe
                165                 170                 175

Thr Leu His Leu Ala Leu Ala Ala Ala Asp Gly Lys Glu Val Val Gly
            180                 185                 190

Lys Gly Trp Gly Gln Arg His Pro Leu Ala Gly Pro Gly Val Pro Gly
        195                 200                 205

Pro Pro Asn Glu Trp Thr Phe Val Tyr Ala Pro Arg Asn Glu Glu Glu
    210                 215                 220

Met Gly Val Val Glu Gln Ile Val Glu Ala Ala Ile Gly Tyr Met Ser
225                 230                 235                 240

Asn Val Pro Ala Leu Glu
                245

<210>  93
<211>  747
<212>  DNA
<213>  Panellus stipticus

<400>  93
atgaacatca acctgaaagc tctgatcgga gtctgtgccg tgctcatcac cgctgcagtg      60

ttccccttcg ttcgtaaaga ctatcacacc tttcttgaag gtggaccatc ctacgcgccg     120

cagaatttgc aaggctatat catcgtgttg gtgctctcac tctttcgagg ggaggagacg     180

ggattggaaa tatacgaccg cttgcccgaa aaacgccgct ggctcgagga gctgcctgtt     240

cgcgaaggcc cgcgcccaaa gacaaccagc cacatcattc agagacaatt gaatcagcac     300

gttgacccgg acttcggaat gaactctttg aaaggctccg tcatccggcg ccttcaatcc     360

cgccaccagg acataactca actcgcactc tcgaaattcg aattccacgc cgaggccata     420

tttctgcgcc ccgatatcgc gatcaacgat cccaaacacg tcccgagcca cgacacggtg     480

cgccgcacaa agcgcgagat agctcacatg cacgactacc atgattacac gtgtcatttg     540

gcgctcgcag cgcaggatgg gaagcaagtg attgcaaaag ggtggggcca gagacatccg     600

ctcgcgggac cgggcatgcc ggggccgccg acggagtgga catttttgta tgcgccgagg     660

aatgaggcgg aggttcaagt gttggagacg attatcgaag cgtcaatcgg gtacatgtcg     720

aacgcaccag ccttgggtgg gagcgag                                         747

<210>  94
<211>  249
<212>  PRT
<213>  Panellus stipticus

<400>  94

Met Asn Ile Asn Leu Lys Ala Leu Ile Gly Val Cys Ala Val Leu Ile
1               5                   10                  15

Thr Ala Ala Val Phe Pro Phe Val Arg Lys Asp Tyr His Thr Phe Leu
            20                  25                  30

Glu Gly Gly Pro Ser Tyr Ala Pro Gln Asn Leu Gln Gly Tyr Ile Ile
        35                  40                  45

Val Leu Val Leu Ser Leu Phe Arg Gly Glu Glu Thr Gly Leu Glu Ile
    50                  55                  60

Tyr Asp Arg Leu Pro Glu Lys Arg Arg Trp Leu Glu Glu Leu Pro Val
65                  70                  75                  80

Arg Glu Gly Pro Arg Pro Lys Thr Thr Ser His Ile Ile Gln Arg Gln
                85                  90                  95

Leu Asn Gln His Val Asp Pro Asp Phe Gly Met Asn Ser Leu Lys Gly
            100                 105                 110

Ser Val Ile Arg Arg Leu Gln Ser Arg His Gln Asp Ile Thr Gln Leu
        115                 120                 125

Ala Leu Ser Lys Phe Glu Phe His Ala Glu Ala Ile Phe Leu Arg Pro
    130                 135                 140

Asp Ile Ala Ile Asn Asp Pro Lys His Val Pro Ser His Asp Thr Val
145                 150                 155                 160

Arg Arg Thr Lys Arg Glu Ile Ala His Met His Asp Tyr His Asp Tyr
                165                 170                 175

Thr Cys His Leu Ala Leu Ala Ala Gln Asp Gly Lys Gln Val Ile Ala
            180                 185                 190

Lys Gly Trp Gly Gln Arg His Pro Leu Ala Gly Pro Gly Met Pro Gly
        195                 200                 205

Pro Pro Thr Glu Trp Thr Phe Leu Tyr Ala Pro Arg Asn Glu Ala Glu
    210                 215                 220

Val Gln Val Leu Glu Thr Ile Ile Glu Ala Ser Ile Gly Tyr Met Ser
225                 230                 235                 240

Asn Ala Pro Ala Leu Gly Gly Ser Glu
                245

<210>  95
<211>  792
<212>  DNA
<213>  Neonothopanus gardneri

<400>  95
atgaatcttc cgtctttcgt ccaacgtctc tccacagcaa gcagtcgcag tatagcgatt      60

acttgcgtag ttgtccttgc ctctgcaatc gcctttccct tcatccgcag agactaccag     120

accttcctgg aagtgggacc ctcgtacgcc ccgcagaact ttagaggata catcatcgtc     180

tgtgtcctct cgttgttccg ccaagaacaa aaaggactcg aaatctacga tcggctccca     240

gagaaacgaa ggtggttgtc cgaccttccc tttcgtgacg ggcccagacc cagcatcaca     300

agccatatca ttcaacgaca gcgtacccaa ctagttgatc cggacttcgc tacccaggag     360

ctcataggca aagtcatccc tcgtgtgcaa gcacgacaca ccgacaaaac attcctcagc 420

acctccaaat tcgaatttca cgcaaaagcc atattcctcc tgccttccat cccaatcaac 480

gaccctctga acgttccaag ccacgacact gtccgacgaa cgaagcgcga gatcgcgcat 540

atgcatgatt atcatgattg cactcttcac atcgctctcg ctgctcagga cggaaaggag 600

gttttgaaga agggatgggg gcaacgacac ccactcgctg gacctggagt gcccggccca 660

ccgacggagt ggacgtttct ctatgcgcct cgaaacgaag aagaggttcg agttgtggag 720

atgattattg aggctgccat aggttacatg acgaatgatc cggcaggaaa agttgtagaa 780

gccactggaa ag 792

<210> 96
<211> 264
<212> PRT
<213> Neonothopanus gardneri

<400> 96

Met Asn Leu Pro Ser Phe Val Gln Arg Leu Ser Thr Ala Ser Ser Arg
1               5                   10                  15

Ser Ile Ala Ile Thr Cys Val Val Val Leu Ala Ser Ala Ile Ala Phe
            20                  25                  30

Pro Phe Ile Arg Arg Asp Tyr Gln Thr Phe Leu Glu Val Gly Pro Ser
        35                  40                  45

Tyr Ala Pro Gln Asn Phe Arg Gly Tyr Ile Ile Val Cys Val Leu Ser
    50                  55                  60

Leu Phe Arg Gln Glu Gln Lys Gly Leu Glu Ile Tyr Asp Arg Leu Pro
65                  70                  75                  80

Glu Lys Arg Arg Trp Leu Ser Asp Leu Pro Phe Arg Asp Gly Pro Arg
                85                  90                  95

Pro Ser Ile Thr Ser His Ile Ile Gln Arg Gln Arg Thr Gln Leu Val
            100                 105                 110

Asp Pro Asp Phe Ala Thr Gln Glu Leu Ile Gly Lys Val Ile Pro Arg
        115                 120                 125

Val Gln Ala Arg His Thr Asp Lys Thr Phe Leu Ser Thr Ser Lys Phe
    130                 135                 140

Glu Phe His Ala Lys Ala Ile Phe Leu Leu Pro Ser Ile Pro Ile Asn
145                 150                 155                 160

Asp Pro Leu Asn Val Pro Ser His Asp Thr Val Arg Arg Thr Lys Arg
                165                 170                 175

Glu Ile Ala His Met His Asp Tyr His Asp Cys Thr Leu His Ile Ala
            180                 185                 190

Leu Ala Ala Gln Asp Gly Lys Glu Val Leu Lys Lys Gly Trp Gly Gln
        195                 200                 205

Arg His Pro Leu Ala Gly Pro Gly Val Pro Gly Pro Pro Thr Glu Trp
    210                 215                 220

Thr Phe Leu Tyr Ala Pro Arg Asn Glu Glu Glu Val Arg Val Val Glu
225                 230                 235                 240

Met Ile Ile Glu Ala Ala Ile Gly Tyr Met Thr Asn Asp Pro Ala Gly
                245                 250                 255

Lys Val Val Glu Ala Thr Gly Lys
            260

<210>  97
<211>  753
<212>  DNA
<213>  Mycena chlorophos

<400>  97
atggtccaac tcacccgaac ctccggattc atcgccgctg cggccattgt tgctgccatc      60

gccttcccgt tcattcgtcg agactaccag acgttccttc gtggtgggcc gtcctatgcc     120

ccacagaaca tccgcggcta tatcatcgtc ctggttctgt ccctcttccg cggcgaggag     180

aagggtcttg caatctacga gccccttcct gagaagcgca catggctgcc ggagcttccg     240

cggcgcgcgg gagaccggcc caagacgacg agccacatca tccaacggca gctcgaccag     300

taccccgacc cggactttgt cctcaaagcc ctgaaagcga cggtcatccc gcgtgtccaa     360

gcccggcaca cagacaagac tcacctcgcg ctgtccaagt tcgagttcca tgctgaggcc     420

atcttcgtgc gcccggaaat cgccatcgac gacccgaagc atatccccag ccacgacacg     480

gtgcgacgga cgaagcgcga gattgcgcac atgcacgact atcacgactg cacgctgcat     540

ttggcgctag cggcgcagga cgcgaagcag gtgctgcaga agggctgggg ccagcgccat     600

ccgctggcag ggcctgggat gcccgggccg cccacggagt ggacgttctt gtatgccccg     660

aggaccgagg aggaagtgaa ggttgtggag accattgtcg aggcctctat cgcgtacatg     720

acgaacgcgg agaagccggt cgagctggtg cag                                  753

<210>  98
<211>  251
<212>  PRT
<213>  Mycena chlorophos

<400>  98

Met Val Gln Leu Thr Arg Thr Ser Gly Phe Ile Ala Ala Ala Ala Ile
1               5                   10                  15

Val Ala Ala Ile Ala Phe Pro Phe Ile Arg Arg Asp Tyr Gln Thr Phe
            20                  25                  30

Leu Arg Gly Gly Pro Ser Tyr Ala Pro Gln Asn Ile Arg Gly Tyr Ile
        35                  40                  45

Ile Val Leu Val Leu Ser Leu Phe Arg Gly Glu Glu Lys Gly Leu Ala
    50                  55                  60

Ile Tyr Glu Pro Leu Pro Glu Lys Arg Thr Trp Leu Pro Glu Leu Pro
65                  70                  75                  80

Arg Arg Ala Gly Asp Arg Pro Lys Thr Thr Ser His Ile Ile Gln Arg
                85                  90                  95

Gln Leu Asp Gln Tyr Pro Asp Pro Asp Phe Val Leu Lys Ala Leu Lys
            100                 105                 110

Ala Thr Val Ile Pro Arg Val Gln Ala Arg His Thr Asp Lys Thr His
        115                 120                 125

Leu Ala Leu Ser Lys Phe Glu Phe His Ala Glu Ala Ile Phe Val Arg
    130                 135                 140

Pro Glu Ile Ala Ile Asp Asp Pro Lys His Ile Pro Ser His Asp Thr
145                 150                 155                 160

Val Arg Arg Thr Lys Arg Glu Ile Ala His Met His Asp Tyr His Asp
                165                 170                 175

Cys Thr Leu His Leu Ala Leu Ala Ala Gln Asp Ala Lys Gln Val Leu
            180                 185                 190

Gln Lys Gly Trp Gly Gln Arg His Pro Leu Ala Gly Pro Gly Met Pro
        195                 200                 205

Gly Pro Pro Thr Glu Trp Thr Phe Leu Tyr Ala Pro Arg Thr Glu Glu
    210                 215                 220

Glu Val Lys Val Val Glu Thr Ile Val Glu Ala Ser Ile Ala Tyr Met
225                 230                 235                 240

Thr Asn Ala Glu Lys Pro Val Glu Leu Val Gln
                245                 250

<210>  99
<211>  801
<212>  DNA
<213>  Artificial sequence

<220>
<223>  Optimized for mammalian expression
       (humanized) nucleic acid, encoding protein SEQ ID
       No:80

<400>  99
atgcgcatta acatctccct ttcatctctt ttcgagcgat tgagcaaact gagttccagg      60

agtattgcaa tcacttgtgg ggttgtcctc gcgagcgcca tcgcattccc catcatccgg     120

agagattatc agacgtttct tgaggtgggc cctagctatg caccacagaa cttccgagga     180

tatatcatcg tgtgtgtact gtcactgttt aggcaagaac aaaagggatt ggctatctat     240

gataggttgc ctgagaaacg gcggtggctc gctgatctcc catttagaga ggggacacga     300

ccgagcatca cttcacacat catacaaaga cagcgaacgc agctcgttga ccaagagttc     360

gcaactaggg aactgattga taaggtgata cccagagtac aggcgcgaca caccgataaa     420

acttttcttt ccacctctaa attcgagttc catgccaaag ctattttctt gttgccttcc     480

ataccgatta atgatcctct gaatattcca tcccacgaca cagttcgacg gacgaaacgc     540

gaaattgcgc acatgcacga ctatcacgat tgcactttgc acctggcact ggctgctcaa     600

gacggaaaag aagttctgaa aaagggttgg gggcaaagac atccgctggc gggacccggt     660

gtacctgggc cgcctacgga atggacattt ttgtacgcac cgaggaacga agaggaggcc     720

agggtcgttg agatgattgt tgaggctagt attgggtaca tgacgaatga tccggctggt     780

aaaattgttg aaaatgcaaa g                                               801

<210>  100
<211>  1266
<212>  DNA
<213>  Artificial sequence

<220>
<223>  Optimized for mammalian expression
       (humanized) nucleic acid, encoding protein SEQ ID
       No:2

<400>  100
atggcgtcat tcgagaacag tcttagcgtg ctgatcgtcg gcgctgggct cggcggtctt      60

gctgcggcaa tcgccctcag gcgacaagga cacgttgtta aaatctatga cagctcatca     120

tttaaggcag agttgggcgc aggcctcgcg gtccccccaa acactttgag atcactgcaa 180

caactgggtt gtaatactga gaaccttaac ggcgtggata acctctgctt cactgcaatg 240

ggttacgatg gcagtgtggg tatgatgaac aatatgaccg attataggga ggcgtacggc 300

actagctgga taatggtcca tcgggttgat ctccacaatg agcttatgcg cgtagcgttg 360

gatccgggcg gattgggacc cccagctacc ttgcacttga atcaccgcgt gactttttgt 420

gatgtcgacg catgcacagt aaccttcacc aatgggacga ctcagtcagc ggatctcatc 480

gtcggcgccg acggtatacg atccactatc cgcagattcg tcctggagga agatgtcaca 540

gttccggcat ccggaatcgt tggtttccgc tggctcgtcc aggctgatgc tttggatcct 600

taccctgaac ttgactggat tgttaaaaag ccccctctcg gcgctaggtt gataagtacg 660

cctcaaaacc cgcagtctgg ggtaggtctc gcggatcgca gaacgatcat tatatacgcg 720

tgtcgaggag gtactatggt aaacgtactt gccgtccatg acgatgagag ggatcagaat 780

acggcagatt ggtccgtgcc agctagcaag gatgatcttt tcagagtttt tcacgactat 840

catcctcgat ttcggcggct gttggagttg gcgcaagaca tcaatctgtg gcagatgcgg 900

gtggtccccg ttctgaagaa atgggtgaac aaaagagtct gtctcttggg ggatgcagcg 960

catgcgtccc tccctacctt ggggcagggt ttcggcatgg ggttggagga cgccgtagcc 1020

cttgggactt tgcttccaaa ggggacgaca gcatcccaaa tagaaacaag acttgccgta 1080

tatgagcagc tccgaaaaga tcgcgccgag ttcgtcgcgg ctgagtccta cgaagaacaa 1140

tatgtaccag aaatgagggg actttacctg cgatccaaag aattgcgcga ccgggtaatg 1200

ggctatgaca taaaggtgga gtccgaaaag gtcttggaga cactgttgcg gtcaagcaat 1260

tccgcc 1266

<210> 101
<211> 2112
<212> DNA
<213> Artificial sequence

<220>
<223> Nucleic acid encoding the fusion protein
      Hispidin-hydroxylase and luciferase

<400> 101
atggcatcgt ttgagaattc tctaagcgtt ttgattgtcg gggccggact tggtgggctt 60

gctgctgcca tcgcgctgcg tcgccaaggg catgtcgtga aaatatacga ctcctctagc 120

ttcaaagccg aacttggtgc gggactcgct gtgccgccta acaccttgcg cagtctacag 180

caacttggtt gcaataccga gaacctcaat ggtgtggata atctttgctt cactgcgatg 240

gggtatgacg ggagtgtagg gatgatgaac aacatgactg actatcgaga ggcatacggt 300

acttcttgga tcatggtcca ccgcgttgac ttgcataacg agctgatgcg cgtagcactt 360

gatccaggtg ggctcggacc tcctgcgaca ctccatctta atcatcgtgt cacattctgc 420

gatgtcgacg cttgcaccgt gacattcacc aacggaccca ctcaatcagc tgatctcatc 480

gttggtgcag acggtatacg ctctaccatt cggcggtttg tcttagaaga agacgtgact 540

gtgcctgcgt caggaatcgt cgggtttcga tggcttgtac aagctgacgc gctggaccca 600

tatcctgaac tcgactggat tgttaaaaag cctcctctag gcgcgcgact gatctccact 660

cctcagaatc cacagtctgg tgttggcttg gctgacaggc gcactatcat catctacgca 720

tgtcgtggcg gcaccatggt caatgtcctt gcagtgcatg atgacgaacg tgaccagaac 780

accgcagatt ggagtgtacc ggcttccaaa gacgatctat ttcgtgtttt ccacgattac 840

catccacgct ttcggcggct tttagagctt gcgcaggata ttaatctctg gcaaatgcgt 900

gttgtacctg ttttgaaaaa atgggttaac aagcgggttt gcttgttagg agatgctgcg 960

cacgcttctt taccgacgtt gggtcaaggt tttggtatgg gcctggaaga tgccgtagca 1020

cttggtacac tccttccaaa gggtaccact gcatctcaga tcgagactcg acttgcggtg 1080

tacgaacagc tacgtaagga tcgtgcggaa tttgttgcgg ctgaatcata tgaagagcaa 1140

tatgttcctg aaatgcgggg actttatctg aggtcaaagg aactgcgtga tagagtcatg 1200

ggttatgata tcaaagtgga gagcgagaag gttctcgaga cgctcctaag aagttctaat 1260

tctgcctccg gaactggggg caacgcctct gacggtggtg ggtctggtgg tatgcgcatt 1320

aacattagcc tctcgtctct cttcgaacgt ctctccaaac ttagcagtcg cagcatagcg 1380

attacatgtg gagttgttct cgcctccgca atcgcctttc ccatcatccg cagagactac 1440

cagactttcc tagaagtggg accctcgtac gctccgcaga actttagagg atacatcatc 1500

gtctgtgtcc tctcgctatt ccgccaagag cagaaagggc tcgccatcta tgatcgtctt 1560

cccgagaaac gcaggtggtt ggccgacctt ccctttcgtg aaggaaccag acccagcatt 1620

accagccata tcattcagcg acagcgcact caactggtcg atcaggagtt tgccaccagg 1680

gagctcatag acaaggtcat ccctcgcgtg caagcacgac acaccgacaa aacgttcctc 1740

agcacatcaa agttcgagtt tcatgcgaag gccatatttc tcttgccttc tatcccaatc 1800

aacgaccctc tgaatatccc tagccacgac actgtccgcc gaacgaagcg cgagattgca 1860

catatgcatg attatcatga ttgcacactt catcttgctc tcgctgcgca ggatggaaag 1920

gaggtgctga agaaaggttg ggacaacgac atcctttggg ctggtcctgg agttcctggt 1980

ccaccaacgg aatggacttt tctttatgcg cctcgcaacg aagaagaggc tcgagtagtg 2040

gagatgatcg ttgaggcttc catagggtat atgacgaacg atcctgcagg aaagattgta 2100

gaaaacgcca ag 2112

<210> 102
<211> 704

<212>  PRT
<213>  Artificial sequence

<220>
<223>  Fusion protein amino acid sequence
       hispidin-hydroxylase and luciferase

<400>  102

Met Ala Ser Phe Glu Asn Ser Leu Ser Val Leu Ile Val Gly Ala Gly
1               5                   10                  15

Leu Gly Gly Leu Ala Ala Ala Ile Ala Leu Arg Arg Gln Gly His Val
            20                  25                  30

Val Lys Ile Tyr Asp Ser Ser Ser Phe Lys Ala Glu Leu Gly Ala Gly
        35                  40                  45

Leu Ala Val Pro Pro Asn Thr Leu Arg Ser Leu Gln Gln Leu Gly Cys
    50                  55                  60

Asn Thr Glu Asn Leu Asn Gly Val Asp Asn Leu Cys Phe Thr Ala Met
65                  70                  75                  80

Gly Tyr Asp Gly Ser Val Gly Met Met Asn Asn Met Thr Asp Tyr Arg
                85                  90                  95

Glu Ala Tyr Gly Thr Ser Trp Ile Met Val His Arg Val Asp Leu His
            100                 105                 110

Asn Glu Leu Met Arg Val Ala Leu Asp Pro Gly Gly Leu Gly Pro Pro
        115                 120                 125

Ala Thr Leu His Leu Asn His Arg Val Thr Phe Cys Asp Val Asp Ala
    130                 135                 140

Cys Thr Val Thr Phe Thr Asn Gly Thr Thr Gln Ser Ala Asp Leu Ile
145                 150                 155                 160

Val Gly Ala Asp Gly Ile Arg Ser Thr Ile Arg Arg Phe Val Leu Glu
                165                 170                 175

Glu Asp Val Thr Val Pro Ala Ser Gly Ile Val Gly Phe Arg Trp Leu
            180                 185                 190

Val Gln Ala Asp Ala Leu Asp Pro Tyr Pro Glu Leu Asp Trp Ile Val
        195                 200                 205

Lys Lys Pro Pro Leu Gly Ala Arg Leu Ile Ser Thr Pro Gln Asn Pro
    210                 215                 220

Gln Ser Gly Val Gly Leu Ala Asp Arg Arg Thr Ile Ile Ile Tyr Ala
225                 230                 235                 240

Cys Arg Gly Gly Thr Met Val Asn Val Leu Ala Val His Asp Asp Glu
                245                 250                 255

Arg Asp Gln Asn Thr Ala Asp Trp Ser Val Pro Ala Ser Lys Asp Asp
            260                 265                 270

Leu Phe Arg Val Phe His Asp Tyr His Pro Arg Phe Arg Arg Leu Leu
        275                 280                 285

Glu Leu Ala Gln Asp Ile Asn Leu Trp Gln Met Arg Val Val Pro Val
    290                 295                 300

Leu Lys Lys Trp Val Asn Lys Arg Val Cys Leu Leu Gly Asp Ala Ala
305                 310                 315                 320

His Ala Ser Leu Pro Thr Leu Gly Gln Gly Phe Gly Met Gly Leu Glu
                325                 330                 335

Asp Ala Val Ala Leu Gly Thr Leu Leu Pro Lys Gly Thr Thr Ala Ser
            340                 345                 350

Gln Ile Glu Thr Arg Leu Ala Val Tyr Glu Gln Leu Arg Lys Asp Arg
        355                 360                 365

Ala Glu Phe Val Ala Ala Glu Ser Tyr Glu Glu Gln Tyr Val Pro Glu
    370                 375                 380

Met Arg Gly Leu Tyr Leu Arg Ser Lys Glu Leu Arg Asp Arg Val Met
385                 390                 395                 400

Gly Tyr Asp Ile Lys Val Glu Ser Glu Lys Val Leu Glu Thr Leu Leu
                405                 410                 415

Arg Ser Ser Asn Ser Ala Ser Gly Thr Gly Gly Asn Ala Ser Asp Gly
            420                 425                 430

Gly Gly Ser Gly Gly Met Arg Ile Asn Ile Ser Leu Ser Ser Leu Phe
        435                 440                 445

Glu Arg Leu Ser Lys Leu Ser Ser Arg Ser Ile Ala Ile Thr Cys Gly
    450                 455                 460

Val Val Leu Ala Ser Ala Ile Ala Phe Pro Ile Ile Arg Arg Asp Tyr

465                 470                 475                 480

Gln Thr Phe Leu Glu Val Gly Pro Ser Tyr Ala Pro Gln Asn Phe Arg
                485                 490                 495

Gly Tyr Ile Ile Val Cys Val Leu Ser Leu Phe Arg Gln Glu Gln Lys
            500                 505                 510

Gly Leu Ala Ile Tyr Asp Arg Leu Pro Glu Lys Arg Arg Trp Leu Ala
        515                 520                 525

Asp Leu Pro Phe Arg Glu Gly Thr Arg Pro Ser Ile Thr Ser His Ile
    530                 535                 540

Ile Gln Arg Gln Arg Thr Gln Leu Val Asp Gln Glu Phe Ala Thr Arg
545                 550                 555                 560

Glu Leu Ile Asp Lys Val Ile Pro Arg Val Gln Ala Arg His Thr Asp
                565                 570                 575

Lys Thr Phe Leu Ser Thr Ser Lys Phe Glu Phe His Ala Lys Ala Ile
            580                 585                 590

Phe Leu Leu Pro Ser Ile Pro Ile Asn Asp Pro Leu Asn Ile Pro Ser
        595                 600                 605

His Asp Thr Val Arg Arg Thr Lys Arg Glu Ile Ala His Met His Asp
    610                 615                 620

Tyr His Asp Cys Thr Leu His Leu Ala Leu Ala Ala Gln Asp Gly Lys
625                 630                 635                 640

Glu Val Leu Lys Lys Gly Trp Gly Gln Arg His Pro Leu Ala Gly Pro
                645                 650                 655

Gly Val Pro Gly Pro Pro Thr Glu Trp Thr Phe Leu Tyr Ala Pro Arg
            660                 665                 670

Asn Glu Glu Glu Ala Arg Val Val Glu Met Ile Val Glu Ala Ser Ile
        675                 680                 685

Gly Tyr Met Thr Asn Asp Pro Ala Gly Lys Ile Val Glu Asn Ala Lys
    690                 695                 700

<210>  103
<211>  1266
<212>  DNA
<213>  Artificial sequence

<220>
<223>  Nucleic acid optimized for expression in plants,
       encoding protein SEQ ID No:2

<400>  103
atggcatctt ttgagaattc tctgagcgtt ttgattgtcg gggccggact tggtgggctt      60

gctgctgcca tcgccctgcg tcgacaaggg catgtcgtga aaatatacga ctcctctagc     120

ttcaaagccg aacttggtgc tggactcgct gtgccgccta acaccttgcg tagtctccag     180

caacttggtt gcaataccga gaacctcaat ggtgtggata atctttgctt cactgctatg     240

gggtatgacg ggagtgtagg gatgatgaac aacatgactg actatcgaga ggcatacggt     300

acttcttgga tcatggtcca cagagttgac ttgcataacg agctgatgag ggtagcactt     360

gatccaggtg ggctcggacc tcctgcaaca ctccatctta atcatcgtgt cacattctgc     420

gatgtcgacg cttgcaccgt gacattcacc aacgggacca ctcaatcagc tgatctcatc     480

gttggtgcag acggtataag atctaccatt cgaaggtttg tcctggaaga agacgtgact     540

gtgcctgcat caggaatcgt cgggtttcga tggcttgtac aagctgacgc tctggaccca     600

tatcctgaac tcgactggat tgttaaaaag cctcctcttg gcgcacgact gatctccact     660

cctcagaatc cacagtctgg tgttggcttg gctgacagga ggactatcat catctacgca     720

tgtcgtggcg gcaccatggt caatgtcctt gcagtgcatg atgacgaacg tgaccagaac     780

accgcagatt ggagtgtacc ggcttccaaa gacgatctct ttcgtgtttt ccacgattac     840

catccacgat ttaggaggct tctcgagctt gctcaggata ttaatctctg gcaaatgcgt     900

gttgtacctg ttttgaaaaa atgggttaac aagagggttt gcttgctcgg agatgctgca     960

cacgcttctc tgccgacgtt gggtcaaggt tttggtatgg gtctggaaga tgccgtagca    1020

cttggtacac tccttccaaa gggtaccact gcatctcaga tcgagactcg acttgctgtg    1080

tacgaacagt tgcgtaagga tcgtgccgaa tttgttgccg ctgaatcata tgaagagcaa    1140

tatgttcctg aaatgcgagg actttatctg aggtcaaagg aactgcgtga tagagtcatg    1200

ggttatgata tcaaagtgga gagcgagaag gttctcgaga cgctccttag aagttctaat    1260

tctgcc                                                               1266


<210>  104
<211>  1032
<212>  DNA
<213>  Aspergillus nidulans

<400>  104
atggtgcagg acacgtcatc tgcttctact tcccccattt tgacacgttg gtacatcgac      60

acaagaccct taacagcatc cacggcagca cttcctcttc tggaaacgtt gcagcctgca     120

gatcagatta gtgtacagaa gtattatcat ttaaaggata aacatatgag tcttgcttct     180

aacttattga agtatctgtt tgttcatcgt aattgtcgta ttccctggag ttctatcgtg 240

atctcccgta cacctgatcc tcaccgtcgt ccatgctaca ttcctccctc aggctctcag 300

gaagattcct tcaaagacgg atacactggt atcaatgttg agttcaatgt atcacaccaa 360

gccagtatgg tcgccattgc tggaaccgca tttactccca attctggtgg cgattctaaa 420

ctgaagcctg aggtgggaat cgacatcaca tgtgtaaacg agcgtcaggg ccgtaacggt 480

gaggagcgtt cattggaatc tcttcgtcaa tatatcgaca tcttctctga agtattctcc 540

acggctgaga tggcaaacat ccgtcgtctt gatggcgtta gtagttcttc tttaagtgct 600

gatcgtcttg ttgactatgg ttaccgtctt ttttatacat actgggccct gaaggaagcc 660

tatatcaaga tgactggtga agcattgctt gcaccatggt tacgtgagct ggagttctcc 720

aatgttgttg cccctgccgc tgttgctgaa tccggtgact ctgccggcga cttcggtgag 780

ccatacacag gagtccgtac gactttatat aaaaatttag ttgaggatgt gcgtatcgag 840

gtagccgccc ttggtggcga ttatcttttt gcaaccgcag ctcgtggagg aggaattgga 900

gccagttcaa ggcccggagg cggccctgat ggtagtggta tccgttcaca ggacccttgg 960

cgtccattta agaaacttga tatcgagcgt gacattcagc catgtgcaac aggtgtatgc 1020

aactgcctta gt 1032

<210> 105
<211> 344
<212> PRT
<213> Aspergillus nidulans

<400> 105

Met Val Gln Asp Thr Ser Ser Ala Ser Thr Ser Pro Ile Leu Thr Arg
1               5                   10                  15

Trp Tyr Ile Asp Thr Arg Pro Leu Thr Ala Ser Thr Ala Ala Leu Pro
            20                  25                  30

Leu Leu Glu Thr Leu Gln Pro Ala Asp Gln Ile Ser Val Gln Lys Tyr
        35                  40                  45

Tyr His Leu Lys Asp Lys His Met Ser Leu Ala Ser Asn Leu Leu Lys
    50                  55                  60

Tyr Leu Phe Val His Arg Asn Cys Arg Ile Pro Trp Ser Ser Ile Val
65                  70                  75                  80

Ile Ser Arg Thr Pro Asp Pro His Arg Arg Pro Cys Tyr Ile Pro Pro
                85                  90                  95

Ser Gly Ser Gln Glu Asp Ser Phe Lys Asp Gly Tyr Thr Gly Ile Asn

            100                 105                 110

Val Glu Phe Asn Val Ser His Gln Ala Ser Met Val Ala Ile Ala Gly
        115                 120                 125

Thr Ala Phe Thr Pro Asn Ser Gly Gly Asp Ser Lys Leu Lys Pro Glu
    130                 135                 140

Val Gly Ile Asp Ile Thr Cys Val Asn Glu Arg Gln Gly Arg Asn Gly
145                 150                 155                 160

Glu Glu Arg Ser Leu Glu Ser Leu Arg Gln Tyr Ile Asp Ile Phe Ser
                165                 170                 175

Glu Val Phe Ser Thr Ala Glu Met Ala Asn Ile Arg Arg Leu Asp Gly
            180                 185                 190

Val Ser Ser Ser Ser Leu Ser Ala Asp Arg Leu Val Asp Tyr Gly Tyr
        195                 200                 205

Arg Leu Phe Tyr Thr Tyr Trp Ala Leu Lys Glu Ala Tyr Ile Lys Met
    210                 215                 220

Thr Gly Glu Ala Leu Leu Ala Pro Trp Leu Arg Glu Leu Glu Phe Ser
225                 230                 235                 240

Asn Val Val Ala Pro Ala Ala Val Ala Glu Ser Gly Asp Ser Ala Gly
                245                 250                 255

Asp Phe Gly Glu Pro Tyr Thr Gly Val Arg Thr Thr Leu Tyr Lys Asn
            260                 265                 270

Leu Val Glu Asp Val Arg Ile Glu Val Ala Ala Leu Gly Gly Asp Tyr
        275                 280                 285

Leu Phe Ala Thr Ala Ala Arg Gly Gly Gly Ile Gly Ala Ser Ser Arg
    290                 295                 300

Pro Gly Gly Gly Pro Asp Gly Ser Gly Ile Arg Ser Gln Asp Pro Trp
305                 310                 315                 320

Arg Pro Phe Lys Lys Leu Asp Ile Glu Arg Asp Ile Gln Pro Cys Ala
                325                 330                 335

Thr Gly Val Cys Asn Cys Leu Ser
            340

<210>  106
<211>  1593
<212>  DNA
<213>  Rhodobacter capsulatus

<400>  106
atgactcttc aatcccaaac tgctaaggat tgcttggcac tggatggtgc actgactttg      60

gtgcagtgcg aggcaatcgc cacgcatcgt agtcgtattt ccgttacgcc agccttgcgt     120

gaacgttgtg ctcgtgccca cgcacgtttg gagcacgcaa tcgcagagca gcgtcatatt     180

tacggtatca ccactggatt tggacccctg gctaaccgtc ttattggagc cgatcagggc     240

gctgagctgc aacagaatct gatttaccat cttgcaacgg gcgttggacc caagctgtcc     300

tgggcagagg cccgtgcact tatgttggca cgtcttaatt ccattttgca gggcgctagt     360

ggtgcttctc ctgaaacgat cgaccgtatc gtcgcagtac ttaacgctgg ctttgctcct     420

gaagttcccg cacaaggtac tgttggcgca agtggcgact tgactcccct tgcccacatg     480

gtactggccc tgcaaggccg tggacgtatg atcgatccta gtggacgtgt gcaagaggct     540

ggagccgtca tggatcgtct ttgcggaggc cctcttacgt tggccgcacg tgacggcctt     600

gctctggtca acggtacaag tgctatgact gctatcgctg ctcttactgg agtcgaggcc     660

gctcgtgcta tcgatgctgc acttcgtcac tccgccgttt tgatggaagt cctgtctgga     720

catgcagaag cctggcaccc cgcctttgca gagttgagac cccacccagg ccagttgcgt     780

gctactgaac gtttggccca ggccctggat ggcgcaggcc gtgtatgccg tactctgacg     840

gctgcccgtc gtcttactgc agccgacctg cgtccagagg accaccccgc acaagacgct     900

tattctctgc gtgtcgttcc tcaactggtt ggtgccgtat gggatactct tgactggcac     960

gaccgtgtcg tgacctgtga gcttaattca gtaaccgata accccatttt tccagagggt    1020

tgcgccgttc ctgcacttca cggaggaaac ttcatgggag tccacgtcgc actggcttca    1080

gacgctttga atgcagccct ggttaccctt gccggtcttg tagagcgtca aattgcccgt    1140

cttaccgacg agaaacttaa taaaggtctg cctgctttcc ttcacggtgg acaggcaggc    1200

cttcagagtg gcttcatggg cgctcaggtt acagcaactg ccctgttggc tgagatgcgt    1260

gcaaatgcaa ctccagtctc agtacaatcc ttgtccacaa acggtgcaaa ccaagacgtc    1320

gtatctatgg gaactatcgc cgcacgtcgt gctcgtgcac aattgttgcc actgtcacag    1380

attcaggcta tcttggcttt ggcattggcc caggctatgg atcttcttga cgatcccgag    1440

ggtcaagccg gatggtcttt gacagcccgt gatttgcgtg accgtatccg tgccgtttct    1500

cccggactgc gtgccgaccg tccactggca ggccatatcg aagctgttgc tcaggacttt    1560

cgtcacccat ctgctgcagc cgatccacca gcc                                 1593

<210>  107
<211>  531

<212>  PRT
<213>  Rhodobacter capsulatus

<400>  107

Met Thr Leu Gln Ser Gln Thr Ala Lys Asp Cys Leu Ala Leu Asp Gly
1               5                   10                  15

Ala Leu Thr Leu Val Gln Cys Glu Ala Ile Ala Thr His Arg Ser Arg
            20                  25                  30

Ile Ser Val Thr Pro Ala Leu Arg Glu Arg Cys Ala Arg Ala His Ala
        35                  40                  45

Arg Leu Glu His Ala Ile Ala Glu Gln Arg His Ile Tyr Gly Ile Thr
    50                  55                  60

Thr Gly Phe Gly Pro Leu Ala Asn Arg Leu Ile Gly Ala Asp Gln Gly
65                  70                  75                  80

Ala Glu Leu Gln Gln Asn Leu Ile Tyr His Leu Ala Thr Gly Val Gly
                85                  90                  95

Pro Lys Leu Ser Trp Ala Glu Ala Arg Ala Leu Met Leu Ala Arg Leu
            100                 105                 110

Asn Ser Ile Leu Gln Gly Ala Ser Gly Ala Ser Pro Glu Thr Ile Asp
        115                 120                 125

Arg Ile Val Ala Val Leu Asn Ala Gly Phe Ala Pro Glu Val Pro Ala
    130                 135                 140

Gln Gly Thr Val Gly Ala Ser Gly Asp Leu Thr Pro Leu Ala His Met
145                 150                 155                 160

Val Leu Ala Leu Gln Gly Arg Gly Arg Met Ile Asp Pro Ser Gly Arg
                165                 170                 175

Val Gln Glu Ala Gly Ala Val Met Asp Arg Leu Cys Gly Gly Pro Leu
            180                 185                 190

Thr Leu Ala Ala Arg Asp Gly Leu Ala Leu Val Asn Gly Thr Ser Ala
        195                 200                 205

Met Thr Ala Ile Ala Ala Leu Thr Gly Val Glu Ala Ala Arg Ala Ile
    210                 215                 220

Asp Ala Ala Leu Arg His Ser Ala Val Leu Met Glu Val Leu Ser Gly
225                 230                 235                 240

His Ala Glu Ala Trp His Pro Ala Phe Ala Glu Leu Arg Pro His Pro
                245                 250                 255

Gly Gln Leu Arg Ala Thr Glu Arg Leu Ala Gln Ala Leu Asp Gly Ala
            260                 265                 270

Gly Arg Val Cys Arg Thr Leu Thr Ala Ala Arg Arg Leu Thr Ala Ala
        275                 280                 285

Asp Leu Arg Pro Glu Asp His Pro Ala Gln Asp Ala Tyr Ser Leu Arg
    290                 295                 300

Val Val Pro Gln Leu Val Gly Ala Val Trp Asp Thr Leu Asp Trp His
305                 310                 315                 320

Asp Arg Val Val Thr Cys Glu Leu Asn Ser Val Thr Asp Asn Pro Ile
                325                 330                 335

Phe Pro Glu Gly Cys Ala Val Pro Ala Leu His Gly Gly Asn Phe Met
            340                 345                 350

Gly Val His Val Ala Leu Ala Ser Asp Ala Leu Asn Ala Ala Leu Val
        355                 360                 365

Thr Leu Ala Gly Leu Val Glu Arg Gln Ile Ala Arg Leu Thr Asp Glu
    370                 375                 380

Lys Leu Asn Lys Gly Leu Pro Ala Phe Leu His Gly Gly Gln Ala Gly
385                 390                 395                 400

Leu Gln Ser Gly Phe Met Gly Ala Gln Val Thr Ala Thr Ala Leu Leu
                405                 410                 415

Ala Glu Met Arg Ala Asn Ala Thr Pro Val Ser Val Gln Ser Leu Ser
            420                 425                 430

Thr Asn Gly Ala Asn Gln Asp Val Val Ser Met Gly Thr Ile Ala Ala
        435                 440                 445

Arg Arg Ala Arg Ala Gln Leu Leu Pro Leu Ser Gln Ile Gln Ala Ile
    450                 455                 460

Leu Ala Leu Ala Leu Ala Gln Ala Met Asp Leu Leu Asp Asp Pro Glu
465                 470                 475                 480

Gly Gln Ala Gly Trp Ser Leu Thr Ala Arg Asp Leu Arg Asp Arg Ile

485                 490                 495

Arg Ala Val Ser Pro Gly Leu Arg Ala Asp Arg Pro Leu Ala Gly His
            500                 505                 510

Ile Glu Ala Val Ala Gln Gly Leu Arg His Pro Ser Ala Ala Ala Asp
        515                 520                 525

Pro Pro Ala
    530

<210>  108
<211>  1560
<212>  DNA
<213>  Escherichia coli

<400>  108
atgaaacctg aagattttcg tgcttccacc cagcggccct ttactggaga agaatatctg      60

aagtctcttc aagacggccg tgagatctat atttacggcg agcgtgtaaa ggatgttaca     120

acgcaccctg cttttcgtaa tgctgctgcc tcagttgccc aactttatga cgccctgcat     180

aaacccgaaa tgcaggactc actttgttgg aatacggaca cggggtccgg tggttacaca     240

cacaagttct ttcgtgtcgc caaatctgcc gatgacttgc gtcaacaacg tgatgctatc     300

gctgaatggt cacgtctgag ttacggctgg atgggccgta cgcccgacta taaggccgcc     360

tttggttgcg cattgggtgc aaatcccggc ttttatggac agtttgaaca aaatgcacgt     420

aactggtata ctcgtatcca ggaaaccggc ttgtacttta atcatgctat tgttaaccca     480

cctatcgatc gtcatttgcc aaccgataag gtcaaggatg tatatattaa acttgaaaag     540

gagacggacg ctggtatcat tgtgtctggc gctaaagtgg tggctacgaa cagtgcattg     600

acgcattaca atatgattgg ttttggttcc gcccaagtga tgggcgagaa ccccgacttt     660

gccttgatgt ttgttgcccc tatggacgct gacggagtaa aacttatttc acgtgcctca     720

tatgaaatgg tagctggtgc cactggttct ccttatgatt atcctctttc atcccgtttt     780

gacgaaaacg acgccatcct ggtcatggac aatgttctta ttccctggga gaacgtgctg     840

atctaccgtg acttcgatcg ttgccgtcgt tggactatgg aaggaggttt cgctcgtatg     900

tatccactgc aggcctgtgt tcgtttggct gtgaagttgg actttatcac agcccttctt     960

aaaaagtcac ttgagtgtac tggaacgttg gagttccgtg gtgtacaggc agatctgggc    1020

gaagtagtag cctggcgtaa tactttctgg gcactgtctg actctatgtg ctccgaggca    1080

acaccatggg tgaacggcgc atacctgccc gatcacgccg ctcttcaaac atatcgtgtg    1140

cttgctccca tggcttatgc taaaatcaaa aatattatcg agcgtaatgt aacttctggt    1200

ctgatttacc tgccatccag tgctcgtgac cttaacaatc ctcaaatcga tcagtatctt    1260

gcaaaatatg tgcgtggctc aaacggaatg gaccatgtgc agcgtatcaa aatcctgaag 1320

ttgatgtggg atgccatcgg ttctgaattt ggcggacgtc atgaactgta tgagattaac 1380

tactctggat ctcaagacga aatcagactt cagtgtttgc gtcaggccca aaattctgga 1440

aatatggata aaatgatggc tatggtagac cgttgtctga gtgagtacga tcaagatggt 1500

tggacagttc ctcatcttca taataatgac gatattaata tgcttgataa gttgcttaaa 1560

<210> 109
<211> 520
<212> PRT
<213> Escherichia coli

<400> 109

Met Lys Pro Glu Asp Phe Arg Ala Ser Thr Gln Arg Pro Phe Thr Gly
1               5                   10                  15

Glu Glu Tyr Leu Lys Ser Leu Gln Asp Gly Arg Glu Ile Tyr Ile Tyr
            20                  25                  30

Gly Glu Arg Val Lys Asp Val Thr Thr His Pro Ala Phe Arg Asn Ala
        35                  40                  45

Ala Ala Ser Val Ala Gln Leu Tyr Asp Ala Leu His Lys Pro Glu Met
    50                  55                  60

Gln Asp Ser Leu Cys Trp Asn Thr Asp Thr Gly Ser Gly Gly Tyr Thr
65                  70                  75                  80

His Lys Phe Phe Arg Val Ala Lys Ser Ala Asp Asp Leu Arg Gln Gln
                85                  90                  95

Arg Asp Ala Ile Ala Glu Trp Ser Arg Leu Ser Tyr Gly Trp Met Gly
            100                 105                 110

Arg Thr Pro Asp Tyr Lys Ala Ala Phe Gly Cys Ala Leu Gly Ala Asn
        115                 120                 125

Pro Gly Phe Tyr Gly Gln Phe Glu Gln Asn Ala Arg Asn Trp Tyr Thr
    130                 135                 140

Arg Ile Gln Glu Thr Gly Leu Tyr Phe Asn His Ala Ile Val Asn Pro
145                 150                 155                 160

Pro Ile Asp Arg His Leu Pro Thr Asp Lys Val Lys Asp Val Tyr Ile
                165                 170                 175

Lys Leu Glu Lys Glu Thr Asp Ala Gly Ile Ile Val Ser Gly Ala Lys

            180                 185                 190

Val Val Ala Thr Asn Ser Ala Leu Thr His Tyr Asn Met Ile Gly Phe
        195                 200                 205

Gly Ser Ala Gln Val Met Gly Glu Asn Pro Asp Phe Ala Leu Met Phe
    210                 215                 220

Val Ala Pro Met Asp Ala Asp Gly Val Lys Leu Ile Ser Arg Ala Ser
225                 230                 235                 240

Tyr Glu Met Val Ala Gly Ala Thr Gly Ser Pro Tyr Asp Tyr Pro Leu
                245                 250                 255

Ser Ser Arg Phe Asp Glu Asn Asp Ala Ile Leu Val Met Asp Asn Val
            260                 265                 270

Leu Ile Pro Trp Glu Asn Val Leu Ile Tyr Arg Asp Phe Asp Arg Cys
        275                 280                 285

Arg Arg Trp Thr Met Glu Gly Gly Phe Ala Arg Met Tyr Pro Leu Gln
    290                 295                 300

Ala Cys Val Arg Leu Ala Val Lys Leu Asp Phe Ile Thr Ala Leu Leu
305                 310                 315                 320

Lys Lys Ser Leu Glu Cys Thr Gly Thr Leu Glu Phe Arg Gly Val Gln
                325                 330                 335

Ala Asp Leu Gly Glu Val Val Ala Trp Arg Asn Thr Phe Trp Ala Leu
            340                 345                 350

Ser Asp Ser Met Cys Ser Glu Ala Thr Pro Trp Val Asn Gly Ala Tyr
        355                 360                 365

Leu Pro Asp His Ala Ala Leu Gln Thr Tyr Arg Val Leu Ala Pro Met
    370                 375                 380

Ala Tyr Ala Lys Ile Lys Asn Ile Ile Glu Arg Asn Val Thr Ser Gly
385                 390                 395                 400

Leu Ile Tyr Leu Pro Ser Ser Ala Arg Asp Leu Asn Asn Pro Gln Ile
                405                 410                 415

Asp Gln Tyr Leu Ala Lys Tyr Val Arg Gly Ser Asn Gly Met Asp His
            420                 425                 430

Val Gln Arg Ile Lys Ile Leu Lys Leu Met Trp Asp Ala Ile Gly Ser
      435                 440                 445

Glu Phe Gly Gly Arg His Glu Leu Tyr Glu Ile Asn Tyr Ser Gly Ser
    450                 455                 460

Gln Asp Glu Ile Arg Leu Gln Cys Leu Arg Gln Ala Gln Asn Ser Gly
465                 470                 475                 480

Asn Met Asp Lys Met Met Ala Met Val Asp Arg Cys Leu Ser Glu Tyr
                485                 490                 495

Asp Gln Asp Gly Trp Thr Val Pro His Leu His Asn Asn Asp Asp Ile
            500                 505                 510

Asn Met Leu Asp Lys Leu Leu Lys
      515                 520

<210>  110
<211>  510
<212>  DNA
<213>  Escherichia coli

<400>  110
atgcagcttg atgagcagcg tttgcgtttt cgtgacgcta tggcatcact tagtgccgct      60

gtgaacatca tcacgactga gggagacgcc ggtcagtgcg gaatcacagc aaccgcagta     120

tgtagtgtta cggacactcc cccatctctg atggtctgta tcaatgccaa tagtgctatg     180

aatccagtct ttcagggtaa tggtaaactt tgtgtcaacg tcctgaatca cgagcaagag     240

ttgatggctc gtcattttgc aggaatgact ggtatggcta tggaagagcg tttttccttg     300

tcttgttggc aaaaaggacc actggcccag cctgtattga aaggttcttt ggcttccctg     360

gagggtgaga tccgtgacgt tcaagccatt ggaactcatc tggtatattt ggtagaaatc     420

aagaatatta tcctgtcagc cgagggccat ggtctgatct actttaaacg tcgttttcac     480

cccgtgatgc tggagatgga ggcagccatt                                      510

<210>  111
<211>  170
<212>  PRT
<213>  Escherichia coli

<400>  111

Met Gln Leu Asp Glu Gln Arg Leu Arg Phe Arg Asp Ala Met Ala Ser
1               5                   10                  15

Leu Ser Ala Ala Val Asn Ile Ile Thr Thr Glu Gly Asp Ala Gly Gln
            20                  25                  30

Cys Gly Ile Thr Ala Thr Ala Val Cys Ser Val Thr Asp Thr Pro Pro
        35                  40                  45

Ser Leu Met Val Cys Ile Asn Ala Asn Ser Ala Met Asn Pro Val Phe
    50                  55                  60

Gln Gly Asn Gly Lys Leu Cys Val Asn Val Leu Asn His Glu Gln Glu
65                  70                  75                  80

Leu Met Ala Arg His Phe Ala Gly Met Thr Gly Met Ala Met Glu Glu
                85                  90                  95

Arg Phe Ser Leu Ser Cys Trp Gln Lys Gly Pro Leu Ala Gln Pro Val
            100                 105                 110

Leu Lys Gly Ser Leu Ala Ser Leu Glu Gly Glu Ile Arg Asp Val Gln
        115                 120                 125

Ala Ile Gly Thr His Leu Val Tyr Leu Val Glu Ile Lys Asn Ile Ile
    130                 135                 140

Leu Ser Ala Glu Gly His Gly Leu Ile Tyr Phe Lys Arg Arg Phe His
145                 150                 155                 160

Pro Val Met Leu Glu Met Glu Ala Ala Ile
                165                 170

<210>  112
<211>  801
<212>  DNA
<213>  Artificial sequence

<220>
<223>  Nucleic acid optimized for expression in plants,
       encoding protein SEQ ID No:80

<400>  112
atgaggataa atatctcttt gtctagtctc tttgagagac tgagcaaatt gtcatcccga      60

tccatcgcaa ttacctgcgg cgtagtcttg gcaagtgcaa tagcattccc aattatccga     120

agagactatc aaacgttcct tgaagtcggt ccgagctatg ccccacagaa cttccgaggc     180

tatatcatcg tttgtgtctt gtcacttttt aggcaagagc agaaagggtt ggcaatctat     240

gacaggcttc cagaaaagag gcgttggctt gccgacttgc cgtttcgtga agggacgagg     300

ccatccataa cctcccacat tatacagcgt cagcgtactc agctggtaga tcaagagttt     360

gcaaccagag aacttatcga taaggtgatc ccacgtgtgc aagcaagaca cacagacaaa     420

acttttctca gtacgtcaaa atttgagttt catgcaaagg ccatcttcct tctcccctct     480

atccctatta atgatcctct taacattccc tcacacgata cggtaagaag aaccaagcgt 540

gagatcgctc acatgcatga ttaccatgat tgcactctgc acttggctct tgctgctcag 600

gatggtaagg aagttttgaa gaaggggtgg ggccagcgtc acccactggc cggaccagga 660

gtcccaggcc ctcctactga gtggaccttc ctttacgcac caaggaacga agaggaggcc 720

agagttgtcg agatgatagt cgaagctagt attggctaca tgactaacga tcctgctggt 780

aagattgtcg aaaatgccaa g 801

<210> 113
<211> 5034
<212> DNA
<213> Artificial squence

<220>
<223> Optimized for expression in plants of a nucleic acid,
 encoding protein SEQ ID No:35

<400> 113
   atgaattcca gcaagaatcc tccttccact cttcttgatg tttttctgga tactgccagg 60
   aaccttgata ccgctttgag gaatgtcttg gaatgcggcg aacacagatg gtcctacaga 120
   gagcttgata ctgtttcatc tgctctcgcc cagcatctta ggtacactgt cggtcttagt 180
   cctactgtcg ccgtcatcag tgaaaaccat ccttatattc tcgctttgat gctggctgta 240
   tggaaacttg gaggcacctt cgctcctatt gatgtccatt ctcctgccga attggtagct 300
   ggcatgctga acatagtctc tccttcttgc ttggttattc cgagctcaga tgtaactaat 360
   caaactcttg cctgcgatct taatatcccc gtcgttgcat ttcacccaca tcaatccact 420
   attcctgagc tgaacaagaa gtacctcacc gattctcaaa tttctccgga tcttcctttt 480
   tcagatccaa acagacctgc tctgtacctc ttcacttcat ccgccacttc tcgaagtaat 540
   ctcaaatgcg tgcctctcac tcacaccttt atcctccgaa acagcctctc taagcgtgca 600
   tggtgcaagc gtatgcgtcc agagacagac tttgacggca tacgtgttct tggatgggcc 660
   ccgtggtctc acgtcctcgc acacatgcaa gacatcggac cactcaccct gcttaatgcc 720
   ggatgctacg tttttgcaac tactccatcc acgtacccta cggaattgaa ggacgacagg 780
   gacctgatat cttgcgccgc aaatgctatc atgtacaagg gcgtcaagtc atttgcttgt 840
   cttccctttg tactcggagg gctgaaggca ctctgcgagt ctgagccatc cgtgaaggcc 900
   catcttcagg tcgaggagag agctcaactc ctgaagtctc tgcaacacat ggaaattctt 960
   gagtgtggag gtgccatgct cgaagcaagt gttgcctctt gggctattga gaactgcatt 1020
   cccatttcta tcggtattgg tatgacggag actggtggag ccctctttgc aggccccgtt 1080
   caggccatca aaaccgggtt ttcttcagag gataaattca ttgaagatgc tacttacttg 1140
   ctcgttaagg atgatcatga gagtcatgct gaggaggata ttaacgaggg tgaactcgtt 1200
   gtgaaaagta aaatgctccc acgaggctac cttggctata gtgatccttc cttctcagtc 1260
   gacgatgctg gctgggttac atttagaaca ggagacagat acagcgttac acctgacgga 1320
   aagttttcct ggctgggccg aaacactgat ttcattcaga tgaccagtgg tgagacgctg 1380
   gatccccgac caattgagag cagtctctgc gaaagttctc ttatttctag agcatgcgtt 1440
   atcggagata aatttctcaa cgggcctgct gctgctgttt gtgctatcat tgagcttgag 1500
   cccacagctg tggaaaaagg acaagctcac agccgtgaga tagcaagagt tttcgcacct 1560
   attaatcgag acttgccgcc tcctcttagg attgcatgga gtcacgtttt ggttctccag 1620
   cccagtgaga agataccgat gacgaagaag ggtaccatct tcaggaagaa aattgagcag 1680
   gtgtttggct ctgccttggg tggcagctct ggagataact ctcaagccac tgctgatgct 1740
   ggcgttgttc gacgagacga gctttcaaac actgtcaagc acataattag ccgtgttctc 1800
   ggagtttccg atgacgaact cctttggacg ttgtcatttg ccgagctcgg aatgacgtca 1860
   gcattggcca ctcgaatcgc caacgagttg aacgaagttt tggttggagt taatctccct 1920
   atcaacgctt gctatataca tgtcgacctt ccttctctga gcaatgccgt ctatgccaaa 1980
   cttgcacacc tcaagctgcc agatcgtact cccgaaccca ggcaagcccc tgtcgaaaac 2040
   tctggtggga aggagatcgt tgtcgttggc caggcctttc gtcttcctgg ctcaataaac 2100
   gatgtcgcct ctcttcgaga cgcattcctg gccagacaag catcatccat tatcactgaa 2160
   ataccatccg ataggtggga ccacgccagc ttctatccca aggatatacg tttcaacaag 2220
   gctggccttg tggatatagc caattatgat catagctttt tcggactgac ggcaaccgaa 2280
   gcactctatc tgtccccaac tatgcgtttg gcactcgaag tttcctttga agccttggag 2340
   aatgctaata tcccggtgtc acaactcaag ggttctcaaa cagctgttta tgttgctact 2400
   acagatgacg gatttgagac ccttttgaat gccgaggccg gctatgatgc ttatacaaga 2460
   ttctatggca ctggtcgagc agcaagtaca gccagcgggc gtataagctg tcttcttgat 2520
   gtccatggac cctctattac tgttgatacg gcatgcagtg gaggggctgt ttgtattgac 2580
   caagcaatcg actatctgca atcaagcagt gcagcagaca ccgctatcat atgtgctagt 2640
   aacacgcact gctggccagg cagcttcagg tttctttccg cacaaggatg ggtatcccca 2700
   ggaggacgat gcgcaacatt tacaactgat gctgatggct acgtgccctc tgagggcgct 2760
   gtcgccttca tattgaaaac ccgagaagca gctatgcgtg acaaggacac tatcctcgcc 2820
   acaatcaaag ctacacagat atcacacaat ggccgatctc aaggtcttgt ggcaccgaat 2880
   gtcaacagcc aagctgacct tcatagaagc ttgcttcaaa aagctggcct tagcccggct 2940
   gatatccgtt tcattgaagc tcatggaaca ggaacgtcac tgggagacct ctcagaaatt 3000
   caagctataa atgatgctta tacctcctct cagccgcgta cgaccggccc actcatagtc 3060
   agcgcttcca aaacggtcat tggtcatacc gaaccagctg gccccttggt cggtatgctg 3120
   agtgtcttga actctttcaa agaaggcgcc gtccctggtc tcgcccatct taccgcagac 3180
   aatttgaatc ccagtctgga ctgttcttct gtgccacttc tcattcccta tcaacctgtt 3240
   cacctggctg cacccaagcc tcaccgagct gctgtaaggt catacggctt ttcaggtacc 3300
   ctgggcggca tcgttctcga ggctcctgac gaagaaagac tggaagaaga gctgccaaat 3360
   gacaagccca tgttgttcgt cgtcagcgca aagacacata cagcacttat cgaatacctg 3420
   gggcgttatc tcgagttcct cttgcaggca aacccccaag attttgtgga catttgttat 3480
   acaagctgcg ttgggcgtga gcactataga tatcgatatg cttgtgtagc aaatgatatg 3540
   gaggacctca taggccaact ccagaaacgt ttgggcagca aggtgccgcc aaagccgtca 3600
   tacaaacgtg gtgctttggc ctttgccttt tctggtcagg gtacacaatt ccgagggatg 3660
   gccacagagc ttgcaaaagc atactccggc ttccgaaaga tcgtgtccga tctcgcaaag 3720
   agagctagcg agttgtcagg tcatgccatt gaccgttttc ttcttgcata tgacataggc 3780
   gctgaaaatg tagctcctga tagtgaggca gaccagattt gcatctttgt gtatcagtgt 3840
   tctgtccttc gttggctgca gactatgggg attagaccca gtgcagtgat aggccatagc 3900
   ctcggggaga tctcagcttc tgtggcagca ggagcacttt ctcttgactc cgctttggat 3960
   cttgtcatct cacgagctcg tcttttgagg tcttcagcaa gtgctcctgc aggaatggca 4020
   gctatgtctg cctctcaaga cgaggttgtg gagttgattg ggaaactcga cctcgacaag 4080
   gctaattctc tcagcgtttc agtcataaat ggtccccaaa atactgtcgt gtccggctct 4140
   tcagctgcta ttgaaagcat agtggctctt gccaaaggga gaaagatcaa agcctctgcc 4200
   ctgaatatca atcaagcttt tcatagtcca tacgtcgaca gtgccgtccc tggtctccgt 4260
   gcttggtcag aaaagcatat ctcctcagct agaccattgc aaattccgct gtattcaacg 4320
   ttgttgggag cacaaatctc tgagggagag atgttgaatc cagatcactg ggtcgaccat 4380
   gcacgaaagc ctgtacagtt cgcacaagca gccacaacca tgaaagaatc cttcaccgga 4440
   gtcatcatag atatcggccc tcaagtagtg gcttggtcac ttctgctcag taacgggctc 4500
   acgtccgtga ctgctctcgc tgcaaaaaga gggagaagtc aacaggtggc tttcctcagc 4560
   gccttggccg atttgtatca agattacggt gttgttcctg attttgtcgg gctttatgct 4620
   cagcaggaag atgcttcaag gttgaagaag acggatatct tgacgtatcc gttccagcgt 4680
   ggcgaagaga ctctttctag tggttctagc actccgacat tggaaaacac ggatttggat 4740
   tccggtaagg aactccttat gggaccgact agagggttgc tccgtgctga cgacttgcgt 4800
   gacagtatcg tttcttctgt gaaggatgtt ctggaactca agtcaaatga agacctcgat 4860
   ttgtctgaaa gtctgaatgc acttggtatg gacagcatca tgttcgctca gctccgaaag 4920
   cgtattgggg aaggactcgg attgaatgtt ccgatggttt ttctgagcga cgccttttct 4980
   attggtgaga tggttagtaa tcttgtggaa caggcagagg cttctgagga caat          5034

<210>  114
<211>  867
<212>  DNA
<213>  Artificial sequence

<220>
<223>  Optimized for expression in plants of a nucleic acid,
       encoding protein SEQ ID No:65

<400>  114
atggctccaa tttcttcaac ttggtctcgt ctcattcgat ttgtggctat tgaaacgtcc      60

ctcgtgcata tcggtgaacc gatagacgcc accatggacg tcggtctggc cagacgagaa     120

ggcaagacga tccaagcata cgagattatt ggatcaggca gtgctcttga cctctcagcc     180

caagtaagta agaatgtgct gactgtaagg gaactcctga tgccgctttc aagagaggaa     240

attaaaactg tacgatgctt ggggttgaac taccctgttc atgccaccga agcaaacgtt     300

gctgttccaa aattcccgaa tttgttctac aaaccagtga cctccctcat tggccccgat     360

ggactcatta ccatcccttc cgttgtccaa cccccgaagg agcatcagtc cgattatgaa     420

gcagaacttg tcattgtcat cgggaaagca gcaaagaatg taagtgagga tgaggctttg     480

gattatgtat tgggatacac tgccgctaac gatatttcct ttaggaaaca ccagcttgca     540

gtctcacaat ggtctttctc taaaggattt ggtagccttt tgctcactat ccgtatggca     600

caaacccact ctggtaacat taatagattc tccagagacc agattttcaa tgtcaagaag     660

acaatttcct tcctgtcaca aggcactaca ctggaaccag gttctatcat tttgactggt     720

acacctgacg gagtgggctt tgtgcgaaat ccaccacttt accttaaaga tggagatgaa     780

gtaatgacct ggattggaag tggaatcgga acattggcca atacagtgca agaagagaag     840

acttgcttcg ctagtggcgg acacgag                                         867

<210>  115
<211>  9
<212>  PRT
<213>  Artificial sequence

<220>
<223>  Amino acid linker sequence

<400>  115

Gly Gly Ser Gly Gly Ser Gly Gly Ser
1               5

<210>  116
<211>  1569
<212>  DNA
<213>  Artificial sequence

<220>
<223>  Optimized for expression in plants of a nucleic acid,
      encoding a phenylalanine-ammonium-lyase Streptomyces maritimus (SEQ
      ID No: 117)

<400>  116
atgacattcg tgatagagct ggacatgaat gtcactcttg atcagttaga ggatgctgca      60

agacaaagaa cacccgtgga attgtctgca cctgtacgtt caagggtccg tgcttcacgt     120

gatgttttag ttaaattcgt acaagatgag cgtgtgattt acggtgtaaa tacttcaatg     180

ggcggattcg tggatcacct agtacctgtg agtcaggccc gtcaattgca agagaattta     240

atcaatgcag ttgccacaaa tgttggagca tatttagatg atacaacggc caggacgatt     300

atgttgagta ggattgtatc cctggcaagg ggcaattctg ccataactcc cgccaacctt     360

gacaaattag tggcagtgtt gaacgcagga atcgtcccat gcatccctga aaaaggttca     420

ttaggtactt caggtgactt aggccctctg gcagctatcg ccttagtatg cgctggccag     480

tggaaggcac gttacaatgg acaaataatg ccaggccgtc aggctctatc agaggccggt     540

gtcgaaccta tggaactttc atataaggac ggcctagcac ttataaacgg tacgtctgga     600

atggtaggcc ttggaactat ggttttacaa gccgctagga ggttagtgga caggtatttg     660

caagtaagtg ccttatctgt agaaggcttg gcaggcatga ctaaaccttt tgaccctcgt     720

gtacacggag ttaagcccca tagaggtcaa agacaagtag cttcccgtct atgggaagga     780

ttggccgatt ctcaccttgc cgtgaacgag cttgatactg aacaaaccct tgcaggtgaa     840

atgggcacgg tggcaaaggc tggaagttta gccatagagg atgcctactc cattcgttgc     900

acccctcaga tcctgggtcc agtcgtagac gtattggata ggatcggtgc aacattacaa     960

gacgagctga attcttcaaa cgataacccc attgtccttc ctgaggaagc agaagtattc    1020

cataatggac actttcatgg acagtatgtc gccatggcaa tggatcacct aaatatggct    1080

cttgctactg ttacgaatct tgccaataga agagtcgaca gatttctaga taaatcaaat    1140

tccaatggac taccagcctt tctttgccgt gaagacccag gccttaggct tggtttgatg    1200

ggcggccagt tcatgactgc atcaatcacc gctgaaaccc gtacactgac aatcccaatg    1260

tctgtacaat cacttacttc cacggcagat tttcaagaca tagtatcctt tggcttcgta    1320

gccgcaagga gagctagaga ggtgttgaca aacgctgctt atgtggtagc ctttgaacta    1380

ctgtgtgcct gtcaggctgt ggacatcaga ggagcagaca agctatcttc tttcacccgt    1440

cctctatatg aaagaaccag gaaaattgtt ccctttttcg acagagatga aacaattact    1500

gactacgttg agaagttagc tgctgattta atagcaggcg agcctgtaga tgccgctgtg    1560

gctgcacat                                                            1569

<210>  117
<211>  523

<212>  PRT
<213>  Streptomyces maritimus

<400>  117

Met Thr Phe Val Ile Glu Leu Asp Met Asn Val Thr Leu Asp Gln Leu
1               5                   10                  15

Glu Asp Ala Ala Arg Gln Arg Thr Pro Val Glu Leu Ser Ala Pro Val
            20                  25                  30

Arg Ser Arg Val Arg Ala Ser Arg Asp Val Leu Val Lys Phe Val Gln
        35                  40                  45

Asp Glu Arg Val Ile Tyr Gly Val Asn Thr Ser Met Gly Gly Phe Val
    50                  55                  60

Asp His Leu Val Pro Val Ser Gln Ala Arg Gln Leu Gln Glu Asn Leu
65                  70                  75                  80

Ile Asn Ala Val Ala Thr Asn Val Gly Ala Tyr Leu Asp Asp Thr Thr
                85                  90                  95

Ala Arg Thr Ile Met Leu Ser Arg Ile Val Ser Leu Ala Arg Gly Asn
            100                 105                 110

Ser Ala Ile Thr Pro Ala Asn Leu Asp Lys Leu Val Ala Val Leu Asn
        115                 120                 125

Ala Gly Ile Val Pro Cys Ile Pro Glu Lys Gly Ser Leu Gly Thr Ser
    130                 135                 140

Gly Asp Leu Gly Pro Leu Ala Ala Ile Ala Leu Val Cys Ala Gly Gln
145                 150                 155                 160

Trp Lys Ala Arg Tyr Asn Gly Gln Ile Met Pro Gly Arg Gln Ala Leu
                165                 170                 175

Ser Glu Ala Gly Val Glu Pro Met Glu Leu Ser Tyr Lys Asp Gly Leu
            180                 185                 190

Ala Leu Ile Asn Gly Thr Ser Gly Met Val Gly Leu Gly Thr Met Val
        195                 200                 205

Leu Gln Ala Ala Arg Arg Leu Val Asp Arg Tyr Leu Gln Val Ser Ala
    210                 215                 220

Leu Ser Val Glu Gly Leu Ala Gly Met Thr Lys Pro Phe Asp Pro Arg
225                 230                 235                 240

Val His Gly Val Lys Pro His Arg Gly Gln Arg Gln Val Ala Ser Arg
                245                 250                 255

Leu Trp Glu Gly Leu Ala Asp Ser His Leu Ala Val Asn Glu Leu Asp
            260                 265                 270

Thr Glu Gln Thr Leu Ala Gly Glu Met Gly Thr Val Ala Lys Ala Gly
        275                 280                 285

Ser Leu Ala Ile Glu Asp Ala Tyr Ser Ile Arg Cys Thr Pro Gln Ile
    290                 295                 300

Leu Gly Pro Val Val Asp Val Leu Asp Arg Ile Gly Ala Thr Leu Gln
305                 310                 315                 320

Asp Glu Leu Asn Ser Ser Asn Asp Asn Pro Ile Val Leu Pro Glu Glu
                325                 330                 335

Ala Glu Val Phe His Asn Gly His Phe His Gly Gln Tyr Val Ala Met
            340                 345                 350

Ala Met Asp His Leu Asn Met Ala Leu Ala Thr Val Thr Asn Leu Ala
        355                 360                 365

Asn Arg Arg Val Asp Arg Phe Leu Asp Lys Ser Asn Ser Asn Gly Leu
    370                 375                 380

Pro Ala Phe Leu Cys Arg Glu Asp Pro Gly Leu Arg Leu Gly Leu Met
385                 390                 395                 400

Gly Gly Gln Phe Met Thr Ala Ser Ile Thr Ala Glu Thr Arg Thr Leu
                405                 410                 415

Thr Ile Pro Met Ser Val Gln Ser Leu Thr Ser Thr Ala Asp Phe Gln
            420                 425                 430

Asp Ile Val Ser Phe Gly Phe Val Ala Ala Arg Arg Ala Arg Glu Val
        435                 440                 445

Leu Thr Asn Ala Ala Tyr Val Val Ala Phe Glu Leu Leu Cys Ala Cys
    450                 455                 460

Gln Ala Val Asp Ile Arg Gly Ala Asp Lys Leu Ser Ser Phe Thr Arg
465                 470                 475                 480

Pro Leu Tyr Glu Arg Thr Arg Lys Ile Val Pro Phe Phe Asp Arg Asp

485                 490                 495

Glu Thr Ile Thr Asp Tyr Val Glu Lys Leu Ala Ala Asp Leu Ile Ala
            500                 505                 510

Gly Glu Pro Val Asp Ala Ala Val Ala Ala His
        515                 520

<210>  118
<211>  1173
<212>  DNA
<213>  Artificial sequence

<220>
<223>  Nucleotide sequence PKS from Aquilaria sinensis,
       optimized for expression in Nicotiana benthamiana

<400>  118
atgggatctc aagatgttgc tggaggagct cttaagggag ttaatccagg aaaggctact      60

attcttgctc ttggaaaggc ttttccatat caacttgtta tgcaagaatt tcttgttgat     120

ggatatttta agaatacttc ttgtaaggat caagaactta agcaaaagct tgctagactt     180

tgtaagacta ctactgttaa gactagatat gttgttatgt ctgaagaaat tcttaataag     240

tatccagaac ttgctgttga aggaattcca actcttaagc aaagacttga tattggaaat     300

gaagctctta ctgaaatggc tattgaagct tctcaagctt gtattaagaa gtggggaaga     360

ccagcttctg aaattactca tcttgtttat gtttcttctt ctgaagctag acttccagga     420

ggagatcttt atcttgctca aggacttgga ctttctccaa gaactaagag agttgttctt     480

tattttatgg gatgttctgg aggagttgct ggacttagag ttgctaagga tattgctgaa     540

aataatccag gatctagagt tcttcttgct acttctgaaa ctactattgt tggatttaag     600

ccaccatctg ctcatagacc atatgatctt gttggagttg ctctttttgg agatggagct     660

ggagctatgg ttattggatc tgatccactt ccaggaactg aatctccact ttttgaactt      720

catactgcta ttcaaaattt tcttccaaat actgaaaaga ctattgatgg aagacttact     780

gaagaaggaa tttcttttaa gcttgctaga gaacttccac aaattgttga agatcatatt     840

gaaggatttt gtggacaact tactggagtt attggacttt ctcataagca atataataag     900

atgttttggg ctgttcatcc aggaggacca gctattctta atagagttga aaagagactt     960

gatcttcatc caaataagct tgatgcttct agaagagctc ttgaagatta tggaaatgct    1020

tcttctaatt ctattgttta tgttcttgat tatatgattg aagaaactct taagatgaag    1080

actgaatctc ttgaaccatc tgaatgggga cttattcttg cttttggacc aggagttact    1140

tttgaaggaa ttcttgctag aaatcttgct gtt                                 1173

<210>  119

<211>  391
<212>  PRT
<213>  Aquilaria sinensis

<400>  119

Met Gly Ser Gln Asp Val Ala Gly Gly Ala Leu Lys Gly Val Asn Pro
1               5                   10                  15

Gly Lys Ala Thr Ile Leu Ala Leu Gly Lys Ala Phe Pro Tyr Gln Leu
            20                  25                  30

Val Met Gln Glu Phe Leu Val Asp Gly Tyr Phe Lys Asn Thr Ser Cys
        35                  40                  45

Lys Asp Gln Glu Leu Lys Gln Lys Leu Ala Arg Leu Cys Lys Thr Thr
    50                  55                  60

Thr Val Lys Thr Arg Tyr Val Val Met Ser Glu Glu Ile Leu Asn Lys
65                  70                  75                  80

Tyr Pro Glu Leu Ala Val Glu Gly Ile Pro Thr Leu Lys Gln Arg Leu
                85                  90                  95

Asp Ile Gly Asn Glu Ala Leu Thr Glu Met Ala Ile Glu Ala Ser Gln
            100                 105                 110

Ala Cys Ile Lys Lys Trp Gly Arg Pro Ala Ser Glu Ile Thr His Leu
        115                 120                 125

Val Tyr Val Ser Ser Ser Glu Ala Arg Leu Pro Gly Gly Asp Leu Tyr
    130                 135                 140

Leu Ala Gln Gly Leu Gly Leu Ser Pro Arg Thr Lys Arg Val Val Leu
145                 150                 155                 160

Tyr Phe Met Gly Cys Ser Gly Gly Val Ala Gly Leu Arg Val Ala Lys
                165                 170                 175

Asp Ile Ala Glu Asn Asn Pro Gly Ser Arg Val Leu Leu Ala Thr Ser
            180                 185                 190

Glu Thr Thr Ile Val Gly Phe Lys Pro Pro Ser Ala His Arg Pro Tyr
        195                 200                 205

Asp Leu Val Gly Val Ala Leu Phe Gly Asp Gly Ala Gly Ala Met Val
    210                 215                 220

Ile Gly Ser Asp Pro Leu Pro Gly Thr Glu Ser Pro Leu Phe Glu Leu

225                 230                 235                 240

His Thr Ala Ile Gln Asn Phe Leu Pro Asn Thr Glu Lys Thr Ile Asp
                245                 250                 255

Gly Arg Leu Thr Glu Glu Gly Ile Ser Phe Lys Leu Ala Arg Glu Leu
            260                 265                 270

Pro Gln Ile Val Glu Asp His Ile Glu Gly Phe Cys Gly Gln Leu Thr
        275                 280                 285

Gly Val Ile Gly Leu Ser His Lys Gln Tyr Asn Lys Met Phe Trp Ala
    290                 295                 300

Val His Pro Gly Gly Pro Ala Ile Leu Asn Arg Val Glu Lys Arg Leu
305                 310                 315                 320

Asp Leu His Pro Asn Lys Leu Asp Ala Ser Arg Arg Ala Leu Glu Asp
                325                 330                 335

Tyr Gly Asn Ala Ser Ser Asn Ser Ile Val Tyr Val Leu Asp Tyr Met
            340                 345                 350

Ile Glu Glu Thr Leu Lys Met Lys Thr Glu Ser Leu Glu Pro Ser Glu
        355                 360                 365

Trp Gly Leu Ile Leu Ala Phe Gly Pro Gly Val Thr Phe Glu Gly Ile
    370                 375                 380

Leu Ala Arg Asn Leu Ala Val
385                 390

<210>  120
<211>  1164
<212>  DNA
<213>  Artificial sequence

<220>
<223>  Nucleotide sequence PKS2 from Aquilaria sinensis,
       optimized for expression in Nicotiana benthamiana

<400>  120
atgtctcaag ctattgctga taatgcttat agacatcatc ttaagagagc tccaactcca      60

ggaaaggcta ctgttcttgc tcttggaaag gcttttccaa agcaagttat tccacaagaa     120

aatcttgttg aaggatatat tagagatact aagtgtgaag atgtttctat taaggaaaag     180

cttgaaagac tttgtaagac tactactgtt aagactagat atactgttat gtctaaggaa     240

attcttgata attatccaga acttgttact gaaggatctc caactattag acaaagactt     300

gaaattgcta atccagctgt tgttgaaatg gctaaggaag cttctcttgc ttgtattaag 360

caatggggaa gaccagctgg agatattact catattgttt atgtttcttc ttctgaaatt 420

agacttccag gaggagatct ttatcttgct aatgaacttg gacttaagaa tgatattaat 480

agaattatgc tttattttct tggatgttat ggaggagtta ctggacttag agttgctaag 540

gatattgctg aaaataatcc aggatctaga attcttctta ctacttctga aactactatt 600

cttggattta gaccaccaaa taagtctaga ccatatgatc ttgttggagc tgctcttttt 660

ggagatggag ctgctgctgt tattattgga gctaatccag aaattggaag agaatctcca 720

tttatggaac ttaattttgc tcttcaacaa tttcttccag gaactcatgg agttattgat 780

ggaagacttt ctgaagaagg aattaatttt aagcttggaa gagatcttcc acaaaagatt 840

gaagataata ttgaagattt ttgtagaaag cttatgatta aggctgatgg agatcttaag 900

gaatttaatg aactttttgg ggctgttcat ccaggaggac cagctattct taatagactt 960

gaatctattc ttgatcttaa gaatggaaag cttgaatgtt ctagaagagc tcttatggat 1020

tatggaaatg tttcttctaa tactattttt tatgttatgg aatatatgag agaagaactt 1080

aagagagaag gatctgaaga atggggactt gctcttgctt ttggaccagg aattactttt 1140

gaaggaattc ttcttagatc tctt 1164

<210> 121
<211> 388
<212> PRT
<213> Aquilaria sinensis

<400> 121

Met Ser Gln Ala Ile Ala Asp Asn Ala Tyr Arg His His Leu Lys Arg
1               5                   10                  15

Ala Pro Thr Pro Gly Lys Ala Thr Val Leu Ala Leu Gly Lys Ala Phe
            20                  25                  30

Pro Lys Gln Val Ile Pro Gln Glu Asn Leu Val Glu Gly Tyr Ile Arg
        35                  40                  45

Asp Thr Lys Cys Glu Asp Val Ser Ile Lys Glu Lys Leu Glu Arg Leu
    50                  55                  60

Cys Lys Thr Thr Thr Val Lys Thr Arg Tyr Thr Val Met Ser Lys Glu
65                  70                  75                  80

Ile Leu Asp Asn Tyr Pro Glu Leu Val Thr Glu Gly Ser Pro Thr Ile
                85                  90                  95

Arg Gln Arg Leu Glu Ile Ala Asn Pro Ala Val Val Glu Met Ala Lys

            100                 105                 110

Glu Ala Ser Leu Ala Cys Ile Lys Gln Trp Gly Arg Pro Ala Gly Asp
        115                 120                 125

Ile Thr His Ile Val Tyr Val Ser Ser Ser Glu Ile Arg Leu Pro Gly
    130                 135                 140

Gly Asp Leu Tyr Leu Ala Asn Glu Leu Gly Leu Lys Asn Asp Ile Asn
145                 150                 155                 160

Arg Ile Met Leu Tyr Phe Leu Gly Cys Tyr Gly Gly Val Thr Gly Leu
                165                 170                 175

Arg Val Ala Lys Asp Ile Ala Glu Asn Asn Pro Gly Ser Arg Ile Leu
            180                 185                 190

Leu Thr Thr Ser Glu Thr Thr Ile Leu Gly Phe Arg Pro Pro Asn Lys
        195                 200                 205

Ser Arg Pro Tyr Asp Leu Val Gly Ala Ala Leu Phe Gly Asp Gly Ala
    210                 215                 220

Ala Ala Val Ile Ile Gly Ala Asn Pro Glu Ile Gly Arg Glu Ser Pro
225                 230                 235                 240

Phe Met Glu Leu Asn Phe Ala Leu Gln Gln Phe Leu Pro Gly Thr His
                245                 250                 255

Gly Val Ile Asp Gly Arg Leu Ser Glu Glu Gly Ile Asn Phe Lys Leu
            260                 265                 270

Gly Arg Asp Leu Pro Gln Lys Ile Glu Asp Asn Ile Glu Asp Phe Cys
        275                 280                 285

Arg Lys Leu Met Ile Lys Ala Asp Gly Asp Leu Lys Glu Phe Asn Glu
    290                 295                 300

Leu Phe Trp Ala Val His Pro Gly Gly Pro Ala Ile Leu Asn Arg Leu
305                 310                 315                 320

Glu Ser Ile Leu Asp Leu Lys Asn Gly Lys Leu Glu Cys Ser Arg Arg
                325                 330                 335

Ala Leu Met Asp Tyr Gly Asn Val Ser Ser Asn Thr Ile Phe Tyr Val
            340                 345                 350

Met Glu Tyr Met Arg Glu Glu Leu Lys Arg Glu Gly Ser Glu Glu Trp
        355                 360                 365

Gly Leu Ala Leu Ala Phe Gly Pro Gly Ile Thr Phe Glu Gly Ile Leu
    370                 375                 380

Leu Arg Ser Leu
385

<210>  122
<211>  1185
<212>  DNA
<213>  Artificial sequence

<220>
<223>  Nucleotide sequence PKS from Arabidopsis thaliana,
       optimized for expression in Nicotiana benthamiana

<400>  122
atgtctaatt ctagaatgaa tggagttgaa aagctttctt ctaagtctac tagaagagtt      60

gctaatgctg gaaaggctac tcttcttgct cttggaaagg cttttccatc tcaagttgtt     120

ccacaagaaa atcttgttga aggatttctt agagatacta agtgtgatga tgcttttatt     180

aaggaaaagc ttgaacatct ttgtaagact actactgtta agactagata tactgttctt     240

actagagaaa ttcttgctaa gtatccagaa cttactactg aaggatctcc aactattaag     300

caaagacttg aaattgctaa tgaagctgtt gttgaaatgg ctcttgaagc ttctcttgga     360

tgtattaagg aatggggaag accagttgaa gatattactc atattgttta tgtttcttct     420

tctgaaatta gacttccagg aggagatctt tatctttctg ctaagcttgg acttagaaat     480

gatgttaata gagttatgct ttattttctt ggatgttatg gaggagttac tggacttaga     540

gttgctaagg atattgctga aaataatcca ggatctagag ttcttcttac tacttctgaa     600

actactattc ttggatttag accaccaaat aaggctagac catatgatct tgttggagct     660

gctctttttg gagatggagc tgctgctgtt attattggag ctgatccaag agaatgtgaa     720

gctccattta tggaacttca ttatgctgtt caacaatttc ttccaggaac tcaaaatgtt     780

attgaaggaa gacttactga agaaggaatt aattttaagc ttggaagaga tcttccacaa     840

aagattgaag aaaatattga agaattttgt aagaagctta tgggaaaggc tggagatgaa     900

tctatggaat ttaatgatat gttttgggct gttcatccag gaggaccagc tattcttaat     960

agacttgaaa ctaagcttaa gcttgaaaag gaaaagcttg aatcttctag aagagctctt    1020

gttgattatg gaaatgtttc ttctaatact attctttatg ttatggaata tatgagagat    1080

gaacttaaga agaagggaga tgctgctcaa gaatggggac ttggacttgc ttttggacca    1140

ggaattactt ttgaaggact tcttattaga tctcttactt cttct                    1185

<210>  123
<211>  395
<212>  PRT
<213>  Arabidopsis thaliana

<400>  123

Met Ser Asn Ser Arg Met Asn Gly Val Glu Lys Leu Ser Ser Lys Ser
1               5                   10                  15

Thr Arg Arg Val Ala Asn Ala Gly Lys Ala Thr Leu Leu Ala Leu Gly
            20                  25                  30

Lys Ala Phe Pro Ser Gln Val Val Pro Gln Glu Asn Leu Val Glu Gly
        35                  40                  45

Phe Leu Arg Asp Thr Lys Cys Asp Asp Ala Phe Ile Lys Glu Lys Leu
    50                  55                  60

Glu His Leu Cys Lys Thr Thr Thr Val Lys Thr Arg Tyr Thr Val Leu
65                  70                  75                  80

Thr Arg Glu Ile Leu Ala Lys Tyr Pro Glu Leu Thr Thr Glu Gly Ser
                85                  90                  95

Pro Thr Ile Lys Gln Arg Leu Glu Ile Ala Asn Glu Ala Val Val Glu
            100                 105                 110

Met Ala Leu Glu Ala Ser Leu Gly Cys Ile Lys Glu Trp Gly Arg Pro
        115                 120                 125

Val Glu Asp Ile Thr His Ile Val Tyr Val Ser Ser Ser Glu Ile Arg
    130                 135                 140

Leu Pro Gly Gly Asp Leu Tyr Leu Ser Ala Lys Leu Gly Leu Arg Asn
145                 150                 155                 160

Asp Val Asn Arg Val Met Leu Tyr Phe Leu Gly Cys Tyr Gly Gly Val
                165                 170                 175

Thr Gly Leu Arg Val Ala Lys Asp Ile Ala Glu Asn Asn Pro Gly Ser
            180                 185                 190

Arg Val Leu Leu Thr Thr Ser Glu Thr Thr Ile Leu Gly Phe Arg Pro
        195                 200                 205

Pro Asn Lys Ala Arg Pro Tyr Asp Leu Val Gly Ala Ala Leu Phe Gly
    210                 215                 220

Asp Gly Ala Ala Ala Val Ile Ile Gly Ala Asp Pro Arg Glu Cys Glu
225                 230                 235                 240

Ala Pro Phe Met Glu Leu His Tyr Ala Val Gln Gln Phe Leu Pro Gly
                245                 250                 255

Thr Gln Asn Val Ile Glu Gly Arg Leu Thr Glu Glu Gly Ile Asn Phe
            260                 265                 270

Lys Leu Gly Arg Asp Leu Pro Gln Lys Ile Glu Glu Asn Ile Glu Glu
        275                 280                 285

Phe Cys Lys Lys Leu Met Gly Lys Ala Gly Asp Glu Ser Met Glu Phe
    290                 295                 300

Asn Asp Met Phe Trp Ala Val His Pro Gly Gly Pro Ala Ile Leu Asn
305                 310                 315                 320

Arg Leu Glu Thr Lys Leu Lys Leu Glu Lys Glu Lys Leu Glu Ser Ser
                325                 330                 335

Arg Arg Ala Leu Val Asp Tyr Gly Asn Val Ser Ser Asn Thr Ile Leu
            340                 345                 350

Tyr Val Met Glu Tyr Met Arg Asp Glu Leu Lys Lys Lys Gly Asp Ala
        355                 360                 365

Ala Gln Glu Trp Gly Leu Gly Leu Ala Phe Gly Pro Gly Ile Thr Phe
    370                 375                 380

Glu Gly Leu Leu Ile Arg Ser Leu Thr Ser Ser
385                 390                 395

<210>  124
<211>  1197
<212>  DNA
<213>  Artificial sequence

<220>
<223>  Nucleotide sequence PKS from Hydrangea macrophylla,
       optimized for expression in Nicotiana benthamiana

<400>  124
atggctacta agtctgttgc tgttgaagaa atgtgtaagg ctcaaaaggc tggaggacca      60

gctactattc ttgctattgg aactgctgtt ccatctaatt gttattatca atctgaatat     120

ccagattttt attttagagt tactaagtct gatcatctta ctgatcttaa gtctaagttt     180

aagagaatgt gtgaaagatc ttctattaag aagagatata tgcatcttac tgaagaaatt     240

cttgaagaaa atccaaatat gtgtactttt gctgctccat ctattgatgg aagacaagat     300

attgttgtta aggaaattcc aaagcttgct aaggaagctg cttctaaggc tattaaggaa 360

tggggacaac caaagtctaa tattactcat cttgtttttt gtactacttc tggagttgat 420

atgccaggat gtgattatca acttactaga cttcttggac ttagaccatc tattaagaga 480

cttatgatgt atcaacaagg atgtcatgct ggaggaactg gacttagact tgctaaggat 540

cttgctgaaa ataataaggg agctagagtt cttgttgttt gttctgaaat gactgttatt 600

aattttagag gaccatctga agctcatatg gattctcttg ttggacaatc tctttttgga 660

gatggagctt ctgctgttat tgttggatct gatccagatc tttctactga acatccactt 720

tatcaaatta tgtctgcttc tcaaattatt gttgctgatt ctgaaggagc tattgatgga 780

catcttagac aagaaggact tacttttcat cttagaaagg atgttccatc tcttgtttct 840

gataatattg aaaatactct tgttgaagct tttactccaa ttcttatgga ttctattgat 900

tctattattg attggaattc tattttttgg attgctcatc caggaggacc agctattctt 960

aatcaagttc aagctaaggt tggacttaag gaagaaaagc ttagagtttc tagacatatt 1020

ctttctgaat atggaaatat gtcttctgct tgtgtttttt ttattatgga tgaaatgaga 1080

aagagatcta tggaagaagg aaagggaact actggagaag gacttgaatg gggagttctt 1140

tttggatttg gaccaggatt tactgttgaa actattgttc ttcattctgt tccaatt 1197

<210> 125
<211> 399
<212> PRT
<213> Hydrangea macrophylla

<400> 125

Met Ala Thr Lys Ser Val Ala Val Glu Glu Met Cys Lys Ala Gln Lys
1               5                   10                  15

Ala Gly Gly Pro Ala Thr Ile Leu Ala Ile Gly Thr Ala Val Pro Ser
            20                  25                  30

Asn Cys Tyr Tyr Gln Ser Glu Tyr Pro Asp Phe Tyr Phe Arg Val Thr
        35                  40                  45

Lys Ser Asp His Leu Thr Asp Leu Lys Ser Lys Phe Lys Arg Met Cys
    50                  55                  60

Glu Arg Ser Ser Ile Lys Lys Arg Tyr Met His Leu Thr Glu Glu Ile
65                  70                  75                  80

Leu Glu Glu Asn Pro Asn Met Cys Thr Phe Ala Ala Pro Ser Ile Asp
                85                  90                  95

Gly Arg Gln Asp Ile Val Val Lys Glu Ile Pro Lys Leu Ala Lys Glu
        100                 105                 110

Ala Ala Ser Lys Ala Ile Lys Glu Trp Gly Gln Pro Lys Ser Asn Ile
        115                 120                 125

Thr His Leu Val Phe Cys Thr Thr Ser Gly Val Asp Met Pro Gly Cys
    130                 135                 140

Asp Tyr Gln Leu Thr Arg Leu Leu Gly Leu Arg Pro Ser Ile Lys Arg
145                 150                 155                 160

Leu Met Met Tyr Gln Gln Gly Cys His Ala Gly Gly Thr Gly Leu Arg
                165                 170                 175

Leu Ala Lys Asp Leu Ala Glu Asn Asn Lys Gly Ala Arg Val Leu Val
            180                 185                 190

Val Cys Ser Glu Met Thr Val Ile Asn Phe Arg Gly Pro Ser Glu Ala
        195                 200                 205

His Met Asp Ser Leu Val Gly Gln Ser Leu Phe Gly Asp Gly Ala Ser
    210                 215                 220

Ala Val Ile Val Gly Ser Asp Pro Asp Leu Ser Thr Glu His Pro Leu
225                 230                 235                 240

Tyr Gln Ile Met Ser Ala Ser Gln Ile Ile Val Ala Asp Ser Glu Gly
                245                 250                 255

Ala Ile Asp Gly His Leu Arg Gln Glu Gly Leu Thr Phe His Leu Arg
            260                 265                 270

Lys Asp Val Pro Ser Leu Val Ser Asp Asn Ile Glu Asn Thr Leu Val
        275                 280                 285

Glu Ala Phe Thr Pro Ile Leu Met Asp Ser Ile Asp Ser Ile Ile Asp
    290                 295                 300

Trp Asn Ser Ile Phe Trp Ile Ala His Pro Gly Gly Pro Ala Ile Leu
305                 310                 315                 320

Asn Gln Val Gln Ala Lys Val Gly Leu Lys Glu Glu Lys Leu Arg Val
                325                 330                 335

Ser Arg His Ile Leu Ser Glu Tyr Gly Asn Met Ser Ser Ala Cys Val
            340                 345                 350

Phe Phe Ile Met Asp Glu Met Arg Lys Arg Ser Met Glu Glu Gly Lys
        355                 360                 365

Gly Thr Thr Gly Glu Gly Leu Glu Trp Gly Val Leu Phe Gly Phe Gly
    370                 375                 380

Pro Gly Phe Thr Val Glu Thr Ile Val Leu His Ser Val Pro Ile
385                 390                 395

<210> 126
<211> 1254
<212> DNA
<213> Artificial sequence

<220>
<223> Nucleotide sequence PKS from Physcomitrella patens,
optimized for expression in Nicotiana benthamiana

<400> 126
atggcttcta gaagagttga agctgctttt gatggacaag ctgttgaact tggagctact 60

attccagctg ctaatggaaa tggaactcat caatctatta aggttccagg acatagacaa 120

gttactccag gaaagactac tattatggct attggaagag ctgttccagc taatactact 180

tttaatgatg gacttgctga tcattatatt caagaattta atcttcaaga tccagttctt 240

caagctaagc ttagaagact ttgtgaaact actactgtta agactagata tcttgttgtt 300

aataaggaaa ttcttgatga acatccagaa tttcttgttg atggagctgc tactgtttct 360

caaagacttg ctattactgg agaagctgtt actcaacttg gacatgaagc tgctactgct 420

gctattaagg aatggggaag accagcttct gaaattactc atcttgttta tgtttcttct 480

tctgaaatta gacttccagg aggagatctt tatcttgctc aacttcttgg acttagatct 540

gatgttaata gagttatgct ttatatgctt ggatgttatg gaggagcttc tggaattaga 600

gttgctaagg atcttgctga aaataatcca ggatctagag ttcttcttat tacttctgaa 660

tgtactctta ttggatataa gtctctttct ccagatagac catatgatct tgttggagct 720

gctctttttg gagatggagc tgctgctatg attatgggaa aggatccaat tccagttctt 780

gaaagagctt tttttgaact tgattgggct ggacaatctt ttattccagg aactaataag 840

actattgatg gaagactttc tgaagaagga atttctttta agcttggaag agaacttcca 900

aagcttattg aatctaatat tcaaggattt tgtgatccaa ttcttaagag agctggagga 960

cttaagtata atgatatttt ttgggctgtt catccaggag gaccagctat tcttaatgct 1020

gttcaaaagc aacttgatct tgctccagaa aagcttcaaa ctgctagaca agttcttaga 1080

gattatggaa atatttcttc ttctacttgt atttatgttc ttgattatat gagacatcaa 1140

tctcttaagc ttaaggaagc taatgataat gttaatactg aaccagaatg gggacttctt 1200

cttgcttttg gaccaggagt tactattgaa ggagctcttc ttagaaatct ttgt          1254

<210>  127
<211>  397
<212>  PRT
<213>  Physcomitrella patens

<400>  127

Met Ser Lys Thr Val Glu Asp Arg Ala Ala Gln Arg Ala Lys Gly Pro
1               5                   10                  15

Ala Thr Val Leu Ala Ile Gly Thr Ala Thr Pro Ala Asn Val Val Tyr
            20                  25                  30

Gln Thr Asp Tyr Pro Asp Tyr Tyr Phe Arg Val Thr Lys Ser Glu His
        35                  40                  45

Met Thr Lys Leu Lys Asn Lys Phe Gln Arg Met Cys Asp Arg Ser Thr
    50                  55                  60

Ile Lys Lys Arg Tyr Met Val Leu Thr Glu Glu Leu Leu Glu Lys Asn
65                  70                  75                  80

Leu Ser Leu Cys Thr Tyr Met Glu Pro Ser Leu Asp Ala Arg Gln Asp
                85                  90                  95

Ile Leu Val Pro Glu Val Pro Lys Leu Gly Lys Glu Ala Ala Asp Glu
            100                 105                 110

Ala Ile Ala Glu Trp Gly Arg Pro Lys Ser Glu Ile Thr His Leu Ile
        115                 120                 125

Phe Cys Thr Thr Cys Gly Val Asp Met Pro Gly Ala Asp Tyr Gln Leu
    130                 135                 140

Thr Lys Leu Leu Gly Leu Arg Ser Ser Val Arg Arg Thr Met Leu Tyr
145                 150                 155                 160

Gln Gln Gly Cys Phe Gly Gly Gly Thr Val Leu Arg Leu Ala Lys Asp
                165                 170                 175

Leu Ala Glu Asn Asn Ala Gly Ala Arg Val Leu Val Val Cys Ser Glu
            180                 185                 190

Ile Thr Thr Ala Val Asn Phe Arg Gly Pro Ser Asp Thr His Leu Asp
        195                 200                 205

Leu Leu Val Gly Leu Ala Leu Phe Gly Asp Gly Ala Ala Ala Val Ile

210                 215                 220

Val Gly Ala Asp Pro Asp Pro Thr Leu Glu Arg Pro Leu Phe Gln Ile
225                 230                 235                 240

Val Ser Gly Ala Gln Thr Ile Leu Pro Asp Ser Glu Gly Ala Ile Asn
                245                 250                 255

Gly His Leu Arg Glu Val Gly Leu Thr Ile Arg Leu Leu Lys Asp Val
            260                 265                 270

Pro Gly Leu Val Ser Met Asn Ile Glu Lys Cys Leu Met Glu Ala Phe
        275                 280                 285

Ala Pro Met Gly Ile His Asp Trp Asn Ser Ile Phe Trp Ile Ala His
    290                 295                 300

Pro Gly Gly Pro Thr Ile Leu Asp Gln Val Glu Ala Lys Leu Gly Leu
305                 310                 315                 320

Lys Glu Glu Lys Leu Lys Ser Thr Arg Ala Val Leu Arg Glu Tyr Gly
                325                 330                 335

Asn Met Ser Ser Ala Cys Val Leu Phe Ile Leu Asp Glu Val Arg Lys
            340                 345                 350

Arg Ser Met Glu Glu Gly Lys Thr Thr Thr Gly Glu Gly Phe Asp Trp
        355                 360                 365

Gly Val Leu Phe Gly Phe Gly Pro Gly Phe Thr Val Glu Thr Val Val
    370                 375                 380

Leu His Ser Met Pro Ile Pro Lys Ala Asp Glu Gly Arg
385                 390                 395

<210>  128
<211>  1179
<212>  DNA
<213>  Artificial sequence

<220>
<223>  Nucleotide sequence PKS from Polygonum cuspidatum,
       optimized for expression in Nicotiana benthamiana

<400>  128
   atggctccag ctgttgctga tattagaaag gctcaaagag ctgaaggacc agctactgtt      60
   cttgctattg gaactgctac tccaccaaat tgtgtttatc aaaaggatta tccagattat     120
   tattttagag ttactaattc tgatcatatg actgatctta aggaaaagtt tagaagaatg     180
   tgtgaaaagt ctaatattga aaagagatat atgtatctta ctgaagaaat tcttaaggaa 240
   aatccaaata tgtgttctta tatgcaaact tcttctcttg atactagaca agatatggtt 300
   gtttctgaag ttccaagact tggaaaggaa gctgctcaaa aggctattaa ggaatgggga 360
   caaccaaagt ctaagattac tcatgttatt atgtgtacta cttctggagt tgatatgcca 420
   ggagctgatt atcaacttac taagcttctt ggacttcatc catctgttaa gagatttatg 480
   atgtatcaac aaggatgttt tgctggagga actgttctta gacttgctaa ggatcttgct 540
   gaaaataata gaggagctag agttcttgtt gtttgttctg aaattactgc tatttgtttt 600
   agaggaccaa ctgatactca tccagattct atggttggac aagctctttt tggagatgga 660
   tctggagctg ttattattgg agctgatcca gatctttcta ttgaaaagcc aatttttgaa 720
   cttgtttgga ctgctcaaac tattcttcca gattctgaag gagctattga tggacatctt 780
   agagaagttg gacttacttt tcatcttctt aaggatgttc caggacttat ttctaagaat 840
   attgaaaaga atcttactga agctttttct ccacttaatg tttctgattg gaattctctt 900
   ttttggattg ctcatccagg aggaccagct attcttgatc aagttgaaac taagcttgga 960
   cttaaggaag aaaagcttaa ggctactaga caagttctta atgattatgg aaatatgtct 1020
   tctgcttgtg ttctttttat tatggatgaa atgagaaaga agtctgttga aaatggacat 1080
   gctactactg gagaaggact tgaatgggga gttctttttg gatttggacc aggacttact 1140
   gttgaaactg ttgttcttca ttctgttcca gttgctaat 1179

<210> 129
<211> 393
<212> PRT
<213> Polygonum cuspidatum

<400> 129

Met Ala Pro Ala Val Ala Asp Ile Arg Lys Ala Gln Arg Ala Glu Gly
1               5                   10                  15

Pro Ala Thr Val Leu Ala Ile Gly Thr Ala Thr Pro Pro Asn Cys Val
            20                  25                  30

Tyr Gln Lys Asp Tyr Pro Asp Tyr Tyr Phe Arg Val Thr Asn Ser Asp
        35                  40                  45

His Met Thr Asp Leu Lys Glu Lys Phe Arg Arg Met Cys Glu Lys Ser
    50                  55                  60

Asn Ile Glu Lys Arg Tyr Met Tyr Leu Thr Glu Glu Ile Leu Lys Glu
65                  70                  75                  80

Asn Pro Asn Met Cys Ser Tyr Met Gln Thr Ser Ser Leu Asp Thr Arg

                85                  90                  95

Gln Asp Met Val Val Ser Glu Val Pro Arg Leu Gly Lys Glu Ala Ala
            100                 105                 110

Gln Lys Ala Ile Lys Glu Trp Gly Gln Pro Lys Ser Lys Ile Thr His
        115                 120                 125

Val Ile Met Cys Thr Thr Ser Gly Val Asp Met Pro Gly Ala Asp Tyr
    130                 135                 140

Gln Leu Thr Lys Leu Leu Gly Leu His Pro Ser Val Lys Arg Phe Met
145                 150                 155                 160

Met Tyr Gln Gln Gly Cys Phe Ala Gly Gly Thr Val Leu Arg Leu Ala
                165                 170                 175

Lys Asp Leu Ala Glu Asn Asn Arg Gly Ala Arg Val Leu Val Val Cys
            180                 185                 190

Ser Glu Ile Thr Ala Ile Cys Phe Arg Gly Pro Thr Asp Thr His Pro
        195                 200                 205

Asp Ser Met Val Gly Gln Ala Leu Phe Gly Asp Gly Ser Gly Ala Val
    210                 215                 220

Ile Ile Gly Ala Asp Pro Asp Leu Ser Ile Glu Lys Pro Ile Phe Glu
225                 230                 235                 240

Leu Val Trp Thr Ala Gln Thr Ile Leu Pro Asp Ser Glu Gly Ala Ile
                245                 250                 255

Asp Gly His Leu Arg Glu Val Gly Leu Thr Phe His Leu Leu Lys Asp
            260                 265                 270

Val Pro Gly Leu Ile Ser Lys Asn Ile Glu Lys Asn Leu Thr Glu Ala
        275                 280                 285

Phe Ser Pro Leu Asn Val Ser Asp Trp Asn Ser Leu Phe Trp Ile Ala
    290                 295                 300

His Pro Gly Gly Pro Ala Ile Leu Asp Gln Val Glu Thr Lys Leu Gly
305                 310                 315                 320

Leu Lys Glu Glu Lys Leu Lys Ala Thr Arg Gln Val Leu Asn Asp Tyr
                325                 330                 335

Gly Asn Met Ser Ser Ala Cys Val Leu Phe Ile Met Asp Glu Met Arg
            340                 345                 350

Lys Lys Ser Val Glu Asn Gly His Ala Thr Thr Gly Glu Gly Leu Glu
        355                 360                 365

Trp Gly Val Leu Phe Gly Phe Gly Pro Gly Leu Thr Val Glu Thr Val
    370                 375                 380

Val Leu His Ser Val Pro Val Ala Asn
385                 390

<210>  130
<211>  1152
<212>  DNA
<213>  Artificial squence

<220>
<223>  Nucleotide sequence of the mutant L132S PKS from Rheum
       palmatum, optimized for expression in Nicotiana benthamiana

<400>  130
atggctactg aagaaatgaa gaagcttgct actgttatgg ctattggaac tgctaatcca      60

ccaaattgtt attatcaagc tgattttcca gatttttatt ttagagttac taattctgat     120

catcttatta atcttaagca aaagtttaag agactttgtg aaaattctag aattgaaaag     180

agatatcttc atgttactga agaaattctt aaggaaaatc caaatattgc tgcttatgaa     240

gctacttctc ttaatgttag acataagatg caagttaagg gagttgctga acttggaaag     300

gaagctgctc ttaaggctat taaggaatgg ggacaaccaa agtctaagat tactcatctt     360

attgtttgtt gttctgctgg agttgatatg ccaggagctg attatcaact tactaagctt     420

cttgatcttg atccatctgt taagagattt atgttttatc atcttggatg ttatgctgga     480

ggaactgttc ttagacttgc taaggatatt gctgaaaata ataagggagc tagagttctt     540

attgtttgtt ctgaaatgac tactacttgt tttagaggac catctgaaac tcatcttgat     600

tctatgattg gacaagctat tcttggagat ggagctgctg ctgttattgt tggagctgat     660

ccagatctta ctgttgaaag accaattttt gaacttgttt ctactgctca aactattgtt     720

ccagaatctc atggagctat tgaaggacat cttcttgaat ctggactttc ttttcatctt     780

tataagactg ttccaactct tatttctaat aatattaaga cttgtctttc tgatgctttt     840

actccactta atatttctga ttggaattct ctttttggga ttgctcatcc aggaggacca     900

gctattcttg atcaagttac tgctaaggtt ggacttgaaa aggaaaagct taaggttact     960

agacaagttc ttaaggatta tggaaatatg tcttctgcta ctgttttttt tattatggat    1020

gaaatgagaa agaagtctct tgaaaatgga caagctacta ctggagaagg acttgaatgg    1080

ggagttcttt ttggatttgg accaggaatt actgttgaaa ctgttgttct tagatctgtt    1140

ccagttattt ct                                                          1152

<210>  131
<211>  384
<212>  PRT
<213>  Artificial sequence

<220>
<223>  Amino acid sequence of the mutant L132S PKS from Rheum
       palmatum, optimized for expression in Nicotiana benthamiana

<400>  131

Met Ala Thr Glu Glu Met Lys Lys Leu Ala Thr Val Met Ala Ile Gly
1               5                   10                  15

Thr Ala Asn Pro Pro Asn Cys Tyr Tyr Gln Ala Asp Phe Pro Asp Phe
            20                  25                  30

Tyr Phe Arg Val Thr Asn Ser Asp His Leu Ile Asn Leu Lys Gln Lys
        35                  40                  45

Phe Lys Arg Leu Cys Glu Asn Ser Arg Ile Glu Lys Arg Tyr Leu His
    50                  55                  60

Val Thr Glu Glu Ile Leu Lys Glu Asn Pro Asn Ile Ala Ala Tyr Glu
65                  70                  75                  80

Ala Thr Ser Leu Asn Val Arg His Lys Met Gln Val Lys Gly Val Ala
                85                  90                  95

Glu Leu Gly Lys Glu Ala Ala Leu Lys Ala Ile Lys Glu Trp Gly Gln
            100                 105                 110

Pro Lys Ser Lys Ile Thr His Leu Ile Val Cys Cys Ser Ala Gly Val
        115                 120                 125

Asp Met Pro Gly Ala Asp Tyr Gln Leu Thr Lys Leu Leu Asp Leu Asp
    130                 135                 140

Pro Ser Val Lys Arg Phe Met Phe Tyr His Leu Gly Cys Tyr Ala Gly
145                 150                 155                 160

Gly Thr Val Leu Arg Leu Ala Lys Asp Ile Ala Glu Asn Asn Lys Gly
                165                 170                 175

Ala Arg Val Leu Ile Val Cys Ser Glu Met Thr Thr Thr Cys Phe Arg
            180                 185                 190

Gly Pro Ser Glu Thr His Leu Asp Ser Met Ile Gly Gln Ala Ile Leu
        195                 200                 205

Gly Asp Gly Ala Ala Ala Val Ile Val Gly Ala Asp Pro Asp Leu Thr
    210                 215                 220

Val Glu Arg Pro Ile Phe Glu Leu Val Ser Thr Ala Gln Thr Ile Val
225                 230                 235                 240

Pro Glu Ser His Gly Ala Ile Glu Gly His Leu Leu Glu Ser Gly Leu
                245                 250                 255

Ser Phe His Leu Tyr Lys Thr Val Pro Thr Leu Ile Ser Asn Asn Ile
            260                 265                 270

Lys Thr Cys Leu Ser Asp Ala Phe Thr Pro Leu Asn Ile Ser Asp Trp
        275                 280                 285

Asn Ser Leu Phe Trp Ile Ala His Pro Gly Gly Pro Ala Ile Leu Asp
    290                 295                 300

Gln Val Thr Ala Lys Val Gly Leu Glu Lys Glu Lys Leu Lys Val Thr
305                 310                 315                 320

Arg Gln Val Leu Lys Asp Tyr Gly Asn Met Ser Ser Ala Thr Val Phe
                325                 330                 335

Phe Ile Met Asp Glu Met Arg Lys Lys Ser Leu Glu Asn Gly Gln Ala
            340                 345                 350

Thr Thr Gly Glu Gly Leu Glu Trp Gly Val Leu Phe Gly Phe Gly Pro
        355                 360                 365

Gly Ile Thr Val Glu Thr Val Val Leu Arg Ser Val Pro Val Ile Ser
    370                 375                 380

<210>  132
<211>  1173
<212>  PRT
<213>  Artificial sequence

<220>
<223>  Nucleotide sequence of PKS from Rheum tataricum,
       optimized for expression in Nicotiana benthamiana

<400>  132

Ala Thr Gly Gly Cys Thr Cys Cys Ala Gly Ala Ala Gly Ala Ala Thr
1               5                   10                  15

Cys Thr Ala Gly Ala Cys Ala Thr Gly Cys Thr Gly Ala Ala Ala Cys
            20                  25                  30

Thr Gly Cys Thr Gly Thr Thr Ala Ala Thr Ala Gly Ala Gly Cys Thr
        35                  40                  45

Gly Cys Thr Ala Cys Thr Gly Thr Thr Cys Thr Thr Gly Cys Thr Ala
    50                  55                  60

Thr Thr Gly Gly Ala Ala Cys Thr Gly Cys Thr Ala Ala Thr Cys Cys
65                  70                  75                  80

Ala Cys Cys Ala Ala Ala Thr Thr Gly Thr Thr Ala Thr Thr Ala Thr
                85                  90                  95

Cys Ala Ala Gly Cys Thr Gly Ala Thr Thr Thr Thr Cys Cys Ala Gly
            100                 105                 110

Ala Thr Thr Thr Thr Thr Ala Thr Thr Thr Thr Ala Gly Ala Gly Cys
        115                 120                 125

Thr Ala Cys Thr Ala Ala Thr Thr Cys Thr Gly Ala Thr Cys Ala Thr
    130                 135                 140

Cys Thr Thr Ala Cys Thr Cys Ala Thr Cys Thr Thr Ala Ala Gly Cys
145                 150                 155                 160

Ala Ala Ala Ala Gly Thr Thr Thr Ala Ala Gly Ala Gly Ala Ala Thr
                165                 170                 175

Thr Thr Gly Thr Gly Ala Ala Ala Ala Gly Thr Cys Thr Ala Thr Gly
            180                 185                 190

Ala Thr Thr Gly Ala Ala Ala Ala Gly Ala Gly Ala Thr Ala Thr Cys
        195                 200                 205

Thr Thr Cys Ala Thr Cys Thr Thr Ala Cys Thr Gly Ala Ala Gly Ala
    210                 215                 220

Ala Ala Thr Thr Cys Thr Thr Ala Ala Gly Gly Ala Ala Ala Ala Thr
225                 230                 235                 240

Cys Cys Ala Ala Ala Thr Ala Thr Thr Gly Cys Thr Thr Cys Thr Thr
                245                 250                 255

Thr Thr Gly Ala Ala Gly Cys Thr Cys Cys Ala Thr Cys Thr Cys Thr
            260                 265                 270

Thr Gly Ala Thr Gly Thr Thr Ala Gly Ala Cys Ala Thr Ala Ala Thr
        275                 280                 285

Ala Thr Thr Cys Ala Ala Gly Thr Thr Ala Ala Gly Gly Ala Ala Gly
    290                 295                 300

Thr Thr Gly Thr Thr Cys Thr Thr Cys Thr Thr Gly Gly Ala Ala Ala
305                 310                 315                 320

Gly Gly Ala Ala Gly Cys Thr Gly Cys Thr Cys Thr Thr Ala Ala Gly
                325                 330                 335

Gly Cys Thr Ala Thr Thr Ala Ala Thr Gly Ala Ala Thr Gly Gly Gly
            340                 345                 350

Gly Ala Cys Ala Ala Cys Cys Ala Ala Ala Gly Thr Cys Thr Ala Ala
        355                 360                 365

Gly Ala Thr Thr Ala Cys Thr Ala Gly Ala Cys Thr Thr Ala Thr Thr
    370                 375                 380

Gly Thr Thr Thr Gly Thr Thr Gly Thr Ala Thr Thr Gly Cys Thr Gly
385                 390                 395                 400

Gly Ala Gly Thr Thr Gly Ala Thr Ala Thr Gly Cys Cys Ala Gly Gly
                405                 410                 415

Ala Gly Cys Thr Gly Ala Thr Thr Ala Thr Cys Ala Ala Cys Thr Thr
            420                 425                 430

Ala Cys Thr Ala Ala Gly Cys Thr Thr Cys Thr Thr Gly Gly Ala Cys
        435                 440                 445

Thr Thr Cys Ala Ala Cys Thr Thr Thr Cys Thr Gly Thr Thr Ala Ala
    450                 455                 460

Gly Ala Gly Ala Thr Thr Thr Ala Thr Gly Thr Thr Thr Thr Ala Thr
465                 470                 475                 480

Cys Ala Thr Cys Thr Thr Gly Gly Ala Thr Gly Thr Thr Ala Thr Gly
                485                 490                 495

Cys Thr Gly Gly Ala Gly Gly Ala Ala Cys Thr Gly Thr Thr Cys Thr
            500                 505                 510

Thr Ala Gly Ala Cys Thr Thr Gly Cys Thr Ala Ala Gly Gly Ala Thr
        515                 520                 525

Ala Thr Thr Gly Cys Thr Gly Ala Ala Ala Ala Thr Ala Ala Thr Ala
    530                 535                 540

Ala Gly Gly Ala Ala Gly Cys Thr Ala Gly Ala Gly Thr Thr Cys Thr
545                 550                 555                 560

Thr Ala Thr Thr Gly Thr Thr Ala Gly Ala Thr Cys Thr Gly Ala Ala
                565                 570                 575

Ala Thr Gly Ala Cys Thr Cys Cys Ala Ala Thr Thr Thr Gly Thr Thr
            580                 585                 590

Thr Thr Ala Gly Ala Gly Gly Ala Cys Cys Ala Thr Cys Thr Gly Ala
        595                 600                 605

Ala Ala Cys Thr Cys Ala Thr Ala Thr Thr Gly Ala Thr Thr Cys Thr
    610                 615                 620

Ala Thr Gly Gly Thr Thr Gly Gly Ala Cys Ala Ala Gly Cys Thr Ala
625                 630                 635                 640

Thr Thr Thr Thr Thr Gly Gly Ala Gly Ala Thr Gly Gly Ala Gly Cys
                645                 650                 655

Thr Gly Cys Thr Gly Cys Thr Gly Thr Thr Ala Thr Thr Gly Thr Thr
            660                 665                 670

Gly Gly Ala Gly Cys Thr Ala Ala Thr Cys Cys Ala Gly Ala Thr Cys
        675                 680                 685

Thr Thr Thr Cys Thr Ala Thr Thr Gly Ala Ala Ala Gly Ala Cys Cys
    690                 695                 700

Ala Ala Thr Thr Thr Thr Thr Gly Ala Ala Cys Thr Thr Ala Thr Thr
705                 710                 715                 720

Thr Cys Thr Ala Cys Thr Thr Cys Thr Cys Ala Ala Ala Cys Thr Ala
                725                 730                 735

Thr Thr Ala Thr Thr Cys Cys Ala Gly Ala Ala Thr Cys Thr Gly Ala
            740                 745                 750

Thr Gly Gly Ala Gly Cys Thr Ala Thr Thr Gly Ala Ala Gly Gly Ala
        755                 760                 765

Cys Ala Thr Cys Thr Thr Cys Thr Thr Gly Ala Ala Gly Thr Thr Gly

770                 775                 780

Gly Ala Cys Thr Thr Thr Cys Thr Thr Thr Thr Cys Ala Ala Cys Thr
785                 790                 795                 800

Thr Thr Ala Thr Cys Ala Ala Ala Cys Thr Gly Thr Thr Cys Cys Ala
                805                 810                 815

Thr Cys Thr Cys Thr Thr Ala Thr Thr Thr Cys Thr Ala Ala Thr Thr
            820                 825                 830

Gly Thr Ala Thr Thr Gly Ala Ala Ala Cys Thr Thr Gly Thr Cys Thr
        835                 840                 845

Thr Thr Cys Thr Ala Ala Gly Gly Cys Thr Thr Thr Thr Ala Cys Thr
    850                 855                 860

Cys Cys Ala Cys Thr Thr Ala Ala Thr Ala Thr Thr Thr Cys Thr Gly
865                 870                 875                 880

Ala Thr Thr Gly Gly Ala Ala Thr Thr Cys Thr Cys Thr Thr Thr Thr
                885                 890                 895

Thr Thr Gly Gly Ala Thr Thr Gly Cys Thr Cys Ala Thr Cys Cys Ala
            900                 905                 910

Gly Gly Ala Gly Gly Ala Ala Gly Ala Gly Cys Thr Ala Thr Thr Cys
        915                 920                 925

Thr Thr Gly Ala Thr Gly Ala Thr Ala Thr Thr Gly Ala Ala Gly Cys
    930                 935                 940

Thr Ala Cys Thr Gly Thr Thr Gly Gly Ala Cys Thr Thr Ala Ala Gly
945                 950                 955                 960

Ala Ala Gly Gly Ala Ala Ala Ala Gly Cys Thr Thr Ala Ala Gly Gly
                965                 970                 975

Cys Thr Ala Cys Thr Ala Gly Ala Cys Ala Ala Gly Thr Thr Cys Thr
            980                 985                 990

Thr Ala Ala Thr Gly Ala Thr Thr  Ala Thr Gly Gly Ala  Ala Ala Thr
        995                 1000                 1005

Ala Thr  Gly Thr Cys Thr Thr  Cys Thr Gly Cys Thr  Thr Gly Thr
    1010                 1015                 1020

Gly Thr Thr Thr Thr Thr Thr Thr Thr Ala Thr Thr Ala Thr Gly
    1025                1030                1035

Gly Ala Thr Gly Ala Ala Ala Thr Gly Ala Gly Ala Ala Ala Gly
    1040                1045                1050

Ala Ala Gly Thr Cys Thr Cys Thr Thr Gly Cys Thr Ala Ala Thr
    1055                1060                1065

Gly Gly Ala Cys Ala Ala Gly Thr Thr Ala Cys Thr Ala Cys Thr
    1070                1075                1080

Gly Gly Ala Gly Ala Ala Gly Gly Ala Cys Thr Thr Ala Ala Gly
    1085                1090                1095

Thr Gly Gly Gly Gly Ala Gly Thr Thr Cys Thr Thr Thr Thr Thr
    1100                1105                1110

Gly Gly Ala Thr Thr Thr Gly Gly Ala Cys Cys Ala Gly Gly Ala
    1115                1120                1125

Gly Thr Thr Ala Cys Thr Gly Thr Thr Gly Ala Ala Ala Cys Thr
    1130                1135                1140

Gly Thr Thr Gly Thr Thr Cys Thr Thr Thr Cys Thr Thr Cys Thr
    1145                1150                1155

Gly Thr Thr Cys Cys Ala Cys Thr Thr Ala Thr Thr Ala Cys Thr
    1160                1165                1170

<210> 133
<211> 391
<212> PRT
<213> Rheum tataricum

<400> 133

Met Ala Pro Glu Glu Ser Arg His Ala Glu Thr Ala Val Asn Arg Ala
1               5                   10                  15

Ala Thr Val Leu Ala Ile Gly Thr Ala Asn Pro Pro Asn Cys Tyr Tyr
            20                  25                  30

Gln Ala Asp Phe Pro Asp Phe Tyr Phe Arg Ala Thr Asn Ser Asp His
        35                  40                  45

Leu Thr His Leu Lys Gln Lys Phe Lys Arg Ile Cys Glu Lys Ser Met
    50                  55                  60

Ile Glu Lys Arg Tyr Leu His Leu Thr Glu Glu Ile Leu Lys Glu Asn
65                  70                  75                  80

Pro Asn Ile Ala Ser Phe Glu Ala Pro Ser Leu Asp Val Arg His Asn
                85                  90                  95

Ile Gln Val Lys Glu Val Val Leu Leu Gly Lys Glu Ala Ala Leu Lys
            100                 105                 110

Ala Ile Asn Glu Trp Gly Gln Pro Lys Ser Lys Ile Thr Arg Leu Ile
        115                 120                 125

Val Cys Cys Ile Ala Gly Val Asp Met Pro Gly Ala Asp Tyr Gln Leu
    130                 135                 140

Thr Lys Leu Leu Gly Leu Gln Leu Ser Val Lys Arg Phe Met Phe Tyr
145                 150                 155                 160

His Leu Gly Cys Tyr Ala Gly Gly Thr Val Leu Arg Leu Ala Lys Asp
                165                 170                 175

Ile Ala Glu Asn Asn Lys Glu Ala Arg Val Leu Ile Val Arg Ser Glu
            180                 185                 190

Met Thr Pro Ile Cys Phe Arg Gly Pro Ser Glu Thr His Ile Asp Ser
        195                 200                 205

Met Val Gly Gln Ala Ile Phe Gly Asp Gly Ala Ala Ala Val Ile Val
    210                 215                 220

Gly Ala Asn Pro Asp Leu Ser Ile Glu Arg Pro Ile Phe Glu Leu Ile
225                 230                 235                 240

Ser Thr Ser Gln Thr Ile Ile Pro Glu Ser Asp Gly Ala Ile Glu Gly
                245                 250                 255

His Leu Leu Glu Val Gly Leu Ser Phe Gln Leu Tyr Gln Thr Val Pro
            260                 265                 270

Ser Leu Ile Ser Asn Cys Ile Glu Thr Cys Leu Ser Lys Ala Phe Thr
        275                 280                 285

Pro Leu Asn Ile Ser Asp Trp Asn Ser Leu Phe Trp Ile Ala His Pro
    290                 295                 300

Gly Gly Arg Ala Ile Leu Asp Asp Ile Glu Ala Thr Val Gly Leu Lys
305                 310                 315                 320

Lys Glu Lys Leu Lys Ala Thr Arg Gln Val Leu Asn Asp Tyr Gly Asn
                325                 330                 335

Met Ser Ser Ala Cys Val Phe Phe Ile Met Asp Glu Met Arg Lys Lys
            340                 345                 350

Ser Leu Ala Asn Gly Gln Val Thr Thr Gly Glu Gly Leu Lys Trp Gly
        355                 360                 365

Val Leu Phe Gly Phe Gly Pro Gly Val Thr Val Glu Thr Val Val Leu
    370                 375                 380

Ser Ser Val Pro Leu Ile Thr
385                 390

<210>  134
<211>  1182
<212>  DNA
<213>  Artificial sequence

<220>
<223>  Nucleotide sequence PKS from Wachendorfia thyrsiflora,
       optimized for expression in Nicotiana benthamiana

<400>  134
atggcttcta ctgaaggaat tcaagcttat agaaataata tggctgaagg accagctact      60

attatggcta ttggaactgc taatccacca aatgttgttg atgcttctac ttttccagat     120

tattattgga gagttactaa ttctgaacat ctttctccag aatatagagt taagcttaag     180

agaatttgtg aaagatcttc tattagaaag agacatcttg ttcttactga acaacttctt     240

aaggaaaatc caactcttac tacttatgtt gatgcttctt atgatgaaag acaatctatt     300

gttcttgatg ctgttccaaa gcttgcttgt gaagctgctg ctaaggctat taaggaatgg     360

ggaagaccaa agactgatat tactcatatg gttgtttgta ctggagctgg agttgatgtt     420

ccaggagttg attataagat gatgaatctt cttggacttc caccaactgt taatagagtt     480

atgctttata atgttggatg tcatgcttct ggaactgttc ttagaattgc taaggatctt     540

gctgaaaata ataagggagc tagagttctt gttgtttctt ctgaagtttc tgttatgttt     600

tttagaggac cagctgaagg agatgttgaa attcttcttg gacaagctct ttttggagat     660

ggatctgctg ctattattgt tggagctgat ccaattgaag gagttgaaaa gccaattttt     720

caaatttttt ctgcttctca aatgactctt ccagaaggag aacatcttgt tgctggacat     780

cttagagaac ttggacttac ttttcatctt aagccacaac ttccaaatac tgtttcttct     840

aatattcata agccacttaa gaaggctttt gaaccactta atattactga ttggaattct     900

atttttggga ttgttcatcc aggaggaaga gctattcttg atcaagttca agaaaagatt     960

ggacttgaag aaaataagct tgatgtttct agatatgttc ttgctgaaaa tggaaatatg 1020

atgtctgctt ctgttttttt tattatggat gaaatgagaa agagatctgc tgctcaagga 1080

tgttctacta ctggagaagg acatgaatgg ggagttcttt ttggatttgg accaggactt 1140

tctattgaaa ctgttgttct tcattctgtt ccactttcta tt 1182

<210> 135
<211> 394
<212> PRT
<213> Wachendorfia thyrsiflora

<400> 135

Met Ala Ser Thr Glu Gly Ile Gln Ala Tyr Arg Asn Asn Met Ala Glu
1               5                   10                  15

Gly Pro Ala Thr Ile Met Ala Ile Gly Thr Ala Asn Pro Pro Asn Val
            20                  25                  30

Val Asp Ala Ser Thr Phe Pro Asp Tyr Tyr Trp Arg Val Thr Asn Ser
        35                  40                  45

Glu His Leu Ser Pro Glu Tyr Arg Val Lys Leu Lys Arg Ile Cys Glu
    50                  55                  60

Arg Ser Ser Ile Arg Lys Arg His Leu Val Leu Thr Glu Gln Leu Leu
65                  70                  75                  80

Lys Glu Asn Pro Thr Leu Thr Thr Tyr Val Asp Ala Ser Tyr Asp Glu
                85                  90                  95

Arg Gln Ser Ile Val Leu Asp Ala Val Pro Lys Leu Ala Cys Glu Ala
            100                 105                 110

Ala Ala Lys Ala Ile Lys Glu Trp Gly Arg Pro Lys Thr Asp Ile Thr
        115                 120                 125

His Met Val Val Cys Thr Gly Ala Gly Val Asp Val Pro Gly Val Asp
    130                 135                 140

Tyr Lys Met Met Asn Leu Leu Gly Leu Pro Pro Thr Val Asn Arg Val
145                 150                 155                 160

Met Leu Tyr Asn Val Gly Cys His Ala Ser Gly Thr Val Leu Arg Ile
                165                 170                 175

Ala Lys Asp Leu Ala Glu Asn Asn Lys Gly Ala Arg Val Leu Val Val
            180                 185                 190

Ser Ser Glu Val Ser Val Met Phe Phe Arg Gly Pro Ala Glu Gly Asp
        195                 200                 205

Val Glu Ile Leu Leu Gly Gln Ala Leu Phe Gly Asp Gly Ser Ala Ala
    210                 215                 220

Ile Ile Val Gly Ala Asp Pro Ile Glu Gly Val Glu Lys Pro Ile Phe
225                 230                 235                 240

Gln Ile Phe Ser Ala Ser Gln Met Thr Leu Pro Glu Gly Glu His Leu
                245                 250                 255

Val Ala Gly His Leu Arg Glu Leu Gly Leu Thr Phe His Leu Lys Pro
            260                 265                 270

Gln Leu Pro Asn Thr Val Ser Ser Asn Ile His Lys Pro Leu Lys Lys
        275                 280                 285

Ala Phe Glu Pro Leu Asn Ile Thr Asp Trp Asn Ser Ile Phe Trp Ile
    290                 295                 300

Val His Pro Gly Gly Arg Ala Ile Leu Asp Gln Val Gln Glu Lys Ile
305                 310                 315                 320

Gly Leu Glu Glu Asn Lys Leu Asp Val Ser Arg Tyr Val Leu Ala Glu
                325                 330                 335

Asn Gly Asn Met Met Ser Ala Ser Val Phe Phe Ile Met Asp Glu Met
            340                 345                 350

Arg Lys Arg Ser Ala Ala Gln Gly Cys Ser Thr Thr Gly Glu Gly His
        355                 360                 365

Glu Trp Gly Val Leu Phe Gly Phe Gly Pro Gly Leu Ser Ile Glu Thr
    370                 375                 380

Val Val Leu His Ser Val Pro Leu Ser Ile
385                 390

<210>  136
<211>  1191
<212>  DNA
<213>  Artificial sequence

<220>
<223>  Nucleotide sequence PKS1 from Piper methysticum,
       optimized for expression in Nicotiana benthamiana

<400>  136

atgtctaaga ctgttgaaga tagagctgct caaagagcta agggaccagc tactgttctt 60

gctattggaa ctgctactcc agctaatgtt gtttatcaaa ctgattatcc agattattat 120

tttagagtta ctaagtctga acatatgact aagcttaaga ataagtttca aagaatgtgt 180

gatagatcta ctattaagaa gagatatatg gttcttactg aagaacttct tgaaaagaat 240

ctttctcttt gtacttatat ggaaccatct cttgatgcta gacaagatat tcttgttcca 300

gaagttccaa agcttggaaa ggaagctgct gatgaagcta ttgctgaatg gggaagacca 360

aagtctgaaa ttactcatct tattttttgt actacttgtg gagttgatat gccaggagct 420

gattatcaac ttactaagct tcttggactt agatcttctg ttagaagaac tatgctttat 480

caacaaggat gttttggagg aggaactgtt cttagacttg ctaaggatct tgctgaaaat 540

aatgctggag ctagagttct tgttgtttgt tctgaaatta ctactgctgt taattttaga 600

ggaccatctg atactcatct tgatcttctt gttggacttg ctctttttgg agatggagct 660

gctgctgtta ttgttggagc tgatccagat ccaactcttg aaagaccact ttttcaaatt 720

gtttctggag ctcaaactat tcttccagat tctgaaggag ctattaatgg acatcttaga 780

gaagttggac ttactattag acttcttaag gatgttccag gacttgtttc tatgaatatt 840

gaaaagtgtc ttatggaagc ttttgctcca atgggaattc atgattggaa ttctattttt 900

tggattgctc atccaggagg accaactatt cttgatcaag ttgaagctaa gcttggactt 960

aaggaagaaa agcttaagtc tactagagct gttcttagag aatatggaaa tatgtcttct 1020

gcttgtgttc tttttattct tgatgaagtt agaaagagat ctatggaaga aggaaagact 1080

actactggag aaggatttga ttggggagtt ctttttggat ttggaccagg atttactgtt 1140

gaaactgttg ttcttcattc tatgccaatt ccaaaggctg atgaaggaag a 1191

<210> 137
<211> 397
<212> PRT
<213> Piper methysticum

<400> 137

Met Ser Lys Thr Val Glu Asp Arg Ala Ala Gln Arg Ala Lys Gly Pro
1               5                   10                  15

Ala Thr Val Leu Ala Ile Gly Thr Ala Thr Pro Ala Asn Val Val Tyr
            20                  25                  30

Gln Thr Asp Tyr Pro Asp Tyr Tyr Phe Arg Val Thr Lys Ser Glu His
        35                  40                  45

Met Thr Lys Leu Lys Asn Lys Phe Gln Arg Met Cys Asp Arg Ser Thr
    50                  55                  60

Ile Lys Lys Arg Tyr Met Val Leu Thr Glu Glu Leu Leu Glu Lys Asn
65                  70                  75                  80

Leu Ser Leu Cys Thr Tyr Met Glu Pro Ser Leu Asp Ala Arg Gln Asp
                85                  90                  95

Ile Leu Val Pro Glu Val Pro Lys Leu Gly Lys Glu Ala Ala Asp Glu
            100                 105                 110

Ala Ile Ala Glu Trp Gly Arg Pro Lys Ser Glu Ile Thr His Leu Ile
        115                 120                 125

Phe Cys Thr Thr Cys Gly Val Asp Met Pro Gly Ala Asp Tyr Gln Leu
    130                 135                 140

Thr Lys Leu Leu Gly Leu Arg Ser Ser Val Arg Arg Thr Met Leu Tyr
145                 150                 155                 160

Gln Gln Gly Cys Phe Gly Gly Gly Thr Val Leu Arg Leu Ala Lys Asp
                165                 170                 175

Leu Ala Glu Asn Asn Ala Gly Ala Arg Val Leu Val Val Cys Ser Glu
            180                 185                 190

Ile Thr Thr Ala Val Asn Phe Arg Gly Pro Ser Asp Thr His Leu Asp
        195                 200                 205

Leu Leu Val Gly Leu Ala Leu Phe Gly Asp Gly Ala Ala Ala Val Ile
    210                 215                 220

Val Gly Ala Asp Pro Asp Pro Thr Leu Glu Arg Pro Leu Phe Gln Ile
225                 230                 235                 240

Val Ser Gly Ala Gln Thr Ile Leu Pro Asp Ser Glu Gly Ala Ile Asn
                245                 250                 255

Gly His Leu Arg Glu Val Gly Leu Thr Ile Arg Leu Leu Lys Asp Val
            260                 265                 270

Pro Gly Leu Val Ser Met Asn Ile Glu Lys Cys Leu Met Glu Ala Phe
        275                 280                 285

Ala Pro Met Gly Ile His Asp Trp Asn Ser Ile Phe Trp Ile Ala His
    290                 295                 300

Pro Gly Gly Pro Thr Ile Leu Asp Gln Val Glu Ala Lys Leu Gly Leu
305                 310                 315                 320

Lys Glu Glu Lys Leu Lys Ser Thr Arg Ala Val Leu Arg Glu Tyr Gly
                325                 330                 335

Asn Met Ser Ser Ala Cys Val Leu Phe Ile Leu Asp Glu Val Arg Lys
            340                 345                 350

Arg Ser Met Glu Glu Gly Lys Thr Thr Thr Gly Glu Gly Phe Asp Trp
        355                 360                 365

Gly Val Leu Phe Gly Phe Gly Pro Gly Phe Thr Val Glu Thr Val Val
    370                 375                 380

Leu His Ser Met Pro Ile Pro Lys Ala Asp Glu Gly Arg
385                 390                 395

<210>  138
<211>  1182
<212>  DNA
<213>  Artificial sequence

<220>
<223>  Nucleotide sequence of PKS2 from Piper methysticum,
       optimized for expression in Nicotiana benthamiana

<400>  138
atgtctaaga tggttgaaga acattgggct gctcaaagag ctagaggacc agctactgtt      60

cttgctattg gaactgctaa tccaccaaat gttctttatc aagctgatta tccagatttt     120

tattttagag ttactaagtc tgaacatatg actcaactta aggaaaagtt taagagaatt     180

tgtgataagt ctgctattag aaagagacat cttcatctta ctgaagaact tcttgaaaag     240

aatccaaata tttgtgctca tatggctcca tctcttgatg ctagacaaga tattgctgtt     300

gttgaagttc caaagcttgc taaggaagct gctactaagg ctattaagga atggggaaga     360

ccaaagtctg atattactca tcttattttt tgtactactt gtggagttga tatgccagga     420

gctgattatc aacttactac tcttcttgga cttagaccaa ctgttagaag aactatgctt     480

tatcaacaag gatgttttgc tggaggaact gttcttagac atgctaagga ttttgctgaa     540

aataatagag gagctagagt tcttgctgtt tgttctgaat ttactgttat gaatttttct     600

ggaccatctg aagctcatct tgattctatg gttggaatgg ctctttttgg agatggagct     660

tctgctgtta ttgttggagc tgatccagat tttgctattg aaagaccact ttttcaactt     720

gtttctacta ctcaaactat tgttccagat tctgatggag ctattaagtg tcatcttaag     780

gaagttggac ttactcttca tcttgttaag aatgttccag atcttatttc taataatatg     840

gataagattc ttgaagaagc ttttgctcca cttggaatta gagattggaa ttctattttt     900

tggactgctc atccaggagg agctgctatt cttgatcaac ttgaagctaa gcttggactt     960

aataaggaaa agcttaagac tactagaact gttcttagag aatatggaaa tatgtcttct 1020

gcttgtgttt gttttgttct tgatgaaatg agaagatctt ctcttgaaga aggaaagact 1080

acttctggag aaggacttga atggggaatt cttcttggat ttggaccagg acttactgtt 1140

gaaactgttg ttcttagatc tgttccaatt tctactgcta at 1182

<210> 139
<211> 394
<212> PRT
<213> Piper methysticum

<400> 139

Met Ser Lys Met Val Glu Glu His Trp Ala Ala Gln Arg Ala Arg Gly
1               5                   10                  15

Pro Ala Thr Val Leu Ala Ile Gly Thr Ala Asn Pro Pro Asn Val Leu
            20                  25                  30

Tyr Gln Ala Asp Tyr Pro Asp Phe Tyr Phe Arg Val Thr Lys Ser Glu
        35                  40                  45

His Met Thr Gln Leu Lys Glu Lys Phe Lys Arg Ile Cys Asp Lys Ser
    50                  55                  60

Ala Ile Arg Lys Arg His Leu His Leu Thr Glu Glu Leu Leu Glu Lys
65                  70                  75                  80

Asn Pro Asn Ile Cys Ala His Met Ala Pro Ser Leu Asp Ala Arg Gln
                85                  90                  95

Asp Ile Ala Val Val Glu Val Pro Lys Leu Ala Lys Glu Ala Ala Thr
            100                 105                 110

Lys Ala Ile Lys Glu Trp Gly Arg Pro Lys Ser Asp Ile Thr His Leu
        115                 120                 125

Ile Phe Cys Thr Thr Cys Gly Val Asp Met Pro Gly Ala Asp Tyr Gln
    130                 135                 140

Leu Thr Thr Leu Leu Gly Leu Arg Pro Thr Val Arg Arg Thr Met Leu
145                 150                 155                 160

Tyr Gln Gln Gly Cys Phe Ala Gly Gly Thr Val Leu Arg His Ala Lys
                165                 170                 175

Asp Phe Ala Glu Asn Asn Arg Gly Ala Arg Val Leu Ala Val Cys Ser
            180                 185                 190

Glu Phe Thr Val Met Asn Phe Ser Gly Pro Ser Glu Ala His Leu Asp
        195                 200                 205

Ser Met Val Gly Met Ala Leu Phe Gly Asp Gly Ala Ser Ala Val Ile
    210                 215                 220

Val Gly Ala Asp Pro Asp Phe Ala Ile Glu Arg Pro Leu Phe Gln Leu
225                 230                 235                 240

Val Ser Thr Thr Gln Thr Ile Val Pro Asp Ser Asp Gly Ala Ile Lys
                245                 250                 255

Cys His Leu Lys Glu Val Gly Leu Thr Leu His Leu Val Lys Asn Val
            260                 265                 270

Pro Asp Leu Ile Ser Asn Asn Met Asp Lys Ile Leu Glu Glu Ala Phe
        275                 280                 285

Ala Pro Leu Gly Ile Arg Asp Trp Asn Ser Ile Phe Trp Thr Ala His
    290                 295                 300

Pro Gly Gly Ala Ala Ile Leu Asp Gln Leu Glu Ala Lys Leu Gly Leu
305                 310                 315                 320

Asn Lys Glu Lys Leu Lys Thr Thr Arg Thr Val Leu Arg Glu Tyr Gly
                325                 330                 335

Asn Met Ser Ser Ala Cys Val Cys Phe Val Leu Asp Glu Met Arg Arg
            340                 345                 350

Ser Ser Leu Glu Glu Gly Lys Thr Thr Ser Gly Glu Gly Leu Glu Trp
        355                 360                 365

Gly Ile Leu Leu Gly Phe Gly Pro Gly Leu Thr Val Glu Thr Val Val
    370                 375                 380

Leu Arg Ser Val Pro Ile Ser Thr Ala Asn
385                 390

<210>  140
<211>  1734
<212>  DNA
<213>  Arabidopsis thaliana

<400>  140
atggaagaag attataagat ggctccacaa gaacaagctg tttctcaagt tatggaaaag      60

caatctaata ataataattc tgatgttatt tttagatcta agcttccaga tatttatatt     120

ccaaatcatc tttctcttca tgattatatt tttcaaaata tttctgaatt tgctactaag 180

ccatgtctta ttaatggacc aactggacat gtttatactt attctgatgt tcatgttatt 240

tctagacaaa ttgctgctaa ttttcataag cttggagtta atcaaaatga tgttgttatg 300

cttcttcttc caaattgtcc agaatttgtt ctttcttttc ttgctgcttc ttttagagga 360

gctactgcta ctgctgctaa tccatttttt actccagctg aaattgctaa gcaagctaag 420

gcttctaata ctaagcttat tattactgaa gctagatatg ttgataagat taagccactt 480

caaaatgatg atggagttgt tattgtttgt attgatgata atgaatctgt tccaattcca 540

gaaggatgtc ttagatttac tgaacttact caatctacta ctgaagcttc tgaagttatt 600

gattctgttg aaatttctcc agatgatgtt gttgctcttc catattcttc tggaactact 660

ggacttccaa agggagttat gcttactcat aagggacttg ttacttctgt tgctcaacaa 720

gttgatggag aaaatccaaa tctttatttt cattctgatg atgttattct ttgtgttctt 780

ccaatgtttc atatttatgc tcttaattct attatgcttt gtggacttag agttggagct 840

gctattctta ttatgccaaa gtttgaaatt aatcttcttc ttgaacttat tcaaagatgt 900

aaggttactg ttgctccaat ggttccacca attgttcttg ctattgctaa gtcttctgaa 960

actgaaaagt atgatctttc ttctattaga gttgttaagt ctggagctgc tccacttgga 1020

aaggaacttg aagatgctgt taatgctaag tttccaaatg ctaagcttgg acaaggatat 1080

ggaatgactg aagctggacc agttcttgct atgtctcttg gatttgctaa ggaaccattt 1140

ccagttaagt ctggagcttg tggaactgtt gttagaaatg ctgaaatgaa gattgttgat 1200

ccagatactg gagattctct ttctagaaat caaccaggag aaatttgtat tagaggacat 1260

caaattatga agggatatct taataatcca gctgctactg ctgaaactat tgataaggat 1320

ggatggcttc atactggaga tattggactt attgatgatg atgatgaact ttttattgtt 1380

gatagactta aggaacttat taagtataag ggatttcaag ttgctccagc tgaacttgaa 1440

gctcttctta ttggacatcc agatattact gatgttgctg ttgttgctat gaaggaagaa 1500

gctgctggag aagttccagt tgcttttgtt gttaagtcta aggattctga actttctgaa 1560

gatgatgtta agcaatttgt ttctaagcaa gttgtttttt ataagagaat taataaggtt 1620

ttttttactg aatctattcc aaaggctcca tctggaaaga ttcttagaaa ggatcttaga 1680

gctaagcttg ctaatggact ttctggatat ccatatgatg ttccagatta tgct 1734

<210> 141
<211> 578
<212> PRT
<213> Arabidopsis thaliana

<400> 141

Met Glu Glu Asp Tyr Lys Met Ala Pro Gln Glu Gln Ala Val Ser Gln
1               5                   10                  15

Val Met Glu Lys Gln Ser Asn Asn Asn Asn Ser Asp Val Ile Phe Arg
            20                  25                  30

Ser Lys Leu Pro Asp Ile Tyr Ile Pro Asn His Leu Ser Leu His Asp
        35                  40                  45

Tyr Ile Phe Gln Asn Ile Ser Glu Phe Ala Thr Lys Pro Cys Leu Ile
    50                  55                  60

Asn Gly Pro Thr Gly His Val Tyr Thr Tyr Ser Asp Val His Val Ile
65                  70                  75                  80

Ser Arg Gln Ile Ala Ala Asn Phe His Lys Leu Gly Val Asn Gln Asn
                85                  90                  95

Asp Val Val Met Leu Leu Leu Pro Asn Cys Pro Glu Phe Val Leu Ser
            100                 105                 110

Phe Leu Ala Ala Ser Phe Arg Gly Ala Thr Ala Thr Ala Ala Asn Pro
        115                 120                 125

Phe Phe Thr Pro Ala Glu Ile Ala Lys Gln Ala Lys Ala Ser Asn Thr
    130                 135                 140

Lys Leu Ile Ile Thr Glu Ala Arg Tyr Val Asp Lys Ile Lys Pro Leu
145                 150                 155                 160

Gln Asn Asp Asp Gly Val Val Ile Val Cys Ile Asp Asp Asn Glu Ser
                165                 170                 175

Val Pro Ile Pro Glu Gly Cys Leu Arg Phe Thr Glu Leu Thr Gln Ser
            180                 185                 190

Thr Thr Glu Ala Ser Glu Val Ile Asp Ser Val Glu Ile Ser Pro Asp
        195                 200                 205

Asp Val Val Ala Leu Pro Tyr Ser Ser Gly Thr Thr Gly Leu Pro Lys
    210                 215                 220

Gly Val Met Leu Thr His Lys Gly Leu Val Thr Ser Val Ala Gln Gln
225                 230                 235                 240

Val Asp Gly Glu Asn Pro Asn Leu Tyr Phe His Ser Asp Asp Val Ile
                245                 250                 255

Leu Cys Val Leu Pro Met Phe His Ile Tyr Ala Leu Asn Ser Ile Met
        260                 265                 270

Leu Cys Gly Leu Arg Val Gly Ala Ala Ile Leu Ile Met Pro Lys Phe
    275                 280                 285

Glu Ile Asn Leu Leu Leu Glu Leu Ile Gln Arg Cys Lys Val Thr Val
    290                 295                 300

Ala Pro Met Val Pro Pro Ile Val Leu Ala Ile Ala Lys Ser Ser Glu
305                 310                 315                 320

Thr Glu Lys Tyr Asp Leu Ser Ser Ile Arg Val Val Lys Ser Gly Ala
                325                 330                 335

Ala Pro Leu Gly Lys Glu Leu Glu Asp Ala Val Asn Ala Lys Phe Pro
        340                 345                 350

Asn Ala Lys Leu Gly Gln Gly Tyr Gly Met Thr Glu Ala Gly Pro Val
    355                 360                 365

Leu Ala Met Ser Leu Gly Phe Ala Lys Glu Pro Phe Pro Val Lys Ser
    370                 375                 380

Gly Ala Cys Gly Thr Val Val Arg Asn Ala Glu Met Lys Ile Val Asp
385                 390                 395                 400

Pro Asp Thr Gly Asp Ser Leu Ser Arg Asn Gln Pro Gly Glu Ile Cys
                405                 410                 415

Ile Arg Gly His Gln Ile Met Lys Gly Tyr Leu Asn Asn Pro Ala Ala
        420                 425                 430

Thr Ala Glu Thr Ile Asp Lys Asp Gly Trp Leu His Thr Gly Asp Ile
    435                 440                 445

Gly Leu Ile Asp Asp Asp Asp Glu Leu Phe Ile Val Asp Arg Leu Lys
    450                 455                 460

Glu Leu Ile Lys Tyr Lys Gly Phe Gln Val Ala Pro Ala Glu Leu Glu
465                 470                 475                 480

Ala Leu Leu Ile Gly His Pro Asp Ile Thr Asp Val Ala Val Val Ala
                485                 490                 495

Met Lys Glu Glu Ala Ala Gly Glu Val Pro Val Ala Phe Val Val Lys
        500                 505                 510

Ser Lys Asp Ser Glu Leu Ser Glu Asp Asp Val Lys Gln Phe Val Ser
        515                 520                 525

Lys Gln Val Val Phe Tyr Lys Arg Ile Asn Lys Val Phe Phe Thr Glu
    530                 535                 540

Ser Ile Pro Lys Ala Pro Ser Gly Lys Ile Leu Arg Lys Asp Leu Arg
545                 550                 555                 560

Ala Lys Leu Ala Asn Gly Leu Ser Gly Tyr Pro Tyr Asp Val Pro Asp
                565                 570                 575

Tyr Ala

## Description

### Field of invention

The group of inventions relates to the field of biotechnology and genetic engineering. In particular, the invention relates to enzymes of bioluminescent system of fungi.

### Background of the invention

Enzymes that can catalyze oxidation of low molecular compounds of luciferins, which is accompanied by light emission or bioluminescence, are referred to as the "luciferases". Luciferin oxidation results in release of oxyluciferin from a complex with the luciferase enzyme.

Luciferases are widely used as the reporter genes in a number of biomedical applications and biotechnologies. For example, luciferases are used to determine viability of cells and activity of promoters or other components of living systems, in studies of carcinogenesis in animal models, in methods for detecting microorganisms or toxic agents in media, as indicators for determining concentrations of various substances, to visualize passage of signaling cascades, etc. [Scott et al., Annu Rev Anal Chem, 2011, 4: 297-319; Badr and Tannous, Trends Biotechnol. 2011, 29: 624-33; Andreu et al., FEMS Microbiol Rev. 2011, 35: 360-94]. Many applications of luciferases are described in reviews [Kaskova et al., Chem Soc Rev., 2016, 45: 6048-6077; Scott et al., Annu Rev Anal Chem, 2011, 4: 297-319; Widder and Falls, IEEE Journal of Selected Topics in Quantum Electronics, 2014, 20: 232-241]. All main applications of luciferases are based on detection of light emitted depending on the phenomenon or signal being studied. Such detection, as a rule, is performed using a luminometer or modified optical microscope.

Thousands of species capable of bioluminescence are known, for which about a dozen of luciferins with various structures and several dozens of corresponding luciferase enzymes have been described. It has been shown that the bioluminescence systems arose independently in various organisms in course of evolution more than forty times [Herring, Journal of Bioluminescence and Chemiluminescence, 1987, 1: 147-63; Haddock et al., Annual Review of Marine Science, 2010; 2: 443-93].

A group of insect luciferases catalyzing oxidation of D-luciferin has been described [de Wet et al., Proc. Natl. Acad. Sci. USA, 1985, 82: 7870-3; de Wet et al., Proc. Natl. Acad. Sci. USA, 1987, 7: 725-37]. A group of luciferases catalyzing oxidation of coelenterazine has been described [O. Shimomura, Bioluminescence: Chemical Principles and Methods, World Scientific Publishing Co. Pte. Ltd, Singapore, 2006, 470 p.]. Bioluminescent systems of ostracods of *Cypridina* genus are known, which are characterized by highly chemically active luciferin and highly stable luciferase [Shimomura et al., Science, 1969, 164: 1299-300]. Bioluminescent systems of dinoflagellates and euphausiids are also known. At present, genes encoding three luciferases from this group are cloned [O. Shimomura, Bioluminescence: Chemical Principles and Methods, World Scientific Publishing Co. Pte. Ltd, Singapore, 2006]. However, this system is still poorly studied, in particular, complete luciferase sequences have not been established yet.

In the last years, a group of luciferases and luciferin of fungi bioluminescent system have been described. Fungi bioluminescence was known over hundreds of years, but the fungal luciferin had been identified only in 2015: it turned out to be 3-hydroxyhispidin, a metabolite capable to penetrate through cell membranes [Purtov et al., Angewandte Chemie, 2015, 54: 8124-28]. The same publication confirms the presence of an enzyme able to hydroxylate hispidin to form luciferin in the fungi lysates, but the said enzyme was not identified. The patent application 2017102986 of 01/30/2017 describes luciferase genes from several fungi that contain luciferin in the form of 3-hydroxyhispidin with the following structure:

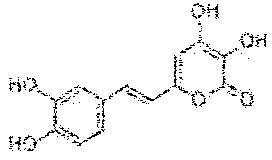

It was shown that fungal luciferases can also catalyze light-emitting oxidation of other chemical compounds with structures shown in Table 1 [Kaskova et al., Sci. Adv. 2017; 3: e1602847]. All these compounds, which are fungal luciferins, including 3-hydroxyhispidin, belong to the group of 6-(2-arylvinyl)-3,4-dihydroxy-2H-pyran-2-ones and have the general formula: 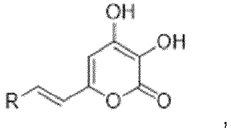 where R is aryl or heteroaryl. **Table 1. Examples of fungal luciferins**

| **IUPAC name of compound (other names of compound)** | **Compound formula** | **IUPAC name of substitution group ("R")** |
|---|---|---|
| (E)-6-(3,4-dihydroxystyryl)-3,4-dihydroxy-2H-pyran-2-one (3-hydroxyhispidin) | 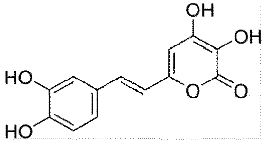 | 3,4-dihydroxyphenyl |
| (E)-3,4-dihydroxy-6-styryl-2H-pyran-2-one | 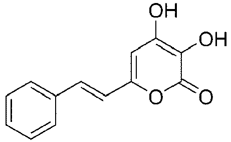 | phenyl |
| (E)-3,4-dihydroxy-6-(4-hydroxystyryl)-2H -pyran- 2-one | 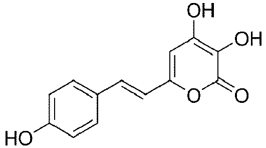 | 4-hydroxyphenyl |
| (E)-3,4-dihydroxy-6-(2-hydroxystyryl)-2H-pyran-2-one | 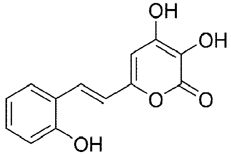 | 2-hydroxyphenyl |
| (E)-3,4-dihydroxy-6-(2,4-dihydroxystyryl)-2H-pyran-2-one | 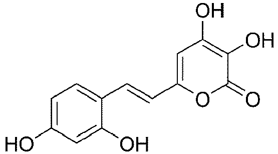 | 2,4-dihydroxyphenyl |
| (E)-3,4-dihydroxy-6- (4-hydroxy-3,5-dimethoxystyryl)-2H-pyran-2-one | 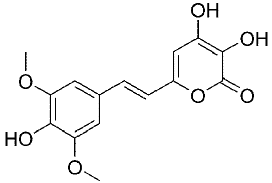 | 4-hydroxy-3,5-dimethoxyphenyl |
| (E)-3,4-dihydroxy-6-(4-hydroxy-3-methoxystyryl)-2H-pyran-2-one | 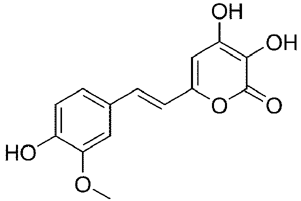 | 4-hydroxy-3-methoxyphenyl |
| (E)-3,4-dihydroxy-6-(2-(6-hydroxynaphthalen-2-yl)vinyl)-2H-pyran-2-one | 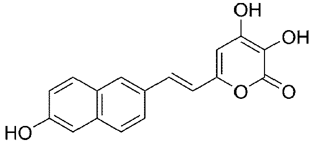 | 6-hydroxynaphthalen-2-yl |
| (E)-6-(4-aminostyryl)-3,4-dihydroxy-2H-pyran-2-one | 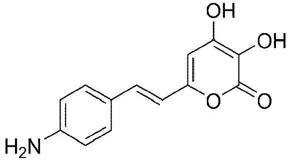 | 4-aminophenyl |
| (E)-6-(4-(diethylamino)styryl)-3,4-hydroxy-2H-pyran-2-one | 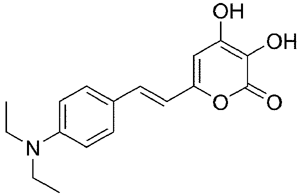 | 4-diethylaminophenyl |
| (E)-6-(2-(1 H-indol-3-yl)vinyl)-3,4-d ihydroxy-2 H-pyran-2-one | 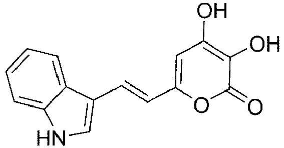 | 1 H-indol-3-yl |
| (E)-3,4-dihydroxy-6-(2,3,6,7-tetrahydro-1H,5H-pyrido[3,2,1-ij]quinolin-9-yl)vinyl)-2H-pyran-2-one | 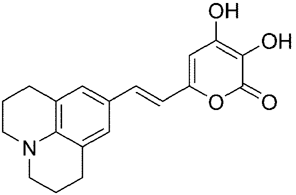 | 2,3,6,7-tetrahydro-1H,5H-pyrido[3,2,1-ij]quinolin-9-yl |

Enzymes that promote either synthesis of luciferins in a living organism or reduction of oxyluciferins back to luciferins are unknown in the overwhelming majority of cases. Therefore, most bioluminescent applications of luciferins involve introducing exogenous luciferase-containing luciferins (e.g. cell culture or organisms) to a system. As a consequence, use of bioluminescent systems stays limited due to a number of reasons comprising, in particular, poor penetrating ability of many luciferins through a cell membrane, chemical instability of luciferins, and complex, multistage, and expensive process of luciferins synthesis.

Enzymes that promote synthesis of luciferin are identified for the only bioluminescent system described in marine bacteria. However, this system is significantly different from other bioluminescent systems. The bacterial luciferin (myristic aldehyde) is oxidized during the reaction, but emits no light [O. Shimomura, Bioluminescence: Chemical Principles and Methods, World Scientific Publishing Co. Pte. Ltd, Singapore, 2006, 470 p.]. Besides the luciferin, key components of the luminescent reaction also include NAD (nicotinamide adenine dinucleotide) and FMN-H₂ (flavin mononucleotide). It is the oxidized derivative of FMN-H₂ that acts as a true light source. The bioluminescent system of marine bacteria is the only one to date that can be fully encoded in a heterologous expression system and considered as the closest prior art of the present invention. However, this system is generally applicable only to prokaryotic organisms. To obtain autonomous bioluminescence, the luxCDABE operon is used, which encodes luciferases (luxA and luxB heterodimers) and luxCDE luciferin biosynthesis proteins acting as the bioluminescence substrate (Meighen 1991). In 2010, this system was used to achieve autonomous luminescence in human cells. However, low bioluminescence intensity level, only 12 times higher than the signal emanating from non-bioluminescent cells, did not allow to apply the developed system for solving the most of applied problems [Close et al. PloS One, 2010, 5 (8):e12441]. Attempts to increase intensity of emitted light were unsuccessful due to toxicity of the bacterial system components for eukaryotic cells [Hollis et al. FEBS Letters, 2001, 506 (2): 140-42].

In this view, identification of enzymes that promote synthesis of luciferin from stable and/or abundant in cells precursor compounds as well as reduction of oxyluciferin back to luciferin is an urgent problem. Identification of such enzymes would enable a simpler and cheaper method for synthesis of luciferin and open the way to creation of autonomous bioluminescent systems. Among them, the bioluminescent systems non-toxic for eukaryotic cells are of particular interest.

### Summary of invention

Applicants have decoded stages of luciferin biosynthesis in the bioluminescent system of fungi and identified the enzymes involved in cyclic circulation of fungal luciferin and the nucleic acid sequences encoding them.

Flowchart below shows stages of fungal luciferin turnover:

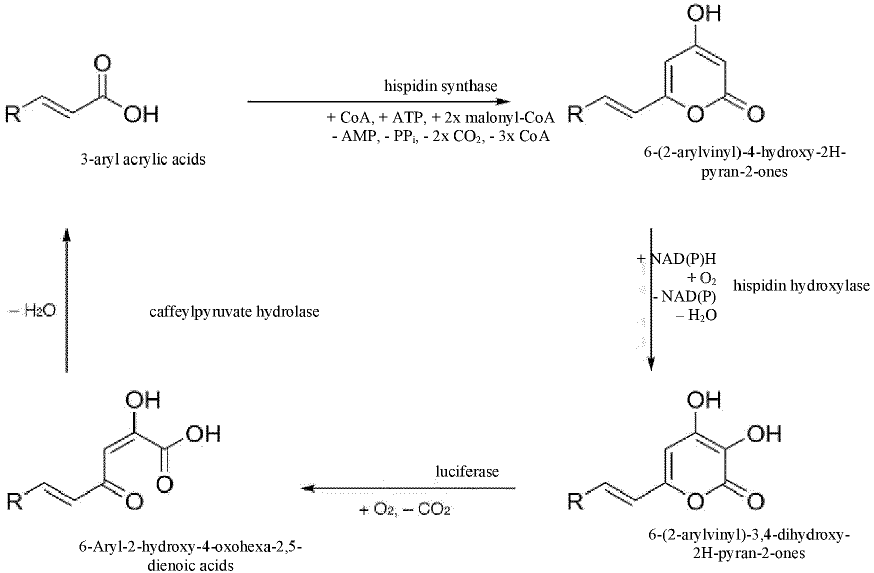

Thus, the present invention first provides isolated fungal luciferin biosynthetic proteins as well as nucleic acids encoding them.

In preferred embodiments, the present invention provides hispidin hydroxylases characterized by the amino acid sequence selected from the following SEQ ID NOs group: 2, 4, 6, 8, 10, 12, 14, 16, 18, 20, 22, 24, 26, 28, as well as essentially similar proteins, homologues, mutants, and derivatives of these hispidin hydroxylases.

In some embodiments, the hispidin hydroxylases of the present invention are characterized by an amino acid sequence that within at least 350 amino acids has at least 60% identity, or at least 65% identity, or at least 70% identity, or at least 75% identity, for example, at least 80% identity, at least 85% identity, at least 90% identity (for example, at least 90%, 91 %, 92%, 93%, 94%, 95%, 96%, 97%, 98%, 98% or 99% identity) with the amino acid sequence selected from the following SEQ ID NOs group: 2, 4, 6, 8, 10, 12, 14, 16, 18, 20, 22, 24, 26, 28.

In some embodiments, the amino acid sequence of the hispidin hydroxylase of the present invention is characterized by presence of several consensus sequences separated by non-conservative amino acid insertion segments characterized by the following SEQ ID NOs: 29-33.

The hispidin hydroxylases of the present invention catalyze the reaction of 6-(2-arylvinyl)-4-hydroxy-2H-pyran-2-one with the structural formula 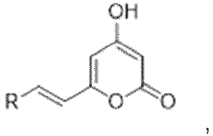 conversion into 6-(2-arylvinyl)-3,4-dihydroxy-2H-pyran-2-one with the structural formula 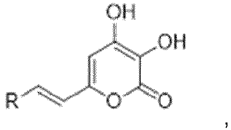 where R is aryl or heteroaryl.

The present invention also provides hispidin synthases characterized by the amino acid sequence selected from the following SEQ ID NOs group: 35, 37, 39, 41, 43, 45, 47, 49, 51, 53, 55, as well as essentially similar proteins, homologues, mutants, and derivatives of these hispidin synthases.

In some embodiments, the amino acid sequence of the hispidin synthase of the present invention is characterized by presence of several consensus sequences separated by non-conservative amino acid insertion segments characterized by the following SEQ ID NOs: 56-63.

In some embodiments, hispidin synthases of the present invention are characterized by an amino acid sequence that has at least 40% identity, for example, at least 45% identity, or at least 50% identity, or at least 55% identity, or at least 60% identity, or at least 65% identity, or at least 70% identity, or at least 75% identity, for example, at least 80% identity, at least 85% identity, at least 90% identity (for example, at least 90%, 91% , 92%, 93%, 94%, 95%, 96%, 97%, 98%, 98% or 99% identity) with the amino acid sequence selected from the following SEQ ID NOs group: 35, 37, 39, 41, 43, 45, 47, 49, 51, 53, 55.

The hispidin synthases of the present invention catalyze the reaction of 3-aryl acrylic acid with the structural formula 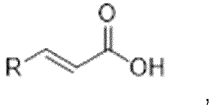 where R is selected from the group aryl or heteroaryl, conversion into 6-(2-arylvinyl)-4-hydroxy-2H-pyran-2-one with the structural formula 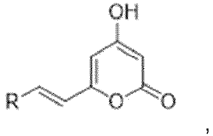 where R is aryl or heteroaryl.

In addition, the present invention provides caffeylpyruvate hydrolases characterized by the amino acid sequence selected from the following SEQ ID NOs group: 65, 67, 69, 71, 73, 75, as well as essentially similar proteins, homologues, mutants, and derivatives of these caffeylpyruvate hydrolases.

In some embodiments, the amino acid sequence of the caffeylpyruvate hydrolase of the present invention is characterized by presence of several consensus sequences separated by non-conservative amino acid insertion segments characterized by the following SEQ ID NOs: 76-78.

In some embodiments, a caffeylpyruvate hydrolase of the present invention is characterized by an amino acid sequence that has at least 60% identity, or at least 65% identity, or at least 70% identity, or at least 75% identity, for example, at least 80% identity, at least 85% identity, at least 90% identity (for example, at least 90%, 91 %, 92%, 93%, 94%, 95%, 96%, 97%, 98%, 98% or 99% identity) with the amino acid sequence selected from the following SEQ ID NOs group: 65, 67, 69, 71, 73, 75.

The caffeylpyruvate hydrolases of the present invention catalyze the reaction of 6-aryl-2-hydroxy-4-oxohexa-2,5-dienoic acids with the structural formula 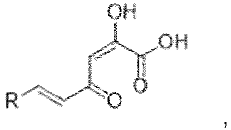 where R is aryl or heteroaryl, conversion into 3-arylacrylic acid with the structural formula

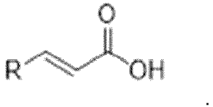

In preferred embodiments, hispidin hydroxylases of the present invention catalyze reaction of preluciferin conversion into fungal luciferin, for example, hispidin conversion into the 3-hydroxyhispidin.

In preferred embodiments, hispidin synthases of the present invention catalyze the conversion of a precursor of preluciferin into the preluciferin, for example, conversion of caffeic acid to hispidin.

In preferred embodiments, caffeylpyruvate hydrolases of the present invention catalyze conversion of fungal oxyluciferin to a precursor of preluciferin, for example, conversion of caffeylpyruvate to caffeic acid.

The present invention also provides application of a protein having the amino acid sequence that within at least 350 amino acids has at least 60% identity, or at least 65% identity, or at least 70% identity, or at least 75% identity, for example, at least 80% identity, at least 85% identity, at least 90% identity (for example, at least 90%, 91%, 92%, 93%, 94%, 95%, 96%, 97%, 98%, 98% or 99% identity) with the amino acid sequence selected from the following SEQ ID NOs group: 2, 4, 6, 8, 10, 12, 14, 16, 18, 20, 22, 24, 26, 28, and/or containing consensus sequences with the SEQ ID NOs 29-33 separated by non-conservative amino acid insertion segments, as hispidin hydroxylase to catalyze in vitro or in vivo reaction of 6-(2-arylvinyl)-4-hydroxy-2H-pyran-2-one with the structural formula 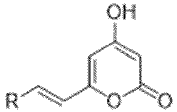 conversion into 6-(2-arylvinyl)-3,4-dihydroxy-2H-pyran-2-one with the structural formula 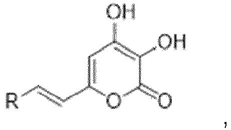 where R is aryl or heteroaryl.

The present invention also provides application of a protein having the amino acid sequence that has at least 45% identity, or at least 50% identity, or at least 55% identity, or at least 60% identity, or at least 65% identity, or at least 70% identity, or at least 75% identity, for example, at least 80% identity, at least 85% identity, at least 90% identity (for example, at least 90%, 91 %, 92%, 93%, 94%, 95%, 96%, 97%, 98%, 98% or 99% identity) with the amino acid sequence selected from the following SEQ ID NOs group: 35, 37, 39, 41, 43, 45, 47, 49, 51, 53, 55, and/or containing consensus sequences with the SEQ ID NOs 56-63 separated by non-conservative amino acid insertion segments, as hispidin synthase to catalyze in vitro or in vivo reaction of 3-aryl acrylic acid with the structural formula 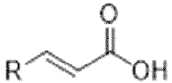 conversion into 6-(2-arylvinyl)-4-hydroxy-2H-pyran-2-one with the structural formula 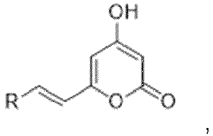 where R is aryl or heteroaryl.

The present invention also provides application of a protein having the amino acid sequence that has at least 60% identity, or at least 65% identity, or at least 70% identity, or at least 75% identity, for example, at least 80% identity, at least 85% identity, at least 90% identity (for example, at least 90%, 91 %, 92%, 93%, 94%, 95%, 96%, 97%, 98%, 98% or 99% identity) with the amino acid sequence selected from the following SEQ ID NOs group: 65, 67, 69, 71, 73, 75, and/or containing consensus sequences with the SEQ ID NOs 76-78 separated by non-conservative amino acid insertion segments, as caffeylpyruvate hydrolase to catalyze in vitro or in vivo reaction of 6-aryl-2-hydroxy-4-oxohexa-2,5-dienoic acids with the structural formula 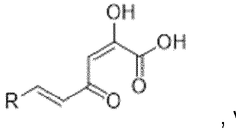 where R is aryl or heteroaryl, conversion into 3-arylacrylic acid with the structural formula

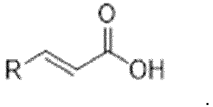

The present invention also provides nucleic acids encoding said hispidin hydroxylases, hispidin synthases, and caffeylpyruvate hydrolases.

In some embodiments, hispidin hydroxylase encoding nucleic acids are provided with amino acid sequence selected from the group:
(a) amino acid sequence presented as the following SEQ ID NOs: 2, 4, 6, 8, 10, 12, 14, 16, 18, 20, 22, 24, 26, 28;
(b) amino acid sequence that within at least 350 amino acids has at least 60% identity, or at least 65% identity, or at least 70% identity, or at least 75% identity, for example, at least 80% identity, at least 85% identity, at least 90% identity (for example, at least 90%, 91%, 92%, 93%, 94%, 95%, 96%, 97%, 98%, 98% or 99% identity) with the amino acid sequence selected from the following SEQ ID NOs group: 2, 4, 6, 8, 10, 12, 14, 16, 18, 20, 22, 24, 26, 28;
(c) amino acid sequence containing consensus sequences presented as the following SEQ ID NOs: 29-33.

In some embodiments, hispidin synthase encoding nucleic acids are provided with amino acid sequence selected from the group:
(a) amino acid sequence presented as the following SEQ ID NOs: 35, 37, 39, 41, 43, 45, 47, 49, 51, 53, 55;
(b) amino acid sequence that has at least 40% identity, for example, at least 45% identity, or at least 50% identity, or at least 55% identity, or at least 60% identity, or at least 65% identity, or at least 70% identity, or at least 75% identity, for example, at least 80% identity, at least 85% identity, at least 90% identity (for example, at least 90%, 91% , 92%, 93%, 94%, 95%, 96%, 97%, 98%, 98% or 99% identity) with the amino acid sequence selected from the following SEQ ID NOs group: 35, 37, 39, 41, 43, 45, 47, 49, 51, 53, 55;
(c) amino acid sequence containing consensus sequences presented as the following SEQ ID NOs: 56-63.

In some embodiments, caffeylpyruvate hydrolases encoding nucleic acids are provided with amino acid sequence selected from the group:
(a) amino acid sequence presented as the following SEQ ID NOs: 65, 67, 69, 71, 73, 75;
(b) amino acid sequence that has at least 60% identity, or at least 65% identity, or at least 70% identity, or at least 75% identity, for example, at least 80% identity, at least 85% identity, at least 90% identity (for example, at least 90%, 91%, 92%, 93%, 94%, 95%, 96%, 97%, 98%, 98% or 99% identity) with the amino acid sequence selected from the following SEQ ID NOs group: 65, 67, 69, 71, 73, 75;
(c) amino acid sequence containing consensus sequences with the SEQ ID NOs 76-78 separated by non-conservative amino acid insertion segments.

The present invention also provides application of the nucleic acid encoding a protein with amino acid sequence that within at least 350 amino acids has at least 60% identity, or at least 65% identity, or at least 70% identity, or at least 75% identity, for example, at least 80% identity, at least 85% identity, at least 90% identity (for example, at least 90%, example, 93%, 94%, 95%, 96%, 97%, 98%, 98% or 99% identity) with the amino acid sequence selected from the following SEQ ID NOs group: 2, 4, 6, 8, 10, 12, 14, 16, 18, 20, 22, 24, 26, 28, and/or containing consensus sequences with the SEQ ID NOs 29-33 separated by non-conservative amino acid insertion segments, to produce in in vitro or in vivo systems the hispidin hydroxylase catalyzing the reaction of 6-(2-arylvinyl)-4-hydroxy-2H-pyran-2-one with the structural formula 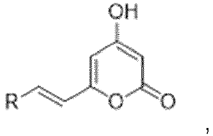 conversion into 6-(2-arylvinyl)-3,4-dihydroxy-2H-pyran-2-one with the structural formula 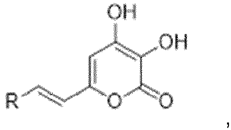 where R is aryl or heteroaryl.

The present invention also provides application of the nucleic acid encoding a protein with amino acid sequence that has at least 45% identity, or at least 50% identity, or at least 55% identity, or at least 60% identity, or at least 65% identity, or at least 70% identity, or at least 75% identity, for example, at least 80% identity, at least 85% identity, at least 90% identity (for example, at least 96%, 97%, 98%, 98% or 99% identity) with the amino acid sequence selected from the following SEQ ID NOs group: 35, 37, 39, 41, 43, 45, 47, 49, 51, 53, 55, and/or containing consensus sequences with the SEQ ID NOs 56-63 separated by non-conservative amino acid insertion segments, to produce in in vitro or in vivo systems the hispidin synthase catalyzing the reaction of 3-aryl acrylic acid with the structural formula 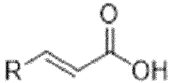 conversion into 6-(2-arylvinyl)-4-hydroxy-2H-pyran-2-one with the structural formula 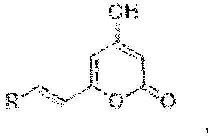 where R is aryl or heteroaryl.

The present invention also provides application of nucleic acid encoding a protein with amino acid sequence that has at least 60% identity, or at least 65% identity, or at least 70% identity, or at least 75% identity, for example, at least 80% identity, at least 85% identity, at least 90% identity (for example, at least 96%, 97%, 98%, 98% or 99% identity) with the amino acid sequence selected from the following SEQ ID NOs group: 65, 67, 69, 71, 73, 75, and/or containing consensus sequences with the SEQ ID NOs 76-78 separated by non-conservative amino acid insertion segments, to produce in in vitro or in vivo systems the caffeylpyruvate hydrolase catalyzing the reaction of 6-aryl-2-hydroxy-4-oxohexa-2,5-dienoic acids with the structural formula 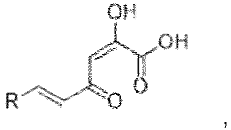 where R is aryl or heteroaryl, conversion into 3-arylacrylic acid with the structural formula

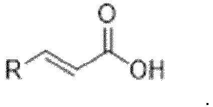

The present invention also provides a fusion protein comprising operatively, directly or via amino acid linkers, cross-linked at least one hispidin hydroxylase of the invention, and/or at least one hispidin synthase of the invention, and/or at least one caffeylpyruvate hydrolase of the invention, and intracellular localization signal, and/or signal peptide, and/or luciferase capable to oxidize the fungal luciferin with light emission.

The luciferase capable to oxidize the fungal luciferin with light emission is known in the art. In preferred embodiments, it has an amino acid sequence substantially similar or identical to an amino acid sequence selected from the following SEQ ID NOs group: 80, 82, 84, 86, 88, 90, 92, 94, 96, 98. For example, it may have an amino acid sequence that is at least 40% identical, for example, at least 45% identical, or at least 50% identical, or at least 55% identical, or at least 60% identical, or at least 70% identical, or at least 75% identical, or at least 80% identical, or at least 85% identical to an amino acid sequence selected from the following SEQ ID NOs group: 80, 82, 84, 86, 88, 90, 92, 94, 96, 98. In many embodiments, the amino acid sequence of said luciferase has at least 90% identity, or at least 95% identity, (e.g. at least 96%, 97%, 98%, 98%, or 99% identity) with an amino acid sequence selected from the following SEQ ID NOs group: 80, 82, 84, 86, 88, 90, 92, 94, 96, 98.

In some embodiments, the fusion protein has the amino acid sequence with SEQ ID NO 101.

The present invention also provides a nucleic acid encoding said fusion protein.

The present invention also provides an expression cassette comprising (a) a domain of transcription initiation, which is functional in a host cell; (b) a nucleic acid encoding a fungal luciferin biosynthesizing enzyme, i.e. hispidin synthase, hispidin hydroxylase or caffeylpyruvate hydrolase, or a fusion protein according to the invention; (c) a domain of transcription termination, which is functional in the host cell.

The present invention also provides a vector for transferring a nucleic acid into a host cell comprising a nucleic acid encoding a fungal luciferin biosynthesizing enzyme of the invention, i.e. hispidin synthase, hispidin hydroxylase, or caffeylpyruvate hydrolase, or a fusion protein of the invention.

The present invention also provides a host cell comprising, as a part of an extrachromosomal element or integrated into genome of the cell as a result of introducing said cassette into said cell, an expression cassette that contains a nucleic acid encoding hispidin synthase and/or hispidin hydroxylase and/or caffeylpyruvate hydrolase of the present invention. Such cell produces at least one of said fungal luciferin biosynthesizing enzymes due to expression of said introduced nucleic acid.

The present invention also provides an antibody obtained using a protein of the invention.

The present invention also provides a method for producing fungal luciferin with the chemical formula 6-2-arylvinyl)-3,4-dihydroxy-2H-pyran-2-one and the structural formula 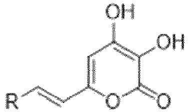 , where R is aryl or heteroaryl, in either in vitro or in vivo system, which comprises combining at least one molecule of hispidin hydroxylase according to the invention with at least one molecule of 6-(2-arylvinyl)-4-hydroxy-2H-pyran-2-one having the structural formula 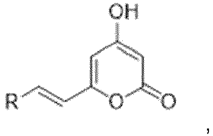 at least one NAD(P)H molecule, and at least one molecular oxygen molecule under physiological conditions.

The present invention also provides a method for producing fungal preluciferin with the chemical formula 6-(2-arylvinyl)-4-hydroxy-2H-pyran-2-one and the structural formula 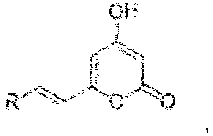 where R is aryl or heteroaryl, in either in vitro or in vivo system, which comprises combining at least one molecule of 3-arylacrylic acid with the structural formula 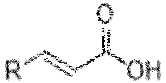 with at least one molecule of hispidin synthase according to the invention, at least one molecule of coenzyme A (CoA), at least one ATP molecule, and at least two malonyl-CoA molecules under physiological conditions.

The present invention also provides a method for in vitro or in vivo producing fungal luciferin, which comprises combining at least one hispidin hydroxylase molecule according to the invention with at least one 3-aryl acrylic acid molecule, at least one molecule of hispidin synthase according to the invention, at least one molecule of coenzyme A, at least one ATP molecule, at least two molecules of malonyl-CoA, at least one NAD(P)H molecule, and at least one molecule of molecular oxygen under physiological conditions.

Methods for producing fungal luciferin and preluciferin can be implemented in a cell or an organism. In this case, said methods comprise introducing into the cell nucleic acids encoding the corresponding luciferin biosynthesizing enzymes (hispidin synthase and/or hispidin hydroxylase) capable of expressing said enzymes in the cell or organism. In preferred embodiments, the nucleic acids are introduced into a cell or organism as a part of an expression cassette or vector of the invention.

In some embodiments, a nucleic acid encoding a 4'-phosphopantotheinyl transferase capable to transfer the 4-phosphopantetheinyl from coenzyme A to serine in the acyl transfer domain of polyketide synthases is additionally introduced into the cell or organism. In some embodiments, the 4'-phosphopantotheinyl transferase has an amino acid sequence substantially similar or identical to SEQ ID NO 105.

The present invention also provides application of the polyketide synthase (PKS) with amino acid sequence that is at least 40%, or at least 45%, or at least 50%, or at least 55 %, or at least 60%, at least 65%, or at least 70%, or at least 80%, or at least 85%, or at least 90%, or at least 91 %, or at least 92%, or at least 93%, or at least 94%, or at least 95%, or at least 96%, or at least at least 97%, or at least 98%, or at least 99% identical to a sequence selected from the following SEQ ID NOs group: 119, 121, 123, 125, 127, 129, 131, 133, 135, 137, 139 to produce hispidin in an in vitro or in vivo system.

In some embodiments, the method for preparing hispidin comprises combining at least one PKS molecule with at least two malonyl-CoA molecules and at least one caffeyl-CoA molecule under physiological conditions. In some embodiments, said method comprises combining at least one PKS molecule with at least two malonyl-CoA molecules, at least one caffeic acid molecule, at least one coenzyme A molecule, at least one coumarate-CoA ligase molecule, and at least one ATP molecule under physiological conditions.

For the purposes of present invention, any coumarate-CoA ligase can be used that catalyzes conversion of caffeic acid into caffeyl-CoA. For example, coumarate-CoA ligase may have an amino acid sequence that is at least 40%, or at least 45%, or at least 50%, or at least 55%, or at least 60%, or at least 65%, or at least 70%, or at least 80%, or at least 85%, or at least 90%, or at least 91 %, or at least 92%, or at least 93%, or at least 94%, or at least 95%, or at least 96%, or at least 97%, or at least 98%, or at least 99% identical to the sequence with SEQ ID NO 141.

Said reaction can be used in any of said methods instead of reaction for producing fungal preluciferin from precursors of preluciferin using the hispidin synthase of the present invention. For example, the reaction can be performed in a cell or organism by introducing an expression cassette with a PKS encoding nucleic acid into the cell or organism. If necessary, a coumarate-CoA ligase encoding nucleic acid can be additionally introduced into the cell or organism.

In some embodiments, 3-aryl acrylic acid biosynthesizing enzymes encoding nucleic acids are further introduced into the same cell or organism. For example, these can be nucleic acids encoding tyrosine ammonia-lyase with an amino acid sequence substantially similar or identical to the amino acid sequence of tyrosine ammonia-lyase of Rhodobacter capsulatus having SEQ ID NO 107 or nucleic acids encoding the HpaB and HpaC components of 4-hydroxyphenylacetate 3-monooxygenase reductase with the amino acid sequences substantially similar to the sequences of HpaB and HpaC components of 4-hydroxyphenylacetate 3-monooxygenase reductase of E. coli having SEQ ID NOs 109 and 111. In some embodiments, the phenylalanine ammonia-lyase encoding nucleic acid with amino acid sequence substantially similar to the amino acid sequence having SEQ ID NO 117 is used.

The present invention also provides methods for producing transgenic bioluminescent cells or organisms, comprising cells or organisms of plants, animals, bacteria, or fungi.

In preferred embodiments, methods for producing transgenic bioluminescent cells or organisms comprise introducing at least one nucleic acid of the invention together with a nucleic acid encoding the luciferase capable to oxidize fungal luciferin with light emission into a cell or organism. Nucleic acids are introduced into a cell or organism in a form such as to enable their expression and production of functional protein products. For example, nucleic acids may be contained in an expression cassette. Nucleic acids can occur in cells as parts of either extrachromosomal elements or integrated into genome of the cell due to insertion of an expression cassette into said cell.

In preferred embodiments, methods for producing transgenic bioluminescent cells or organisms comprise introducing a nucleic acid encoding a hispidin hydroxylase of the invention and a nucleic acid encoding a luciferase capable to oxidize fungal luciferin with light emission into the cell or organism. As a result, said cell or organism acquires the ability to bioluminescence in the presence of fungal preluciferin with the chemical formula 6-(2-arylvinyl)-4-hydroxy-2H-pyran-2-one and structural formula 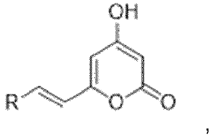 where R is aryl or heteroaryl.

In some embodiments, a hispidin hydroxylase and luciferase fusion protein encoding nucleic acid is introduced into the cell instead of nucleic acids encoding hispidin synthase and luciferase.

In some embodiments, methods for producing transgenic bioluminescent cells or organisms also comprise introducing a hispidin synthase encoding nucleic acid of the invention into the cell or organism. Said cell or organism acquires the ability to bioluminescence in the presence of the precursor of fungal preluciferin in the form of 3-arylacrylic acid with the structural formula 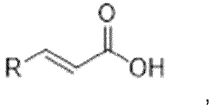 where R is aryl or heteroaryl.

In some embodiments, a PKS encoding nucleic acid is introduced into a cell instead of hispidin synthase encoding nucleic acid.

In some embodiments, methods for producing transgenic bioluminescent cells or organisms also comprise introducing a caffeylpyruvate hydrolase encoding nucleic acid of the invention into the cell or organism to increase intensity of the bioluminescence.

In some embodiments, methods for producing transgenic bioluminescent cells or organisms also include introducing a 4'-phosphopantotheinyl transferase encoding nucleic acid into the cell or organism.

In some embodiments, methods for producing transgenic bioluminescent cells or organisms also comprise introducing a coumarate-CoA ligase encoding nucleic acid into the cell or organism.

In some embodiments, methods for producing transgenic bioluminescent cells or organisms also include introducing 3-aryl acrylic acid biosynthesizing enzymes encoding nucleic acids into the cell or organism.

The present invention also provides transgenic bioluminescent cells and organisms obtained by the said methods and containing one or more nucleic acids of the invention as part of an extrachromosomal element or integrated into the genome of the cell.

In some embodiments, transgenic bioluminescent cells and organisms of the invention are capable of autonomous bioluminescence without exogenous addition of luciferin, preluciferin, and precursor of preluciferin.

The present invention also provides combinations of proteins and nucleic acids of the invention as well as products and kits containing the proteins and nucleic acids of the invention. For example, combinations of nucleic acids are provided for producing autonomously luminous cells, cell lines, or transgenic organisms; assaying the activity of promoters, or labeling cells.

In some embodiments, kits for producing fungal luciferin and/or fungal preluciferin are provided comprising said hispidin hydroxylase, and/or hispidin synthase, and/or PKS, or encoding them nucleic acids.

In some embodiments, kits are provided for producing a bioluminescent cell or bioluminescent transgenic organism comprising a hispidin hydroxylase encoding nucleic acid and a luciferase encoding nucleic acid, said luciferase being capable to oxidize fungal luciferin with light emission. The kit may also contain a caffeylpyruvate hydrolase encoding nucleic acid. The kit may also contain a hispidin synthase or PKS encoding nucleic acid. The kit may also contain 4'-phosphopantotheinyl transferase encoding nucleic acid and/or coumarate-CoA ligase encoding nucleic acid and/or 3-aryl acrylic acid biosynthesizing enzymes encoding nucleic acids. The kit may also contain additional components such as buffer solutions, antibodies, fungal luciferin, fungal preluciferin, precursor of fungal preluciferin, etc. The kit may also contain the kit application guide. In some embodiments, the nucleic acids are provided in expression cassettes or vectors for introduction into cells or organisms.

In preferred embodiments, cells or transgenic organisms of the invention are capable to produce fungal luciferin from precursors. In some embodiments, cells and transgenic organisms of the invention are capable of bioluminescence in presence of precursor of fungal luciferin. In some embodiments, cells or transgenic organisms of the invention are capable of autonomous bioluminescence.

In preferred embodiments of above disclosed methods and application, the preluciferin with chemical formula 6-(2-arylvinyl)-4-hydroxy-2H-pyran-2-one selected from the following group:
(E)-6-(3,4-dihydroxystyryl)-4-hydroxy-2H-pyran-2-one (hispidin),
(E)-4-dihydroxy-6-styryl-2H-pyran-2-one,
(E)-4-hydroxy-6-(4-hydroxystyryl)-2H-pyran-2-one (bisnoryangonin),
(E)-4-hydroxy-6-(2-hydroxystyryl)-2H-pyran-2-one,
(E)-4-hydroxy-6-(2,4-dihydroxystyryl)-2H-pyran-2-one,
(E)-4-hydroxy-6-(4-hydroxy-3,5-dimethoxystyryl)-2H-pyran-2-one,
(E)-4-hydroxy-6-(4-hydroxy-3-methoxystyryl)-2H-pyran-2-one,
(E)-4-hydroxy-6-(2-(6-hydroxynaphthalen-2-yl)vinyl)-2H-pyran-2-one,
(E)-6-(4-aminostyryl)-4-hydroxy-2H-pyran-2-one,
(E)-6-(4-(diethylamino)styryl)-4-hydroxy-2H-pyran-2-one,
(E)-6-(2-(1H-indol-3-yl)vinyl)-4-hydroxy-2H-pyran-2-one,
(E)-4-hydroxy-6-(2,3,6,7-tetrahydro-1H,5H-pyrido[3,2,1-ij]quinolin-9-yl)vinyl)-2H-pyran-2-one is used.

In preferred embodiments, a 3-aryl acrylic acid selected from the group comprising caffeic acid, cinnamic acid, paracoumaric acid, coumaric acid, umbellic acid, sinapic acid, and ferulic acid is suitable for the purposes of the present invention.

In preferred embodiments, 3-hydroxyhispidine is used as the luciferin, hispidin as the preluciferin, and caffeic acid as the precursor of preluciferin.

One object of the present invention is to provide an effective method for producing autonomous bioluminescent systems with visible luminescence, including those based on eukaryotic non-luminous cells and organisms.

Another object of the present invention is to provide a new effective method for synthesizing hispidin or functional analogues thereof.

Another object of the present invention is to provide a new effective method for synthesizing fungal luciferins or functional analogues thereof.

Another object of the present invention is to provide autonomously luminous cells or organisms.

The object of the present invention is achieved by identifying stages of luciferin conversion in bioluminescent fungi and identifying amino acid and nucleotide sequences of proteins involved in luciferin biosynthesis. The function of all proteins has been demonstrated for the first time.

### Brief description of drawings

Fig. 1 shows a multiple amino acid sequence alignment of hispidin hydroxylases. FAD/NAD(P)-binding domain is underlined. Consensus sequences are shown below alignment.
Fig. 2 shows a multiple amino acid sequence alignment of hispidin synthases. Consensus sequences are shown below alignment.
Fig. 3 shows a multiple amino acid sequence alignment of caffeylpyruvate hydrolases. Consensus sequences are shown below alignment.
Fig. 4 shows luminescence intensities of *Pichia pastoris* cells expressing hispidin hydroxylase and luciferase (A) or only luciferase (B), and luminescence intensities of wild type yeast (C), when the colonies are sprayed with 3-hydroxyhispidin (luciferin, left plot) or hispidin (preluciferin, right plot).
Fig. 5 presents luminescence intensity of HEK293NT cells expressing hispidin hydroxylase and luciferase compared with that of HEK293NT cells expressing luciferase only when adding hispidin.
Fig. 6 shows luminescence curves of HEK293T cells expressing: (1) hispidin hydroxylase and luciferase genes separately when adding hispidin; (2) hispidin hydroxylase and luciferase chimeric protein gene when adding hispidin; (3) hispidin hydroxylase and luciferase chimeric protein gene when adding 3-hydroxyhispidine.
Fig. 7 illustrates ability of transfected *Pichia pastoris* cells to autonomous bioluminescence in contrast to wild-type cells. On the left: cells on Petri dish under daylight, on the right: cells in the dark.
Fig. 8 shows luminescence of a culture of transfected *Pichia pastoris* cells in the dark.
Fig. 9 shows autonomously bioluminescent transgenic plants *Nicotiana benthamiana.* Photo on the left was taken in ambient light, photo on the right was taken in the dark.

### Embodiments of invention

### Definitions

Various terms related with objects of the present invention are used above as well as in the description and in claims below. The terms "comprises" and "comprising" in the description of this invention are interpreted as "comprises, but not limited to". The said terms are not intended to be interpreted as "consists only of".

Terms "luminescence" and "bioluminescence" are interchangeable for the purposes of present invention and refer to the phenomenon of light emission in course of a chemical reaction catalyzed by the enzyme luciferase.

Terms "capable to react", "promote a reaction" and the like in relation to the activity of a protein mean that said protein is an enzyme that catalyzes the indicated reaction.

For the purposes of present invention, term "luciferase" means a protein that has ability to catalyze oxidation of a chemical compound (luciferin) by molecular oxygen such that the oxidation reaction is accompanied by light emission (luminescence or bioluminescence) and formation of oxidized luciferin.

For the purposes of present invention, term "fungal luciferin" means a chemical compound selected from the group of 6-(2-arylvinyl)-3,4-dihydroxy-2H-pyran-2-ones with the structural formula 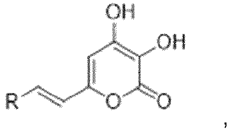 where R is aryl or heteroaryl.

Fungal luciferin is oxidized by a group of luciferases, hereinafter referred to as "luciferases capable to oxidize fungal luciferin with light emission" or the like. Such luciferases were found in bioluminescent fungi, for example, they are described in application RU2017102986/10 (005203) dated 30.01.2017. Amino acid sequences of the luciferases useful for methods and combinations of the present invention are substantially similar or identical to amino acid sequences selected from the following SEQ ID NOs group: 80, 82, 84, 86, 88, 90, 92, 94, 96, 98. In many embodiments of the present invention, luciferases useful for purposes of the present invention are characterized by amino acid sequences that are at least 40% identical, for example, at least 45% identical, or at least 50% identical, or at least 55% identical, or at least 60% identical, or at least 70% identical, or at least 75% identical, or at least 80% identical, or at least 85% identical to an amino acid sequence selected from the following SEQ ID NOs group: 80, 82, 84, 86, 88, 90, 92, 94, 96, 98. In many cases luciferases are characterized by amino acid sequences that have at least 90% identity (for example, at least 91%, at least 92%, at least 93%, at least 94%, at least 95%, at least 96%, at least 97%, at least 98%, at least 99% identity or 100% identity) with an amino acid sequence selected from the following SEQ ID NOs group: 80, 82, 84, 86, 88,90, 92, 94, 96, 98.

Oxidation of fungal luciferin produces a "fungal oxyluciferin", a product with the chemical formula 6-aryl-2-hydroxy-4-oxohexa-2,5-dienoic acid and the structural formula

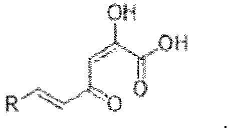

Term "fungal preluciferin" or simply "preluciferin" is used herein to refer to compounds from the group of 6-(2-arylvinyl)-4-hydroxy-2H-pyran-2-ones with the structural formula 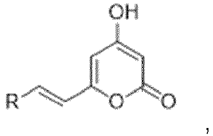 where R is aryl or heteroaryl. The preluciferin is converted to fungal luciferin in a chemical reaction catalyzed by an enzyme of the present invention.

Term "precursor of preluciferin" is used herein to refer to compounds belonging to a group of 3-aryl acrylic acids with the structural formula 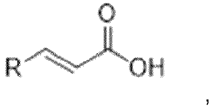 where R is aryl or heteroaryl. Preluciferins are formed from 3-aryl acrylic acids in course of a chemical reaction catalyzed by an enzyme of the present invention.

Examples of fungal luciferins are presented in Table 1. Examples of fungal luciferin related preluciferins, oxyluciferins, and of preluciferins are shown in Table 2.

**Table 2. Examples of fungal luciferin related preluciferins, oxyluciferins, and of preluciferins (names of compounds are presented in accordance with IUPAC nomenclature; traditional names are shown in bold under structural formulas).**

| **Luciferin** | **Precursor of preluciferin** | **Preluciferin** | **Oxyluciferin** |
|---|---|---|---|
| (E)-6-(3,4-dihydroxysty ryl)-3,4-dihydroxy-2H-pyran-2-one(3-**hydroxyhis pidin)** | (E)-3-(3,4-dihydroxyphenyl)prope noic acid | (E)-6-(3,4-dihydroxystyryl)-4-dihydroxy-2H-pyran-2-one | (2E,5E)-6-(3,4-dihydroxystyryl)-2-hydroxy-4-oxohexa-2,5-dienoic acid |
| | 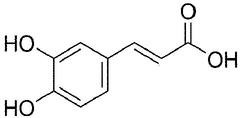 | 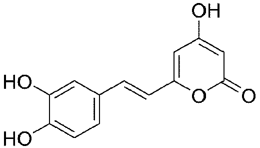 | 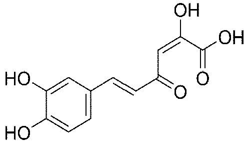 |
| | **caffeic acid** | **hispidin** | **caffeylpyruvate** |
| (E)-3,4-dihydroxy-6-styryl-2H-pyran-2-one | (E)-3-phenylpropenoic acid | (E)-4-dihydroxy-6-styryl-2H-pyran-2-one | (2E,5E)-2-hydroxy-4-oxo-6-phenylhexa-2,5-dienoic acid |
| | 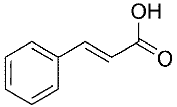 | 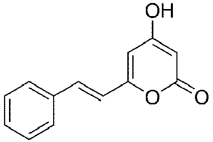 | |
| | **cinnamic acid** | | |
| | | | 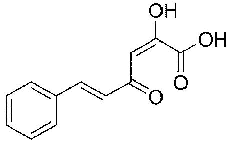 |
| (E)-3,4-dihydroxy-6-(4-hydroxystyry I)-2H-pyran-2-one | (E)-3-(4-hydroxyphenyl)propen oic acid | (E)-4-hydroxy-6-(4-hydroxystyryl)-2H-pyran-2-one | (2E,5E)-2-hydroxy-6-(4-hydroxyphenyl)-4-oxohexa-2,5-dienoic acid |
| | 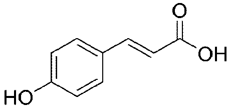 | 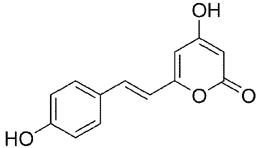 | 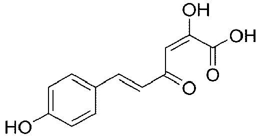 |
| | **paracoumaric acid** | **bisnoryangonin** | |
| (E)-3,4-dihydroxy-6-(2-hydroxystyry l)-2H-pyran-2-one | (E)-3-(2-hydroxyphenyl)propen oic acid | (E)-4-hydroxy-6-(2-hydroxystyryl)-2 H-pyran-2-one | (2E,5E)-2-hydroxy-6-(2-hydroxyphenyl)-4-oxohexa-2,5-dienoic acid |
| | 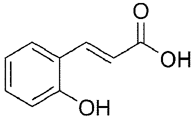 | 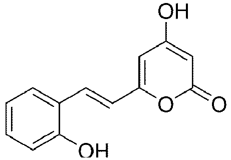 | 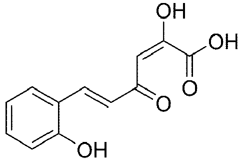 |
| | **coumaric acid** | | |
| (E)-3,4-dihydroxy-6-(2,4-dihydroxysty ryl)-2 H-pyran-2-one | (E)-3-(2,4-dihydroxyphenyl)prope noic acid | (E)-4-hydroxy-6-(2,4-dihydroxystyryl)-2H-pyran-2-one | (2E,5E)-2-hydroxy-6-(2.4-hydroxyphenyl)-4-oxohexa-2,5-dienoic acid |
| | 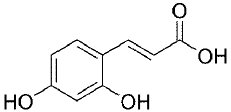 | 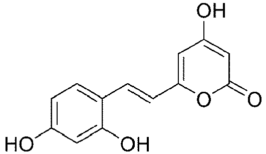 | 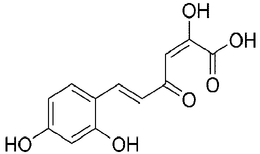 |
| | **umbellic acid** | | |
| (E)-3,4-dihydroxy-6-(4-hydroxy-3,5-dimethoxyst yryl)-2H-pyran-2-one | (E)-3-(4-hydroxy-3,5-dimethoxyphenyl)prop enoic acid | (E)-4-hydroxy-6-(4-hydroxy-3,5-dimethoxystyryl)-2H-pyran-2-one | (2E,5E)-2-hydroxy-6-(4-hydroxy-3,5-dimethoxyphenyl)-4-oxohexa-2,5-dienoic acid |
| | | | |
| | 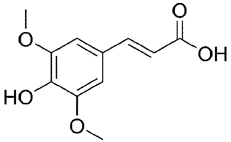 | 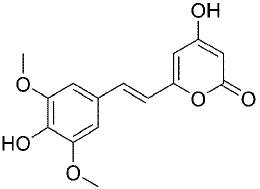 | 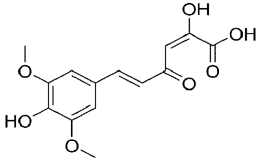 |
| | **sinapic acid** | | |
| (E)-3,4-dihydroxy-6-(4-hydroxy-3-methoxystyr yl)-2H-pyran-2-one | (E)-3-(4-hydroxy-3-methoxyphenyl)propen oic acid | (E)-4-hydroxy-6-(4-hydroxy-3-methoxystyryl)-2H-pyran-2-one | (2E,5E)-2-hydroxy-6-(4-hydroxy-3-methoxyphenyl)-4-oxohexa-2,5-dienoic acid |
| | 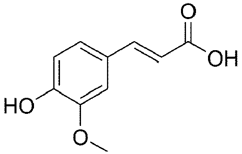 | 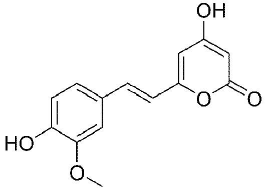 | 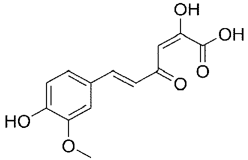 |
| | **ferulic acid** | | |
| (E)-3,4-dihydroxy-6-(2-(6-hydroxynap hthalen-2-yl)vinyl)-2H-pyran-2-one | (E)-3-(6-hydroxynaphthalen-2-yl)propenoic acid | (E)-4-hydroxy-6-(2-(6-hydroxynaphthalen-2-yl)vinyl)-2H-pyran-2-one | (2E,5E)-2-hydroxy-6-(6-hydroxynaphthalen-2-yl)-4-oxohexa-2,5-dienoic acid |
| | 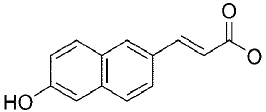 | 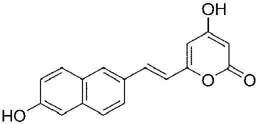 | 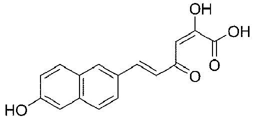 |
| | **3-(6-hyd roxynaphthalen-2-yl)acrylic acid** | | |
| (E)-6-(4-aminostyryl) -3,4-dihydroxy-2H-pyran-2-one | (E)-3-(4-aminophenyl)propenoi c acid | (E)-6-(4-aminostyryl)-4-hydroxy-2H-pyran-2-one | (2E,5E)-6-(4-aminophenyl)-2-hydroxy-4-oxohexa-2,5-dienoic acid |
| | 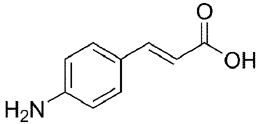 | 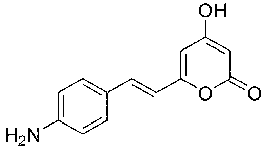 | 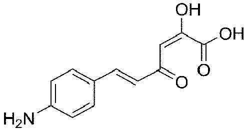 |
| | **4-aminocinnamic acid** | | |
| (E)-6-(4-(diethylamin o)styryl)-3,4-hydroxy-2H-pyran-2-one | (E)-3-(4-diethylaminophenyl)pr openoic acid | (E)-6-(4-(diethylamino)styryl)-4-hydroxy-2H-pyran-2-one | (2E,5E)-6-(4-(diethylamino)phenyl-2-hydroxy-4-oxohexa-2,5-dienoic acid |
| | 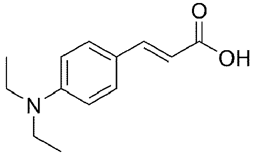 | 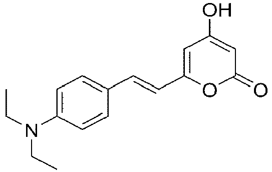 | 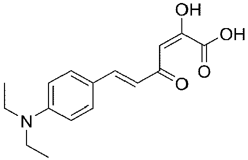 |
| | **4-diethylaminocinnami c acid** | | |
| (E)-6-(2-(1 H-indol-3-yl)vinyl)-3,4-dihydroxy-2H-pyran-2-one | (E)-3-(1 H-indol-3-yl)propenoic acid | (E)-6-(2-(1H-indol-3-yl)vinyl)-4-hydroxy-2H-pyran-2-one | (2E,5E)-2-hydroxy-6-(1 H-indol-3-yl)-4-oxohexa-2,5-dienoic acid |
| | 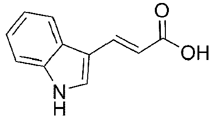 | 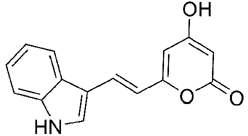 | 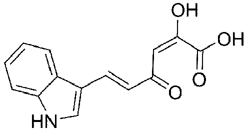 |
| | **3-indolylacrylic acid** | | |
| (E)-3,4-dihydroxy-6-(2,3,6,7-tetrahydro-1 H,5H-pyrido[3,2,1-ij]quinolin-9-yl)vinyl)-2H-pyran-2-one | (E)-3-(2,3,6,7-tetrahydro-1H,5H-pyrido[3,2,1-ij]quinolin-9-yl)propenoic acid | (E)-4-hydroxy-6-(2,3,6,7-tetrahydro-1 H,5H-pyrido[3,2,1-ij]quinolin-9-yl)vinyl)-2H-pyran-2-one | (2E,5E)-2-hydroxy-4-oxo-6-(2,3,6,7-tetrahydro-1 H,5H-pyrido[3,2,1-ij]quinolin-9-yl)hexa-2,5-dienoic acid |
| | 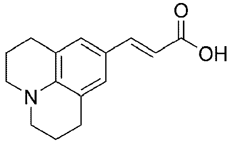 | | |
| | | 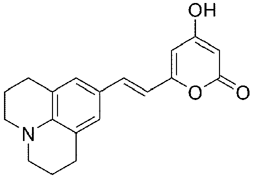 | 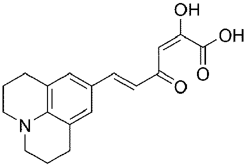 |

Term "aryl" or "aryl substituent" refers to an aromatic radical in a single or fused carbocyclic ring system containing from five to fourteen ring members. In a preferred embodiment, the ring system contains from six to ten ring members. In addition, one or more hydrogen atoms can be replaced with a substituent selected from acyl, acylamino, acyloxy, alkenyl, alkoxy, alkyl, alkynyl, amino, aryl, aryloxy, azido, carbamoyl, carboalkoxy, carboxy, carboxyamido, carboxyamino, cyano, disubstituted amino, formyl, guanidino, halogen, heteroaryl, heterocyclyl, hydroxy, iminoamino, monosubstituted amino, nitro, oxo, phosphonamino, sulfinyl, sulfonamino, sulfonyl, thio, thioacylamino, thioureido or ureido group. Examples of aryl groups include, but are not limited to, phenyl, naphthyl, biphenyl, and terphenyl. Besides, term "aryl", as used herein, refers to groups with the aromatic ring linked to one or more non-aromatic rings.

Term "heterocyclic aromatic substituent", "heteroaryl substituent" or "heteroaryl" refers to an aromatic radical that contains from one to four heteroatoms or hetero groups selected from O, N, S, or SO, in a single or fused heterocyclic ring system containing from five up to fifteen ring members. In a preferred embodiment, the heteroaryl ring system contains from six to ten ring members. In addition, one or more hydrogen atoms can be replaced with a substituent selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halogen, heteroaryl, heterocyclyl, hydroxy, iminoamino, monosubstituted amino, nitro, oxo, phosphonamino, sulfinyl, sulfonamino, sulfonyl, thio, thioacylamino, thioureido or ureido group. Examples of heteroaryl groups include, but are not limited to, pyridinyl, thiazolyl, thiadiazolyl, isoquinolinyl, pyrazolyl, oxazolyl, oxadiazoyl, triazolyl, and pyrrolyl groups. Besides, term "heteroaryl", as used herein, refers to groups with the heteroaromatic ring linked to one or more non-aromatic rings.

Names of chemical compounds are used in the present invention in accordance with the international IUPAC nomenclature. Traditional names are presented as well (if any).

Term "luciferin biosynthesizing enzyme", or "enzyme involved in cyclic turnover of luciferin conversions", or the like is used to mean an enzyme that catalyzes the conversion of a preluciferin precursor to preluciferin, and/or preluciferin to fungal luciferin, and/or oxyluciferin to a preluciferin precursor, in *in vitro* and/or *in vivo* systems. The term "fungal luciferin biosynthesizing enzyme" does not cover luciferases, unless otherwise specified.

Term "hispidin hydroxylase" is used herein to describe the enzyme that catalyzes reaction of converting preluciferin to fungal luciferin, for example, synthesizing 3-hydroxyhispidin from hispidin.

Term "hispidin synthase" is used herein to describe an enzyme capable to catalyze synthesis of fungal preluciferin from a precursor of preluciferin, for example, synthesis of hispidin from caffeic acid.

Term "PKS" is used herein to describe an enzyme belonging to the group of type III polyketide synthases capable to catalyze synthesis of hispidin from caffeyl-CoA.

Term "caffeylpyruvate hydrolase" is used herein to describe an enzyme capable to catalyze decomposition of fungal oxyluciferin into simpler compounds, for example, to form a precursor of preluciferin. For example, it can catalyze conversion of caffeylpyruvate to caffeic acid.

Term "functional analogue" is used in the present invention to describe chemical compounds or proteins that perform the same function and/or can be used for the same purpose. For example, all fungal luciferins listed in Table 1 are functional analogs of each other.

Term "ATP" refers to adenosine triphosphate, which is the main carrier of energy in the cell and has the structural formula:

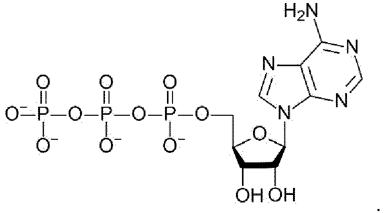

Term "NAD(P)H" is used herein to refer to the reduced nicotinamide adenine dinucleotide phosphate (NADPH) moiety or nicotinamide adenine dinucleotide (NADH) moiety. Term "NAD(P)" is used to refer to the oxidized form of nicotinamide adenine dinucleotide phosphate (NADP) or nicotinamide adenine dinucleotide (NAD). Nicotinamide adenine dinucleotide: 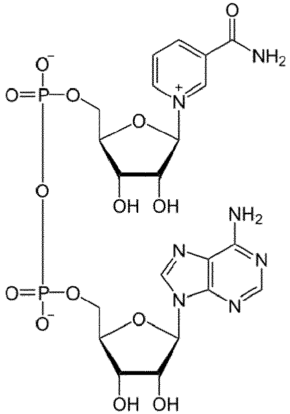 and nicotinamide adenine dinucleotide phosphate: 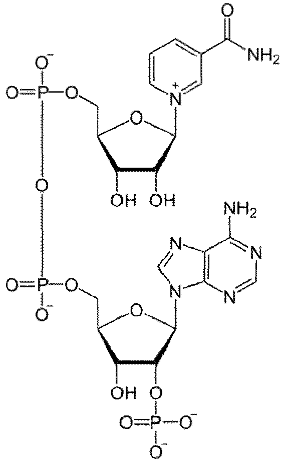 are dinucleotides built from nicotinic acid amide and adenine linked by a chain consisting of two D-ribose residues and two phosphoric acid residues. NADP differs from NAD by presence of additional phosphoric acid residue attached to hydroxyl of a D-ribose residue. Both compounds are widespread in nature and participate in many redox reactions, performing function of carriers of electrons and hydrogen, which it receives from oxidized substances. The reduced forms transfer the received electrons and hydrogen to other substances.

Terms "coenzyme A" or "CoA" refers to a coenzyme well known from the prior art, which is involved in oxidation or synthesis of fatty acids, biosynthesis of fats, oxidative transformations of carbohydrate decomposition products and has the structural formula:

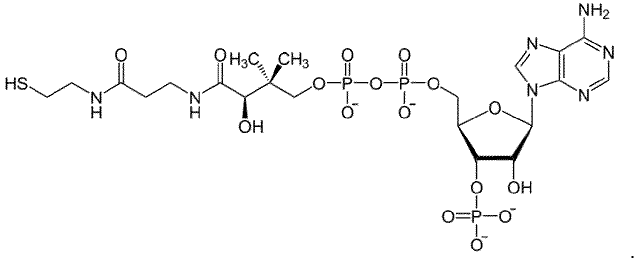

Term "malonyl-CoA" refers to a derivative of coenzyme A formed during synthesis of fatty acids and containing a malonic acid residue:

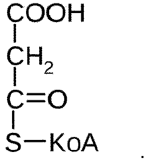

Term "coumaroyl-CoA" refers to the thioester of coenzyme A and coumaric acid:

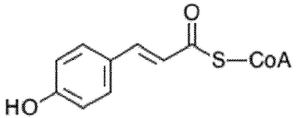

Term "caffeyl-CoA" refers to the thioester of coenzyme A and caffeic acid:

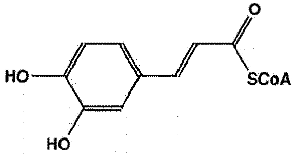

Term "mutant" or "derivative", as used herein, refers to a protein disclosed in the present invention, wherein one or more amino acids are added to, and/or substituted at, and/or removed (deleted) from, and/or incorporated (inserted) into N-terminus, and/or C-terminus, and/or a native amino acid sequence within a protein of the present invention. As used here, the term "mutant" refers to a nucleic acid moiety that encodes a mutant protein. Besides, the term "mutant", as used herein, refers to any variant that is shorter or longer than the protein or nucleic acid disclosed in the present invention.

Term "homology" is used to describe the relationship between nucleotide or amino acid sequences, which is determined by the degree of identity and/or similarity between said sequences under comparison.

As used herein, an amino acid or nucleotide sequence is "substantially identical" or "substantially the same" as a reference sequence, if the amino acid or nucleotide sequence has at least 40% identity with the sequence selected within the reference domain. Hence, the substantially similar sequences include those having, for example, at least 40% identity, or at least 50% identity, or at least 55% identity, or at least 60% identity, or at least 62% identity, or at least 65% identity, or at least 70% identity, or at least 75% identity, for example, at least 80% identity, at least 85% identity, at least 90% identity (for example, 90%, 91 %, 92%, 93%, 94%, 95%, 96%, 97%, 98%, 98% or 99% identity). Two sequences that are identical to one another are also substantially similar. For the purposes of the present invention, length of sequences to be compared must be at least 100 or more amino acids, preferably at least 200 amino acids, for example, 300 amino acids or more. In particular, it is possible to compare full length amino acid sequences of proteins. For nucleic acids, length of sequences to be compared must be at least at least 300 or more nucleotides; preferably at least 600 nucleotides, including 900 or more nucleotides.

One example of the algorithm suitable for determining sequence identity percentage and sequence similarity is the BLAST algorithm described by Altschul et al., J. Mol. Biol. 215: 403-410 (1990). Software for performing BLAST analyzes is available through the National Biotechnology Information Center (http-//www.ncbi.nlm.nih.gov/). This algorithm comprises, first of all, search of high-scoring segment pairs (HSP) by identifying short words of length W in the test sequence, which either completely coincide or satisfy a certain positive-valued threshold score T when aligned with a word of the same length in a database sequence. T is referred to as the neighborhood word score threshold (Altschul et al., 1990). These initial neighborhood word hits act as seeds for initiating searches of longer HSPs containing them. Then these word hits are extended in both directions along each sequence for as far as the cumulative alignment score can be increased. For nucleotide sequences, cumulative scores are calculated using parameters M (reward score set for a pair of matching residues; it is always > 0) and N (penalty score set for mismatching residues; it is always < 0). To calculate the cumulative value for amino acid sequences, a scoring matrix is used. Extension of the word hits in each direction is halted when the cumulative alignment score falls off by the quantity X from its maximum achieved value; the cumulative score goes to zero or below due to accumulation of one or more negative-scoring residue alignments; or the end of either sequence is reached. BLAST algorithm parameters W, T, and X determine the sensitivity and speed of alignment. In the BLASTN program (for nucleotide sequences), the default word length (W) is 11, the expected value (E) is 10, the drop-off (cutoff) is 100, M = 5, N = -4, and comparison is performed on both strands. In the BLASTP program (for amino acid sequences), the default word length (W) is 3, the expected value (E) is 10, and a BLOSUM62 scoring matrix is used (see Henikoff and Henikoff, Proc. Natl. Acad. Sci. USA 89: 10915 (1989)).

In addition to calculating the sequence identity percentage, BLAST algorithm also performs statistical similarity analysis between two sequences (see, for example, Karlin and Altschul, Proc. Nat'l. Acad. Sci. USA 90: 5873-5787 (1993)). One of parameters provided by the BLAST algorithm to determine the similarity is the lowest cumulative probability (P(N)), which indicates the probability of random coincidence between two nucleotide or amino acid sequences. For example, a test nucleic acid sequence is considered to be similar to a reference sequence if the lowest cumulative probability in comparing the test nucleic acid sequence with the reference nucleic acid sequence is less than 0.1, more preferably less than 0.01, and most preferably less than 0.001.

Term "consensus sequence" refers to an archetypal amino acid sequence used as a reference for comparison of all variants of a particular protein or sequence of interest. Consensus sequences and methods for determining them are well known to those skilled in the art. For example, a consensus sequence can be determined from multiple comparisons of known homologous proteins by identifying the amino acids most frequently occurring at a given position in the entire set of related sequences.

Term "conserved sequence" is used to designate a nucleotide sequence in a nucleic acid or a sequence of amino acids in a polypeptide chain that stays completely or virtually unchanged in the course of evolution in different organisms. Accordingly, a "non-conserved sequence" is a sequence that varies considerably among the compared organisms.

Term "amino acid insertion segment" means one or more amino acids within a polypeptide chain that are between protein fragments (protein domains, linkers, consensus sequences) under consideration. It should be obvious to those skilled in the art that the amino acid insertion segments and fragments under consideration are operatively linked and form a single polypeptide chain.

Domain structure of a protein can be determined using any suitable software known in the art. For example, a Simple Modular Architecture Research Tool (SMART) software available in Internet at http://smart.embl-heidelberg.de can be used for this purpose [Schultz et al., PNAS 1998; 95: 5857-5864; Letunic I, Doerks T, Bork P, Nucleic Acids Res 2014; doi:10.1093/nar/gku949].

Term "operatively linked" or the like in description of fusion proteins refers to polypeptide sequences that occur in a physical and functional relationship with one another. In most preferred embodiments, functions of polypeptide components of the chimeric molecule are not altered as compared with functional properties of the isolated polypeptide components. For example, the hispidin hydroxylase of the present invention can be operatively linked to a fusion partner of interest, e.g. luciferase. In this case, the fusion protein retains the properties of hispidin hydroxylase while the polypeptide of interest retains its original biological activity, for example, the ability to oxidize luciferin with light emission. In some embodiments of the present invention, activities of the fusion partners may be reduced compared with activities of the isolated proteins. Such fusion proteins also find application within the scope of the present invention.

Term "operatively linked" or the like in description of nucleic acids means that the nucleic acids are covalently linked in such a way that there are no reading frame malfunctions or stop signs at their junctions. As it is obvious to any person skilled in the art, nucleotide sequences encoding a fusion protein with the "operatively linked" components (proteins, polypeptides, linker sequences, amino acid insertion segments, protein domains, etc.) are composed of fragments encoding said components, these fragments being covalently linked in such a way that a full-length fusion protein is produced during transcription and translation of the nucleotide sequence.

Term "operatively linked" in description of a nucleic acid relationship with regulatory coding sequences (promoters, enhancers, transcription terminators) means that the sequences are located and linked in such a way that the regulatory sequence will affect the expression level of the coding nucleic acid or nucleic acid sequence.

In the context of the present invention, "linking" of nucleic acids means that two or more nucleic acids are linked together using any means known in the art. As a non-limiting example, nucleic acids can be linked together using DNA ligase or polymerase chain reaction (PCR) during annealing. Nucleic acids can also be linked by chemical synthesis of a nucleic acid using a sequence of two or more separate nucleic acids.

Terms "regulatory elements" or "regulatory sequences" refer to the sequences involved in a coding nucleic acid expression regulation. Regulatory elements include promoters, termination signals, and other sequences that affect the expression of a nucleic acid. They typically also comprise the sequences required for proper translation of the nucleotide sequence.

Term "promoter" is used to describe an untranslated and non-transcribed DNA sequence upstream of the coding region that contains a RNA polymerase binding site as well as transcription initiating DNA binding site. Promoter region can also comprise another gene expression regulating elements.

Term "functional", as used here, refers to a nucleotide or amino acid sequence that can play a role in a particular test or task. Term "functional", if used to describe luciferases, means that the protein has the ability to produce the reaction of luciferin oxidation accompanied by luminescence. The same term "functional", if used to describe hispidin hydroxylases, means that the protein has the ability to catalyze reaction of converting at least one of the preluciferins shown in Table 2 to the corresponding luciferin. The same term "functional", if used to describe hispidin synthases, means that the protein has the ability to catalyze reaction of converting at least one of precursors of preluciferin to preluciferin, for example, converting caffeic acid to hispidin. The same term "functional", if used to describe caffeylpyruvate hydrolases, means that the protein has the ability to catalyze reaction of converting at least one of oxyluciferins to precursor of preluciferin (for example, converting caffeylpyruvate to caffeic acid).

Term "enzymatic properties", as used here, refers to the ability of a protein to catalyze a given chemical reaction.

Term "biochemical properties", as used here, refers to protein folding and comprises maturation rate, half-life, catalysis rate, pH and temperature stability, and other similar properties.

Term "spectral properties", as used here, refers to spectra, quantum yield, luminescence intensity, and other similar properties.

Reference to a nucleotide sequence "encoding" a polypeptide means that the polypeptide is produced during mRNA transcription and translation in accordance with this nucleotide sequence. At that, both the coding strand, identical to the mRNA and generally used in the sequence listing, and the complementary strand, which is used as a template for transcription, can be indicated. As it is obvious to any person skilled in the art, this term also covers any degenerate nucleotide sequences encoding the same amino acid sequence. Nucleotide sequences encoding a polypeptide comprise sequences containing introns.

Terms "expression cassette" or "cassette of expression" are used herein in sense of a nucleic acid sequence capable to regulate expression of a particular nucleotide sequence in an appropriate host cell. As a rule, the "expression cassette" contains a heterologous nucleic acid encoding a protein or a functional fragment thereof operatively linked to a promoter and termination signals. Typically, it also contains sequences required for proper translation of a significant nucleotide sequence. The expression cassette may be one that occurs in nature (including host cells), but has been produced in a recombinant form useful for expression of the heterologous nucleic acid. However, in many cases, the "expression cassette" is heterologous with respect to the host, i.e. particular nucleic acid sequence of this expression cassette does not occur naturally in the host cell and must be introduced into the host cell or into progenitor of the host cell by means of transformation. Expression of the nucleotide sequence can be regulated by a constitutive promoter or an inducible promoter that initiates transcription only when the host cell is open to a specific external stimulus. In the case of a multicellular organism, the promoter may also have specificity to a particular tissue, or organ, or developmental stage.

"Heterologous" or "exogenous" nucleic acid means a nucleic acid never occurring in a wild-type host cell.

Term "endogenous" refers to a native protein or nucleic acid in its natural position within genome of the organism.

Term "specifically hybridizes", as used herein, refers to an association between two single-stranded nucleic acid molecules or sufficiently complementary sequences such as to permit the hybridization under predetermined conditions commonly used in the art (sometimes the term "substantially complementary" is used).

An "isolated" nucleic acid moiety or isolated protein is a nucleic acid moiety or protein occurring separately from its natural environment due to human activities and therefore is not a product of nature. An isolated nucleic acid molecule or an isolated protein can occur in a purified form or in an unnatural environment such as, for example (which is not meant to be limited), a recombinant prokaryotic cell, plant cell, animal cell, non-bioluminescent fungus cell, transgenic organism (fungus, plant, animal), etc.

"Transformation" is the process for introducing a heterologous nucleic acid into a host cell or organism. In particular, "transformation" means a stable integration of DNA moiety into genome of a target organism of interest.

Term "transformed / transgenic / recombinant" refers to a host organism such as bacterium, plant, fungus, or animal, which was modified by introducing a heterologous nucleic acid moiety. This nucleic acid moiety may be either stably integrated into the host genome, or occur as an extrachromosomal moiety. Such an extrachromosomal moiety may be capable of self-replication. It should be understood that transgenic or stably transformed cells, tissues or organisms include both end products of the transformation process, but also transgenic progeny. Terms "non-transformed," "non-transgenic," "non-recombinant," or "wild-type" refer to a natural host organism or host cell, for example, a bacterium or plant, that contain no heterologous nucleic acid moieties.

Term "autonomously luminous" or "autonomously bioluminescent" refers to transgenic organisms or host cells that are capable of bioluminescence without exogenous addition of luciferins, preluciferins, or precursors of preluciferins.

Term "4'-phosphopantotheinyl transferase" is used herein to mean an enzyme that transfers 4-phosphopantotheinyl from coenzyme A to serine in the acyl transfer domain of polyketide synthase. 4'-phosphopantotheinyl transferases are naturally expressed by many plants and fungi and are known in the art [Gao Menghao et al., Microbial Cell Factories 2013, 12:77]. It will be obvious to those skilled in the art that any functional variant of 4'-phosphopantotheinyl transferase can be used for purposes of the present invention. For example, the NpgA 4'-phosphopantotheinyl transferase of *Aspergillus nidulans* (SEQ ID NOs 104, 105) described in [Gao Menghao et al., Microbial Cell Factories 2013, 12:77], or a homologue or mutant thereof, i.e. a protein with amino acid sequence substantially similar or identical to the sequence having SEQ ID NO 105. Another example is a 4'-phosphopantotheinyl transferase having at least 40% identity, including at least 50% identity, or at least 55% identity, or at least 60% identity, or at least 62% identity, or at least 65% identity, or at least 70% identity, or at least 75% identity, for example, at least 80% identity, or at least 85% identity, or at least 90% identity (for example, 90%, 91 %, 92%, 93%, 94%, 95%, 96%, 97%, 98%, 98% or 99% identity) with the sequence characterized by SEQ ID NO 105.

Nucleotides are designated according to their bases using the following standard abbreviations: adenine (A), cytosine (C), thymine (T) and guanine (G). Similarly, amino acids are designated by the following standard abbreviations: alanine (Ala; A), arginine (Arg; R), asparagine (Asn; N), aspartic acid (Asp; D), cysteine (Cys; C), glutamine (Gln; Q), glutamic acid (Glu; E), glycine (Gly; G), histidine (His; H), isoleucine (He; 1), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V).

Present invention is aimed to identification of new fungal luciferin biosynthesis enzymes, nucleic acids able to encode these enzymes, and proteins able to catalyze certain stages of the fungal luciferin biosynthesis. The invention also provides for application of nucleic acids for producing said enzymes in a cell or organism. Methods for *in vitro* or *in vivo* preparation of chemical compounds identical to fungal luciferins and preluciferins are also provided. Vectors comprising nucleic acid described in the present invention are also provided. In addition, the present invention provides expression cassettes comprising the nucleic acid of the present invention and regulatory elements necessary for nucleic acid expression in a selected host cell. Besides, cells, stable cell lines, transgenic organisms (e.g. plants, animals, fungi, or microorganisms) including nucleic acids, vectors, or expression cassettes of the present invention are also provided. Present invention also provides combinations of nucleic acids to obtain autonomously luminous cells, cell lines, or transgenic organisms. In preferred embodiments, cells or transgenic organisms are capable to produce fungal luciferin from precursors. In some embodiments, cells or transgenic organisms are capable to produce fungal preluciferin from precursors. In some embodiments, cells or transgenic organisms are capable of bioluminescence in the presence of a fungal luciferin precursor. In some embodiments, cells or transgenic organisms are capable of autonomous bioluminescence. Combinations of proteins for producing luciferin or its precursors from more simple chemical compounds are also provided. The present invention also provides a kit containing nucleic acids, vectors, or expression cassettes of the present invention for producing luminous cells, cell lines, or transgenic organisms.

### Proteins

As previously stated, this invention provides for proteins involved in fungal luciferin biosynthesis (cyclic system of transformations) as enzymes.

Proteins of this invention could be obtained from natural sources or by means of recombinant technologies. For example, wild-type proteins could be isolated from bioluminescent fungi, e.g. fungi of Basidiomycota type, predominantly of Basidiomycetes class, in particular, Agaricales order. For example, wild-type proteins could be isolated from such fungi as Neonothopanus nambi, Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos, etc. Proteins of this invention could also be obtained by expression of recombinant nucleic acid, coding protein sequence in respective host or in cell-free expression system, as described in the "Nucleic Acids" section. In some embodiments proteins are used inside host cells, in which nucleic acids capable of expression are introduced to code the said proteins.

In preferred embodiments the claimed proteins are quickly folded after expression in a host cell. "Quick folding" is understood to be the fact that proteins reach their tertiary structure which ensures their enzymic property over a short period of time. In these embodiments, proteins are folded within the period of time which generally does not exceed approximately 3 days, normally does not exceed approximately 2 days and prevalently does not exceed approximately 12-24 hours.

In some embodiments, proteins are used in isolated form. Any common techniques, where suitable methods of protein purification are described in the Guide to Protein Purification (Deuthser ed., Academic Press, 1990), could be used for protein purification. For example, lysate could be prepared from the initial source and purified using HPLC, displacement chromatography, gel electrophoresis, affinity chromatography, etc.

If proteins of the invention are in isolated form, it means that this protein is substantially free from other proteins or other natural biological molecules, such as oligosaccharides, nucleic acids and their fragments, etc., where the term "substantially free from" in this case means that less than 70%, normally less than 60% and prevalently less than 50% of the said composition, comprising the isolated protein, is the other natural biological molecule. In some embodiments the said proteins are substantially in purified form, where the term "substantially purified form" means purity equal at least 95%, normally equal at least 97% and prevalently equal at least 99%.

Proteins of the invention retain activity at temperatures below 50°C, prevalently at temperatures maximum 45°C, i.e. they retain activity at temperatures 20-42°C and could be used in heterologous expression systems *in vitro* and *in vivo.*

The claimed proteins have pH stability within the range from 4 to 10, prevalently within the range from 6.5 to 9.5. Optimum pH stability of the claimed proteins is within the range from 6.8 to 8.5, e.g. between 7.3-8.3.

The claimed proteins are active in physiological conditions. The term "physiological conditions" in this invention is intended to refer to a medium having the temperature within the range from 20 to 42°C, pH within the range from 6.8 to 8.5, saline and osmolarity of 300-400 mOsm/l. In particular, the term "physiological conditions" includes intracellular medium, cell-free preparation and liquids extracted from living organisms, such as blood plasma. "Physiological conditions" could be created artificially. For example, reaction mixtures, ensuring "physiological conditions", could be created by combining known chemical compounds. Methods of such media creation are well known from the prior art. Non-limiting examples include:

### 1) Ringer's solution isotonic to mammal blood plasma.

Ringer's solution consists of 6.5 g of NaCl, 0.42 g of KCI and 0.25 g of CaCl₂, dissolved in 1 litre of double-distilled water. When preparing the solution, the salts are added sequentially, each subsequent salt is added only after dissolving the previous one. In order to prevent from calcium carbonate sedimentation, it is recommended to pass carbon dioxide through sodium bicarbonate solution. The solution is prepared with fresh distilled water.

### 2) Versene solution

The Versene solution is a mixture of EDTA and inorganic salts dissolved in distilled water or in water for injection sterilized by membrane filtration using filters with final pore size of 0.22 µm. 1 l of Versene solution comprises 8.0 g of NaCl, 0.2 g of KCI, 1.45 g of disodium phosphate dodecahydrate, 0.2 g of potassium dihydrogen phosphate, 0.2 g of palkelate, double-distilled water - up to 1 l. Versene solution buffer capacity should be minimum 1.4 ml. Chloride ion content - from 4.4 to 5.4 g/l, EDTA - minimum 0.6 mmol/l.

### 3) phosphate-buffered saline (PBS, Na-phosphate buffer)

Na-phosphate buffer consists of 137 mM of NaCl, 10 mM of Na₂HPO₄, 1.76 mM of KH₂PO₄. The buffer could also contain KCI at concentration of up to 2.7 mM. The following is used to prepare 1 litre of normal strength Na-phosphate buffer: 8.00 g of NaCl, 1.44 g of Na2HPO4, 0.24 g of KH2PO4, 0.20 g of KCI (optionally). Dissolving in 800 ml of distilled water. The required pH is adjusted using hydrochloric acid or sodium hydroxide. Then distilled water is added to a total volume of 1 liter.

Specific proteins of interest are enzymes involved in cyclic fungal luciferin biosynthesis, their mutants, homologs and derivatives. Each of these specific types of polypeptide structures of interest will be further individually analyzed in more details.

### Hispidin-hydroxylases

Hispidin-hydroxylases of this invention are proteins able to catalyze luciferin synthesis from preluciferin. In other words, these are enzymes catalyzing reaction of transformation of 6-(2-arylvinyl)-4-hydroxy-2H-pyran-2-one, having the structural formula 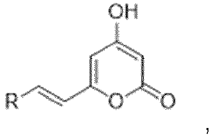 into 6-(2-arylvinyl)-3,4-dihydroxy-2H-pyran-2-one, having the structural formula 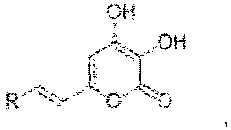 where R - aryl or heteroaryl.

The reaction is carried out in physiological conditions *in vitro* and *in vivo* in the presence of at least one molecule of NAD(P)H and at least of one molecule of molecular oxygen (O₂) per one molecule of 6-(2-arylvinyl)-4-hydroxy-2H-pyran-2-one:

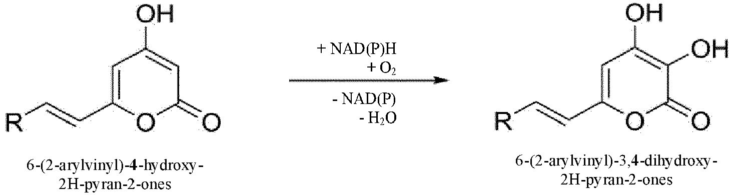

Hispidin-hydroxylases of interest include proteins from bioluminescent fungi *Neonothopanus nambi, Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos,* which amino acid sequences are shown in SEQ ID NOs: 2, 4, 6, 8 10, 12, 14, 16, 18, 20, 22, 24, 26, 28, and also their functional mutants, homologs and derivatives.

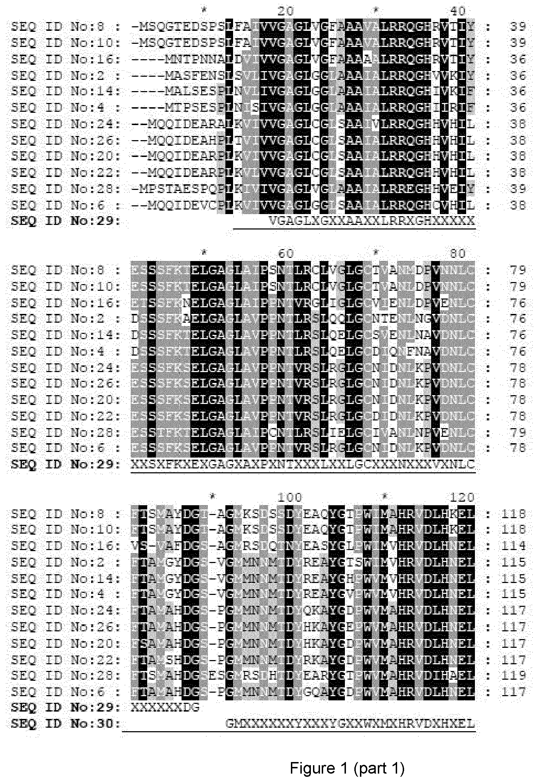

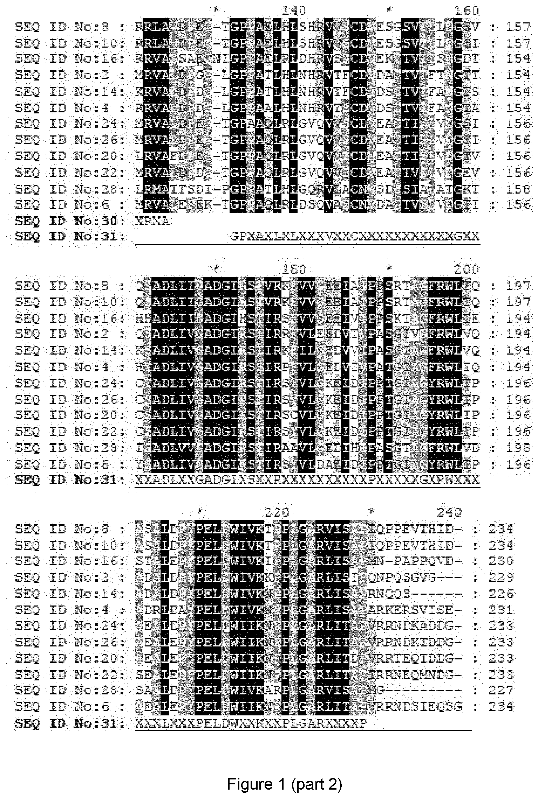

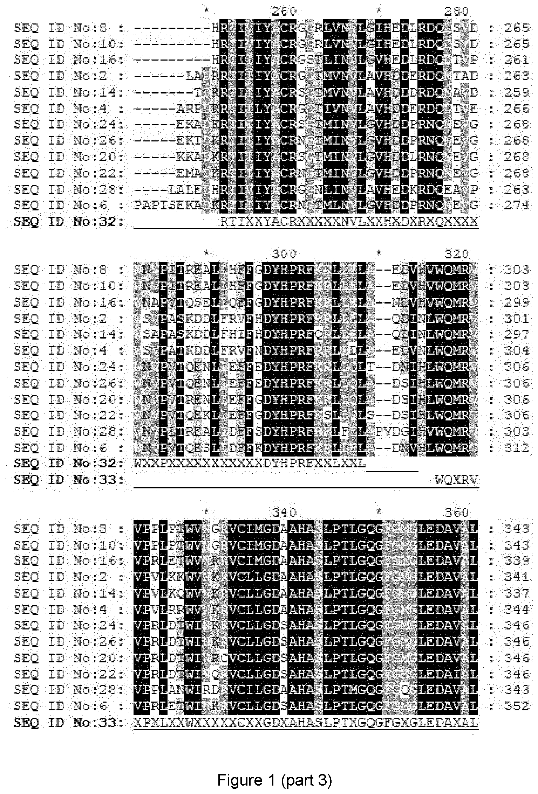

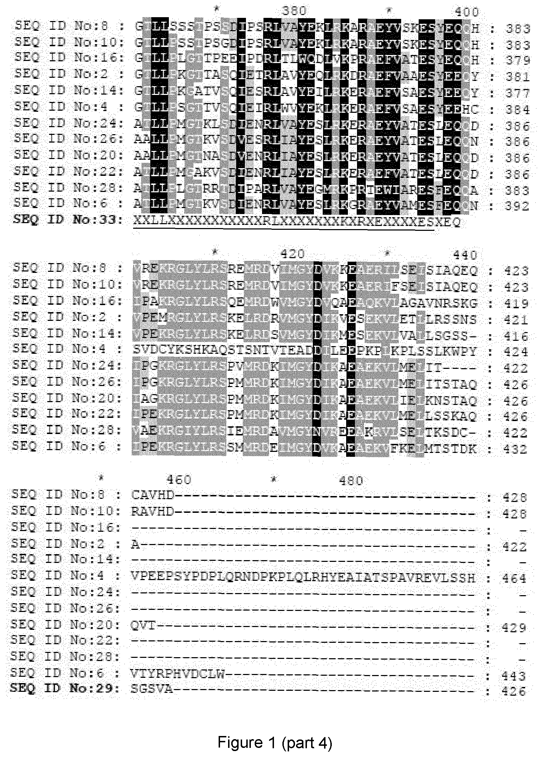

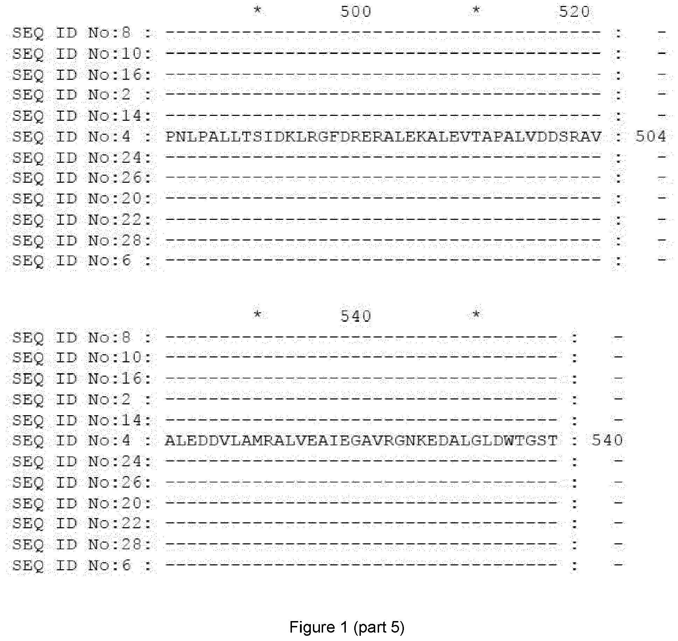

In preferred embodiments hispidin hydroxylases of this invention are characterized by presence of FAD/NAD(P) binding domain, IPR002938 - code of InterPro public database available on the Internet at the website http://www.ebi.ac.uk/interpro). The said domain is involved in binding flavine adenine dinucleotide (FAD) and nicotinamide adenine dinucleotide (NAD) in multiple enzymes, adding the hydroxyl group to substrate, and multiple organisms found in metabolic pathways. Hispidin-hydroxylases of this invention comprise the said domain with the length of 350-385 amino acids, prevalently 360-380 amino acids, e.g. 364-377 amino acids, floxed N- and C-terminal non-conservative amino acid sequences having lower percentage of identity with each other. Position of FAD/NAD binding domain in the claimed hispidin hydroxylases is illustrated at multiple alignment of individual protein amino acid sequences in Fig. 1.

Hispidin-hydroxylase homologs or mutants are also provided, which sequence differs from the above mentioned specific amino acid sequences claimed in the invention, i.e. SEQ ID NO: 2, 4, 6, 8 10, 12, 14, 16, 18, 20, 22, 24, 26, 28. Homologs or mutants of interest have at least minimum 40% of identity, e.g. minimum 45% of identity, or minimum 50% of identity, or minimum 55% of identity, or minimum 60% of identity, or minimum 65% of identity, or minimum 70% of identity, or minimum 75% of identity, e.g. minimum 80% of identity, minimum 85% of identity, minimum 90% of identity (e.g. at least 90%, 91%, 92%, 93%, 94%, 95%, 96%, 97%, 98%, 98% or 99% of identity) with protein, which amino acid sequence is selected from the group of SEQ ID NOs: 2, 4, 6, 8 10, 12, 14, 16, 18, 20, 22, 24, 26, 28, for at least 350 amino acids. Particularly it relates to amino acid sequences which provide for protein functional sites, i.e. to the sequence of FAD/NAD binding domain being the part of hispidin hydroxylases.

In preferred embodiments hispidin hydroxylase amino acid sequence of this invention is characterized by presence of several conservative amino acid motifs (consensus sequences) typical of this enzyme group only. These consensus sequences are shown in SEQ ID NOs: 29-33. Consensus sites inside hispidin hydroxylase amino acid sequences are operatively bound via amino acid inserts with lower insertions.

### Hispidin-synthases

Hispidin-synthases of this invention are proteins able to catalyze preluciferin synthesis from its precursors. In other words, these are enzymes catalyzing reaction of transformation of 3-arylacrylic acid with the structural formula 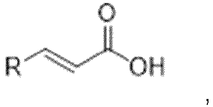 where R - aryl or heteroaryl in 6-2-arylvinyl)-4-hydroxy-2H-pyran-2-one, having the structural formula 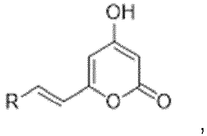 where R - aryl or heteroaryl.

Examples of 3-arylacrylic acids being the precursors of preluciferins are given in Table 2.

The reaction is carried out in physiological conditions in vitro and in vivo in the presence of at least one molecule of coenzyme A, at least on molecule of ATP and at least two molecules of malonyl-CoA:

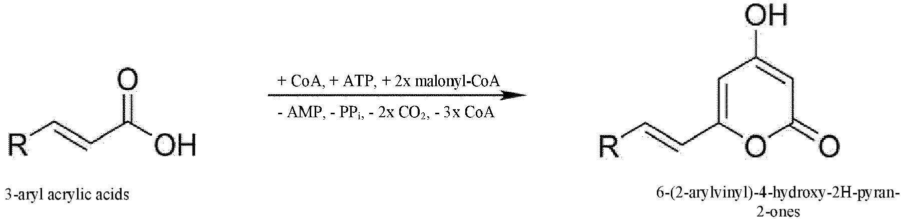

Hispidin-synthases of interest include proteins from bioluminescent fungi *Neonothopanus nambi, Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos,* which amino acid sequences are shown in SEQ ID NOs: 35, 37, 39, 41, 43, 45, 47, 49, 51, 53, 55, and also their functional mutants, homologs and derivatives.

In preferred embodiments hispidin-synthase amino acid sequence of this invention is characterized by presence of several conservative amino acid motifs (consensus sequences) typical of this enzyme group only. These consensus sequences are shown in SEQ ID NOs: 56-63. Consensus sites inside hispidin-synthase amino acid sequences are operatively bound via amino acid inserts with lower insertions.

In many embodiments of this invention the relevant amino acid sequences of homologs and mutants of specific hispidin-synthases are characterized by substantial identity with sequences shown in SEQ ID NOs: 35, 37, 39, 41, 43, 45, 47, 49, 51, 53, 55, which is, for example, at least minimum 40% of identity, e.g. minimum 45% of identity, or minimum 50% of identity, or minimum 55% of identity, or minimum 60% of identity, or minimum 65% of identity, or minimum 70% of identity, or minimum 75% of identity, e.g. minimum 80% of identity, minimum 85% of identity, minimum 90% of identity (e.g. at least 90%, 91 %, 92%, 93%, 94%, 95%, 96%, 97%, 98%, 98% or 99% of identity) for all protein amino acid sequence.

In preferred embodiments hispidin-synthases of this invention are polydomain proteins related to polyketide synthase superfamily. In preferred embodiments hispidin-synthases of this invention are subjected to post-translation modification, namely, transfer of 4-phosphopantetheinyl from coenzyme A to serine in acyl carrier domain of polyketide synthase is required for their maturation. Enzymes - 4'-phosphopantetheinyl transferases performing such modification are known from the prior art [Gao Menghao et al., Microbial Cell Factories 2013, 12:77]. 4'-phosphopantetheinyl transferases are expressed in nature by many plants and fungi, in which cells the functional hispidin-synthase of this invention maturates without introduction of additional enzymes or nucleic acids coding them. At the same time introduction of 4'-phosphopantetheinyl transferase coding sequence into host cells is required for maturation of hispidin-synthase in cells of some lower fungi (e.g. yeast) and animals. It is obvious to those skilled in the art that any functional variant of 4'-phosphopantetheinyl transferase, known from the prior art, could be used for the purposes of this invention. For example, there could be used 4'-phosphopantetheinyl transferase NpgA from *Aspergillus nidulans* (SEQ ID NO 104, 105), described in [Gao Menghao et al., Microbial Cell Factories 2013, 12:77], any it's homolog or mutant with confirmed activity.

### Caffeoyl pyruvate hydrolases

Caffeoyl pyruvate hydrolases of this invention are proteins able to catalyze transformation of oxyluciferin, which is 6-aryl-2-hydroxy-4-oxohexa-2,5-diene acid having the structural formula 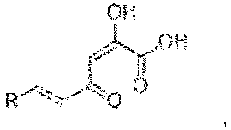 where R is aryl or heteroaryl, conversion into 3-arylacrylic acid with the structural formula 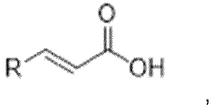 where R - aryl or heteroaryl.

Examples of oxyluciferins are given in Table 2.

The reaction is carried out in physiological conditions in vitro and *in vivo*:

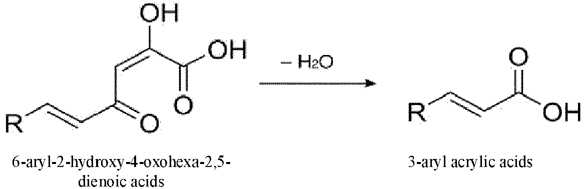

In preferred embodiments caffeylpyruvate hydrolases of this invention transform caffeylpyruvate into caffeic acid. In preferred embodiments they transform oxyluciferin shown in Table 2 into preluciferin precursor.

Caffeoyl pyruvate hydrolases of interest include proteins from bioluminescent fungi Neonothopanus nambi, Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos, which amino acid sequences are shown in SEQ ID NOs: 65, 67, 69, 71, 73, 75, and also their functional mutants, homologs and derivatives.

In preferred embodiments caffeylpyruvate hydrolase amino acid sequence of this invention (including homologs and mutants of interest) is characterized by presence of several conservative amino acid motifs (consensus sequences) typical of this enzyme group only. These consensus sequences are shown in SEQ ID NOs: 76-78. Consensus sites inside caffeylpyruvate hydrolase amino acid sequences are operatively bound via amino acid inserts with lower insertions.

In many embodiments of this invention the relevant amino acid sequences of caffeylpyruvate hydrolase are characterized by substantial identity with sequences shown in SEQ ID NOs: 65, 67, 69, 71, 73, 75, which is, for example, at least minimum 40% of identity, e.g. minimum 45% of identity, or minimum 50% of identity, or minimum 55% of identity, or minimum 60% of identity, or minimum 65% of identity, or minimum 70% of identity, or minimum 75% of identity, e.g. minimum 80% of identity, minimum 85% of identity, minimum 90% of identity (e.g. at least 90%, 91%, 92%, 93%, 94%, 95%, 96%, 97%, 98%, 98% or 99% of identity) for all protein amino acid sequence.

Homologs of the above-described specific proteins (i.e proteins with amino acid sequences SEQ ID NO: 2, 4, 6, 8 10, 12, 14, 16, 18, 20, 22, 24, 26, 28, 35, 37, 39, 41, 43, 45,47,49, 51, 53, 55, 65, 67, 69, 71, 73, 75) could be isolated from natural sources. Homologs could be found in many organisms (fungi, plants, microorganisms, animals). In particular, homologs could be found in different kinds of bioluminescent fungi, e.g. fungi of Basidiomycota type, predominantly of Basidiomycetes class, in particular, Agaricales order. Also, non-bioluminescent fungi and plants producing hispidin, such as *Pteris ensiformis,* are of special interest as a source of protein homologs of this invention [Yung-Husan Chen et al., «Identification of phenolic antioxidants from Sword Brake fern (Pteris ensiformis Burm.)», Food Chemistry, Volume 105, Issue 1, 2007, pp. 48-56], *Inonotus xeranticus* [In-Kyoung Lee et al., «Hispidin Derivatives from the Mushroom Inonotus xeranticus and Their Antioxidant Activity», J. Nat. Prod., 2006, 69 (2), pp. 299-301], *Phellinus sp.* [In-Kyoung Lee et al., «Highly oxygenated and unsaturated metabolites providing a diversity of hispidin class antioxidants in the medicinal mushrooms Inonotus and Phellinus». Bioorganic & Medicinal Chemistry. 15 (10): 3309-14.], *Equisetum arvense* [Markus Herderich et al., «Establishing styrylpyrone synthase activity in cell free extracts obtained from gametophytes of Equisetum arvense L. by high performance liquid chromatography-tandem mass spectrometry». Phytochem. Anal., 8: 194-197.].

Proteins which are derivatives or mutants of the above-described proteins naturally occurring are also provided. Mutants and derivatives can retain biological properties of wild-type proteins (e.g. naturally occurring) or can have biological properties different from wild-type proteins. Mutations include replacements of one or more amino acids, deletion or insertion of one or more amino acids, N-terminal replacements or truncations, or extensions, C-terminal replacements or truncations, or extensions, etc. Mutants and derivatives can be obtained using standard methods of molecular biology, as described in details in the "Nucleic Acids" section. Mutants are substantially identical to wild-type proteins, i.e. have at least 40% of identity with them inside the region selected for comparison. Therefore, substantially similar sequences include those which have, for example, at least 40% of identity, or at least 50% of identity, or at least 55% of identity, or at least 60% of identity, or at least 62% of identity, or at least 65% of identity, or at least 70% of identity, or at least 75% of identity, for example, at least 80% of identity, or at least 85% of identity, or at least 90% of identity (e.g. 90%, 91%, 92%, 93%, 94%, 95%, 96%, 97%, 98%, 98% or 99% of identity) inside the region selected for comparison. In many embodiments homologs of interest have far higher identity of sequence, for example, 70%, 75%, 80%, 85%, 90% (e.g. 92%, 93%, 94%) or higher, e.g. 95%, 96%, 97%, 98%, 99%, 99.5%, especially for a sequence of amino acids, which provide protein functional regions.

Derivatives can also be obtained using standard methods and include changing by means of RNA, chemical modifications, modifications after translation and after transcription, etc. For example, derivatives could be obtained by such methods as modified phosphorylation or glycosylation, or acetylation, or lipidation, or by different types of segregation at maturation, etc.

Methods well known to those skilled in the art are used for searching functional mutants, homologs and derivatives. For example, functional screening of the expression library comprising variants (e.g. protein mutant forms or homologous proteins, or protein derivatives). Expression library is obtained by cloning of nucleic acids coding the tested variants of proteins into expression vector and their entry into appropriate host cells. Methods of operation with nucleic acids are described in detail in the "Nucleic Acids" section. In order to identify functional enzymes of this invention an appropriate substrate is added to the cells expressing the nucleic acids being tested. Formation of the expected product of reaction catalyzed by functional enzyme could be detected by HPLC methods using synthetic variants of the expected reaction products as standards. For example, hispidin or other preluciferin, shown in Table 2, can be used as a substrate to identify functional hispidin hydroxylases. The expected reaction product is fungal luciferin. Preluciferin precursor (e.g. caffeic acid) can be used as a substrate to identify hispidin-synthases, and the corresponding fungi preluciferin is the reaction product. It should be noted that host cells shall express 4'-phosphopantetheinyl transferase, promoting protein post-translational modification, for screening functional hispidin-synthases.

Oxyluciferin (Table 2) is used as a substrate for searching functional caffeylpyruvate hydrolases, and the tested reaction product is a preluciferin precursor - 3-arylacrylic acid.

In many embodiments of this invention the bioluminescent reaction can be used for searching functional enzymes of this invention. In this case, for the purpose of expression library preparation the cells producing luciferase able to oxidize fungal luciferin with luminescence emission, and functional enzymes promoting production of fungal luciferin from a product of enzymatic reaction performed by test protein.

Thus, host cells producing functional luciferase, which substrate is fungal luciferin, are used for screening functional hispidin hydroxylases. When adding preluciferin to the cells comprising functional variant of hispidin hydroxylase, fungal luciferin is formed, and luminescence appears due to fungal luciferin oxidation with luciferase.

Host cells additionally producing functional luciferase, which substrate is fungal luciferin, and functional hispidin hydroxylase, are used for screening functional hispidin synthases. When adding preluciferin precursor to such cells, fungal luciferin is formed, and luminescence appears due to fungal luciferin oxidation with luciferase.

Host cells producing functional luciferase, which substrate is fungal luciferin, functional hispidin hydroxylase and functional hispidin synthase, are used for screening functional caffeylpyruvate hydrolases. When adding oxyluciferin to such cells, fungal luciferin is formed, and luminescence appears due to fungal luciferin oxidation with luciferase.

Any luciferases able to oxidize luciferin with luminescence emission, selected from the group of 6-(2-arylvinyl)-3,4-dihydroxy-2H-pyran-2-ones having the common formula 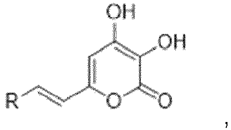 where R - aryl or heteroaryl, can be used for screening. Non-limiting examples of luciferins are given in Table 1. Non-limiting examples of suitable luciferases are described in the section "Application, combinations and methods of use" below.

Luciferin oxidation with luciferase is accompanied with emission of luminescence detected. Light emitted during the oxidation can be detected by standard methods (for example, visual observation, observation by means of night vision devices, spectrophotometry, spectrofluorimetry, using of image photographic recording, using of special equipment for detection of luminescence and fluorescence, such as, e.g. IVIS Spectrum In Vivo Imaging System (Perkin Elmer), etc.). Recorded luminescence could be emitted within intensity range from one photon to luminescence easily perceptible to the eye, e.g. with intensity of 1 cd and bright luminescence with intensity, e.g. 100 cd and more. Light emitted at oxidation of 3-hydroxy hispidin is within the range from 400 to 700 nm, prevalently within the range from 450 to 650 nm, with emission maximum at 520-590 nm. Light emitted at oxidation of other 6-(2-arylvinyl)-3,4-dihydroxy-2H-pyran-2-ones could have emission maximum shift (Table 3).

**Table 3. Emission maximums for a series of fungi**

| **Substance** | **Emission maximum, nm** |
|---|---|
| 3-hydroxy hispidin | 538 |
| (E)-3,4-dihydroxy-6-(4-hydroxystyryl)-2H-pyran-2-one | 520 |
| (E)-6-(2-(1H-indol-3-yl)vinyl)-3,4-dihydroxy-2H-pyran-2-one, | 480 |
| (E)-6-(4-(diethylamino)styryl)-3,4-dihydroxy-2H-pyran-2-one, | 504 |
| (E)-3,4-dihydroxy-6-(2-(2,3,6,7-tetrahydro-1H,5H-pyrido[3,2,1-ij]quinolin-9-yl)vinyl)-2H-pyran-2-one, | 534 |
| (E)-3,4-dihydroxy-6-(2-(6-hydroxynaphthalen-2-yl)vinyl)-2H-pyran-2-one | 564 |

Examples of functional screening using bioluminescence are described in the experimental part below.

The invention also covers fusion proteins, including protein of this invention. Its homolog. mutant, including shortened or elongated form. The protein of the invention could be operatively fused with intracellular localization signal (e.g. nuclear localization signal, localization signal in mitochondria, or in peroxisomes, or in lysosomes, or in Goldgi apparatus, or in other cell organelles), signal peptide promoting protein isolation into intercellular space, transmembrane domain or with any protein or polypeptide (fusion partner) of interest. Fusion proteins could include operatively cross-linked, e.g. hispidin hydroxylase and/or hispidin synthase, and/or caffeylpyruvate hydrolase, claimed in the invention, with fusion partner linked to C- or N-terminal. Non-limiting examples of fusion partners could include proteins of this invention having other enzymic function, antibodies or their linking fragments, ligands or receptors, luciferases able to us fungi luciferins as substrates in bioluminescent reaction. In some embodiments a fusion partner and protein of the invention are operatively cross-linked via linking sequence (peptide linker) promoting independent fusion protein folding and functioning. Methods of fusion proteins production are well known to those skilled in the art.

In some embodiments fusion proteins include hispidin hydroxylase of the invention and luciferase able to oxidize fungal luciferin with luminescence emission, which are operatively cross-linked via short peptide linker. Such fusion protein can be used for obtaining bioluminescence *in vitro* and *in vivo* in the presence of a preluciferin (e.g. in the presence of hispidin). It is obvious to those skilled in the art that any functional hispidin hydroxylase described above could be used with any functional luciferase to produce a fusion protein. Specific examples of fusion proteins are described in the Experimental Part below. Examples of luciferases which could be used at producing fusion proteins are described in the section "Application, combinations and methods of use" below.

### Nucleic acids

This invention provides for nucleic acids coding enzymes of fungal luciferin biosynthesis, mutants and homologs of these proteins, including shortened and elongated forms.

Nucleic acid, as herein used, is an isolated DNA molecule, such as genomic DNA molecule or cDNA molecule, or RNA molecule, such as mRNA molecule. In particular, the said nucleic acids are cDNA molecules having open reading frame, which codes luciferin biosynthesis enzyme of the invention, and capable, under appropriate conditions, to ensure enzyme expression of the invention.

The term "cDNA" is for description of nucleic acids, which reflect arrangement of sequence elements located in native, mature mRNA, where sequence elements are exons and 5-' and 3'-noncoding regions. Immature mRNA could have exons separated by intervening introns, which, if present, are removed during post-translational RNA spicing to form mature mRNA having open reading frame.

Genomic sequence of interest could include nucleic acid present between initiating codon and terminating codon, as determined in the said sequences, including all introns, which are normally present in a native chromosome. Genomic sequence of interest could additionally include 5'- and 3'-untranslated regions in the mature mRNA, as well as specific transcrpitional and translational regulatory sequences, such as promoters, enhancers, etc., including flanking genomic DNA approximately 1 kbp in size, but possibly even more, at 5'- or 3'-terminal of the transcribed region.

The invention also covers nucleic acids, which are homologous, substantially similar, identical, derivatives or mimetics of nucleic acids coding proteins of this invention.

The claimed nucleic acids are present in the environment different from their natural medium, e.g. they are isolated, present in enriched quantities, or present or expressed in *vitro* or in a cell, or in an organism, other than their naturally occurring environment.

Specific nucleic acids of interest include nucleic acids, which code hispidin hydroxylase or hispidin synthase, or caffeylpyruvate hydrolase described in "Proteins" section above. Each of these specific nucleic acids of interest is individually disclosed in more details.

### Nucleic acids coding hispidin hydroxylases.

In preferred embodiments nucleic acids of the invention code proteins able to catalyze reaction of transformation of 6-(2-arylvinyl)-4-hydroxy-2H-pyran-2-one (preluciferin) having the structural formula 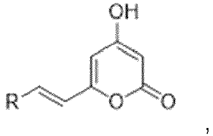 into 6-(2-arylvinyl)-3,4-dihydroxy-2H-pyran-2-one (fungal luciferin), having the structural formula

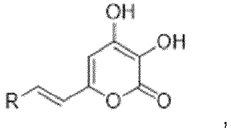

### where R - aryl or heteroaryl.

In preferred embodiments nucleic acids code hispidin hydroxylases, which amino acid sequences are characterized by presence of several conservative amino acid motifs (consensus sequences) shown in SEQ ID NO: 29-33.

Specific examples of nucleic acids include nucleic acids coding hispidin hydroxylases, which amino acid sequences are shown in SEQ ID NOs: 2, 4, 6, 8 10, 12, 14, 16, 18, 20, 22, 24, 26, 28. Examples of nucleic acids, coding the said proteins, are given in SEQ ID NOs: 1, 3,5, 7, 9, 11, 13, 15, 17, 19, 21, 23, 25, 27. Also, functional mutants, homologs and derivatives of the above mentioned specific nucleic acids are of interest.

In preferred embodiments nucleic acids of the invention code proteins, which amino acid sequences are at least 60%, or at least 65%, or at least 70%, or at least 80%, or at least 85%, or at least 90%, or at least 91 %, or at least 92%, or at least 93%, or at least 94%,or at least 95%, or at least 96%, or at least 97%, or at least 98%, or at least 99% identical to the sequences shown in SEQ ID NOs: 2, 4, 6, 8 10, 12, 14, 16, 18, 20, 22, 24, 26, 28, for at least 350 amino acids.

### Nucleic acids coding hispidin-synthases

In preferred embodiments nucleic acids of the invention code proteins able to catalyze reaction of transformation of 3-arylacrylic acid with the structural formula 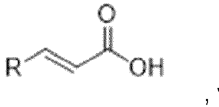 where R - aryl or heteroaryl in 6-2-arylvinyl)-4-hydroxy-2H-pyran-2-one, having the structural formula 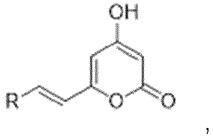 where R - aryl or heteroaryl.

In preferred embodiments nucleic acids code hispidin-synthases, which amino acid sequences are characterized by presence of several conservative amino acid motifs (consensus sequences) shown in SEQ ID NOs: 56-63.

Specific examples of nucleic acids include nucleic acids coding hispidin-synthases of the invention, which amino acid sequences are shown in SEQ ID NOs: 35, 37, 39, 41, 43, 45, 47, 49, 51, 53, 55. Examples of nucleic acids, coding the said proteins, are given in SEQ ID NOs: 34, 36,38,40,42, 44, 46, 48, 50, 52, 54.

Also, functional mutants, homologs and derivatives of the above mentioned specific nucleic acids are of interest.

In preferred embodiments nucleic acids of the invention code proteins, which amino acid sequences are at least 45%, normally at least 50%, e.g. at least 55%, or at least 60%, or at least 65%, or at least 70%, or at least 80%, or at least 85%, or at least 90%, or at least 91%,or at least 92%, or at least 93%, or at least 94%,or at least 95%, or at least 96%, or at least 97%, or at least 98%, or at least 99% identical to the sequences shown in SEQ ID NOs: 35, 37, 39, 41, 43, 45,47,49, 51, 53, 55, for all protein polypeptide chain.

### Nucleic acids coding caffeylpyruvate hydrolases

In preferred embodiments nucleic acids of the invention code proteins able to catalyze reaction of transformation of oxyluciferin with the structural formula 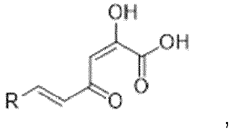 where R is aryl or heteroaryl, conversion into 3-arylacrylic acid with the structural formula 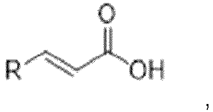 where R is selected from aryl, heteroaryl group.

In preferred embodiments nucleic acids code caffeylpyruvate hydrolases, which amino acid sequences are characterized by presence of several conservative amino acid motifs (consensus sequences) shown in SEQ ID NOs: 76-78. Specific examples of nucleic acids include nucleic acids coding caffeylpyruvate hydrolases, which amino acid sequences are shown in SEQ ID NOs: 65, 67, 69, 71, 73, 75. Examples of nucleic acids, coding the said proteins, are given in SEQ ID NOs: 64, 66, 68, 70,72, 74.

Also, nucleic acids, coding functional mutants, homologs and derivatives of the above-mentioned proteins, are of interest.

In preferred embodiments nucleic acids of the invention code proteins, which amino acid sequences are at least 60%, or at least 65%, or at least 70%, or at least 80%, or at least 85%, or at least 90%, or at least 91%,or at least 92%, or at least 93%, or at least 94%,or at least 95%, or at least 96%, or at least 97%, or at least 98%, or at least 99% identical to the sequences shown in SEQ ID NOs: 65, 67, 69, 71, 73, 75, for all protein polypeptide chain.

Nucleic acids of interest (for example, nucleic acids coding homologs of proteins characterized by amino acid sequences shown in SEQ ID NOs: 2, 4, 6, 8 10, 12, 14, 16, 18, 20, 22, 24, 26, 28, 35, 37, 39, 41, 43, 45,47,49, 51, 53,55, 65, 67, 69, 71, 73, 75), could be isolated from any organisms (fungi, plants, microorganisms, animals), in particular, from different kinds of bioluminescent fungi, e.g. fungi of Basidiomycota type, predominantly of Basidiomycetes class, in particular, Agaricales order, e.g. from bioluminescent fungi Neonothopanus nambi, Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos, etc. TAIso, non-bioluminescent fungi and plants producing hispidin, such as *Pteris ensiformis,* are of special interest as a source of nucleic acids coding homologs of proteins of this invention [Yung-Husan Chen et al., «Identification of phenolic antioxidants from Sword Brake fern (Pteris ensiformis Burm.)», Food Chemistry, Volume 105, Issue 1, 2007, pp. 48-56], *Inonotus xeranticus* [In-Kyoung Lee et al., «Hispidin Derivatives from the Mushroom Inonotus xeranticus and Their Antioxidant Activity», J. Nat. Prod., 2006, 69 (2), pp. 299-301], *Phellinus sp.* [In-Kyoung Lee et al., «Highly oxygenated and unsaturated metabolites providing a diversity of hispidin class antioxidants in the medicinal mushrooms Inonotus and Phellinus». Bioorganic & Medicinal Chemistry. 15 (10): 3309-14.], *Equisetum arvense* [Markus Herderich et al., «Establishing styrylpyrone synthase activity in cell free extracts obtained from gametophytes of Equisetum arvense L. by high performance liquid chromatography-tandem mass spectrometry». Phytochem. Anal., 8: 194-197.].

Homologs are identified by any of the variety of methods. cDNA fragment of this invention could be used as a hybridization probe versus cDNA library from the target organism, using low stringency conditions. The probe could be a large fragment or one or shorter degenerate primer. Nucleic acids, having sequence similarity, are detected by hybridization in low stringency conditions, for example, at 50°C and 6×SSC (0.9 M of sodium chloride /0.09 M of sodium citrate) followed by washing at 55×C in 1×SSC (01.15 M of sodium chloride/0.015 M of sodium citrate). Sequence identity could be determined by hybridization in high stringency conditions, for example, at 50°C or higher and 0.1 xSSC (15 mM of sodium chloride/ 1.5 mM of sodium citrate). Nucleic acids having the region substantially identical to the presented sequences, e.g. allelic variants, genetically modified variants of nucleic acid, etc., are bound with the presented sequences in high stringency conditions of hybridization. Using probes, in particular, labeled probes of DNA sequences, enables to recover homologous or similar genes.

Homologs could be identified by means of polymerase chain reaction from genomic or cDNA library. Oligonucleotide primers, representing the fragments of known sequences of specific nucleic acids, could be used as primers for PCR. In preferable aspect oligonucleotide primers have degenerate structure and correspond to nucleic acid fragments coding conservative regions of protein amino acid sequence, e.g. consensus sequences are shown in SEQ ID NOs: 29-33, 56-63, 76-78. Full-length coding sequences then could be detected by means of 3'- and 5'-RACE methods, well known from the prior art.

Homologs could also be identified in the results of whole-genome sequencing of organisms by comparison of amino acid sequences deduced on the basis of sequencing and amino acid sequences SEQ ID NOs: 2, 4, 6, 8 10, 12, 14, 16, 18, 20, 22, 24, 26, 28, 35, 37, 39, 41, 43, 45,47,49, 51, 53,55, 65, 67, 69, 71, 73, 75. Sequence identity is determined based on reference sequence. Algorithms for sequence analysis are known in the art, e.g. BLAST, described in Altschul et al., J. Mol. Biol., 215, pp. 403-10 (1990). For the purposes of this invention, in order to determine the level of identity and similarity between nucleotide sequences and amino acid sequences there could be used a comparison of nucleotide and amino acid sequences performed by means of Blast software package provided by National Center for Biotechnology Information (http://www.ncbi.nlm.nih.gov/blast) using nicked alignment with standard parameters.

Nucleic acids which are hybridized with the above nucleic acids in stringent conditions, preferably in high stringency conditions (i.e. complementary to nucleic acids described before) are also provided. Example of hybridization in high stringency conditions is hybridization at 50°C or higher and 0.1×SSC (15 mM of sodium chloride/ 1.5 mM of sodium citrate). Other example of hybridization in high stringency conditions is overnight incubation at 42°C in 50% formamide, 5xSSC (150 mM NaCl, 15 m M trisodium citrate), 50 mM sodium phosphate (pH 7.6), 5 x Denhardt's solution, 10% dextran sulphate and µg/ml of salmon sperm denatured cut DNA, with preliminarily washing in 0.1×SSC at approximately 65°C. Other high stringency conditions of hybridization are known in the art and also can be used for identification of nucleic acids of the invention.

Nucleic acids coding variants, mutants or derivatives of proteins of the invention are also provided. Mutants or derivatives could be obtained from nucleic acid template, selected from the above described nucleic acids, by modification, deletion or adding of one or more nucleotides in template sequence or their combination to obtain a variant of nucleic acid template. Modifications, additions or deletions could be performed by any method known in the art (see, for example, Gustin et al., Biotechniques (1993) 14: 22; Barany, Gene (1985) 37: 111-123; and Colicelli et al., Mol. Gen. Genet. (1985) 199:537-539, Sambrook et al., Molecular Cloning: A Laboratory Manual, (1989), CSH Press, pp.15.3-15.108), including error-prone PCR, shuffling, oligonucleotide-directed mutagenesis, assembly PCR, paired PCR mutagenesis, mutagenesis *in vivo,* cassette mutagenesis, recursive ensemble mutagenesis, exponential ensemble mutagenesis, oligonucleotide-directed mutagenesis, random mutagenesis, genetic reassembly, gene site saturation mutagenesis (GSSM), synthetic ligation reassembly (SLR) or their combinations. Modifications, additions or deletions could also be performed by method including recombination, recursive sequence recombination, phosphorothioate-modified DNA mutagenesis, uracil template mutagenesis, double-skip mutagenesis, point reducing mismatch mutagenesis, recovery deficient strain mutagenesis, chemical mutagenesis, radiation mutagenesis, deleted mutagenesis, restriction selective mutagenesis, restriction mutagenesis with purification, artificial gene synthesis, multiple mutagenesis, creation of chimeric multiple nucleic acids and their combinations. Nucleic acids coding shortened and elongated variants of the said luciferases are also under the scope of this invention. As used herein, these protein variants comprise amino acid sequences with modified C-, N-, or both terminals of polypeptide chain.

In preferred embodiments the homologs and mutants under discussion are functional enzymes able to perform fungal luciferin biosynthesis, e.g. fungi luciferin. Homologs and mutants of the interest could have altered properties, such as rate of maturation in a host cell, aggregability or dimerizability, half-life period or other biochemical properties, including substrate binding constant, thermal stability, pH stability, activity temperature optimum, activity pH optimum, Michaelis-Menten constant, substrate specificity, side issue range. In some embodiments homologs and mutants have the same properties as the claimed proteins.

Nucleic acids, coding functional homologs and mutants of this invention, could be identified during functional tests, for example, at expression library functional screening, described in "Proteins" section.

Besides, degenerate variants of nucleic acids, which code proteins of this invention, are also provided. Degenerate variants of nucleic acids include replacements of nucleic acid codons by other codons coding the same amino acids. In particular, the degenerate variants of nucleic acids are created to increase expression in a host cell. In this embodiment nucleic acid codons, which are not preferable or are less preferable in host cell genes, are replaced by codons which are excessively presented in the coding sequences in the host cell genes, where the said replaced codons code the same amino acid. In particular, humanized versions of nucleic acids of this invention are of the interest. As used herein, the term "humanized" refers to the replacements done in nucleic acid sequence to optimize codons for protein expression in mammal cells (Yang et al., Nucleic Acids Research (1996) 24: 4592-4593). See also US Pat. No. 5795737, describing protein humanization, which disclosure is incorporated herein by reference. Variants of nucleic acids optimized for expression in plant cells are of particular interest. Examples of such nucleic acids, coding proteins of this invention, are given in SEQ ID NOs: 103, 113 and 114.

The claimed nucleic acids could be isolated and obtained substantially in purified form. Principally, the purified form means that nucleic acids are at least approximately 50% pure, normally at least approximately 90% pure and normally are "recombinant", i.e. floxed by one or more nucleotides, which it is normally not bound with in a chromosome naturally occurring in its natural host organism.

The claimed nucleic acids could be artificially synthesized. Methods for producing nucleic acids are well known from the prior art. For example, accessibility of information about amino acid sequence or information about nucleotide sequence enables to produce isolated molecules of nucleic acids of this invention by means of oligonucleotide synthesis. In case of availability of information about amino acid sequence there could be synthesized several nucleic acids different from each other due to degeneracy of genetic code. Methods for selection of codon variants for the required host are well known in the art.

Synthetic oligonucleotides could be produced by phosphoramidite method and obtained constructs could be purified by such methods well known in the art as high performance liquid chromatography (HPLC) or other methods as described, for example, in Sambrook et al., Molecular Cloning: A Laboratory Manual, 2nd Ed., (1989) Cold Spring Harbor Press, Cold Spring Harbor, NY, and according to the instruction described, for example, in United States Dept. of HHS, National Institute of Health (NIH) Guidelines for Recombinant DNA Research. Long two-stranded DNA molecules of this invention could be synthesized as follows: several smaller fragments, which contain suitable terminals capable of cohesion with adjacent fragment, could be synthesized with the required complementarity. Adjacent fragments could be cross-linked by means of DNA lygase, recombination-based methods, or PCR-based method.

Nucleic acids coding fusion proteins, including proteins of this invention, are also provided. Examples of such proteins are given in "Proteins" section above. Nucleic acids coding fusion proteins could be artificially synthesized as described above.

Expression cassettes or systems, used *inter alia* for obtaining the claimed proteins (i.e. hispidin hydroxylases, hispidin synthases and caffeylpyruvate hydrolases) or fusion proteins on their basis or for replication of the claimed nucleic acid molecules, are also provided. Expression cassette could exist as extrachromosomal element or could be included into cell genome resulting from introduction of the said expression cassette into the cell. When introducing expression cassette into the cell a protein product is formed coded by nucleic acid of the invention; in this case it is said that protein is "produced" or "expresses" by the cell. Any expression system, including, for example, bacterial systems, yeast, plants, insects, amphibians or mammal cells, is applicable. Target nucleic acid in the expression cassette is operatively bound with regulatory sequences, which could include promoters, enhancers, terminator sequences, operators, repressors and inductors. Generally, the expression cassette comprises at least (a) transcription initiation region, functional in the host cell; (b) nucleic acid of the invention and (c) transcription termination region, functional in the host cell. Methods for obtaining expression cassettes or systems able to express the desired product are known to those skilled in the art.

Vector and other nucleic acid structures, comprising the claimed nucleic acids, are also provided. Suitable vectors include viral and nonviral vectors, plasmids, cosmids, phages, etc., and are used for cloning, amplification, expression, transfer, etc. of the nucleic acid sequence of this invention into suitable host. Selection of suitable vector is obvious to those skilled in the art. Full-length nucleic acid or its part is generally introduced to the vector by DNA lygase linking to the site split by restriction enzymes in the vector. Alternatively, the desired nucleotide sequence could be inserted by homologous recombination *in vivo,* normally, by linking homologous regions to the vector at flanks of the desired nucleotide sequence. Homologous regions are added by oligonucleotide ligation or by polymerase chain reaction, using primers, including, for example, as homologous regions, as a part of the desired nucleotide sequence. The vector, as a rule, has an origin of replication, promoting its reproduction in host cells as a result of its introduction into the cell as an extrachromosomal element. The vector could also comprise regulatory elements promoting expression of nucleic acid in the host cell and obtaining recombinant functional protein. In the expression vector the said nucleic acid is functionally bound to a regulatory sequence, which could include promoters, enhancers, terminators, operators, repressors, silencers, insulators, and inductors. For the purpose of expression of functional proteins or their shortened forms the coding nucleic acids are operatively cross-linked to the nucleic acids comprising at least regulatory sequences and transcription start site. Also, these nucleic acids could comprise sequences coding histidine tag (6 His tag), signal peptide or functional protein domains. In many embodiments the vectors promote integration of nucleic acid, operatively bound with regulatory elements, into the host cell genome. A vector could comprise expression cassette for a selectable marker, such as fluorescent protein (e.g. gfp), antibiotic resistance gene (e.g. ampicillin, or kanamycin, or neomycin, or hygromycin, etc. resistance gene), genes conditioning resistance to herbicides, such as genes conditioning resistance to phosphinothricin and sulphonamide herbicides, or other selectable marker known from the prior art.

A vector could comprise additional expression cassettes, including nucleic acids coding 4'-phosphopantetheinyl transferase, 3-arylacrylic acid synthesis proteins (for example, described in the section "Application, combinations and methods of use"), luciferases, etc.

The above expression systems could be used in prokaryotic or eukaryotic hosts. To obtain protein, there could be used such host cells as *E*. *coli, B. subtilis, S*. *cerevisiae,* insect cells or higher organism cells, which are not human embryonic cells, such as yeast, plants (e.g. *Arabidopsis thaliana, Nicotiana benthamiana, Physcomitrella patens),* vertebrata, e.g. COS 7 cells, HEK 293, CHO, Xenopus oocytes, etc.

Cell lines, which steadily produce proteins of the invention, could be selected by methods known in the art (for example, co-transfection with selectable marker, such as dhfr, gpt, antibiotic resistance genes (ampicillin, or kanamycin, or neomycin, or hygromycin, etc.), that enables to identify and isolate transfected cells, which comprise a gene included into the genome or incorporated into the extrachromosomal element.

If any above said host cell or other host cells or organisms suitable for replication and/or expression of nucleic acids of the invention are used, the obtained replicated nucleic acid, expressed protein or polypeptide are within the scope of the invention as a product of the host cell or organism. A product could be isolated by suitable method known in the art.

In many embodiments of this invention the cell is co-transfected with several expression cassettes comprising nucleic acids of the invention coding different enzymes of fungal luciferin biosynthesis. In some embodiments the expression cassette comprising nucleic acid coding luciferase, able to oxidize fungal luciferin with luminescence emission, is additionally introduced to the cell. In some cases, the expression cassettes are combined in one vector, which is used for cell transformation. In some embodiments the nucleic acids coding 4'-phosphopantetheinyl transferase and/or 3-arylacrylic acid synthesis proteins are additionally introduced to the cell.

Short DNA fragments of the claimed nucleic acids, which are used as PCR primers, rolling circle amplifications, hybridization screening probes, etc. are also provided. Long DNA fragments are used to obtain encoded polypeptides, as described above. However, for geometric amplification reactions, such as PCR, a pair of short DNA fragments, i.e. primers, is used. Exact primer sequence is not critical for the invention, however, for the most of applications the primers will be hybridized with the claimed sequence in stringent conditions, as known in the art. It is preferable to select a pair of primers, which give an amplification product from at least approximately 50 nucleotides, preferably from at least approximately 100 nucleotides, and could extend for the entire sequence of nucleic acid. Algorithms of primer sequences selection are normally known and available in commercial software packages. Amplification primers are hybridized with complementary DNA chains and will seed amplification counter reactions.

Nucleic acid molecules of this inventions can also be used to determine gene expression in biological specimen. The method where cells are examined for presence of specific nucleotide sequences, such as genomic DNA or RNA, is well known in the art. In brief, DNA or mRNA is isolated from a cell specimen. mRNA could be amplified by means of RT-PCR, using reverse transcriptase to form complementary DNA chain followed by amplification by means of polymerase chain reaction, using specific primers for the claimed DNA sequences. Alternatively, mRNA specimen is isolated by means of gel electrophoresis, transferred to a suitable carrier, e.g. nitrocellulose, nylone, etc., and then it is tested by a fragment of the claimed DNA as a probe. There also could be used other methods, such as oligonucleotide ligation analyses, hybridization *in situ* and hybridization by DNA-probes, immobilized on a hard array. Detection of mRNA hybridizing with the claimed sequence indicates gene expression in the specimen.

### Transgenic organisms

Transgenic organisms, transgenic cells and transgenic cell lines expressing nucleic acids of this invention are also provided. Transgenic cells of this invention include one or several nucleic acids under examination in this invention, which are present as transgene. For the purposes of this invention there could be used any suitable host cell, including prokaryotic (e.g. Escherichia coli, Streptomyces sp., Bacillus subtilis, Lactobacillus acidophilus, etc.) or eukaryotic host cells, which are not human embryonic cells. Transgenic organisms of this invention could be prokaryotic or eukaryotic organisms, including bacteria, cyanobacteria, fungi, plants and animals, where one or more organism cells comprising heterologous nucleic acid of the invention are introduced to by incorporating it due to human manipulation, for example, in line with transgenic techniques known in the art.

In one embodiment of this invention the transgenic organism could be a prokaryotic organism. Methods for transformation of prokaryotic host cells are well known in the art (see, for example, Sambrook et al. (Molecular Cloning: A Laboratory Manual, 2nd Ed., (1989) Cold Spring Harbor Laboratory Press and Ausubel et al., Current Protocols in Molecular Biology (1995) John Wiley & Sons, Inc).

In the other embodiment of this invention the said transgenic organism could be a fungus, e.g. yeast. Yeasts are widely used as a carrier for heterologous gene expression (see, for example, Goodey et al., Yeast biotechnology, D R Berry et al., eds, (1987) Allen and Unwin, London, pp. 401-429, and Kong et al., Molecular and Cell Biology of Yeasts, E. F. Walton and G. T. Yarronton, eds, Blackie, Glasgow (1989) pp. 107-133). There are several yeast vectors available, including integrating vectors, which require recombination with host genome for its maintenance, and also autonomously replicating plasmid vectors.

The other host organism is an animal organism. Transgenic animals could be obtained using transgenic techniques known in the art and described in standard manuals (such as: Pinkert, Transgenic Animal Technology: A Laboratory Handbook, 2nd edition (2003) San Diego: Academic Press; Gersenstein and Vinterstein, Manipulating the Mouse Embryo: A Laboratory Manual, 3rd ed, (2002) Nagy A. (Ed), Cold Spring Harbor Laboratory; Blau et al., Laboratory Animal Medicine, 2nd Ed., (2002) Fox J.G., Anderson L.C., Loew F.M., Quimby F.W. (Eds), American Medical Association, American Psychological Association; Gene Targeting: A Ptactical Approach by Alexandra L. Joyner (Ed.) Oxford University Press; 2nd edition (2000)). For example, transgenic animals could be obtained by homologous recombination within a framework of which an endogenous locus is changed. Alternatively, nucleic acid structure is integrated into a genome in random mode. Vectors for stable integration include plasmids, retroviruses and other animal viruses, YAC, etc.

Nucleic acid could be introduced int a cell directly or indirectly due to introduction to the cell precursor by means of cautious genetic manipulation, such as microinjection, or by recombinant virus infection or using recombinant virus vector, transfection, transformation, gene gun delivery or transconjugation. Techniques of nucleic acid (e.g. DNA) molecules transfer into such organisms are well known and described in standard manuals, such as Sambrook et al. (Molecular Cloning: A Laboratory Manual, 3nd Ed., (2001) Cold Spring Harbor Press, Cold Spring Harbor, NY).

The term "genetic manipulation" does not include classic crossbreeding or *in vitro* fertilization but rather refers to introduction of nucleic acid recombinant molecule. The said nucleic acid molecule could be integrated into a chromosome or could be extrachromosomal replicating DNA.

DNA structures for homologous recombination include at least a part of nucleic acid of the invention, where nucleic acid of the invention is operatively linked to homology regions, to target locus. For random integration it is not necessary to include homology regions into DNA structures to facilitate recombination. Positive and negative selection markers could also be included. Methods for obtaining the cells comprising target gene modifications by homologous recombination are known in the art. Different techniques of mammal cells transfection are described, for example, in the paper Keown et al., Meth. Enzymol. (1990) 185:527-537).

In case of embryonic stem cells (ES) there could be used ES cell line, or embryonic cells could be obtained fresh from a host organism, such as a mouse, rat, guineapig, etc. Such cells are grown on a corresponding fibroblast nurse layer or are grown in the presence of leukemia inhibitory factor (LIF). Transformed ES or embryonic cells could be used for creation of transgenic animals using the relevant technique known in the art.

Transgenic animals could be any animals different from a human, including mammal, different from a human (e.g. mouse or rat), bird or amphibia, etc., and they are used in functional tests, at drug screening, etc.

Transgenic plants could also be obtained. Methods for obtaining transgenic plant cells are described in the patents No.No. US 5767367, US5750870, US5739409, US5689049, US5689045, US5674731, US5656466, US5633155, US5629470, US5595896, US5576198, US5538879 and US5484956, which descriptions are referenced to in this invention. Methods for obtaining transgenic plants are summarized in the following reviews: Plant Biochemistry and Molecular Biology (eds. Lea and Leegood, John Wiley & Sons (1993) pp. 275-295 and Plant Biotechnology and Transgenic Plants (eds. Oksman-Caldentey and Barz) (2002) 719 p.

For obtaining transgenic host organism there could be used, for example, embryogenic explants comprising somatic cells. After cells or tissues harvesting the exogenous DNA of interest is introduced into plant cells, and there are many different techniques available for such introduction. Availability of isolated protoplasts enables the introduction using DNA-mediated gene transfer protocols, including incubation of protoplasts with deproteinized DNA, such as plasmid, including exogenous coding sequence of interest, in the presence of multivalent cations (e.g. PEG or poly-L-ornithine); or according to the protoplast electroporation method in the presence of naked DNA, including exogenous sequence of interest. Then, there is selecting the protoplasts, which succeeded in exogenous DNA uptake, growing them up to callus formation and finally obtaining the transgenic plants by contacting the enhancing factors, such as auxins and cytokinins, taken in relevant quantities and ratio.

Plants could be obtained by other suitable methods, such as "gene gun"-based method or Agrobacterium-mediated transformation, known to those skilled in the art.

### Antibodies

The term "antibody" herein refers to a polypeptide or a group of polypeptides, including at least one antibody active site (antigen-binding site). The term "antigen-binding site" refers to a space structure, which surface parameters and charge distribution are complementary to antigen epitope: it promotes antibody binding with the relevant antigen. The term "antibody" covers, for example, antibodies of vertebrate animals, chimeric antibodies, hybrid antibodies, humanized antibodies, modified antibodies, monovalent antibodies, Fab fragments, and single-domain antibodies.

Antibodies specific for proteins of this invention are applicable in affinity chromatography, immunological screening, in detection and identification of proteins of the invention (hispidin hydroxylases, hispidin synthases and caffeylpyruvate hydrolases). Antibodies of interest are bound with antigen polypeptides or proteins, or protein fragments, which are described in "Protein" section. Antibodies of the invention could be immobilized to a carrier and used in immunological screening or affinity chromatographic column to detect and/or separate polypeptides, proteins or protein fragments, or cells including such polypeptides, proteins, or protein fragments. Alternatively, such polypeptides, proteins or protein fragments could be immobilized in such a way as to detect antibodies capable of linking with them specifically.

Antibodies specific for proteins of this invention, as polyclonal as monoclonal, could be obtained using standard methods. Generally, first of all, a protein is used to immunize suitable mammal, preferably, a mouse, rat, rabbit or goat. Rabbits and goats are preferable objects for obtaining polyclonal sera due to obtaining considerable volume of blood serum, and also availability of marked antirabbit and antigoat antibodies. Normally, immunization is carried out by mixing or emulsifying the specific protein in physiological saline, preferably with an adjuvant, such as Freund adjuvant, followed by introduction of the obtained mixture or emulsion parenterally (normally, by hypodermic or intramuscular injection). Normally, sufficient doses are 50-200 µg per one injection.

In different embodiments of the invention recombinant or natural proteins are used for immunization in native or denatured form. Protein fragments or synthetic polypeptides, comprising part of protein amino acid sequence of the invention, could also be used for immunization.

Immunization is normally boosted in 2-6 weeks by one or several additional protein injections in physiological saline, preferably with incomplete Freund adjuvant. Alternatively, antibodies could also be obtained by *in vitro* immunization using methods known in the art, which are equivalent to *in vitro* immunization from the perspective of this invention purposes. Polyclonal antisera are obtained by blood sampling from immunized animals into glass or plastic vessel followed by blood incubation at 25°C within 1 hour and then by incubation at 4°C within 2-18 hours. Serum is extracted by centrifugation (for example, at 1000 g within 10 minutes). 20-50 ml of blood could be obtained from rabbits at a time.

Monoclonal antibodies are obtained using standard Kohler-Milstein technique (Kohler & Milstein, 1975, Nature, 256, 495-496) or its modifications. Normally, a mouse or rat is immunized in accordance with the above information. However, in contrast to blood sampling from animals to obtain serum, this technique involves splenectomy (and, what is not necessary, extraction of some large lymph nodes) and tissue maceration to separate individual cells. If desired, spleen cells could be screened (after extraction of non-specifically adherent cells) by application of cell suspension on a plate or in a separate plate well coated by protein-antigen. B-lymphocytes, expressing membrane-bound immunoglobulin specific for the tested antigen, are bound on the plate in such a way that they are not washed from it with suspension residue. Then, there is fusing the resulting B-lymphocytes or all macerated splenocytes with myeloma cells resulting in formation of hybridomas: then, they are incubated in a selective medium (e.g. in HAT medium, comprising hypoxanthine, aminopterin and thymidine). The resulting hybridomas are plated in limiting incubation and tested for response of antibodies, which are specifically bound with antigen used for immunization (and which are not bound with extraneous agents). Then, the selected hybridomas secreting monoclonal antibodies (mAb) are incubated either *in vitro* (e.g. in fermentors in the form of a hollow fibre bundle or in glass vessels for tissue cultures), or *in vivo* (in ascites fluid in mice).

Antibodies (as polyclonal as monoclonal) could be tagged using standard methods. The suitable tags are fluorophores, chromophores, radionuclides (in particular, 32P and 1251), electron-dense reagents, enzymes, and ligands, for which specific binding partners are known). Enzymes are normally detected by their catalytic activity. For example, horseradish peroxidase is generally detected by its ability to convert 3,3',5,5'-tetramethylbenzidine (TMB) into blue pigment, quantitatively assessed at spectrophotometer. The term "specific binding partner" refers to a protein able to bind molecule-ligand at high specificity level, as for example, in case with antigen and monoclonal antibody specific for it. The other examples of specific binding partners are biotin and avidin (or streptavidin), immunoglobulin-G and protein-A, and also multiple pairs of receptors and their ligands, known in the art. Other variants and capabilities are obvious to those skilled in the art and are considered as equivalent in the scope of this invention.

Antigens, immunogens, polypeptides, proteins, or protein fragments of this invention cause formation of specific binding partners - antibodies. The said antigens, immunogens, polypeptides, proteins, or protein fragments of this invention include immunogenic compositions of this invention. Such immunogenic compositions could additionally comprise or include adjuvants, carriers, or other compositions, which stimulate or enhance, or stabilize antigens, polypeptides, proteins or protein fragments of this invention. Such adjuvants and carriers are obvious to those skilled in the art.

### Application, combinations, and methods of use

This invention provides for application of fungal luciferin biosynthesis proteins as enzymes catalyzing reactions (1) of luciferin synthesis (namely, 6-(2-arylvinyl)-3,4-dihydroxy-2H-pyran-2-one (fungal luciferin), having the structural formula 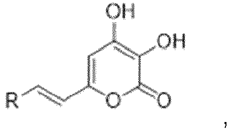 where R - aryl or heteroaryl, from preluciferin (namely, 6-2-arylvinyl)-4-hydroxy-2H-pyran-2-one), having the structural formula 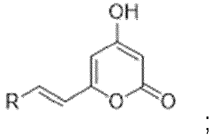 or preluciferin synthesis from 3-arylacrylic acid (preluciferin precursor) with the structural formula 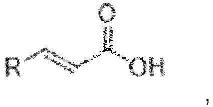 where R is selected from aryl or heteroaryl group; or 3-arylacrylic acid synthesis from 6-aryl-2-hydroxy-4-oxohexa-2,5-diene acid (oxyluciferin) with the structural formula

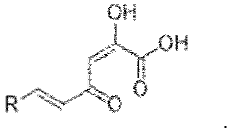

Fungal luciferin biosynthesis proteins are applied in many embodiments of this invention, and their non-limiting examples are given in this chapter below.

Fungal luciferin biosynthesis proteins, which application is ensured by this invention, could be obtained from different natural sources or by recombinant technologies. For example, wild-type proteins could be isolated from bioluminescent fungi, e.g. fungi of Basidiomycota type, predominantly of Basidiomycetes class, in particular, Agaricales order. For example, wild-type proteins could be isolated from bioluminescent fungi Neonothopanus nambi, Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos, etc. They could also be obtained by expression of recombinant nucleic acid, coding protein sequence in respective host or in cell-free expression system.

In some embodiment's proteins are applied inside host cells, where they are expressed and perform fungal luciferin cyclic transformations. In other embodiments isolated recombinant or natural proteins or extracts comprising proteins of the application are used.

Fungal luciferin biosynthesis proteins are active in physiological conditions.

In some embodiment's proteins - hispidin hydroxylases are applied *in vitro* and *in vivo* to obtain luciferin, which is oxidized by bioluminescent fungi luciferases, their homologs and mutants with luminescence emission. Therefore, this invention provided for application of hispidin hydroxylases of this invention to catalyze the transformation of 6-(2-arylvinyl)-4-hydroxy-2H-pyran-2-one (preluciferin) having the structural formula 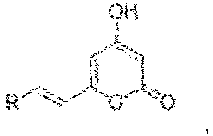 into 6-(2-arylvinyl)-3,4-dihydroxy-2H-pyran-2-one (fungal luciferin), having the structural formula 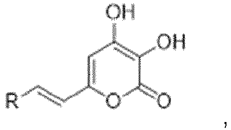 where R - aryl or heteroaryl.

Method for obtaining fungal luciferin from preluciferin includes combination of at least one molecule of hispidin hydroxylase with at least one molecule of 6-(2-arylvinyl)-4-hydroxy-2H-pyran-2-one, with at least one molecule of NAD(P)H and with at least one molecule of molecular oxygen (O2). The reaction is carried out in physiological conditions in *vitro* and *in vivo* at the temperature from 20 to 42°C, and also the reaction could be carried out in cells, tissues and host organisms expressing hispidin hydroxylase. In preferred embodiments the said cells, tissues and organisms comprise sufficient amount of NAD(P)H and molecular oxygen to carry out the reaction. Exogenously delivered 6-(2-arylvinyl)-4-hydroxy-2H-pyran-2-one or endogenous 6-(2-arylvinyl)-4-hydroxy-2H-pyran-2-one produced in cells, tissues and organisms could be used in the reaction.

In preferred embodiments hispidin hydroxylases of this invention synthesize 3-hydroxyhispidin from hispidin. In preferred embodiments they synthesize at least one functional analogue of 3-hydroxyhispidin from the corresponding preluciferin shown in Table 2. In some embodiments hispidin hydroxylases of this invention synthesize 6-(2-arylvinyl)-3,4-dihydroxy-2H-pyran-2-one from any corresponding 6-(2-arylvinyl)-4-hydroxy-2H-pyran-2-one, having the structural formula 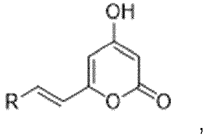 where R - aryl or heteroaryl.

The obtained 6-(2-arylvinyl)-3,4-dihydroxy-2H-pyran-2-one is applied for luminescence emitting *in vitro* and *in vivo* systems comprising functional luciferase, identifying fungal luciferin as a substrate.

For this invention purposes the proteins, which amino acid sequences are shown in SEQ ID NOs: 2, 4, 6, 8 10, 12, 14, 16, 18, 20, 22, 24, 26, 28, and also their mutants, homologs and derivatives are applicable as hispidin-hydroxylases. For example, there could be used functional hispidin hydroxylases 2, 4, 6, 8 10, 12, 14, 16, 18, 20, 22, 24, 26, 28, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99% identical for at least 350 amino acids. For example, they could be at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99% identical for all protein polypeptide chain.

In preferred embodiments for this invention purposes the proteins, which amino acid sequences are characterized by presence of several conservative amino acid motifs (consensus sequences) shown in SEQ ID NOs: 29-33, are applicable as hispidin-hydroxylases. Consensus sites inside hispidin hydroxylase amino acid sequences are operatively linked via amino acid inserts with lower insertions (Fig. 1).

In some embodiments hispidin-synthase proteins are applied in vitro and in vivo to produce fungal luciferin from its precursor, i.e applied to catalyze the transformation of 3-arylacrylic acids with the structural formula 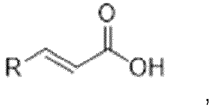 where R is selected from aryl, heteroaryl group, into 6-(2-arylvinyl)-4-hydroxy-2H-pyran-2-one, having the structural formula 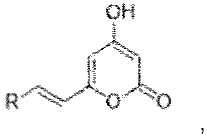 where R - aryl or heteroaryl.

Method for obtaining preluciferin includes combination of at least one molecule of hispidin-synthase with at least one molecule of 3-arylacrylic acid, with at least one molecule of coenzyme A, at least one molecule of AMP and at least two molecules of malonyl-CoA.

The reaction is carried out in physiological conditions at the temperature from 20 to 42°C, and also the reaction could be carried out in cells, tissues and host organisms expressing hispidin-synthase. In preferred embodiments the said cells, tissues and organisms comprise sufficient amount of coenzyme A, malonyl-CoA and AMP to carry out the reaction.

Exogenously delivered 3-arylacrylic acid or 3-arylacrylic acid produced in cells, tissues and organisms could be used in the reaction.

For example, hispidin-synthases of this invention could be used for producing hispidin from caffeic acid. In preferred embodiments they synthesize functional analogue of hispidin (6-(2-arylvinyl)-4-hydroxy-2H-pyran-2-one) from 3-arylacrylic acid shown in Table 2.

The obtained 6-(2-arylvinyl)-4-hydroxy-2H-pyran-2-one is applied for producing fungal luciferin in the presence of hispidin hydroxylase of this invention. Hispidin and its functional analogues are also applied in the medical field, since they exhibit antioxidant and antitumor properties; there is some evidence that hispidin is able to prevent obesity [Be Tu et al., Drug Discov Ther. 2015 Jun; 9 (3): 197-204; Nguyen et al., Drug Discov Ther. 2014 Dec; 8 (6): 238-44; Yousfi et al., Phytother Res. 2009 Sep; 23 (9): 1237-42].

For this invention purposes the proteins, which amino acid sequences are shown in SEQ ID NOs: 35, 37, 39, 41, 43, 45,47,49, 51, 53, 55, and also their mutants, homologs and derivatives are applicable as hispidin-synthases. For example, there could be used functional hispidin-synthases with amino acid sequence identical to the sequence selected from the group of SEQ ID NOs: 35, 37, 39, 41, 43, 45,47,49, 51, 53, 55, at least 40%, prevalently at least 45%, normally at least 50%, e.g. at least 55%, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99% identical.

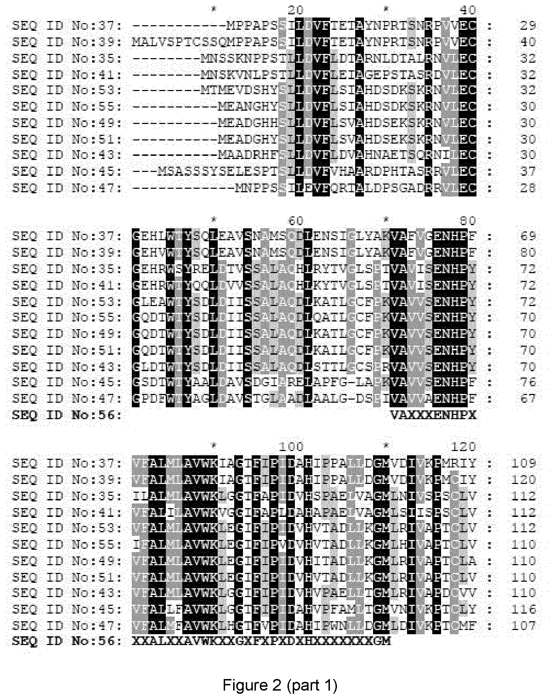

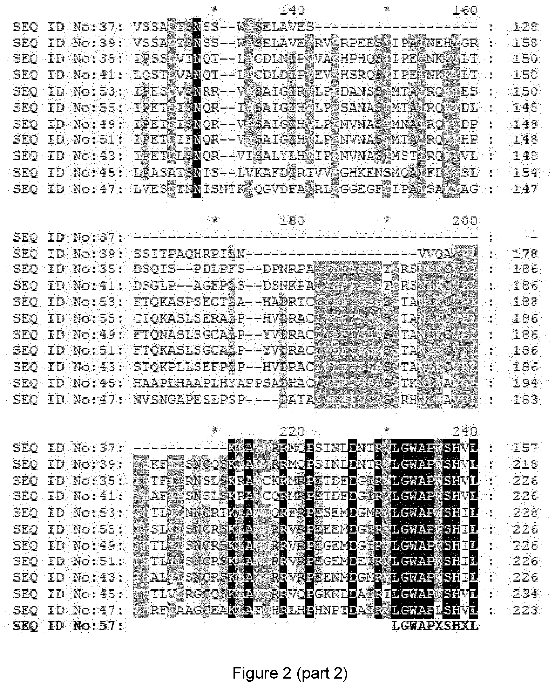

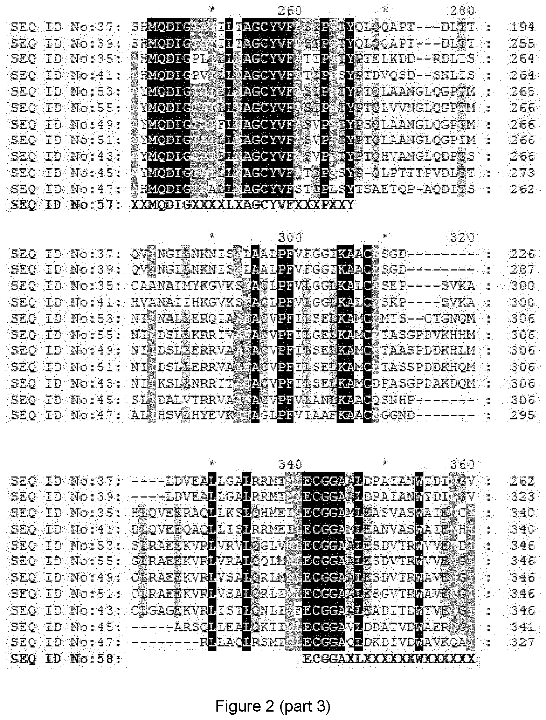

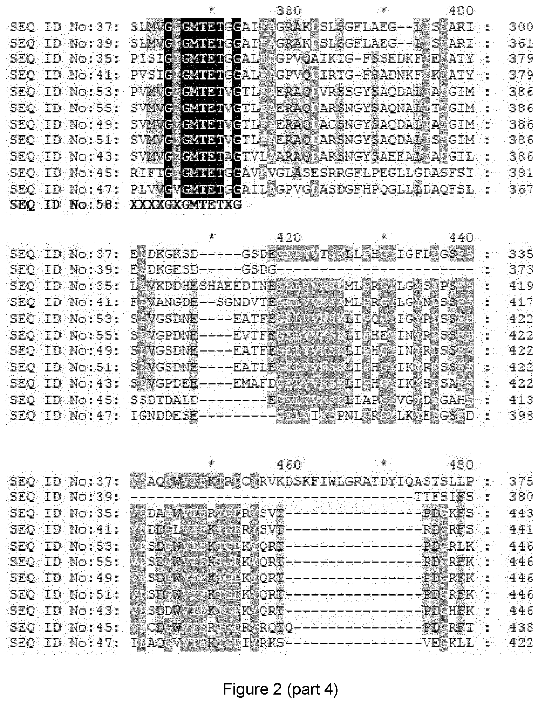

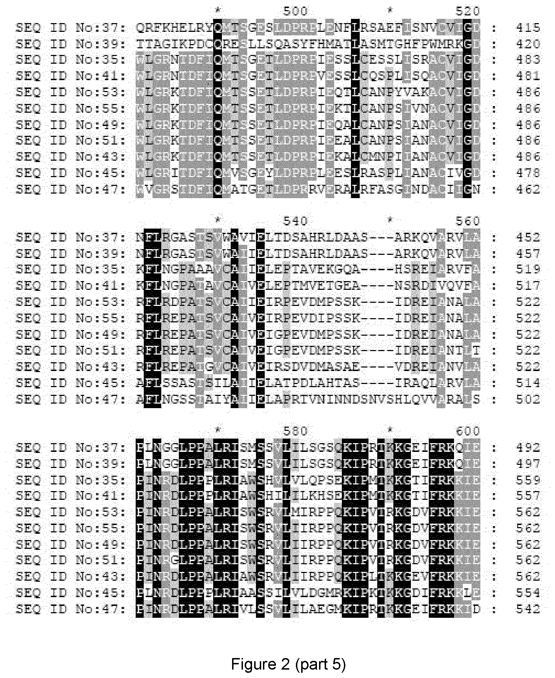

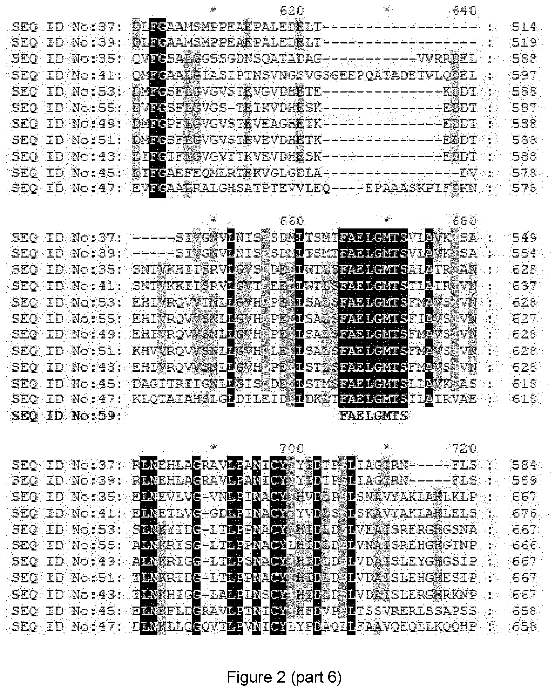

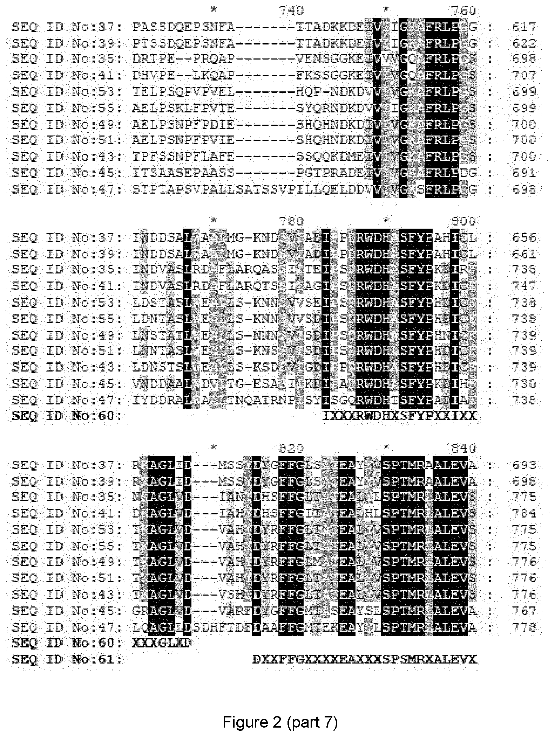

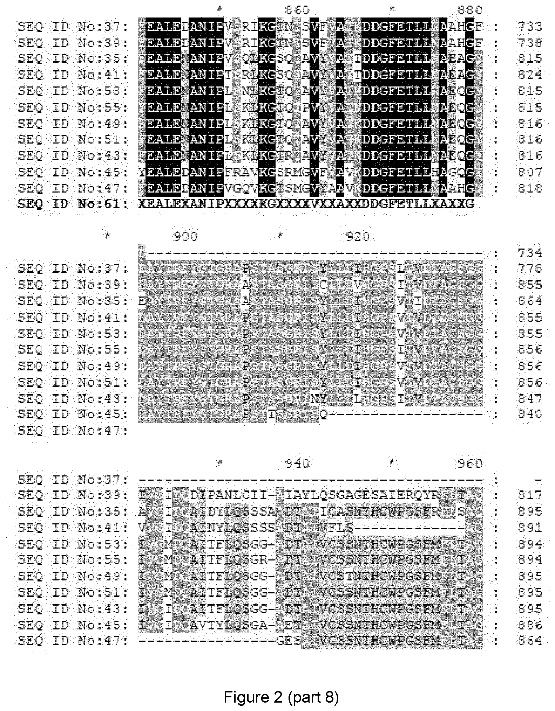

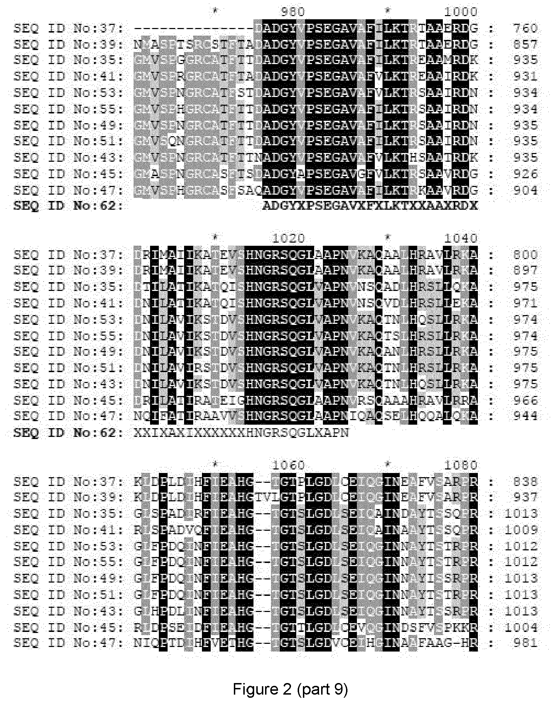

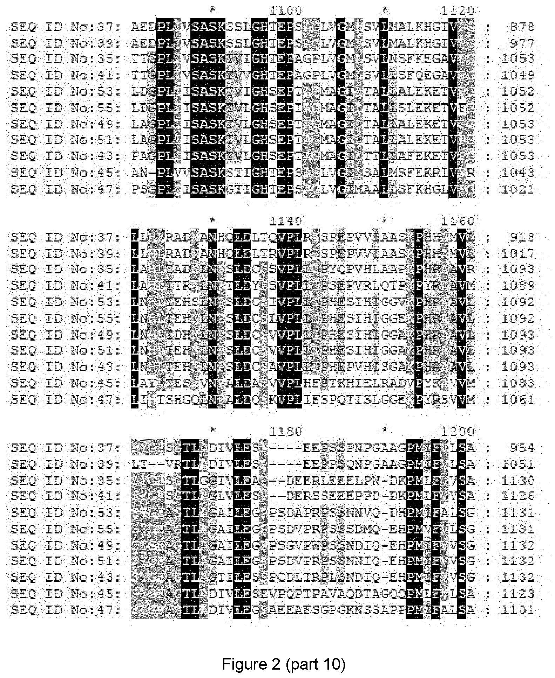

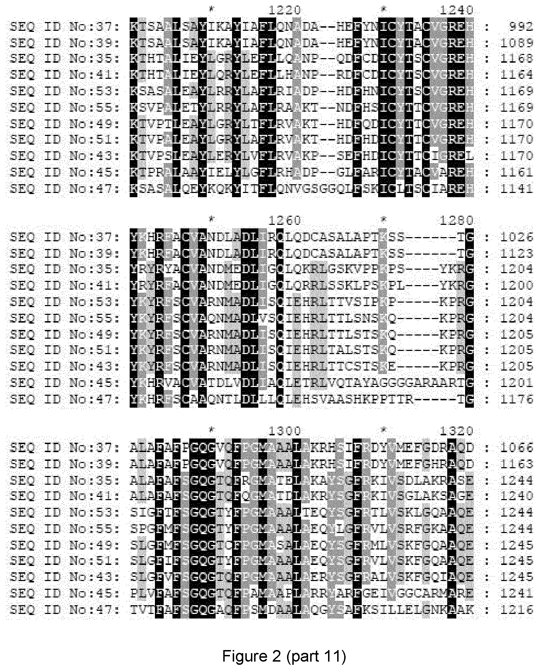

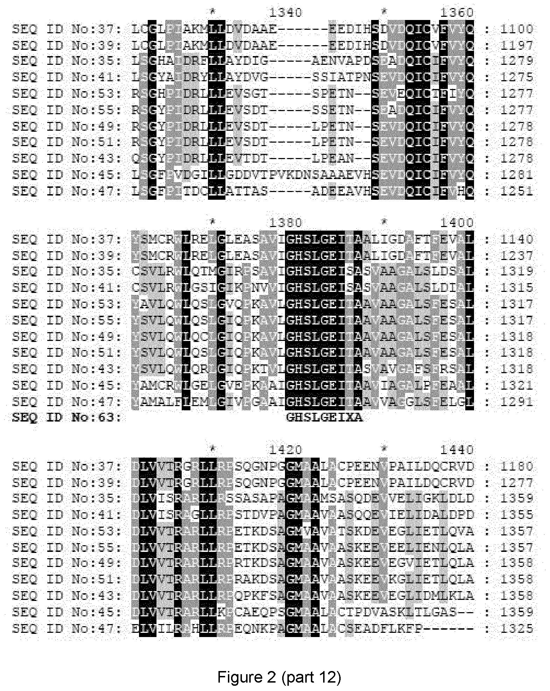

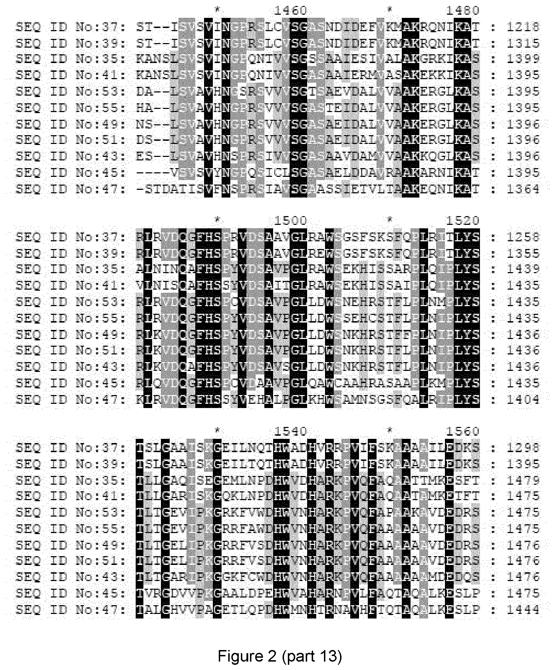

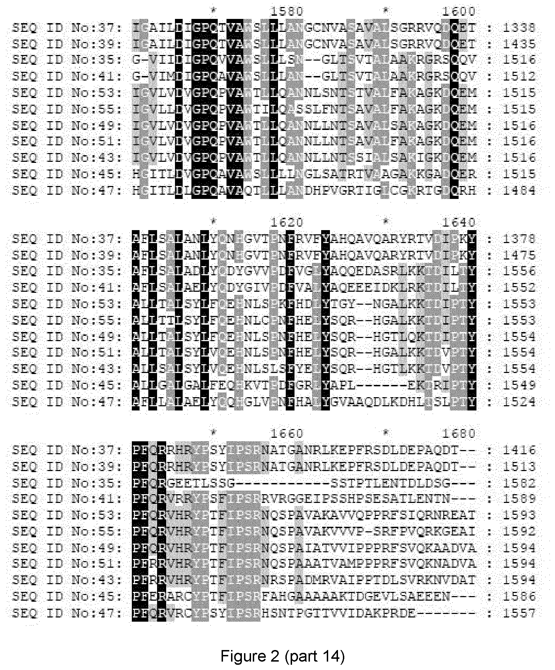

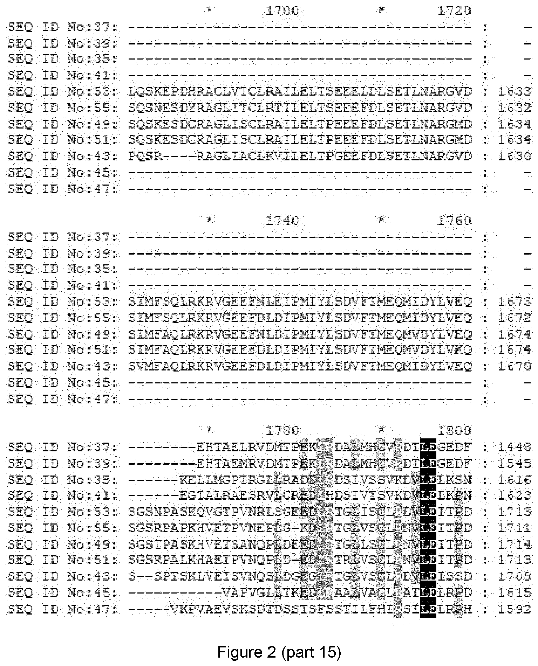

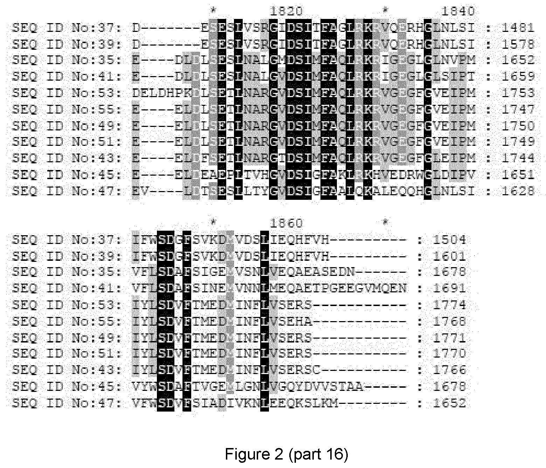

In preferred embodiments for this invention purposes the proteins, which amino acid sequences are characterized by presence of several conservative amino acid motifs (consensus sequences) shown in SEQ ID NOs: 56-63, are applicable as hispidin-synthases. Consensus sites inside hispidin-synthase amino acid sequences are operatively linked via amino acid inserts with lower insertions (Fig. 2).

In some embodiments caffeylpyruvate hydrolase proteins are applied *in vitro* and *in vivo* for producing 3-arylacrylic acids with the structural formula 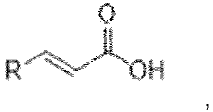 where R is selected from aryl, heteroaryl group, from 6-aryl-2-hydroxy-4-oxohexa-2,5-diene acid with the structural formula 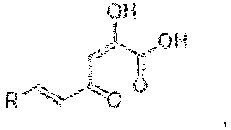 where R - aryl or heteroaryl. The reaction is carried out in physiological conditions *in vitro* and *in vivo.* Caffeoyl pyruvate hydrolases of this invention are applied in autonomous bioluminescence systems described in detail below.

For this invention purposes the proteins, which amino acid sequences are shown in SEQ ID NOs: 65, 67, 69, 71, 73, 75, and also their functional mutants, homologs and derivatives are applicable as caffeoyl pyruvate hydrolases. For example, there could be used functional caffeylpyruvate hydrolases with amino acid sequence identical to the sequence selected from the group of SEQ ID NOs: 65, 67, 69, 71, 73, 75, at least 60%, at least 65%, at least 70%, at least 80%, at least 85%, at least 90%, or at least 91%, or at least 92%, or at least 93%, or at least 94%, or at least 95%, or at least 96%, or at least 97%, or at least 98%, or at least 99% identical.

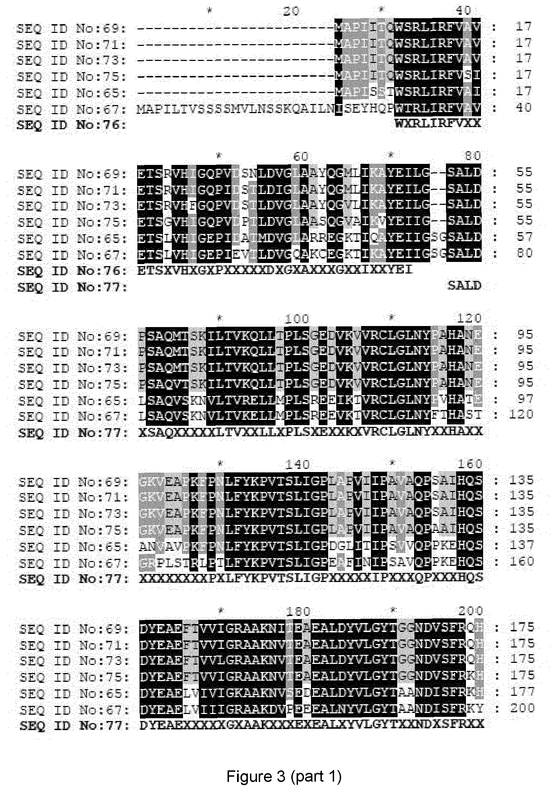

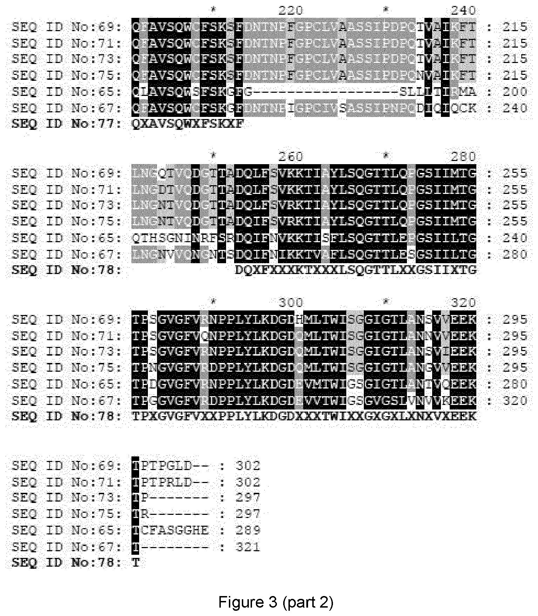

In preferred embodiments for this invention purposes the proteins, which amino acid sequences are characterized by presence of several conservative amino acid motifs (consensus sequences) shown in SEQ ID NOs: 76-78, are applicable as caffeoyl pyruvate hydrolases. Consensus sites inside caffeylpyruvate hydrolase amino acid sequences are operatively linked via amino acid inserts with lower insertions (Fig. 3).

Protein combinations applicable in the methods of this invention are also provided. In preferred embodiments the combinations include functional hispidin hydroxylase and functional hispidin synthase. This combination is applied for producing 6-(2-arylvinyl)-3,4-dihydroxy-2H-pyran-2-one from 3-arylacrylic acid with the structural formula 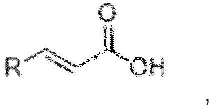 where R is aryl or heteroaryl. For example, the combination could be used for producing caffeic acid hydroxyhispidin. The reaction is carried out in physiological conditions in the presence of at least one molecule of hispidin hydroxylase, at least one molecule of hispidin synthase, at least one molecule of 3-arylacrylic acid, at least one molecule of coenzyme A, at least one molecule of AMP, at least two molecules of malonyl-CoA, at least one molecule of NAD(P)H and at least of one molecule of molecular oxygen (O2).

In some embodiments the combination also includes luciferase able to use 6-(2-arylvinyl)-3,4-dihydroxy-2H-pyran-2-one with the structural formula 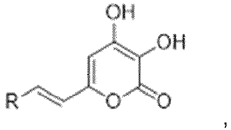 where R - aryl or heteroaryl, as luciferin. Oxidation of the said 6-(2-arylvinyl)-3,4-dihydroxy-2H-pyran-2-one by such luciferase is accompanied with bioluminescence and formation of oxyluciferin (6-aryl-2-hydroxy-4-oxohexa-2,5-diene acid).

Any protein characterized by the above activity could be used as a luciferase. For example, known luciferases from bioluminescent fungi, including those described in the application RU Nº2017102986/10005203 dd 30.01.2017, and also their homologs, mutants and fused proteins having luciferase activity.

In many embodiments of this invention the luciferases, applicable for this invention purposes, are characterized by amino acid sequences, which are at least 40% identical, e.g. at least 45% identical, or at least 50% identical, or at least 55% identical, or at least 60% identical, or at least 70% identical, or at least 75% identical, or at least 80% identical,or at least 85% identical to the amino acid sequence selected from the group of SEQ ID NOs: 80, 82, 84, 86, 88, 90 , 92, 94, 96, 98. Luciferases are often characterized by amino acid sequences, which have the following identity to the amino acid sequence selected from the group of SEQ ID NOs: 80, 82, 84, 86, 88,90 , 92, 94, 96, 98, minimum 90% of identity (e.g. at least 91%, minimum 92%, minimum 93%, minimum 94%, minimum 95%, minimum 96%, minimum 97%, minimum 98%, minimum 99% of identity or 100% of identity).

Mutants can retain biological properties of wild-type luciferase, from which they have been obtained, or can have biological properties different from wild-type proteins. The term "biological properties" of luciferases according to this invention refers, without limitation, to capability to oxidize different luciferins; biochemical properties, such as *in vivo* and/or *in vitro* stability (e.g. half-life); rate of maturation; tendency to aggregation or oligomerization, and also other similar properties. Mutations include changes of one or more amino acids, deletion, or insertion of one or more amino acids, replacements or truncations, or N-terminal truncations or extensions, C-terminal truncations or extensions, etc.

In some embodiments of the invention the luciferases are used in isolated form, i.e. they are substantially free from other proteins or other natural biological molecules, such as oligosaccharides, nucleic acids and their fragments, etc., where the term "substantially free from" in this case means that less than 70%, normally less than 60% and prevalently less than 50% of the said composition, comprising the isolated protein, is the other natural biological molecule. In some embodiments the said proteins are substantially in purified form, where the term "substantially purified form" means purity equal at least 95%, normally equal at least 97% and prevalently equal at least 99%.

In some embodiments the luciferases are used as part of extracts obtained from bioluminescent fungi or host cells comprising nucleic acids coding recombinant luciferases.

In many embodiments the luciferases are in heterologous expression systems (in cells or organisms of this invention), which comprise nucleic acids coding recombinant luciferases.

Methods for producing recombinant proteins, in particular, luciferases, as in isolated form, or as part of extracts, or in heterologous expression systems, are well known in the art and described in "Nucleic Acids" section. Protein purification methods are described in "Proteins" section.

In preferred embodiments the luciferases retain activity at temperatures below 50°C, prevalently at temperatures maximum 45°C, i.e. they retain activity at temperatures 20-42°C and could be used in heterologous expression systems *in vitro* and *in vivo.* Normally, the described luciferases have pH stability within the range from 4 to 10, prevalently within the range from 6.5 to 9.5. Optimum pH stability of the claimed proteins is within the range from 7.0 to 8.5, e.g. between 7.3-8.0. In preferred embodiments the said luciferases are active in physiological conditions.

Combination of hispidin hydroxylase and luciferase oxidizing fungal luciferin with luminescence emission is applied in methods of hispidin and its functional analogues identification in biological objects: cells, tissues or organisms. The method includes contact of the test biological object or extract, obtained from it, with combination of isolated hispidin hydroxylase and said luciferase in suitable reaction buffer creating physiological conditions and comprising the required components to carry out reactions. A person skilled in the art could make a variety of reaction buffers satisfying this condition. Non-limiting example of the reaction buffer could be 0.2 M sodium phosphate buffer (pH 7.0-8.0) laced with 0.5 M of Na₂SO₄, 0.1% of dodecylmaltoside (DDM), 1 mM of NADPH.

Presence of hispidin or its functional analogue is determined by occurrence of the detectable luminescence - bioluminescence. Methods for detecting the detectable luminescence are described above in "Proteins" section when describing the functional screening. methods.

Combination of hispidin hydroxylase, hispidin synthase and luciferase oxidizing fungal luciferin with luminescence emission is applied in methods for identifying 3-arylacrylic acid with the structural formula 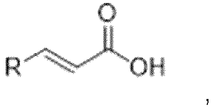 where R is aryl or heteroaryl, in biological objects. The method includes contact of the test biological object or extract, obtained from it, with combination of isolated hispidin hydroxylase, hispidin synthase and luciferase creating physiological conditions and comprising the required components to carry out reactions. A person skilled in the art could make a variety of reaction buffers satisfying this condition. Non-limiting example of the reaction buffer could be 0.2 M sodium phosphate buffer (pH 7.0-8.0) laced with 0.5 M of Na₂SO₄, 0.1% of dodecylmaltoside (DDM), 1 mM of NADPH, 10 mM of ATP, 1 mM of CoA, 1 mM of malonyl-CoA.

Presence of 3-arylacrylic acid is determined by occurrence of the detectable luminescence - bioluminescence. Methods for detecting the detectable luminescence are described above in "Proteins" section when describing the functional screening. methods.

In some embodiments instead of the combination of hispidin hydroxylase and luciferase oxidizing fungal luciferin with luminescence emission there could be used a fusion protein described in "Protein" section above. A fusion protein simultaneously exhibits hispidin hydroxylase activity and luciferase activity and it could be used in any methods instead of the combination of the said enzymes.

In some embodiments instead of the above hispidin-synthase there is used a type III polyketide synthase characterized by amino acid sequence identical to the amino acid sequence selected from the group of SEQ ID NOs: 119, 121, 123, 125, 127, 129, 131, 133, 135, 137, 139. For the purposes of this invention there are applicable type III polyketide synthases having the amino acid sequence identical to the sequence selected from the group of SEQ ID NOs: 119, 121, 123, 125, 127, 129,131, 133, 135, 137, 139 at least 40%, prevalently at least 45%, normally at least 50%, e.g. at least 55%, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99% identical.

The representatives of the said polyketide synthases (PKS) are identified in many plant organisms; their and/or their mutant capability to catalyze bisnoryangonin synthesis from coumaryl-CoA is known in the art [Lim et al., Molecules, 2016 Jun 22;21(6)]. The Applicants have demonstrated that the said enzymes are also able to catalyze hispidin synthesis from caffeyl-CoA *in vitro* and *in vivo:*

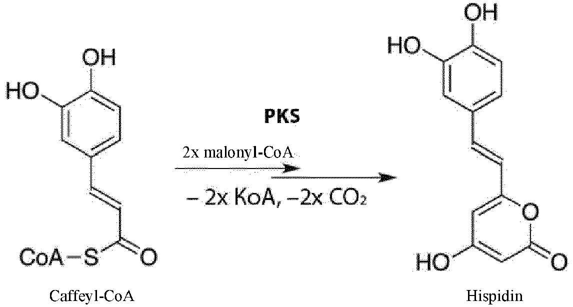

Therefore, application of the said proteins for hispidin synthesis is also within the scope of this invention.

In some embodiments of the invention PKS are used in isolated form, i.e. they are substantially free from other proteins or other natural biological molecules, such as oligosaccharides, nucleic acids and their fragments, etc., where the term "substantially free from" in this case means that less than 70%, normally less than 60% and prevalently less than 50% of the said composition, comprising the isolated protein, is the other natural biological molecule. In some embodiments the said proteins are substantially in purified form, where the term "substantially purified form" means purity equal at least 95%, normally equal at least 97% and prevalently equal at least 99%.

In many embodiments PKS are in heterologous expression systems (in cells or organisms of this invention), which comprise nucleic acids coding recombinant enzymes.

Methods for producing recombinant proteins, as in isolated form, or as part of extracts, or in heterologous expression systems, are well known in the art and described in "Nucleic Acids" section. Protein purification methods are described in "Proteins" section.

In preferred embodiments PKS retain activity at temperatures below 50°C, prevalently at temperatures maximum 45°C, i.e. they retain activity at temperatures 20-42°C and could be used in heterologous expression systems *in vitro* and *in vivo.* Normally, the described PKS have pH stability within the range from 4 to 10, prevalently within the range from 6.0 to 9.0. Optimum pH stability of the claimed proteins is within the range from 6.5 to 8.5, e.g. between 7.0-7.5. In preferred embodiments the said PKS are active in physiological conditions.

Method for obtaining hispidin includes combination of at least one molecule of type III polyketide synthases, described above, with at least two molecules of malonyl-CoA and at least one molecule of caffeyl-CoA.

In some embodiments the method includes producing caffeyl-CoA from caffeic acid during enzymatic reaction catalyzed by coumarate-CoA ligase. In this case the method includes combination of type III polyketide synthases, described above, with at least one molecule of caffeic acid, with at least one molecule of coenzyme A, at least one molecule of coumarate-CoA ligase, at least one molecule of ATP and at least two molecules of malonyl-CoA.

For the purposes of this invention there could be used any coumarate-CoA ligase enzymes, known in the art, which perform reaction of coenzyme A addition to caffeic acid with caffeyl-CoA formation:

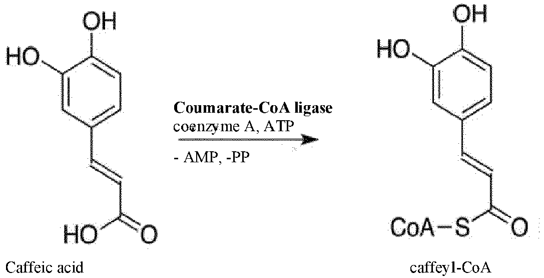

In particular, there could be used coumarate-CoA ligase 1 from *Arabidopsis thaliana,* having amino acid and nucleic sequences shown in SEQ ID NO: 141, and also its functional mutants and homologs. For example, for the purposes of this invention it is applicable the functional coumarate-CoA ligase, which amino acid sequence has minimum 40% of identity, e.g. minimum 45% of identity, or minimum 50% of identity, or minimum 55% of identity, or minimum 60% of identity, or minimum 65% of identity, or minimum 70% of identity, or minimum 75% of identity, e.g. minimum 80% of identity, minimum 85% of identity, minimum 90% of identity (e.g. at least 90%, 91%, 92%, 93%, 94%, 95%, 96%, 97%, 98%, 98% or 99% of identity) with amino acid sequence shown in SEQ ID NO: 141.

All the said reactions are carried out in physiological conditions at the temperature from 20 to 50°C, and also the reaction could be carried out in cells, tissues and host organisms expressing functional enzymes.

PKS and coumarate-CoA ligase combined with hispidin hydroxylase of this invention could be used for producing 3-hydroxyhispidin from caffeic acid. The reaction is carried out in physiological conditions in the presence of at least one molecule of hispidin hydroxylase, at least one molecule of PKS, at least one molecule of coumarate-CoA ligase, at least one molecule of caffeic acid or caffeyl-CoA, at least one molecule of coenzyme A, with at least one molecule of ATP, with at least one molecule of NAD(P)H, with at least of one molecule of oxygen, and at least two molecules of malonyl-CoA.

Also, this invention provides for application of nucleic acids coding enzymes of fungal luciferin biosynthesis, mutants and homologs of these proteins, including shortened and elongated forms, and fusion proteins to obtain enzymes involved in fungal luciferin biosynthesis *in vitro* and\or *in vivo.*

In preferred embodiments there is provided application of nucleic acids coding hispidin hydroxylases of the invention, namely proteins characterized by amino acid sequences shown in SEQ ID NOs: 2, 4, 6, 8 10, 12, 14, 16, 18, 20, 22, 24, 26, 28, and also their functional homologs, mutants and derivatives. In preferred embodiments nucleic acids code proteins, which amino acid sequences are at least 40%, prevalently at least 45%, normally at least 50%, e.g. at least 55%, at least 60%, at least 65%, at least 70%, at least 80%, at least 85%, at least 90%, or at least 91%,or at least 92%, or at least 93%, or at least 94%,or at least 95%, or at least 96%, or at least 97%, or at least 98%, or at least 99% identical to the sequences shown in SEQ ID NOs: 2, 4, 6, 8 10, 12, 14, 16, 18, 20, 22, 24, 26, 28, for at least 350 amino acids. In preferred embodiments nucleic acids code proteins, which amino acid sequences are characterized by presence of several conservative amino acid motifs (consensus sequences) shown in SEQ ID NO: 29-33.

There is also provided application of nucleic acids coding hispidin-synthases, namely proteins characterized by amino acid sequences shown in SEQ ID NOs: 35, 37, 39, 41, 43, 45, 47, 49, 51, 53, 55, and also their functional homologs, mutants and derivatives. In preferred embodiments nucleic acids of the invention code proteins, which amino acid sequences are at least 40%, prevalently at least 45%, normally at least 50%, e.g. at least 55%, or at least 60%, or at least 65%, or at least 70%, or at least 80%, or at least 85%, or at least 90%, or at least 91%,or at least 92%, or at least 93%, or at least 94%,or at least 95%, or at least 96%, or at least 97%, or at least 98%, or at least 99% identical to the sequences shown in SEQ ID NOs: 35, 37, 39, 41, 43, 45,47,49, 51, 53, 55, for all protein polypeptide chain. In preferred embodiments nucleic acids code proteins, which amino acid sequences are characterized by presence of several conservative amino acid motifs (consensus sequences) shown in SEQ ID NOs: 56-63.

There is also provided application of nucleic acids coding caffeylpyruvate hydrolases, namely proteins characterized by amino acid sequences shown in SEQ ID NOs: 65, 67, 69, 71, 73, 75, and also their functional homologs, mutants and derivatives. In preferred embodiments nucleic acids of the invention code proteins, which amino acid sequences are at least 40%, prevalently at least 45%, normally at least 50%, e.g. at least 55%, or at least 60%, or at least 65%, or at least 70%, or at least 80%, or at least 85%, or at least 90%, or at least 91%,or at least 92%, or at least 93%, or at least 94%,or at least 95%, or at least 96%, or at least 97%, or at least 98%, or at least 99% identical to the sequences shown in SEQ ID NOs: 65, 67, 69, 71, 73, 75, for all protein polypeptide chain. In preferred embodiments nucleic acids code proteins, which amino acid sequences are characterized by presence of several conservative amino acid motifs (consensus sequences) shown in SEQ ID NOs: 76-78.

The above groups of nucleic acids are applied for producing recombinant proteins of hispidin hydroxylases, hispidin synthases and caffeylpyruvate hydrolases, and also for expression of these proteins in heterologous expression systems.

In particular, nucleic acids coding hispidin hydroxylases are applied for obtaining producer cells of 6-(2-arylvinyl)-3,4-dihydroxy-2H-pyran-2-one, having the structural formula 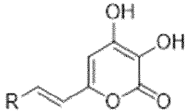 from exogenous or endogenous 6-(2-arylvinyl)-4-hydroxy-2H-pyran-2-one, having the structural formula 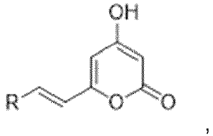 where R - aryl or heteroaryl.

Nucleic acids coding caffeylpyruvate hydrolases are applied for obtaining cells and organisms able to transform oxyluciferin into preluciferin precursor.

Nucleic acids coding hispidin-synthases are applied for obtaining producer cells of the above 6-(2-arylvinyl)-4-hydroxy-2H-pyran-2-one from the corresponding 3-arylacrylic acid. For example, cells expressing hispidin-synthase are applied for producing hispidin from caffeic acid.

In some embodiment's nucleic acids coding hispidin-synthases are applied for producing hispidin from tyrosine. In the said embodiments, nucleic acids, coding enzymes promoting synthesis of caffeic acid from tyrosine, are additionally introduced into the cells. Such enzymes are known in the art. For example, there could be used a combination of nucleic acids coding tyrosine-ammonia-lyase Rhodobacter capsulatus, and the components HpaB and HpaC of E. coli 4-hydroxyphenyl acetate 3-monooxygenase-reductase as described in [Lin and Yan. Microb Cell Fact. 2012, 4;11:42]. It is obvious to those skilled in the art that alternatively there could be used any other known in the art enzymes transforming tyrosine into caffeic acid, for example, enzymes, which amino acid sequences are substantially identical to the amino acid sequences of tyrosine-ammonia-lyase Rhodobacter capsulatus, and the components HpaB and HpaC of E. coli 4-hydroxyphenyl acetate 3-monooxygenase-reductase, shown in SEQ ID NOs: 107, 109 and 111. For example, the said enzymes could have amino acid sequences which have minimum 40% of identity, e.g. minimum 45% of identity, or minimum 50% of identity, or minimum 55% of identity, or minimum 60% of identity, or minimum 65% of identity, or minimum 70% of identity, or minimum 75% of identity, e.g. or minimum 80% of identity, or minimum 85% of identity, or minimum 90% of identity (e.g. at least 90%, 91%, 92%, 93%, 94%, 95%, 96%, 97%, 98%, 98% or 99% of identity) with amino acid sequence shown in SEQ ID NO: 107, 109 and 111 respectively.

In some embodiments, nucleic acids coding hispidin-synthases are applied for obtaining producer cells of hispidin functional analogues from aromatic compounds, including aromatic amino acids and their derivatives. In the said embodiments, nucleic acids, coding enzymes promoting synthesis of 3-arylacrylic acids, from which hispidin functional analogues are biosynthesized, are additionally introduced into the cells. Such enzymes are known in the art. For example, for cinnamic acid biosynthesis there could be used nucleic acid coding phenylalanine-ammonia-lyase *Streptomyces maritimus,* as described in [Bang, H.B., Lee, Y.H., Kim, S.C. et al. Microb Cell Fact (2016) 15: 16. https://doi.org/10.1186/s12934-016-0415-9]. It is obvious to those skilled in the art that alternatively there could be used any other known in the art enzymes transforming aromatic amino acids and other aromatic compounds into 3-aryl acrylic acids. For example, for cinnamic acid biosynthesis there could be any functional phenylalanine-ammonia-lyase, e.g. phenylalanine-ammonia-lyase, which amino acid sequence is substantially similar to the sequence shown in SEQ ID NOs: 117, for example, which sequence is identical to the sequence of SEQ ID NO: 117 at least 40%, including minimum 45% of identity, or minimum 50% of identity, or minimum 55% of identity, or minimum 60% of identity, or minimum 65% of identity, or minimum 70% of identity, or minimum 75% of identity, e.g. minimum 80% of identity, minimum 85% of identity, minimum 90% of identity (e.g. at least 90%, 91%, 92%, 93%, 94%, 95%, 96%, 97%, 98%, 98% or 99% of identity).

In some embodiments for obtaining host cells expressing functional hispidin-synthase it is required to co-transfect them by nucleic acid coding hispidin-synthases of the invention and by nucleic acid coding 4'-phosphopantetheinyl transferase able to transfer 4'-phosphopantetheinyl from coenzyme A to serine in acyl carrier domain of polyketide synthases. In other embodiments the selected host cells, for example, plant cells or cells of some lower fungi (e.g. *Aspergillus*)*,* comprise endogenous 4'-phosphopantetheinyl transferase and co-transfection is not required.

Application of nucleic acid combinations of the invention is also provided. Thus, a combination of the nucleic acids, coding hispidin hydroxylase and hispidin synthase, is applied for obtaining producer cells of 6-(2-arylvinyl)-3,4-dihydroxy-2H-pyran-2-one from 3-arylacrylic acid, for example, for producing 3-hydroxyhispidin from caffeic acid and/or tyrosine. In other embodiments a combination of the nucleic acids includes a nucleic acid coding 4'-phosphopantetheinyl transferase. In some embodiments a combination of the nucleic acids includes the nucleic acids coding enzymes promoting 3-arylacrylic acid synthesis from the cell metabolites, e.g. enzymes promoting caffeic acid synthesis from tyrosine or cinnamic acid synthesis from phenylalanine.

In some embodiments a combination of the nucleic acids, coding PKS and coumarate-CoA ligase, is used for obtaining hispidin producer cells from caffeic acid. For the purposes of this invention it is applicable a nucleic acid coding functional PKS, which amino acid sequence is substantially similar or identical to the sequence selected from the group SEQ ID NOs: 119, 121, 123, 125, 127, 129, 131, 133, 135, 137, 139; e.g. PKS, which amino acid sequence is identical to the sequence selected from the group SEQ ID NOs: 119, 121, 123, 125, 127, 129,131, 133, 135, 137, 139 at least 40%, prevalently at least 45%, normally at least 50%, e.g. at least 55%, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99% identical. The nucleic acid coding functional coumarate-CoA ligase, catalyzing reaction of coenzyme A addition to caffeic acid with caffeyl-CoA formation, is also applicable for the purposes of this invention. For example, there could be used a nucleic acid coding functional coumarate-CoA ligase, which amino acid sequence is identical to the sequence shown SEQ ID NO: 141, or has minimum 40% of identity, e.g. minimum 45% of identity, or minimum 50% of identity, or minimum 55% of identity, or minimum 60% of identity, or minimum 65% of identity, or minimum 70% of identity, or minimum 75% of identity, e.g. minimum 80% of identity, minimum 85% of identity, minimum 90% of identity (e.g. at least 90%, 91%, 92%, 93%, 94%, 95%, 96%, 97%, 98%, 98% or 99% of identity).

In some embodiments a combination of the nucleic acid coding hispidin hydroxylases of the invention and the nucleic acid coding PKS is used. In preferred embodiments the combination also includes a nucleic acid coding coumarate-CoA ligase. The combination is applied for obtaining 3-hydroxyhispidin producer cells from caffeic acid and/or caffeyl-CoA.

In some embodiments a combination of nucleic acids includes nucleic acids coding enzymes promoting synthesis of caffeic acid from tyrosine.

Combinations of the nucleic acids of the inventions used together with the nucleic acid coding luciferase, able to oxidize fungal luciferin with luminescence emission, are of special interest. Nucleic acid molecules, coding luciferases for the purposes of this invention, could be cloned from biological sources, for example, from fungi of Basidiomycota type, predominantly of Basidiomycetes class, in particular, Agaricales order, or obtained by techniques of genetic modification. Luciferase mutants having luciferase activity could be obtained using standard techniques of molecular biology, such as described above in details in "Nucleic Acids" section. Mutations include changes of one or more amino acids, deletion, or insertion of one or more amino acids, replacements or truncations, or N-terminal truncations or extensions, C-terminal truncations or extensions, etc. In preferred embodiments these nucleic acids code luciferases, which amino acid sequences are at least 40% identical, e.g. at least 45% identical, or at least 50% identical, or at least 55% identical, or at least 60% identical, or at least 70% identical, or at least 75% identical, or at least 80% identical, or at least 85% identical to the amino acid sequence selected from the group of SEQ ID NOs: 80, 82, 84, 86, 88, 90 , 92, 94, 96, 98. For example, they could have amino acid sequences which have minimum 90% of identity (e.g. minimum 91%, minimum 92%, minimum 93%, minimum 94%, minimum 95%, minimum 96%, minimum 97%, minimum 98%, minimum 99% or 100% of identity) with amino acid sequence selected from the group SEQ ID NOs: 80, 82, 84, 86, 88,90 , 92, 94, 96, 98. Non-limiting examples of nucleic acids, coding luciferases, are given in SEQ ID NOs: 79, 81, 83, 85, 87, 89, 91, 93 and 95.

In some embodiments a combination of the nucleic acid coding hispidin hydroxylase of the invention and the nucleic acid coding the above luciferase is used. The combination is widely applicable when labeling organisms, tissues, cells, cell organelles or proteins by bioluminescence. Methods for labeling organisms, tissues, cells, cell organelles or proteins by luciferase are well known in the art and presuppose introduction of a nucleic acid, coding luciferase, into a host cell, for example being a part of an expression cassette promoting luciferase expression in the said cell, tissue or organism. When adding the suitable luciferin to cells, tissue or organism, expressing a luciferase, detectable luminescence occurs. When labeling cell organelles or proteins, the nucleic acid, coding luciferase, is operatively bound with the nucleic acid coding respectively the localization signal in the test cell organelle or test protein. At co-expression in the cells of luciferase and hispidin-synthase of this invention the biological objects (cells, tissues, organisms, cell organelles or proteins) acquire the ability to emit luminescence in the presence of not only fungal luciferin, but of preluciferin as well (the latter in most cases is more stable in the presence of ambient oxygen).

Also, the combination of the nucleic acids is applicable in study of two promoters activity dependency in heterologous expression systems. In this case a nucleic acid operatively bound with promoter A, coding luciferase, and a nucleic acid operatively bound with promoter B, coding hispidin hydroxylase, are introduced into a host cell. Adding luciferin or preluciferin, or preluciferin and luciferase mixture to cell (or cell extracts) aliquots, it is possible to detect by occurrence of luminescence emission the activity of one promoter A (luminescence emission is detected in the presence of luciferin only), of one promoter B (luminescence emission is detected in the presence of preluciferin and luciferase mixture) or of both promoters (luminescence emission is detected in all cases).

In some embodiments the combination also comprises a nucleic acid coding hispidin-synthase. In some embodiments the combination additionally comprises a nucleic acid coding 4'-phosphopantetheinyl transferase.

In some embodiments the combination comprises a nucleic acid coding hispidin-synthase, a nucleic acid coding luciferase, a nucleic acid coding PKS, a nucleic acid coding coumarate-CoA ligase.

The combinations are widely applicable when labeling organisms, tissues, cells, cell organelles or proteins by bioluminescence. In this embodiment in order to obtain luminescence emission, a suitable preluciferin precursor, e.g. caffeic acid or coumaric acid, is added to biological objects expressing hispidin hydroxylase, luciferase and hispidin synthase or hispidin hydroxylase, luciferase, PKS and coumarate-CoA ligase.

The combinations are also applicable in methods of study of three promoters activity dependency in heterologous expression systems. The methods presuppose introduction of nucleic acid coding luciferase under promoter A control, of nucleic acids, coding hispidin hydroxylase under promoter B control and nucleic acid coding hispidin synthase (or PKS), under promoter B control, into the host cell. If co-expression of 4'-phosphopantetheinyl transferase is required for maturation of functional hispidin-synthase, it is also introduced into the cell under control of any suitable constitutive or inducible promoter. When adding a suitable preluciferin precursor to the cells (or their extracts) the detectable luminescence appears, it indicates a simultaneous activation of all three promoters.

The combinations are also applicable at producing transgenic luminous organisms. In preferred embodiments the transgenic organisms are obtained from the organisms , which wild type is not capable of bioluminescence. Nucleic acids, coding target proteins, are introduced into a transgenic organism as a part of expression cassette or vector, which exist in the organism as extrachromosomal elements, or are integrated into the organism genome, as described above in "Transgenic Organisms" section, and promote expression of target proteins. Transgenic organisms of the invention are different in that they express at least hispidin hydroxylase, except for luciferase, which substrate is fungal luciferin. In preferred embodiments they also express hispidin-synthase. In other preferred embodiments they also express PKS. In other preferred embodiments they also express PKS. In some embodiments they also express coumarate-CoA ligase. It is known that endogenous coumarate-CoA ligase is present in many plant organisms, therefore, its additional introduction is carried out in cases, when endogenous coumarate-CoA ligase is absent.

In some embodiments they also express caffeylpyruvate hydrolase. In contrast to the organisms expressing only luciferase, the transgenic organisms, obtained by using nucleic acids of the invention, acquire the ability to emit luminescence in the presence of preluciferins and/or preluciferin precursors - 3-arylacrylic acids (prevalently, caffeic acid) - which are the cheapest and the most stable substrate for obtaining bioluminescence, which could be added to water for plant watering, or to microorganism culture medium, or to feed or to animal (e.g. fish) habitat. Bioluminescent transgenic organisms (plants, or animals, or fungi) are applicable as luminescence sources and also they are used for ornamental purposes. Bioluminescent transgenic organisms, cells and cell cultures could also be used in different screenings, where bioluminescence intensity is changed depending on external influence. For example, they could be used at analysis of different factors effect on activity of promoters controlling expression of exogenous nucleic acids.

Autonomously bioluminescent transgenic organisms, which are also provided by this invention, are of special interest.

In some embodiments the said organisms have at least one 3-arylacrylic acid, as a metabolite, with the structural formula 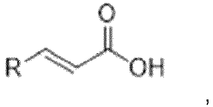 where R is aryl or heteroaryl.

higher and lower plants, including flowering plants and mosses could be mentioned as non-limiting examples. In order to obtain autonomously luminescence-producing transgenic plants, the nucleic acids, coding hispidin hydroxylase, hispidin synthase and luciferase able to oxidize fungal luciferin with luminescence emission, and able to express the corresponding enzymes, are introduced into these plants. Since plants normally comprise endogenous 4'-phosphopantetheinyl transferase, additional introduction of nucleic acid, coding this enzyme to obtain autonomously luminescence-producing plants, is generally not required.

In some embodiments the organisms, which do not naturally produce 3-arylacrylic acids, are used to obtain autonomously bioluminescent transgenic organisms. The examples of such organisms are animals and a variety of microorganisms, e.g. yeasts and bacteria. In this case, nucleic acids, capable of expression, coding enzymes promoting 3-arylacrylic acid biosynthesis from the cell metabolites, for example, caffeic acid from tyrosine, are additionally introduced into organisms to obtain autonomous bioluminescence. If necessary, nucleic acid coding 4'-phosphopantetheinyl transferase is also introduced into organisms.

In some embodiments to obtain autonomously luminescence-producing organisms, the nucleic acids, able to express corresponding enzymes, coding PKS, hispidin hydroxylase and luciferase able to oxidize fungal luciferin with luminescence emission, are introduced into these organisms. In preferred embodiments the said cells, tissues and organisms comprise sufficient amount of caffeyl-CoA and malonyl-CoA to carry out hispidin synthesis.

In cases, when transgenic organism does not produce sufficient amount of caffeyl-CoA during normal metabolic processes, the nucleic acid coding coumarate-CoA ligase, and also, if necessary, enzymes of caffeic acid biosynthesis from tyrosine, is also introduced into the said cells or organisms.

In preferred embodiments the combination of nucleic acids for obtaining autonomously bioluminescent cells or transgenic organisms also comprises a nucleic acid coding caffeylpyruvate hydrolase. As demonstrated in the experimental part below, caffeylpyruvate hydrolase expression results in increasing bioluminescence intensity of autonomously bioluminescent cells or transgenic organisms. In preferred embodiments bioluminescence intensity increases at least 1.5 times, prevalently at least 2 times, normally at least 5 times, e.g. 7-9 times, e.g. 8 or more times.

Autonomously bioluminescent transgenic organisms (plants, or animals, or fungi) and also cells and cell structures are different from transgenic organisms, cells and cell cultures expressing luciferase only and known in the art, in that no exogenous adding of luciferin or its precursor is required for their luminescence.

In some embodiments instead of combination of nucleic acids coding hispidin hydroxylase and luciferase the nucleic acid coding fusion protein of these two enzymes is used. It is obvious to those skilled in the art that the said fusion protein and the combination of nucleic acids coding hispidin hydroxylase and luciferase are interchangeable objects in all methods of use. It is also obvious that on the basis of the nucleic acids of the invention there could be produced other fusion proteins, which will retain properties of fusion partners; such fusion proteins and nucleic acids coding them could be used without limitation instead of combinations of individual proteins and nucleic acids.

In all applications and methods described above the nucleic acids could be in the form of expression cassettes, which could be used to promote the coding sequence expression in a host cell. Nucleic acid could be introduced into a host cell as a part of the vector for expression in suitable host cell or not including it into the vector, for example, it could be integrated into a liposome or viral particle. Alternatively, the purified molecule of nucleic acid could be integrated directly into the host cell using suitable means, e.g. by direct endocytic uptake. Gene construct could be introduced directly into the host organism cells (e.g. plant) by transfection, infection, microinjection, cell fusion, protoplast fusion, using microparticle bombardment or by means of "gene gun" (gun for shooting with microparticles carrying gene constructs).

Application of polyclonal and monoclonal antibodies of the invention is also provided. They are applied in staining tissues, cells, or organisms to localize expressed or natural hispidin hydroxylases, hispidin synthases and caffeylpyruvate hydrolases of the invention. Methods for staining by means of specific antibodies are well known in the art and described, for example, in [V.L. Bykov Cytology and general histology]. Direct immunohistochemical technique is based on the reaction of specific binding labeled antibodies directly with detectable substance, indirect immunohistochemical technique is based on that unlabeled primary antibodies are bound with detectable substance and then they are detected by means of secondary labeled antibodies, provided that, the primary antibodies are antigens for secondary antibodies. Antibodies are also applicable for stopping enzymatic reaction. Contact of antibody with specific binding partner results in inhibiting the enzymatic reaction. Antibodies are also applicable in methods for purification of recombinant and natural proteins of the invention by affinity chromatography. Affinity chromatography techniques are known in the art and described, for example, in Ninfa et al (2009). Fundamental Laboratory Approaches for Biochemistry and Biotechnology (2 ed.). Wiley. p. 133.; Cuatrecasas (1970). JBC. Retrieved November 22, 2017].

### Sets and products

The next embodiment of the invention is a product, which includes the above described hispidin hydroxylase, or hispidin synthases, or caffeylpyruvate hydrolases, or nucleic acid coding the above enzyme, preferably with the elements for promoting target protein expression in host cell, e.g. expression vector or cassette, comprising nucleic acid coding the target protein. Alternatively, nucleic acids could comprise flanking sequences for its incorporation into the target vector. Nucleic acids could be included in promoter-free vectors intended for easy cloning of target regulatory elements. Recombinant proteins could be lyophilized or dissolved in a buffer solution. Nucleic acids could be lyophilized or precipitated in an alcoholic solution or dissolved in water or buffer solution.

In some embodiment the product includes cells expressing one or several above nucleic acids.

In some embodiment the product includes a transgenic organism expressing one or several above nucleic acids.

In some embodiment the product includes antibodies for staining and/or inhibition and/or affinity chromatography of the above enzymes.

The product is a container with a label and instructions for use attached thereto. The acceptable containers are, for example, bottles, ampoules, glass tubes, syringes, cell plates, Petri dishes, etc. The container could be made of different materials, such as glass or polymer materials. Selection of suitable container is obvious to those skilled in thee art.

Besides, the product could include other products required commercially or from a consumer point of view, e.g.: reaction buffer or components for its preparation, buffer for dilution and/or solution and/or storage of proteins and nucleic acids, or components for its preparation, deionized water, secondary antibodies to specific antibodies of the invention, cell culture medium or components for its preparation, nutrition for transgenic organism.

The products also include instructions for implementation of the proposed methods. The instructions could be in different forms, provided that, one or several such forms could be attached to the product, e.g. the instruction could be a file in electronic format and/or on paper.

The invention also relates to the kits which could be applied for different purposes. The kit could include a combination of proteins of the invention or combination of nucleic acids of the invention, preferably with the elements for promoting target protein expression in host cell, e.g. expression vector or cassette, comprising nucleic acid coding the target protein. In some embodiments the kit could also comprise a nucleic acid coding luciferase, able to oxidize fungal luciferin with luminescence emission. In some embodiments the kit could also comprise nucleic acids coding enzymes involved in biosynthesis of caffeic acid from tyrosine. In some embodiments the kit could also comprise a nucleic acid coding 4'-phosphopantetheinyl transferase. In some embodiments the kit could also comprise a nucleic acid coding PKS. In some embodiments the kit could also comprise a nucleic acid coding coumarate-CoA ligase.

In some embodiments the kit could also comprise antibodies for purification of recombinant proteins or for staining the expressed proteins in host cells. In some embodiments the kit could also comprise primers, complementary to regions of the said nucleic acid, for amplification of nucleic acid or its fragment. In some embodiments the kit could also comprise one or several fungal luciferins and/or preluciferins and/or preluciferin precursors. The said compounds could be in the form of dry powder, in the form of organic solvent solution, in the form of water solution. In some embodiment the kit could include cells comprising one or several above nucleic acids. In some embodiments the kit could comprise a transgenic organism of the invention, e.g. producer strain or transgenic autonomously bioluminescent plant. All the kit components are placed into suitable containers. Generally, the kits also include instructions for use.

The following examples are given for better understanding the invention. These examples are given for illustration purposes only and shall not be interpreted as limiting the scope of invention in any way.

All publications, patents and patent applications mentioned in this specification are incorporated herein by reference. Though the above invention has been described in considerable details by illustration and example for purposes of clarity, it is obvious to those skilled in the art, based on the ideas disclosed in this invention, that some alterations and modifications could be introduced without departing from the spirit and scope of the proposed embodiments of the invention.

### Experimental Part (Examples)

### Example 1. Isolation of hispidin hydroxylase sequences

Total RNA from Neonothopanus nambi mycelium was isolated according to the method described in [Chomczynski and Sacchi, Anal. Biochem., 1987, 162, 156-159]. cDNA was amplified by means of SMART PCR cDNA Synthesis Kit (Clontech, USA) according to the manufacturer's protocol. The obtained cDNA was used for amplification of coding sequence of luciferase, which nucleotide and amino acid sequence are shown in SEQ ID NOs: 79, 80. Coding sequence was cloned into pGAPZ vector (Invitrogen, USA) according to the manufacturer's protocol and transformed into *E.coli* competent cells of XL1 Blue strain. Bactria were cultivated on Petri dishes in the presence of antibiotic Zeocin. In 16 hours the colonies were rinsed from the dishes, intensively mixed, and plasmid DNA was isolated from them by means of plasmid DNA isolation kit (Evrogen, Russia). The isolated plasmid DNA was linearized at restriction site AvrII and used for transformation of *Pichia pastoris* GS115 cells. Electroporation was carried out according to the method, using lithium acetate and dithiothreitol, described in [Wu and Letchworth, Biotechniques, 2004, 36:152-4]. Electroporated cells were dispersed in Petri dishes with RDB medium, comprising 1 M of sorbitol, 2% (weight/volume) of glucose, 1.34 % (weight/volume) of yeast nitrogen base (YNB), 0.005 % (weight/volume) of amino acids mixture, 0.00004 % (weight/volume) of biotin and 2 % (weight/volume) of agar. The obtained colonies were sprayed with 3-hydroxyhispidin solution, detecting luciferase presence in cells by occurrence of luminescence. Luminescence emitted by colonies was detected by means of IVIS Spectrum CT (PerkinElmer, USA). Colonies, where luminescence was detected in response to addition of 3-hydroxyhispidin, were selected for further work.

Then, the amplified total cDNA from *Neonothopanus nambi* was cloned into pGAPZ vector and transformed into *E.coli* competent cells of XL1 Blue strain. Bactria were cultivated on Petri dishes in the presence of antibiotic Zeocin. In 16 hours, the colonies were rinsed from the dishes, intensively mixed, and plasmid DNA was isolated from them by means of plasmid DNA isolation kit (Evrogen, Russia). The isolated plasmid DNA was linearized at restriction site AvrII and used for transformation of *Pichia pastoris* GS115 yeast cells, constitutively expressing *Neonothopanus nambi* luciferase. Transformation was carried out by electrocorporation technique, as described above. The cells were dispersed in Petri dishes with RDB medium, comprising 1 M of sorbitol, 2% (weight/volume) of glucose, 1.34 % (weight/volume) of yeast nitrogen base (YNB), 0.005 % (weight/volume) of amino acids mixture, 0.00004 % (weight/volume) of biotin and 2% (weight/volume) of agar. Diversity in resulting library of *Neonothopanus nambi* cDNA in yeasts was about one million of clones. The obtained colonies were sprayed with hispidin solution, detecting hispidin hydroxylase presence in cells by occurrence of luminescence. Luminescence emitted by colonies was detected by means of IVIS Spectrum CT (PerkinElmer, USA). Cells expressing luciferase only and wild yeast cells were used as negative control. When screening the library, the colonies, where luminescence was detected, were selected and used for PCR as a matrix with standard plasmid primers. PCR products were sequenced by Sanger method to determine sequence of the expressed gene. The obtained sequence of hispidin hydroxylase nucleic acid is shown in SEQ ID NO: 1. The amino acid sequence coded by it is shown in SEQ ID NO: 2.

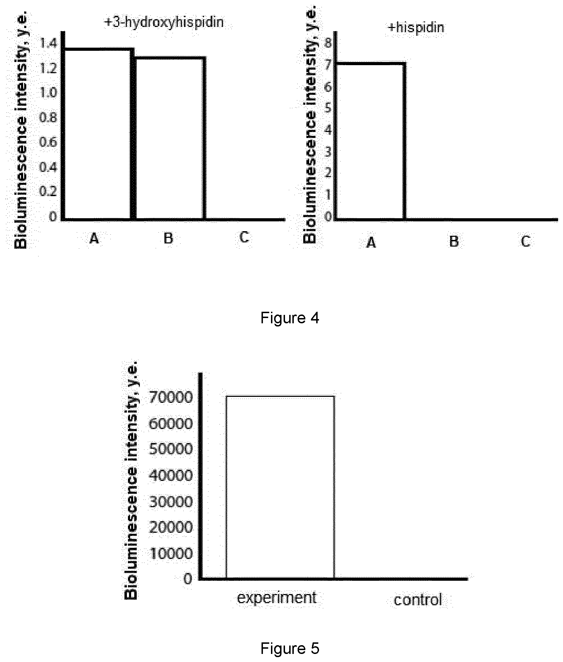

Fig. 4 illustrates luminescence of *Pichia pastoris* cells expressing hispidin hydroxylase and luciferase or luciferase only, or wild yeasts when spraying the colonies with 3-hydroxyhispidin (luciferin) and hispidin (preluciferin). The data demonstrate that luciferin is produced in cells in the presence of hispidin hydroxylase.

At the next step genomic DNA was isolated from the fungi *Armillaria fuscipes, Armillaria gallica, Armillaria ostoyae, Armillaria mellea, Guyanagaster necrorhiza, Mycena citricolor, Mycena chlorophos, Neonothopanus nambi, Neonothopanus gardneri, Omphalotus olearius and Panellus stipticus,* and whole-genome sequencing was performed by Illumina HiSeq technique (Illumina, USA) according to the manufacturer's recommendations. Sequencing results were used for prediction of hypothetical protein amino acid sequences and to search for hispidin hydroxylase homologs from *Neonothopanus nambi.* Homologs search was carried out by means of a software provided by National Center for Biotechnology Information. Search for amino acid sequences in the data of fungal genome sequencing in NCBI Genbank database. Standard search parameters blastp were used at search. As result, there were identified the sequences of hispidin hydroxylase homologs from *Neonothopanus nambi* - in *Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos.*

Nucleotide and amino acid sequences of hispidin-synthase homologs of *Neonothopanus nambi* are shown in SEQ ID NOs: 3-28.

All identified enzymes are substantially identical to each other. Degree of amino acid sequences identity is shown in Table 4.

**Table 4. Percent identity of hispidin hydroxylase full-length natural protein amino acid sequences.**

| | **SEQ ID NO:2** | **SEQ ID NO:4** | **SEQ ID NO:6** | **SEQ ID NO:16** | **SEQ ID NO:8** | **SEQ ID NO:14** | **SEQ ID NO:20** | **SEQ ID NO:22** | **SEQ ID NO:24** | **SEQ ID NO:26** | **SEQ ID NO:28** |
|---|---|---|---|---|---|---|---|---|---|---|---|
| **SEQ ID NO:2** | 100 | 61 | 71 | 72 | 70 | 89 | 72 | 73 | 73 | 73 | 69 |
| **SEQ ID NO:4** | 61 | 100 | 50 | 48 | 48 | 63 | 49 | 51 | 50 | 50 | 45 |
| **SEQ ID NO:6** | 71 | 50 | 100 | 69 | 69 | 71 | 85 | 86 | 87 | 88 | 67 |
| **SEQ ID NO:6** | 72 | 48 | 69 | 100 | 76 | 72 | 71 | 72 | 72 | 72 | 71 |
| **SEQ ID NO:18** | 72 | 48 | 70 | 99 | 76 | 72 | 71 | 73 | 73 | 73 | 71 |
| **SEQ ID NO:8** | 70 | 48 | 69 | 76 | 100 | 71 | 70 | 70 | 71 | 71 | 73 |
| **SEQ ID NO:10** | 70 | 48 | 69 | 76 | 99 | 71 | 70 | 70 | 71 | 71 | 73 |
| **SEQ ID NO:14** | 89 | 63 | 71 | 72 | 71 | 100 | 72 | 75 | 73 | 74 | 71 |
| **SEQ ID NO:20** | 72 | 49 | 85 | 71 | 70 | 72 | 100 | 91 | 92 | 93 | 68 |
| **SEQ ID NO:22** | 73 | 51 | 86 | 72 | 70 | 75 | 91 | 100 | 93 | 93 | 69 |
| **SEQ ID NO:24** | 73 | 50 | 87 | 72 | 71 | 73 | 92 | 93 | 100 | 95 | 69 |
| **SEQ ID No:26** | 73 | 50 | 88 | 72 | 71 | 74 | 93 | 93 | 95 | 100 | 69 |
| **SEQ ID NO:28** | 69 | 45 | 67 | 71 | 73 | 71 | 68 | 69 | 69 | 69 | 100 |

From *Panellus stipticus* and *Mycena citricolor* there were isolated several highly homologous hispidin hydroxylase amino acid sequences characterized by single amino acid substitutions. Their nucleotide and amino acid sequences are shown in SEQ ID NOs 7-13 (*Panellus stipticus*) and SEQ ID NOs 15-18 (*Mycena citricolor*)*.* Further study of the said proteins' properties had not detect influence of these substitutions on enzymatic properties.

Coding sequences of the detected homologs (SEQ ID NOs: 3-28) were cloned and transformed into *Pichia pastoris* GS115 cells, constitutively expressing *Neonothopanus nambi* luciferase according to the above protocol. The obtained colonies were sprayed with hispidin solution, detecting hispidin hydroxylase presence in cells by occurrence of luminescence. Luminescence emitted by colonies was detected by means of IVIS Spectrum CT (PerkinElmer, USA). All colonies, expressing the test genes (SEQ ID NOs: 3,5,7,9,11,13,15,17,19, 21, 23, 25,27), produced luminescence 1000-100000000 times more at spraying with hispidin solution, than control cells, that confirms a capability of enzymes coded by tested genes to catalyze hispidin transformation into 3-hydroxyhispidin (fungal luciferin).

Structural analysis of detected enzymes amino acid sequences was done, Analysis performed by means of software SMART (Simple Modular Architecture Research Tool), available on the Internet at the website http://smart.embl-heidelberg.de [Schultz et al., PNAS 1998; 95: 5857-5864; Letunic I, Doerks T, Bork P Nucleic Acids Res 2014; doi:10.1093/nar/gku949] have revealed that all detected proteins comprise FAD/NAD(P)-binding domain, IPR002938 - code of InterPro public database available on the Internet at the website http://www.ebi.ac.uk/interpro). This domain is involved in binding FAD and NAD in some enzymes, in particular, monooxygenases - the representatives of a large enzyme family, adding the hydroxyl group to substrate, and multiple organisms found in metabolic pathways. The detected hispidin hydroxylases, except for FAD/NAD(P)-binding domain, comprise N- and C-terminal amino acid sequences operatively bound with it (Fig. 1). Using multiple alignment and comparison of amino acid sequences of the detected hispidin hydroxylases (Fig. 1) it was revealed that they comprise several conservative amino acid motifs (consensus sequences) typical of this enzyme group only (SEQ ID NOs: 29-33). Consensus sites inside amino acid sequences are operatively bound via amino acid inserts.

### Example 2. Expression of hispidin hydroxylase and fungal luciferase in mammal cells and their combined use for cell labeling

Coding sequences of hispidin hydroxylase and luciferase from *Neonothopanus nambi,* obtained according to Example 1, were optimized (humanized) for expression in mammal cells. Optimized nucleic acids (SEQ ID NOs: 99 and 100) were obtained synthetically. Coding sequence of hispidin hydroxylase was cloned into pmKate2-keratin vector (Evrogen, Russia), using restriction sites Nhel and Notl instead of the sequence coding fusion protein mKate2-keratin. Luciferase sequence was amplified by PCR, treated by restriction endonucleases Nhel and EcoRV (New England Biolabs, Ipswich, MA) and ligated into lentiviral vector pRRLSIN.cPPT.EF1. Plasmid DNA was purified by means of plasmid DNA purification kits (Evrogen). Plasmid DNA, comprising luciferase gene, was used for development of stably expressing lines HEK293NT. Vector particles were obtained by calcium-phosphate transfection (Invitrogen, Carlsbad, CA) of HELK293T cells according to the protocol provided at the manufacturer website. 1,500,000 cells were put in 60 mm cultural dish 24 hours before transfection. About 4 and 1.2 µg of packaging plasmids pR8.91 and pMD.G, and also 5 µg of transfer plasmid, comprising luciferase sequence, were used for transfection. Viral particles were harvested 24 hours after transfection, 10 times concentrated and used for transduction of HEK293NT cells. About 100% of HEK293NT cells stably expressed *Neonothopanus nambi*luciferase.

The obtained cells were subjected to re-transfection by the vector comprising coding sequence of hispidin hydroxylase using transfection reagent FuGENE HD (Promega, USA) according to the manufacturer's protocol. 24 hours after transfection hispidin at concentration of 800 µg/ml was added to the medium and cell luminescence was detected by means of IVIS Spectrum CT (PerkinElmer). The obtained cells emitted luminescence with intensity more than by two orders of magnitude exceeding the signal outgoing from untransfected control cells (Fig. 5).

The cells were visualized in transmitted light in green luminescence detection channel. Expression of *Neonothopanus nambi* hispidin hydroxylase in human cells resulted in occurrence of distinct luminous signal in green spectrum in the presence of hispidin enabling to distinguish transfected cells from untransfected ones.

### Example 3. Use of hispidin hydroxylase with hispidin analogues in cell lysate

HEK293NT cells, expressing luciferase and hispidin hydroxylase of *Neonothopanus nambi,* obtained according to Example 2, were rinsed from Petri dishes 24 hours after transfection with Versene solution laced with 0.025% of trypsin, the medium was replaced by phosphate-buffered saline with pH 8.0 by centrifugation, the cells were resuspended, lysed by ultrasound in Bioruptor (Diagenode, Belgium) within 7 minutes at 0°C in conditions recommended by the manufacturer, and 1 mM of NADPH (Sigma-Aldrich, USA), and also hispidin or one of its analogues were added to the medium: (E)-4-hydroxy-6-(4-hydroxystyryl)-2H-pyran-2-one, (E)-6-(2-(1H-indol-3-yl)vinyl)-4-hydroxy-2H-pyran-2-one, (E)-6-(2-(1,2,3,5,6,7-hexahydropyrido[3,2,1-ij]quinolin-9-yl)vinyl)-4-hydroxy-2H-pyran-2-one, E)-6-(4-(diethylamino)styryl)-4-hydroxy-2H-pyran-2-one, or (E)-4-hydroxy-6-(2-(6-hydroxynaphthalene-2-yl)vinyl)-2H-pyran-2-one at concentration of 660 µg/ml. Bioluminescence spectra were detected by spectrofluorometer Varian Cary Eclipse. Luminescence in lysates was observed at addition of all said hispidin functional analogues. Depending on luciferin used the expected displacement of luminescence peak was observed.

### Example 4. Obtaining recombinant hispidin hydroxylases

Polyhistidine sequence (His tag) was operatively attached to 5'-end of nucleic acids coding hispidin-3-hydroxylases and luciferase from *Neonothopanus nambi,* obtained according to the examples 1 and 2. The obtained structures were cloned into pET-23 vector by means of restriction endonucleases BamHI and HindIII. The vector was used for transformation of *Escherichia coli* cells of BL21-DE3 strain. The cells were dispersed in Petri dishes with LB medium, comprising 1.5% of agar, 100 µg/ml of ampicillin, and incubated overnight at 37°C. Then, *Escherichia coli* colonies were transferred into 4 ml of liquid LB medium laced with ampicillin, incubated overnight at 37°C with fluctuation. 1 ml of overnight culture was transferred into 100 ml of Overnight Express Autoinduction medium (Novagen), where ampicillin was preliminarily added to. The culture was grown at 37°C within 2.5 hours until reaching optical density of 0.6 OE at 600 nm, and then it was grown at room temperature within 16 hours. Then, the cells were pelleted at 4500 rpm within 20 minutes in centrifuge Eppendorf 5810R, resuspended in 35 ml of the buffer (50 mM of Tris HCI pH 8.0, 150 mM of NaCl). The cells were sonicated and pelleted again. TALON resin metal affinity chromatography (Clontech, USA) was used for purification of recombinant proteins. Presence of the expected recombinant product was confirmed by electrophoresis.

Aliquots of isolated recombinant hispidin hydroxylases were used for testing functionality and stability. For determination of functionality 15 µl of isolated recombinant protein solution were put into a glass tube, comprising 100 µl of the buffer (0.2 M of Na-phosphate buffer, 0.5 M of Na₂SO₄, 0.1% dodecylmaltoside (DDM) pH 8.0), 0.5 µl of purified recombinant luciferase of *Neonothopanus nambi,* 1 mM of NADPH and 0.2 µM of hispidin. The glass tube was placed into a luminometer. Activity of isolated recombinant proteins resulted in luminescence at combination with hispidin and its analogues described in Example 3, in the presence of *Neonothopanus nambi* luciferase. In all cases the luminescence intensity when using hispidin was the highest when using *Neonothopanus nambi* hispidin hydroxylase and the lowest when using *Armillaria mellea* hispidin hydroxylase.

### Example 5. Obtaining of 3-hydroxyhispidin, (E)-3,4-dihydroxy-6-styryl-2H-pyran-2-one and (E)-3,4-dihydroxy-6-(4-hydroxystyryl)-2H-pyran-2-one using recombinant hispidin hydroxylase

The isolated recombinant hispidin hydroxylase from *Neonothopanus nambi,* obtained according to Example 4, was added to reaction mixtures comprising 1 mM of NADPH and 0.2 µM of hispidin, (E)-4-hydroxy-6-styryl-2H-pyran-2-one or (E)-4-hydroxy-6-(4-hydroxystyryl)-2H-pyran-2-one in 100 µl of the buffer (0.2 M of Na-phosphate buffer, 0.5 M of Na₂SO₄, 0.1% dodecylmaltoside (DDM) pH 8.0). In 30 minutes, the reaction mixture was analyzed by HPLC using synthetic luciferins as standards. Chromatography demonstrated occurrence of peaks corresponding to 3d position hydroxylated derivatives: 3-hydroxyhispidin, (E)-3,4-dihydroxy-6-styryl-2H-pyran-2-one and (E)-3,4-dihydroxy-6-(4-hydroxystyryl)-2H-pyran-2-one.

### Example 6. Bioluminescence detection by hispidin hydroxylase and luciferase fusion protein

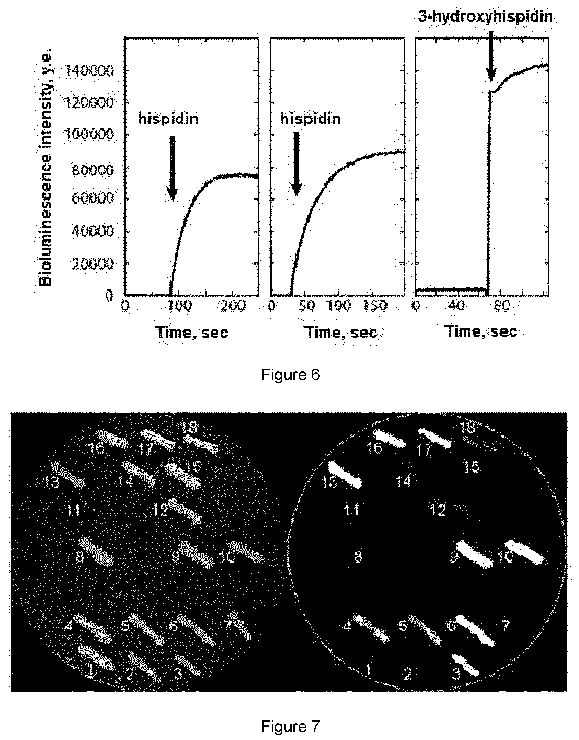

Humanized DNA sequences coding hispidin-hydroxylase and luciferase of *Neonothopanus nambi,* obtained according to Example 2, were operatively cross-linked to each other by flexible short peptide linker with amino acid sequence GGSGGSGGS (SEQ ID NOs:115). Nucleotide and amino acid sequences of the obtained fused protein are shown in SEQ ID NO 101 and 102. Nucleic acid coding fused protein was cloned into pEGFP-N1 vector (Clontech, USA) instead of EGFP gene under Cytomegaloviral promoter control. The obtained structure was transfected into HEK293T cells. Analogous vectors, comprising individual genes of hispidin hydroxylase and luciferase, were also co-transfected. Transfection was performed by transfection agent FuGENE HD (Promega, USA) according to the manufacturer's protocol. 24 hours after transfection 1 million of cells were resuspended in 0.5 ml of PBS, and luminescence without adding hispidin and with addition of hispidin (10 µg per 1 million of cells) was recorded by luminometer. Addition of hispidin caused cell luminescence in green spectrum (Fig. 6). Addition of 3-hydroxyhispidin also caused bioluminescent signal. Expression of hispidin hydroxylase and luciferase fusion proteins enables to use more stable luciferin precursors (hispidin, bisnoryangonin and others) instead of one luciferase for cell bioluminescent labeling and it does not require co-transfection of two nucleic acids into cells.

### Example 7. Preparation of polyclonal antibodies

Coding sequences of hispidin hydroxylases of *Neonothopanus nambi* (SEQ ID NO: 1) and *Armillaria mellea* (SEQ ID NO: 19) were synthetically obtained in the form of linear double-stranded DNA and cloned into expression vectors pQE-30 (Qiagen, Germany) in such a way, that recombinant proteins comprised histidine tag at N-terminal. After expression in E. *coli,* recombinant proteins were purified by metal affinity resin TALON (Clontech, USA) in denaturating conditions. Purified protein products emulsified in Freund adjuvant were used for four rabbit immunizations at month intervals. Rabbit blood was sampled the tenth or eleventh day after immunizations. Activity of the obtained polyclonal antisera was tested by ELISA and Western immunoblotting methods on the panel of purified recombinant hispidin hydroxylases obtained according to Example 4.

Antibodies, obtained at rabbit immunization with protein from *Neonothopanus nambi,* demonstrated activity against denatured and nondenaturated hispidin hydroxylase of *Neonothopanus nambi* and against denatured hispidin hydroxylase of *Neonothopanus gardneri.*

Antibodies, obtained at rabbit immunization with protein from *Armillaria mellea* were active against denatured and nondenaturated hispidin hydroxylase of Armillaria mellea, *Armillaria gallica, Armillaria ostoyae and Armillaria fuscipes.*

### Example 8. Obtaining of transgenic plants expressing Neonothopanus nambi hispidin hydroxylase and luciferase

Coding sequences of *Neonothopanus nambi* hispidin hydroxylase and luciferase were optimized for expression in *Physcomitrella patens* moss cells. Then, *in silico* there was created an expression cassette comprising promoter of rice aktl gene, human cytomegalovirus 5'-untranslated region coding hispidin hydroxylase sequence optimized for expression in plant cells (SEQ ID NO 103), terminating codon, *Agrobacterium osc* gene terminator sequence, rice ubiquitin promoter, coding sequence of *Neonothopanus nambi* luciferase (SEQ ID NO 112) optimized for expression in moss cells, *Agrobacterium tumefaciens* nos gene terminator.

The obtained sequence was synthesized in such a way, that all said fragments appeared to be operatively cross-linked to each other, and cloned by Gibson assembly technique [Gibson et al., Nat Methods, 2009, 6:343-5] into expression vector pLand#1 (Institut Jean-Pierre Bourgin, France), between DNA fragments coincident with locus of *Physcomitrella patens* moss genomic DNA between sequences of highly expressed moss genes Pp3c16_6440V3.1 and Pp3c16_6460V3.1. The vector pLand#1 also comprised a guide RNA (sgRNA) sequence for Cas9 nuclease, complementary to the region of the same DNA locus.

Plasmid DNA product was co-transformed together with the expression vector comprising Cas9 nuclease sequence under *Arabidopsis thaliana* ubiquitin promoter, into *Physcomitrella patens* moss protoplasts according to the polyethylenglycol transformation protocol described in [Cove et al., Cold Spring Harb Protoc., 2009, 2]. Then protoplasts were incubated in BCD medium within two days under darkroom conditions with fluctuation at 50 rpm to regenerate cell wall. Then protoplasts were transferred to Petri dishes comprising agar and BCD medium and grown at 16 hours lighting within a week. Transformed moss colonies were screened from external genomic primers by PCR to determine the progress of gene construct integration into genome, transferred to fresh Petri dishes and grown in the same lighting conditions within 30 days.

The obtained moss gametophytes were soaked in BCD medium comprising hispidin at concentration of 900 µg/ml, and analyzed by means of IVIS Spectrum In Vivo Imaging System (Perkin Elmer). All analyzed transgenic plant demonstrated bioluminescence with intensity minimum by two orders of magnitude exceeding the signal of control plants expressing luciferase only, incubated in the same solution with hispidin.

### Example 9. Identification of hispidin-synthases and caffeylpyruvate hydrolases

Fungal luciferin precursors, such as hispidin, relate to a large group of chemical compounds - polyketide derivatives. Such compounds could be theoretically obtained from 3-arylacrylic acids, in which aromatic substituent, including aryl or heteroaryl are 3d position substituents. It is known in the art that enzymes involved in synthesis of polyketides and their derivatives are multidomain complexes related to polyketide synthase protein superfamily. At the same time, no polyketide synthase, able to catalyze transformation of 3-arylacrylic acid into substituted 4-hydroxy-2H-pyran-2-one, has been known in the art. Screening of Neonothopanus nambi cDNA library was used to search for target polyketide synthase.

It is known, that to obtain functional polyketide synthases in heterologous expression yeast system, it is required to introduce additionally into the culture a gene expressing 4'-phosphopantetheinyl transferase - enzyme transferring 4-phosphopantetheinyl from coenzyme A to serine in acyl carrier domain of polyketide synthase [Gao Menghao et al., Microbial Cell Factories 2013, 12:77]. NpgA gene of 4'-phosphopantetheinyl transferase from *Aspergillus nidulans* (SEQ ID NOs 104, 105), known in the art, was obtained synthetically and cloned into pGAPZ vector. Plasmid was linearized at restriction site AvrII and used for transformation of *Pichia pastoris* GS115 yeast line constitutively expressing *Neonothopanus nambi,* luciferase and hispidin hydroxylase, obtained according to Example 1. Diversity in resulting library of *Neonothopanus nambi* cDNA in yeasts was about one million of clones.

*Neonothopanus nambi* cDNA library expressed in the said *Pichia pastorisyeast* line was obtained according to the protocol given in Example 1 and was used for identification of hispidin-synthases and caffeylpyruvate hydrolases. The cells were dispersed in Petri dishes with RDB medium, comprising 1 M of sorbitol, 2% (weight/volume) of glucose, 1.34 % (weight/volume) of yeast nitrogen base (YNB), 0.005 % (weight/volume) of amino acids mixture, 0.00004 % (weight/volume) of biotin and 2 % (weight/volume) of agar.

The obtained colonies were sprayed with caffeic acid (potential hispidin precursor) solution, detecting hispidin-synthase presence in cells by occurrence of luminescence. Luminescence emitted by colonies was detected by means of IVIS Spectrum CT (PerkinElmer, USA). Cells expressing only luciferase and hispidin hydroxylase, and wild yeast cells were used as negative control. When screening the library, the colonies, where luminescence was detected, were selected and used for PCR as a matrix with standard plasmid primers. PCR products were sequenced by Sanger method to determine sequence of the expressed gene. The obtained sequence of hispidin-synthase nucleic acid is shown in SEQ ID NO: 34. The amino acid sequence coded by it is shown in SEQ ID NO: 35.

Then, the obtained *Pichia pastoris* yeast line, comprising *Neonothopanus nambi* luciferase, hispidin hydroxylase and hispidin synthase genes integrated into genome, and also NpgA gene of 4'-phosphopantetheinyl transferase from *Aspergillus nidulans,* was used for identification of enzyme catalyzing transformation of oxyluciferin ((2E,5E)-6-(3,4-dihydroxyphenyl)-2-hydroxy-4-oxohexa-2,5-diene acid) into caffeic acid. The cell line was again transformed by linearized plasmid library of *Neonothopanus nambi* genes, which was obtained at the first step of work. The colonies were sprayed with caffeoyl pyruvate solution, detecting target enzyme presence in cells by occurrence of luminescence. Luminescence emitted by colonies was detected by means of IVIS Spectrum CT (PerkinElmer, USA). Cells expressing only luciferase and hispidin hydroxylase, and wild yeast cells were used as negative control. When screening the library, the colonies, where luminescence was detected, were selected and used for PCR as a matrix with standard plasmid primers. PCR products were sequenced by Sanger method to determine sequence of the expressed gene. The obtained sequence of isolated enzyme nucleic acid is shown in SEQ ID NO: 64. The amino acid sequence coded by it is shown in SEQ ID NO: 65. The identified enzyme was called as caffeylpyruvate hydrolase.

### Example 10. Identification of Neonothopanus nambi hispidin-synthase and Neonothopanus nambi caffeylpyruvate hydrolase homologs

Data of whole-genome sequencing from bioluminescent fungi, obtained according to Example 1, were used to search for homologs of *Neonothopanus nambi* hispidin-synthase and caffeylpyruvate hydrolase. Homologs search was carried out by means of a software provided by National Center for Biotechnology Information. Search for amino acid sequences in the data of fungal genome sequencing in NCBI Genbank database. Standard search parameters blastp were used at search.

There were identified the sequences of hispidin-synthase homologs from *Neonothopanus nambi* - in *Armillaria fuscipes, Armillaria mellea, Guyanagaster necrorhiza, Mycena citricolor, Neonothopanus gardneri, Omphalotus olearius, Panellus stipticus, Armillaria gallica, Armillaria ostoyae, Mycena chlorophos.* Their nucleotide and amino acid sequences are shown in SEQ ID NO 36-55. All identified enzymes were substantially identical to each other. Degree of amino acid sequences identity is shown in Table 5.

**Table 5. Percent identity of hispidin-synthase full-length natural protein amino acid sequences.**

| | SEQ ID NO:35 | SEQ ID NO:53 | SEQ ID NO:43 | SEQ ID NO:45 | SEQ ID NO:37 | SEQ ID NO:41 | SEQ ID NO:55 | SEQ ID NO:47 | SEQ ID NO:49 | SEQ ID NO:51 |
|---|---|---|---|---|---|---|---|---|---|---|
| SEQ ID NO:35 | 100 | 56 | 57 | 56 | 51 | 81 | 57 | 50 | 57 | 57 |
| SEQ ID NO:53 | 56 | 100 | 80 | 52 | 45 | 55 | 88 | 46 | 88 | 88 |
| SEQ ID NO:43 | 57 | 80 | 100 | 52 | 47 | 56 | 83 | 45 | 85 | 86 |
| SEQ ID NO:45 | 56 | 52 | 52 | 100 | 53 | 54 | 52 | 54 | 53 | 53 |
| SEQ ID NO:37 | 51 | 45 | 47 | 53 | 100 | 51 | 46 | 51 | 47 | 47 |
| SEQ ID NO:41 | 81 | 55 | 56 | 54 | 51 | 100 | 55 | 50 | 56 | 56 |
| SEQ ID NO:55 | 57 | 88 | 83 | 52 | 46 | 55 | 100 | 46 | 90 | 91 |
| SEQ ID NO:47 | 50 | 46 | 45 | 54 | 51 | 50 | 46 | 100 | 46 | 46 |
| SEQ ID NO:49 | 57 | 88 | 85 | 53 | 47 | 56 | 90 | 46 | 100 | 95 |
| SEQ ID NO:51 | 57 | 88 | 86 | 53 | 47 | 56 | 91 | 46 | 95 | 100 |

From *Panellus stipticus* there were isolated two highly homologous hispidin-synthase amino acid sequences characterized by single amino acid substitution. Their nucleotide and amino acid sequences are shown in SEQ ID NO 36-39.

The identified enzymes were tested for capability to transform caffeic acid into hispidin using technique described in Example 9.

Multiple alignment of identified protein amino acid sequences enabled to identify several highly homologous fragments of amino acid sequence typical of this enzyme group. Consensus sequences for these fragments are shown in SEQ ID NOs: 70-77. The said sequences are separated by long amino acid sequences as shown in Fig. 2.

*Neonothopanus nambi* caffeylpyruvate hydrolase homolog sequences were identified in *Neonothopanus gardneri, Armillaria mellea, Armillaria fuscipes, Armillaria gallica, Armillaria ostoyae.* Nucleotide and amino acid sequences of the identified homologs are shown in SEQ ID NOs: 66-75. The identified enzymes were tested for capability to transform caffeoyl pyruvate into caffeic acid using technique described in Example 9.

All identified enzymes are substantially identical to each other and have a length of 280-320 amino acids. Degree of amino acid sequences identity is shown in Table 6.

**Table 6. Percent identity of caffeoyl pyruvate hydrolase full-length natural protein amino acid sequences.**

| | SEQ ID NO:65 | SEQ ID NO:73 | SEQ ID NO:75 | SEQ ID NO:67 | SEQ ID NO:69 | SEQ ID NO:71 |
|---|---|---|---|---|---|---|
| SEQ ID NO:65 | 100 | 64 | 64 | 64 | 64 | 64 |
| SEQ ID NO:73 | 64 | 100 | 92 | 62 | 96 | 95 |
| SEQ ID NO:75 | 64 | 92 | 100 | 62 | 90 | 90 |
| SEQ ID NO:67 | 64 | 62 | 62 | 100 | 60 | 61 |
| SEQ ID NO:69 | 64 | 96 | 90 | 60 | 100 | 97 |
| SEQ ID NO:71 | 64 | 95 | 90 | 61 | 97 | 100 |

Analysis performed by means of software SMART (Simple Modular Architecture Research Tool), available on the Internet at the website http://smart.embl-heidelberg.de [Schultz et al., PNAS 1998; 95: 5857-5864; Letunic I, Doerks T, Bork P Nucleic Acids Res 2014; doi:10.1093/nar/gku949] revealed that all detected proteins comprise a fumarylacetoacetase domain (EC 3.7.1.2) of about 200 amino acids long, located closer to C-terminal, however, conserved region starts approximately from 8 amino acid according to numbering of *Neonothopanus nambi* caffeylpyruvate hydrolase amino acids. Multiple alignment enabled to identify consensus sequences (SEQ ID NOs 76-78), typical of this protein group, separated by amino acid inserts with lower identity. Position of consensus sequences is shown in (Fig. 3).

### Example 11. Obtaining of recombinant hispidin-synthases and caffeylpyruvate hydrolase and their use for obtaining bioluminescence

Polyhistidine (His tag) coding sequence was operatively attached to 5'-ends of nucleic acids coding hispidin-synthase and caffeylpyruvate hydrolase of *Neonothopanus nambi,* obtained according to Example 9, and the obtained structures were cloned into pET-23 vector by means of restriction endonucleases Notl and Sacl. The vectors were used for transformation of *Escherichia coli* cells of BL21-DE3-codon+ strain, performed by electroporation. The trnsformed cells were dispersed in Petri dishes with LB medium, comprising 1.5% of agar, 100 µg/ml of ampicillin, and incubated overnight at 37°C. Then, *Escherichia coli* colonies were transferred into 4 ml of liquid LB medium comprising 100 µg/ml of ampicillin, incubated overnight at 37°C with fluctuation. 1 ml of overnight culture was transferred into 200 ml of Overnight Express Autoinduction medium (Novagen), where ampicillin was preliminarily added to. The culture was incubated at 37°C within 3 hours until reaching optical density of 0.6 OE at 600 nm, and then it was incubated at room temperature within 16 hours. Then, the cells were pelleted at 4500 rpm within 20 minutes in centrifuge Eppendorf 5810R, resuspended in 20 ml of the buffer (50 mM of Tris HCI pH 8.0, 150 mM of NaCl), lysed by ultrasound in Bioruptor (Diagenode, Belgium) within 7 minutes at 0°C in conditions recommended by the manufacturer and pelleted again. Protein was obtained from lysate by Talon resin affinity chromatography (Clontech, USA). Presence of the expected recombinant product was confirmed by electrophoresis as bands of the expected length were available.

Aliquots of isolated recombinant proteins were used for testing functionality and stability.

For determination of hispidin synthase functionality 30 µl of isolated recombinant protein solution were put into a glass tube, comprising 100 µl of the buffer (0.2 M of Na-phosphate buffer, 0.5 M of Na₂SO₄, 0.1% dodecylmaltoside (DDM) pH 8.0, all components - Sigma-Aldrich, USA), 0.5 µl of purified recombinant luciferase of Neonothopanus nambi, obtained according to Example 4, 1 mM of NADPH (Sigma-Aldrich, USA), 15 µl of purified recombinant hispidin hydroxylase of *Neonothopanus nambi,* obtained according to Example 4, 10 mM of ATP (ThermoFisher Scientific, USA), 1 mM of CoA (Sigma-Aldrich, USA), 1 mM of malonyl-CoA (Sigma-Aldrich, USA). The glass tube was placed into a luminometer GloMax 20/20 (Promega, USA). The reaction mixtures demonstrated bioluminescence at adding of 20 µM of caffeic acid into the solution (Sigma-Aldrich, USA). Maximum emission of the emitted luminescence was 520-535 nm.

For determination of caffeylpyruvate hydrolase functionality 10 µl of isolated recombinant protein solution were put into a glass tube, comprising 100 µl of the buffer (0.2 M of Na-phosphate buffer, 0.5 M of Na₂SO₄, 0.1% dodecylmaltoside (DDM) pH 8.0, 0.5 µl of luciferase of *Neonothopanus nambi,* 1 mM of NADPH (Sigma-Aldrich, USA), 15 µl of hispidin hydroxylase 10 mM of ATP (ThermoFisher Scientific, USA), 1 mM of CoA (Sigma-Aldrich, USA), 1 mM of malonyl-CoA (Sigma-Aldrich, USA), 30 µl of purified recombinant hispidin synthase. The glass tube was placed into a luminometer GloMax 20/20 (Promega, USA). Bioluminescence of the reaction mixture was detected at adding of 25 µM of caffeoyl pyruvate into the solution, being indicative of the test enzyme capability to decompose caffeoyl pyruvate to caffeic acid. Maximum emission of the emitted luminescence was 520-535 nm.

The obtained enzymes were used for obtaining luminescence (bioluminescence) in reaction with *Neonothopanus nambi* luciferase and hispidin hydroxylase, obtained according to Example 4. 5 µl of each isolated recombinant protein solution were put into a glass tube, comprising 100 µl of the buffer (0.2 M of Na-phosphate buffer, 0.5 M of Na₂SO₄, 0.1% dodecylmaltoside (DDM) pH 8.0), 1 mM of NADPH (Sigma-Aldrich, USA), 10 mM of ATP (ThermoFisher Scientific, USA), 1 mM of CoA (Sigma-Aldrich, USA), 1 mM of malonyl-CoA (Sigma-Aldrich, USA) and 0.2 µM of one of 3-arylacrylic acids: paracoumaric acid (Sigma-Aldrich, USA), cinnamic acid (Sigma-Aldrich, USA) or ferulic acid (Abcam, USA). In the other experiment instead of substituted 3-arylacrylic acid the analogues of fungal oxyluciferin - (2E,5E)-2-hydroxy-6-(4-hydroxyphenyl)-4-oxohexa-2,5-diene, (2E,5E)-2-hydroxy-4-oxo-6-phenylhexa-2,5-diene, or (2E,5E)-2-hydroxy-6-(4-hydroxy-3-methoxyphenyl)-4-oxohexa-2,5-diene acids - also at concentration of 0.2 µM were put into a glass tube. The glass tubes were placed into a luminometer. Activity of the isolated recombinant proteins resulted in luminescence in each of the described reactions.

### Example 13. Obtaining hispidin from caffeic acid

Expression cassette, comprising nucleic acid coding *Neonothopanus nambi* hispidin-synthase (SEQ ID NOs 34, 35), under control of J23100 promoter, and expression cassette, comprising NpgA gene of 4'-phosphopantetheinyl transferase from *Aspergillus nidulans* (SEQ ID NOs 104, 105) under control of araBAD promoter, floxed by homology regions to SS9 site, were obtained synthetically and cloned into bacterial expression vector comprising Zeocin resistance cassette. The obtained structure was used for transformation and integration into *E*. *coli* BW25113 genome by means of lambda bacteriophage protein-mediated recombination, as described in Bassalo et al. [ACS Synth Biol. 2016 Jul 15;5(7):561-8], using selection for Zeocin resistance. Integration of full-length structure was confirmed by PCR from primers specific to SS9 homology regions, and then the correctness of the integrated structure was verified by sequencing of genomic DNA PCR product by Sanger method.

The obtained *E*. *coli* strain was used for producing hispidin. At the first step the bacteria were incubated in five 50 ml plastic tubes in LB medium within 10 hours at 200 rpm fluctuation at 37°C. 250 ml of the obtained culture were added to 3.3 litres of fermentation medium into a fermenter Biostat B5 (Braun, Germany) so that initial culture optical density at 600 nm was about 0.35. Fermentation medium comprised 10 g/l of peptone, 5 g/l of caffeic acid, 5 g/l of yeast extract, 10 g/l of NaCl, 25 g/l of glucose, 15 g/l of (NH₄)₂SO₄, 2 g/l of KH₂PO₄, 2 g/l of MgSO₄·7 H₂O, 14.7 mg/l of CaCl₂, 0.1 mg/l of thiamine, 1.8 mg/l and 0.1% of the solution composed of: EDTA 8 mg/l, COCl₂·6 H₂O 2.5 mg/l, MnCl₂·4H₂O 15 mg/l, CuCl₂·2H₂O 1.5 mg/l, H₃BO₃ 3 mg/l, Na₂MoO₄·2H₂O 2.5 mg/l, Zn(CH₃COO)₂·2H₂O 13 mg/l, iron (III) citrate 100 mg/l, thiamine hydrochloride 4.5 mg/l. Fermentation was carried out at 37 °C, with 3 l/min aeration and 200 rpm mixing. After 25 hours of cultivation arabinose was added to the culture up to final concentration of 0.1 mM. pH was automatically controlled by adding of NH₄OH, reducing pH to 7.0. The solution comprising 500 g/l of glucose, 5 g/l of caffeic acid, 2 g/l of arabinose, 25 g/l of trypton, 50 g/l of yeast extract, 17.2 g/l of MgSO₄·7H₂O₇ 7.5 g/l of (NH₄)SO₄, 18 g/l of ascorbic acid, was added to a fermenter to maintain glucose level every time when pH increased to 7.1. After 56 hours of cultivation the hispidin concentration in the medium was 1.23 g/l. Fermenter medium and also hispidin purified from it by HPLC were active in bioluminescence reaction with *Neonothopanus nambi* hispidin hydroxylase and luciferase.

### Example 13. Obtaining 3-hydroxyhispidin from caffeic acid

Expression cassette, comprising nucleic acid coding *Neonothopanus nambi* hispidin hydroxylase (SEQ ID NOs 1, 2) under control of J23100 promoter, was obtained synthetically and cloned into bacterial expression vector comprising spectinomycin resistance gene. The obtained vector was transformed into *E. coli* cells expressing *Neonothopanus nambi* hispidin-synthase, Zeocin resistance gene and NpgA gene, obtained according to Example 12. The obtained bacteria were used for producing 3-hydroxyhispidin by fermentation according to the protocol described in Example 12, however with adding of spectinomycin at concentration of 50 mg/ml in all media used for cultivation. After 48 hours of cultivation the 3-hydroxyhispidin concentration in the medium was 2.3 g/l. Fermenter medium and also 3-hydroxyhispidin purified from it by HPLC were active in bioluminescence reaction with *Neonothopanus nambi* luciferase.

### Example 14 Obtaining hispidin from cell metabolites and tyrosine

*E. coli* strain, effectively producing tyrosine and caffeic acid, was obtained for producing biosynthetic hispidin from tyrosine. *E. coli* strain was obtained as described in [Lin and Yan. Microb Cell Fact. 2012 Apr 4; 11:42]. *E. coli* BW25113 line with integrated mutant gene of acY (lacY A177C) permease at attB site providing uniform consumption of arabinose by bacteria cells was taken as a basis for strain development. Expression cassettes comprising coding sequences of *Rhodobacter capsulatus* tyrosine-ammonia-lyase genes (SEQ ID NOs: 106, 107), and the components HpaB and HpaC of *E. coli* 4-hydroxyphenyl acetate 3-monooxygenase-reductase (SEQ ID NOs: 108-111), each under control of constitutive J23100 promoter, were obtained synthetically and integrated into genome of *E. coli* strain as described in Example 12. At the next step the plasmid obtained according to Example 12 and comprising coding sequence of *Neonothopanus nambi* hispidin-synthase under control of constitutive J23100 promoter, Zeocin resistance cassette from pGAP-Z vector, and also NpgA gene were integrated into *E. coli* genome. Integration into *E. coli* genome was performed by means of lambda bacteriophage protein-mediated recombination according to the technique from [Bassalo et al., ACS Synth Biol. 2016; 5(7):561-568]. Integration of full-length structure was confirmed by PCR from primers specific to SS9 homology regions (5'-CGGAGCATTTTGCATG-3' and 5'-TGTAGGATCAAGCTCAG-3'), and then the correctness of the integrated structure was verified by sequencing of genomic DNA PCR product by Sanger method. The obtained bacteria strain was used for producing biosynthetic hispidin in a fermenter.

Bacteria were cultivated in a fermenter, as described in Example 12, with the only difference - caffeic acid was not added to bacteria culture media. Biosynthetic hispidin was isolate from the medium by HPLC. The obtained strain was able to produce 1.20 mg/l of hispidin per 50 hours of fermentation. The obtained product purity was 97.3%. Adding of tyrosine to culture media at concentration of 10 g/ml enabled to increase hispidin output to 108.3 mg/ml.

### Example 15. Development of autonomously bioluminescent yeast Pichia pastoris

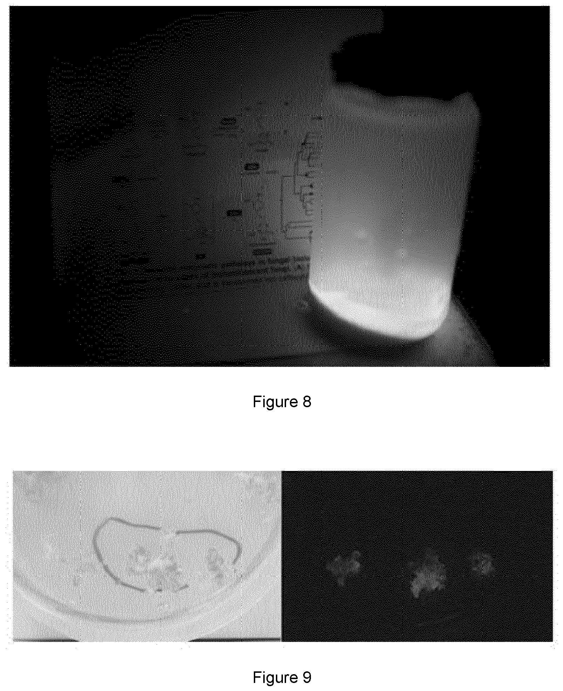

For the purpose of autonomously bioluminescent yeast *Pichia pastoris* development there were synthesized expression cassettes comprising, under control of GAP promoter and tAOX1 terminator, coding sequences of *Neonothopanus nambi* luciferase (SEQ ID NOs: *79, 80), Neonothopanus nambi* hispidin hydroxylase (SEQ ID NOs: 1, 2), *Neonothopanus nambi* hispidin-synthase (SEQ ID NOs: 34, 35), *Neonothopanus nambi* caffeylpyruvate hydrolase (SEQ ID NOs: 64, 65), *Aspergillus nidulans* NpgA protein (SEQ ID NOs: 104, 105), Rhodobacter capsulatus tyrosine-ammonia-lyase (SEQ ID NOs: 106, 107), and the components HpaB and HpaC of *E. coli* 4-hydroxyphenyl acetate 3-monooxygenase-reductase (SEQ ID NOs: 108-111). Each expression cassette was floxed by BsmBI restriction enzyme recognition sequences. Homology regions to MET6 *Pichia pastoris* gene (Uniprot F2QTU9), floxed by BsmBI restriction enzyme sites, were also obtained syntheticaly. Synthetic DNA was treated by BsmBI restriction enzymes and combined into one plasmid according to Golden Gate cloning protocol, described in [Iverson et al., ACS Synth Biol. 2016 Jan 15;5(1)-99-103]. 10 fmol of each DNA fragment were mixed in reaction comprising normal strength buffer for DNA lygase (Promega, USA), 20 units of DNA lygase activity (Promega, USA), 10 units of DNA restriction endonuclease activity in a total volume of 10 µl. The obtained reaction mixture was put into an amplifier and incubated at 16°C and 37°C according to the following protocol: 25 cycles of incubation at 37°C within 1.5 min and at 16°C - 3 min, then single incubation at 50°C within 5 min, and then single incubation at 80°C within 10 min. 5 µl of reaction mixture were transformed into *E.coli* chemically competent cells. Correctness of plasmid DNA assembly was confirmed by Sanger method, and purified plasmid DNA product was used for transformation of *Pichia pastoris* GS11 cells by electroporation. Electroporation was carried out according to the method, using lithium acetate and dithiothreitol, described in [Wu and Letchworth, Biotechniques, 2004, 36:152-4]. Electroporated cells were dispersed in Petri dishes with RDB medium, comprising 1 M of sorbitol, 2% (w/v) of glucose, 1.34 % (w/v) of yeast nitrogen base (YNB), 0.005 % (w/v) of amino acids mixture, 0.00004 % (w/v) of biotin and 2 % (w/v) of agar. Integration of gene cassette into genome was confirmed by PCR from primers annealed at a homology region. The obtained yeast strain comprising correct genome insert was able to illuminate autonomously in contrast to wild yeast strain (Fig. 7, 8).

### Example 16. Development of autonomously bioluminescent flowering plants

For the purpose of autonomously bioluminescent flowering plants development based on pBI121 vector (Clontech, USA) there was created a binary vector for agrobacterium transformation comprising coding sequences of *Neonothopanus nambi* luciferase optimized for expression in plants *(SEQ ID NO: 112), Neonothopanus nambi* hispidin hydroxylase (SEQ ID NO: 103), *Neonothopanus nambi* hispidin-synthase (SEQ ID NO: 113), *Neonothopanus nambi* caffeylpyruvate hydrolase (SEQ ID NO: *114)* and kanamycin resistance gene, each gene is under control of 35S promoter from cauliflower mosaic virus. Sequences for expression cassettes assembly were obtained synthetically, the vector was assembled according to Golden Gate cloning protocol, described in [Iverson et al., ACS Synth Biol. 2016 Jan 15;5(1):99-103].

*Arabidopsis thaliana* was transformed by co-cultivation of plant tissue with *Agrobacterium tumefaciens* bacteria of AGLO strain [Lazo et al., Biotechnology, 1991 Oct; 9(10):963-7], comprising the created binary vector. Transformation was performed using co-cultivation of *Arabidopsis thaliana* root segments (C24 ecotype), as described in [Valvekens et al., 1988, Proc. Nat. Acad. Sci. USA 85, 5536-5540]. Arabidopsis thaliana roots were cultivated in agarized Gamborg medium B-5 with 20 g/l of glucose, 0.5 g/l of 2,4-dichlorophenoxyacetic acid and 0.05 g/l of kinetin within 3 days. Then, roots were cut into pieces 0.5 cm long and transferred into 10 ml of liquid Gamborg medium B-5 with 20 g/l of glucose, 0.5 g/l of 2,4-dichlorophenoxyacetic acid and 0.05 g/l of kinetin, and 1.0 ml of agrobacteria overnight culture medium was added. Explants with agrobacteria were co-cultivated within 2-3 minutes. Then, the explants were put on sterile filters in Petri dishes with agarized medium of the same composition. After 48 hours of incubation in a thermostat at 25°C the explants were transferred to fresh medium with 500 mg/l of cefotaxime and 50 mg/l of kanamycin. In three weeks, regeneration of plants on selective medium, comprising 50 mg/l of kanamycin, was started. Transgenic plants took roots and were transferred to germination medium or soil. Bioluminescence was visualized by means of IVIS Spectrum In Vivo Imaging System (Perkin Elmer). More than 90% of transgenic plants emitted luminescence minimum by two orders of magnitude exceeding the signal from wild-type plants.

*Nicotiana benthamiana* was transformed by co-cultivation of plant tissue with *Agrobacterium tumefaciens* bacteria of AGLO strain [Lazo et al., Biotechnology, 1991 Oct; 9(10):963-7], comprising the created binary vector. Transformation was performed using co-cultivation of *Nicotiana benthamiana*leaf segments. Then, leaves were cut into pieces 0.5 cm long and transferred into 10 ml of liquid Gamborg medium B-5 with 20 g/l of glucose, 0.5 g/l of 2,4-dichlorophenoxyacetic acid and 0.05 g/l of kinetin, and 1.0 ml of agrobacteria overnight culture medium was added. Explants with agrobacteria were co-cultivated within 2-3 minutes. Then, the explants were put on sterile filters in Petri dishes with agarized medium of the same composition. After 48 hours of incubation in a thermostat at 25°C the explants were transferred to fresh medium with 500 mg/l of cefotaxime and 50 mg/l of kanamycin. In three weeks, regeneration of plants on selective medium, comprising 50 mg/l of kanamycin, was started. Transgenic plants took roots and were transferred to germination medium or soil. Bioluminescence was visualized by means of IVIS Spectrum In Vivo Imaging System (Perkin Elmer). More than 90% of transgenic plants emitted luminescence minimum by two orders of magnitude exceeding the signal from wild-type plants. Photos of autonomously luminescent Nicotiana benthamiana are given in Fig. 9.

For the purpose of development of autonomously bioluminescent *Agrostis stolonifera* L. there were cloned into pBl121 vector (Clontech, USA) the coding sequences of fungal luciferin metabolic cascade genes, optimized for expression in plants and floxed by Bsal restriction endonuclease *sites:Neonothopanus nambi luciferase (SEQ ID NO: 126), Neonothopanus nambi hispidin hydroxylase (SEQ ID NO: 117), Neonothopanus nambi hispidin-synthase (SEQ ID NO: 127), Neonothopanus nambi caffeylpyruvate hydrolase (SEQ ID NO: 128)* and herbicide glyphosate resistance gene *(bar* gene*)*. *Each sequence* was *under control* of CmYLCV promoter [Stavolone et al., Plant Mol Biol. 2003 Nov;53(5):663-73]. The sequences were synthesized according to standard technique. The vector was assembled according to Golden Gate cloning protocol. Transformation was performed by the method of embryogenic callus agrobacterium transformation. Overnight culture of *Agrobacterium tumefaciens* bacteria of AGLO strain [Lazo et al., Biotechnology, 1991 Oct; 9(10):963-7], comprising the created binary vector, was added to liquid medium. After two days of co-cultivation in agarized Murashige and Skoog medium the plants were transferred to fresh medium with 500 mg/l of cefotaxime and 10 mg/l of phosphinothricin. Plant regeneration started in three weeks. Transgenic plants were replanted into the medium with half Murashige and Skoog salt content and 8 mg/l of phosphinothricin for rootage. Rooted plants were set out in a greenhouse. About 25% of the obtained plants with correct and complete integration into metabolic cascade genome had bioluminescence exceeding bioluminescence of control wild-type plants.

Organisms able to emit luminescence in certain tissues or at certain times of the day are of special interest. Such organisms consume resources required for luminescence more efficiently. For the purpose of development of autonomously bioluminescent roses emitting luminescence only in petals, there were selected several rose varieties with white petals. On the basis of pBl121 vector (Clontech, USA) there were created two binary vectors for agrobacterium transformation comprising metabolic cascade from the coding sequences of *Neonothopanus nambi* luciferase, *Neonothopanus nambi* hispidin hydroxylase, *Neonothopanus nambi* hispidin synthase, *Neonothopanus nambi* caffeylpyruvate hydrolase and neomycin resistance gene, which were optimized for expression in plants. All genes, except for luciferase gene, were put under control of cauliflower mosaic virus 35S promoter. In one of the vectors luciferase gene was put under control of rose chalcone synthase promoter, and in the other - under control of chrysanthemum chalcone UEP1 promoter. There were used synthetic nucleic acids required for vector assembly, floxed by Bsal restriction enzyme recognition sites, and the vector was assembled according to Golden Gate cloning protocol. Rosa hybrida L. cv. Tinike transgenic plants were obtained by co-cultivation of embryogenic callus with *Agrobacterium tumefaciens* bacteria of AGL0 strain [Lazo et al., Biotechnology, 1991 Oct; 9(10):963-7], comprising on of the above binary vectors. Cultivation was performed in liquid medium comprising Murashige and Skoog macro- and micro-salts, with addition of 1-2 mg/l of kinetin, 3 mg/l of 2,4-dichlorophenoxyacetic acid and 1 mg/l of 6-benzylaminopurine within 40 minutes. Callus was transferred to agarized medium of the same composition. In two days, the explants were transferred to fresh Murashige and Skoog medium with 500 mg/l of cefotaxime and 50 mg/l of kanamycin. Shoot formation and regeneration took place in 5-8 weeks. Shoots were transferred to propagation or rooting medium. Rooted shoots were set out into peat mixture in a greenhouse. Flowering was observed in 8 weeks. Plants with mature flowers were visualized in IVIS Spectrum In Vivo Imaging System (Perkin Elmer). All tested plants of each tested structure autonomously emitted luminescence minimum by three orders of magnitude exceeding the signal from wild-type plants. Luminescence was emitted form petal tissues only, confirming tissue-specific functioning of promoters.

For the purpose of development of autonomously bioluminescent plants, where bioluminescence is controlled by circadian rhythms and activated at night time there was used earlier obtained binary vector for agrobacterium transformation comprising coding sequences of *Neonothopanus nambi* luciferase, *Neonothopanus nambi,* hispidin hydroxylase, *Neonothopanus nambi* hispidin synthase, *Neonothopanus nambi* caffeylpyruvate hydrolase and neomycin resistance gene, and each gene is under control of 35S promoter from cauliflower mosaic virus. Promoter for expression of *Neonothopanus nambi* luciferase was replaced by promoter of CAT3 gene from *Arabidopsis thaliana.* Transcription from CAT3 gene promoter is controlled by circadian rhythms and activated at nighttime. CAT3 promoter sequence is known in the art [Michael and McClung, Plant Physiol. 2002 Oct;130(2):627-38]. *Arabidopsis thaliana* was transformed by co-cultivation of plant tissue with *Agrobacterium tumefaciens* bacteria of AGLO strain [Lazo et al., Biotechnology, 1991 Oct; 9(10):963-7], comprising the created binary vector. Transformation was performed using co-cultivation of Arabidopsis thaliana root segments (C24 ecotype), as described in [Valvekens et al., 1988, Proc. Nat. Acad. Sci. USA 85, 5536-5540]. Arabidopsis thaliana roots were cultivated in agarized Gamborg medium B-5 with 20 g/l of glucose, 0.5 g/l of 2,4-dichlorophenoxyacetic acid and 0.05 g/l of kinetin within 3 days. Then, roots were cut into pieces 0.5 cm long and transferred into 10 ml of liquid Gamborg medium B-5 with 20 g/l of glucose, 0.5 g/l of 2,4-dichlorophenoxyacetic acid and 0.05 g/l of kinetin, and 1.0 ml of agrobacteria overnight culture medium was added. Explants with agrobacteria were co-cultivated within 2-3 minutes. Then, the explants were put on sterile filters in Petri dishes with agarized medium of the same composition. After 48 hours of incubation in a thermostat at 25°C the explants were transferred to fresh medium with 500 mg/l of cefotaxime and 50 mg/l of kanamycin. In three weeks, regeneration of plants on selective medium, comprising 50 mg/l of kanamycin, was started. Transgenic plants took roots and were transferred to germination medium, they were grown in natural day-night cycle conditions. Bioluminescence was visualized by means of IVIS Spectrum In Vivo Imaging System (Perkin Elmer), placing the plants into the instrument for 24 hours and recording bioluminescence intensity every half hour. Plants emitted luminescence within 24 hours, however, bioluminescence intensity was significantly modulated by circadian rhythms: integral luminous intensity at nighttime exceeded integral luminosity at day time more than 1000 times for 85% of tested plants.

### Example 17. Development of transgenic autonomously bioluminescent lower plants

Autonomously bioluminescent moss *Physcomitrella patens* was developed by protoplast co-transformation with plasmids using the method described in Example 8. There were synthetically obtained expression cassettes, including, optimized for expression in plants, coding sequences of *Neonothopanus nambi* luciferase (SEQ ID NO: 112), *Neonothopanus nambi* hispidin hydroxylase (SEQ ID NO: 103), *Neonothopanus nambi* hispidin-synthase (SEQ ID NO: 113), *Neonothopanus nambi* caffeylpyruvate hydrolase (SEQ ID NO: 114) and kanamycin resistance gene, each is under control of rice actin 2 promoter. Expression cassettes were operatively cross-linked in pBl121 vector (Clontech, C 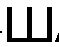 A) in such a way, that the structure including full metabolic cascade and kanamycin resistance gene were floxed by sequences coincident with moss genome target locus sequence. The vector was assembled according to Golden Gate cloning protocol Golden Gate [Iverson et al., ACS Synth Biol. 2016 Jan 15;5(1):99-103]. Guide RNA gene, complementary to target region in the moss genome, was also cloned into the vector. Plasmid with specified genes was co-transformed with plasmid for constitutive expression of Cas9 nuclease according to the polyethylenglycol transformation protocol described in [Cove et al., Cold Spring Harb Protoc., 2009, 2]. The obtained transformed protoplasts were incubated in dark conditions within 24 hours in BG-11 medium, and then were transferred to Petri dishes with BG-11 medium and 8.5% agar. Visualization in IVIS Spectrum In Vivo Imaging System (Perkin Elmer) was performed a month after growing in Petri dishes at continuous lighting. 70% of tested plants emitted luminescence exceeding the signal from wild-type plants minimum by an order of magnitude.

### Example 18. Development of transgenic luminescent animals

Transgenic fish *Danio rerio,* comprising gene of *Neonothopanus nambi* hispidin hydroxylase were created according to the technique described in [Hisano et al., Sci Rep., 2015, 5:8841]. The technique includes expression of guide RNA and Cas9 nuclease for making a breakpoint in the region homologous to the guide RNA sequence. For the purpose of development of transgenic animals there were ordered synthetic DNA fragments comprising guide RNA sequences from pX330 plasmid, Addgene #42230 and mRNA of Cas9 nuclease under control of bacteriophage polymerase T7 promoter. The obtained fragments were used for transcription *in vitro* by means of reagents from MAXIscript T7 kit (Life Technologies, USA), and synthesized RNA was purified by means of DNA isolation kit (Evrogen, Russia).

Coding sequence of *Neonothopanus nambi* hispidin hydroxylase floxed by 50-nucleotide sequences from *krtt1c19e Danio rerio gene, described in [*Hisano et al., Sci Rep., 2015, 5:8841*],* was obtained synthetically and cloned into pEGFP/C1 plasmid base comprising pUC origin and kanamycin resistance cassette. The obtained vector, Cas9 nuclease mRNA and guide RNA were dissolved in injection buffer (40 mM HEPES (pH 7.4), 240 mM KCI with addition of 0.5% of phenol red) and injected into 1-2 cell embryos of the earlier obtained *Danio rerio* line, stably expressing *Neonothopanus nambi* luciferase, in the volume of about 1-2 nl. About 12 from 48 embryos survived the injection and demonstrated normal development on the fourth day after fertilization.

Hispidin solution was intravenously injected into *Danio rerio* larvae for recording bioluminescent signal according to the technique described in [Cosentino et al., J Vis Exp. 2010; (42): 2079]. Bioluminescence was recorded by means of IVIS Spectrum In Vivo Imaging System (Perkin Elmer). After recording, genomic DNA was isolated from larvae to confirm integration of hispidin hydroxylase into genome. All larvae with correct integration of *Neonothopanus nambi* hispidin hydroxylase gene into genome demonstrated bioluminescence intensity minimum by two orders of magnitude exceeding the signal outgoing from wild-type fish after hispidin solution injection.

### Example 19. Study of caffeylpyruvate hydrolase effect on luminescence of autonomously bioluminescent organisms

For the purpose of study of caffeylpyruvate hydrolase effect on luminescence of autonomously bioluminescent organisms there was used a binary vector for agrobacterium transformation comprising coding sequences of *Neonothopanus nambi* luciferase, *Neonothopanus nambi* hispidin hydroxylase, *Neonothopanus nambi* hispidin synthase, *Neonothopanus nambi* caffeylpyruvate hydrolase and kanamycin resistance gene, and each gene is under control of 35S promoter from cauliflower mosaic virus, obtained according to Example 16, and control vector characterized in that caffeylpyruvate hydrolase sequence was removed from it. The vectors were used for transformation of *Arabidopsis thaliana* in the same conditions according to the protocol described in Example 16. Bioluminescence was visualized by means of IVIS Spectrum In Vivo Imaging System (Perkin Elmer). Comparison of bioluminescence intensities of the plants expressing all four genes of *Neonothopanus nambi* bioluminescent system with the plants expressing only luciferase, hispidin hydroxylase and hispidin synthase, has revealed that the plants additionally expressing caffeylpyruvate hydrolase have on average 8.3 times more bright bioluminescence. The data provided indicate that expression of caffeylpyruvate hydrolase enables to increase bioluminescent cascade efficiency, that results in increasing the intensity of luminescence emitted by plants.

### Example 20. Effect of external adding caffeic acid on transgenic organism bioluminescence

Autonomously bioluminescent transgenic plants *Nicotiana benthamiana,* obtained according to Example 16, were transferred to soil and cultured within eight weeks. Then, plant stem was cut and placed in water for two hours, after that, bioluminescence intensity was measured by IVIS Spectrum In Vivo Imaging System (Perkin Elmer). Then, plants were transferred to one of five water solutions at caffeic acid concentration of 0.4 g/l, 0.8 g/l, 1.6 g/l, 3.2 g/l, or 6.4 g/l, and control plants were placed in water. After two more hours of incubation in the caffeic acid solution or in water bioluminescence intensity was measured again. In all cases the bioluminescence intensity of the plants incubated in caffeic acid solution increased as compared to the intensity before placing in caffeic acid solution, and the largest changes were observed in plants incubated in the solution at a concentration of 6.4 g/l. Control plants incubated in water did not demonstrated significant change in bioluminescence intensity within four hours after incubation start.

### Example 21. Use of fungal bioluminescent system genes for activity assay of promoters and intracellular logical integration of external signals.

Coding sequences of *Neonothopanus nambi* hispidin hydroxylase, hispidin synthase and luciferase were used for monitoring simultaneous activation of several promoters. Synthetic expression cassettes comprising coding sequence of *Neonothopanus nambi* hispidin-synthase (SEQ ID NOs: 34, 35) under control of E. coli araBAD promoter induced by arabinose, coding sequence of hispidin hydroxylase (SEQ ID NOs 1, 2) under control of T7/lacO promoter induced by IPTG, and luciferase gene (SEQ ID NOs: 79, 80) under control of pRha promoter induced by rhamnose, and also NpgA gene (SEQ ID NOs: 104, 105) under control of constitutive J23100 promoter (Registry of Standard Biological parts, Part:BBa_J23100). The obtained synthetic nucleic acids were cloned into MoClo_Level2 vector [Weber et al., PLoS One. 2011 Feb 18;6(2):e16765] instead of the insert comprising LacZ gene, using Bpil restriction endonuclease. The obtained vector was transformed into *E.coli* BL21 (NEB, USA) strain competent cells comprising genomic copy of T7 bacteriophage polymerase.

For the purpose of determining a possibility of recording simultaneous activation of several promoters the cells obtained at the previous step were grown within a night in a flask with 100 ml LB medium with addition of ampicillin at a concentration of 100 mg/l. The next day the cell culture aliquots were placed for 120 minutes at 24°C and 200 rpm into one of the media with following composition:
1. LB medium with addition of 1% arabinose,
2. LB medium with addition of 0.2% rhamnose,
3. LB medium with addition of 0.5% IPTG,
4. LB medium with addition of 1% arabinose and 0.2% rhamnose,
5. LB medium with addition of 1% arabinose and 0.5% IPTG,
6. LB medium with addition of 0.2% rhamnose and 0.5% IPTG,
7. LB medium with addition of 1% arabinose, 0.2% rhamnose and 0.5% IPTG,
8. LB medium (control).

After incubation the cells were pelleted, the medium was replaced with phosphate-buffered saline with pH 7.4 (Sigma-Aldrich, USA) with addition of caffeic acid (Sigma-Aldrich, USA) at concentration of 1 g/l, the cells were resuspended by pipetting. Cell bioluminescence was analyzed in half an hour by means of luminometer GloMax 20/20 (Promega, USA). The experiment was repeated in triplicate. Of eight tested samples bioluminescence intensity was significantly different from bioluminescence of test bacteria incubated in LB medium (medium No. 8) only for bacteria incubated in medium No. 7 (LB medium with addition of 1% arabinose, 0.2% rhamnose and 0.5% IPTG). Therefore, bacteria luminescence was indicative of placing the bacteria into the medium ensuring simultaneous activation of three different promoters. In this experiment the bacteria cells integrated information about presence of substances, inducing promotor activity. in external medium and signaled by luminescence only when all three substances were present in the medium simultaneously, performing logical operation "AND" intracellularly.

Synthetic expression cassettes comprising (1) coding sequence of hispidin hydroxylase (SEQ ID NOs: 1, 2) under control of Odf2 promoter according to [Pletz et al., Biochim Biophys Acta. 2013 Jun;1833(6):1338-46]; (2) coding sequence of hispidin-synthase (SEQ ID NOs: 34, 35) under control of cycline-dependent kinase CDK7 promoter; (3) luciferases (SEQ ID NOs: 79, 80) under control of CCNH gene promoter were cloned into pmKate2-keratin vector (Evrogen, Russia) instead of sequences of cytomegaloviral promoter abd mKate2-keratin insert. Also, the coding sequence of NpgA gene (SEQ ID NOs: 104, 105) was cloned into pmKate2-keratin vector instead of mKate2-keratin insert sequence. All obtained vectors were co-transfected into HEK293T cells by transfection agent FuGENE HD (Promega, USA) according to the manufacturer's protocol. 24 hours after transfection caffeic acid at concentration of 5 mg/ml was added to the medium and cell luminescence was detected by means of Leica TCS SP8 microscope. Luminescence enabled to identify simultaneous activation of Odf2, CCNH and CDK7 promotors, with luminescence intensity being related to cell cycle stage.

The obtained data indicate that fungal bioluminescent system genes could be used for monitoring simultaneous activation of several promoters, for detecting presence of different substances and their combinations in medium, and also for intracellular logical integration of external signals.

### Example 22. Identification of hispidin in plant extracts

Coding sequences of *Neonothopanus nambi* hispidin hydroxylase and luciferase, obtained according to Example 1, were cloned into pET23 vector under control of T7 promoter. Purified plasmid DNA products were used for protein transcription and translation *in vitro* by means of PURExpress In Vitro Protein Synthesis Kit (NEB, USA). The obtained reaction mixture was used for analysis of presence and concentration of hispidin and its functional analogues in lysates of about 19 different plants (*Chrysanthemum sp., Ananas comosus, Petunia atkinsiana, Picea abies, Urtica dioica, Solanum lycopersicum, Nicotiana benthamiana, Nicotiana tobacum, Arabidopsis thaliana, Rosa glauca, Rosa rubiginosa, Equisetum arvense, Equisetum telmateia, Polygala sabulosa, Rosa rugosa, Clematis tashiroi, Kalanchoe sp., Triticum aestivum, Dianthus caryophyllus*) by adding 2 µl of plant lysate to 100 µl of the reaction mixture and recording luminescence intensity by luminometer GloMax (Promega, USA). It was determined that maximum concentration of hispidin and its functional analogues is in *Equisetum arvense* and *Equisetum telmateia* lysates. Hispidin or its functional analogues were also identified in *Polygala sabulosa, Rosa rugosa and Clematis tashiroi* lysates.

### Example 23. Identification of PKS able to catalyze hispidin synthesis and their use for producing hispidin in vitro and in vivo.

Fungal luciferin precursors, such as hispidin, relate to a group of polyketide derivatives. It is known in the art that enzymes involved in polyketide synthesis in plants relate to polyketide synthase protein superfamily, and plant polyketide synthases, in contrast with fungal polyketide synthases, are comparatively compact proteins using CoA ethers of acids, including 3-arylacrylic acids. No polyketide synthase, able to catalyze transformation of caffeic acid CoA ether into hispidin, has been known in the art, however, hispidin is present in many plant organisms.

Using bioinformatic analysis there were selected 11 polyketide synthases potentially able to catalyze hispidin synthesis from the following sources:
Aquilaria sinensis (2 enzymes),
Hydrangea macrophylla,
Arabidopsis thaliana,
Physcomitrella patens,
Polygonum cuspidatum,
Rheum palmatum,
Rheum tataricum,
Wachendorfia thyrsiflora,
Piper methysticum (two enzymes).

The selected nucleotide sequences for expression in *Pichia pastoris* yeast cells and *Nicotiana benthamiana* plant cells were optimized. The resulting nucleic acids were obtained synthetically and cloned into pGAPZ vector and used for verifying ability of the expressed proteins to synthesize hispidin.

For this purpose, in genome of *Pichia pastoris* GS115 yeast line, constitutively expressing *Neonothopanus nambi* luciferase and hispidin hydroxylase, obtained according to Example 1, there was additionally introduced pGAPZ plasmid, comprising gene of *Arabidopsis thaliana* coumarate-CoA ligase 1 (which nucleotide and amino acid sequence are shown in SEQ ID NOs: 140, 141), also obtained by oligonucleotide synthesis. The plasmid was linearized at restriction site Avrll and used for transformation into *Pichia pastoris* GS115 cells.

The obtained yeast cells, constitutively expressing *Neonothopanus nambi* luciferase and hispidin hydroxylase and *Arabidopsis thaliana* coumarate-CoA ligase 1, were linearized by plasmids comprising coding PKS sequences, and dispersed in Petri dishes with RDB medium, comprising 1 M of sorbitol, 2% (weight/volume) of glucose, 1.34 % (weight/volume) of yeast nitrogen base (YNB), 0.005 % (weight/volume) of amino acids mixture, 0.00004 % (weight/volume) of biotin and 2% (weight/volume) of agar. To identify enzymes having hispidin-synthase activity, the obtained colonies were sprayed with caffeic acid solution, detecting hispidin-synthase presence in cells by occurrence of luminescence. Luminescence emitted by colonies was detected by means of IVIS Spectrum CT (PerkinElmer, USA). Yeast line constitutively expressing luciferase, hispidin hydroxylase and coumarate-CoA ligase 1, and also wild yeast cells were used as negative control. Of tested genes 11 enzymes had hispidin-synthase activity, and their sequence is shown in SEQ ID NOs: 118, 120, 122, 124, 126, 128, 130, 132, 134, 136, 138. Amino acid sequences coded by then are shown in SEQ ID NOs: 119, 121, 123, 125, 127, 129, 131, 133, 135, 137, 139 respectively. The highest activity was demonstrated by enzymes from PKS1 and PKS2 from Aquilaria sinensis (SEQ ID NOs:119, 121), PKS from Arabidopsis thaliana (SEQ ID NO:123) and PKS from Hydrangea macrophylla (SEQ ID NO:125).

Nucleic acid coding PKS from *Hydrangea macrophylla (*SEQ ID NOs: 124, 125) was used for producing recombinant protein according to the technique described in Example 4. Presence of the expected recombinant product was confirmed by electrophoresis as bands of the expected length were available. Aliquots of the isolated recombinant protein were used for verifying functionality of: 30 µl of isolated recombinant protein solution were put into a glass tube, comprising 100 µl of the buffer (0.2 M of Naphosphate buffer, 0.5 M of Na₂SO₄, 0.1% dodecylmaltoside (DDM) pH 8.0, all components - Sigma-Aldrich, USA), 0.5 µl of purified recombinant luciferase of Neonothopanus nambi, obtained according to Example 4, 1 mM of NADPH (Sigma-Aldrich, USA), 15 µl of purified recombinant hispidin hydroxylase of *Neonothopanus nambi,* obtained according to Example 4, 10 mM of ATP (ThermoFisher Scientific, USA), 1 mM of CoA (Sigma-Aldrich, USA), 1 mM of malonyl-CoA (Sigma-Aldrich, USA). The glass tube was placed into a luminometer GloMax 20/20 (Promega, USA). The reaction mixtures demonstrated bioluminescence at adding of 20 µM of caffeyl-CoA into the solution. Maximum emission of the emitted luminescence was 520-535 nm.

Nucleic acid coding PKS2 from Aquilaria sinensis (SEQ ID NO: 120, 121) was used for producing hispidin producer strain. For this purpose there were synthesized the expression cassette comprising the nucleic acid SEQ ID NO: 120 under control of constitutive J23100 promoter, and expression cassette comprising the nucleic acid SEQ ID NO: 140, coding 4-coumarate-CoA ligase 1 from *Arabidopsis thaliana* under control of araBAD promoter; bothe expression cassettes were floxed by homology regions to SS9 site. The expression cassettes were cloned into bacterial expression vector, comprising Zeocin resistance cassette, and were used for transformation and integration into *E. coli* BW25113 genome by means of lambda bacteriophage protein-mediated recombination, as described in Bassalo et al. [ACS Synth Biol. 2016 Jul 15;5(7):561-8], using selection for Zeocin resistance. Integration of full-length structure was confirmed by PCR from primers specific to SS9 homology regions, and then the correctness of the integrated structure was verified by sequencing of genomic DNA PCR product by Sanger method.

The obtained *E. coli* strain was used for producing hispidin. At the first step the bacteria were incubated in five 50 ml plastic tubes in LB medium within 10 hours at 200 rpm fluctuation at 37°C. 250 ml of the obtained culture were added to 3.3 litres of fermentation medium into a fermenter Biostat B5 (Braun, Germany) so that initial culture optical density at 600 nm was about 0.35. Fermentation medium comprised 10 g/l of peptone, 5 g/l of caffeic acid, 5 g/l of yeast extract, 10 g/l of NaCl, 25 g/l of glucose, 15 g/l of (NH₄)₂SO₄, 2 g/l of KH₂PO4, 2 g/l of MgSO₄·7H₂O, 14.7 mg/l of CaCl₂, 0.1 mg/l of thiamine, 1.8 mg/l and 0.1% of the solution composed of: EDTA 8 mg/l, COCl₂·6H₂O 2.5 mg/l, MnCl₂·4H₂O 15 mg/l, CuCl₂·2H₂O 1.5 mg/l, H₃BO₃ 3 mg/l, Na₂MoO₄·2H₂O 2.5 mg/l, Zn(CH₃COO)₂·2H₂O 13 mg/l, iron (III) citrate 100 mg/l, thiamine hydrochloride 4.5 mg/l. Fermentation was carried out at 37 °C, with 3 l/min aeration and 200 rpm mixing. After 25 hours of cultivation arabinose was added to the culture up to final concentration of 0.1 mM. pH was automatically controlled by adding of NH₄OH, reducing pH to 7.0. The solution comprising 500 g/l of glucose, 5 g/l of caffeic acid, 2 g/l of arabinose, 25 g/l of trypton, 50 g/l of yeast extract, 17.2 g/l of MgSO₄·7H₂O, 7.5 g/l of (NH₄)SO₄, 18 g/l of ascorbic acid, was added to a fermenter to maintain glucose level every time when pH increased to 7.1. After 56 hours of cultivation the hispidin concentration in the medium was 3.48 g/l. Fermenter medium and also hispidin purified from it by HPLC were active in bioluminescence reaction with *Neonothopanus nambi* hispidin hydroxylase and luciferase, obtained according to Example 4.

For the purpose of autonomously bioluminescent yeast Pichia pastoris development there were used expression cassettes comprising, under control of GAP promoter and tAOX1 terminator, coding sequences of Neonothopanus nambi luciferase (SEQ ID NOs: 79, 80), *Neonothopanus nambi* hispidin hydroxylase (SEQ ID NOs: 1, 2), *Neonothopanus nambi* hispidin-synthase (SEQ ID NOs: 34, 35), *Neonothopanus nambi* caffeylpyruvate hydrolase (SEQ ID NOs: 64, 65), Rhodobacter capsulatus tyrosine-ammonia-lyase (SEQ ID NOs: 106, 107), and the components HpaB and HpaC of *E. coli* 4-hydroxyphenyl acetate 3-monooxygenase-reductase (SEQ ID NOs: 108-111), obtained according to Example 15, and also there were synthesized similar expression cassettes comprising coding sequences of 4-coumarate-CoA ligase 1 from *Arabidopsis thaliana* (SEQ ID NOs: 140, 141) and three PKS: from Aquilaria sinensis (SEQ ID NOs: 120, 121), PKS from Arabidopsis thaliana (SEQ ID NOs: 122, 123) and PKS from Hydrangea macrophylla (SEQ ID NO: 124, 125). Each expression cassette was floxed by BsmBI restriction enzyme recognition sequences. Homology regions to MET6 *Pichia pastoris* gene (Uniprot F2QTU9), floxed by BsmBI restriction enzyme sites, were also obtained syntheticaly. Synthetic DNA was treated by BsmBI restriction enzymes and combined into one plasmid according to Golden Gate cloning protocol, described in [Iverson et al., ACS Synth Biol. 2016 Jan 15;5(1):99-103]. There were produced three plasmids different in PKS in their composition. The obtained plasmids were used for producing transgenic yeasts *Pichia pastoris* according to the technique described in Example 15. Integration of gene cassette into genome was confirmed by PCR from primers annealed at a homology region. All three obtained yeast strains comprising correct genome insert were able to illuminate autonomously in contrast to wild yeast strain.

For development of autonomously bioluminescent flowering plants based on pBI121 vector (Clontech, USA) there was created a set of binary vectors for agrobacterium transformation comprising coding sequences of *Neonothopanus nambi* luciferase optimized for expression in plants *(SEQ ID NO: 112), Neonothopanus nambi* hispidin hydroxylase (SEQ ID NO: 103), *Neonothopanus nambi* caffeylpyruvate hydrolase (SEQ ID NO: *114),* kanamycin resistance gene, and PKS *(SEQ ID NOs:* 122, 123), each gene is under control of 35S promoter from cauliflower mosaic virus. Sequences for expression cassettes assembly were obtained synthetically, the vector was assembled according to Golden Gate cloning protocol, described in [Iverson et al., ACS Synth Biol. 2016 Jan 15;5(1):99-103]. *Nicotiana tabacum* was transformed by co-cultivation of plant tissue with Agrobacterium tumefaciens bacteria of AGLO strain [Lazo et al., Biotechnology, 1991 Oct; 9(10):963-7], comprising the created binary vector. Transformation was performed using co-cultivation of *Nicotiana tabacum* leaf segments. Then, leaves were cut into pieces 0.5 cm long and transferred into 10 ml of liquid Gamborg medium B-5 with 20 g/l of glucose, 0.5 g/l of 2,4-dichlorophenoxyacetic acid and 0.05 g/l of kinetin, and 1.0 ml of agrobacteria overnight culture medium was added. Explants with agrobacteria were co-cultivated within 2-3 minutes. Then, the explants were put on sterile filters in Petri dishes with agarized medium of the same composition. After 48 hours of incubation in a thermostat at 25°C the explants were transferred to fresh medium with 500 mg/l of cefotaxime and 50 mg/l of kanamycin. In three weeks, regeneration of plants on selective medium, comprising 50 mg/l of kanamycin, was started. Transgenic plants took roots and were transferred to germination medium or soil. Bioluminescence was visualized by means of IVIS Spectrum In Vivo Imaging System (Perkin Elmer). More than % of transgenic plants emitted luminescence minimum by three orders of magnitude exceeding the signal from wild-type plants.

### Example 24. Nucleic acid combinations

### Combination 1:

Composition: (a) Nucleic acid coding hispidin hydroxylase, which amino acid sequence is selected from the group of SEQ ID NOs: 2, 4, 6, 8 10, 12, 14, 16, 18, 20, 22, 24, 26, 28; and (b) Nucleic acid coding luciferase, which amino acid sequence is selected from the group: 80, 82, 84, 86, 88, 90, 92, 94, 96, 98.

The combination could be used for obtaining bioluminescence in expression systems *in vitro* or *in vivo* in the presence of a substance selected from the group of 6-(2-arylvinyl)-4-hydroxy-2H-pyran-2-ones, having the structural formula 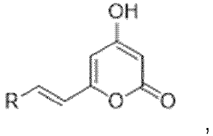 where 6 position substituent is 2-arylvinyl or 2-heteroarylvinyl substituent (R-CH=CH-), including 2-(3,4-dihydroxystyryl), 2-(4-hydroxystyryl), 2-(4-(diethylamino)styryl), 2-(2-(1H-indol-3-yl)vinyl), 2-(2-(1,2,3,5,6,7-hexahydropyrido[3,2,1-ij]quinolin-9-yl)vinyl), 2-(6-hydroxynaphthalene-2-yl)vinyl.

The said combination could also be used in study of two promoters dependency in heterologous expression systems.

The said combination could also be used for identifying hispidin and its analogues in biological objects.

The said combination could also be used for cell labeling by bioluminescence occurring in the presence of hispidin and its functional analogues.

### Combination 2:

Composition: (a) Nucleic acid coding hispidin hydroxylase, which amino acid sequence is selected from the group of SEQ ID NOs: 2, 4, 6, 8 10, 12, 14, 16, 18, 20, 22, 24, 26, 28; and (b) Nucleic acid coding hispidin-synthase, which amino acid sequence is selected from the group of SEQ ID NOs: 35, 37, 39, 41, 43, 45, 47, 49, 51, 53, 55.

The combination could be used for producing fungal luciferin in expression systems *in vitro* or *in vivo* from a substance selected from substituted acrylic acid with the structural formula 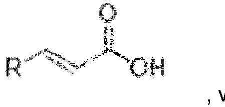 where R is aryl or heteroaryl (e.g. from caffeic acid).

### Combination 3:

Includes all the components specified in Combination 2, and also nucleic acid coding luciferase, which amino acid sequence is selected from the group: 80, 82, 84, 86, 88, 90, 92, 94, 96, 98.

The combination could be used for obtaining bioluminescence in expression systems *in vitro* or *in vivo* in the presence of a substance selected from substituted acrylic acid with the structural formula 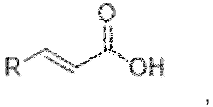 where R is aryl or heteroaryl.

The combination could be used for producing bioluminescent cells and transgenic organisms. The said combination could also be used in study of three promoters dependency in heterologous expression systems.

### Combination 4:

Includes all the components specified in Combination 3, and also nucleic acid coding caffeylpyruvate hydrolase, which amino acid sequence is selected from the group of SEQ ID NOs: 65, 67, 69, 71, 73, 75.

The combination could be used for producing bioluminescent cells and transgenic organisms.

### Combination 5:

Composition: (a) Nucleic acid coding hispidin-synthase, which amino acid sequence is selected from the group of SEQ ID NOs: 35, 37, 39, 41, 43, 45, 47, 49, 51, 53, 55; and (b) Nucleic acid coding gene of 4'-phosphopantetheinyl transferase, which amino acid sequence is shown in SEQ ID NO: 105

The combination could be used for producing hispidin from caffeic acid in expression systems *in vitro* and *in vivo.*

### Combination 6:

Composition: (a) Nucleic acid coding hispidin-synthase, which amino acid sequence is selected from the group of SEQ ID NOs: 35, 37, 39, 41, 43, 45, 47, 49, 51, 53, 55; (b) Nucleic acid coding gene of 4'-phosphopantetheinyl transferase, which amino acid sequence is shown in SEQ ID NO: 105; and (c) nucleic acids coding enzymes of 3-arylacrylic acid biosynthesis with the structural formula 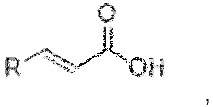 where R is aryl or heteroaryl from cell metabolites (e.g. nucleic acids coding tyrosine-ammonia-lyase and the components HpaB and HpaC of 4-hydroxyphenyl acetate 3-monooxygenase-reductase).

The combination could be used for producing hispidin from tyrosine in expression systems *in vitro* and *in vivo.*

Combinations 2-4 could also include the coding sequence of 4'-phosphopantetheinyl transferase NpgA gene (SEQ ID NOs: 104, 105) or other enzyme demonstrating the same activity.

### Combination 7:

Composition: (a) Nucleic acid coding hispidin hydroxylase, which amino acid sequence is selected from the group of SEQ ID NOs: 2, 4, 6, 8 10, 12, 14, 16, 18, 20, 22, 24, 26, 28; and (b) Nucleic acid coding PKS, which amino acid sequence is selected from the group of SEQ ID NOs: 119, 121, 123, 125, 127, 129, 131, 133, 135, 137, 139.

The combination could be used for producing 3-hydroxyhispidin from caffeyl-CoA in expression systems in vitro or in vivo.

### Combination 8:

Includes all the components specified in Combination 7, and also nucleic acid coding luciferase, which amino acid sequence is selected from the group: 80, 82, 84, 86, 88, 90, 92, 94, 96, 98. The combination could be used for obtaining bioluminescence *in vitro* or *in vivo* in the presence of caffeyl-CoA.

### Combination 9:

Includes all the components specified in Combination 8, and also nucleic acid coding caffeylpyruvate hydrolase, which amino acid sequence is selected from the group of SEQ ID NOs: 65, 67, 69, 71, 73, 75.

The combination could be used for producing bioluminescent cells and transgenic organisms.

### Combination 10:

Composition: (a) Nucleic acid coding PKS, which amino acid sequence is selected from the group of SEQ ID NOs: 119, 121, 123, 125, 127, 129, 131, 133, 135, 137, 139; and
(b) Nucleic acid coding 4-coumarate-CoA ligase 1 from Arabidopsis thaliana, which amino acid sequence is shown in SEQ ID NO: 141.

The combination could be used for producing hispidin from caffeic acid in expression systems *in vitro* and *in vivo.*

### Combination 11:

Includes all the components specified in Combination 10, and also nucleic acids coding enzymes of caffeic acid biosynthesis (e.g. nucleic acids coding tyrosine-ammonia-lyase and the components HpaB and HpaC of 4-hydroxyphenyl acetate 3-monooxygenase-reductase).

The combination could be used for producing hispidin from tyrosine in expression systems *in vitro* and *in vivo.*

### Example 25. Combinations of recombinant proteins [0486] Combination 1:

Composition: (a) hispidin hydroxylase, which amino acid sequence is selected from the group of SEQ ID NOs: 2, 4, 6, 8 10, 12, 14, 16, 18, 20, 22, 24, 26, 28; and (b) hispidin-synthase, which amino acid sequence is selected from the group of SEQ ID NOs: 35, 37, 39, 41, 43, 45, 47, 49, 51, 53, 55.

The combination could be used for producing fungal luciferin from a substance selected from 3-arylacrylic acid with the structural formula 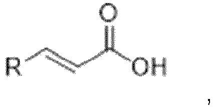 where R is aryl or heteroaryl (e.g. from caffeic acid).

### Combination 2:

Includes the components specified in Combination 1, and also luciferase, which amino acid sequence is selected from the group: 80, 82, 84, 86, 88, 90, 92, 94, 96, 98.

The combination could be used for detecting in a sample presence of 3-arylacrylic acid with the structural formula 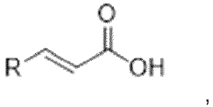 where R is aryl or heteroaryl (e.g. from caffeic acid).

### Combination 3:

Composition: (a) hispidin hydroxylase, which amino acid sequence is selected from the group of SEQ ID NOs: 2, 4, 6, 8 10, 12, 14, 16, 18, 20, 22, 24, 26, 28; and (b) PKS, which amino acid sequence is selected from the group of SEQ ID NOs: 119, 121, 123, 125, 127, 129, 131, 133, 135, 137, 139; and (c) 4-coumarate-CoA ligase 1 from Arabidopsis thaliana, which amino acid sequence is shown in SEQ ID NO: 141. The combination could be used for producing fungal luciferin from caffeic acid.

### Example 25. Kits

In the examples below the nucleic acids could be included in the expression cassettes or vectors and operatively cross-linked to regulatory elements for their expression in a host cell. Alternatively, nucleic acids could comprise flanking sequences for its incorporation into the target vector. Nucleic acids could be included in promoter-free vectors intended for easy cloning of target regulatory elements.

**Reagent Kit No. 1** includes a purified product of hispidin-synthase of the invention, and it could be used for producing hispidin from caffeic acid. The kit could also be used for producing the other of 6-(2-arylvinyl)-4-hydroxy-2H-pyran-2-one, having the structural formula 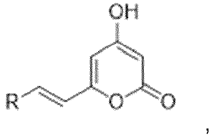 from the corresponding 3-arylacrylic acid with the structural formula 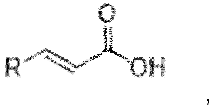 where R is aryl or heteroaryl.

Reagent kit could also include a reaction buffer. For example, 0.2 M sodium phosphate buffer (pH 8.0) laced with 0.5 M of Na₂SO₄, 0.1% of dodecylmaltoside (DDM), 1 mM of NADPH, 10 mM of ATP, 1 mM of CoA, 1 mM of malonyl-CoA, or components for reaction buffer preparation.

Reagent kit could also include deionized water.

Reagent kit could also include directions for use.

**Reagent Kit No. 2** includes a purified product of hispidin synthase of the invention and purified product of hispidin hydroxylase of the invention, and it could be used for producing fungal luciferin from a substance selected from 3-arylacrylic acid with the structural formula 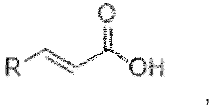 where R is aryl or heteroaryl (e.g. from caffeic acid).

Reagent kit could also include a reaction buffer: 0.2 M sodium phosphate buffer (pH 8.0) laced with 0.5 M of Na₂SO₄, 0.1% of dodecylmaltoside (DDM), 1 mM of NADPH, 10 mM of ATP, 1 mM of CoA, 1 mM of malonyl-CoA, or components for reaction buffer preparation.

Reagent kit could also include deionized water.

Reagent kit could also include directions for use.

**Reagent Kit No. 3** includes a purified product of hispidin hydroxylase of the invention, and it could be used for producing fungal luciferin from 6-(2-arylvinyl)-4-hydroxy-2H-pyran-2-one, having the structural formula 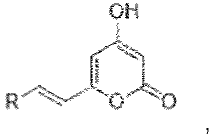 where R is aryl or heteroaryl. For example, the kit could be used for producing 3-hydroxyhispidin from hispidin.

Reagent kit could also include a reaction buffer. For example, 0.2 M sodium phosphate buffer (pH 8.0) laced with 0.5 M of Na₂SO₄, 0.1% of dodecylmaltoside (DDM), 1 mM of NADPH.

Reagent kit could also include deionized water.

Reagent kit could also include directions for use.

**Reagent Kit No. 4 and No. 5** differ from the kits No. 2 and No. 3 in that they comprise purified luciferase, which substrate is 6-(2-arylvinyl)-3,4-dihydroxy-2H-pyran-2-one, having the structural formula 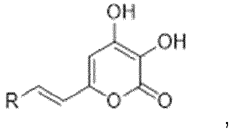 where R is aryl or heteroaryl.

The kits could be used for identifying 3-arylacrylic acid with the structural formula 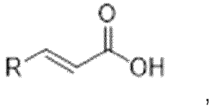 where R is aryl or heteroaryl (e.g. from caffeic acid), and/or 6-(2-arylvinyl)-4-hydroxy-2H-pyran-2-one, having the structural formula 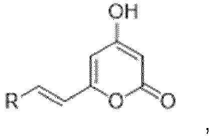 where R is aryl or heteroaryl, (e.g. hispidin) in biological specimens, e.g. in plant extracts, fungal extracts and in microorganisms.

Reagent kits could also include a reaction buffer (see description of kits 2 and 3) for reacting, or components for reaction buffer preparation.

Reagent kit could also include deionized water.

Reagent kit could also include directions for use.

Reagent kit could also include caffeic acid. For example, water solution of caffeic acid or residue for dissolving in water.

Reagent kit could also include hispidin.

### Kit applications

For identification of caffeic acid presence in test specimen it is required to add 5 µl of enzyme mixture to 95 µl of ice-cold reaction buffer in a cuvette, mix carefully, add 5 µl of the test specimen, mix carefully again, and place into a luminometer. Integrate bioluminescent signal within two minutes at maximum 30°C. make control measurements under the same conditions with addition of 5 µl of caffeic acid solution or 5 µl of water instead of the test specimen aliquot. It could be said that caffeic acid is present in the specimen in the detected amounts, if luminescence emitted by the test specimen exceeds a background signal recored from a specimen with water.

Sensitivity: the kit enables to determine presence of caffeic acid in a medium at concentration exceeding 1 nM.

Storage conditions: all kit components should be stored at temperature not exceeding -20°C.

For identification of hispidin presence in test specimen it is required to add 5 µl of enzyme mixture to 95 µl of ice-cold reaction buffer in a cuvette, mix carefully, add 5 µl of the test specimen, mix carefully again, and place into a luminometer. Integrate bioluminescent signal within two minutes at maximum 30°C. make control measurements under the same conditions with addition of 5 µl of hispidin or 5 µl of water instead of the test specimen aliquot. It could be said that hispidin is present in the specimen in the detected amounts, if luminescence emitted by the test specimen exceeds a background signal recored from a specimen with water.

Sensitivity: the kit enables to determine presence of hispidin in a medium at concentration exceeding 100 pM.

Storage conditions: all kit components should be stored at temperature not exceeding -20°C.

**Reagent Kit No. 6** includes nucleic acid coding hispidin hydroxylase of the invention. For example, hispidin hydroxylase, which amino acid sequence is selected from the group of SEQ ID NOs: 2, 4, 6, 8 10, 12, 14, 16, 18, 20, 22, 24, 26, 28.

Reagent kit could also comprise directions for use of nucleic acid.

Reagent kit could also comprise deionized water or buffer for dissolving lyophilized nucleic acid and/or diluting nucleic acid solution.

Reagent kit could also comprise primers, complementary to regions of the said nucleic acid, for amplification of nucleic acid or its fragment.

Reagent kit could be used for producing recombinant hispidin hydroxylase of the invention or for hispidin hydroxylase expression in cells and/or cell lines, and/or organisms. After nucleic acid expression in cells, cell lines and/or organisms these cells, cell lines and/or organisms acquire the ability to catalyze transformation of exogenous or endogenous 6-(2-arylvinyl)-4-hydroxy-2H-pyran-2-one, having the structural formula 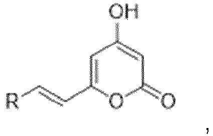 into 6-(2-arylvinyl)-3,4-dihydroxy-2H-pyran-2-one, having the structural formula 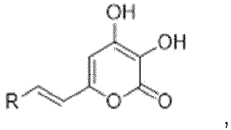 where R is aryl or heteroaryl. For example, they acquire the ability to catalyze transformation of hispidin into 3-hydroxyhispidin.

Reagent Kit **No. 7** includes nucleic acid coding hispidin-synthase of the invention. For example, hispidin-synthase, which amino acid sequence is selected from the group of SEQ ID NOs: 35, 37, 39, 41, 43, 45, 47, 49, 51, 53, 55.

Reagent kit could also comprise directions for use of nucleic acid.

Reagent kit could also comprise deionized water or buffer for dissolving lyophilized nucleic acid and/or diluting nucleic acid solution.

Reagent kit could also comprise primers, complementary to regions of the said nucleic acid, for amplification of nucleic acid or its fragment.

Reagent kit could also comprise nucleic acid coding 4'-phosphopantetheinyl transferase, e.g. 4'-phosphopantetheinyl transferase, having amino acid sequence shown in SEQ ID NO 105.

Reagent kit could be used for producing recombinant hispidin synthase of the invention or for hispidin hydroxylase expression in cells and/or cell lines, and/or organisms.

After nucleic acid expression in cells, cell lines and/or organisms these cells, cell lines and/or organisms acquire the ability to catalyze transformation of 3-arylacrylic acid with the structural formula 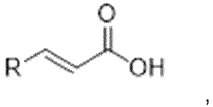 where R is aryl or heteroaryl, into 6-2-arylvinyl)-4-hydroxy-2H-pyran-2-one, having the structural formula

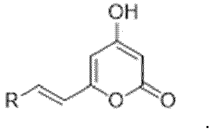

For example, they acquire the ability to catalyze transformation of caffeic acid into hispidin and/or cinnamic acid into (E)-4-hydroxy-6-styryl-2H-pyran-2-one and/or paracoumaric acid into bisnoryangonin and/or (E)-3-(6-hydroxynaphthalen-2-yl) of propenoic acid into (E)-4-hydroxy-6-(2-(6-hydroxynaphthalen-2-yl)vinyl)-2H-pyran-2-one and/or (E)-3-(1H-indol-3-yl) of propenoic acid into (E)-6-(2-(1H-indol-3-yl)vinyl)-4-hydroxy-2H-pyran-2-one.

Reagent kit could also comprise nucleic acids coding tyrosine-ammonia-lyase and the components HpaB and HpaC of 4-hydroxyphenyl acetate 3-monooxygenase-reductase. The kit with such composition could be used for producing hispidin from tyrosine in expression systems *in vitro* and *in vivo.*

**Reagent Kit No. 8** includes nucleic acid coding hispidin synthase of the invention and nucleic acid coding hispidin hydroxylase of the invention. For example, hispidin-synthase, which amino acid sequence is selected from the group of SEQ ID NOs: 35, 37, 39, 41, 43, 45, 47, 49, 51, 53, 55; and hispidin hydroxylase, which amino acid sequence is selected from the group of SEQ ID NOs: 2, 4, 6, 8 10, 12, 14, 16, 18, 20, 22, 24, 26, 28.

Reagent kit could also include directions for use of nucleic acids.

Reagent kit could also comprise deionized water or buffer for dissolving lyophilized nucleic acid and/or diluting nucleic acid solution.

Reagent kit could also comprise primers, complementary to regions of the nucleic acids included into the kit, for amplification of these nucleic acids or their fragments.

Reagent kit could also comprise nucleic acid coding 4'-phosphopantetheinyl transferase, e.g. 4'-phosphopantetheinyl transferase, having amino acid sequence shown in SEQ ID NO 105.

Reagent kit could also comprise nucleic acids coding enzymes of 3-arylacrylic acid biosynthesis from cell metabolites, e.g. nucleic acids coding tyrosine-ammonia-lyase and the components HpaB and HpaC of 4-hydroxyphenyl acetate 3-monooxygenase-reductase.

The kit could be used for any purposes described for kits 6 and 7. The kit could be used for expression of hispidin hydroxylase and hispidin synthase in cells and/or cell lines, and/or organisms. After nucleic acid expression in cells, cell lines and/or organisms these cells, cell lines and/or organisms acquire the ability to produce 6-(2-arylvinyl)-3.4-dihydroxy-2H-pyran-2-one, having the structural formula 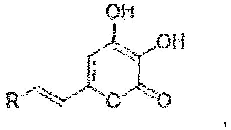 r where R is aryl or heteroaryl, from the corresponding 3-arylacrylic acid with the structural formula

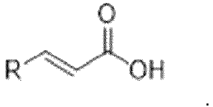

The kit could be used for expression of hispidin hydroxylase and hispidin synthase together with tyrosine-ammonia-lyase and the components HpaB and HpaC of 4-hydroxyphenyl acetate 3-monooxygenase-reductase in cells and/or cell lines, and/or organisms. After nucleic acid expression in cells, cell lines and/or organisms these cells, cell lines and/or organisms acquire the ability to produce hispidin from tyrosine and cell metabolites.

**Reagent Kit No. 9** includes nucleic acid coding hispidin hydroxylase of the invention. For example, hispidin hydroxylase, which amino acid sequence is selected from the group of SEQ ID NOs: 2, 4, 6, 8 10, 12, 14, 16, 18, 20, 22, 24, 26, 28 and nucleic acid coding luciferase able to oxidize at least one of fungal luciferins with luminescence emission. For example, there could be selected luciferase, which amino acid sequence is selected from the group of SEQ ID NOs: 80, 82, 84, 86, 88, 90, 92, 94, 96, 98.

Reagent kit could also include directions for use of nucleic acids.

Reagent kit could also comprise deionized water or buffer for dissolving lyophilized nucleic acid and/or diluting nucleic acid solution.

Reagent kit could also comprise primers, complementary to regions of the nucleic acids included into the kit, for amplification of these nucleic acids or their fragments.

The kit could be used for labeling of cells and/or cell lines, and/or organisms, where the said cells, cell lines and/or organisms acquire bioluminescence ability in the presence of exogenous or endogenous fungal preluciferin as a result of expression of the said nucleic acids. For example, they acquire bioluminescence ability in the presence of hispidin.

The kit could be also used for study of target gene promoters co-activation.

The kit could also include a nucleic acid coding hispidin-synthase of the invention, e.g. hispidin-synthase, which amino acid sequence is selected from the group of SEQ ID NOs: 35, 37, 39, 41, 43, 45, 47, 49, 51, 53, 55. In this case the kit could be used for producing cells, cell lines and transgenic organisms capable of bioluminescence in the presence of exogenous or endogenous 3-arylacrylic acid with the structural formula 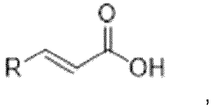 where R is aryl or heteroaryl. For example, in the presence of 3-arylacrylic acid selected from the following group: caffeic acid or cinnamic acid, or paracoumaric acid, or coumaric acid, or umbellic acid, or sinapic acid, or ferulic acid. In particular, the kit could be used for producing autonomously bioluminescent transgenic organisms, e.g. plants or fungi).

The kit could also include a nucleic acid coding 4'-phosphopantetheinyl transferase, e.g. 4'-phosphopantetheinyl transferase, having amino acid sequence shown in SEQ ID NO 105, or similar.

The kit could also comprise nucleic acids coding enzymes of 3-arylacrylic acid biosynthesis from cell metabolites.

The kit could also comprise a nucleic acid coding caffeylpyruvate hydrolase of the invention, e.g. caffeylpyruvate hydrolase, which amino acid sequence is selected from the group of SEQ ID NOs: 65, 67, 69, 71, 73, 75.

The kit could be also used for any purposes described for kits No. 6 and No. 8.

The kit could be also used for producing cell lines enabling to identify caffeic acid in test specimen.

**Reagent Kit No.10** includes *Agrobacterium tumefaciens* cells of AGLO strain, carrying plasmid comprising coding sequences of hispidin hydroxylase, hispidin synthase, luciferase, phosphopantetheinyl transferase NpgA gene and antibiotic resistance gene (e.g. kanamycin) under control of suitable promoter, e.g. 35S promoter from cauliflower mosaic virus.

Reagent kit could also include primers for determining correctness of expression cassette integration into dicotyledon flowering plant cells.

Reagent kit could be used for growing autonomously bioluminescent dicotyledon plants.

Reagent kit could also include directions for use.

**Method of application:** Make transformation of a dicotyledon plant using agrobacteria cells from the kit according to the protocol perfectly suitable for this plant species. Make plant selection in antibiotic medium (e.g. kanamycin). Make correction of expression cassette full-length integration using PCR with kit primers.

Storage conditions: competent agrobacteria cells should be stored at a temperature not exceeding -70°C, it is allowed to store caffeic acid solution at temperatures not exceeding -20°C.

### Reagent Kit No. 11

The kit includes a purified product of PKS and a purified product of hispidin hydroxylase of the invention, and it could be used for producing fungal luciferin from caffeyl-CoA. Reagent kit could also include a reaction buffer: 0.2 M sodium phosphate buffer (pH 8.0) laced with 0.5 M of Na₂SO₄, 0.1% of dodecylmaltoside (DDM), 1 mM of NADPH, 10 mM of ATP, 1 mM of malonyl-CoA, or components for reaction buffer preparation. Reagent kit could also include deionized water. Reagent kit could also include directions for use.

### Reagent Kit No. 12

The kit includes a nucleic acid coding PKS and a nucleic acid coding hispidin hydroxylase of the invention. For example, PKS which amino acid sequence is selected from the group of SEQ ID NOs: 119, 121, 123, 125, 127, 129, 131, 133, 135, 137, 139 and hispidin hydroxylase, which amino acid sequence is selected from the group of SEQ ID NOs: 2, 4, 6, 8 10, 12, 14, 16, 18, 20, 22, 24, 26, 28.

Reagent kit could also include directions for use of nucleic acids. Reagent kit could also comprise deionized water or buffer for dissolving lyophilized nucleic acid and/or diluting nucleic acid solution. Reagent kit could also comprise primers, complementary to regions of the nucleic acids included into the kit, for amplification of these nucleic acids or their fragments.

Reagent kit could also comprise a nucleic acid coding coumarate-CoA ligase, e.g. coumarate-CoA ligase, having amino acid sequence shown in SEQ ID NO 141.

Reagent kit could also comprise nucleic acids coding enzymes of caffeic acid biosynthesis from cell metabolites, e.g. nucleic acids coding tyrosine-ammonia-lyase and the components HpaB and HpaC of 4-hydroxyphenyl acetate 3-monooxygenase-reductase. Reagent kit could also comprise a nucleic acid coding caffeylpyruvate hydrolase of the invention.

Reagent kit could be used for expression of hispidin hydroxylase and PKS in cells and/or cell lines, and/or organisms. After nucleic acid expression in cells, cell lines and/or organisms these cells, cell lines and/or organisms acquire the ability to produce 3-hydroxyhispidin from caffeic acid. The kit could also be used for expression of hispidin hydroxylase and PKS together with coumarate-CoA ligase, caffeoyl pyruvate hydrolase and/or combination of tyrosine-ammonia-lyase and the components HpaB and HpaC of 4-hydroxyphenyl acetate 3-monooxygenase-reductase in cells and/or cell lines, and/or organisms. After nucleic acid expression in cells, cell lines and/or organisms these cells, cell lines and/or organisms acquire the ability to produce 3-hydroxyhispidin from tyrosine and cell metabolites.

The kit could also comprise a nucleic acid coding luciferase able to oxidize 3-hydroxyhispidin with luminescence emission. In this case the kit could be used for labeling of cells and/or cell lines, and/or organisms, where the said cells, cell lines and/or organisms acquire bioluminescence ability in the presence of exogenous or endogenous hispidin and/or caffeyl-CoA, and/or caffeic acid as a result of expression of the said nucleic acids. For example, cells, cell lines and/or organisms acquire autonomous bioluminescence ability.

## Claims

1. A fungal luciferin biosynthesis protein selected from the group:
(a) hispidin hydroxylases having the amino acid sequence that within at least 350 amino acids has at least 60% identity with the amino acid sequence selected from the following SEQ ID NOs group: 2, 4, 6, 8, 10, 12, 14, 16, 18, 20, 22, 24, 26, 28, or contains consensus sequences with the SEQ ID NOs 29-33 separated by non-conservative amino acid insertion segments, the hispidin hydroxylase catalyzing conversion of 6-(2-arylvinyl)-4-hydroxy-2H-pyran-2-one with the structural formula 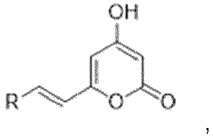 into 6-(2-arylvinyl)-3,4-dihydroxy-2H-pyran-2-one with the structural formula 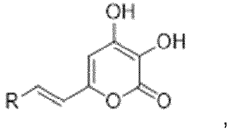 where R is aryl or heteroaryl;
(b) hispidin synthases having the amino acid sequence that has at least 45% identity with the amino acid sequence selected from the following SEQ ID NOs group: 35, 37, 39, 41, 43, 45, 47, 49, 51, 53, 55, or contains consensus sequences with the SEQ ID NOs 56-63 separated by non-conservative amino acid insertion segments, the hispidin synthase catalyzing conversion of 3-aryl acrylic acid with the structural formula 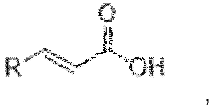 where R is aryl or heteroaryl, into 6-(2-arylvinyl)-4-hydroxy-2H-pyran-2-one with the structural formula 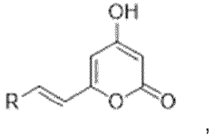 where R is aryl or heteroaryl;
(c) caffeylpyruvate hydroxylases having the amino acid sequence that has at least 60% identity with the amino acid sequence selected from the following SEQ ID NOs group: 65, 67, 69, 71, 73, 75, or contains consensus sequences with the SEQ ID NOs 76-78 separated by non-conservative amino acid insertion segments, the caffeylpyruvate hydroxylase catalyzing conversion of 6-aryl-2-hydroxy-4-oxohexa-2,5-dienoic acid with the structural formula 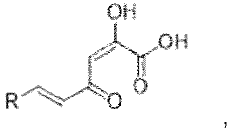 where R is aryl or heteroaryl, conversion into 3-arylacrylic acid with the structural formula

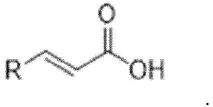

2. The protein according to claim 1, wherein the amino acid sequence of hispidin hydroxylase has at least 65% identity, or at least 70% identity, or at least 75% identity, or at least 80% identity, or at least 85% identity, or at least 90% identity, or at least 95% identity with an amino acid sequence selected from the following group of SEQ ID NOs: 2, 4, 6, 8 10, 12, 14, 16, 18, 20, 22, 24, 26, 28.

3. The protein according to claim 2, wherein the amino acid sequence of hispidin hydroxylase is selected from the following group of SEQ ID NOs: 2, 4, 6, 8 10, 12, 14, 16, 18, 20, 22, 24, 26, 28, or has at least 90%, 91%, 92%, 93%, 94%, 95%, 96%, 97%, 98%, 98%, or 99% identity with it.

4. The protein according to claim 1, wherein the amino acid sequence of hispidin synthase has at least 50% identity, or at least 55% identity, or at least 60% identity, or at least 65% identity, or at least 70% identity, or at least 75% identity, or at least 80% identity, or at least 85% identity, or at least 90% identity, or at least 95% identity with an amino acid sequence selected from the following group of SEQ ID NOs: 35, 37, 39, 41, 43, 45, 47, 49, 51, 53, 55.

5. The protein according to claim 4, wherein the amino acid sequence of hispidin synthase is selected from the following group of SEQ ID NOs: 35, 37, 39, 41, 43, 45, 47, 49, 51, 53, 55, or has at least 90%, 91%, 92%, 93%, 94%, 95%, 96%, 97%, 98%, 98%, or 99% identity with it.

6. The protein according to claim 1, wherein the amino acid sequence of caffeylpyruvate hydrolase has at least 65% identity, or at least 70% identity, or at least 75% identity, or at least 80% identity, or at least 85% identity, or at least 90% identity, or at least 95% identity with an amino acid sequence selected from the following group of SEQ ID NOs: 65, 67, 69, 71, 73, 75.

7. The protein according to claim 6, wherein the amino acid sequence of caffeylpyruvate synthase is selected from the following group of SEQ ID NOs: 65, 67, 69, 71, 73, 75, or has at least 90%, 91%, 92%, 93%, 94%, 95%, 96%, 97%, 98%, 98%, or 99% identity with it.

8. A fusion protein, which comprises operatively cross-linked hispidin hydroxylase, and/or hispidin synthase, and/or caffeylpyruvate hydrolase according to claim 1, and luciferase capable to oxidize fungal luciferin with light emission, and/or intracellular localization signal, and/or signal peptide.

9. The fusion protein according to claim 8, wherein amino acid sequence of luciferase is at least 40% identical, for example, at least 45% identical, or at least 50% identical, or at least 55% identical, or at least 60% identical, or at least 70% identical, or at least 75% identical, or at least 80% identical, or at least 85% identical to an amino acid sequence selected from the following SEQ ID NOs group: 80, 82, 84, 86, 88, 90, 92, 94, 96, 98.

10. The fusion protein according to claim 9, wherein the amino acid sequence has the SEQ ID No. 101.

11. The protein according to any one of claims 1-10, wherein 6-(2-arylvinyl)-4-hydroxy-2H-pyran-2-one is selected from the group:
(E)-6-(3,4-dihydroxystyryl)-4-hydroxy-2H-pyran-2-one,
(E)-4-dihydroxy-6-styryl-2H-pyran-2-one,
(E)-4-hydroxy-6-(4-hydroxystyryl)-2H-pyran-2-one,
(E)-4-hydroxy-6-(2-hydroxystyryl)-2H-pyran-2-one,
(E)-4-hydroxy-6-(2,4-dihydroxystyryl)-2H-pyran-2-one,
(E)-4-hydroxy-6-(4-hydroxy-3,5-dimethoxystyryl)-2H-pyran-2-one,
(E)-4-hydroxy-6-(4-hydroxy-3-methoxystyryl)-2H-pyran-2-one,
(E)-4-hydroxy-6-(2-(6-hydroxynaphthalen-2-yl)vinyl)-2H-pyran-2-one,
(E)-6-(4-aminostyryl)-4-hydroxy-2H-pyran-2-one,
(E)-6-(4-(diethylamino)styryl)-4-hydroxy-2H-pyran-2-one,
(E)-6-(2-(1H-indol-3-yl)vinyl)-4-hydroxy-2H-pyran-2-one,
(E)-4-hydroxy-6-(2,3,6,7-tetrahydro-1H,5H-pyrido[3,2,1-ij]quinolin-9-yl)vinyl)-2H-pyran-2-one.

12. The protein according to any one of claims 1-7, wherein 3-aryl acrylic acid is selected from the group comprising: caffeic acid, cinnamic acid, paracoumaric acid, coumaric acid, umbellic acid, sinapic acid, and ferulic acid.

13. A use of fungal luciferin biosynthesis protein selected from the group:
(a) the amino acid sequence that within at least 350 amino acids has at least 60% identity with the amino acid sequence selected from the following SEQ ID NOs group: 2, 4, 6, 8, 10, 12, 14, 16, 18, 20, 22, 24, 26, 28, or contains consensus sequences with the SEQ ID NOs 29-33 separated by non-conservative amino acid insertion segments, as hispidin hydroxylases catalyzing conversion of 6-(2-arylvinyl)-4-hydroxy-2H-pyran-2-one with the structural formula 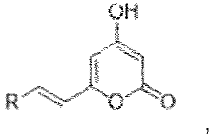 into 6-(2-arylvinyl)-3,4-dihydroxy-2H-pyran-2-one with the structural formula 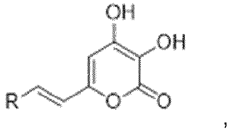 where R is aryl or heteroaryl;
(b) the amino acid sequence that has at least 45% identity with the amino acid sequence selected from the following SEQ ID NOs group: 35, 37, 39, 41, 43, 45, 47, 49, 51, 53, 55, or contains consensus sequences with the SEQ ID NOs 56-63 separated by non-conservative amino acid insertion segments, as hispidin synthases catalyzing conversion of 3-aryl acrylic acid with the structural formula 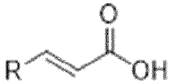 into 6-(2-arylvinyl)-4-hydroxy-2H-pyran-2-one with the structural formula 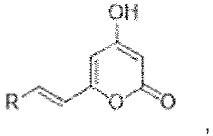 where R is aryl or heteroaryl;
(c) the amino acid sequence that has at least 60% identity with the amino acid sequence selected from the following SEQ ID NOs group: 65, 67, 69, 71, 73, 75, or contains consensus sequences with the SEQ ID NOs 76-78 separated by non-conservative amino acid insertion segments, as caffeylpyruvate hydroxylase catalyzing conversion of 6-aryl-2-hydroxy-4-oxohexa-2,5-dienoic acid with the structural formula 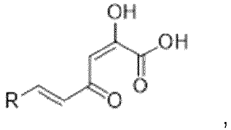 where R is aryl or heteroaryl, conversion into 3-arylacrylic acid with the structural formula

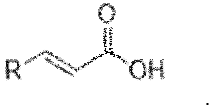

14. The use according to claim 13, wherein the amino acid sequence of hispidin hydroxylase has at least 65% identity, or at least 70% identity, or at least 75% identity, or at least 80% identity, or at least 85% identity, or at least 90% identity, or at least 95% identity with an amino acid sequence selected from the following group of SEQ ID NOs: 2, 4, 6, 8 10, 12, 14, 16, 18, 20, 22, 24, 26, 28.

15. The use according to claim 13, wherein the amino acid sequence of hispidin synthase has at least 50% identity, or at least 55% identity, or at least 60% identity, or at least 65% identity, or at least 70% identity, or at least 75% identity, or at least 80% identity, or at least 85% identity, or at least 90% identity, or at least 95% identity with an amino acid sequence selected from the following group of SEQ ID NOs: 35, 37, 39, 41, 43, 45, 47, 49, 51, 53, 55.

16. The use according to claim 13, wherein the amino acid sequence of caffeylpyruvate hydrolase has at least 65% identity, or at least 70% identity, or at least 75% identity, or at least 80% identity, or at least 85% identity, or at least 90% identity, or at least 95% identity with an amino acid sequence selected from the following group of SEQ ID NOs: 65, 67, 69, 71, 73, 75.

17. The use according to any one of claims 13-16, wherein 6-(2-arylvinyl)-4-hydroxy-2H-pyran-2-one is selected from the group:
(E)-6-(3,4-dihydroxystyryl)-4-hydroxy-2H-pyran-2-one (hispidin),
(E)-4-dihydroxy-6-styryl-2H-pyran-2-one,
(E)-4-hydroxy-6-(4-hydroxystyryl)-2H-pyran-2-one (bisnoryangonin),
(E)-4-hydroxy-6-(2-hydroxystyryl)-2H-pyran-2-one,
(E)-4-hydroxy-6-(2,4-dihydroxystyryl)-2H-pyran-2-one,
(E)-4-hydroxy-6-(4-hydroxy-3,5-dimethoxystyryl)-2H-pyran-2-one,
(E)-4-hydroxy-6-(4-hydroxy-3-methoxystyryl)-2H-pyran-2-one,
(E)-4-hydroxy-6-(2-(6-hydroxynaphthalen-2-yl)vinyl)-2H-pyran-2-one,
(E)-6-(4-aminostyryl)-4-hydroxy-2H-pyran-2-one,
(E)-6-(4-(diethylamino)styryl)-4-hydroxy-2H-pyran-2-one,
(E)-6-(2-(1H-indol-3-yl)vinyl)-4-hydroxy-2H-pyran-2-one,
(E)-4-hydroxy-6-(2,3,6,7-tetrahydro-1H,5H-pyrido[3,2,1-ij]quinolin-9-yl)vinyl)-2H-pyran-2-one.

18. The use according to any one of claims 13-16, wherein 3-aryl acrylic acid is selected from the group comprising: caffeic acid, cinnamic acid, paracoumaric acid, coumaric acid, umbellic acid, sinapic acid, and ferulic acid.

19. A nucleic acid encoding the fungal luciferin biosynthesis protein according to any one of claims 1-10, selected from the group:
(a) hispidin hydroxylases having the amino acid sequence that within at least 350 amino acids has at least 60% identity with the amino acid sequence selected from the following SEQ ID NOs group: 2, 4, 6, 8, 10, 12, 14, 16, 18, 20, 22, 24, 26, 28, or contains consensus sequences with the SEQ ID NOs 29-33 separated by non-conservative amino acid insertion segments, the hispidin hydroxylase catalyzing conversion of 6-(2-arylvinyl)-4-hydroxy-2H-pyran-2-one with the structural formula 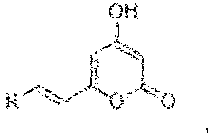 into 6-(2-arylvinyl)-3,4-dihydroxy-2H-pyran-2-one with the structural formula 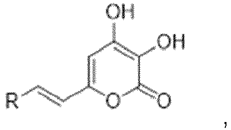 where R is aryl or heteroaryl;
(b) hispidin synthases having the amino acid sequence that has at least 45% identity with the amino acid sequence selected from the following SEQ ID NOs group: 35, 37, 39, 41, 43, 45, 47, 49, 51, 53, 55, or contains consensus sequences with the SEQ ID NOs 56-63 separated by non-conservative amino acid insertion segments, the hispidin synthase catalyzing conversion of 3-aryl acrylic acid with the structural formula 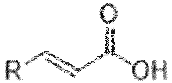 into 6-(2-arylvinyl)-4-hydroxy-2H-pyran-2-one with the structural formula 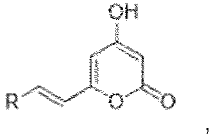 where R is aryl or heteroaryl;
(c) caffeylpyruvate hydroxylases having the amino acid sequence that has at least 60% identity with the amino acid sequence selected from the following SEQ ID NOs group: 65, 67, 69, 71, 73, 75, or contains consensus sequences with the SEQ ID NOs 76-78 separated by non-conservative amino acid insertion segments, the caffeylpyruvate hydroxylase catalyzing conversion of 6-aryl-2-hydroxy-4-oxohexa-2,5-dienoic acid with the structural formula 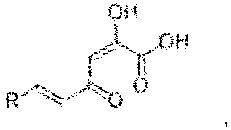 where R is aryl or heteroaryl, conversion into 3-arylacrylic acid with the structural formula

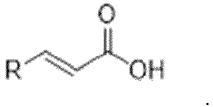

20. The nucleic acid according to claim 19, wherein the amino acid sequence of hispidin hydroxylase has at least 65% identity, or at least 70% identity, or at least 75% identity, or at least 80% identity, or at least 85% identity, or at least 90% identity, or at least 95% identity with an amino acid sequence selected from the following group of SEQ ID NOs: 2, 4, 6, 8 10, 12, 14, 16, 18, 20, 22, 24, 26, 28.

21. The nucleic acid according to claim 20, wherein the amino acid sequence is selected from the following group of SEQ ID NOs: 2, 4, 6, 8 10, 12, 14, 16, 18, 20, 22, 24, 26, 28, or has at least 96%, 97%, 98%, 98%, or 99% identity with it.

22. The nucleic acid according to claim 19, wherein the amino acid sequence of hispidin synthase has at least 50% identity, or at least 55% identity, or at least 60% identity, or at least 65% identity, or at least 70% identity, or at least 75% identity, or at least 80% identity, or at least 85% identity, or at least 90% identity, or at least 95% identity with an amino acid sequence selected from the following group of SEQ ID NOs: 35, 37, 39, 41, 43, 45, 47, 49, 51, 53, 55.

23. The nucleic acid according to claim 22, wherein the amino acid sequence of hispidin synthase is selected from the following group of SEQ ID NOs: 35, 37, 39, 41, 43, 45, 20, 49, 51, 53, 55, or has at least 96%, 97%, 98%, 98%, or 99% identity with it.

24. The nucleic acid according to claim 19, wherein the amino acid sequence of caffeylpyruvate hydrolase has at least 65% identity, or at least 70% identity, or at least 75% identity, or at least 80% identity, or at least 85% identity, or at least 90% identity, or at least 95% identity with an amino acid sequence selected from the following group of SEQ ID NOs: 65, 67, 69, 71, 73, 75.

25. The nucleic acid according to claim 24, wherein the amino acid sequence of caffeylpyruvate synthase is selected from the following group of SEQ ID NOs: 65, 67, 69, 71, 73, 75, or has at least 96%, 97%, 98%, 98%, or 99% identity with it.

26. The nucleic acid encoding the fusion protein according to any one of claims 8-10.

27. The nucleic acid according to claim 19, wherein 6-(2-arylvinyl)-4-hydroxy-2H-pyran-2-one is selected from the group:
(E)-6-(3,4-dihydroxystyryl)-4-hydroxy-2H-pyran-2-one (hispidin),
(E)-4-dihydroxy-6-styryl-2H-pyran-2-one,
(E)-4-hydroxy-6-(4-hydroxystyryl)-2H-pyran-2-one (bisnoryangonin),
(E)-4-hydroxy-6-(2-hydroxystyryl)-2H-pyran-2-one,
(E)-4-hydroxy-6-(2,4-dihydroxystyryl)-2H-pyran-2-one,
(E)-4-hydroxy-6-(4-hydroxy-3,5-dimethoxystyryl)-2H-pyran-2-one,
(E)-4-hydroxy-6-(4-hydroxy-3-methoxystyryl)-2H-pyran-2-one,
(E)-4-hydroxy-6-(2-(6-hydroxynaphthalen-2-yl)vinyl)-2H-pyran-2-one,
(E)-6-(4-aminostyryl)-4-hydroxy-2H-pyran-2-one,
(E)-6-(4-(diethylamino)styryl)-4-hydroxy-2H-pyran-2-one,
(E)-6-(2-(1H-indol-3-yl)vinyl)-4-hydroxy-2H-pyran-2-one,
(E)-4-hydroxy-6-(2,3,6,7-tetrahydro-1H,5H-pyrido[3,2,1-ij]quinolin-9-yl)vinyl)-2H-pyran-2-one.

28. The nucleic acid according to claim 19, wherein 3-aryl acrylic acid is selected from the group comprising: caffeic acid, cinnamic acid, paracoumaric acid, coumaric acid, umbellic acid, sinapic acid, and ferulic acid.

29. An expression cassette comprising: (a) a domain of transcription initiation, which is functional in a host cell; (b) the nucleic acid according to claim 19, and (c) a domain of transcription termination, which is functional in the host cell.

30. A host cell containing at least one expression cassette according to claim 29 as a part of an extrachromosomal element or integrated into the cell genome as a result of introducing said cassette into said cell, wherein said cell expresses at least one of the functional proteins for fungal luciferin biosynthesis.

31. An antibody that binds to at least one protein according to any one of claims 1-7.

32. A use of a nucleic acid encoding a fungal luciferin biosynthesis protein selected from the group:
(a) the amino acid sequence that within at least 350 amino acids has at least 60% identity with the amino acid sequence selected from the following SEQ ID NOs group: 2, 4, 6, 8, 10, 12, 14, 16, 18, 20, 22, 24, 26, 28, or contains consensus sequences with the SEQ ID NOs 29-33 separated by non-conservative amino acid insertion segments, to produce in in vitro or in vivo systems the hispidin hydroxylase catalyzing the reaction of 6-(2-arylvinyl)-4-hydroxy-2H-pyran-2-one with the structural formula 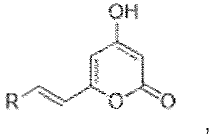 conversion into 6-(2-arylvinyl)-3,4-dihydroxy-2H-pyran-2-one with the structural formula 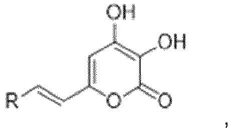 where R is aryl or heteroaryl;
(b) the amino acid sequence that has at least 45% identity with the amino acid sequence selected from the following SEQ ID NOs group: 35, 37, 39, 41, 43, 45, 47, 49, 51, 53, 55, or contains consensus sequences with the SEQ ID NOs 56-63 separated by non-conservative amino acid insertion segments, to produce in in vitro or in vivo systems the hispidin synthase catalyzing the reaction of conversion 3-aryl acrylic acid with the structural formula 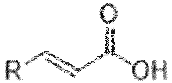 into 6-(2-arylvinyl)-4-hydroxy-2H-pyran-2-one with the structural formula 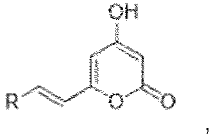 where R is aryl or heteroaryl;
(c) the amino acid sequence that has at least 60% identity with the amino acid sequence selected from the following SEQ ID NOs group: 65, 67, 69, 71, 73, 75, or contains consensus sequences with the SEQ ID NOs 76-78 separated by non-conservative amino acid insertion segments, to produce in in vitro or in vivo systems the caffeylpyruvate hydrolase catalyzing the reaction of an 6-aryl-2-hydroxy-4-oxohexa-2,5-dienoic acid with the structural formula 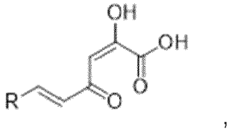 where R is aryl or heteroaryl, conversion into 3-arylacrylic acid with the structural formula

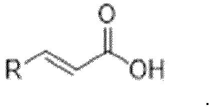

33. The use according to claim 32, wherein the nucleic acid is used to express a fungal luciferin biosynthesis protein contained in an expression cassette that also comprises a domain of transcription initiation, which is functional in a host cell and a domain of transcription termination, which is functional in the host cell.

34. The use according to claim 33, wherein the expression cassette is used in a host cell.

35. A method of biosynthesis a fungal luciferin with the chemical formula 6-(2-arylvinyl)-3,4-dihydroxy-2H-pyran-2-one and the structural formula 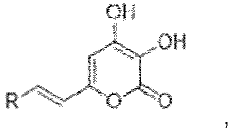 where R is aryl or heteroaryl, in either *in vitro* or *in vivo* system, which comprises combining at least one moiety of hispidin hydroxylase according to any one of claims 1-3 with at least one moiety of 6-(2-arylvinyl)-4-hydroxy-2H-pyran-2-one having the structural formula 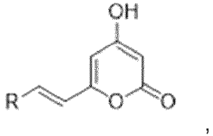 at least one NAD(P)H moiety, and at least one molecular oxygen moiety under physiological conditions.

36. The method according to claim 35, wherein the reaction is performed in a cell or organism, the method comprising introducing into the cell of the expression cassette according to claim 29 that contains a hispidin hydroxylase encoding nucleic acid.

37. The method according to claim 36, comprising introducing into a cell or organism the expression cassette further containing: (a) a domain of transcription initiation, which is functional in a host cell; (b) a nucleic acid, which encodes the luciferase capable to oxidize fungal luciferin with light emission, and (c) a domain of transcription termination, which is functional in the host cell, wherein the said cell or organism acquires the ability to bioluminescence in the presence of said fungal luciferin.

38. The method according to claim 37, wherein the nucleic acid, which encodes the luciferase, is operatively fused with the nucleic acid, which encodes the hispidin hydroxylase to form the nucleic acid of claim 26.

39. A method of biosynthesis a fungal luciferin with the chemical formula 6-(2-arylvinyl)-4-hydroxy-2H-pyran-2-one and the structural formula 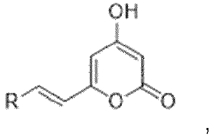 where R is aryl or heteroaryl, in either *in vitro* or *in vivo* system, which comprises combining at least one moiety of 3-aryl acrylic acid with the structural formula 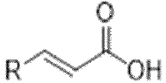 with at least one moiety of hispidin synthase according to the to any one of claims 1, 4, 5, at least one moiety of coenzyme A, at least one ATP moiety, and at least two malonyl-CoA moieties under physiological conditions.

40. The method according to claim 39, wherein the reaction is performed in a cell or organism, the method comprising introducing into the cell of the expression cassette according to claim 29 that contains a hispidin synthase encoding nucleic acid.

41. The method according to claim 40, further comprising introducing into the cell or organism a nucleic acid encoding a 4'-phosphopantotheinyl transferase and capable to transfer the 4-phosphopantotheinyl from coenzyme A to serine in the acyl transfer domain of polyketide synthases.

42. The method according to claim 41, wherein the 4'-phosphopantotheinyl transferase has an amino acid sequence at least 40% identical to amino acid sequence with SEQ ID No. 105.

43. The method according to any one of claims 40-42, further comprising introducing into the cell or organism nucleic acids, which encode enzymes for biosynthesis of 3-aryl acrylic acid from cell metabolites with the structural formula 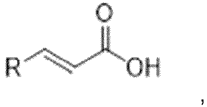 where R is aryl or heteroaryl.

44. The method according to claim 43, wherein the enzymes for biosynthesis of 3-aryl acrylic acid are selected from the group of:
(a) tyrosine ammonia-lyase with an amino acid sequence at least 40% identical to the amino acid sequence having SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID NOs 109 and 111;
(b) phenylalanine ammonia-lyase with an amino acid sequence at least 40% identical to the amino acid sequence having SEQ ID No.117.

45. A method of biosynthesis a fungal luciferin with the chemical formula 6-(2-arylvinyl)-3,4-dihydroxy-2H-pyran-2-one and the structural formula 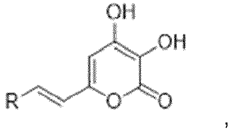 where R is aryl or heteroaryl, in either *in vitro* or *in vivo* system, which comprises combining at least one moiety of 3-aryl acrylic acid having the structural formula 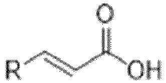 with at least one moiety of hispidin synthase according to the to any one of claims 1, 4, 5; at least one moiety of coenzyme A; at least one ATP moiety; at least two malonyl-CoA moieties; at least one moiety of hispidin hydroxylase according to the to any one of claims 1-3; at least one NAD(P)H moiety, and at least one molecular oxygen moiety under physiological conditions.

46. The method according to claim 45, wherein the reaction is performed in a cell or organism, the method comprising introducing into the cell of the expression cassette according to claim 29 that contains a hispidin synthase encoding nucleic acid, and the expression cassette according to claim 29 that contains a hispidin hydroxylase encoding nucleic acid.

47. The method according to claim 46, further comprising introducing into the cell or organism a nucleic acid encoding a 4'-phosphopantotheinyl transferase and capable to transfer the 4-phosphopantotheinyl from coenzyme A to serine in the acyl transfer domain of polyketide synthases.

48. The method according to claim 41, wherein the 4'-phosphopantotheinyl transferase has an amino acid sequence at least 40% identical to amino acid sequence with SEQ ID No. 105.

49. The method according to any one of claims 46-48, further comprising introducing into the cell or organism nucleic acids, which encode enzymes for biosynthesis of 3-aryl acrylic acid from cell metabolites with the structural formula 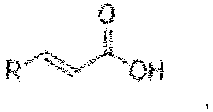 where R is aryl or heteroaryl.

50. The method according to claim 49, wherein the enzymes for biosynthesis of 3-aryl acrylic acid are selected from the group of:
(a) tyrosine ammonia-lyase with an amino acid sequence at least 40% identical to the amino acid SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID NOs 109 and 111;
(b) phenylalanine ammonia-lyase with an amino acid sequence at least 40% identical to the amino acid sequence having SEQ ID No.117.

51. A method of producing transgenic bioluminescent cell or organism comprising introducing an expression cassette according to claim 29 into the cell or organism, said expression cassette comprising a hispidin hydroxylase encoding nucleic acid and containing (a) a domain of transcription initiation, which is functional in a host cell; (b) a nucleic acid, which encodes the luciferase capable to oxidize fungal luciferin with light emission, and (c) a domain of transcription termination, which is functional in the host cell, wherein said cell acquires the ability to bioluminescence in the presence of fungal preluciferin with the chemical formula 6-(2-arylvinyl)-4-hydroxy-2H-pyran-2-one and structural formula 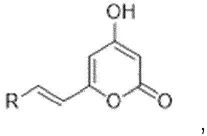 where R is aryl or heteroaryl.

52. The method according to claim 51, further comprising introducing into the cell or organism a nucleic acid encoding the hispidin synthase according to any one of claims 19, 22, 23, as a part of an expression cassette according to claim 29, wherein said cell acquires the ability to bioluminescence in the presence of an exogenous or endogenous precursor of fungal preluciferin, which is 3-aryl acrylic acid with the structural formula 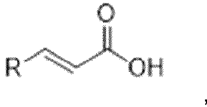 where R is aryl or heteroaryl.

53. The method according to claim 52, further comprising introducing into the cell or organism a nucleic acid encoding the caffeylpyruvate hydrolase according to any one of claims 19, 24, 25.

54. The method according to any one of claims 52-53, further comprising introducing into the cell or organism a nucleic acid encoding the 4'-phosphopantotheinyl transferase and capable to transfer the 4-phosphopantotheinyl from coenzyme A to serine in the acyl transfer domain of polyketide synthases.

55. The method according to any one of claims 52-54, further comprising introducing into the cell or organism nucleic acids, which encode enzymes for biosynthesis of 3-aryl acrylic acid from cell metabolites.

56. The method according to claim 55, wherein the enzymes for biosynthesis of 3-aryl acrylic acid are selected from the group of:
(a) tyrosine ammonia-lyase with an amino acid sequence at least 40% identical to the amino acid SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID NOs 109 and 111;
(b) phenylalanine ammonia-lyase with an amino acid sequence at least 40% identical to the amino acid sequence having SEQ ID No.117.

57. A method of producing transgenic bioluminescent cell or organism comprising introducing a nucleic acid according to claim 26 in the form of an expression cassette according to claim 29 into the cell or organism, wherein said cell acquires the ability to bioluminescence in the presence of fungal preluciferin with the chemical formula 6-(2-arylvinyl)-4-hydroxy-2H-pyran-2-one and structural formula 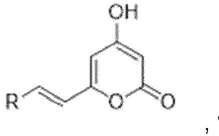 where R is aryl or heteroaryl.

58. The method according to claim 57, further comprising introducing into the cell or organism a nucleic acid encoding the hispidin synthase according to any one of claims 19, 22, 23, as a part of an expression cassette according to claim 29, wherein said cell acquires the ability to bioluminescence in the presence of an exogenous or endogenous precursor of fungal preluciferin, which is 3-aryl acrylic acid with the structural formula 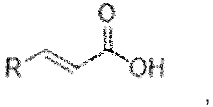 where R is aryl or heteroaryl.

59. The method according to claim 58, further comprising introducing into the cell or organism a nucleic acid encoding the caffeylpyruvate hydrolase according to any one of claims 19, 24, 25.

60. The method according to any one of claims 58-59, further comprising introducing into the cell or organism a nucleic acid encoding the 4'-phosphopantotheinyl transferase and capable to transfer the 4-phosphopantotheinyl from coenzyme A to serine in the acyl transfer domain of polyketide synthases.

61. The method according to any one of claims 58-60, further comprising introducing into the cell or organism nucleic acids, which encode enzymes for biosynthesis of 3-aryl acrylic acid from cell metabolites.

62. The method according to claim 61, wherein the enzymes for biosynthesis of 3-aryl acrylic acid are selected from the group of:
(a) tyrosine ammonia-lyase with an amino acid sequence at least 40% identical to the amino acid SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID NOs 109 and 111;
(b) phenylalanine ammonia-lyase with an amino acid sequence at least 40% identical to the amino acid sequence having SEQ ID No.117.

63. A transgenic organism capable of bioluminescence in the presence of fungal luciferin and/or fungal preluciferin, containing at least a nucleic acid encoding the hispidin hydroxylase according to any one of claims 19-21 as a part of an extrachromosomal element or integrated into genome of a cell as a result of introducing an expression cassette according to claim 29 into said cell and a nucleic acid encoding the luciferase capable to oxidize fungal luciferin with light emission.

64. A transgenic organism capable of autonomous bioluminescence, wherein said organism contains at least a nucleic acid encoding the hispidin hydroxylase according to any one of claims 19-21; a nucleic acid encoding the hispidin synthase according to any one of claims 19, 22, 23 as a part of an extrachromosomal element or integrated into genome of a cell as a result of introducing an expression cassette according to claim 29 into said cell, and a nucleic acid encoding the luciferase capable to oxidize fungal luciferin with light emission.

65. The transgenic organism according to claim 64, which contains a nucleic acid encoding the caffeylpyruvate hydrolase according to any one of claims 19, 24, 25.

66. A vector for transferring a nucleic acid into a host cell comprising at least one nucleic acid according to any one of claims 19-26.

67. A kit for producing fungal luciferin and/or fungal preluciferin comprising hispidin hydroxylase and hispidin synthase according to claim 1.

68. The kit for producing fungal luciferin and/or fungal preluciferin in *in vitro* and/or *in vivo* systems, comprising a nucleic acid encoding the hispidin hydroxylase and a nucleic acid encoding the hispidin synthase according to claim 19.

69. A kit for producing a bioluminescent cell or organism, comprising a nucleic acid encoding the hispidin hydroxylase, a nucleic acid encoding the hispidin synthase according to claim 19, and a nucleic acid encoding the luciferase capable to oxidize fungal luciferin with light emission.

70. The kit according to claim 69, further containing a nucleic acid according to claim 19, encoding a caffeylpyruvate hydrolase.

71. The kit according to any one of claims 68-70 further comprising a nucleic acid encoding the 4'-phosphopantotheinyl transferase and/or nucleic acids encoding enzymes for biosynthesizing the 3-aryl acrylic acid.

72. An use of polyketide synthase with amino acid sequence that is at least 40%, or at least 45%, or at least 50%, or at least 55 %, or at least 60%, at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99%, or completely identical to a sequence selected from the following SEQ ID NOs group: 119, 121, 123, 125, 127, 129, 131, 133, 135, 137, 139, to produce hispidin in an *in vitro* or *in vivo* system.

73. A method of producing hispidin in an *in vitro* or *in vivo* system comprising combining at least one PKS moiety according to claim 72 with at least two malonyl-CoA moieties and at least one caffeyl-CoA moiety under physiological conditions.

74. The method according to claim 73, wherein at least one caffeic acid moiety, at least one coenzyme A moiety, at least one coumarate-CoA ligase moiety, and at least one ATP moiety are added to the reaction mixture instead of at least one moiety of caffeyl-CoA.

75. The method according to claim 73 or claim 74, wherein the reaction is performed in a cell or organism, the method comprising introducing the expression cassette that contains a nucleic acid encoding the type III polyketide synthase according to claim 72.

76. The method according to claim 75, further comprising introducing a nucleic acid encoding the coumarate-CoA ligase into the cell or organism.

77. The method according to claim 76, wherein the coumarate-CoA ligase has an amino acid sequence that is at least 40%, or at least 45%, or at least 50%, or at least 55%, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99%, or completely identical to the sequence with SEQ ID No. 141.

78. The method according to any one of claims 75-77, further comprising introducing into the cell or organism nucleic acids, which encode enzymes for biosynthesis of caffeic acid.

79. The method according to claim 78, wherein the enzymes for biosynthesis of 3-aryl acrylic acid are selected from the group of:
(a) tyrosine ammonia-lyase with an amino acid sequence at least 40% identical to the amino acid SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID NOs 109 and 111;
(b) phenylalanine ammonia-lyase with an amino acid sequence at least 40% identical to the amino acid sequence having SEQ ID No.117.

80. The method according to claim 51, further comprising introducing into a cell or organism an expression cassette containing: (a) a domain of transcription initiation, which is functional in a host cell; (b) a nucleic acid, which encodes the type III polyketide synthase according to claim 72, and (c) a domain of transcription termination, which is functional in the host cell, wherein said cell acquires the ability to bioluminescence in the presence of exogenous or endogenous caffeyl-CoA.

81. The method according to claim 80, further comprising introducing into a cell or organism an expression cassette containing: (a) a domain of transcription initiation, which is functional in a host cell; (b) a nucleic acid, which encodes the coumarate-CoA ligase, and (c) a domain of transcription termination, which is functional in the host cell, wherein said cell acquires the ability to bioluminescence in the presence of caffeic acid.

82. The method according to claim 81, wherein the coumarate-CoA ligase has an amino acid sequence that is at least 40%, or at least 45%, or at least 50%, or at least 55%, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99%, or completely identical to the sequence with SEQ ID No. 141.

83. The method according to any one of claims 80-82, further comprising introducing into the cell or organism a nucleic acid encoding the caffeylpyruvate hydrolase according to any one of claims 19, 24, 25.

84. The method according to any one of claims 80-83, further comprising introducing into the cell or organism nucleic acids, which encode enzymes for biosynthesis of 3-aryl acrylic acid from cell metabolites.

85. The method according to claim 84, wherein the enzymes for biosynthesis of 3-aryl acrylic acid are selected from the group of:
(a) tyrosine ammonia-lyase with an amino acid sequence at least 40% identical to the amino acid SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID Nos 109 and 111;
(b) phenylalanine ammonia-lyase with an amino acid sequence at least 40% identical to the amino acid sequence having SEQ ID No.117.

86. The method according to claim 57, further comprising introducing into a cell or organism the expression cassette containing: (a) a domain of transcription initiation, which is functional in a host cell; (b) a nucleic acid, which encodes the type III polyketide synthase according to claim 72, and (c) a domain of transcription termination, which is functional in the host cell, wherein the said cell acquires the ability to bioluminescence in the presence of exogenous or endogenous caffeyl-CoA.

87. The method according to claim 86, further comprising introducing into a cell or organism an expression cassette containing: (a) a domain of transcription initiation, which is functional in a host cell; (b) a nucleic acid, which encodes the coumarate-CoA ligase, and (c) a domain of transcription termination, which is functional in the host cell, wherein said cell acquires the ability to bioluminescence in the presence of caffeic acid.

88. The method according to claim 87, wherein the coumarate-CoA ligase has an amino acid sequence that is at least 40%, or at least 45%, or at least 50%, or at least 55%, or at least 60%, or at least 65%, or at least 70%, or at least 80%, or at least 85%, or at least 90%, or at least 91%, or at least 92%, or at least 93%, or at least 94%, or at least 95%, or at least 96%, or at least 97%, or at least 98%, or at least 99%, or completely identical to the sequence with SEQ ID No. 141.

89. The method according to any one of claims 86-88, further comprising introducing into the cell or organism a nucleic acid encoding the caffeylpyruvate hydrolase according to any one of claims 19, 24, 25.

90. The method according to any one of claims 87-89, further comprising introducing into the cell or organism nucleic acids, which encode enzymes for biosynthesis of 3-aryl acrylic acid from cell metabolites.

91. The method according to claim 90, wherein the enzymes for biosynthesis of 3-aryl acrylic acid are selected from the group of:
(a) tyrosine ammonia-lyase with an amino acid sequence at least 40% identical to the amino acid SEQ ID No. 107; HpaB and HpaC components of 4-hydroxyphenylacetate 3-monooxygenase reductase at least 40% identical to the amino acid sequences of HpaB and HpaC components of 4-hydroxyphenylacetate 3-monooxygenase reductase of E. coli having SEQ ID Nos 109 and 111;
(b) phenylalanine ammonia-lyase with an amino acid sequence at least 40% identical to the amino acid sequence having SEQ ID No.117.

92. A transgenic organism capable of bioluminescence in the presence of 3-hydroxyhispidine, and/or hispidin, and/or caffeic acid, produced using any of methods according to any one of claims 80-91.

93. A kit for producing hispidin, comprising the polyketide synthase according to claim 72 and coumarate-CoA ligase or nucleic acids encoding them.

94. A kit for producing a bioluminescent cell or organism, comprising a nucleic acid encoding the hispidin hydroxylase, a nucleic acid encoding the polyketide synthase according to claim 72, and a nucleic acid encoding the luciferase capable to oxidize fungal luciferin with light emission.

95. The kit according to claim 94, further containing a nucleic acid encoding the caffeylpyruvate hydrolase according to claim 19.

96. The it according to any one of claims 93-94, further comprising a nucleic acid encoding the coumarate-CoA ligase and/or nucleic acids encoding enzymes for biosynthesis the 3-aryl acrylic acid.
